# EUROPEAN PATENT APPLICATION

(11) **EP 1 911 838 A1**
(43) Date of publication of application: **16.04.2008**
(21) Application number: 06121179.3
(22) Date of filing: 25.09.2006
(51) Int. Cl.: C12N 9/12

(54) **Crystalline forms of PKC alpha kinase, methods of making such crystals, and uses thereof**

(71) Applicant: Novartis AG, 4056 Basel (CH)
(72) Inventor: The designation of the inventor has not yet been filed
(74) Representative: Bourgarel, Denis Jacques Marie

(57) **Abstract**

The present invention relates generally to the crystalline forms of liganded or unliganded human PKC alpha kinase, the methods of making such crystals and their use in drug discovery. The invention more specifically relates to the use of the three-dimensional structure of the PKC alpha kinase for designing and/or identifying ligands which inhibit the biological function of PKC alpha kinase.

## Description

### Field of the Invention

The present invention relates generally to the crystalline forms of liganded or unliganded human PKC alpha kinase, the methods of making such crystals and their use in drug discovery. The invention more specifically relates to the use of the three-dimensional structure of the PKC alpha kinase for designing and/or identifying ligands which inhibit the biological function of PKC alpha kinase and/or other PKC Conventional kinases.

### Background of the Invention

Members of the protein kinase C (PKC) family of serine/threonine kinases play a critical role in the regulation of cellular differentiation and the proliferation of diverse cell types. Upon cell activation, isoform-specific signals are required to trigger translocation from the cytosol to the membrane and to induce conformational changes, which displace the pseudo substrate moiety from the catalytic domain and enable PKC to phosphorylate protein substrates. Recent studies of gene-knockout mice have shown that at least three isotypes are critical for T-cell activation: PKC alpha, beta and theta. The role of the different PKC isoforms in the immune system has recently been reviewed [Tan et al. 2003]. In particular, PKC alpha is up-regulated shortly after T cell stimulation and PKC alpha-deficient mice have a Th1 defect and strongly reduced interferon-γ production.

Based on their cofactor requirements, the PKC isoforms are classified as Conventional, Novel or Atypical PKCs. The C1 domains bind phosphatidyl-serine and diacylglycerol / phorbol esters. The C2 domains bind anionic lipids and the C3-C4 domains are the N- and C-terminal lobes of the catalytic kinase domain. PS is the pseudo substrate domain, which prevents access to the active site in the inactive conformation. PKC alpha is most probably trans-phosphorylated in the activation loop by the upstream kinase PDK-1. The turn motif and the hydrophobic motif are subsequently self phosphorylated [Newton et al. 2003]. Once phosphorylated the hydrophobic motif binds to the hydrophobic motif binding pocket in the N-terminal lobe of the kinase domain.

Structural information is available for various domains of the PKC isoforms. In the regulatory region, the C1 domain of PKC delta (residues 231-280), and the C2 domains of PKC alpha (residues 155-293), PKC beta (residues 157-289) and PKC delta (residues 1-123) [Zhang et al. 1995; Pappa et al. 1998; Sutton et al. 1998; Verdaguer et al. 1999 and Ochoa et al. 2002] have been described. The catalytic domain of PKC has been much more challenging to crystallize and only three structures are available: two complexes of PKC theta [Rummel et al. 2004, Xu et al. 2004] and one of the maleimide BIM1 bound to PKC 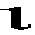 [Messerschmidt et al. 2005]. However, there have been no crystals reported of any other Conventional PKC kinase domain. Such structural information would provide valuable information for discovery of PKC kinase inhibitors, and more specifically, for the design of selective inhibitor of Conventional PKC kinase, e.g. PKC alpha kinase.

The present invention provides for the first time the three dimensional structure of the PKC alpha kinase domain.

### Summary of the Invention

It is an object of the present invention to provide a crystal comprising PKC alpha kinase catalytic domain and the three-dimensional structure of PKC alpha kinase catalytic domain, thereby enabling the design and/or identification of ligands that specifically bind to PKC alpha kinase and/or inhibit PKC alpha kinase biological activity.

The present invention relates to:
(i) a crystal of the PKC alpha kinase with or without a ligand ;
(ii) a method of making a crystal of the PKC alpha kinase domain ;
(iii) methods of using said PKC alpha kinase crystal and its structural coordinates for screening, identifying and designing inhibitors of PKC Conventional kinases and more specifically PKC alpha kinases.

### Detailed Description of the Invention

The nucleotide and amino acid sequences of full-length sequence of human PKC alpha kinase are known (Finkenzeller et al., 1990) and set forth in Genbank Accession (X52479), REFSEQ number (NM002737) and UniProt/SwissProt (P17252).2.

The present invention provides PKC alpha kinase in crystallized form. In particular, it provides crystals of PKC alpha kinase catalytic domain in complex with ligands.

The crystals according to the invention preferably belong to space group P2₁2₁2₁. In one embodiment of the present invention, a crystal of the catalytic domain of PKC alpha kinase comprises a unit cell dimension of a = 44.2Å ± 10Å, b = 101.3Å ± 10Å, c = 252.0Å ± 10Å, or more preferably a = 44.2Å ± 5Å, b = 101.3Å ± 5Å, c = 252.0Å ± 5Å.

Depending on the conditions used for crystallization, the parameters characterising the unit cell may vary with a limited range, at least within the range of the resolution. The resolution of the X-ray crystallography is typically ≤ 5 Angstroms and by means of the purification method described therein, it is possible to provide crystals of such high internal order that a resolution of ≤ 2Å can be achieved.

The term "unit cell" according to the invention refers to the basic shape block. The entire volume of a crystal may be constructed by regular assembly of such blocks. Each unit cell comprises a complete representation of the unit of pattern, the repetition of which builds up the crystal.

The term "space group" according to the invention refers to the arrangement of symmetry elements of a crystal.

In another embodiment of the invention, a crystal of a PKC alpha kinase catalytic domain bound to at least one ligand is provided wherein said crystal has a three-dimensional structure characterized by the structure coordinates of Table 1 or Table 2.

The term "structure coordinates" or "atomic coordinates" refers to mathematical coordinates derived from the mathematical equations related to the pattern obtained on diffraction of a monochromatic beam of X-rays by the atoms (scattering centers) of a crystal comprising PKC alpha catalytic domain. The diffraction data are used to calculate an electron density map of the repeating unit of the crystal. The electron density maps are used to establish the positions of the individual atoms within the unit cell of the crystal.

In a preferred embodiment of the invention, said PKC alpha kinase catalytic domain comprises the sequence of SEQ ID. No. 1 starting from residues 320 to residues 672, a fragment or a homologue thereof or a mutant thereof.

A "fragment" of PKC alpha kinase catalytic domain comprises more than 50% consecutive amino acids of the reference sequence of the PKC alpha kinase catalytic domain according to SEQ ID No.1, more preferably at least 80%, most preferably at least 90% of SEQ ID NO.1.

As used herein, a "homologue" of that sequence shares at least 70% identity, preferably 80% identity, more preferably 90%, and even more preferably 95% identity with the corresponding SEQ ID NO:1 when performing optimal alignment. Optimal alignment of sequences for determining a comparison window may be conducted by the local homology algorithm of Smith and Waterman (J. Theor. Biol., 91 (2) pgs. 370-380 (1981), by the homology alignment algorithm of Needleman and Wunsch, J. Miol. Biol., 48(3) pgs. 443-453 (1972), by the search for similarity via the method of Pearson and Lipman, PNAS, USA, 85(5) pgs. 2444-2448 (1988) or by computerized implementations of these algorithms (GAP, BESTFIT, FASTA and TFASTA in the Wisconsin Genetics Software Package Release 7.0, Genetic Computer Group, 575, Science Drive, Madison, Wisconsin).

The best alignment (i.e., resulting in the highest percentage of identity over the comparison window) generated by the various methods is selected for determining percentage identity.

The term "ligand" according to the invention, refers to a molecule or group of molecules that bind to one or more specific sites of PKC alpha kinase, for example to the binding pocket of PKC alpha kinase. Ligands according to the invention are preferably low molecular weight molecules.

The term "low molecular weight molecules" according to the invention refers to preferably organic compounds generally having a molecular weight less than about 1000, more preferably less than about 500.

Specific examples of ligands are indolylmaleimides derivatives as described in PCT application WO02/38561 or in US6,645,970.

More preferably, said ligand inhibits PKC alpha kinase biological activity. Inihibition of PKC alpha kinase biological activity can be determined according to biological assay described for example in Zhang H.C. et al. (2005). A compound is considered as an PKC alpha kinase inhibitor if it has an IC50 ranging from 0.01 nM to 1.0 µM.

Such inhibitors include, without limitation, the following indolylmaleimide Compound (I), (II) or (III) as defined below:

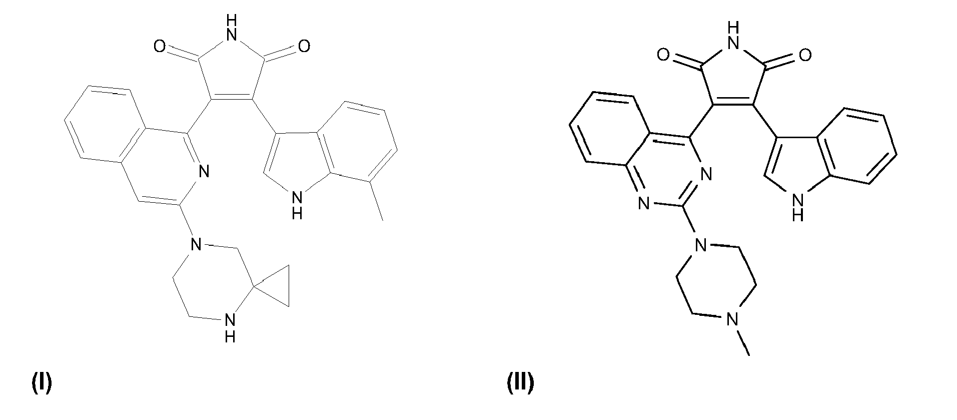

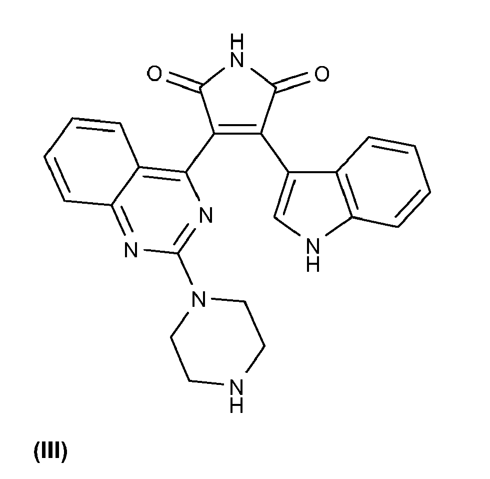

PKC alpha kinase inhibitor can also be a "peptide" or a "peptide derivative", which terms are intended to embrace a "peptidomimetic" or "peptide analogue" which complement the three-dimensional structure of the binding pocket of PKC alpha kinase or can be designed with improved physical or chemical properties to bind with the three-dimensional binding pocket of the PKC alpha kinase as provided in the present invention.

The term "mutant" refers to a mutated sequence of SEQ ID NO:1 by deletion, insertion or preferably replacement of one or more selected amino acids, provided that such mutant sequence shares at least 90% identity, more preferably 95%, and even more preferably 99% identity with the corresponding fragment sequence of SEQ ID NO:1 when performing optimal alignment. Preferably, a mutant of PKC alpha kinase is a single mutant of PKC alpha kinase. In a specific embodiment of the invention, a PKC alpha kinase mutant is a PKC alpha kinase having a mutation in one or more of the phosphorylation site in catalytic domain. In one specific embodiment, a mutant is the mutant sequence defined by SEQ ID NO.2, corresponding to the PKC alpha fragment 320-672 with the following mutation: T497E.

Methods for the preparation of protein mutants are commonly known in the art. For example, PKC alpha mutants may be prepared by expression of PKC alpha DNA previously modified in its coding region by oligonucleotide directed mutagenesis.

As used herein, the term "binding pocket" refers to the region of human PKC alpha that, as a result of its shape and physico-chemical properties favorably associates with another chemical entity or compound. Preferably, it refers to the ATP-binding site, lined at least by the following amino acids: M343 - L355, L364 - 1369, F399 - T409, E418 - L425, D467 - D472 and K478 - F482, and more specifically at least by the following amino acids: L345, G346, F350, G351, V353, A366, K368, T401, M417, Y419, V420, M470, A480 and D481 . It may further comprise the following hydrophobic motif binding site, lined at least by the following amino acids Y365 - L393 and H404 - M417, and more specifically at least by the following amino acids: 1367, K372, V375, 1376, V381, T384, M385, K388, C406, Q408, L413 and F415.

As used herein, the numbering of the amino acid residues is in agreement with the SwissProt/SwissProt UniProt entry P17252.

The invention further provides a method for making a crystal of PKC alpha comprising the catalytic domain of a PKC alpha kinase, said method comprising the following steps:
(i) expressing a construct comprising the nucleotide sequence encoding PKC alpha catalytic domain for production of PKC alpha protein,
(ii) purifying a sufficient amount of the PKC alpha protein produced at step (i) under appropriate conditions for crystallization,
(iii) crystallizing the PKC alpha protein purified at step (ii).

In a specific embodiment, the construct comprises the mutant sequence of SEQ ID NO:2.

The produced PKC alpha protein to be used for crystallization may be biologically active or inactive. Such ability may be determined by morphological, biochemical or viability analysis well-known in the art.

In one specific embodiment of the invention, the construct comprises a mutant sequence encoding a constitutively inactive conformation of the PKC alpha catalytic domain.

Expression of recombinant PKC alpha catalytic domain is achievable in eukaryotic or prokaryotic systems or in vitro expression systems. For example, recombinant human PKC alpha may be expressed in insect cells, such as Sf21 cells, using a suitable recombinant baculovirus system or in bacteria.

According to a preferred embodiment, PKC alpha is bound to at least one ligand at any step prior to crystallization. For example, expression of PKC alpha in step (i) occurs in the presence of a ligand, e.g, in the presence of Compound (I), (II) or (III) as defined above.

The kinase may be expressed as a fusion protein, e.g. a glutathione-S-transferase (GST) or histidine-tagged fusion protein. If desired, the fusion partner is removed before crystallization.

In the present invention, purified PKC alpha is preferably at least 90 % homogeneous. Protein homogeneity is determinable according to analytical methods well-known in the art, e.g. sequence analysis, electrophoresis, spectroscopic or chromatographic techniques.

For carrying out step (iii) of crystallization of the method for making a crystal, various methods can be used including vapor diffusion, dialysis or batch crystallization according to methods known in the art ("Crystallization of Biological Macromolecules", A. McPherson, Cold Spring Harbor Laboratory Press, Cold Spring Harbor, New York, USA).

In vapor diffusion crystallization, a small volume (i.e., a few microliters) of protein solution is mixed with a solution containing a precipitant. This mixed volume is suspended over a well containing a small amount, i.e. about 1 ml, of precipitant. Vapor diffusion from the drop to the well will result in crystal formation in the drop.

The dialysis method of crystallization utilizes a semipermeable size-exclusion membrane that retains the protein but allows small molecules (i.e. buffers and precipitants) to diffuse in and out. In dialysis, rather than concentrating the protein and the precipitant by evaporation, the precipitant is allowed to slowly diffuse through the membrane and reduce the solubility of the protein while keeping the protein concentration fixed.

The batch method generally involves the slow addition of a precipitant to an aqueous solution of protein until the solution just becomes turbid, at this point the container can be sealed and left undisturbed for a period of time until crystallization occurs. In the batch technique the precipitant and the target molecule solution are simply mixed. Supersaturation is achieved directly rather than by diffusion. Often the batch technique is performed under oil. The oil prevents evaporation and extremely small drops can be used. For this, the term "microbatch" is used. A modification of this technique is not to use paraffin oil (which prevents evaporation completely) but rather use silicone oil or a mixture of silicone and paraffin oils so that a slow evaporation is possible.

The claimed invention can encompass any and all methods of crystallization. One skilled in the art can choose any of such methods and vary the parameters such that the chosen method results in the desired crystals.

One preferred method of crystallization of PKC alpha involves mixing a PKC alpha solution with a "reservoir buffer". For crystal formation, the concentration of the precipitating agent in the mixture has to be increased, e.g. by addition of precipitating agent, for example by titration, or by allowing the concentration of precipitating agent to balance by diffusion between the crystallization buffer and a reservoir buffer (not necessarily the same as the original reservoir buffer). Under suitable conditions such diffusion of precipitating agent occurs along the gradient of precipitating agent, e.g. from the reservoir buffer having a higher concentration of precipitating agent into the crystallization buffer having a lower concentration of precipitating agent. Diffusion may be achieved e.g. by vapour diffusion techniques allowing diffusion of water in the common gas phase. Known techniques are e.g. vapour diffusion methods, such as the "hanging drop" or the "sitting drop" method. In the vapour diffusion method a drop of crystallization buffer containing the protein is hanging above or sitting beside a much larger pool of reservoir buffer. Alternatively, the balancing of the precipitating agent can be achieved through a semipermeable membrane that separates the crystallization buffer from the reservoir buffer and prevents dilution of the protein into the reservoir buffer.

Formation of PKC alpha crystals can be achieved under various conditions which are essentially determined by the following parameters: pH, presence of salts and additives, precipitating agent, protein concentration and temperature. The pH may range, for example, from about 4.0 to 9.0. In a specific embodiment, the crystallization conditions comprises the use of a reservoir solution containing 20-23% PEG3350 and about 0.2M di ammonium hydrogen citrate. In another specific embodiment, free interface diffusion in glass capillaries is used, containing 4ml buffer 20-30% PEG3350, 0.2M di ammonium hydrogen citrate.

In another specific embodiment, the invention relates to a method for making a co-crystal of the PKC alpha catalytic domain in complex with a ligand, said method comprising:
a. expressing a construct comprising the nucleotide sequence encoding PKC alpha catalytic domain as shown in SEQ ID NO:2 in the presence of a ligand, preferably the compound (I), (II) or (III),
b. purifying a sufficient amount of the PKC alpha protein produced at step (i) over a Ni-NTA column and a size exclusion column at a temperature preferably below 10°C,
c. crystallizing the PKC alpha protein purified at step (b), using hanging drop vapor diffusion with a drop size of 2µl protein solution plus 1 µl reservoir solution.

Preferably, in the above method, no ATP is added to the protein after Ni-NTA column purification.

The crystal form of PKC alpha crystal may also be used for exchanging the ligand by soaking compounds of interest, for example, for compound optimization, or for the discovery of novel scaffolds in fragment based screening approaches.

The present invention also relates to a crystal comprising the PKC alpha catalytic domain obtainable by the above methods.

The present invention provides in Table 1 the structure coordinates of human PKC alpha in complex with the Compound (I) :

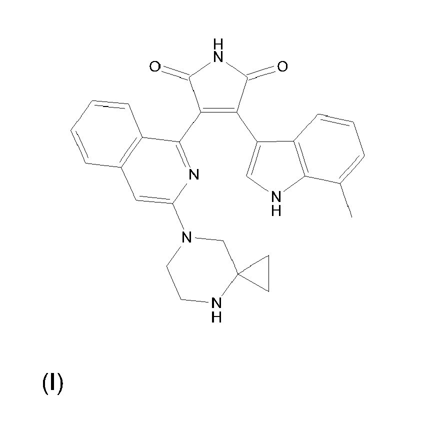

The present invention further provides in Table 2 the structure coordinates of human PKC alpha in complex with the Compound (II) :

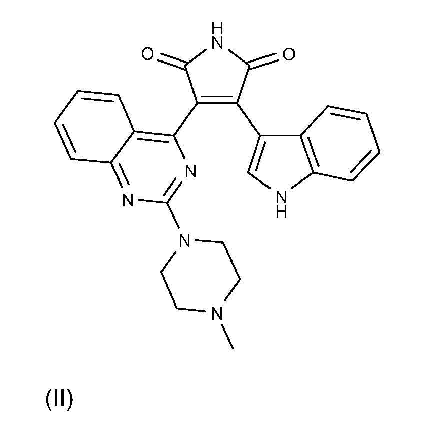

Structure coordinates of a crystalline composition of this invention may be stored in a machine-readable form on a machine-readable storage medium, e.g. a computer hard drive, diskette, DAT tape, etc., for display as a three-dimensional shape or for other uses involving computer-assisted manipulation of, or computation based on, the structural coordinates or the three-dimensional structures they define. For example, data defining the three dimensional structure of a protein of the PKC family, or portions or structurally similar homologues of such proteins, may be stored in a machine-readable storage medium, and may be displayed as a graphical three-dimensional representation of the protein structure, typically using a computer capable of reading the data from said storage medium and programmed with instructions for creating the representation from such data.

In one embodiment of the invention, a method is provided for determining the three-dimensional structure of a complex comprising the catalytic domain of Conventional PKC kinase, preferably PKC alpha kinases, a fragment or homologue thereof bound to at least one ligand, comprising:
(i) making a crystal of PKC as defined above;
(ii) collecting x-ray diffraction data in the form of atomic coordinates for said crystal; and,
(iii) utilizing the atomic coordinates of Table 1 or Table 2 or a selected portion of it to determine the three-dimensional structure of said Conventional PKC, a fragment, or homologue thereof.

According to the present invention, a three-dimensional PKC alpha model is obtainable from a PKC crystal comprising the catalytic domain of PKC alpha, fragment or homologue thereof.

The present invention also relates to a computer readable medium having stored a model of the PKC alpha crystal structure. In a preferred embodiment, said model is built from all or a selected portion of it, of the atomic coordinates of Table 1 or Table 2 derived from the X-ray diffraction data.

By "selected portion", it is meant the structure coordinates of at least 10 consecutive amino acids shown in Table 1 or Table 2 and preferably at least 50 amino acids, and more preferably at least 100 consecutive amino acids.

In a preferred embodiment, a selected portion corresponds to the structure coordinates of the amino acids forming at least the binding pocket of PKC alpha, or at least the ATP binding sites and/or the hydrophobic motif binding site and more preferably the catalytic domain of PKC alpha kinase.

The knowledge obtained from the three-dimensional model of the catalytic domain of PKC alpha can be used in various ways. For example, it can be used to identify chemical entities, for example, small organic and bioorganic molecules such as peptidomimetics and synthetic organic molecules that bind to PKC alpha and preferably block or prevent a PKC alpha-mediated or associated process or event, or that act as PKC alpha agonists. Furthermore, this information can be used to design and prepare PKC mutants, e.g. mutants with altered catalytic activity, model the three-dimensional structure and solve the crystal structure of proteins, such as PKC homologues, PKC mutants or PKC co-complexes, involving e.g. molecular replacement or homology modeling.

The term "molecular replacement" refers to a method that involves generating a preliminary structural model of a crystal whose structural coordinates are unknown, by orienting and positioning a molecule whose structural coordinates are known, e.g., the PKC alpha coordinates within the unit cell of the unknown crystal, so as to best account for the observed diffraction pattern of the unknown crystal. Phases can then be calculated from this model, and combined with the observed amplitudes to give an approximated Fourier synthesis of the structure whose coordinates are unknown. This in turn can be subject to any of the several forms of refinement to provide a final accurate structure of the unknown crystal. Using the structural coordinates provided by this invention, molecular replacement may be used to determine the structural coordinates of a crystalline co complex, unknown ligand, mutant, or homolog, or of a different crystalline form of PKC. Additionally, the claimed crystal and its coordinates may be used to determine the structural coordinates of a chemical entity that associates with PKC.

"Homology modeling" according to the invention involves constructing a model of an unknown structure using structural coordinates of one or more related proteins, protein domains and/or one subdomains such as PKC. Homology modeling may be conducted by fitting common or homologous portions of the protein or peptide whose three dimensional structure is to be solved to the three dimensional structure of homologous structural elements. Homology modeling can include rebuilding part or all of a three dimensional structure with replace of amino acids or other components by those of the related structure to be solved.

Based on the three-dimensional structure of PKC alpha as provided in the present invention and using the atomic coordinates of Table 1 or Table 2, or a selected portion of it, the effects of site-specific mutations can be predicted. More specifically, the structural information provided herein permits the identification of desirable sites for amino acid modification, particularly amino acid mutation resulting in substitutional, insertional or deletional variants. Such variants may be designed to have special properties, particularly properties distinct from wild-type PKC alpha, such as altered catalytic activity. Substitutions, deletions and insertions may be combined to arrive at a desired variant. Such variants can be prepared by methods well-known in the art, e.g. starting from wild-type PKC alpha or by de novo synthesis.

The PKC alpha structural information provided herein is useful for the design of ligands which are capable of selectively interacting with PKC Conventional kinases or with PKC alpha kinase, but not other non-Conventional kinases or kinases other than PKC alpha, and thereby specifically modulating the biological activity of PKC Conventional kinases and not other non-Conventional kinases. As used herein, the classification of PKC isoforms in Conventional, Novel, Atypical and other isoforms is done according to Newton (2001).

Chemical entities that have a surface that mimics the accessible surface of the binding pocket of PKC alpha can be constructed by those skilled in the art. By way of example, the skilled artisan can screen three-dimensional structural databases of compounds to identify those compounds that position appropriate functional groups in similar three dimensional structural arrangement, then build combinatorial chemistry libraries around such chemical entities to identify those with high affinity to the binding pocket of PKC alpha.

In one embodiment of the invention, a method is provided for identifying a ligand that binds to the PKC alpha comprising the steps of:
(i) using the three-dimensional structure of the catalytic domain of PKC alpha to select and/or design a potential ligand that binds to PKC alpha,
(ii) identifying among the potential ligand selected in step (i), those ligands that bind to PKC alpha in an *in vitro* or cell-based assay.

In a specific embodiment of the invention, the cell-based assay used at step (ii) is designed to identify ligands which inhibit the biological activity of PKC alpha.

Ligands, such as small molecular weight compounds can be identified from screening compound databases or libraries and using a computational means to form a fitting operation to a binding site on the PKC. The three dimensional structure of PKC as provided in the present invention by the structure coordinates of Table 1 or Table 2 or a selected portion of it, can be used together with various docking programs.

The potential inhibitory or binding effect of a chemical entity on PKC alpha may be analyzed prior to its actual synthesis and testing by the use of computer-modeling techniques. If the theoretical structure of the given chemical entity suggests insufficient interaction and association between it and PKC alpha, the need for synthesis and testing of the chemical entity is obviated. However, if computer modeling indicates a strong interaction, the molecule may then be synthesized and tested for its ability to bind to PKC alpha. Thus, expensive and time-consuming synthesis of inoperative compounds may be avoided.

This "in silico" analysis may begin by visual inspection of, for example, the binding pocket on a computer screen based on the structural coordinates of Table 1 or Table 2 in whole or in part. Selected fragments or chemical entities may then be positioned in a variety of orientations, or "docked," within the binding pocket of PKC. Docking may be accomplished using software such as Quanta and SYBYL, followed by energy minimization and molecular dynamics with standard molecular mechanics force fields, such as CHARMM and AMBER. Specialized computer programs may be of use for selecting interesting fragments or chemical entities. These programs include, for example, GRID, available from Oxford University, Oxford, UK; 5 MCSS or CATALYST, available from Molecular Simulations, Burlington, MA; AUTODOCK, available from Scripps Research Institute, La Jolla, CA; DOCK, available from University of California, San Francisco, CA, and XSITE, available from University College of London, UK.

Preferred is a method for designing a PKC alpha inhibitor which interacts at the ATP binding site of PKC alpha or any other binding sites. One approach enabled by this invention is the use of the structure coordinates of PKC alpha to design chemical entities that bind to or associate with PKC alpha and alter the physical properties of the chemical entities in different ways. Thus, properties such as, for example, solubility, affinity, specificity, potency, on/off rates, or other binding characteristics may all be altered and/or maximized. One may design desired chemical entities by probing a PKC alpha comprising the catalytic domain with a library of different entities to determine optimal sites for interaction between candidate chemical entities and PKC alpha. For example, high-resolution X-ray diffraction data collected from crystals saturated with solvent allows the determination of where each type of solvent molecule adheres. Small molecules that bind tightly to those sites can then be designed and synthesized and tested for the desired activity. Once the desired activity is obtained, the molecules can be further altered to maximize desirable properties.

Once a compound has been designed or selected by the above methods, the efficiency with which that compound may bind to PKC alpha may be tested and modified for the maximum desired characteristic(s) using computational or experimental evaluation. Various parameters can be maximized depending on the desired result. These include, but are not limited to, specificity, affinity, on/off rates, hydrophobicity, solubility, and other characteristics readily identifiable by the skilled artisan.

In a preferred embodiment, the structure coordinates of Table 1 or Table 2 of PKC alpha kinase is used in the above computer-based design step.

In one specific embodiment of the method, the ligand is selected and/or designed among the potential ligands that virtually bind to the residues lining the ATP binding site, and/or the hydrophobic motif binding site, according to a computer aided modeling program. More preferably, the ligand is selected and/or designed among those that virtually bind to at least one or more amino acids of the ATP binding site selected from the group consisting of: M343 - L355, L364 - 1369, F399 - T409, E418 - L425, D467 - D472 and K478 - F482, and/or at least with one or more amino acids of the hydrophobic motif binding site regions selected from the group consisting of: Y365 - L393 and H404 - M417.

In a more preferred embodiment of the above method, the ligand is selected and/or designed among those that virtually bind to at least one or more amino acids of the ATP binding site selected from the group consisting of: L345, G346, F350, G351, V353, A366, K368, T401, M417, Y419, V420, M470, A480 and D481, and/or at least with one or more amino acids of the hydrophobic motif binding sites selected from the group consisting of: 1367, K372, V375, 1376, V381, T384, M385, K388, C406, Q408, L413 and F415.

The invention further relates to a method for selecting a ligand that binds to PKC alpha, comprising:
a. co-crystallizing or incubating a candidate compound or mix of compounds with PKC alpha under appropriate conditions,
b. determining by X-ray or NMR methods the amino acids of PKC alpha which interacts with the candidate compound,
c. selecting the compound which interacts at least with one or more amino acids of the binding pocket and hydrophobic motif binding site selected among the group consisting of M343 - L355, L364 - I369, F399 - T409, E418 - L425, D467 - D472, K478 - F482, Y365 - L393 and H404 - M417.

For carrying out step b., mapping of the binding site of ligand is usually performed by recording NMR spectra with and without the candidate compound, and identifying those resonances of the protein that are affected by ligand binding. This requires assignment of the protein resonance prior to the analysis, or comparison with the pattern of chemical shift changes that occur upon binding of ligands with known binding sites. Alternatively, competition experiments using said ligands with known binding sites can yield equivalent information.

The present invention further provides methods to design novel ligands of PKC, using fragment linking approaches. Compounds binding to different binding regions of PKC are first selected. The ligands are linked together based on the spatial orientation, so that the designed novel compounds fits within the two binding sites.

The invention thus relates to a method to design ligands to PKC alpha, wherein said method comprises:
a. providing a first ligand that binds to one or more amino acids of a first binding region of PKC alpha, for example the ATP binding site,
b. providing a second ligand that binds to one or more amino amino acids of a second binding region of PKC alpha, for example the hydrophobic motif binding site, and,
c. linking said first ligand to said second ligand to design a ligand that binds to the first and second binding pockets of PKC alpha.

Preferably, a first ligand is selected among the ligands that interact with one or more amino acids selected among the group consisting of: M343 - L355, L364 - 1369, F399 - T409, E418 - L425, D467 - D472 and K478 - F482, and more preferably one or more amino acids selected among the group consisting of L345, G346, F350, G351, V353, A366, K368, T401, M417, Y419, V420, M470, A480 and D481.

The selection of an appropriate linking group is made by maintaining the spatial orientation of the ligands to one another and to PKC alpha based upon principles of bond angle and bond length information well known in the organic chemical art.

In a specific embodiment, a second ligand at step b. is selected among the ligand that interacts at least with one or more amino acids of the binding hydrophobic motif binding site selected among the group consisting of Y365 - L393 and H404 - M417 and more preferably 1367, K372, V375, 1376, V381, T384, M385, K388, C406, Q408, L413 and F415.

The following examples serve to illustrate the present invention but should not be construed as a limitation thereof. The invention particularly relates to the specific embodiments described in these examples.

### LEGENDS OF THE FIGURES

Figure 1: Thin rod like crystals of PKC alpha - Construct 4/Compound (I) with fine brushes of needles at both ends. (Diameter - 30 microns)
Figure 2 : Overall fold of PKC alpha - Construct 4/Compound (I). The polypeptide chain is colored using a rainbow color scheme from blue at the N-terminus to red at the C-terminus. The inhibitor of formula (I) (yellow carbons) is located in the ATP binding pocket of the kinase domain.
Figure 3 : The maleimide moiety (green carbons) of Compound (I) makes two hydrogen bonds with the hinge region of PKC alpha (residues E418 and V420), one to a water molecule and one to residue D467.
Figure 4: The figure shows the RMS deviations between the C-alpha atoms of the three molecules of PKC alpha - Construct 4 in the asymmetric unit. The curve B indicates the differences between molecule B and the molecule A, and the curve C shows the difference between the molecule C and the molecule A.
Figure 5: Crystal of the PKC alpha - Construct 4/Compound (II) complex.
Figure 6: Overall fold of the PKC alpha - Construct 4/Compound (II) complex.
Figure 7: Initial unbiased difference electron density clearly shows the conformation of the inhibitor Compound (II) in the ATP binding pocket of PKC alpha-Construct 4.
Figure 8: Compound (II) in the ATP binding pocket of PKC alpha-Construct 4 with the hinge to the left and the P-loop above (blue).
Figure 9: Superposition of the two structures of PKC alpha-Construct 4 presented here. The bulkiness of the Diaza-spiro-octyl group of Compound (I) forces the P-loop in a slightly more open conformation than it is observed for Compound (II).

### EXAMPLES

### List of abbreviations

| Abbreviation | Description |
|---|---|
| BIM | Bis-indolyl-maleimide |
| BV | Baculovirus |
| FCS | Fetal Calf Serum |
| MOI | Multiplicity of Infection |
| MPD | Methyl-pentane-diol |
| Ni-NTA | Nickel-nitrilotriacetic acid |
| NPL | Novartis Plasmid Number |
| NVP | Novartis Product Number |
| pfu | plaque forming units |
| PKC | Protein Kinase C |
| rms | root mean square |
| rpm | revolutions per minute |
| RT | room temperature |
| TCEP | Tris-(2-carboxyethyl) phosphine |
| wcp | wet cell pellet |

### Example 1: Crystal structure of PKC alpha kinase in complex with Compound (I) at 2.0 Å resolution

### 1. Methods

Cloning of the human PKC alpha(320-672)T497E-His gene into a pBAKPac8 transfer vector. The plasmid encoding the human PKC alpha is from the orf cDNA clone library of Invitrogen and has the clone ID IOH29644. The sequence corresponds to the Genbank accession number NM_002737.2. The DNA encoding the fragment from amino acid 320 to 672 has been cloned by PCR with the oligonucleotides of
SEQ ID NO:3
   (5'-GATTCGTCATCAATGGTGATGATGGTGATGTACTGCACTCTGTAAGATGGGGTG-3')/
SEQ ID NO:4
   (5'-GGATCCCTGCAGGCCTCGAGTTCCCACCATGCCCTCTGAAGACAGGAAACAAC-3').
   The obtained PCR fragment was integrated into plasmid pXI342i (as described in PCT application WO2004/076654) or following the published method [Geiser, Cebe, Drewello, and Schmitz, 2001]. Finally the mutation T497E was introduced by integrating the PCR fragment amplified from the original cDNA clone with
SEQ ID NO:5
   (5'-ATGGATGGAGTCACGACCAGGGAGTTCTGTGGGACTCCAGATTATATCG-3') and SEQ ID NO:3 into pXI525b. The resulting plasmid is called pXI525e. In short, the human PKC alpha(320-672)T497E is in front of an uncleavable (His)6-tag and is under the control of the polyhedrin promoter. The DNA sequence of the clone was confirmed by DNA sequencing.

The new Baculovirus transfer vector pXI525e was used for the transfection of Sf9 insect cells according to the protocol proposed by Clontech. Other constructs were also tested and are summarized hereafter:

| | |
|---|---|
| PKC alpha/Baculovirus: | |
| 1. M²⁹⁹ELRQ..... | T⁴⁹⁷....S⁶⁵⁷.....LQSAV⁶⁷² - 6His |
| 2. M²⁹⁹ELRQ..... | E⁴⁹⁷....S⁶⁵⁷.....LQSAV⁶⁷² - 6His |
| 3. (M)P³²⁰SEDRKQP..... | T⁴⁹⁷... S⁶⁵⁷.....LQSAV⁶⁷² - 6His |
| 4. (M)P³²⁰SEDRKQP..... | E⁴⁹⁷... S⁶⁵⁷.....LQSAV⁶⁷² - 6His |
| 5. (M)P³²⁰SEDRKQP..... | E⁴⁹⁷...E⁶⁵⁷.....LQSAV⁶⁷² - 6His |
| 6. (M)P³²⁷SNNL.... | T⁴⁹⁷... S⁶⁵⁷.....LQSAV⁶⁷² - 6His |
| 7. (M)P³²⁷SNNL.... | E⁴⁹⁷... S⁶⁵⁷.....LQSAV⁶⁷² - 6His |
| 8. M¹ADVF..... | T⁴⁹⁷...S⁶⁵⁷.....LQSAV⁶⁷² - 6His |
| | |
| PKC alpha/E.coli: | |
| 9. M²⁹⁹ELRQ..... | T⁴⁹⁷....S⁶⁵⁷.....LQSAV⁶⁷²-6His |

The Construct 4 led to the crystal structures presented here.

### Protein Expression

Methods for PKC alpha expression are described here with focus on the construct 4. In general, methods for Baculovirus (BV) and insect cell cultures as given by [O'Reilly, et al. 1994] were used. Recombinant BV was produced by transfection of Sf9 cells with the Bac-to-Bac transfection system from CLONTECH. A single direct amplification round of the rec. BV from plaques coming directly from the incubated and diluted transfection mixture was used to obtain a sufficient amount of BV needed for first optimization and 1-2 expression cultures at the liter-scale. In cases where higher amounts of BV for re-supply were needed, as for Construct 4, a conventional multi-step BV amplification was applied as follows: BV from suspended single plaques were amplified in a second amplification round in attached Sf9 cells cultured at 27°C in 25cm²-TC-flasks with 10 ml medium TC100 (Invitrogen)+10%FCS (Invitrogen), and in a third amplification round in suspended Sf9 cells cultured at 27°C, shaken at 90 rpm in 500 ml-Erlenmeyer flasks with 100 ml medium TC100+10%FCS+0.1 % Pluronic F-68 (Invitrogen). Direct and conventional amplification resulted in rec. Baculovirus suspensions with titers in the range of 0.6 - 2.0x10⁸ pfu/ml, determined by plaque assay. Shake flask cultures for testing PKC alpha-expression was carried out as follows: Suspended Sf21 cells were grown as preculture at 27°C and 90 rpm for three days in 100 ml medium SF900 II (Invitrogen) + 1 % FCS in 500 ml-Erlenmeyer flasks to reach a cell density of ca. 3x10⁶ cells/ml. Main cultures with a final volume of 50 ml were set up by dilution of the preculture to a cell density of 1.5x10⁶ cells/ml in ExCell420 (JRH) + 1 % FCS medium in 200 ml-Erlenmeyer flasks and the additions of rec. Baculovirus suspension of the third amplification for a MOI=1 and of typically 5 ml 10x concentrated inhibitor solution (100µM). The cultures were then further incubated for 3 days post infection under the same conditions.

The following protocol was applied for the liter-scale production of PKC alpha - Construct 4/Compound (I), leading to the crystal structure: Sf21 cells were precultured in medium SF900 II + 1% FCS for 3 days at 27°C, shaken at 90 rpm in 100 ml - cultures in shake flasks, as given above. Precultures were diluted to 1.5 x 10⁶ c/ml in medium EX-CELL 420 + 1% FCS, and 800 ml culture was dispensed in a 5 liter-Erlenmeyer flask, rec. Baculovirus for MOI=1 was added, a 10x conc. solution of Compound (I) in expression medium for a final conc. of 10 µM was added and the culture was cultivated for 60-66 hours at 27°C, shaken at 90 rpm. PKC alpha - Construct 4/Compound (I) - expressing cells were harvested by two subsequent centrifugations at 400g at RT for 20 min, first in 1 liter - centrifuge bottles and then after resuspension of the pelleted cells in 20 ml PBS, pH 6.2 with a protease inhibitor mix (Complete, Roche) in 50 ml-plastic tubes and frozen and stored at -80°C prior to extraction.

At harvest, for 50 ml - and for 800 ml-cultures, 2 x 1 ml - samples for cell parameter analysis with ViCell and SDS-PAGE analysis and 30 ml - samples for cell fresh weight determination were taken and the two latter samples were centrifuged at 300g at RT for 10 min. For testing PKC alpha expression, cell pellets from the 1ml - samples were taken up in 1 ml 1xSDS-PAGE buffer and an amount of such total lysates normalized for cell yield (0.2 mg fresh weight cells) was applied on the gels for SDS-PAGE analysis. PKC alpha expression was estimated from gels stained with Coomassie Blue and from Western blots treated with anti-PKC alpha-antibody (Santa Cruz Biotechnology, # sc-8393). Solubility testing of the expressed PKC alpha - Construct 4/Compound (I) was carried out by comparing the band intensities on Western blots of samples from the supernatant or pellet fraction of cellular extracts after high speed centrifugation.

A stock solution in DMSO of 10 mM of Compound (I), stored at -20°C, was each time freshly diluted typically to 100 µM (10x concentrated) in expression medium and filter sterilized through a 0.22 µm-filter.

### Purification

Due to the sensitivity of the protein all the purification steps were carried out on ice or at 4°C. A 10g wet cell pellet was resuspended in approximately 100 ml lysis buffer (300mM NaCl, 50mM Tris HCl, 3mM Tris-(carboxyethyl) phosphine = TCEP, 1mM NaF, 10µl Na3VO4, 10%glycerol, pH 8.0 ), containing 4 tablets of protease inhibitor cocktail Complete Mini, EDTA free (Roche) and 100µl Phosphatase Inhibitor cocktail 1 and 2 (Sigma). The suspension was treated with a hand homogenizer 10 times and the insoluble part removed by centrifugation for 60min with 30000g at 4°C. The supernatant was passed through a 5µm filter then loaded on a Ni-NTA-Superflow column (Pharmacia; 1cm diameter, 3ml volume, flow rate 1-3ml/min, pre-equilibrated with lysis buffer). The column was then washed with lysis buffer plus 10mM imidazole until absorption was close to zero (app. 15 column volumes) and then eluted with a gradient from 10 to 500mM imidazole in lysis buffer. Fractions containing the PKC alpha/Inhibitor complex (colored) were pooled. The pool was concentrated in a pre-cooled Ultrafree Cell MW cutoff 30000 to 1-2ml and loaded on a HiLoad Superdex75 (16/60) prep-grade (Amersham, now GE Healthcare), equilibrated in SPX buffer (200mM NaCl, 50mM imidazole, 2% glycerol, 1mM NaF, 2mM TCEP, pH = 8.0).

Colored fractions were concentrated in an Ultrafree cell to a final protein concentration of 10-15mg/ml, purity 96% (determined by HPLC).

### Crystallization

With an initial screen of 480 different crystallization conditions, only one crystallization condition was found. The optimized crystallization condition for the successful Construct 4 - Compound (I) complex required a protein solution at 6 mg/ml PKC alpha in 0.2M NaCl, 0.05M imidazole pH 8.0, 2mM TCEP, 1 mM NaF, 2% glycerol. The method used was hanging drop vapor diffusion, with a drop size of 2µl protein solution plus 1µl reservoir solution (1 ml of 22% PEG-3350w/v, 0.2M (NH₄)₂H-citrate) at 4°C. Flat rod-like crystals typically appeared within 2-7 days at 4°C. Single fragments of the bundles of crystals that grew were treated with cryo-buffer (20% glycerol, 23% PEG-3350 w/v, 200mM (NH₄)₂H-citrate) at 4°C by increasing the glycerol concentration slowly by adding cryo buffer gradually to the original drop. Subsequently, the crystals were frozen by dipping them in liquid nitrogen and stored for data collection.

### Data collection and analysis

Due to the small crystal size (thin rods of about 30 microns in diameter) synchrotron radiation was a must for these crystals. The data set was collected at the PXII (X10SA) beam line at the Swiss Light Source (SLS) in Villigen, Switzerland. This beam line is equipped with a MARCCD detector and a cryo-stream to keep the crystals at 100K. Annealing (blocking of the cryo stream for app. 6-8sec) of the crystal in situ improved the resolution from about 3.9 Å to 2.0 Å. Images were collected with 0.5° oscillation each and a crystal-to-detector distance of 240 mm. To collect a full data set the crystal was translated along the longest dimension. Raw diffraction data were processed and scaled with XDS / Xscale [Kabsch 1988] within the APRV program package [Kroemer et al. 2004]. Structure factor amplitudes for negative and very weak intensities were estimated using Bayesian statistics [French and Wilson, 1978].

### Structure determination and refinement

The structure of the PKC alpha/Compound (I) complex was determined by molecular replacement with the program PHASER [McCoy et al. 2005] using a truncated model of the 2.0Å structure of the PKC theta complex [Rummel et al. 2004] as a starting model. The sequence identity between PKC alpha and PKC theta is 53.6 %. After the sequence of the model had been corrected to the human PKC alpha sequence, it was refined with REFMAC [Murshudov et al., 1997] which is part of the CCP4 program suite [Collaborative Computational Project, Number 4, 1994]. Model building was carried with the program O version 9.0 [Jones et al., 1991]. Cross-validation was used throughout refinement using a test set comprising 5% of the reflections.
Pictures were made with the program PYMOL (DeLano Scientific LLC).

### 2. Results

### Expression of PKC alpha/Compound (I) complex

The Construct 4, which led finally to the co-crystal structure of Table 1, was sufficiently expressed with the PKC-specific inhibitor Compound (I) present in the culture. In the absence of the inhibitor, only residual levels of PKC alpha - Construct 4 protein were expressed. The same expression behavior was also observed for the PKC alpha Constructs 1, 2, 3, 5, 6 and 7. Strong stimulation of expression of proteins from PKC alpha - Construct 4 was also observed for 7 other PKC inhibitors added to the culture. The full-length version PKC alpha, Construct 8, was also expressed in absence of an inhibitor, but the expressed protein was unstable and degraded and could thus not be purified.

A dose-response experiment showed that the level of PKC alpha - Construct 4 expression correlates with the Compound (I) concentration in the medium (in the range of 0-30 µM). Maximal expression occured at 30 µM Compound (I), which is slightly higher than at 10 µM. 10 µM Compound (I) was then used for liter scale expression of PKC alpha - Construct 4 for purification and crystallization. Experiments on the kinetics of expression showed that 60-66 hours post infection time is optimal for expression of PKC alpha - Construct 4/Compound (I) suitable for crystallization.
For the liter scale production of PKC alpha - Construct 4/Compound (I) leading to the crystal structure, a cellular yield of 14.71 g fresh weight cells per 790 ml culture was obtained and 16 mg of Construct 4/Compound (I) was isolated after the Ni-column. This means that the level of expression of soluble PKC alpha - Construct 4/Compound (I) was at least 20 mg/l for this experiment. SDS-PAGE analysis of the total lysate revealed a strong band of the correct size on a Coomassie-stained gel and a correspondingly strong band on a Western blot treated with anti-PKC alpha-antibody. Approximately 50% of the PKC alpha - Construct 4/Compound (I) was found to be solubly expressed.

### Purification

For none of the constructs of the kinase domain without a phosphorylation site mutation, Constructs 1, 3 and 6, purification of the protein was possible. For the four constructs with one or two mutations, Constructs 2, 4, 5 and 7, protein has been purified and screened for crystallization. For the Constructs 2, 5 and 7, phosphorylation and dephosphorylation experiments have been performed without resulting in a more homogenous phosphorylation state of the molecules. Best recovery of purified protein per gram wet cell pellet was obtained from Construct 4, which also produced crystals in complex with Compound (I). The expression of the full length protein, corresponding to Construct 8, resulted in a break down product of app. 50kDa and traces of the desired protein and purification of the protein was thus not possible.
Mass spectroscopic analysis on the purified PKC alpha-Construct 4/Compound (I) complex showed that the protein preparation was sufficiently homogeneous for crystallization (41304.0 Da = 10.5% (des-Met, mono-phosphorylated); 41383.2 Da = 81.5% (des-Met, phosphorylated at T638 and S657); 41466.0 Da = 8% (des-Met, 3x phosphorylated)). The expected mass of the des-Met protein is 41385 Dalton (for the 354 residues plus the His-tag, the mutation T497E and the two phosphorylation sites). The expected mass was in agreement with the measurements.

### The crystals of the PKC alpha - Compound (I) complex

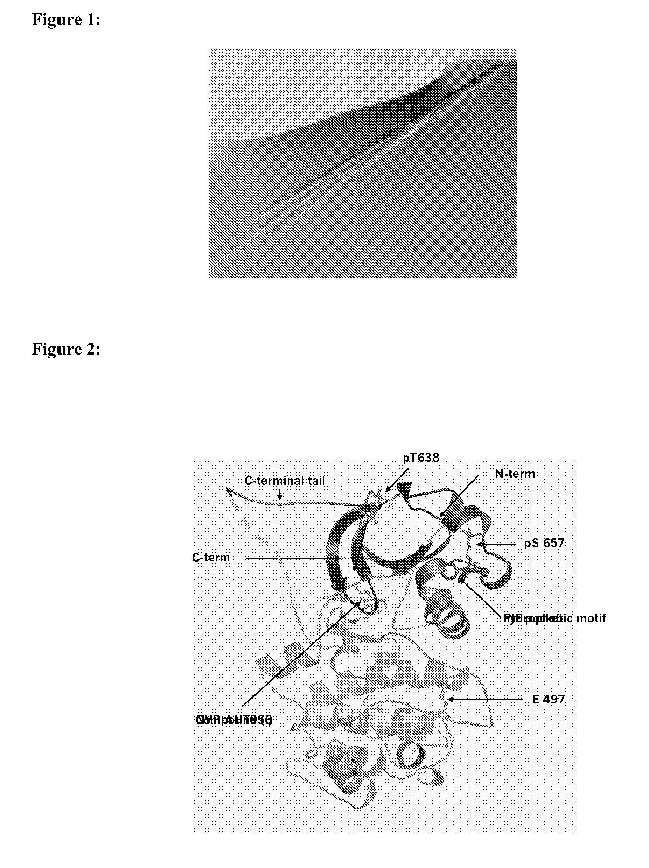

The Construct 4 crystallized comprises residues 320 to 672 and a C-terminal tag of 6 histidine residues. The phosphorylation site in the activation loop is mutated in this construct in order to stabilise the kinase in an active conformation (T497E). The asymmetric unit of the crystal contains 3 molecules. The crystals grew as thin needles of about 30 microns in diameter (Figure 1). The chemical structure of the Compound (I), 3-[3-(4,7-Diaza-spiro[2.5]oct-7-yl) -isoquinolin-1-yl]-4-(7-methyl-1H-indol-3-yl)-pyrrole-2,5-dione, present in the crystal, is shown in formula (I) given below. The X-ray diffraction data statistics for the PKC alpha - Construct 4/Compound (I) crystal can be found in Table 3.

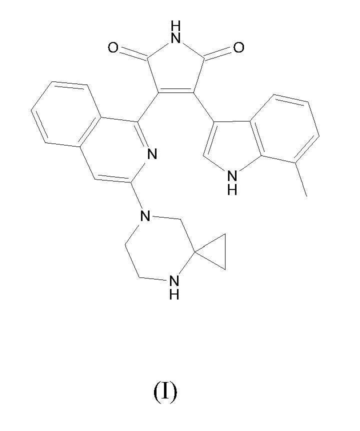

**Table 3: X-ray diffraction data of the PKC alpha - Construct 4/Compound (I) crystal**

| | **PKC alpha - compound (I)** |
|---|---|
| Synchrotron beam line | SLS (PXII, X10SA) |
| Wavelength | 1.0005 |
| Detector | MARCCD225 |
| Oscillation range | 0.5 |
| Distance | 240 mm |
| Number of images used | 250 |
| Temperature of data collection | 100K |
| Number of crystals | 1 |
| Space group | P2₁2₁2₁ |
| Unit cell dimensions a, b, c, | 44.20Å, 101.30Å, 252.0Å |
| α, β, γ | 90.0°, 90.0°, 90.0° |
| Number of monomers / asymmetric unit | 3 |
| Packing coefficient | 2.23 Å³/Dalton |
| Solvent content | 44.5% |
| Overall B-factor from Wilson Plot (resolution range) | 31.1Å² |
| | 80.0Å - 2.0Å |
| Resolution range (last shell) | 79.06 Å -2.00 Å |
| | (2.08 Å - 2.00 Å) |
| Number of observations | 304484 |
| Number of rejections | 1585 |
| Number of unique reflection | 70108 |
| Mosaicity | 0.17 |
| Overall: | |
| Data redundancy | 4.3 |
| Data completeness (last shell) | 90.1 % (57.5 %) |
| < I/σ (I)> (last shell) | 10.0(1.8) |
| R_{merge} (last shell) | 10.5% (68.5%) |
| Reflections with I > 3σ (I) | 64% |

### The structure of the PKC alpha - construct 4 in complex with Compound (I)

The overall fold of the molecule is shown as a ribbon drawing in Figure 2. PKC alpha shows the classical bi-lobal kinase fold. The N-terminal lobe (residues 320 - 420) consists mainly of a five stranded β-sheet and one α-helix. There are 10-15 residues at the N-terminus which are disordered. The C-terminal domain (residues 421 - 618) consists mainly of α-helical elements. The C-terminal tail (residues 618 - 672) containing the two phosphorylation sites (T638 and S657) wraps loosely around the N-terminal lobe and is partially disordered. The first part of this segment is not visible at all (residues 619 to 626), and the second part is only visible because it is in direct contact with a neighboring molecule in the crystal (residues 627 to 650). In solution both segments are expected to be highly flexible. The third part of the C-terminal tail is attached to the N-terminal domain. In particular the hydrophobic motif (...FXXFS...), which can be activated by the phosphorylation of the serine residue, binds with the two phenylalanine side chains in the hydrophobic motif binding pocket. After the hydrophobic motif the chain extends to form a 6th strand in the β-sheet of the N-terminal lobe. The last few C-terminal residues including the His-tag are partially disordered in the three molecules in the asymmetric unit, with the amount of disorder depending on the crystal packing. The inhibitor is sitting between the two domains in the ATP binding site.

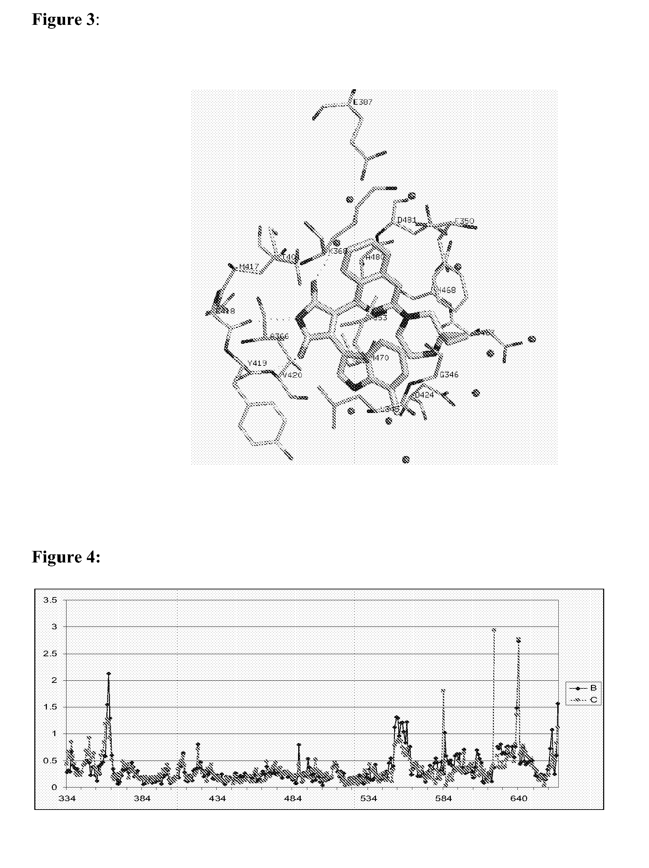

The refinement statistics is given in Table 4. Close contacts between the inhibitor and the protein are listed in Table 5. Figure 3 shows the hydrogen bonding network of Compound (I) in the binding site.

**Table 4: Refinement statistics on the crystal structure of PKC alpha - Construct 4/Compound (I)**

| **Data used in refinement:** | **PKC alpha - Compound (I)** |
|---|---|
| - resolution range (last shell) | 79.06 Å - 2.00 Å |
| | (2.08 Å - 2.00 Å) |
| - intensity cutoff (σ(F)) | none |
| - number of reflections | 66712 |
| - completeness [working + test set] (last shell) | 90.1 % |
| - test set | 4.8% |
| Fit to data used in refinement: | |
| - overall R_{cryst} (last shell) | 20.9 % (26.0%) |
| - overall R_{free} (last shell) | 26.4 % (34.3 %) |
| - overall mean FOM | |
| Number of non-hydrogen atoms used in the refinement: | |
| -Total | 9026 |
| Overall mean temperature factor (B): (4.0Å-2.0Å) | 29.1 Å² |
| Overall mean temperature factor from Wilson plot | 31.1 Å² |
| RMS deviations from ideal geometry: | |
| - bond length | 0.008 |
| - bond angles | 1.2° |
| -estimated standard deviation from Luzzatti plot (cross validated) | 29.7 Å |
| - G factor from PROCHECK | 0.1 |

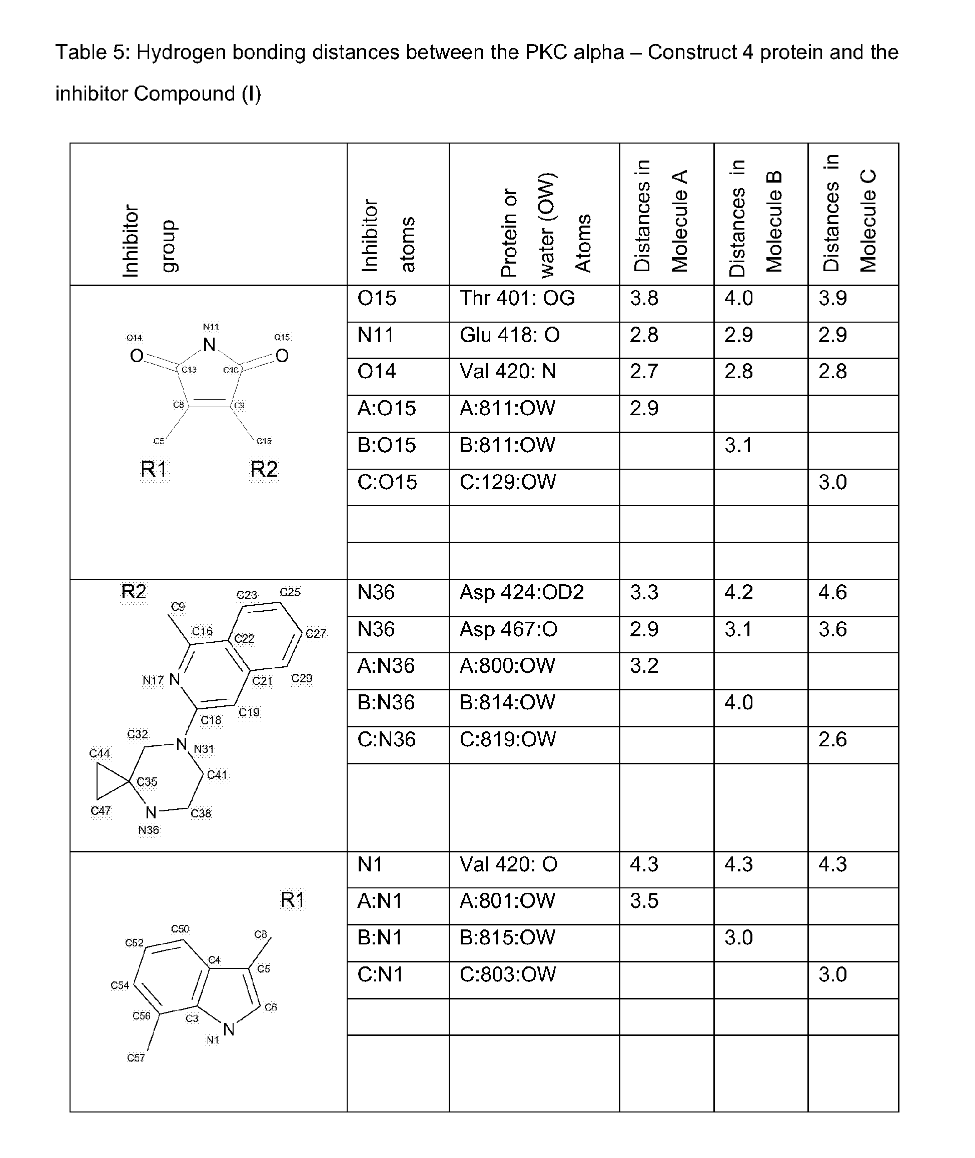

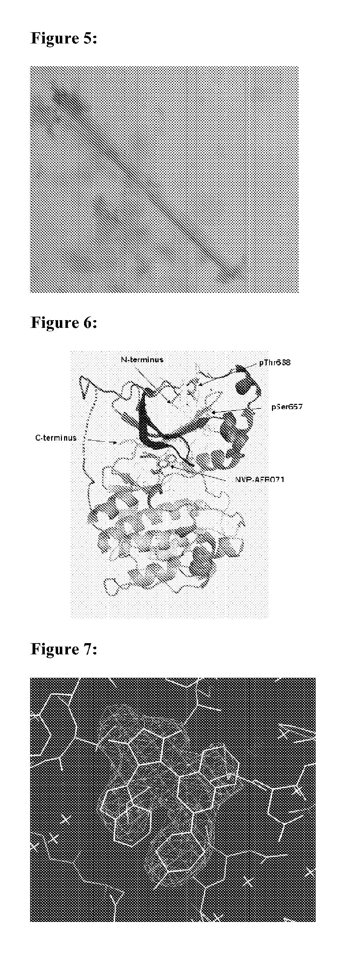

There are three protein molecules in the asymmetric unit of the crystal. The root mean square deviations between the Cα atoms of the three molecules are shown in Figure 4. The main differences can be explained by the different crystal contacts.

### Example 2: Crystal structure of PKC alpha kinase in complex with Compound (II) at 2.8 Å resolution

### 1. Methods

### Molecular biology for PKC alpha construct generation

The same procedure was used for generation of PKC alpha - Constructs 1-8, as described in Example 1. This includes also PKC alpha - Construct 4, PKC-alpha(320-672)T497E-His, which was used for obtaining the crystal structure of PKC alpha - Construct 4/Compound II.
The constitutions of PKC alpha - Constructs 1-8 are listed in Example 1.

### Protein Expression

Generation and amplification of recombinant BV for PKC alpha - Constructs 1-8, including Construct 4, was done as described in Example 1.
The same procedure for testing PKC alpha-expression in 50 mL-shake flask cultures was carried out as given in Example 1. However, typically 5 mL of a 10x concentrated solution of Compound (II) (400µM) in medium EX-CELL 420 + 1 % FCS was added to the cultures at the moment of BV infection to give a final concentration of 40 µM Compound (II).
For purification and successful crystallization and crystal structure elucidation for Construct 4/Compound (II), the same protocol was used for the liter-scale production of Construct 4/Compound (II) as described in Example 1 for Construct 4/Compound (I), except that 40 µM Compound (II) was used in the culture. A stock solution of 10 mM of Compound (II) in DMSO, stored at -20°C, was each time freshly diluted typically to 400 µM (10x concentrated) in expression medium and filter-sterilized through a 0.22 µm-filter prior to application. The same protocol for harvesting the cells expressing PKC alpha - Construct 4/Compound (II) was employed as described in Example 1.
Sample preparation and performing of cell parameter analysis and analysis of PKC alpha expression by SDS-PAGE was done as described in Example 1.

### Purification

The subsequently described protocol for purification of PKC alpha - Construct 4/Compound (II) differs only in minor points from that given in Example 1: Due to the sensitivity of the protein all the purification steps were carried out on ice or at 4°C. A 10g wet cell pellet was resuspended in app.100ml lysis buffer (300mM NaCl, 50mM Tris HCl, 3mM TCEP, 1mM NaF, 10µl Na3VO4, 10% glycerol, pH 8.0), containing 4 tablets of protease inhibitor cocktail Complete Mini, EDTA free (Roche) and 100µl Phosphatase Inhibitor cocktail 1 and 2 (Sigma). The suspension was treated with a hand homogenizer 10x and the insoluble part was centrifuged 60-180min with 30000g at 4°C. Subsequently the supernatant was passed through a 5µm filter. The supernatant was loaded on a Ni-NTA-Superflow column (Pharmacia; 1cm diameter, 3-4ml volume, flow 1-3ml/min, equilibrated with lysis buffer), washed with lysis buffer until absorption was close to zero (app. 15 column volumes), washed with lysis buffer plus 10 and 25mM imidazole and eluted with a gradient from 25 to 500mM imidazole in lysis buffer. Inhibitor containing fractions (colored) were pooled.
The pool was concentrated in a precooled Ultrafree Cell MW cutoff 30000 to 1-2ml, loaded on a HiLoad Superdex75 (16/60) prep grade (Pharmacia) and equilibrated in SPX buffer (200mM NaCl, 50mM imidazole, 1% glycerol, 1mM NaF, 2mM TCEP, pH = 8.0). Colored fractions were concentrated in an Ultrafree cell to a final protein concentration of 10-20mg/ml (determined by HPLC).
Mass spectroscopic analysis showed that the protein preparation was sufficiently pure for crystallization. The expected mass of the desMeth protein was 41385 Dalton for the 594 residues plus the His-tag and the two phosphorylation sites, which is in agreement with the measurements.

### Crystallization

The optimized crystallization condition for the successful Construct 4 - Compound (II) complex required a protein solution at 22 mg/ml PKCα in 0.2M NaCl, 0.05M imidazole pH 8.0, 2mM TCEP, 1 mM NaF, 2% glycerol. The protein was crystallized with the free interface diffusion method in glass capillaries (4 µl crystallization buffer containing 20-30% PEG3350 and 0.2 M di ammonium hydrogen citrate against 2 µl protein solution, 22mg/ml ). The hanging drop method also worked but did not give large enough crystals in that case at 4°C. Flat rod-like crystals typically appeared within 2-7 days at 4°C.
Single fragments of the bundles of crystals that grew were treated with cryo-buffer (20% glycerol, 23% PEG-3350 w/v, 200mM (NH₄)₂H-citrate) at 4°C by increasing the glycerol concentration slowly by adding cryo buffer gradually to the original drop. Subsequently, the crystals were frozen by dipping them in liquid nitrogen and stored for data collection.

### Data collection and analysis

Due to the small crystal size (thin rods of about 30 microns in diameter) synchrotron radiation was a must for these crystals. The data set was collected at the PXII (X10SA) beam line at the Swiss Light Source (SLS) in Villigen, Switzerland. This beam line is equipped with a MARCCD detector and a cryo-stream to keep the crystals at 100K. Images were collected with 1° oscillation each and a crystal-to-detector distance of 250 mm. To collect a full data set the crystal was translated along the longest dimension. Raw diffraction data were processed and scaled with XDS / Xscale [Kabsch 1988] within the APRV program package [Kroemer et al. 2004]. Structure factor amplitudes for negative and very weak intensities were estimated using Bayesian statistics [French and Wilson, 1978].

### Structure determination and refinement

The structure of the PKC alpha - Construct 4/Compound (II) complex was determined by molecular replacement with the PKC alphs -Construct 4/Compound (I) structure. The structure was refined using the programs COOT [Emsley P and Cowtan K., 2004] and REFMAC [Murshudov et al., 1997] which is part of the CCP4 program suite [Collaborative Computational Project, Number 4, 1994]. Cross-validation was used throughout refinement using a test set comprising 5% of the reflections. Pictures were prepared using the programs COOT and PYMOL (DeLano Scientific LLC).

### 2. Results

### Expression of PKC alpha - Construct 4/Compound (II) complex

The Construct 4, which led finally to the co-crystal structure of PKC alpha - Construct 4/Compound (II) shown in Figure 6, was sufficiently expressed with the PKC-specific inhibitor Compound (II) present in the culture. In the absence of the inhibitor, only residual levels of Construct 4 protein were expressed. The same expression behavior was also observed for the PKC alpha Constructs 1, 2, 3, 5, 6 and 7.
A dose-response experiment showed that maximal expression of PKC alpha - Construct 4/Compound (II) was obtained with 40 µM Compound (II) in the culture medium.

For the liter scale production of PKC alpha - Construct 4/Compound (II) leading to the crystal structure, a cellular yield of 14.63 g fresh weight cells per 760 ml culture was obtained and 14.9 mg of PKC alpha - Construct 4/Compound (II) was isolated after the Ni-column. This means that the level of expression of soluble PKC alpha - Construct 4/Compound (II) was at least 19 mg/l for this experiment. SDS-PAGE analysis of the total lysate revealed a strong band of the correct size on a Coomassie-stained gel and a correspondingly strong band on a Western blot treated with anti-PKC alpha-antibody. Approximately 50% of the PKC alpha - Construct 4/Compound (II) was found to be solubly expressed.

### Purification

For Compound (II), purification was only performed for PKC alpha-Construct 4/Compound (II). Mass spectroscopic analysis on the purified PKC alpha-Construct 4/Compound (II) complex showed that the protein preparation was sufficiently homogeneous for crystallization (41304.6 Da = 7% (des-Met, mono-phosphorylated); 41384.4 Da = 93% (des-Met, phosphorylated at T638 and S657)). The expected mass of the des-Met protein is 41385 Dalton (for the 354 residues plus the His-tag, the mutation T497E and the two phosphorylation sites). The expected mass was in agreement with the measurements.

### The crystals of the PKC alpha - Compound (II) complex

The construct crystallized is identical with the one of Example 1. Crystals of PKC alpha in complex with Compound (II) grew in the same space group as the once with Compound (I). Again they grew as thin needles of about 30 microns in diameter with the tendency to split ends (Figure 5). The asymmetric unit of the crystal contains again 3 molecules. The chemical structures of the inhibitors 3-(1H-Indol-3-yl)-4-[2-(4-methyl-piperazin-1-yl)-quiazolin -4-yl]-pyrrole-2,5-dione present in the crystals is shown in formula (II). The X-ray diffraction data statistics can be found in Table 6 below. 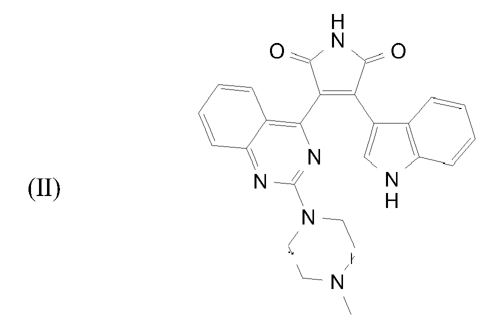 Table 6: X-ray diffraction data of the PKC alpha - Construct 4/Compound (II) crystal

| | | **PKC alpha + Compound (II)** |
|---|---|---|
| Synchrotron beam line | | SLS (PXII, X10SA) |
| Wavelength | | 0.9788 |
| Detector | | MARCCD225 |
| Oscillation range | | 1.0 |
| Distance | | 250 mm |
| Number of images used | | 120 |
| Temperature of data collection | | 100K |
| Number of crystals | | 1 |
| Space group | | P2₁2₁2₁ |
| Unit cell dimensions | a, b, c, | 43.9Å, 100.7Å, 251.3Å |
| | α, β, γ | 90.0°, 90.0°, 90.0° |
| Number of monomers / asymmetric unit | | 3 |
| Packing coefficient | | 2.23 Å³/Dalton |
| Solvent content | | 44.5% |
| Overall B-factor from Wilson Plot (resolution range) | | 80.0Å - 2.8Å |
| Resolution range (last shell) | | 64.4 Å -2.80 Å (2.92 Å - 2.80 Å) |
| Number of observations | | 127470 |
| Number of rejections | | 780 |
| Number of unique reflection | | 28331 |
| Mosaicity | | 0.14 |
| Overall: | | |
| Data redundancy | | 4.5 |
| Data completeness (last shell) | | 99.5 % (98.5 %) |
| < I/ σ (I)> (last shell) | | 9.2 (2.3) |
| R_{merge} (last shell) | | 13.8% (66.4%) |
| Reflections with I > 3σ(l) | | 64.9% (24.2%) |

### The structure of the PKC alpha - Construct 4 in complex with Compound (II)

The structure of PKC alpha - Construct 4/Compound (II) was solved using the structure of the PKC alpha - Construct 4/Compound (I) complex for molecular replacement. Due to the large unit cell with three molecules in the asymmetric unit and the relatively low resolution a full refinement was not possible. Only coordinates (x,y,z) and an overall B-factor were refined. The refinement statistics is given in Table 7. The overall fold (Figure 6) corresponds closely to the one observed for the complex with Compound (I). Due to the good starting model, the initial, unbiased difference electron density (Figure 7), calculated from the diffraction data, unambiguously showed how the Compound (II) is oriented in the active site.

**Table 7: Refinement statistics on the crystal structure of PKC alpha - Construct 4/Compound (II)**

| Table | **PKC alpha - Compound (II)** |
|---|---|
| - resolution range | 64.42 - 2.80 Å |
| (last shell) | (2.873 - 2.80 Å) |
| - intensity cutoff (sigma(F)) | None |
| - number of reflections | 26913 |
| - completeness [working + test set] (last shell) | 98.59 % |
| - test set | 5.0 % |
| Fit to data used in refinement: | |
| - overall R_{cryst} (last shell) | 20.97 % (24.9 %) |
| - overall R_{free} (last shell) | 28.70 % (30.9 %) |
| - overall mean FOM | 0.79 |
| Total Number of non-hydrogen atoms used in the refinement: | 8960 |
| Overall mean temperature factor (B): (4.0Å-2.8Å) | 48.27Å² |
| - estimated standard deviation based on ML | 35.7Å |
| RMS deviations from ideal geometry: | |
| - bond length | 0.019 |
| - bond angles | 1.849° |
| - G factor from PROCHECK | -0.1 |

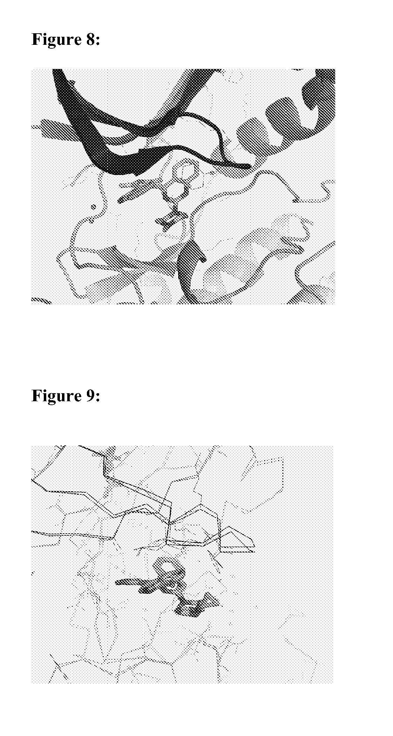

Figure 8 shows how Compound (II) is located in the ATP-binding pocket of the kinase underneath the P-loop. Comparing the two structures clearly indicates that there are small but significant differences in the positioning of the compound as well as in the positioning of the P-loop (Figure 9). Compound (I) is with the diaza-spiro-octyl group bulkier than Compound (II). This forces the P-loop in a slightly more open conformation. Compound (II) however can enter a little bit deeper into the binding pocket which is reflected in the hydrogen bond distances given in Table 8. The conformational changes between these two structures are indeed small but they give a good example how small differences in the inhibitor can have long range effects on the structure. There are also differences between the three molecules in the asymmetryc unit, residues 550-570 and 626-640. These are caused by different crystal contacts and are not relevant for the compound binding in the active site.

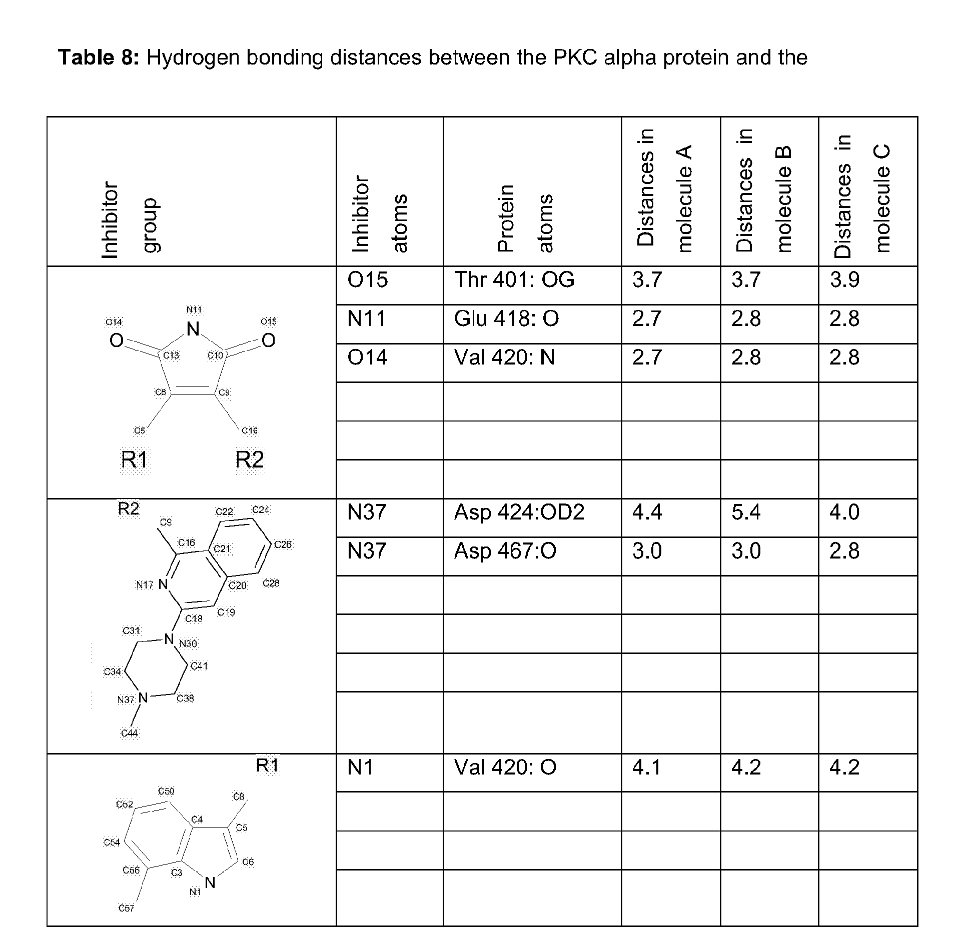

### Conclusion

These two new PKC alpha complex structures can now serve as basis for drug design, aiming for the improvement of the specificity of the compounds between the different PKC isoforms. All the residues in the ATP binding sites of PKC alpha and PKC theta are identical, except one. PKC alpha has Met470 and PKC theta has Leu511 at the corresponding positions. The fact that these two residues occupy about the same space and have about the same volume, makes it difficult, to specifically target these small differences. However Methionin residues are known to be more flexible than Leucin residues. The -S-CH3 of the Methionin side chain group might be displaced easier than the Leucin side chain. Furthermore the flexibility of the structural elements around the ATP binding site can be explored as well.

### References

Collaborative Computational Project, Number 4 (1994). The CCP4 suite: Programs for Protein Crystallography. Acta Cryst; D50:760-763.
Geiser M, Cebe R, Drewello D, Schmitz R. (2001). Integration of PCR fragments at any specific site within cloning vectors without the use of restriction enzymes and DNA ligase. Biotechniques; 31:88-92.
Emsley P, Cowtan K (2004). Coot: model-building tools for molecular graphics. Acta Cryst; D60:2126-2132 Part 12 Sp Iss.
Finkenzeller et al. (1990). Sequence of human protein kinase C. Nucl. Acids Res. 18: 2183
French GS, Wilson, KS (1978). On the Treatment of Negative Intensity Observations. Acta Cryst; A34:517-525.
Jones TA, Zou JY, Cowan SW, Kjeldgaard M (1991). Improved methods for binding protein models in electron density maps and the location of errors in these models. Acta Cryst; A47:110-119.
Kabsch W (1988). J Appl Crystallogr; 21:67-71.
Kroemer M, Dreyer MK, Wendt KU (2004). APRV - a program for automated data processing, refinement and visualization. Acta Crystallogr; D60:1679-1682.
McCoy AJ, Grosse-Kunstleve RW, Storoni LC, Read RJ (2005). Likelyhood-enhanced fast translation functions. Acta Cryst. D61, 458-464.
Messerschmidt A, Macieira S, Velarde M, Bädeker M, Benda Ch, Jestel A, Brandstetter H, Neuefeind T, Blaesse M (2005). Crystal Structure of the Catalytic Domain of Human Atypical Protein Kinase C-iota Reveals Interaction Mode of Phosphorylation Site in Turn Motif. J Mol Biol; 352:918-931.
Murshudov GN, Vagin AA, Dodson EJ (1997). Refinement of Macromolecular Structures by the Maximum-Likelihood Method. Acta Crys; D53:240-255.
Needleman and Wunsch (1972) J. Miol. Biol. 48: 443-453
Newton AC (2001). Protein kinase C. Structural and spatial regulation by phosphorylation, cofactors, and macromolecular interactions. Chem. Rev. 101: 2353-2364.
Newton AC (2003). Regulationj of the ABC kinases by phosphorylation: protein kinase C as a paradigm. Biochem J; 370:361-371.
Ochoa WF, Corbalán-Garcia S, Eritja R, Rodríguez-Alfaro JA, Gómez-Fernández JC, Fita I, Verdaguer N (2002). Additional Binding Sites for Anionic Phospholipids and Calcium Ions in the Crystal Structures of Complexes of the C2 Domain of Protein Kinase C alpha. J Mol Biol; 320:277-291.

O'Reilly DR, Miller LK and Luckow VA, (1994). Baculovirus Expression Vectors. Oxford University Press, NY.
Pappa H, Murray-Rust J, Decker LV, Parker PJ & McDonald NQ (1998). Crystal structure of the C2 domain from protein kinase C-delta. Structure; 6:885-894.
Pearson and Lipman, (1988) PNAS, USA, 85: 2444-2448
Rummel G, Stark W, Strauss A, Wagner J (2004). Crystal structure and three-dimensional structure of the catalytic domain of human protein kinase C-theta and inhibitor complexes for use in drug discovery. PCT lnt. Appl; WO 2004/076654
Smith and Waterman (1981) J. Theor. Biol., 91: 370-380
Sutton RB, Sprang SR (1998). Structure of the protein kinase Cβphospholipid-binding C2 domain complexed with Ca2+. Structure; 6:1395-1405.
Tan Seng-Lai & Parker Peter J (2003). Emerging and Diverse Roles of PKC in Immune Cell Signaling. Biochemical Journal; 376(3):545-552.
Verdaguer N, Corbalan-Garcia S, Ochoa WF, Fita I, Gómez-Fernández JC (1999). Ca2+ bridges the C2 membrane-binding domain of protein kinase C alpha directly to phosphatidylserine. EMBO J; 18:6329-6338.
Xu Z-B, Chaudhary D, Olland S, Wolfrom S, Czerwinski R, Malakian K, Lin L, Stahl ML, Joseph-McCarthy D, Benander C, Fitz L, Greco R, Somers WS, Mosyak L (2004). Catalytic Domain Crystal Structure of Protein Kinase C-theta (PKC-theta). J Biol Chem; 279(48):50401-50409. (PCT lnt. Appl; WO2005/097983)
Zhang G, Kazanietz MG, Blumberg PM, Hurley JH (1995). Crystal structure of the Cys2 Activator-Binding domain of protein Kinase C. delta in complex with phorbol ester. Cell; 81:917-924.
Zhang H.C. et al. (2005). Novel indolylindazoylmaleimides as inhibitors of protein kinase C-beta: Synthesis, biological activity, and cardiovascular safety. J. Med. Chem., 48,1725-1728, Supporting Information, p.S1-S7

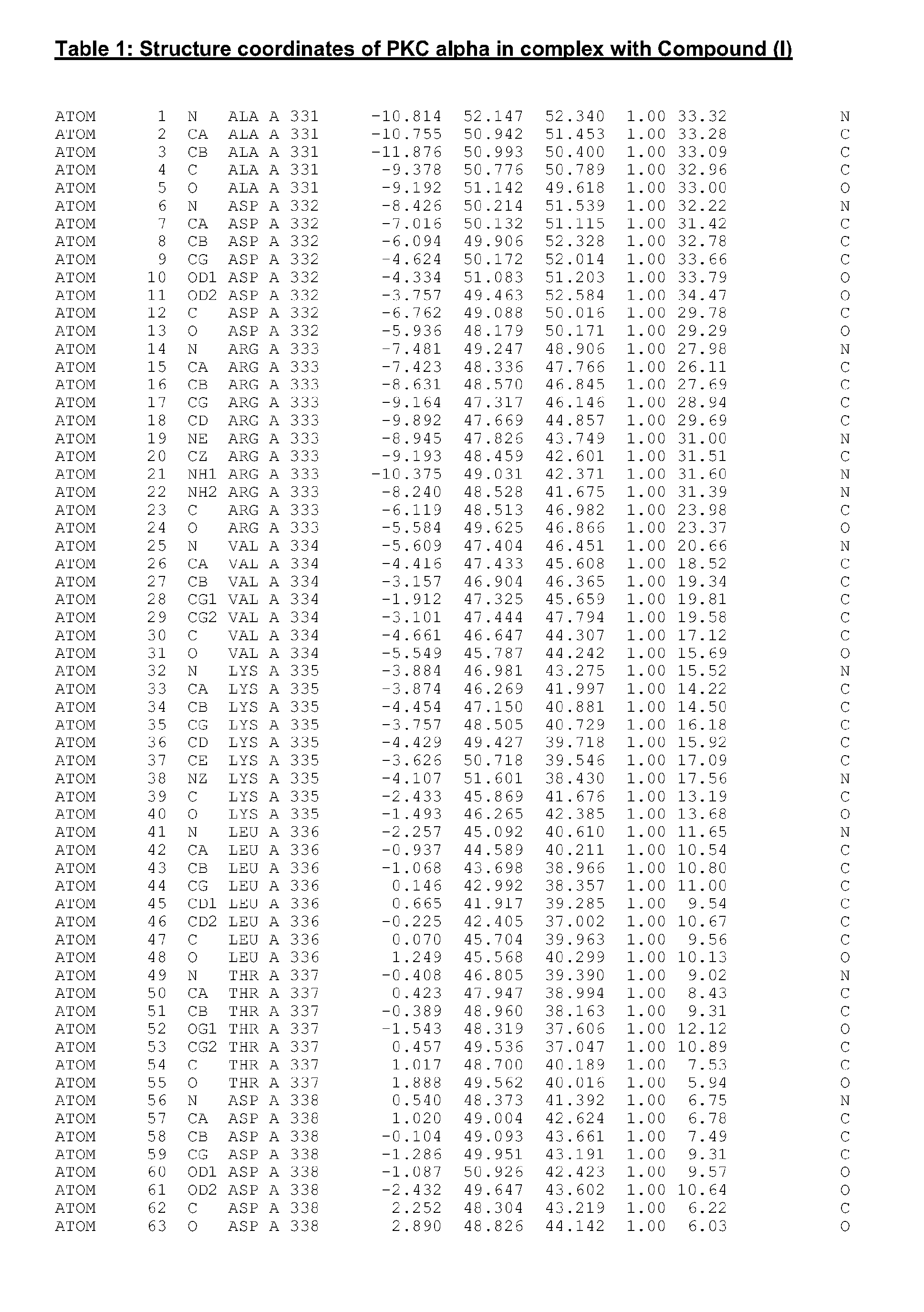

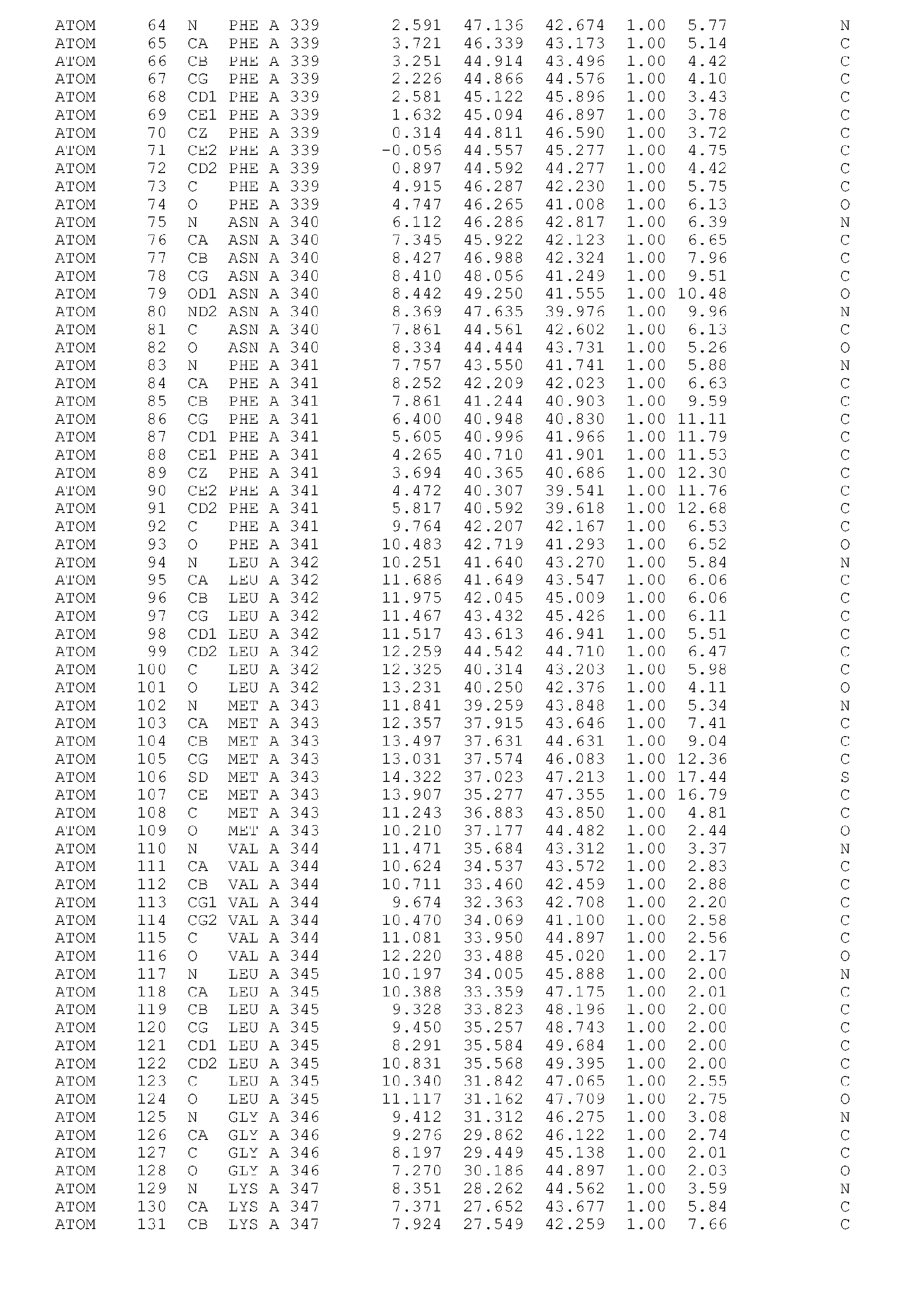

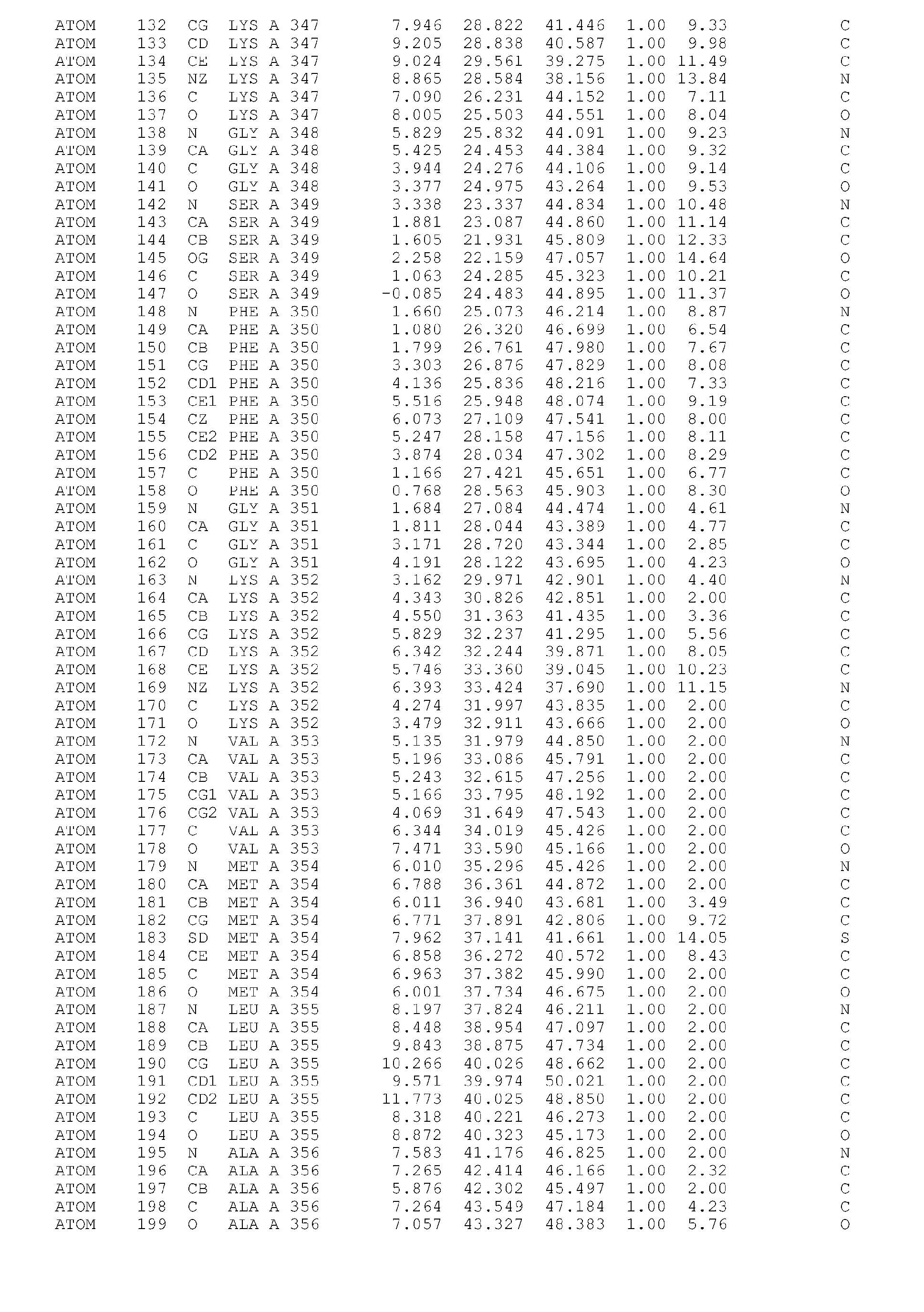

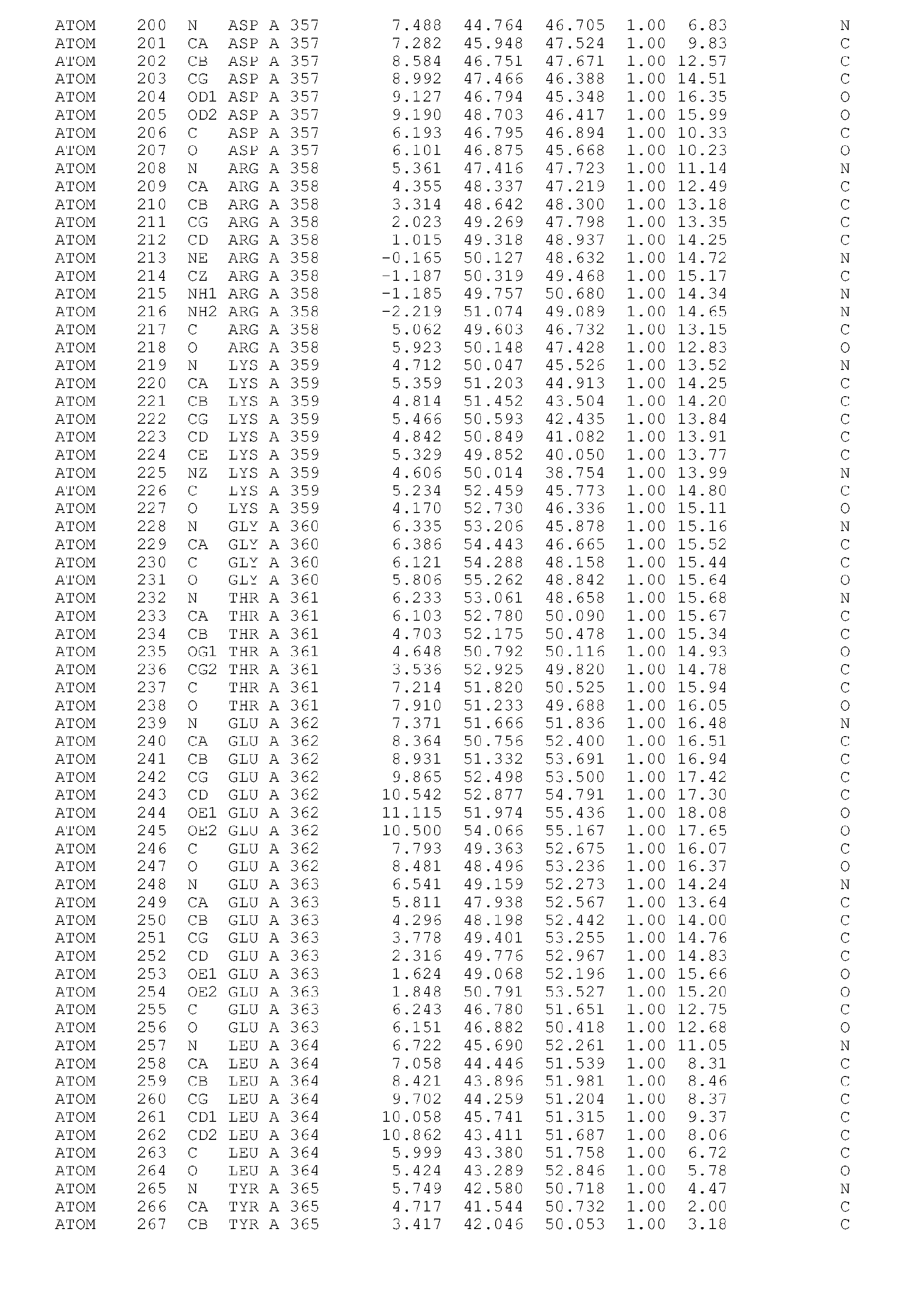

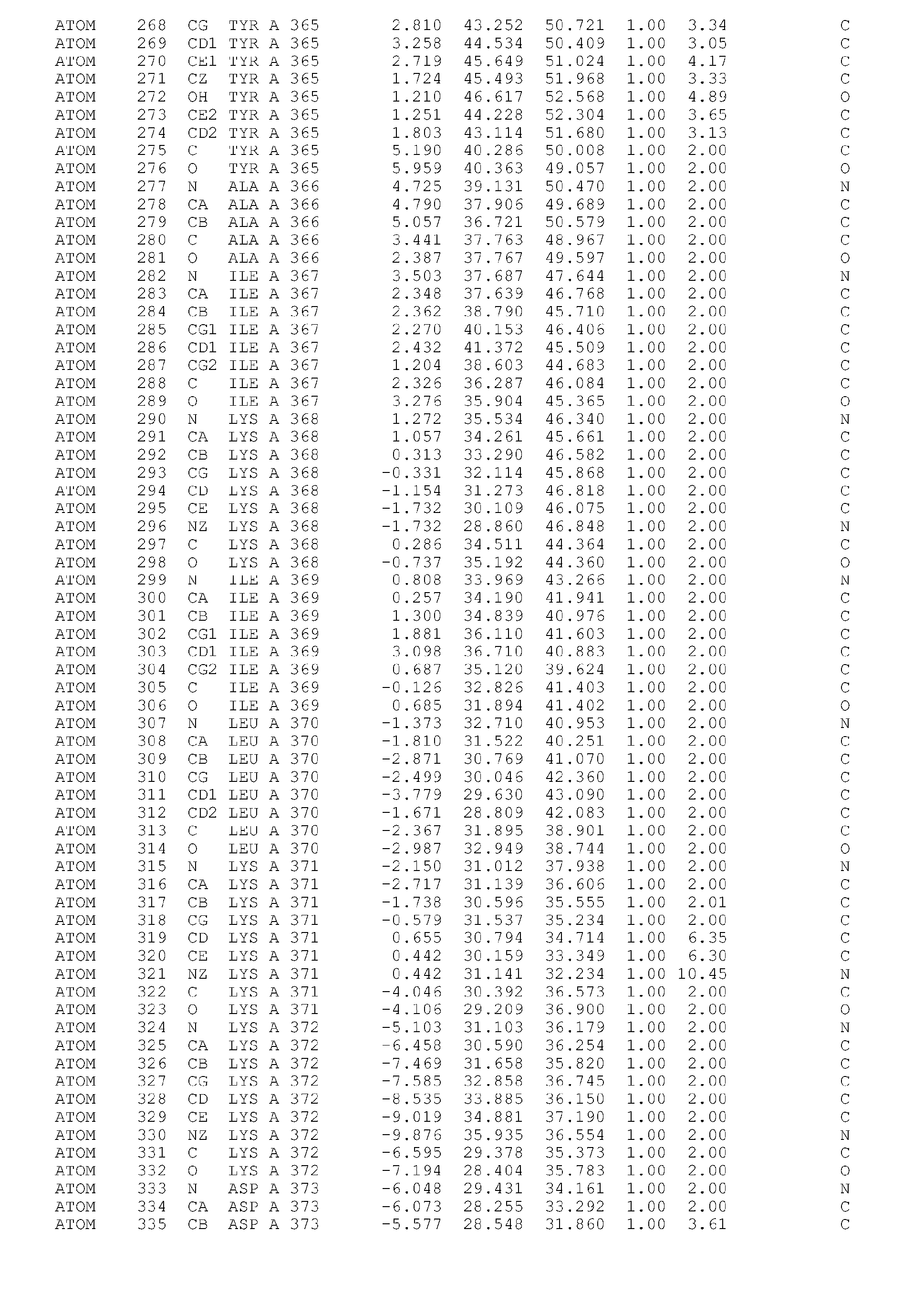

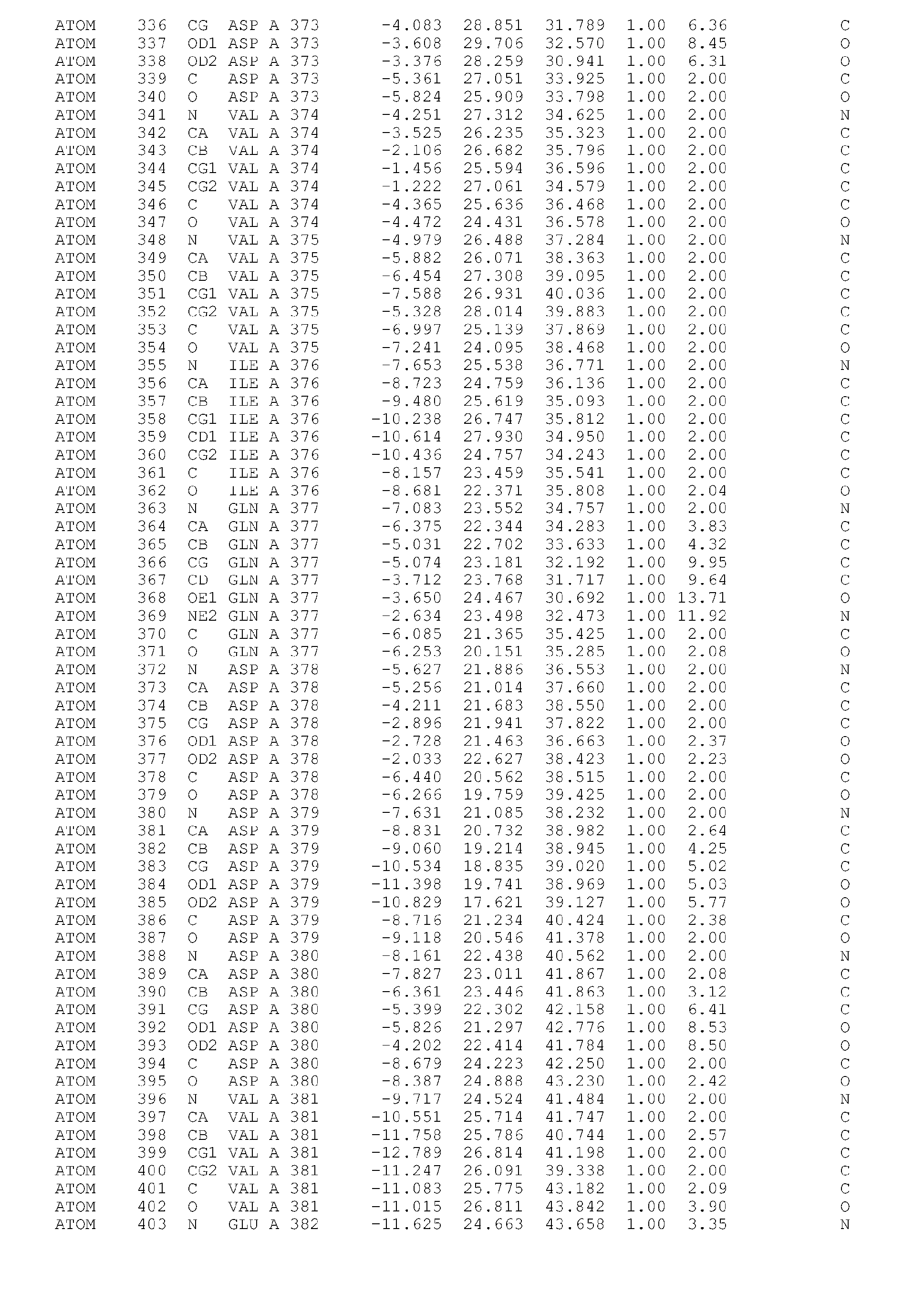

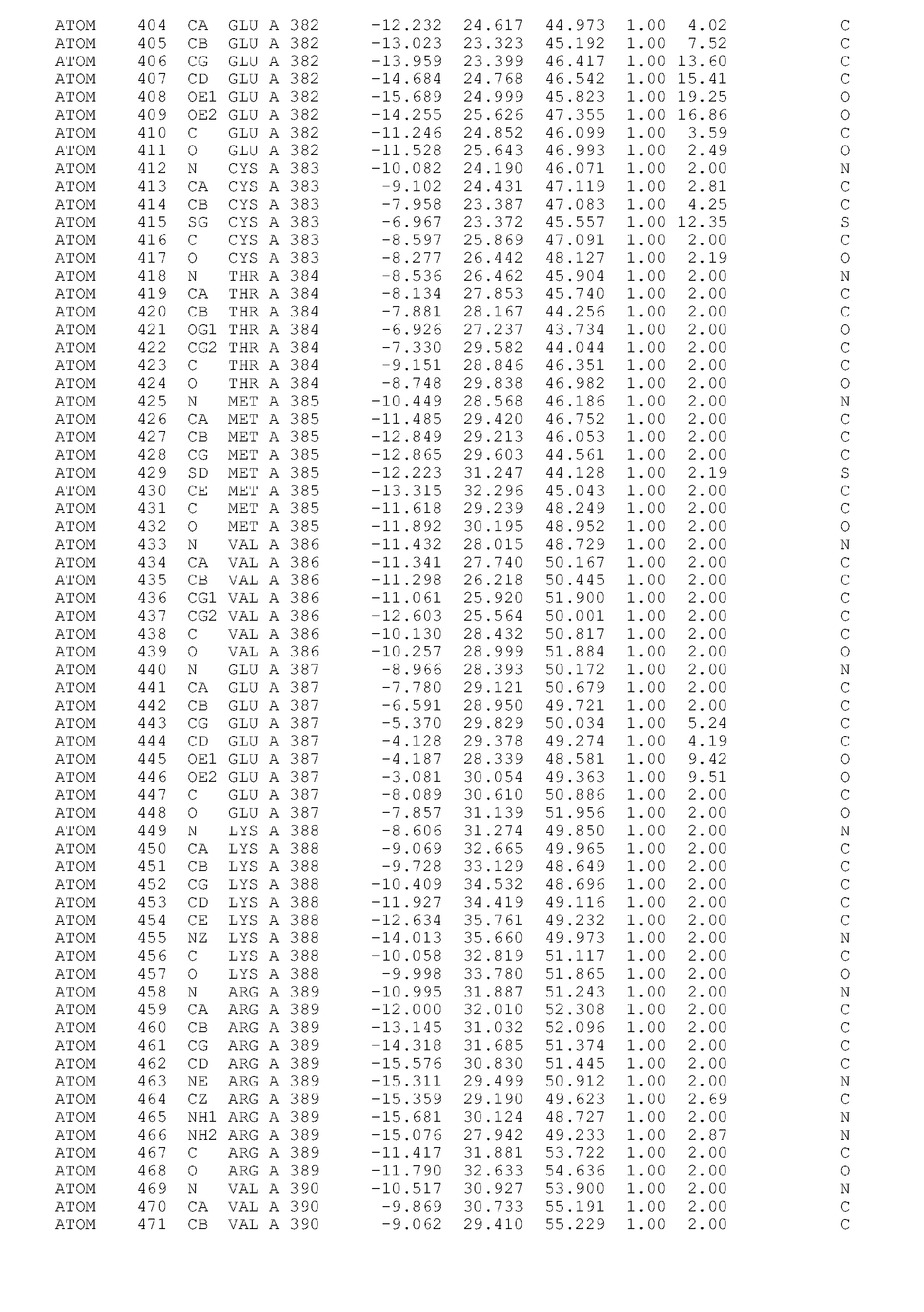

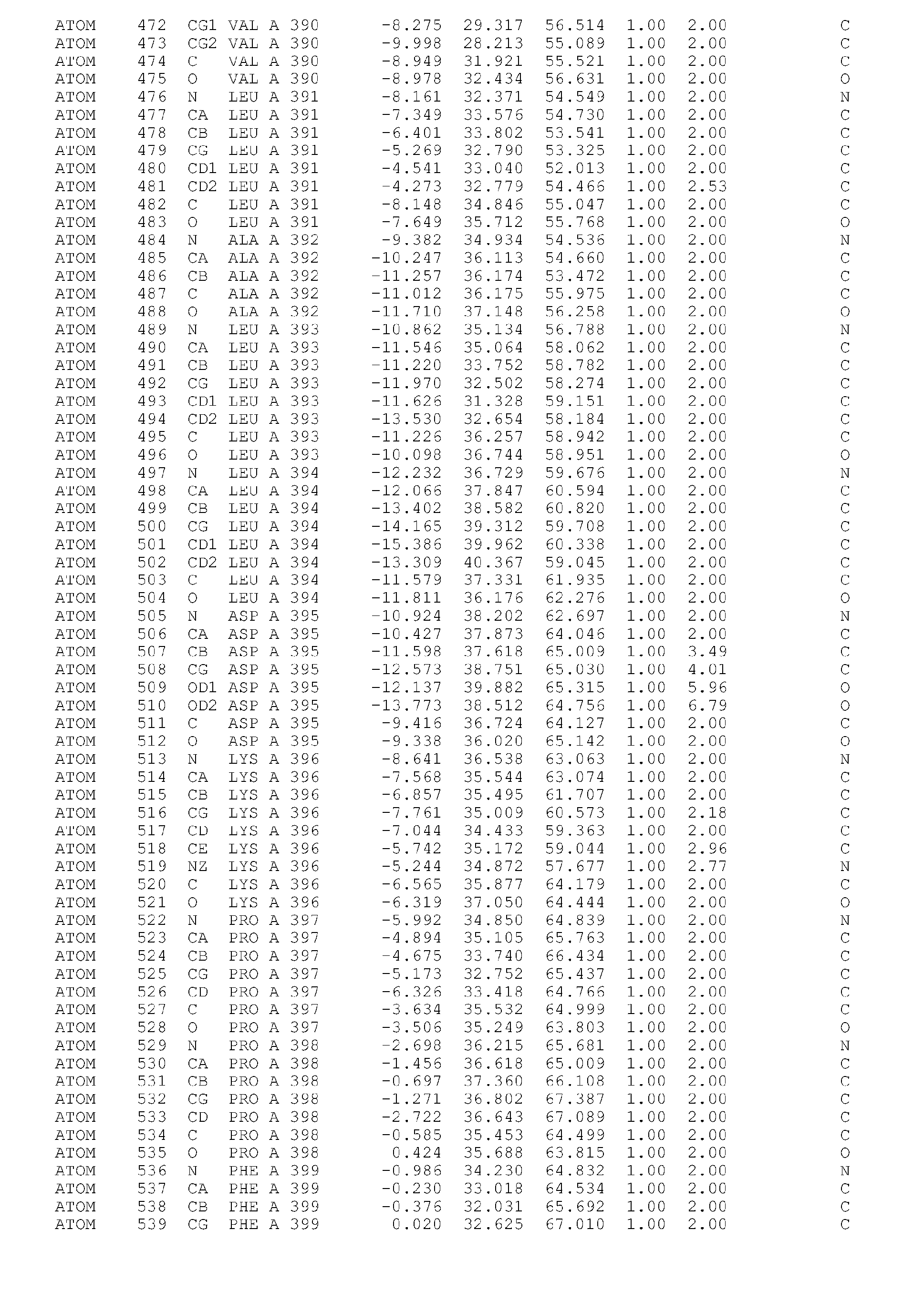

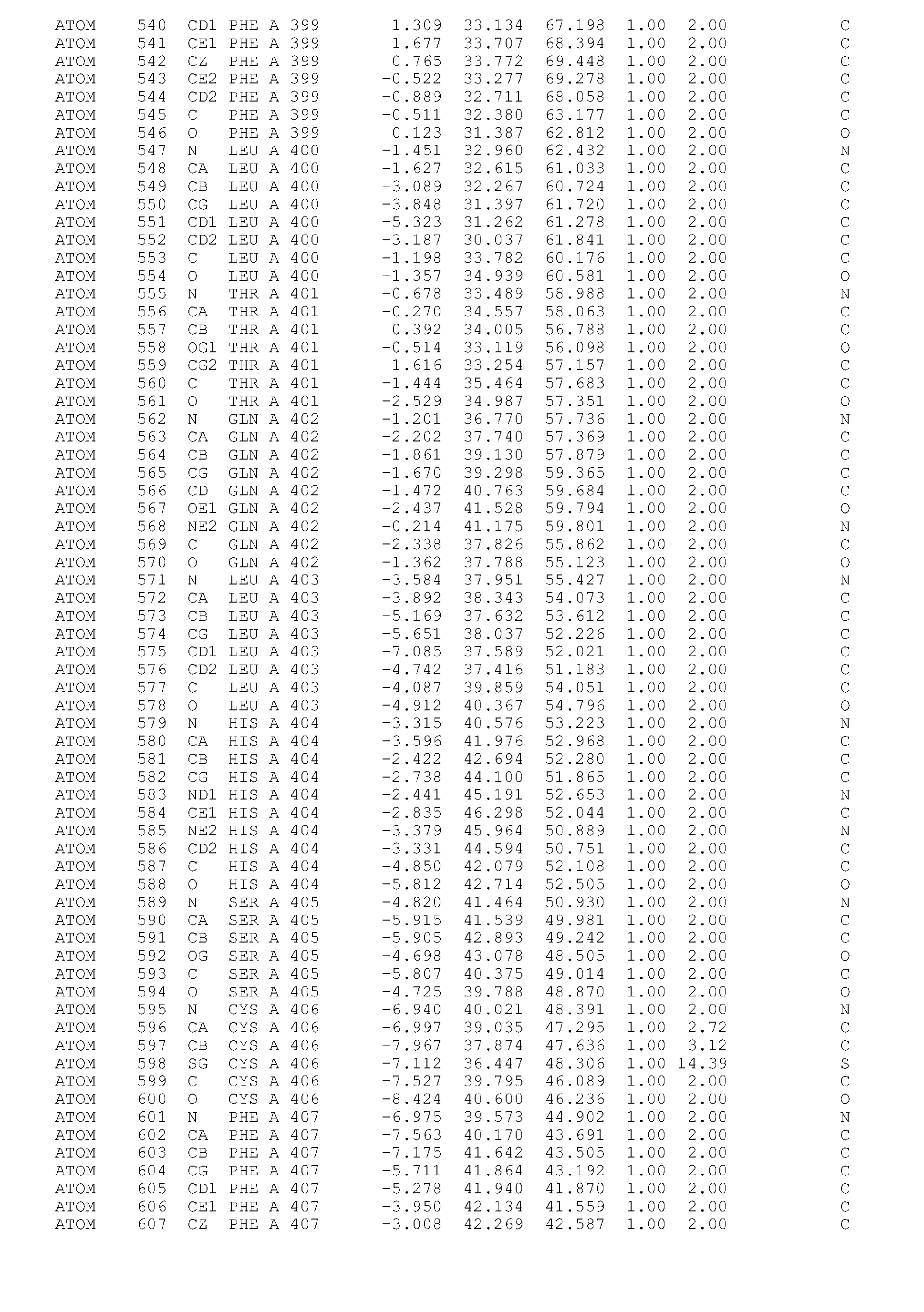

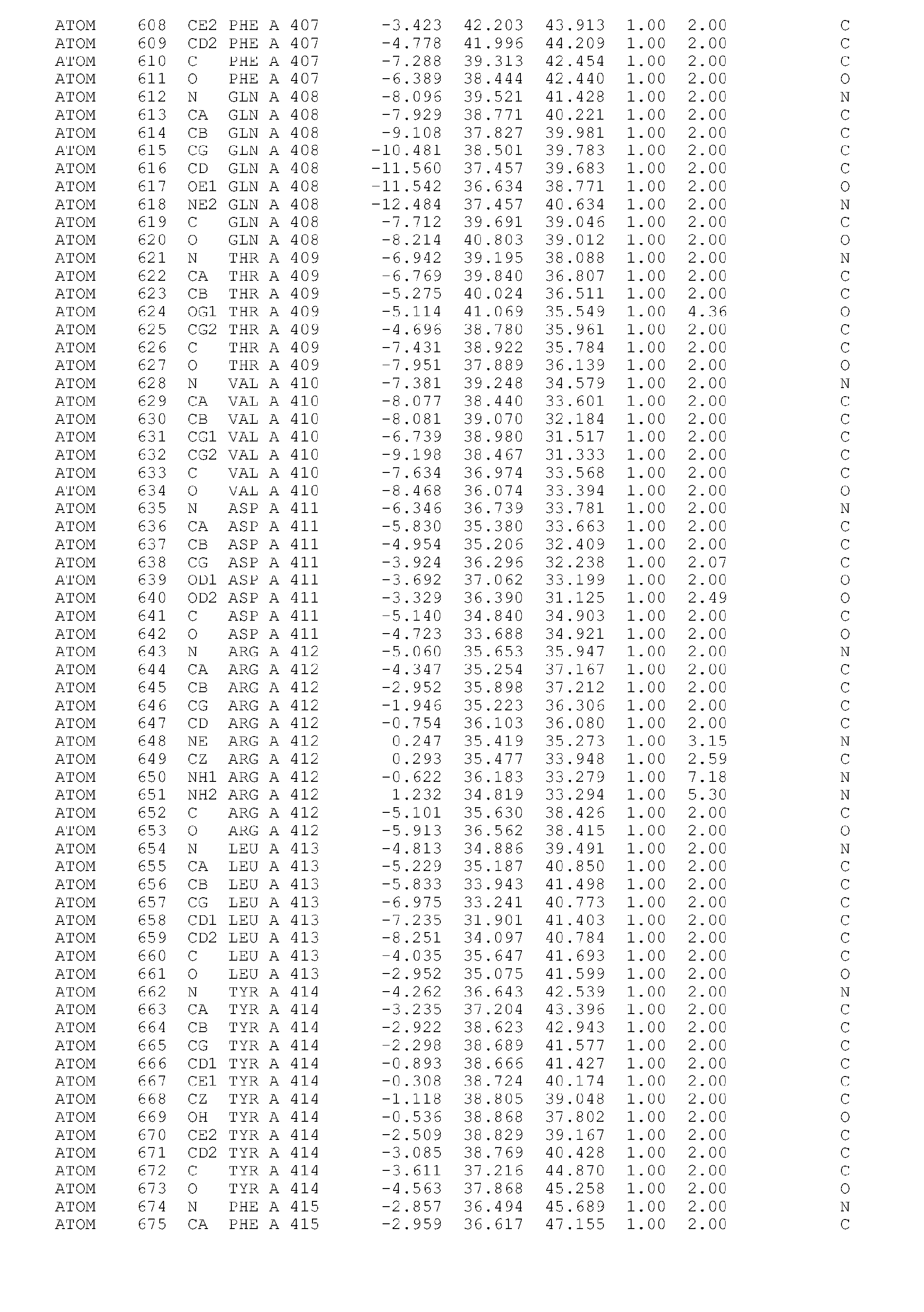

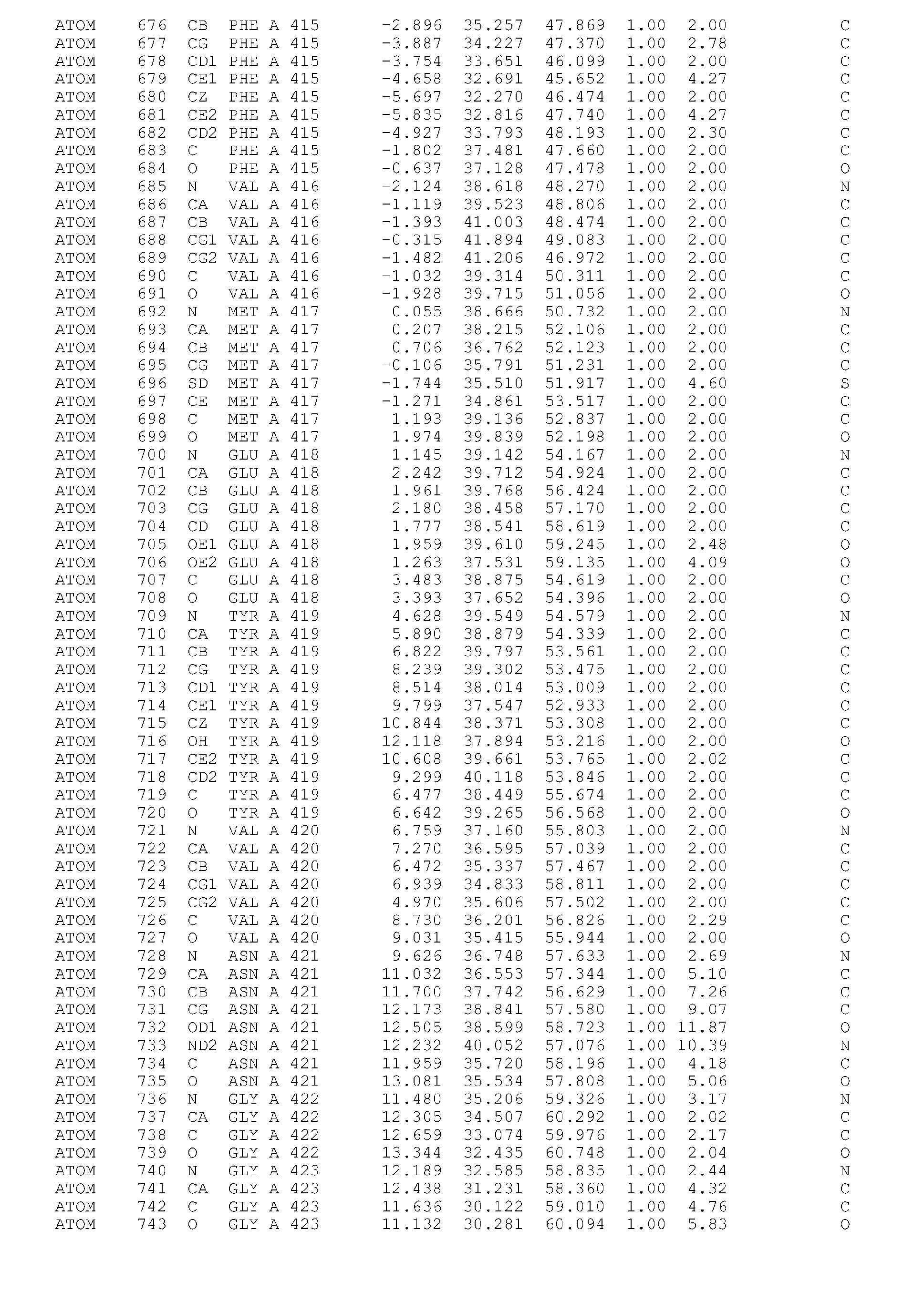

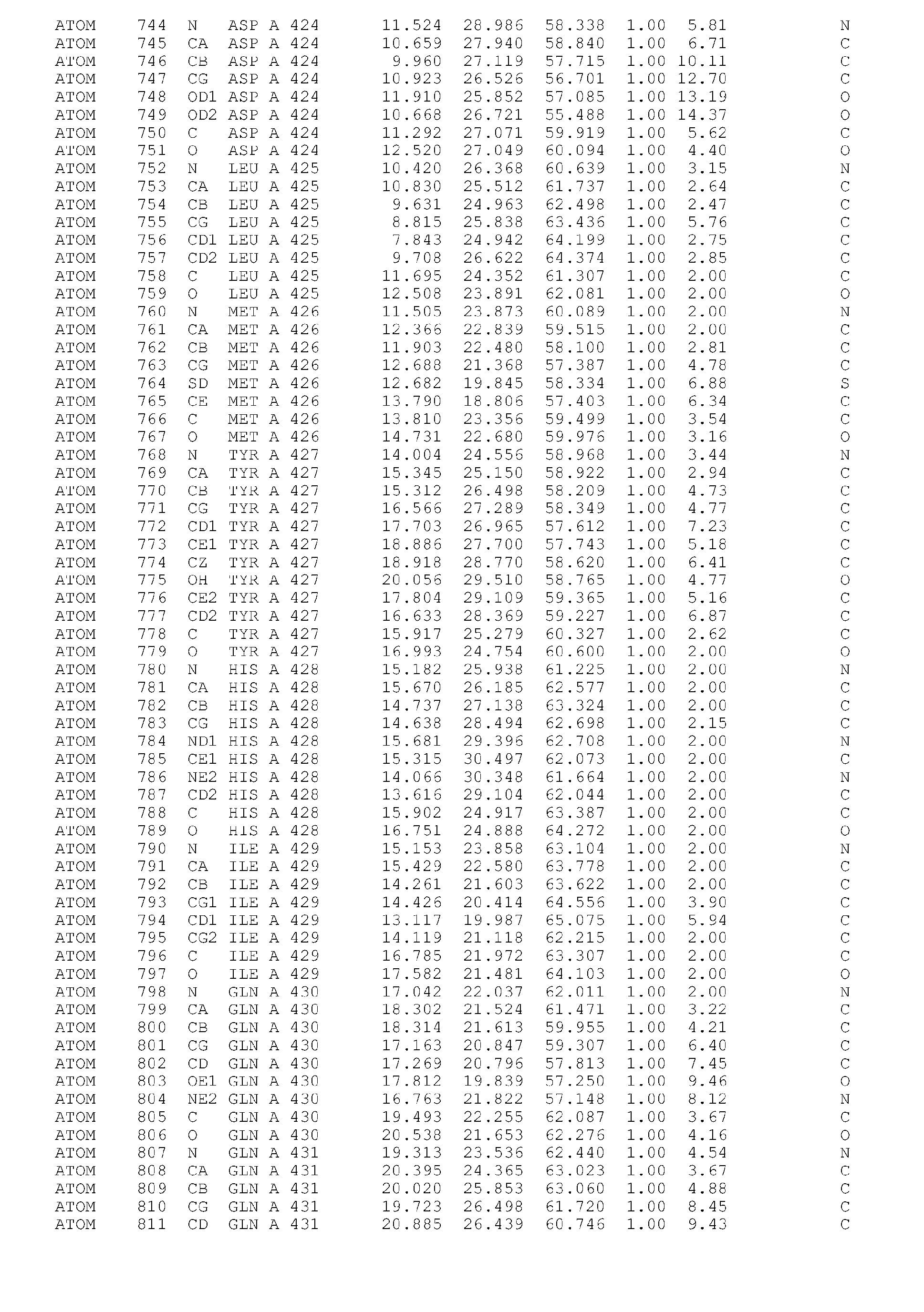

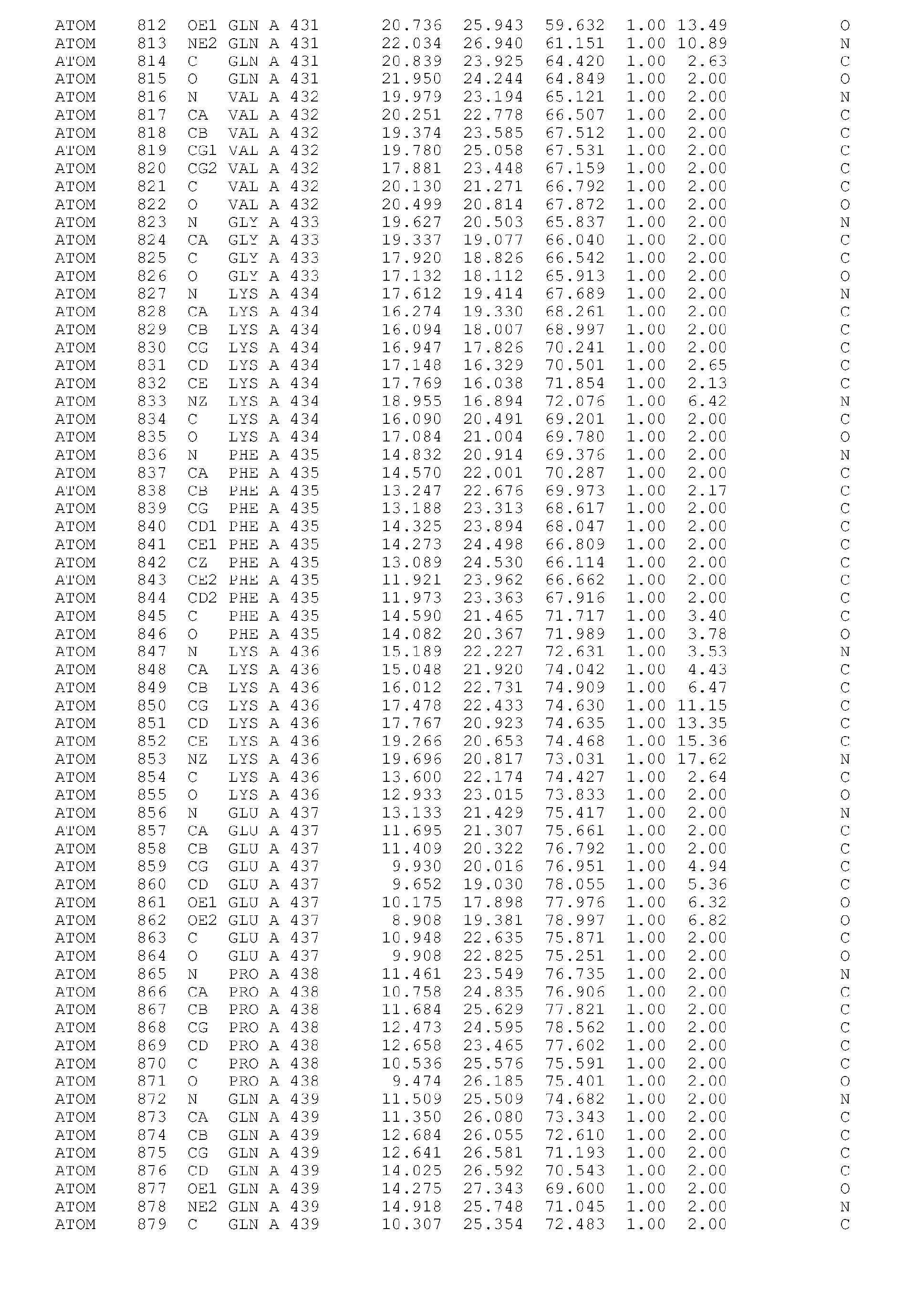

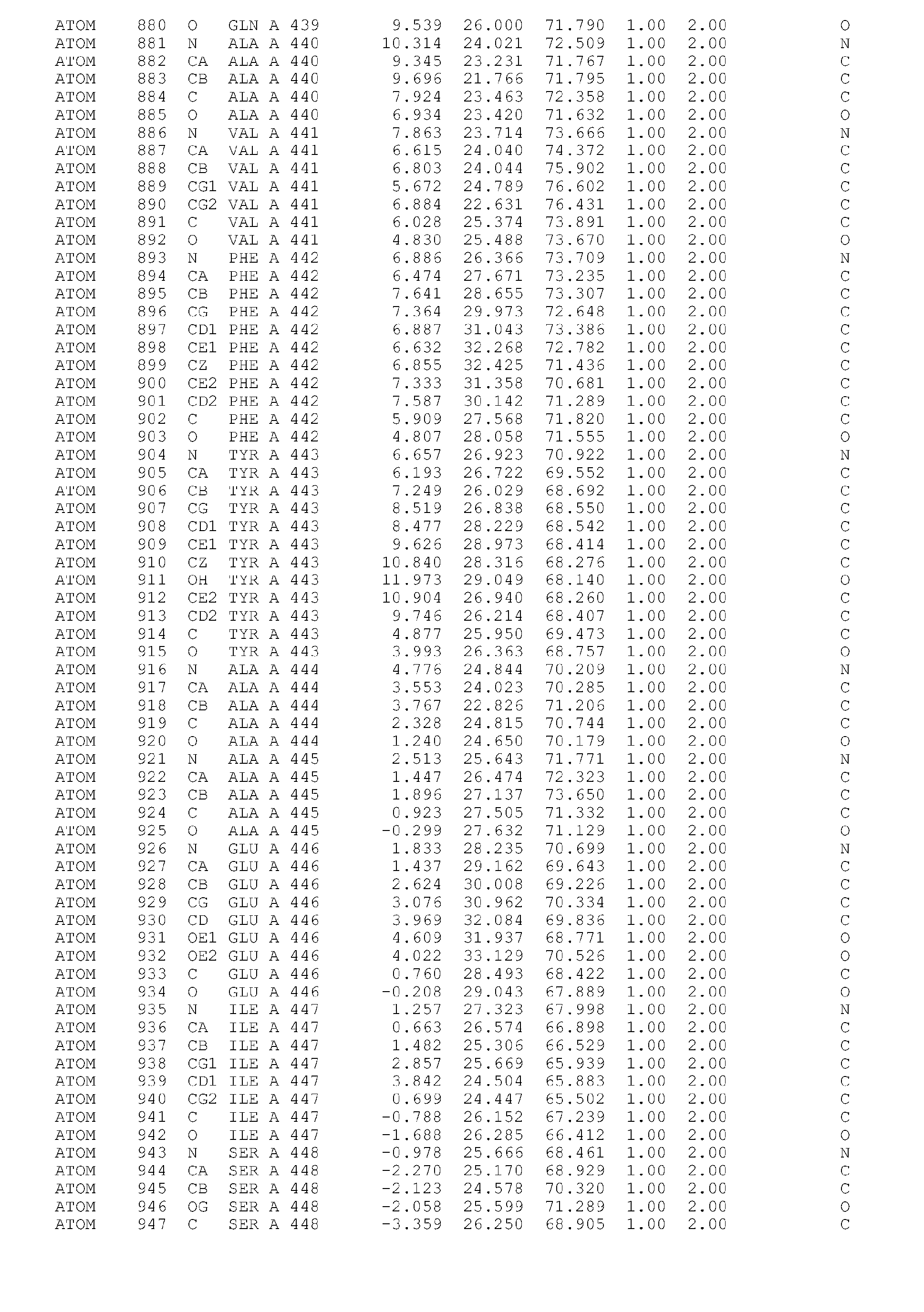

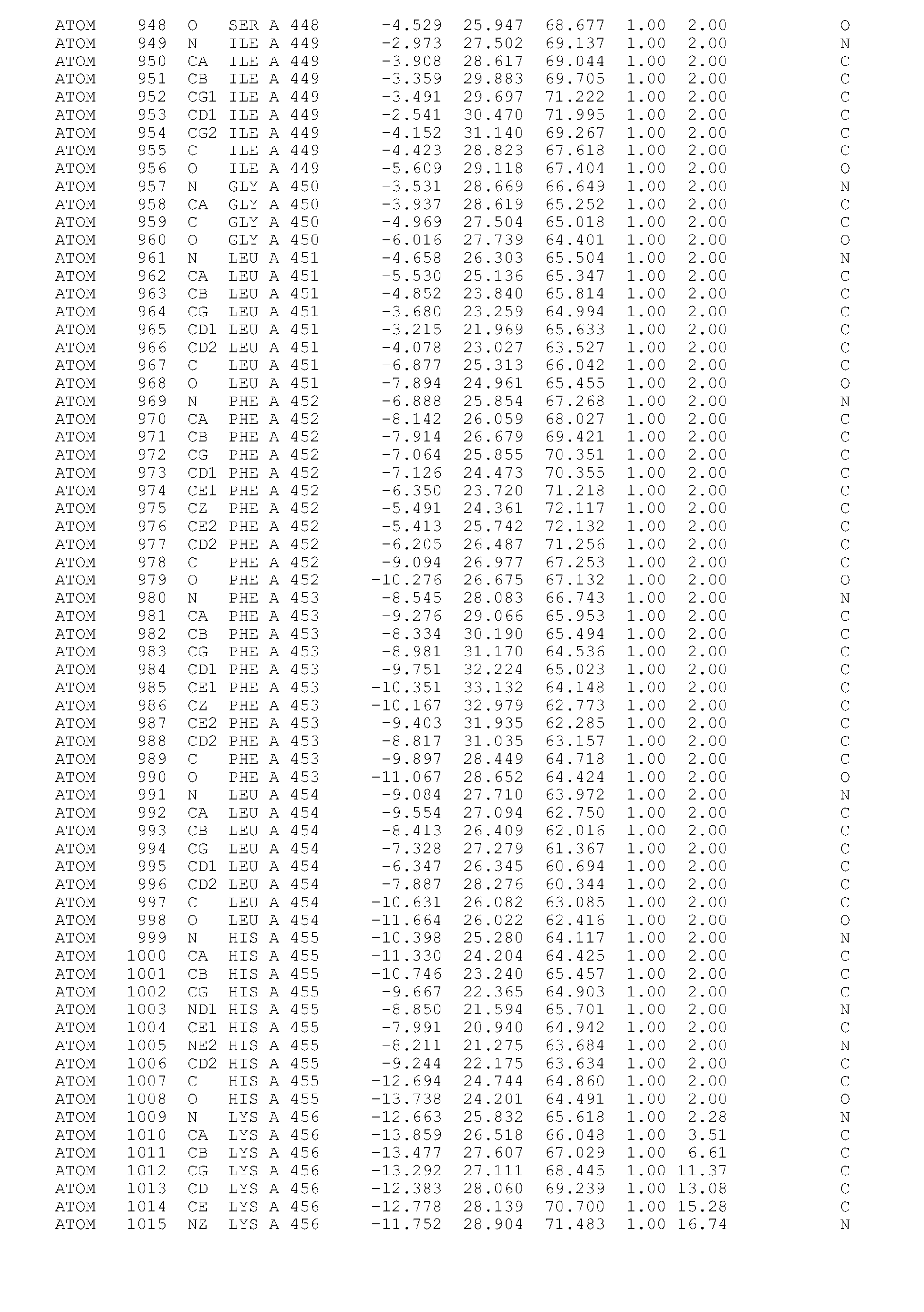

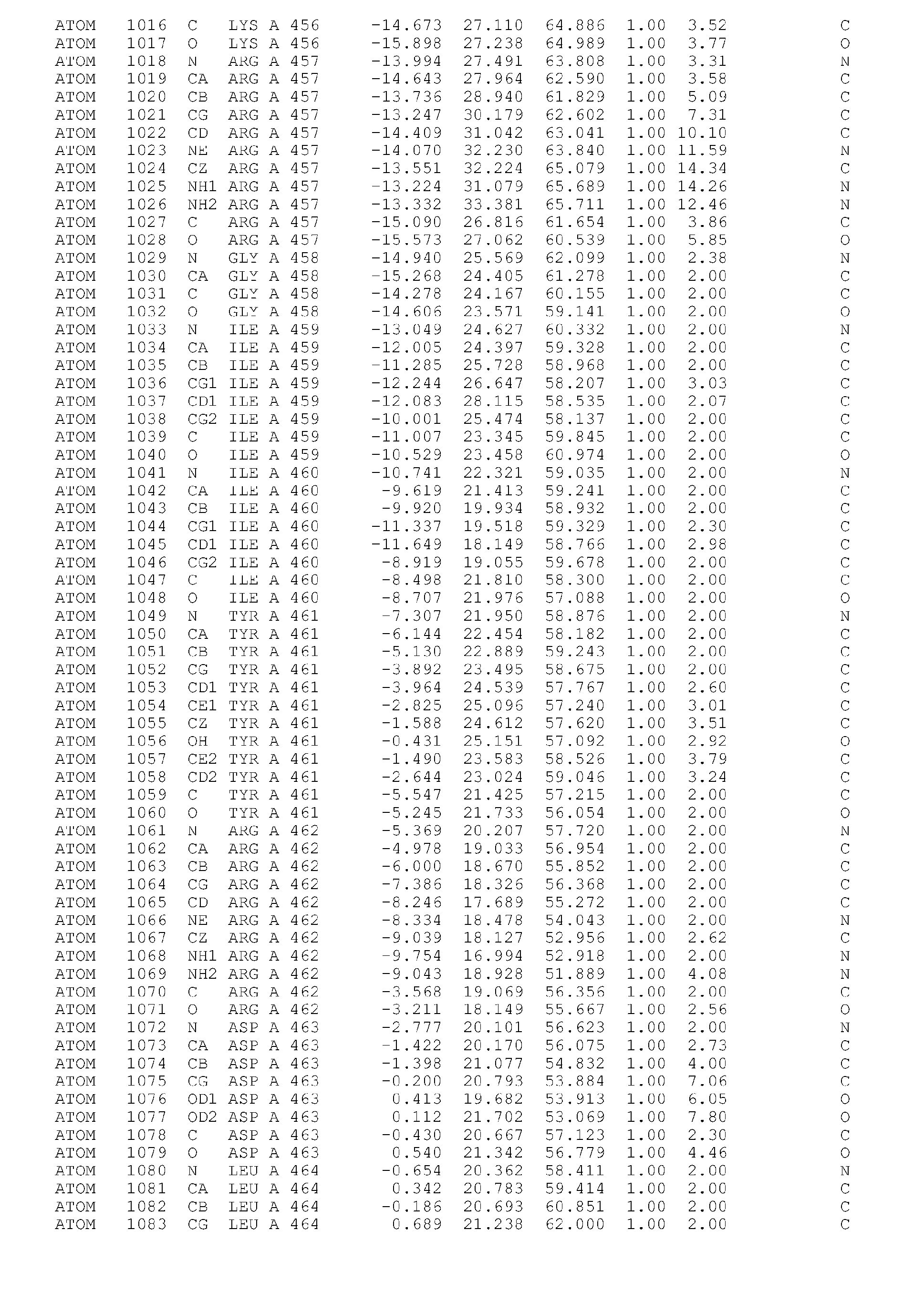

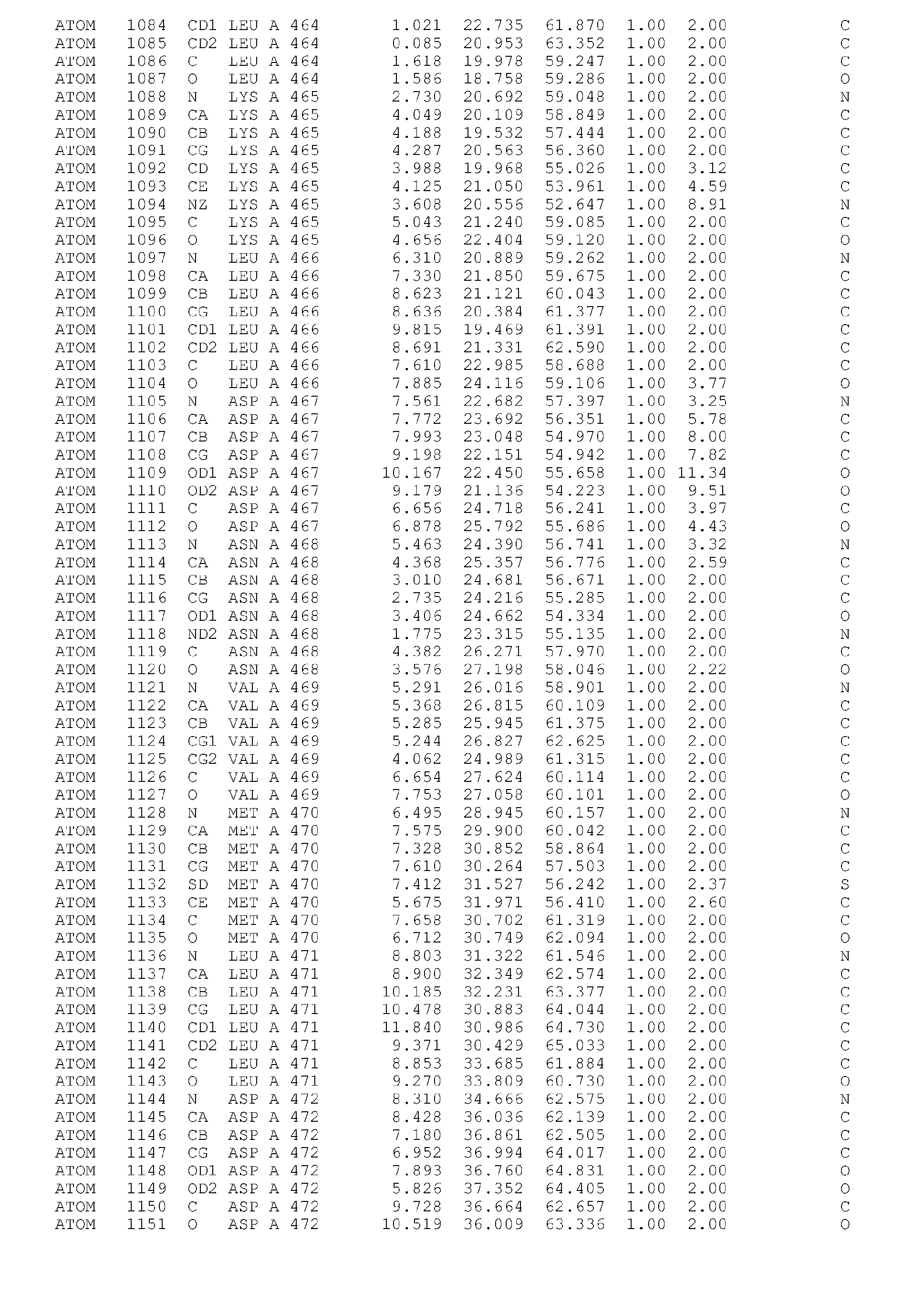

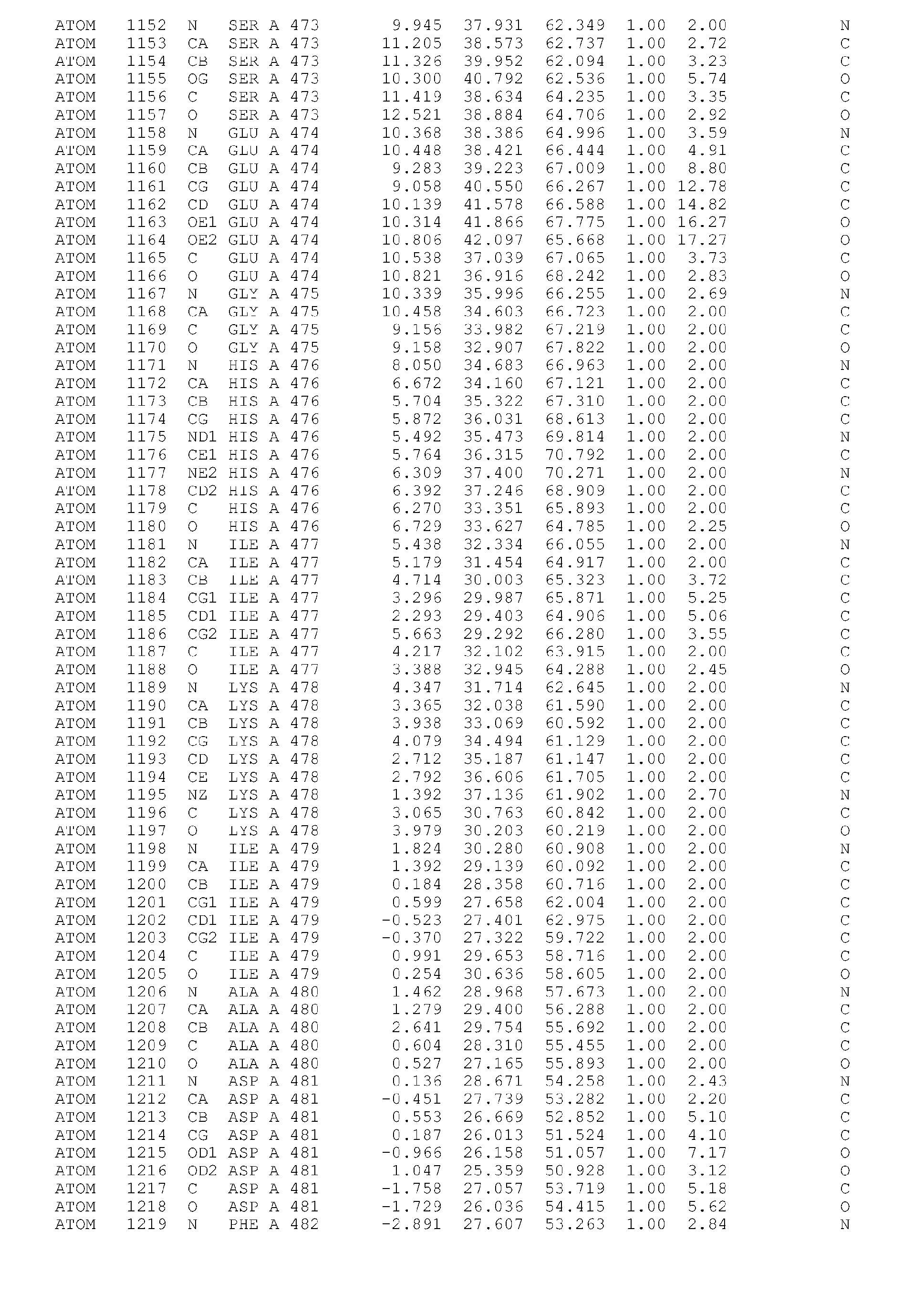

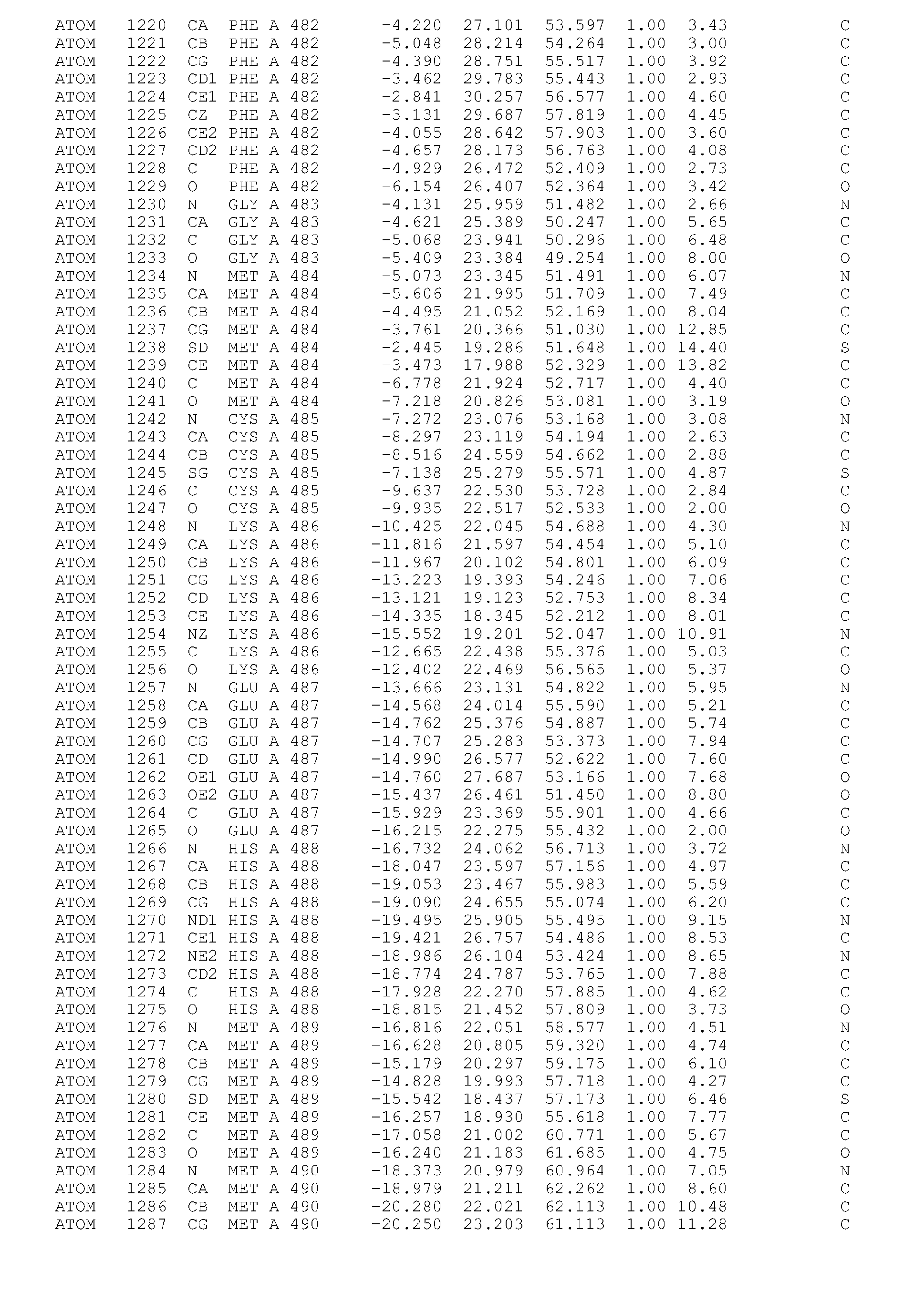

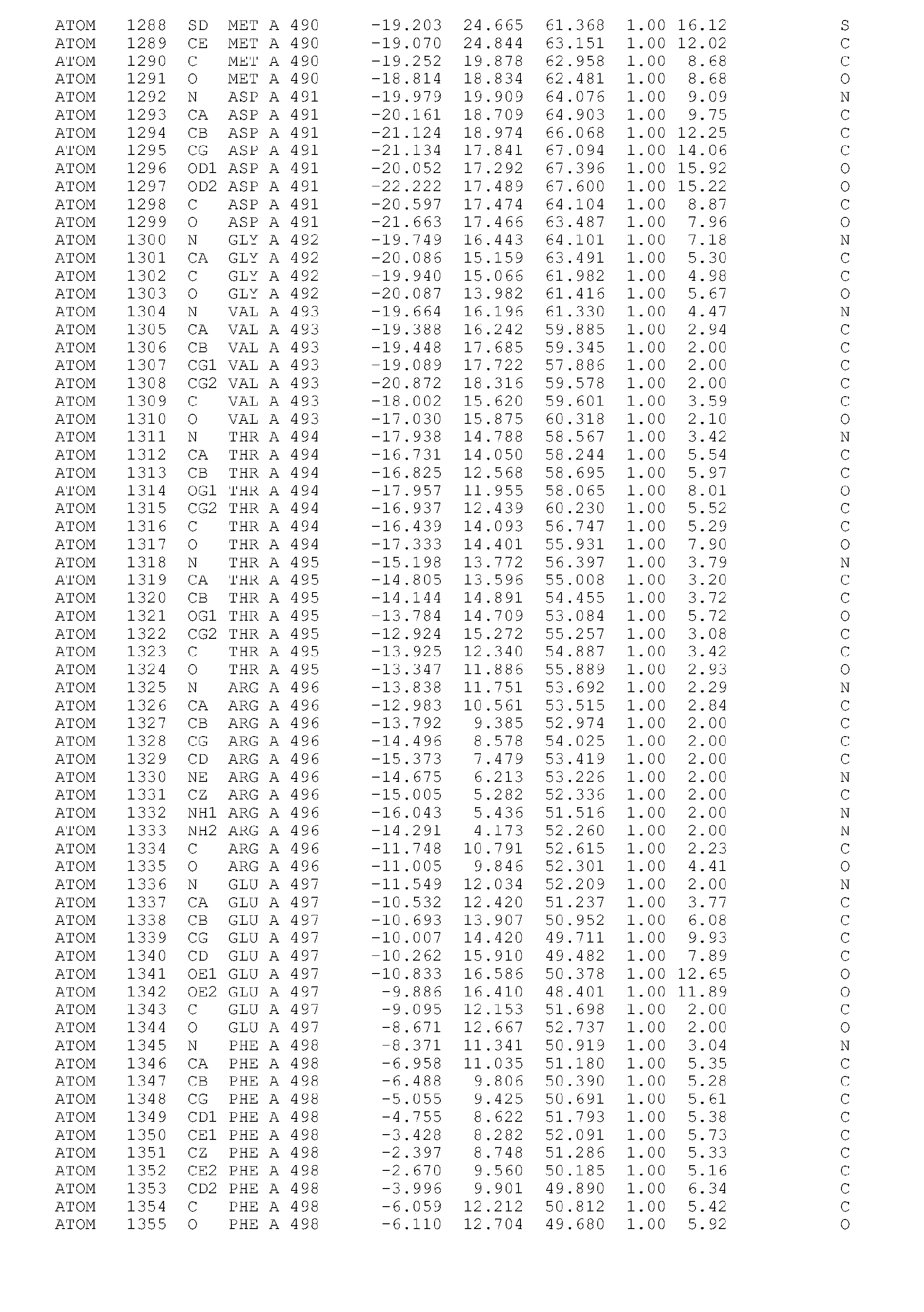

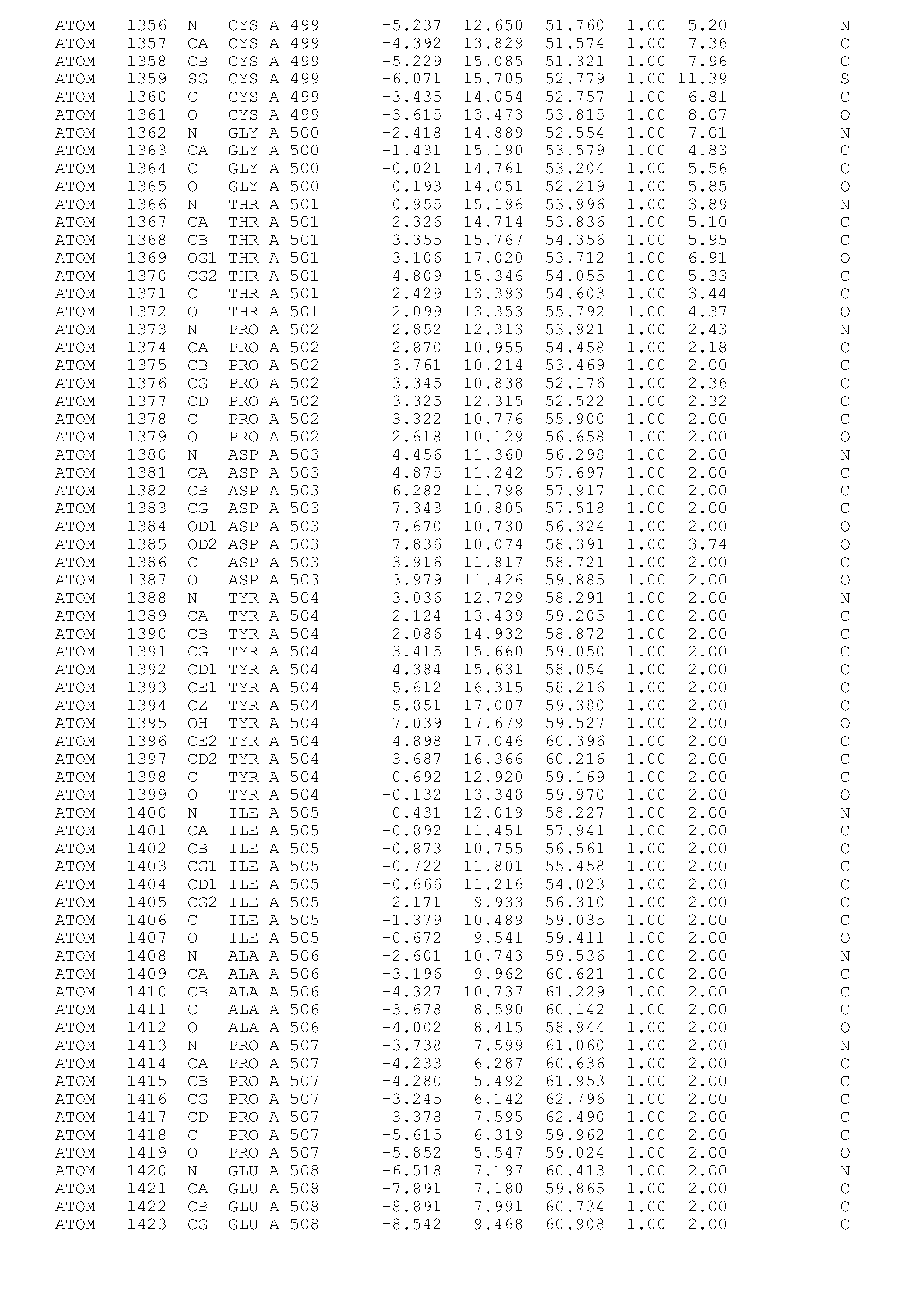

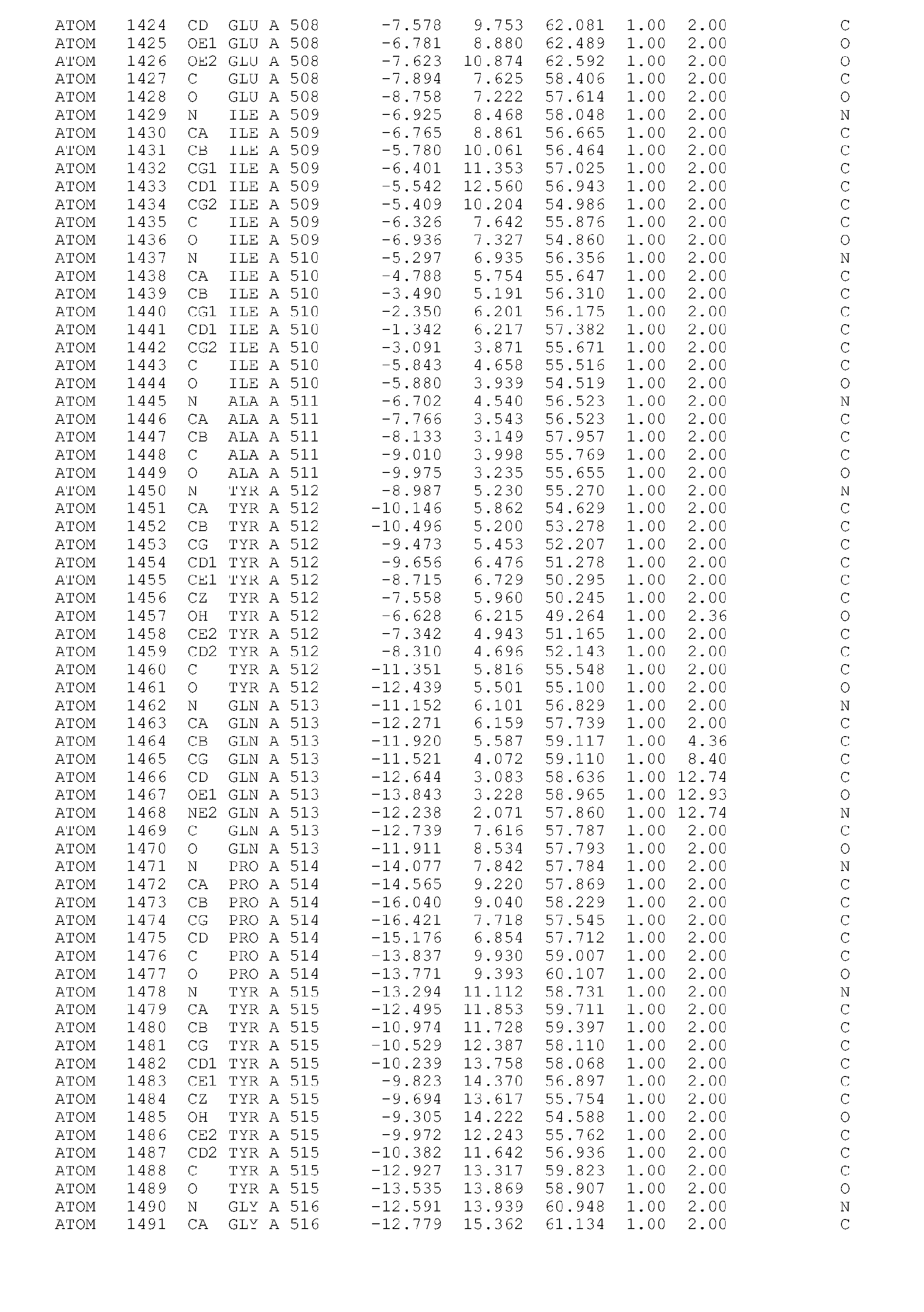

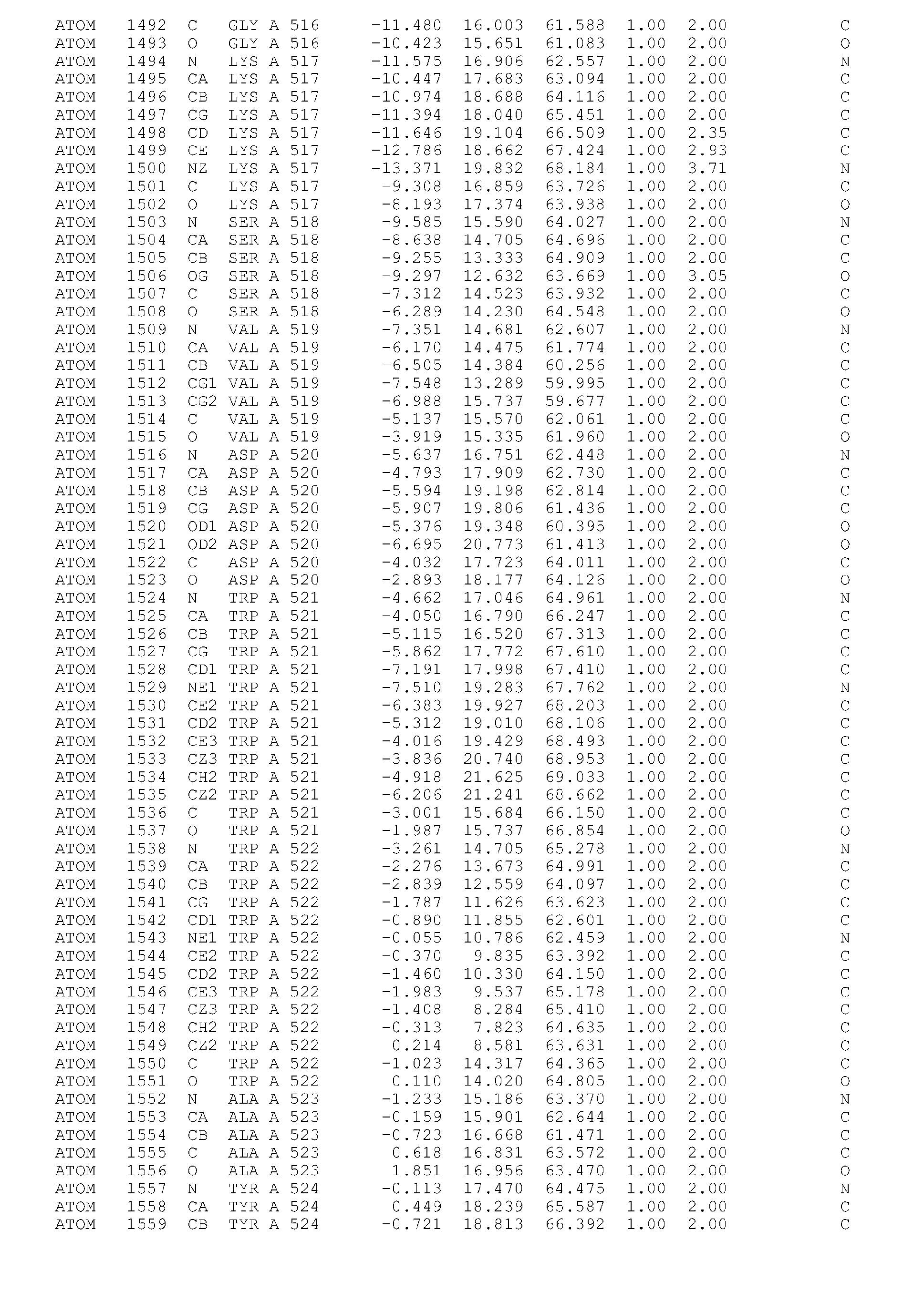

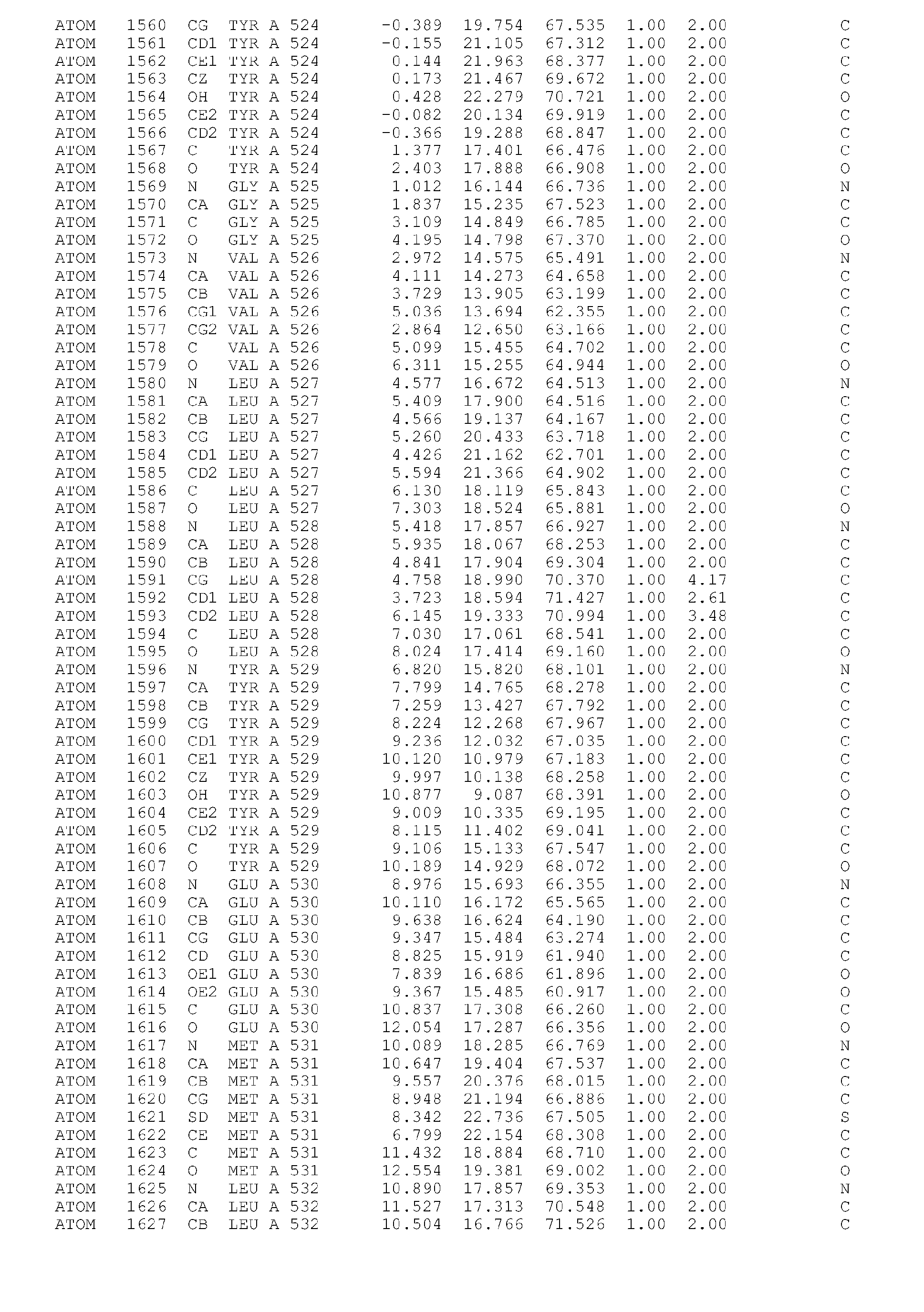

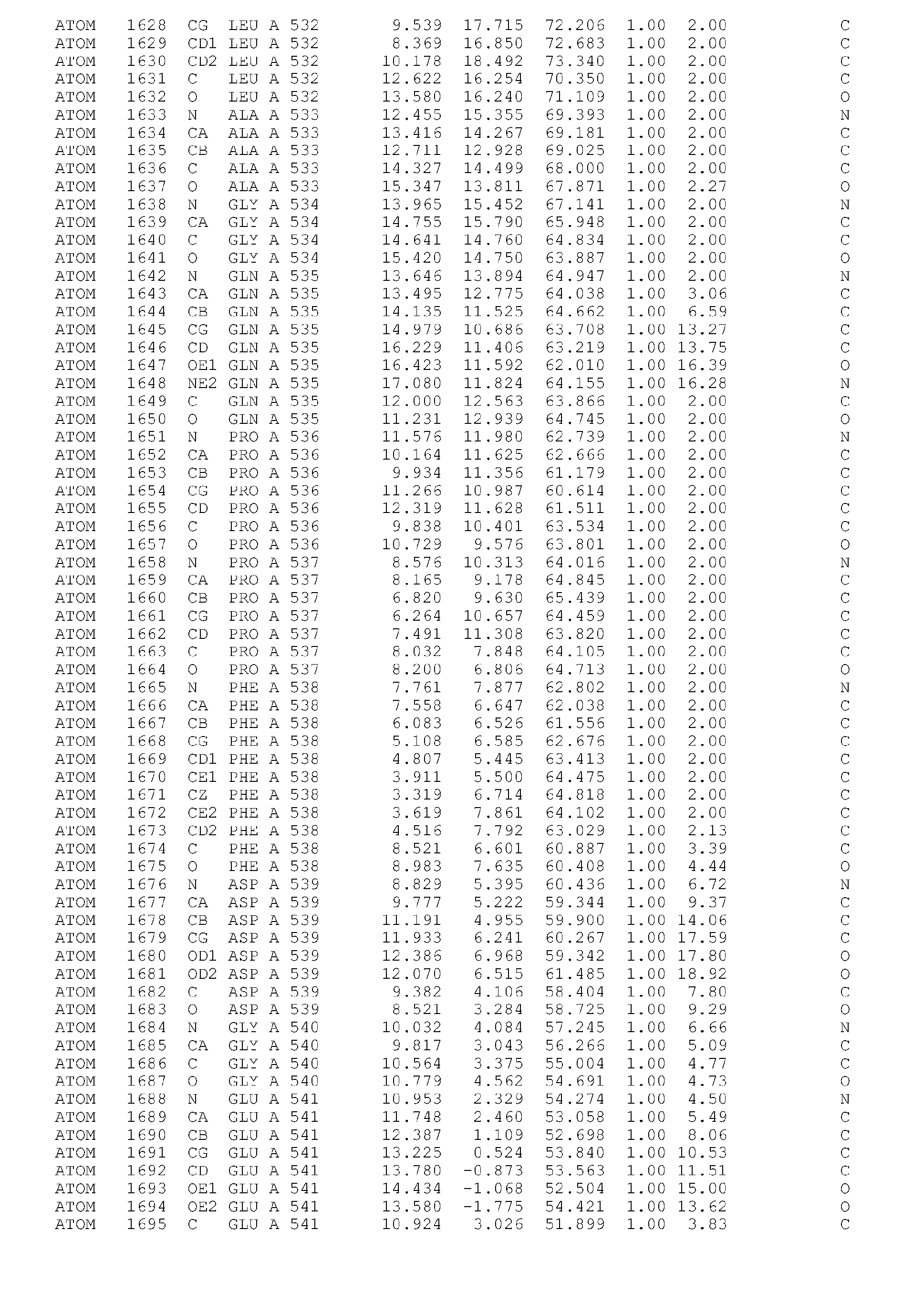

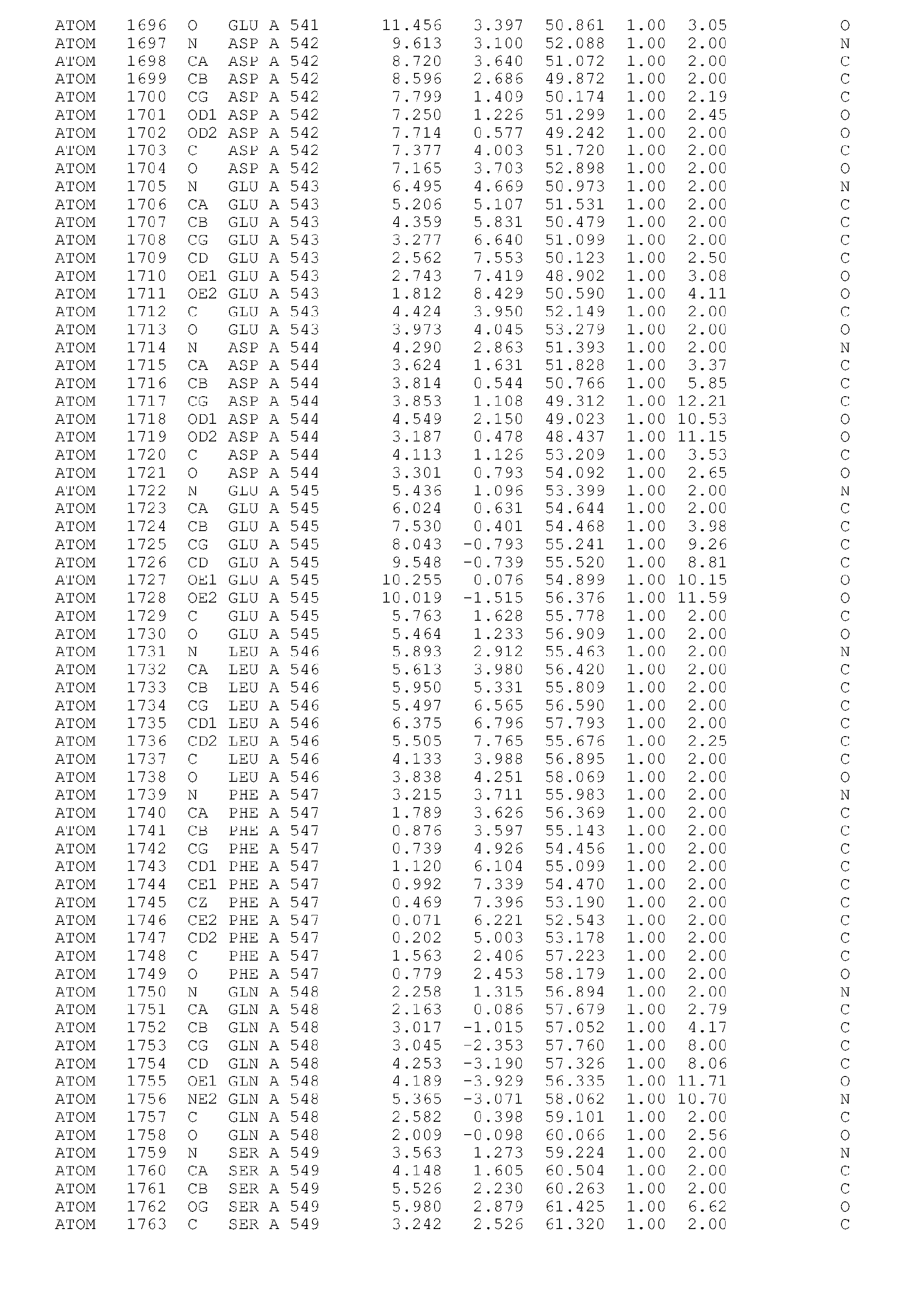

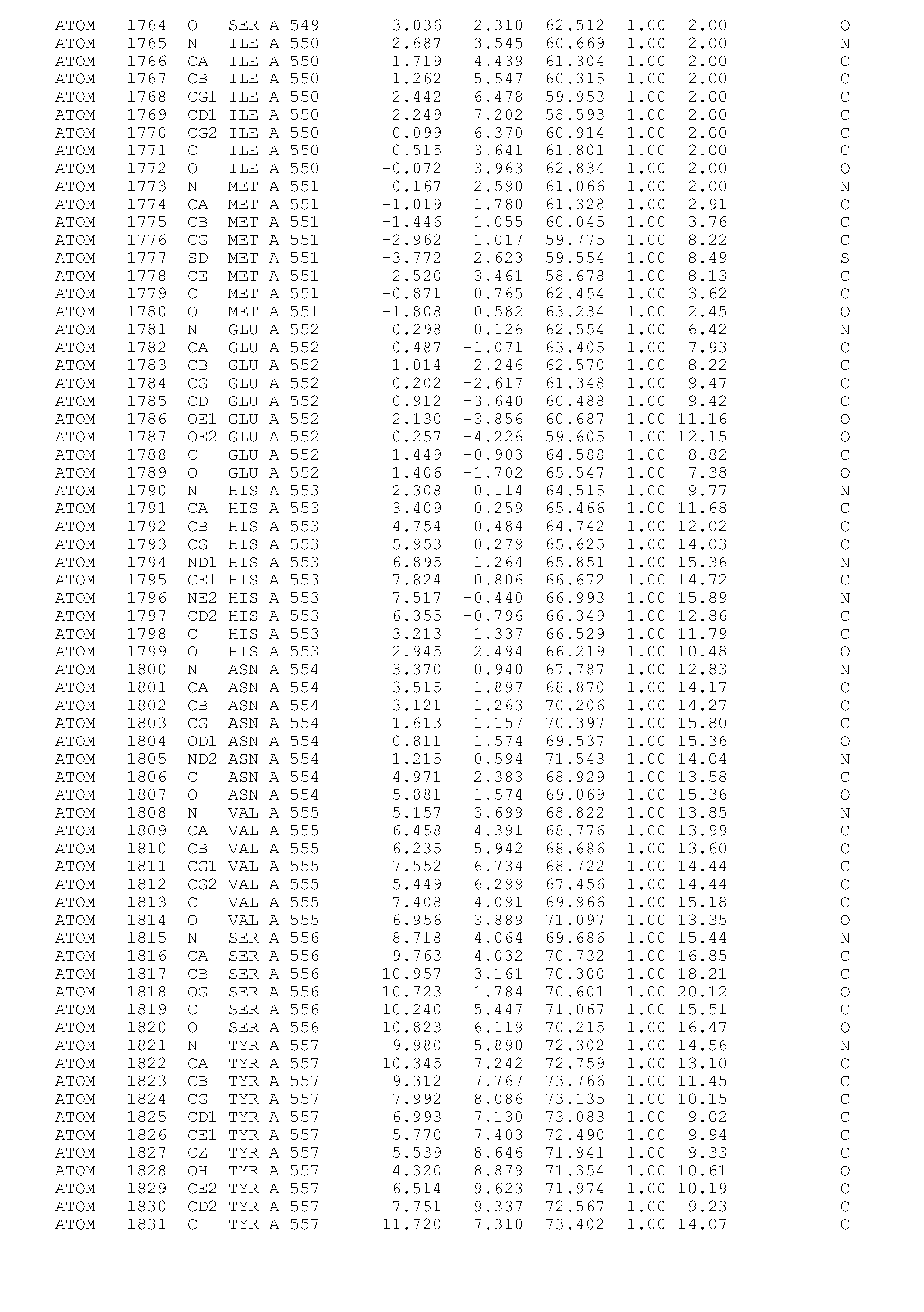

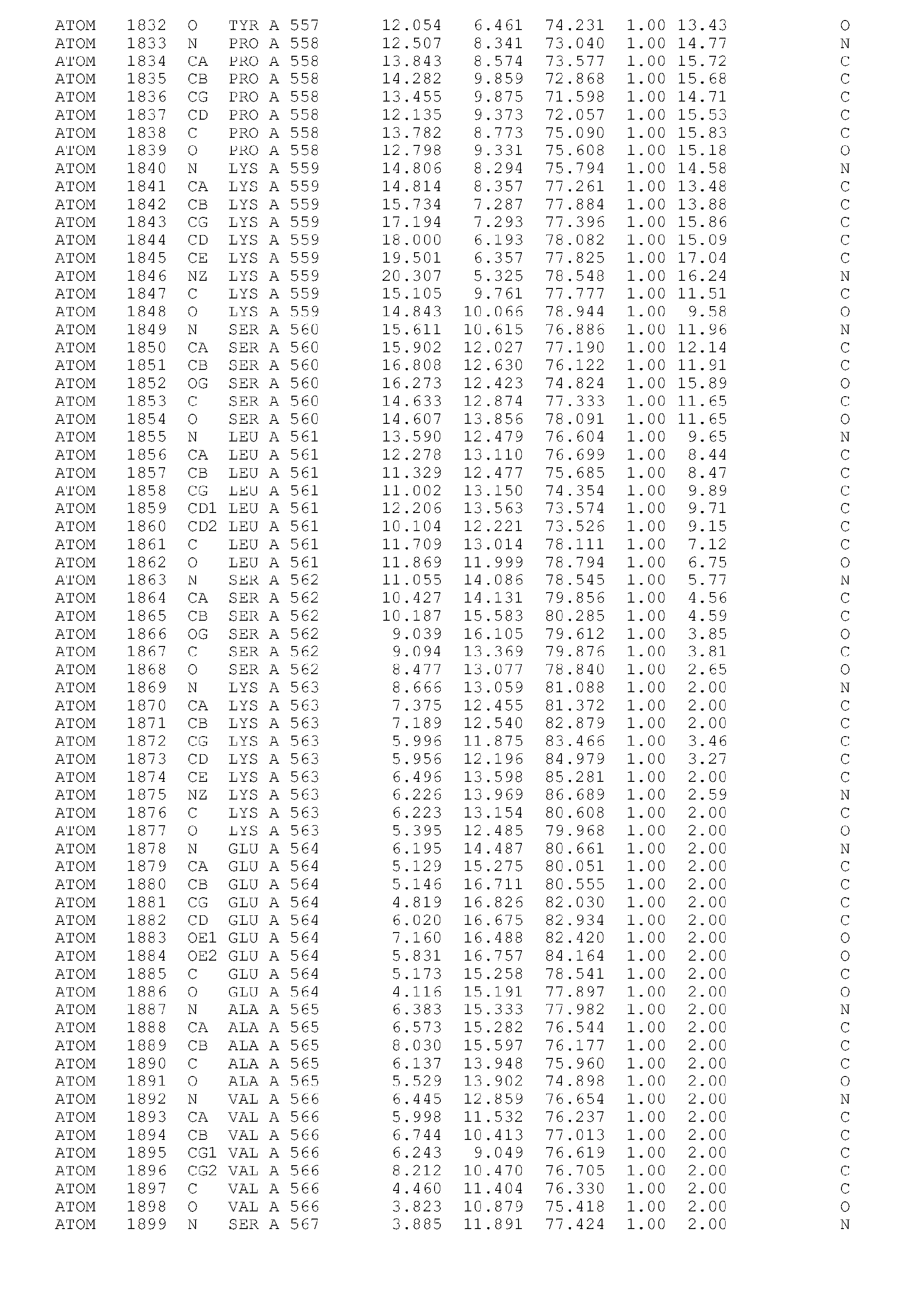

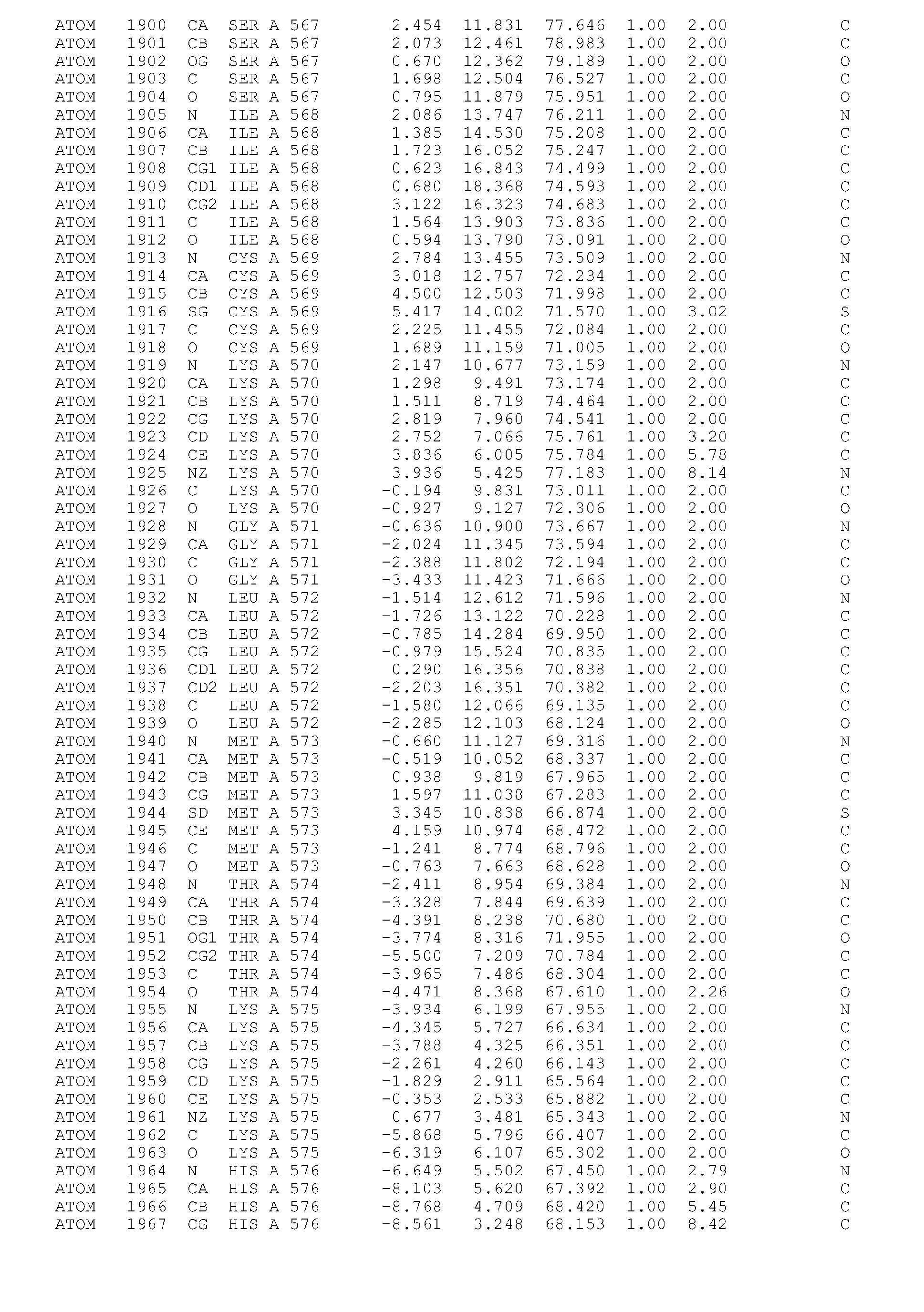

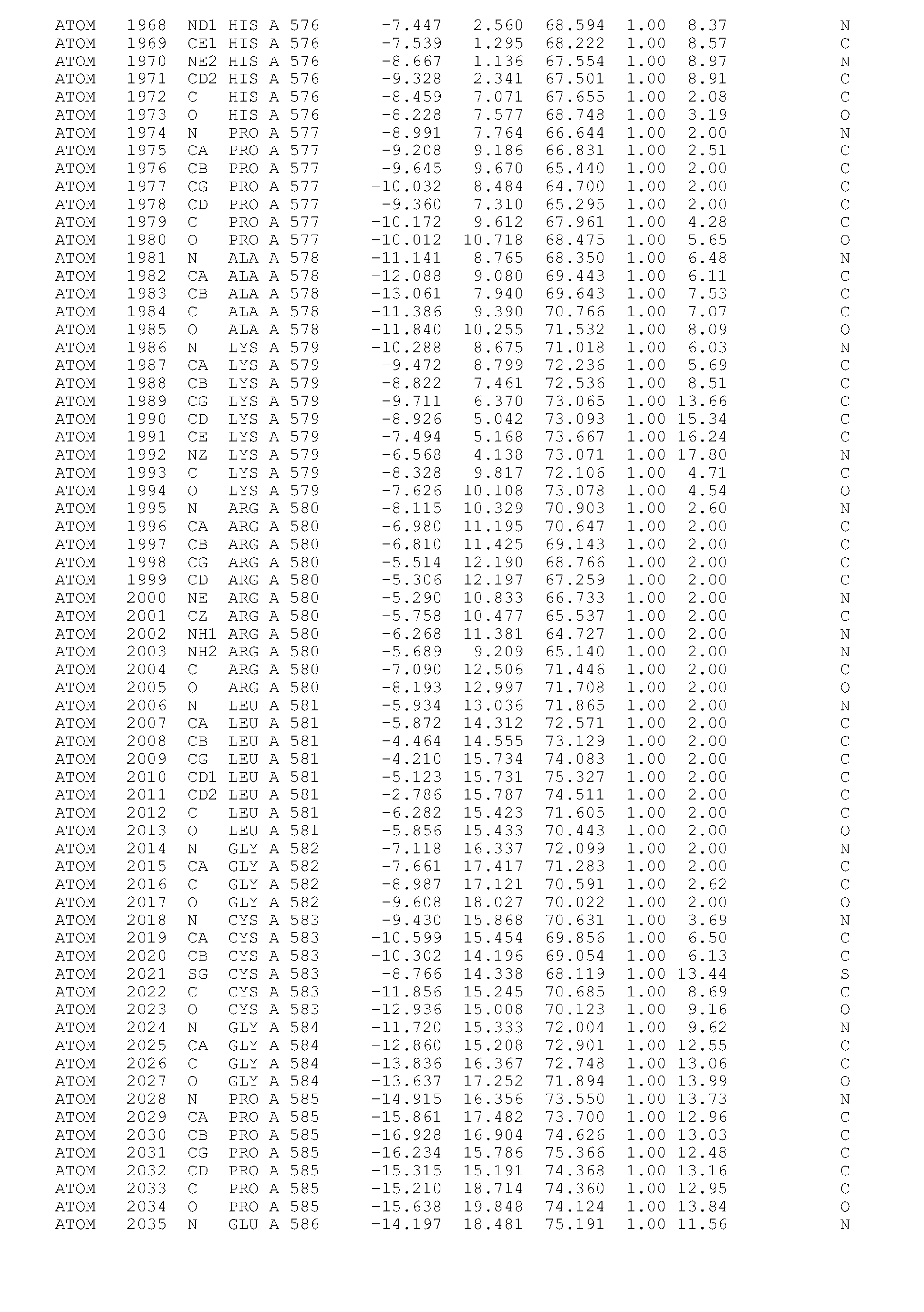

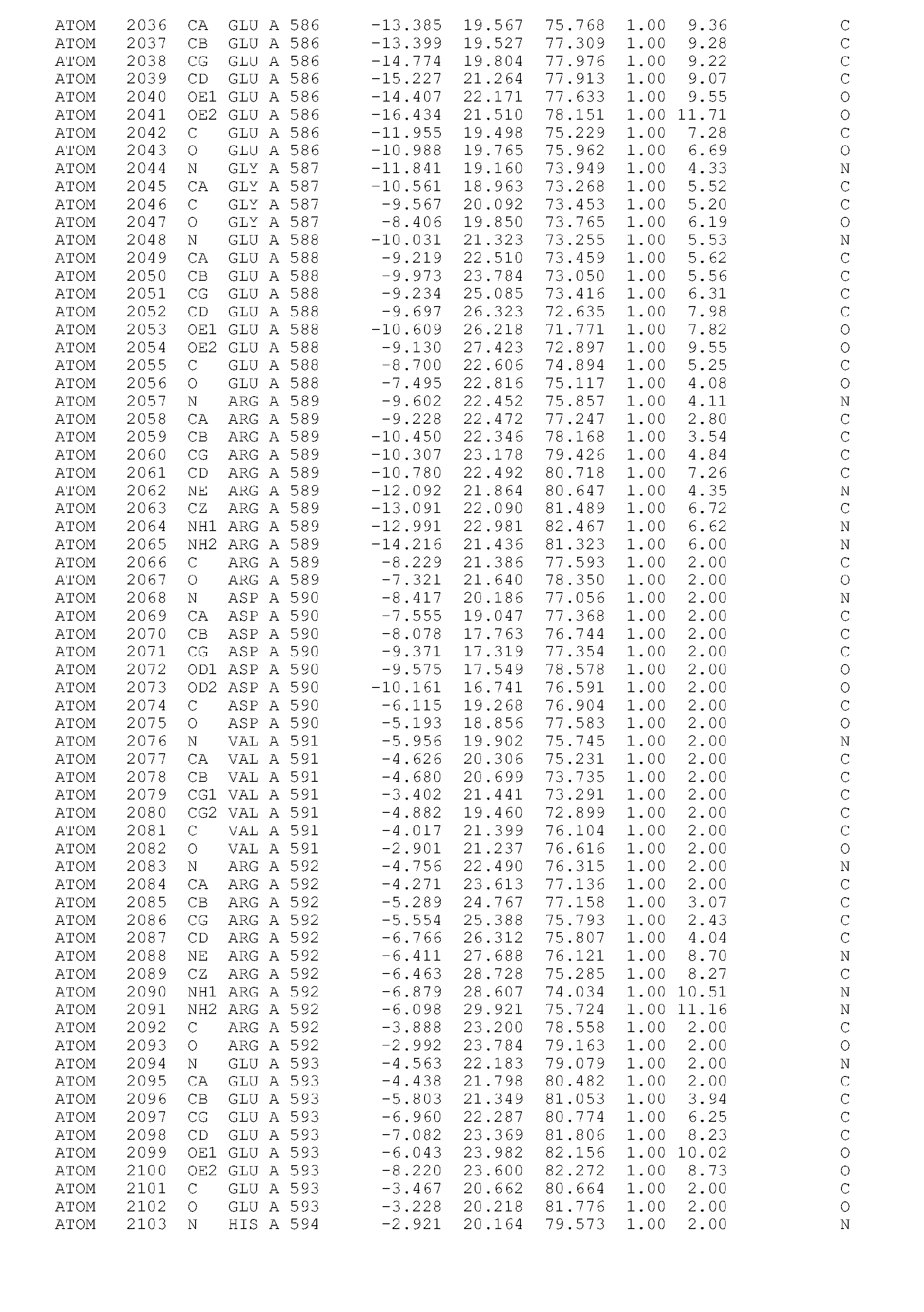

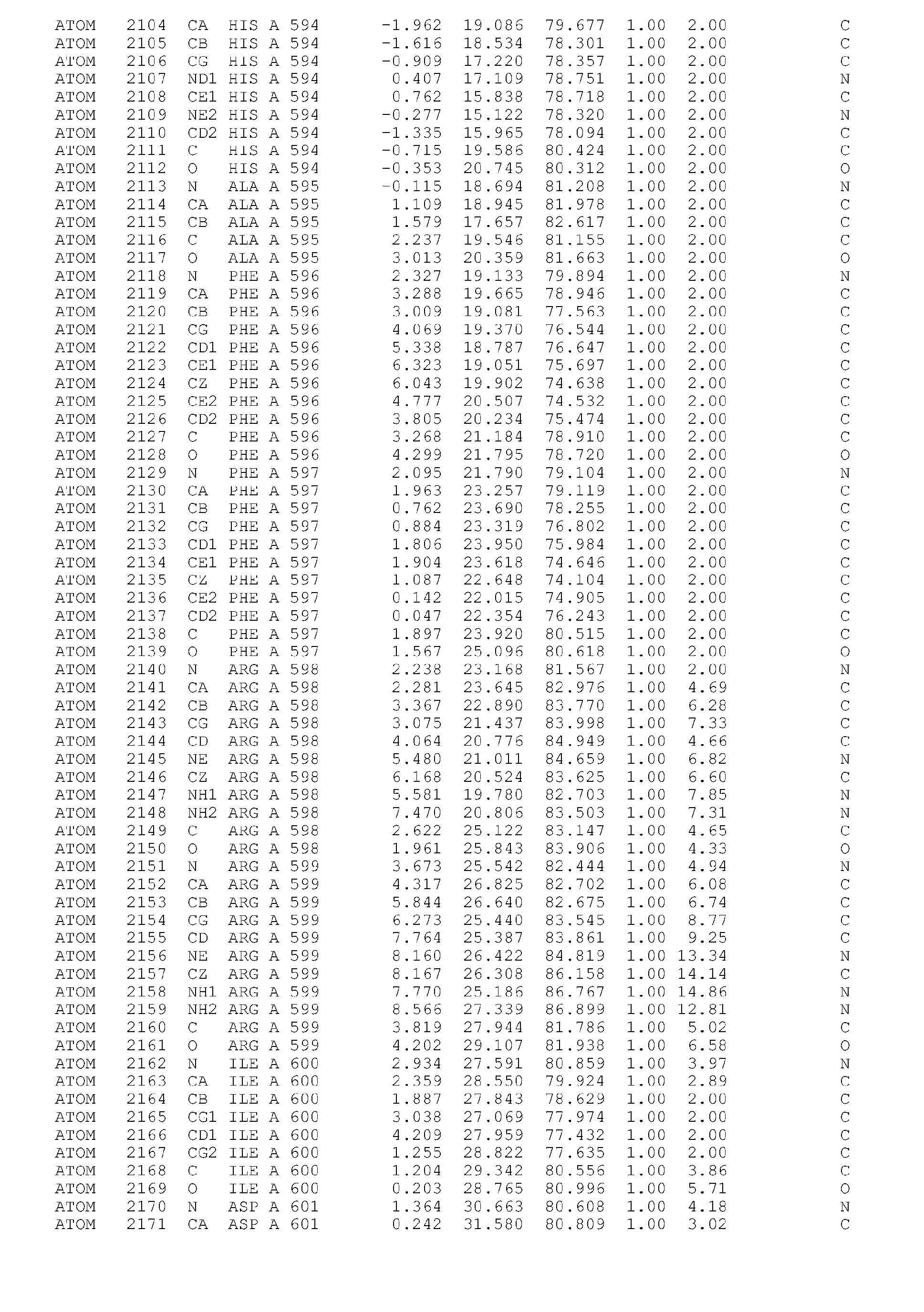

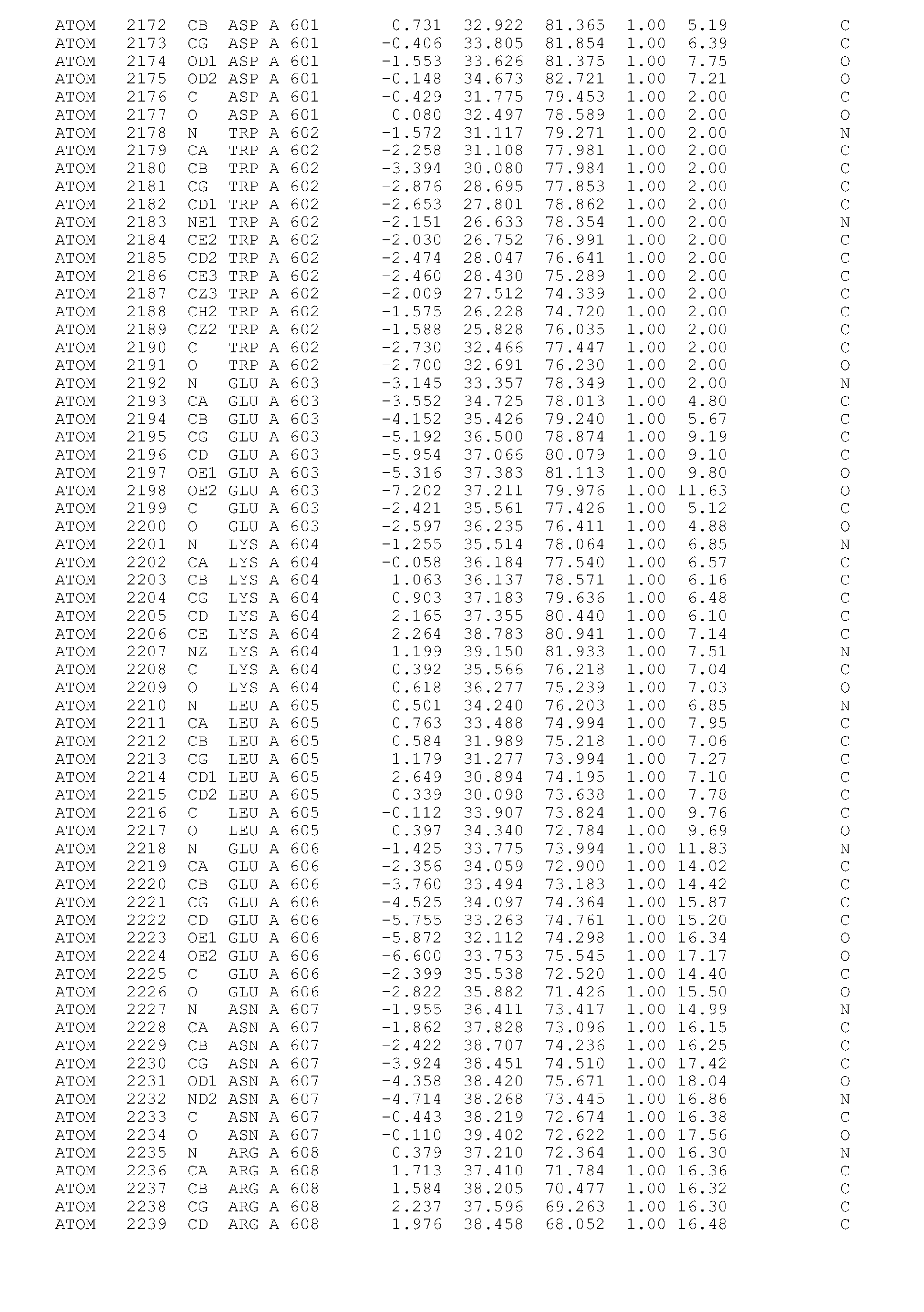

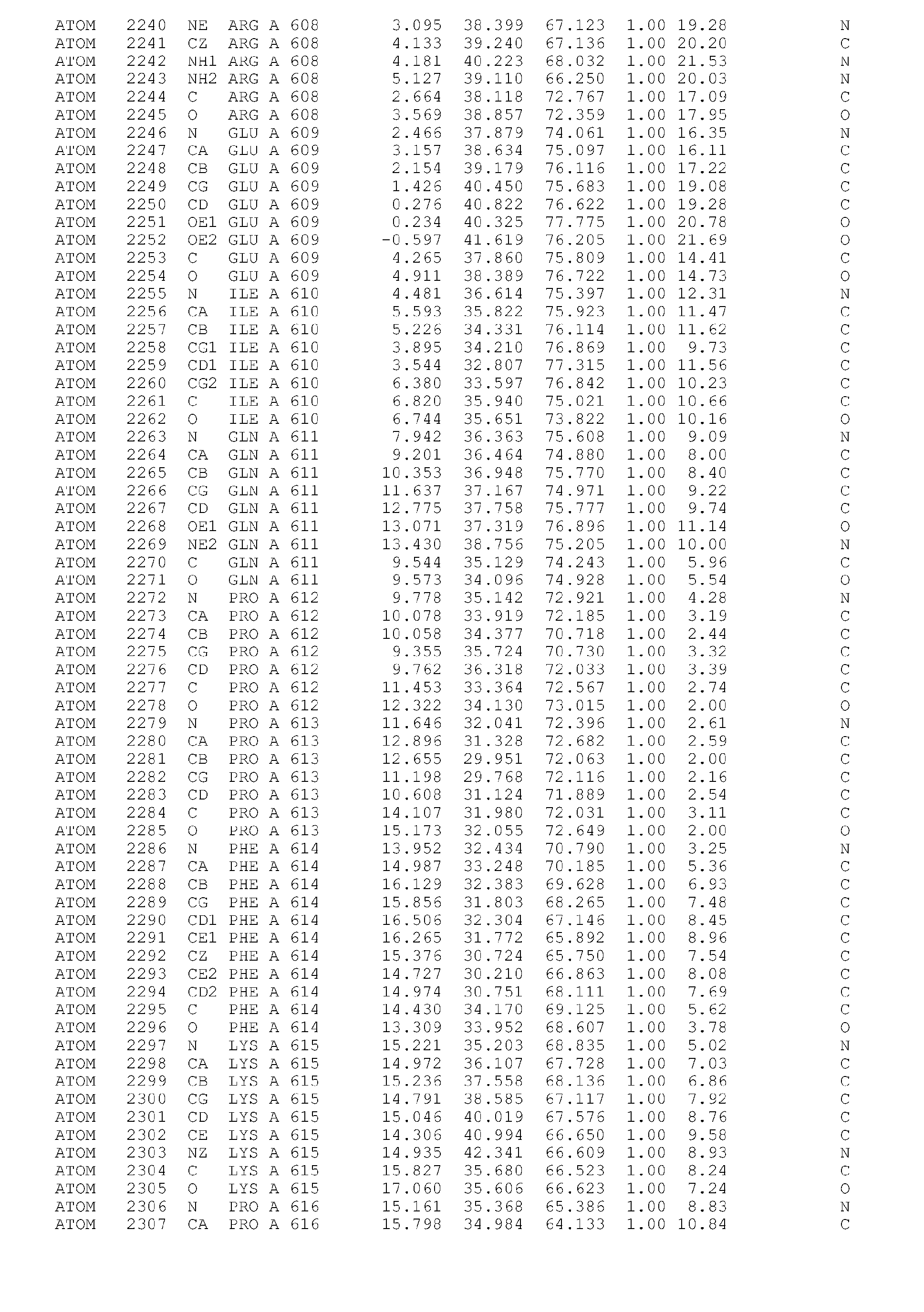

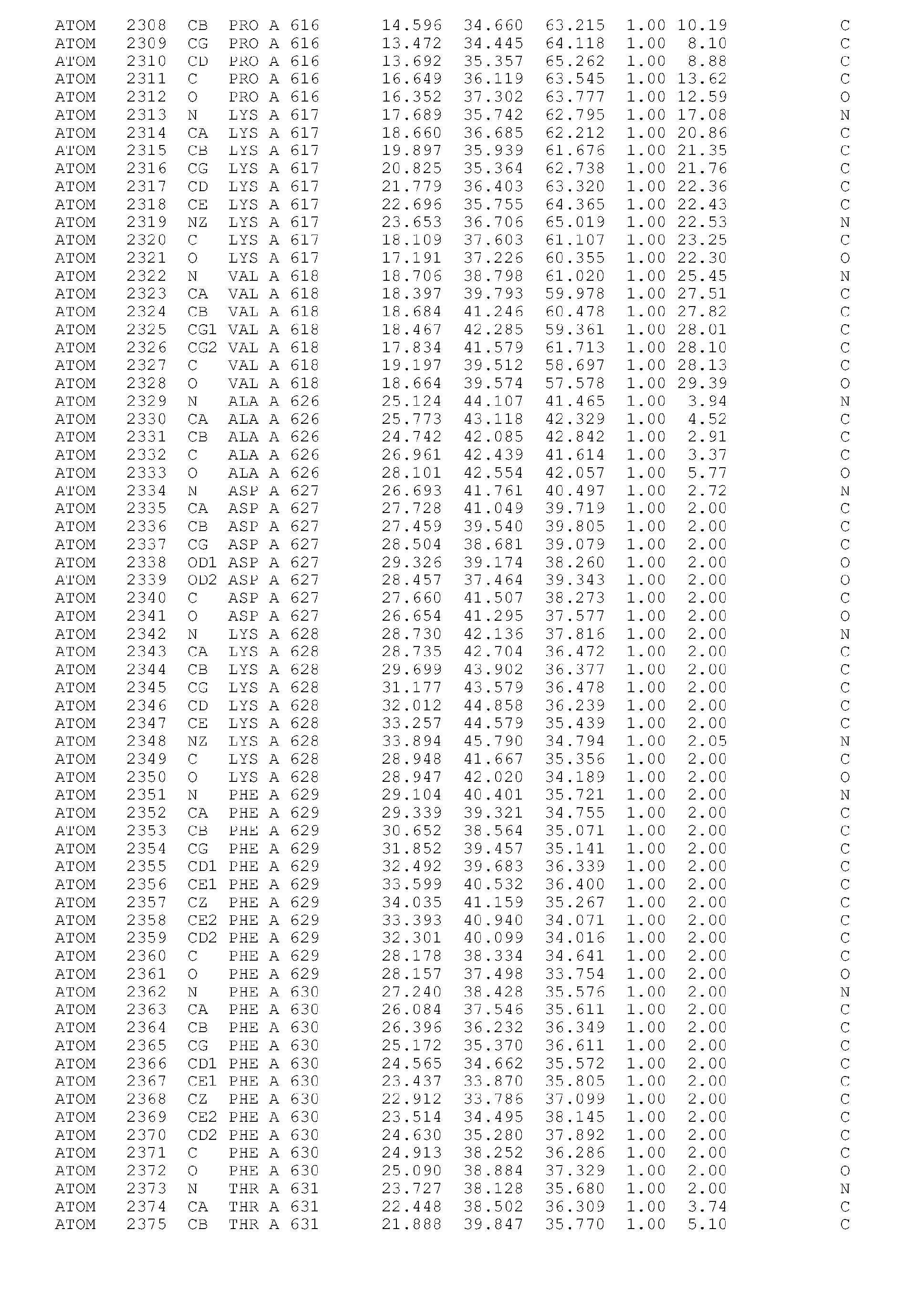

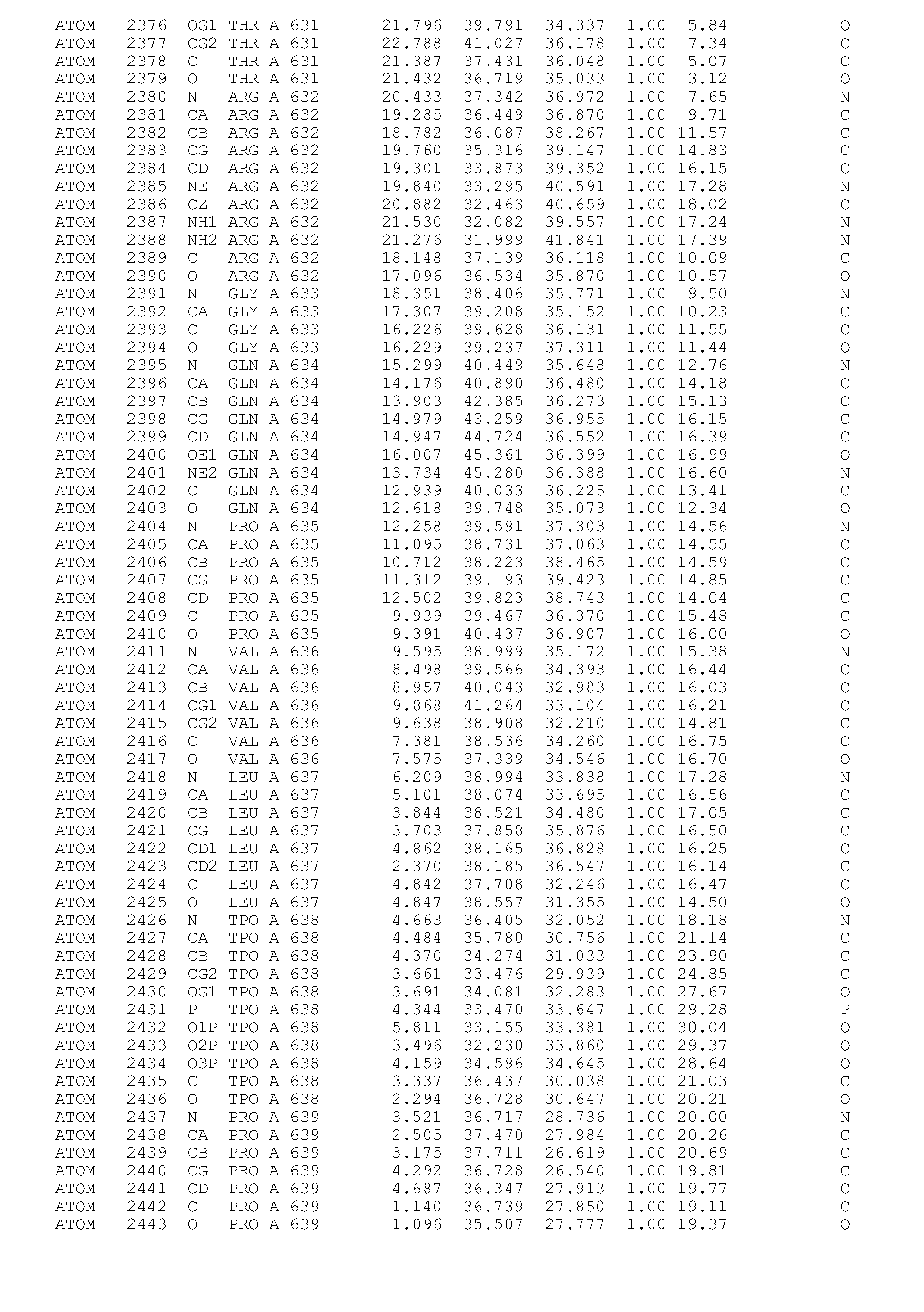

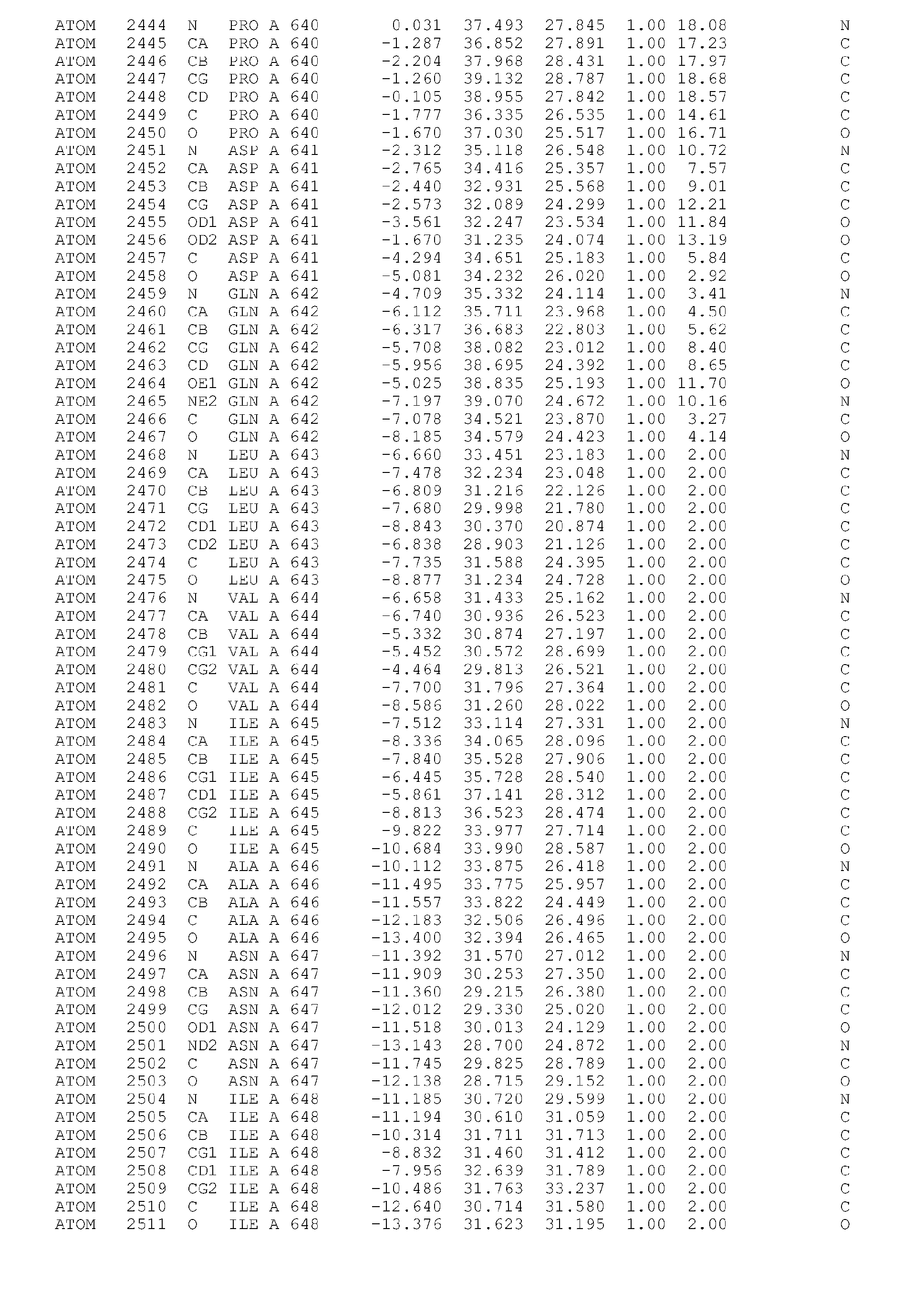

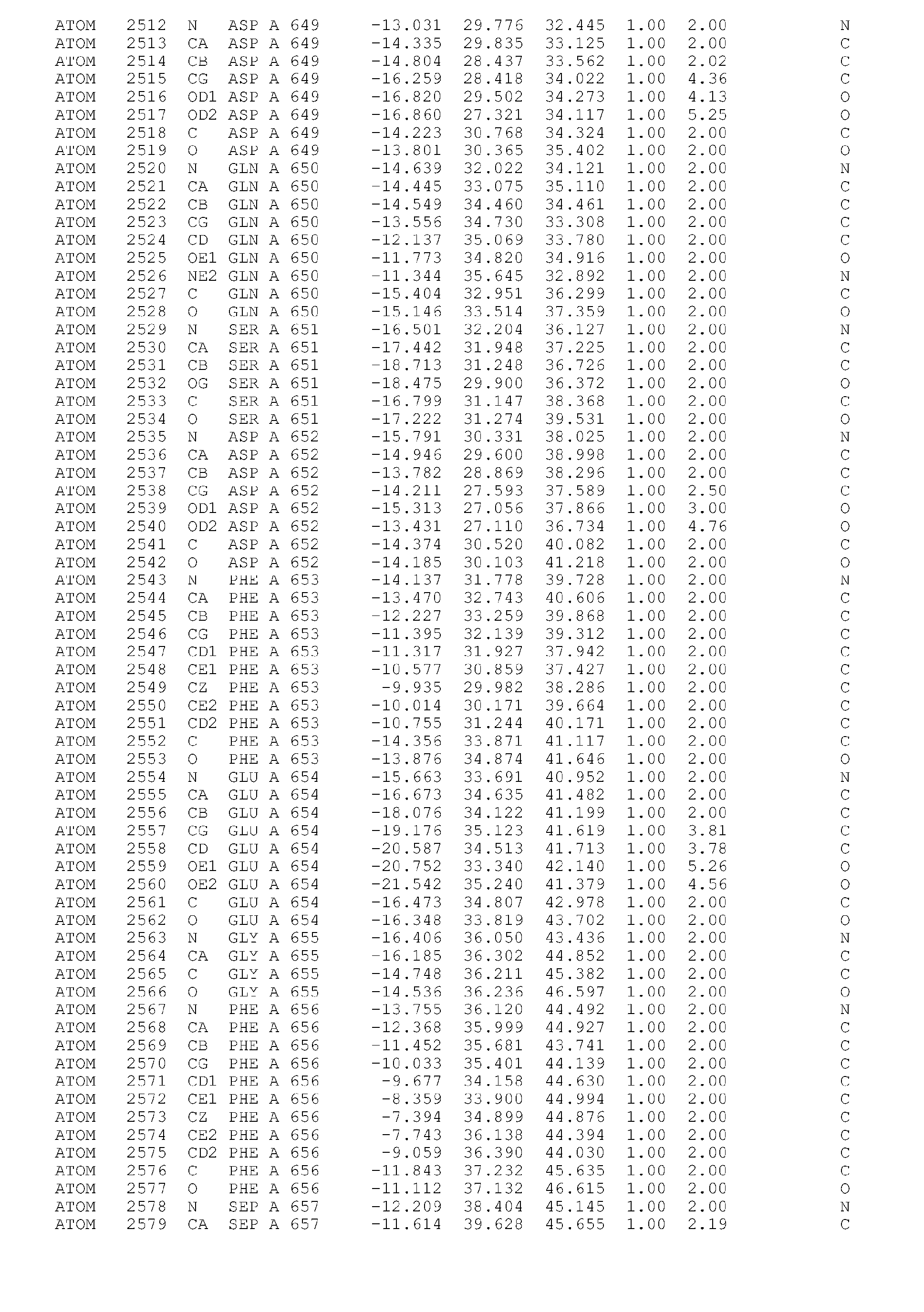

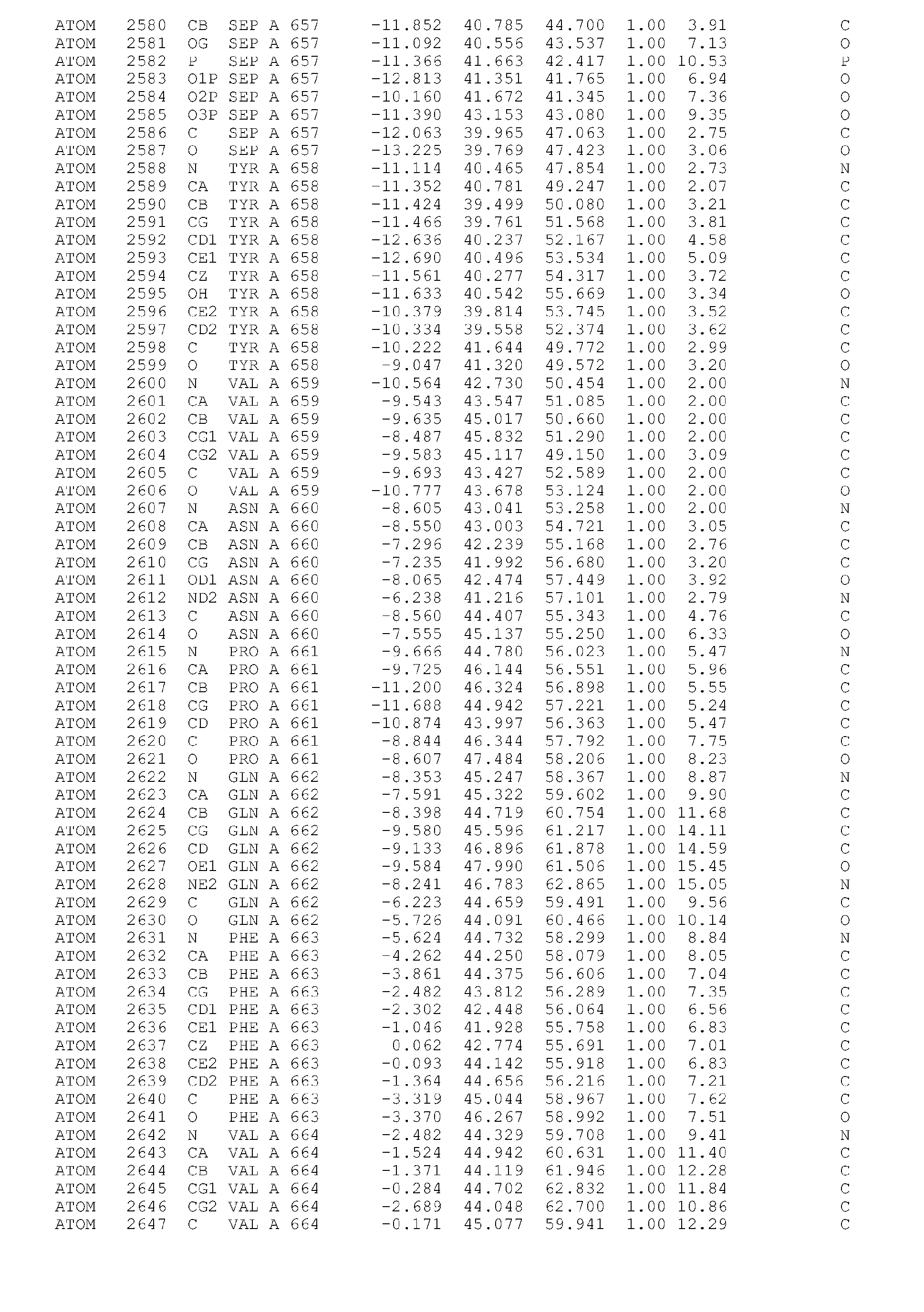

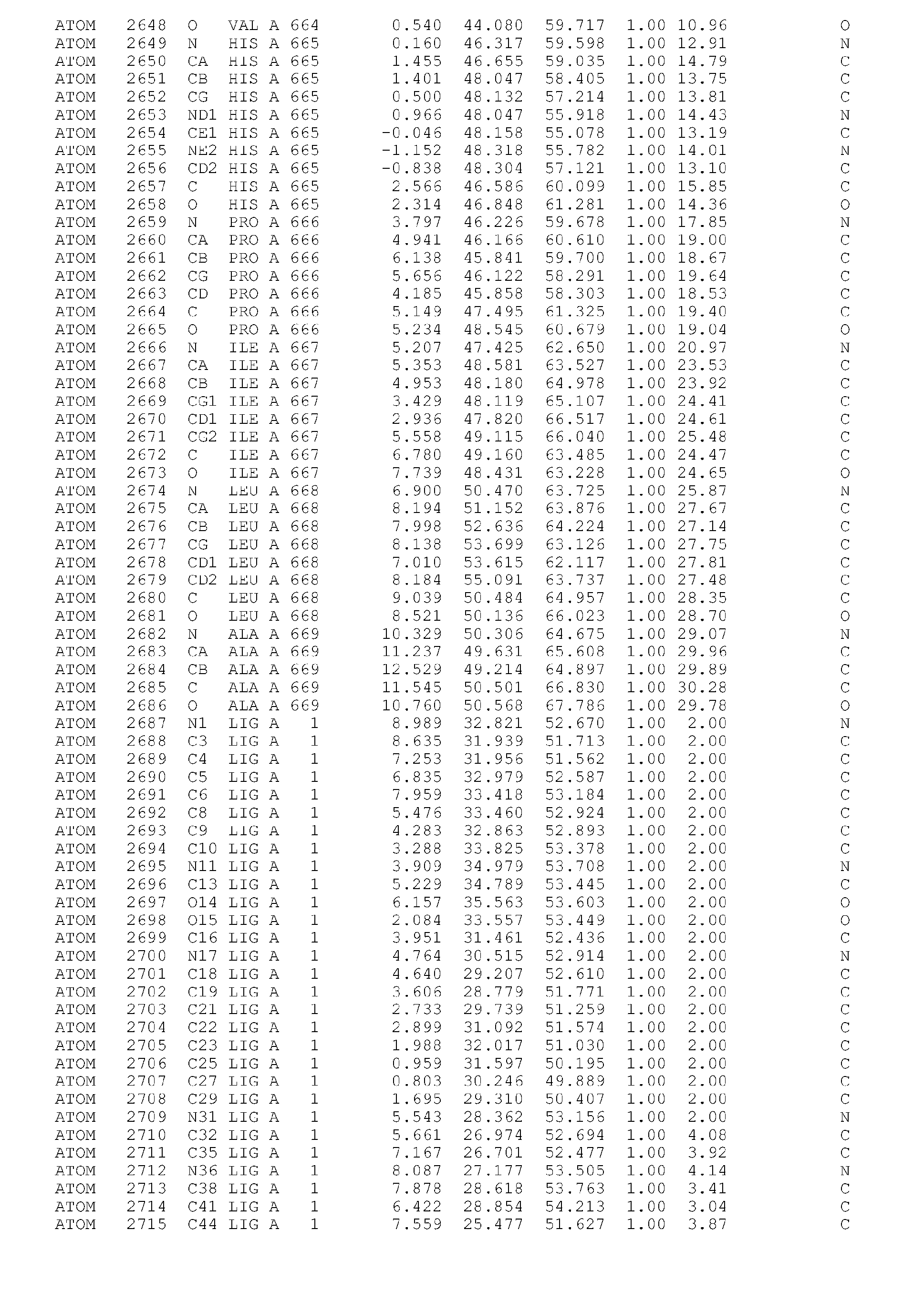

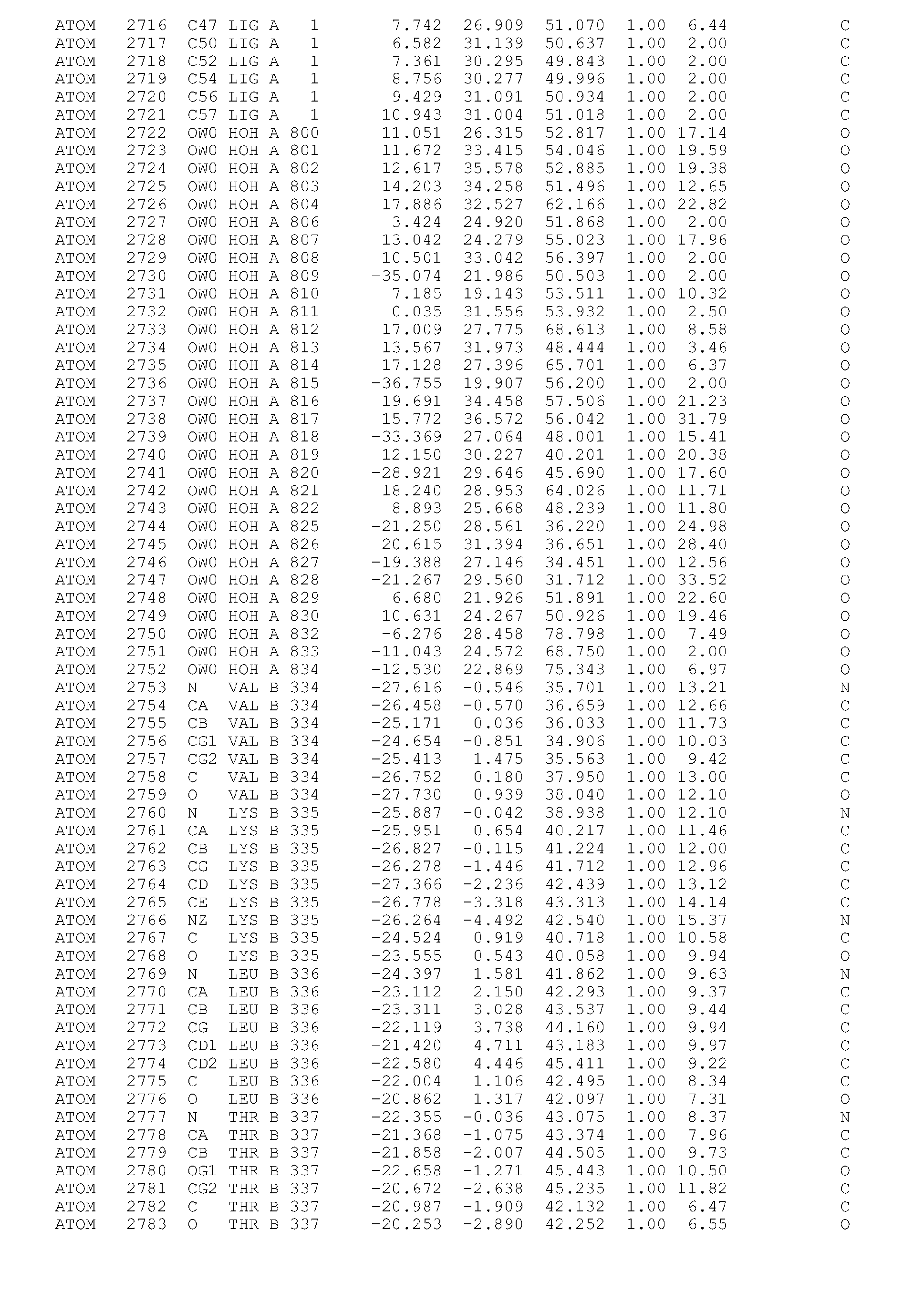

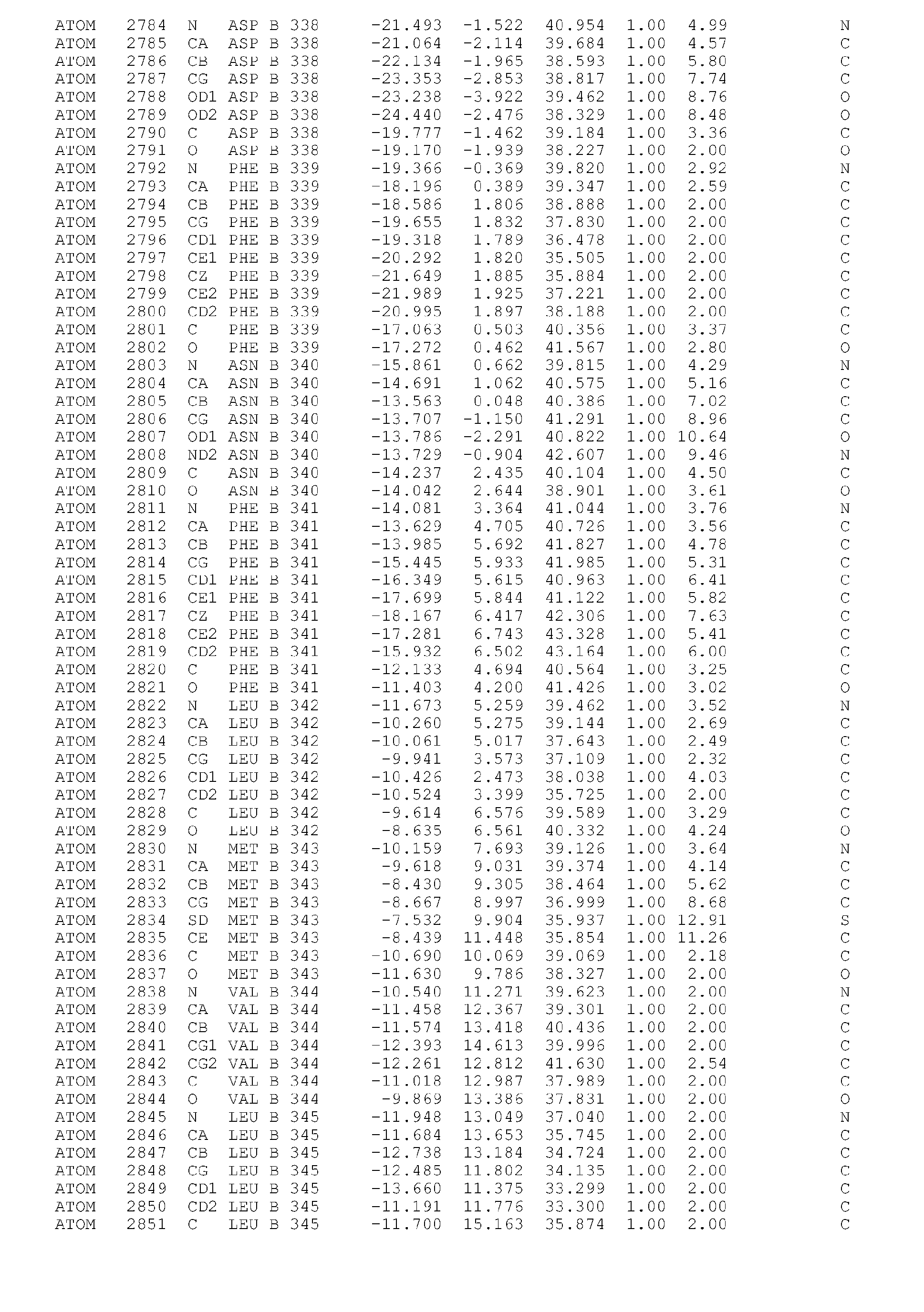

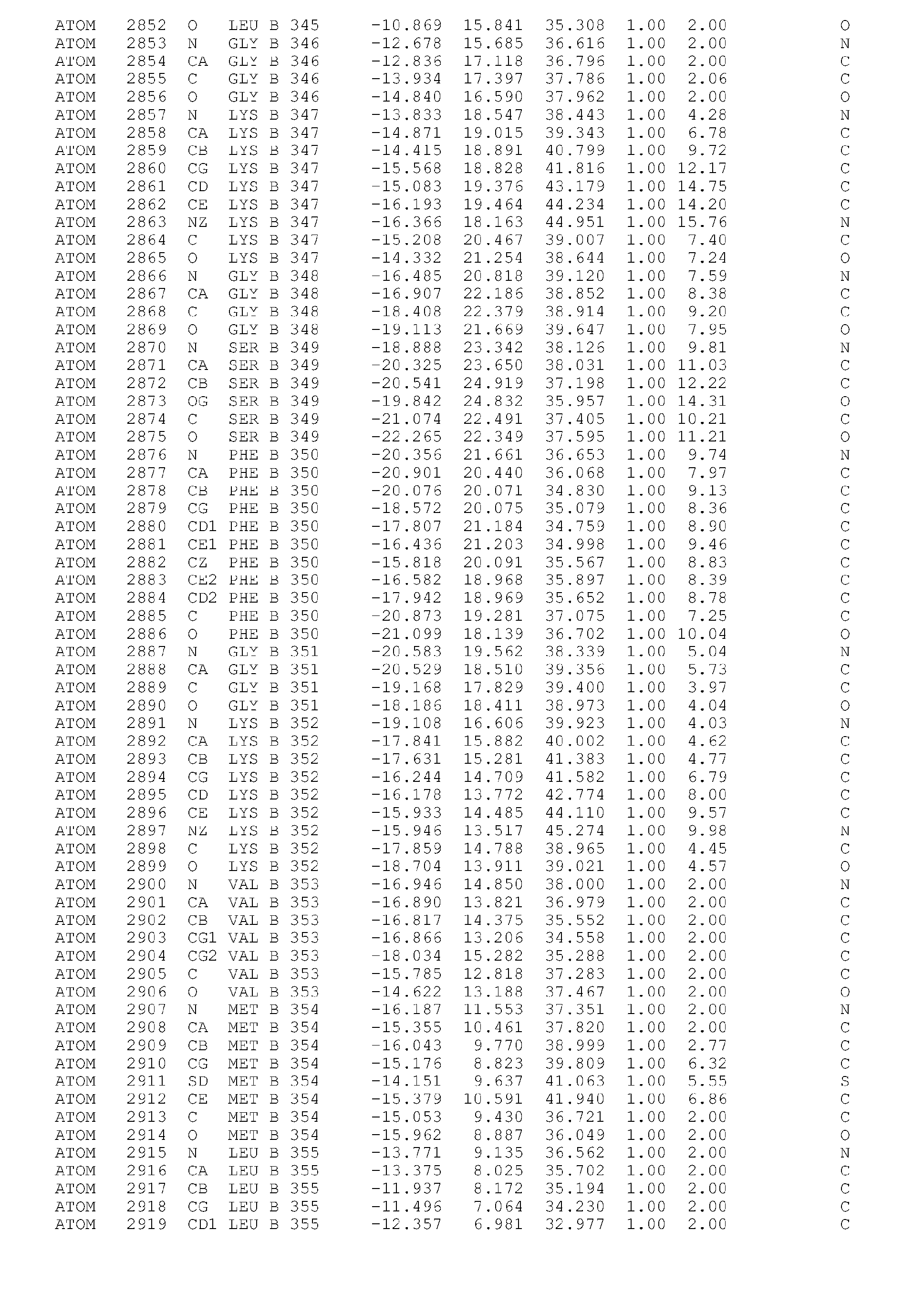

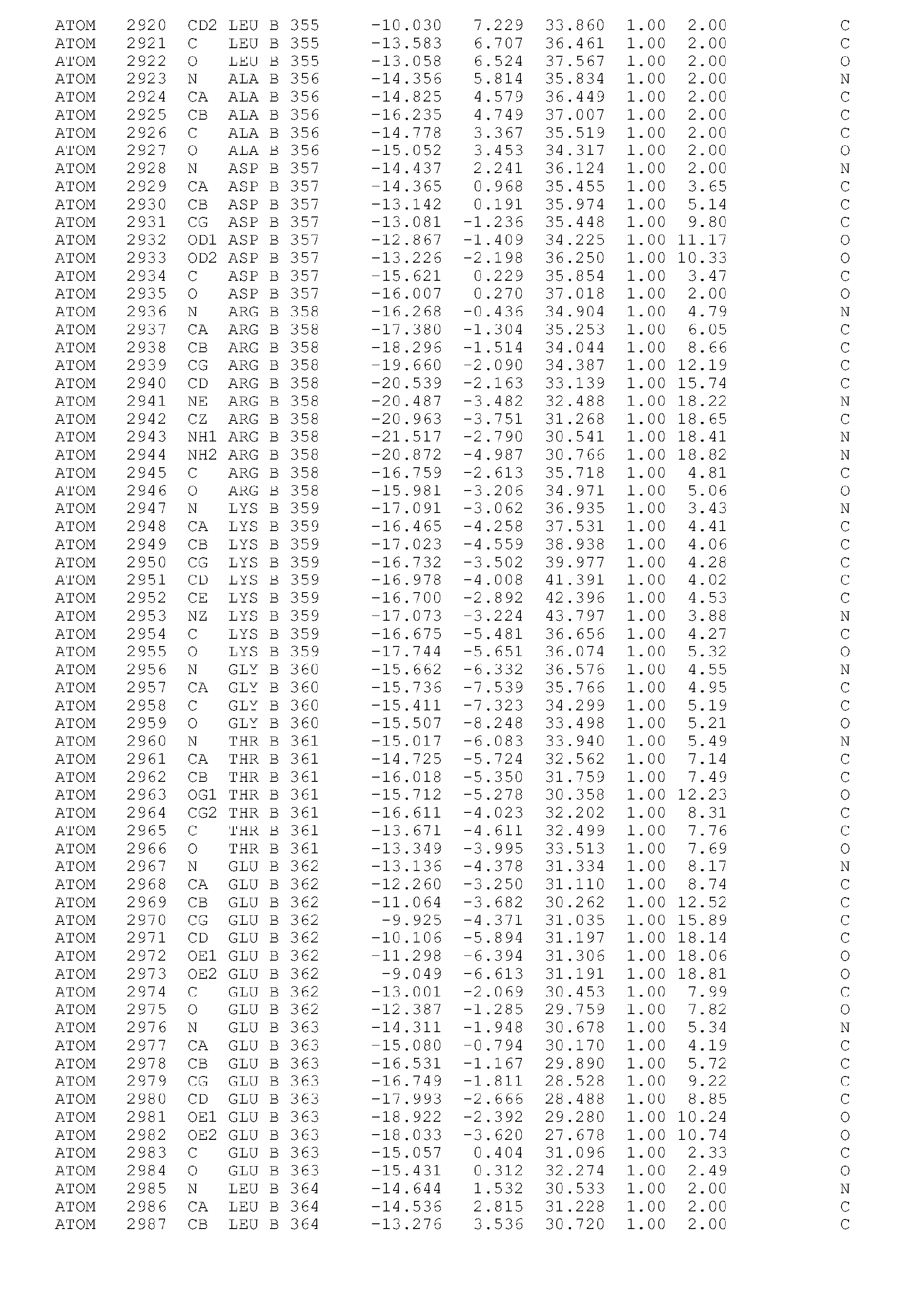

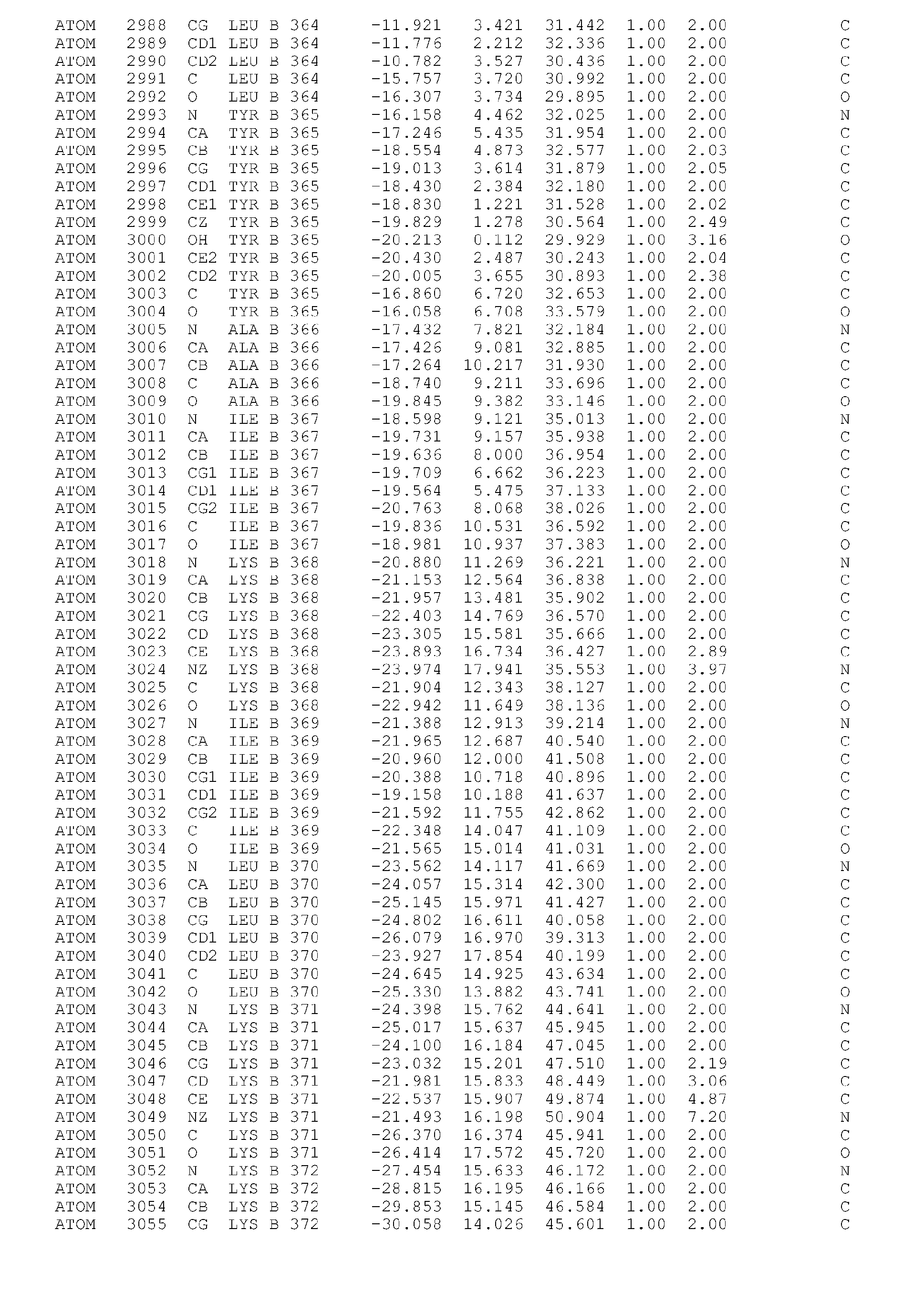

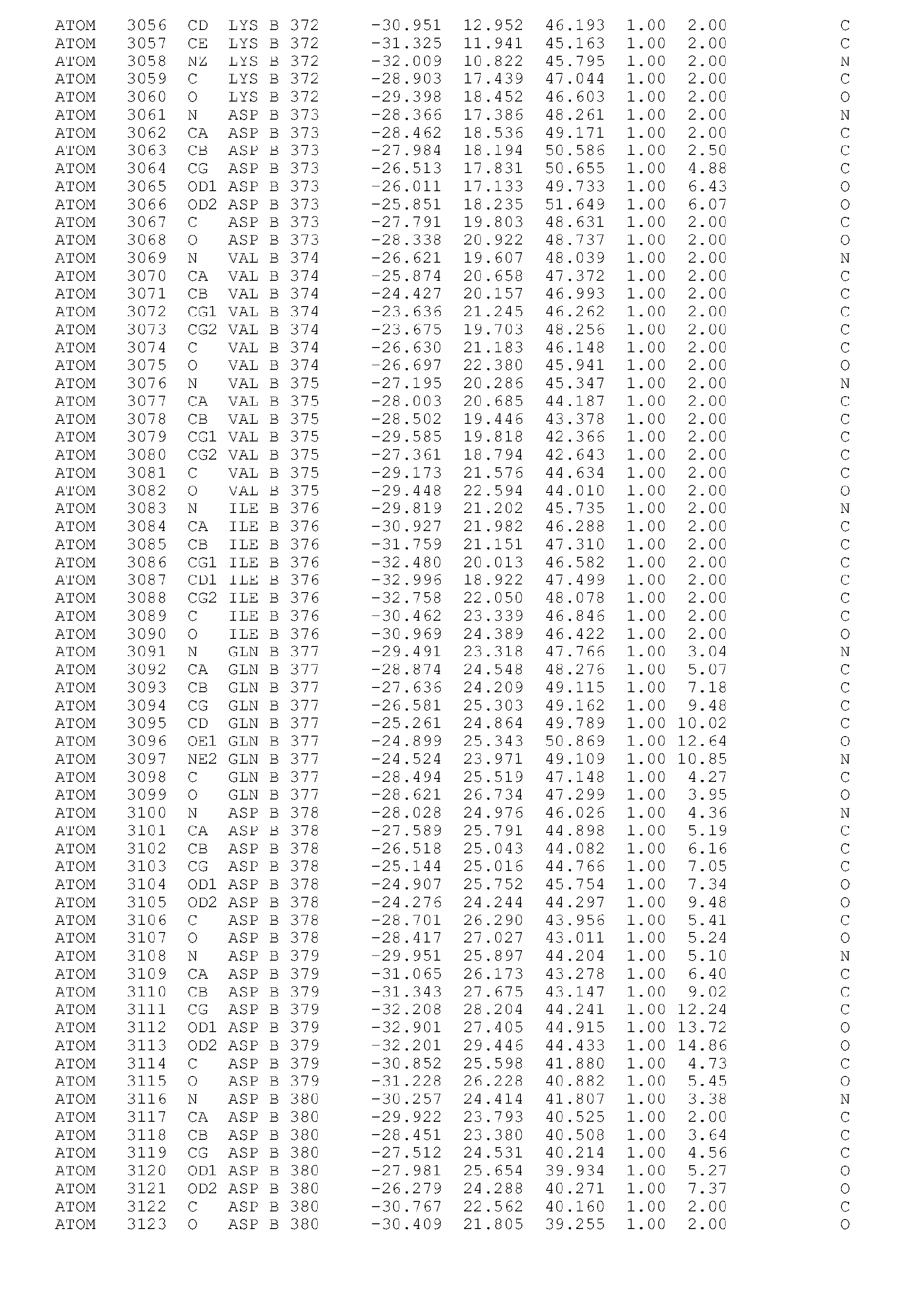

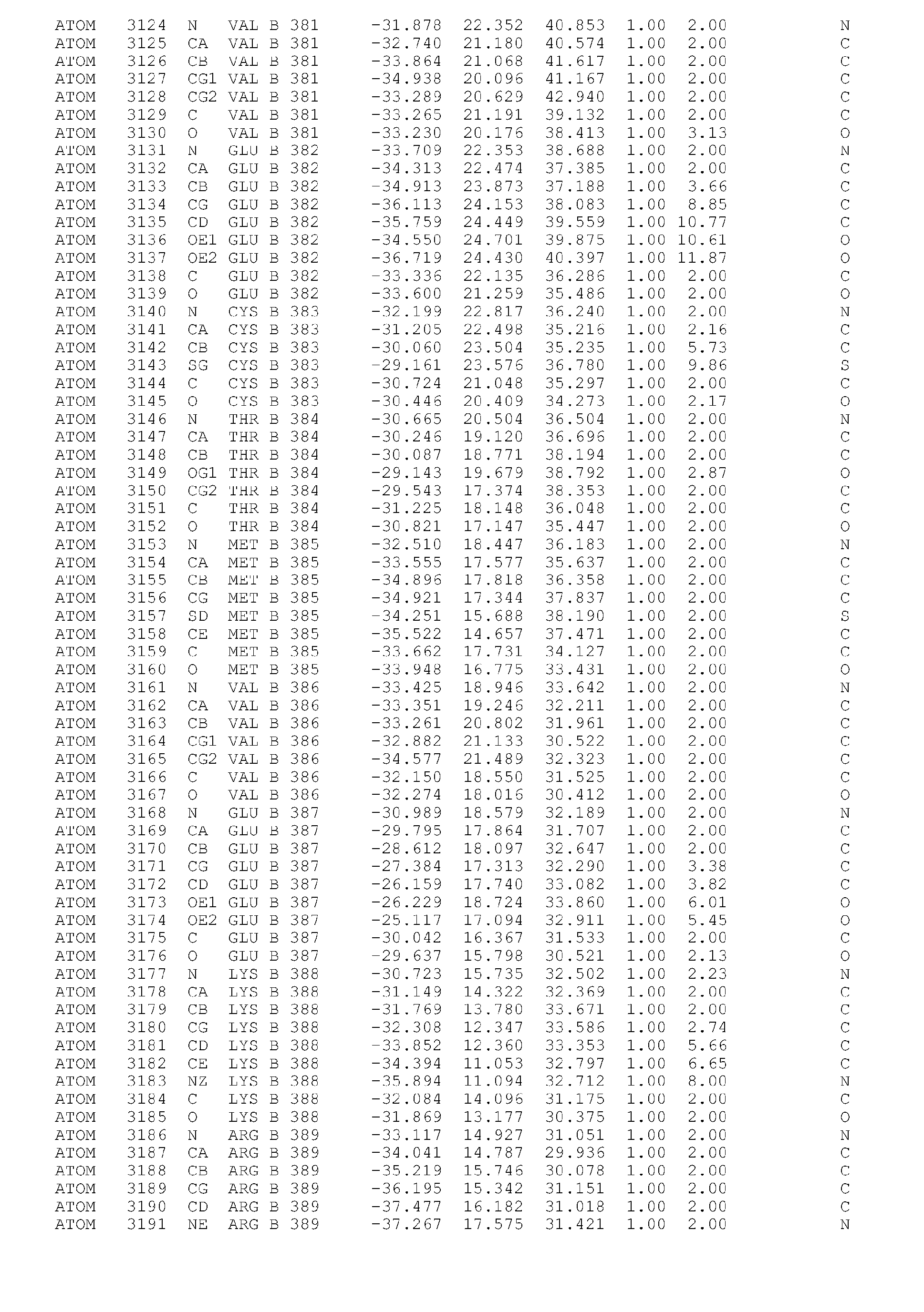

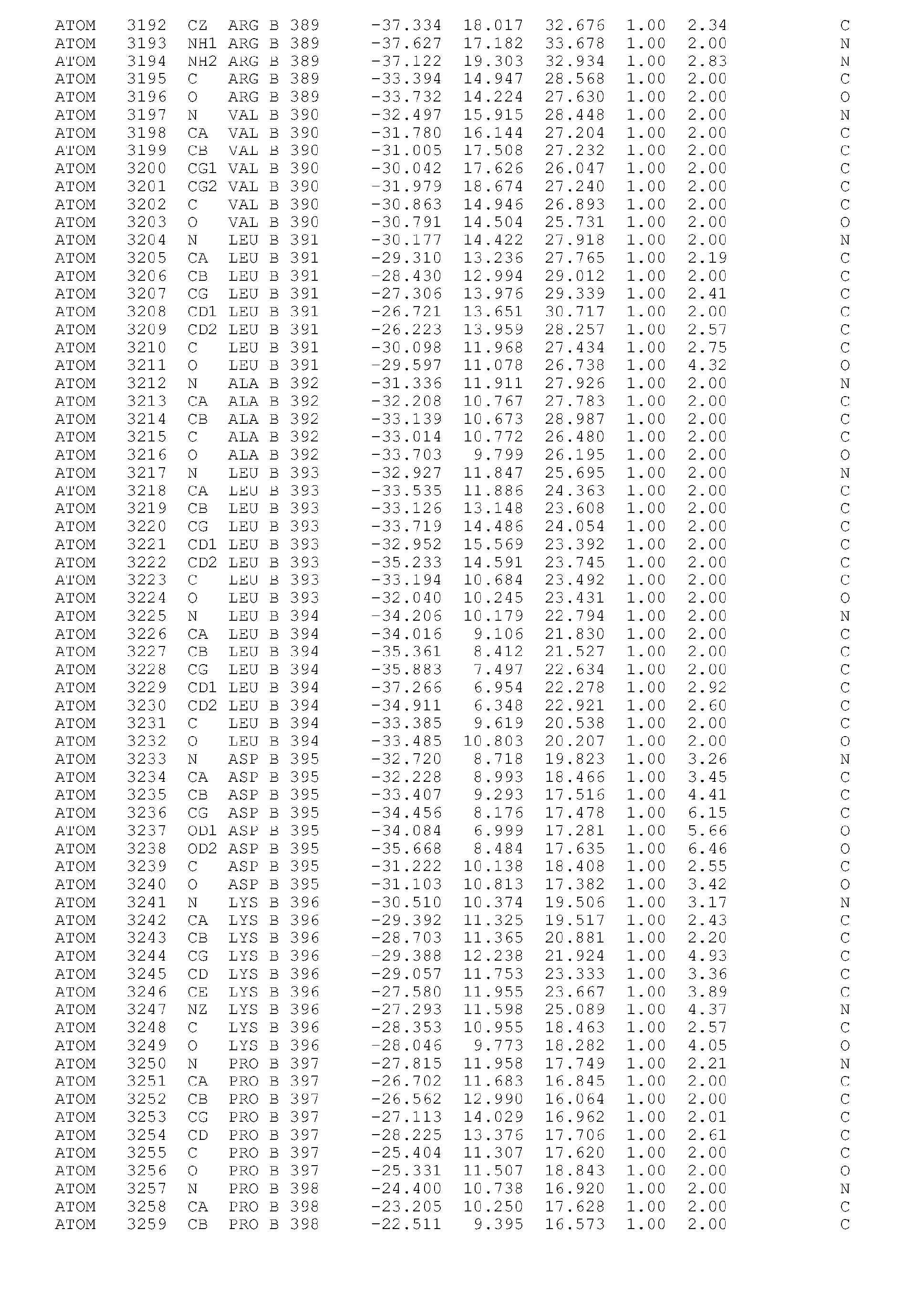

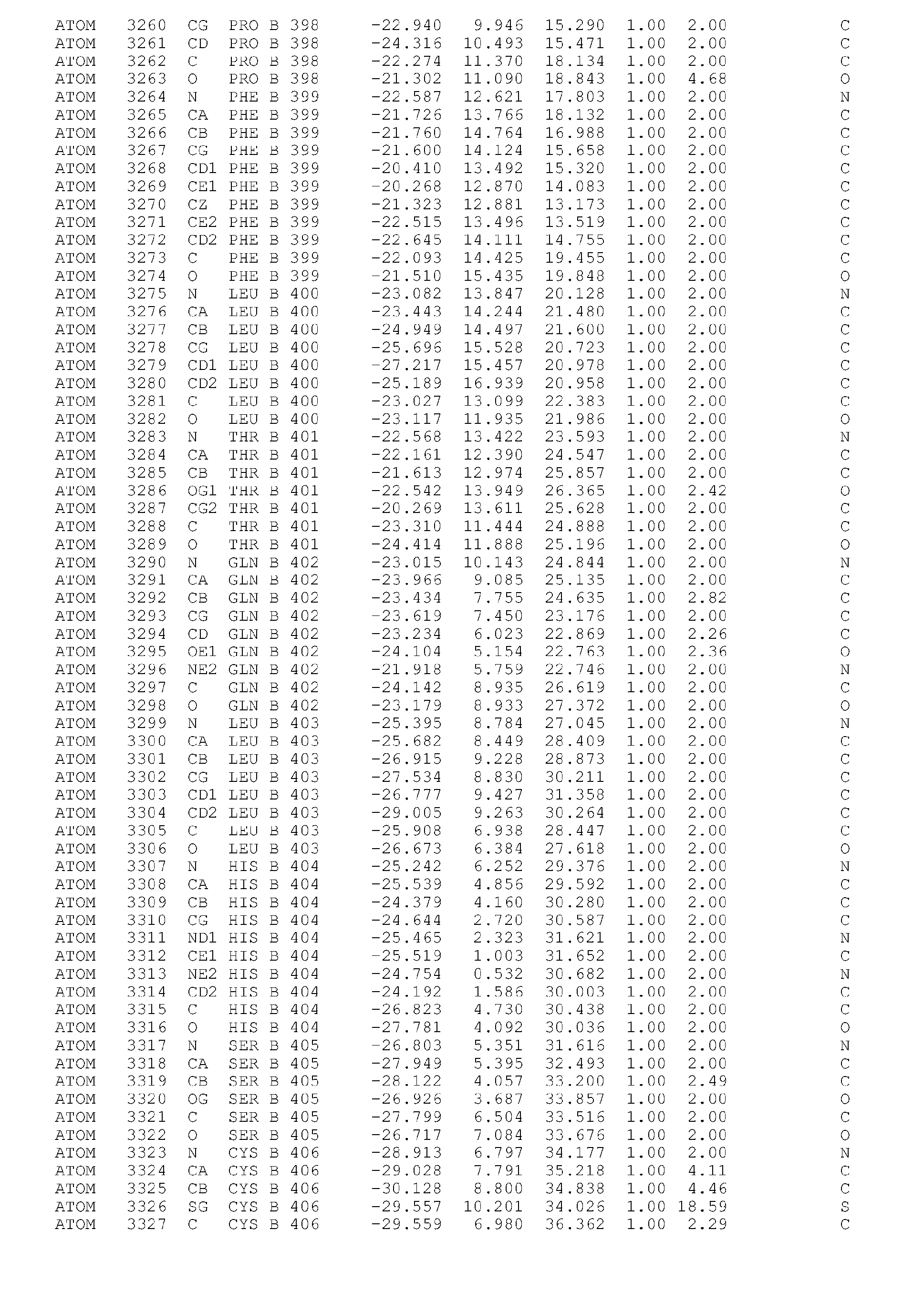

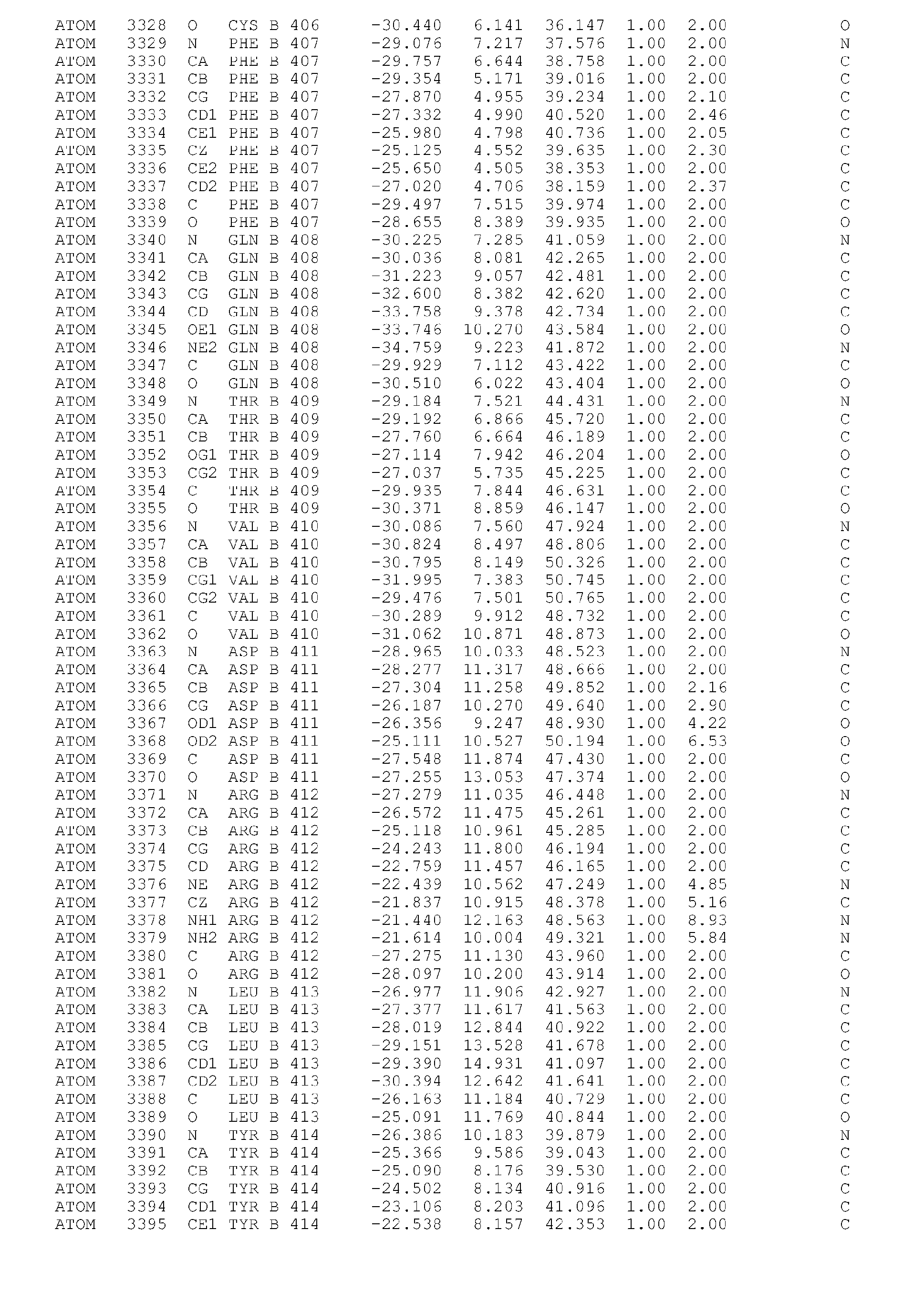

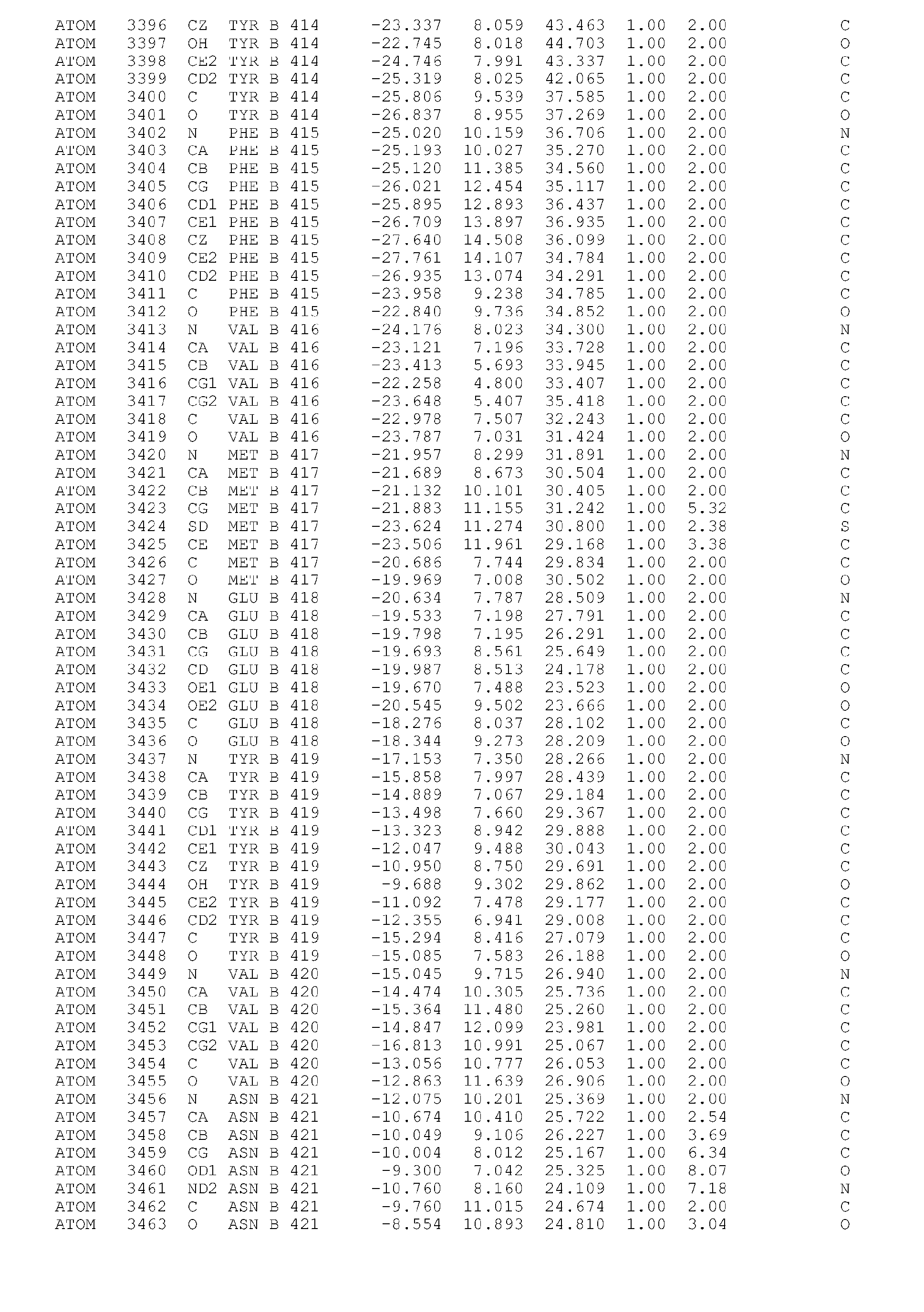

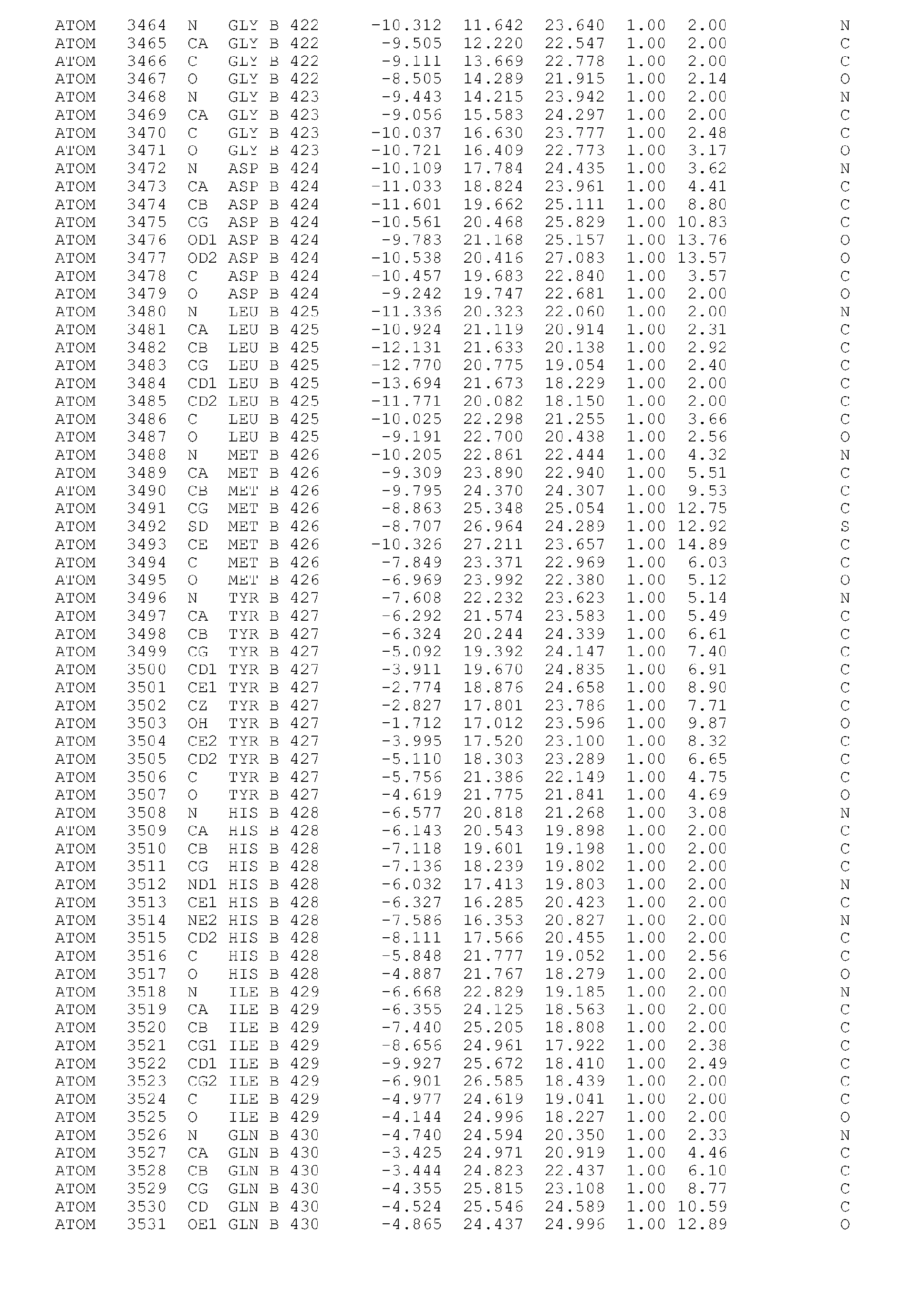

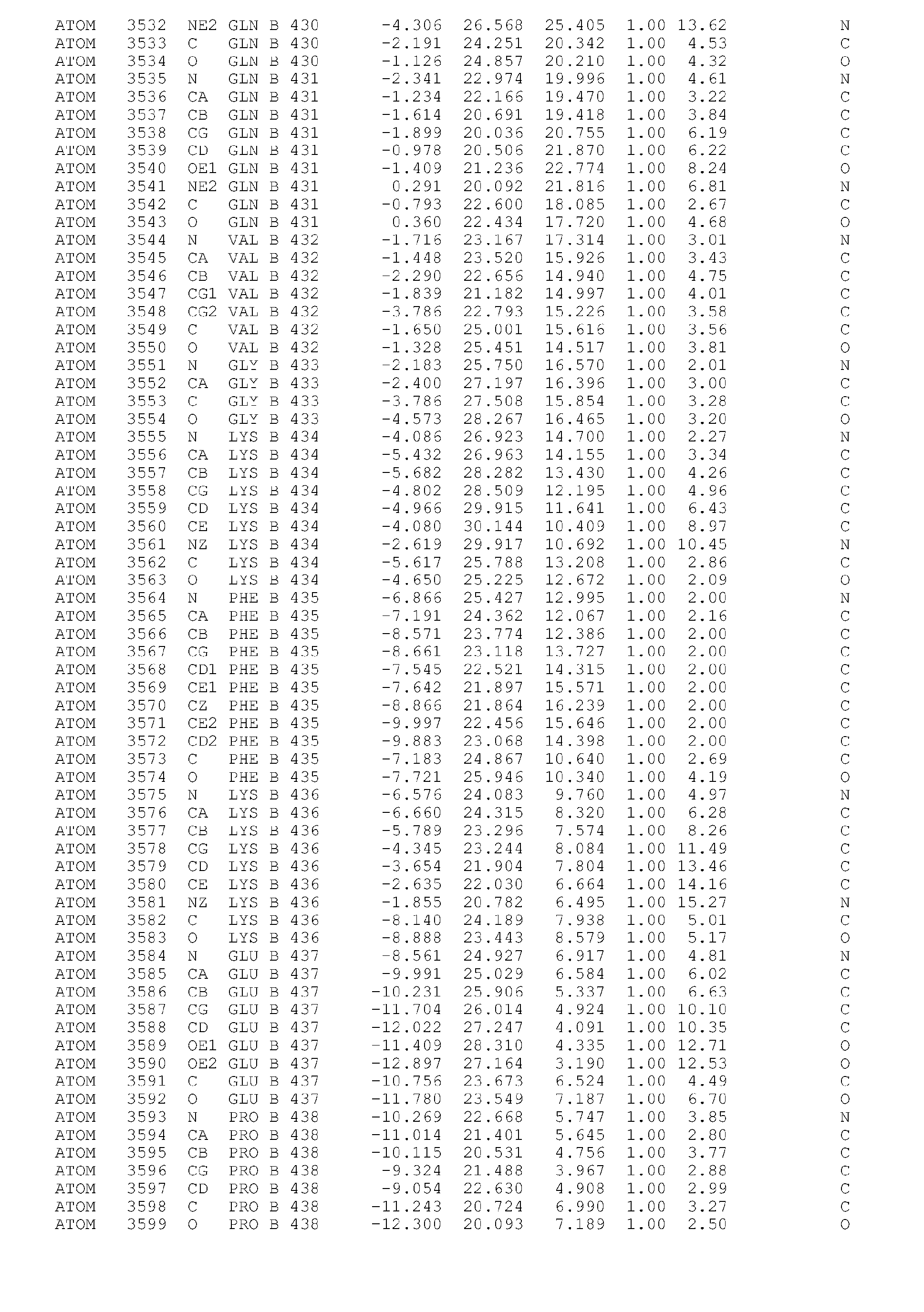

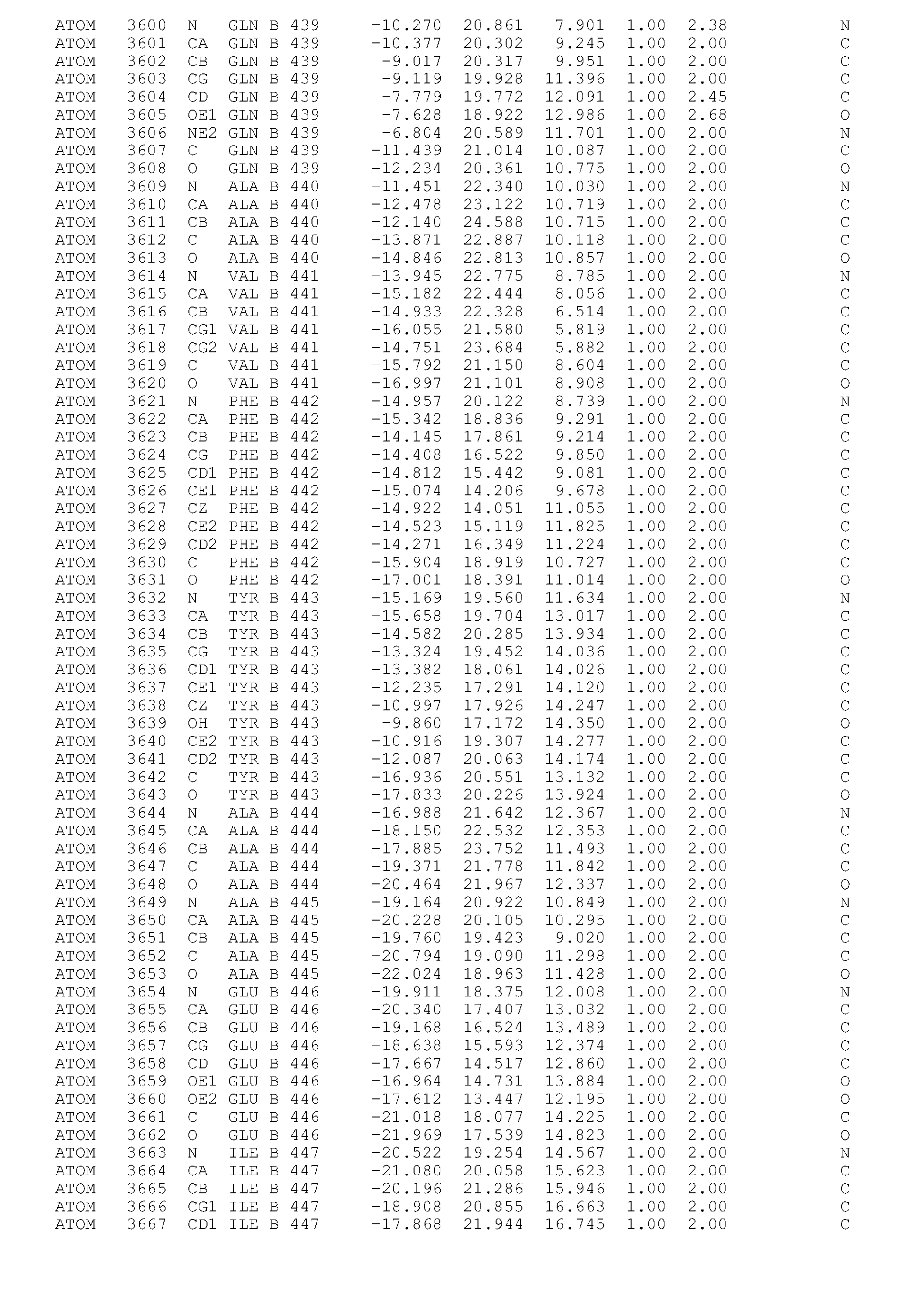

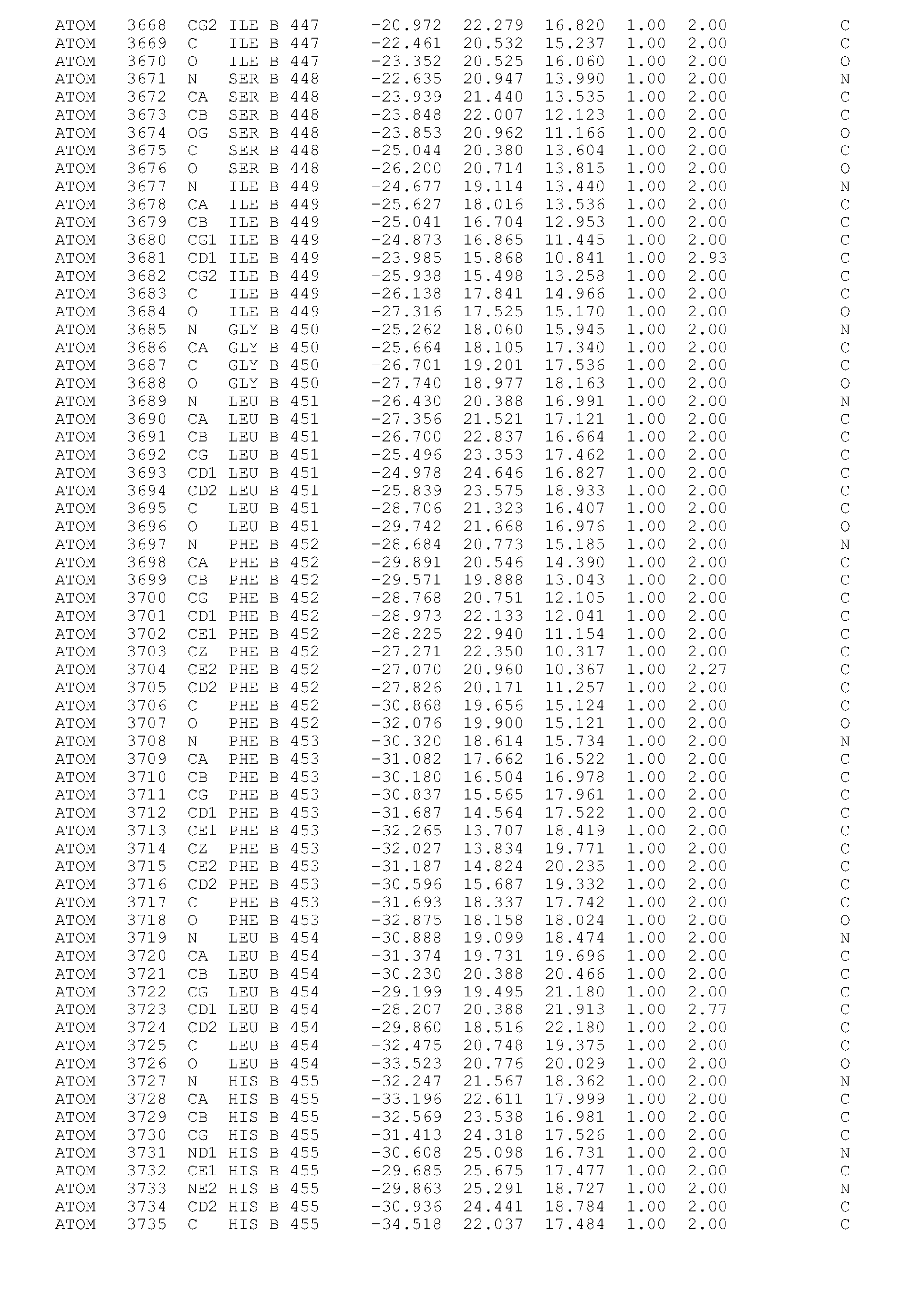

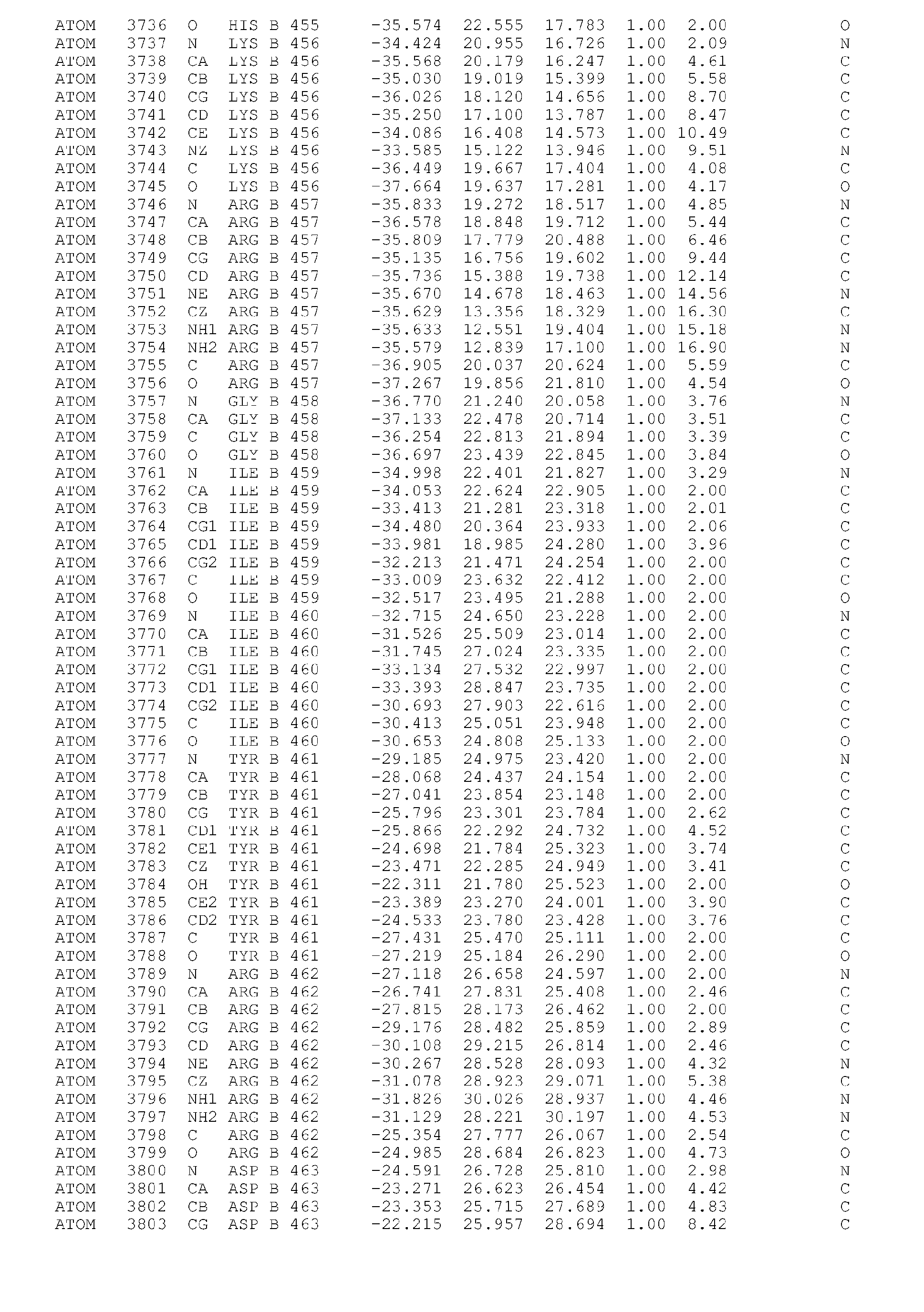

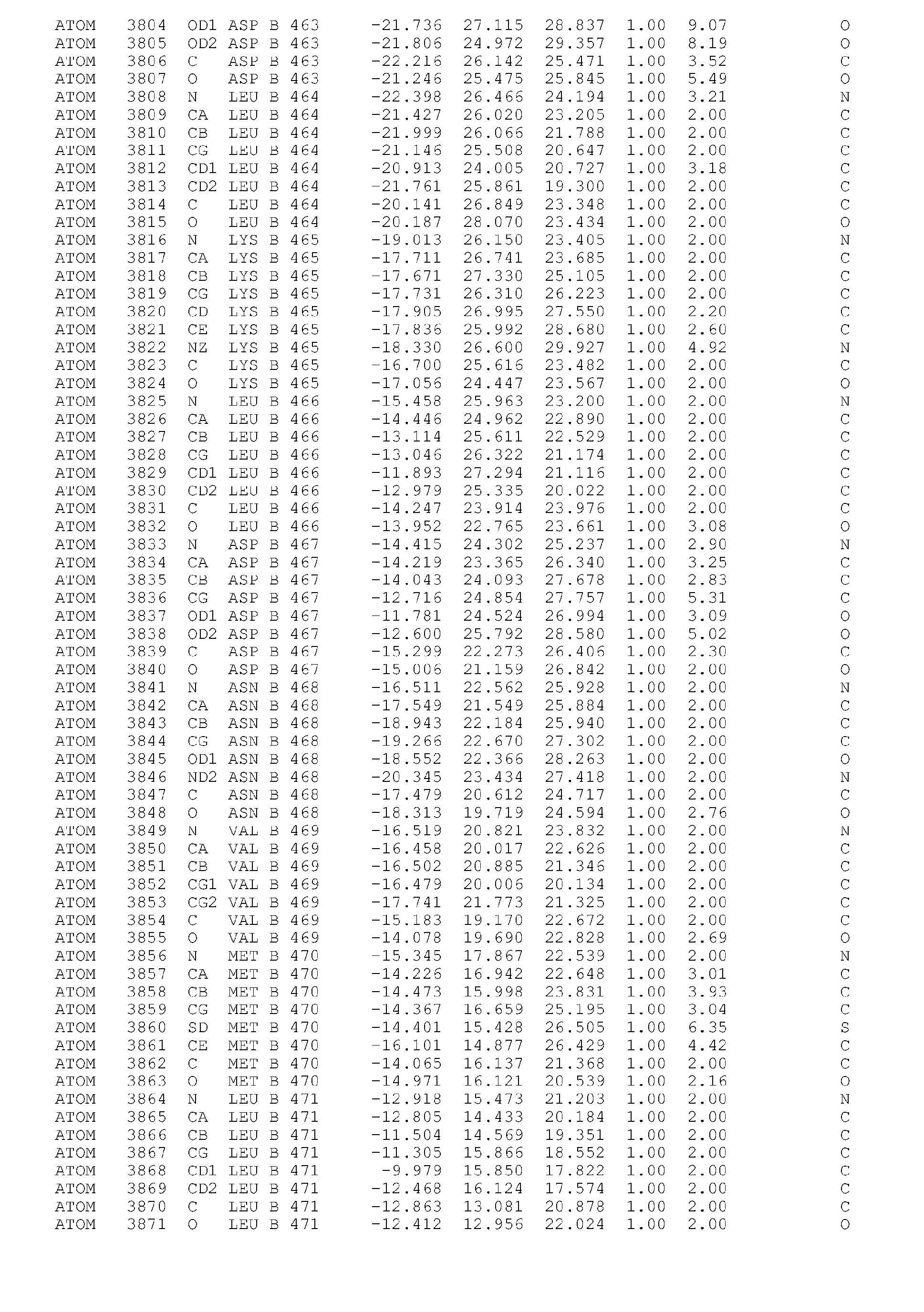

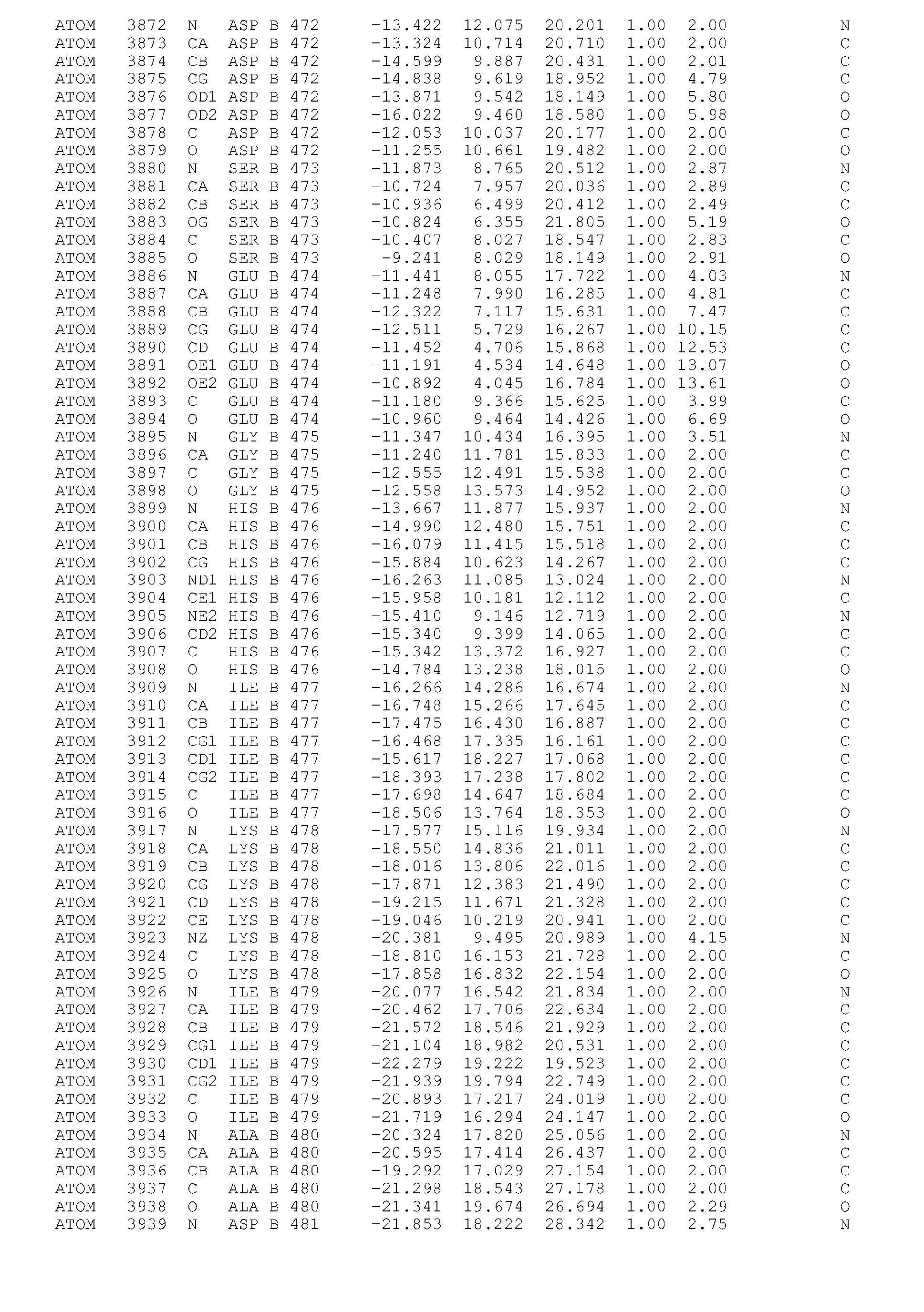

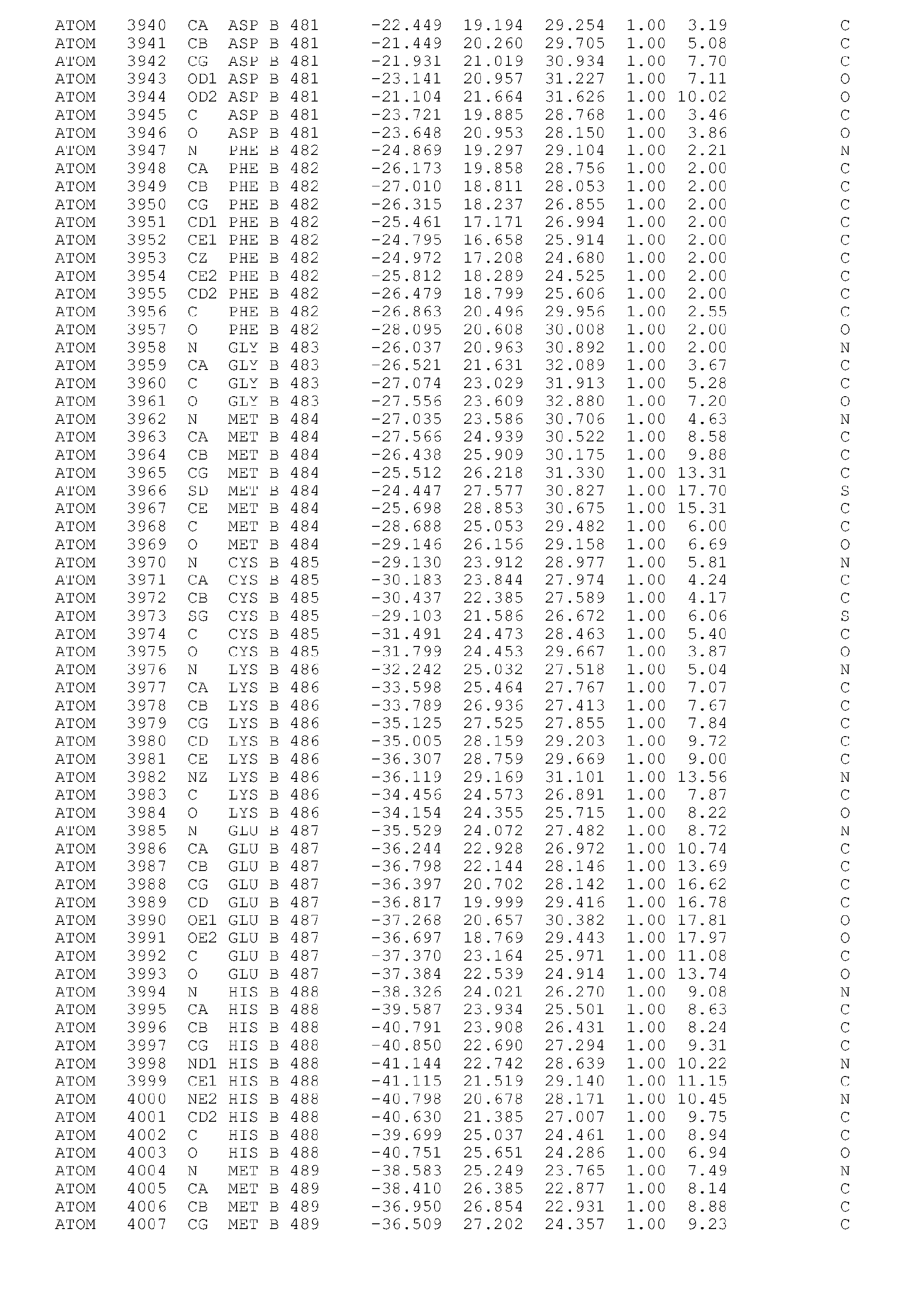

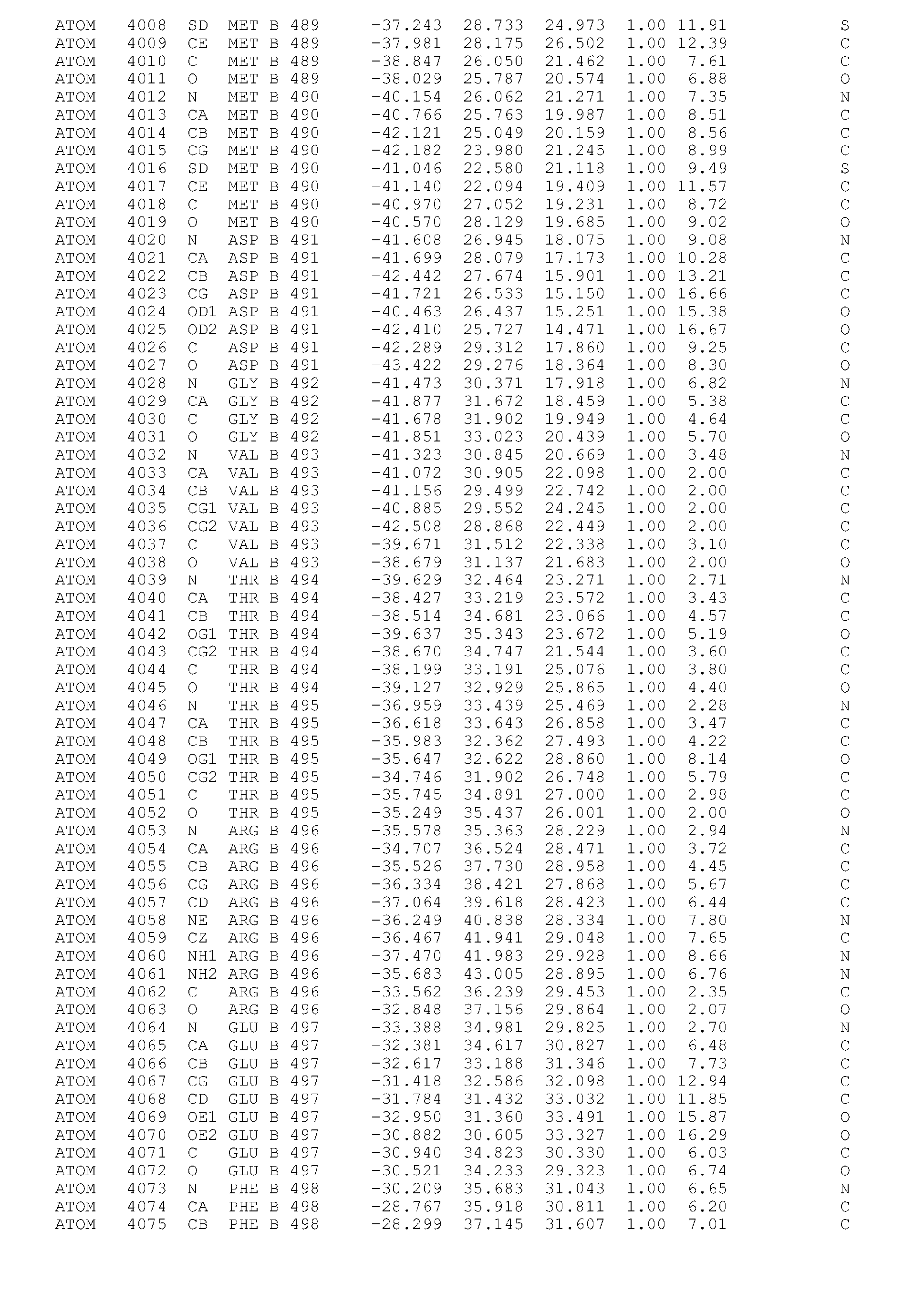

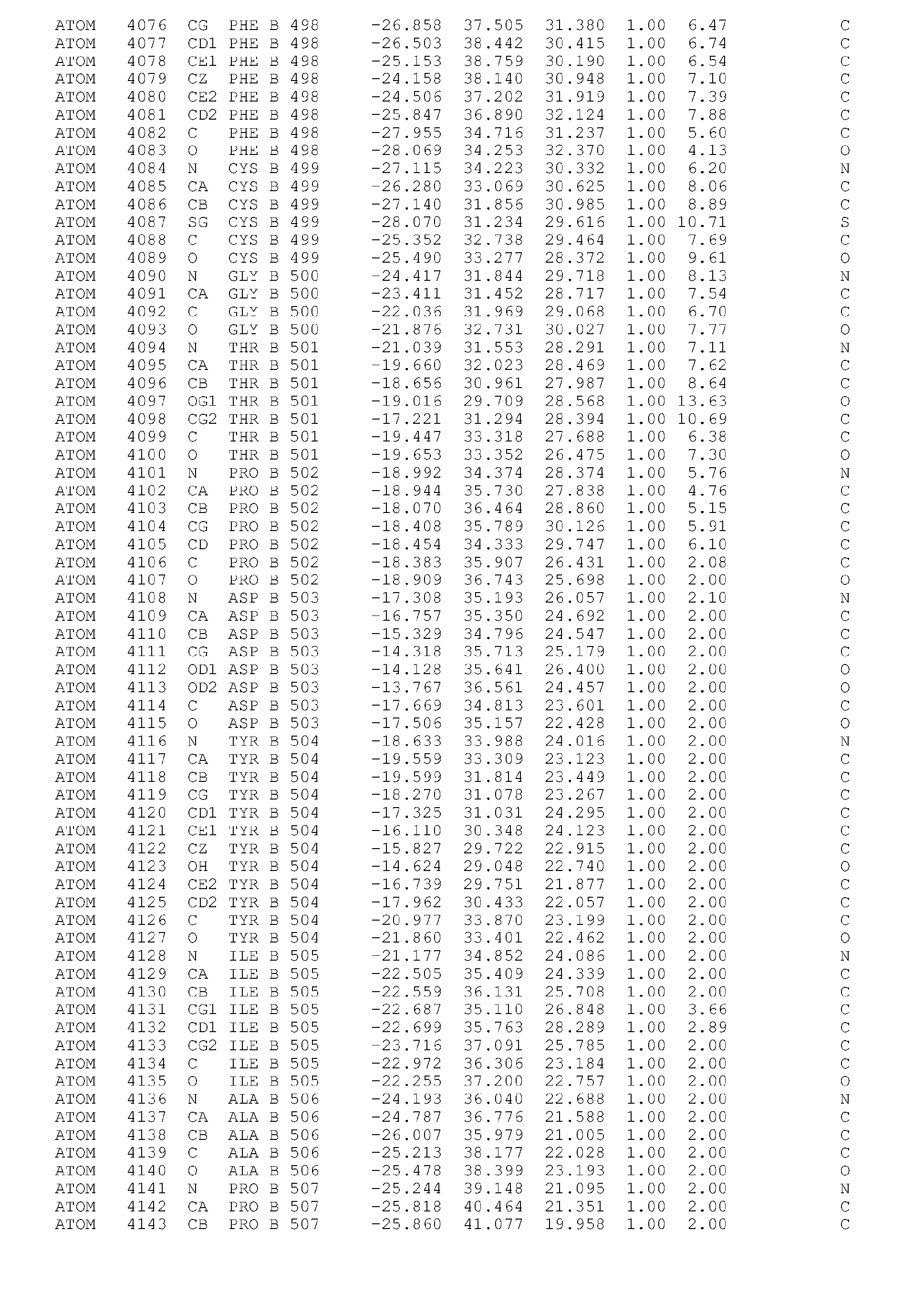

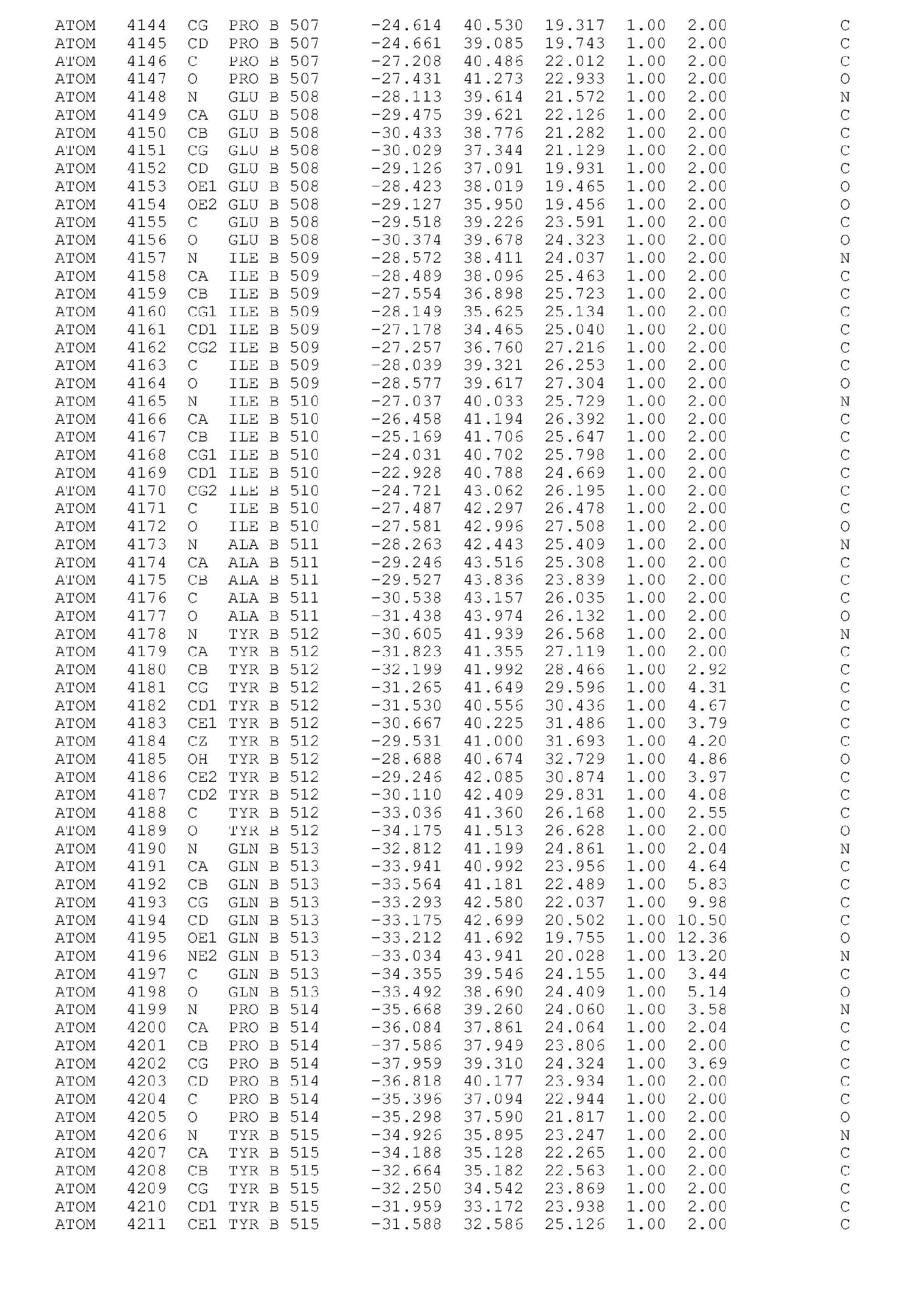

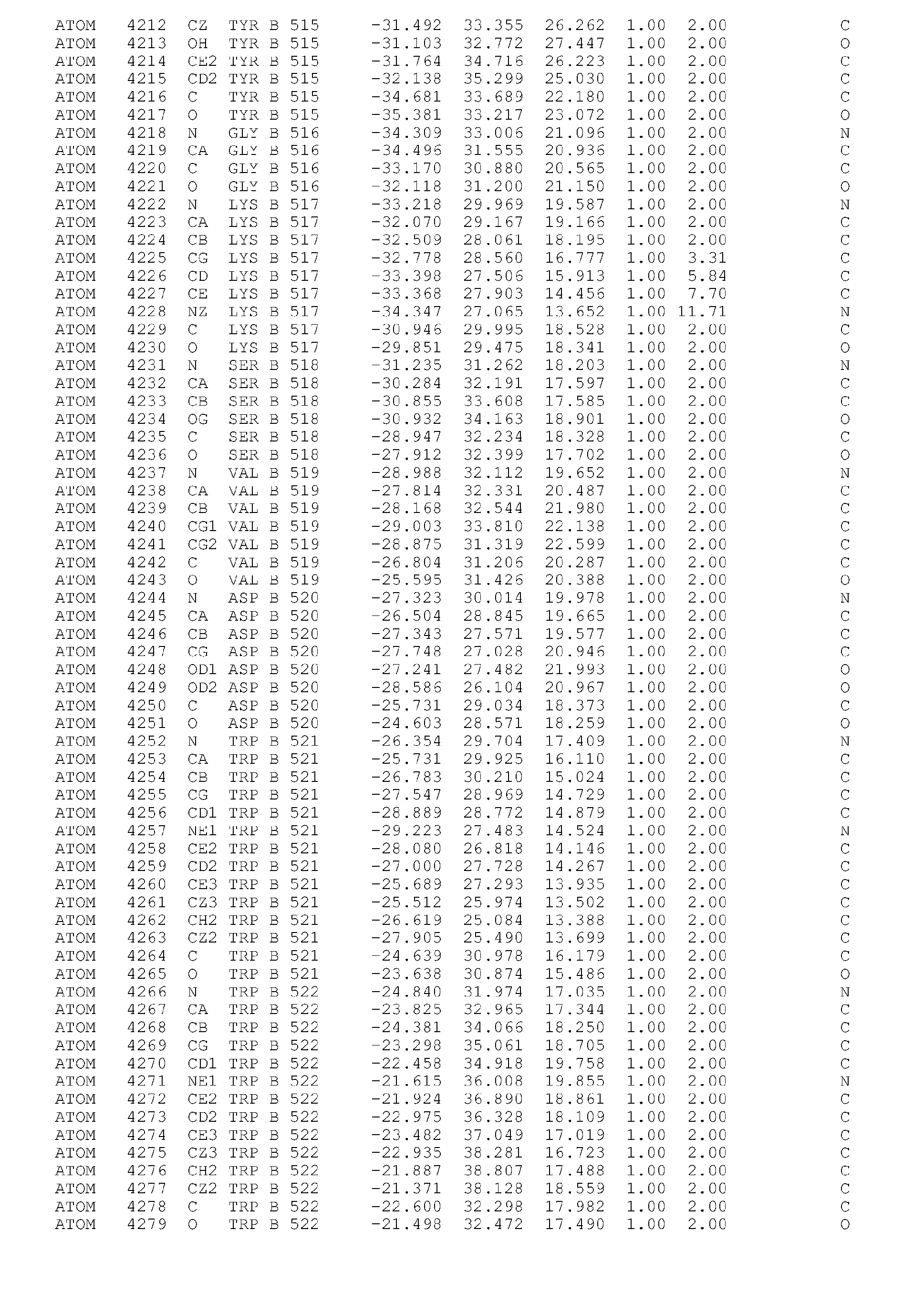

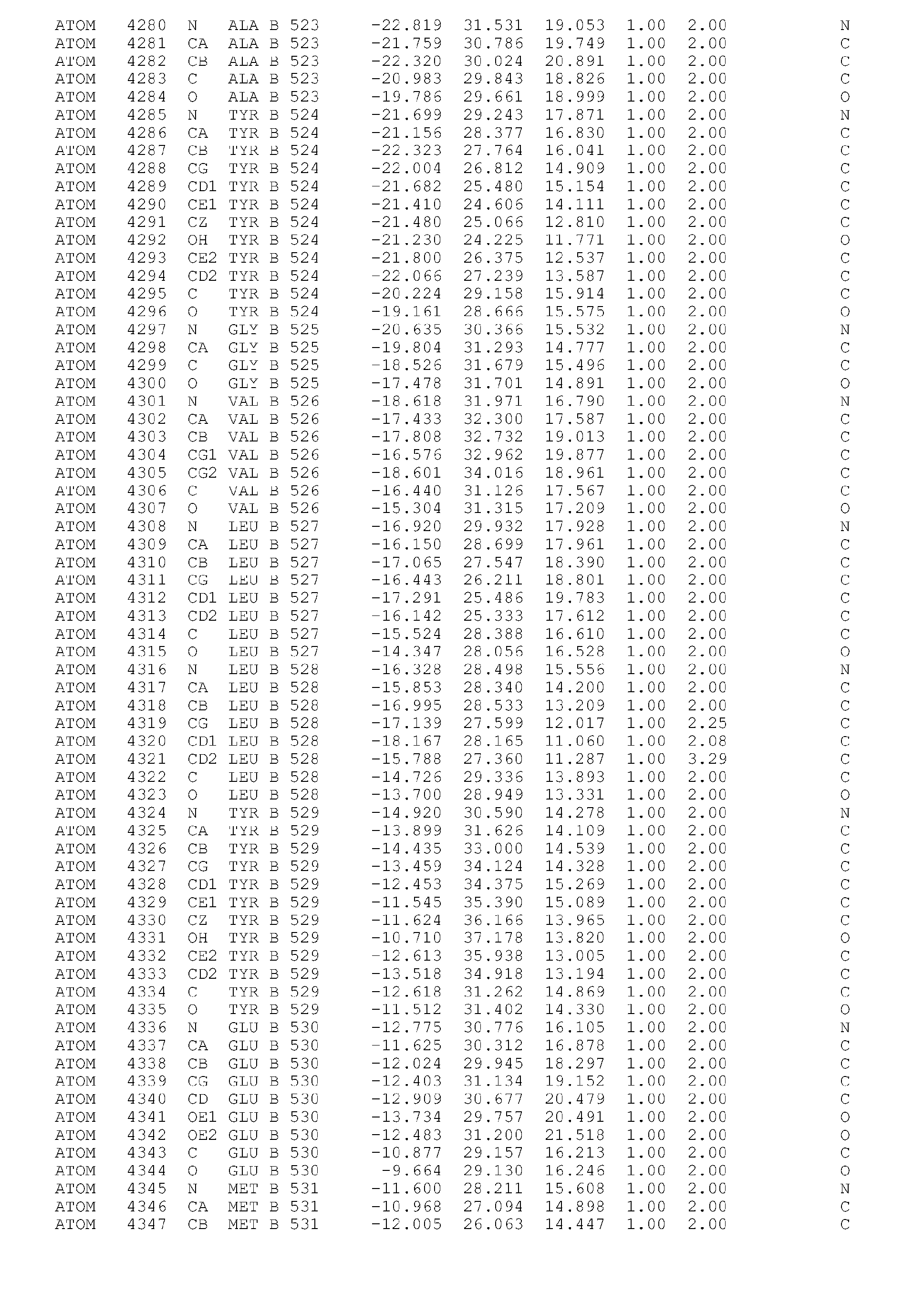

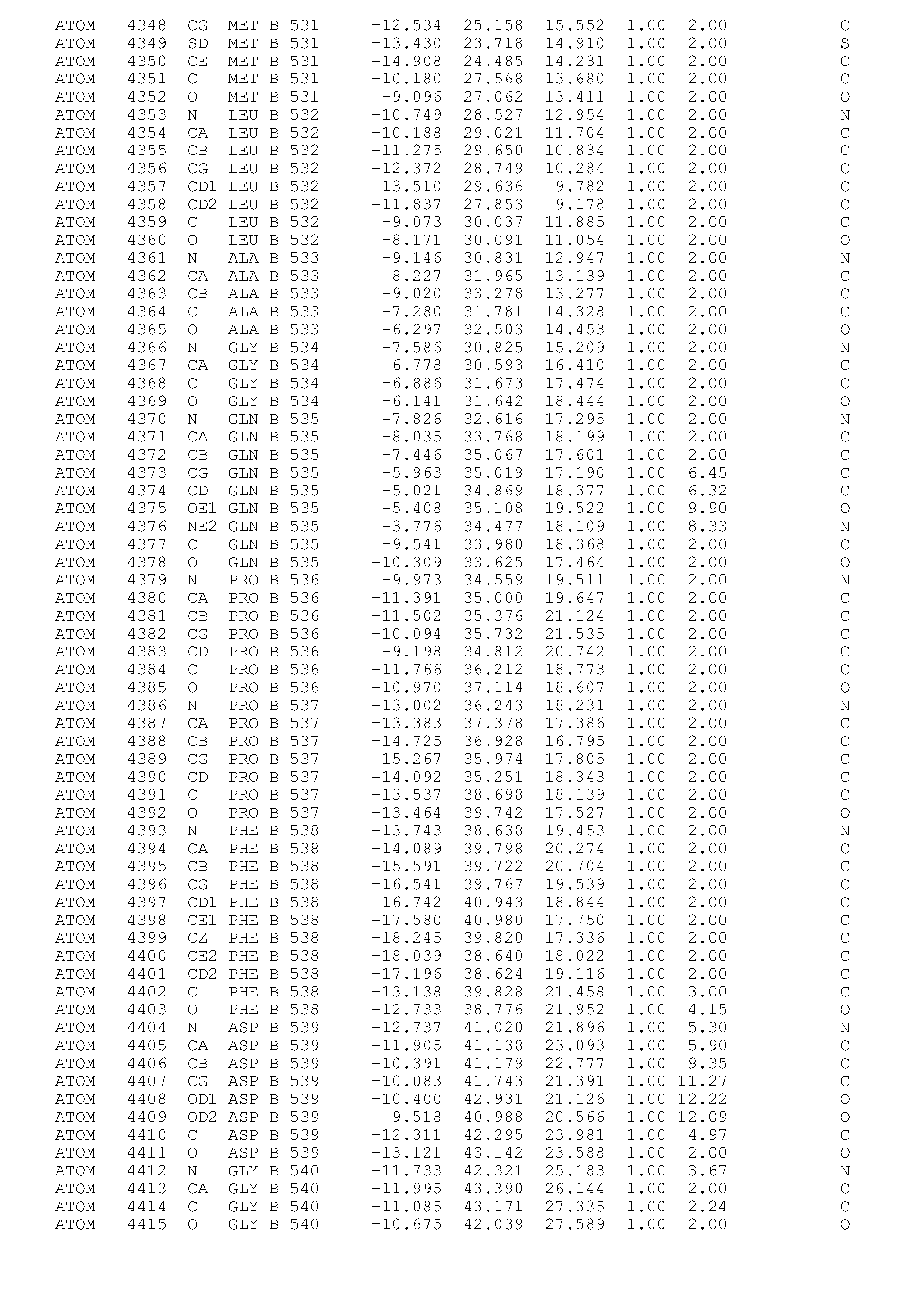

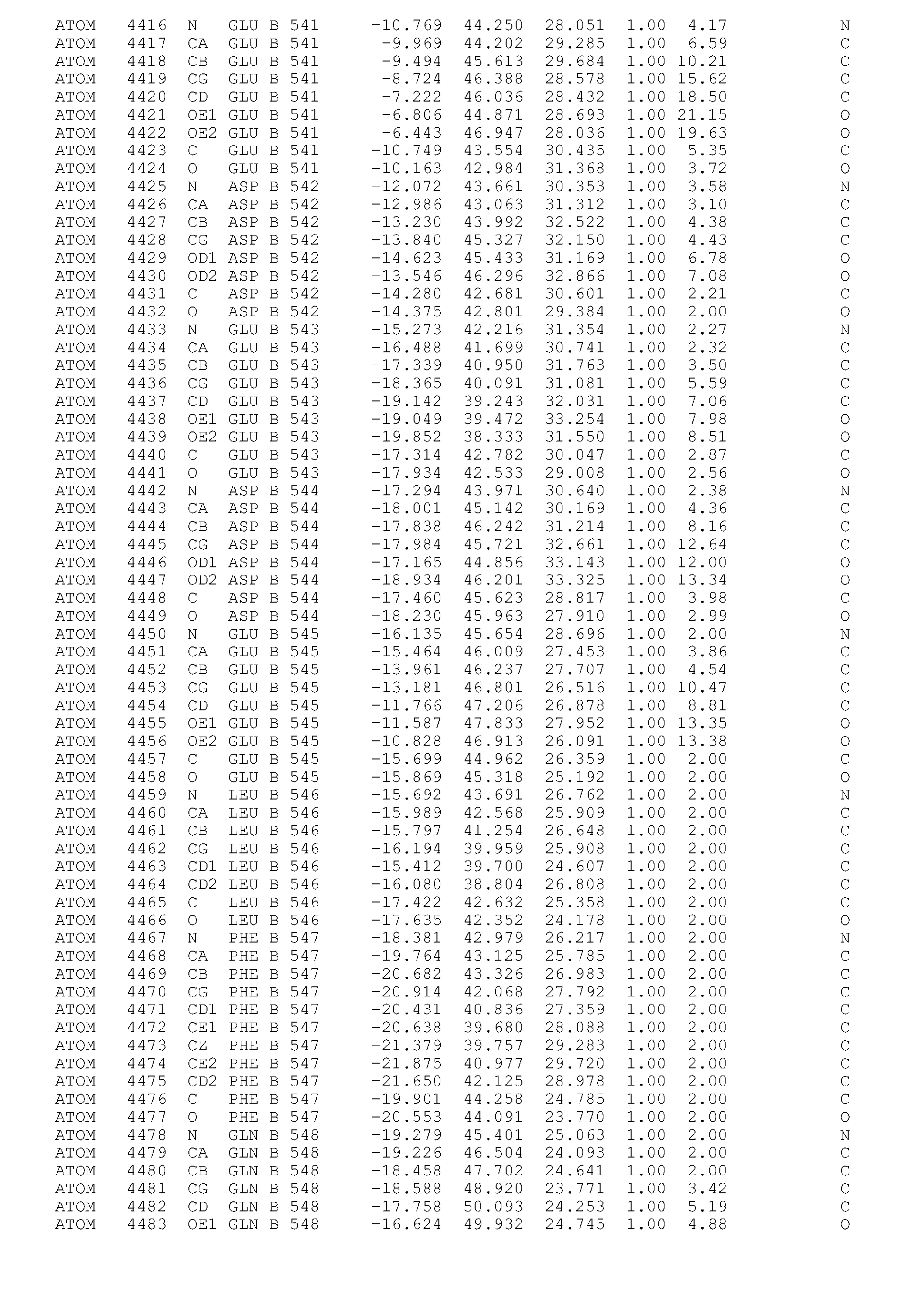

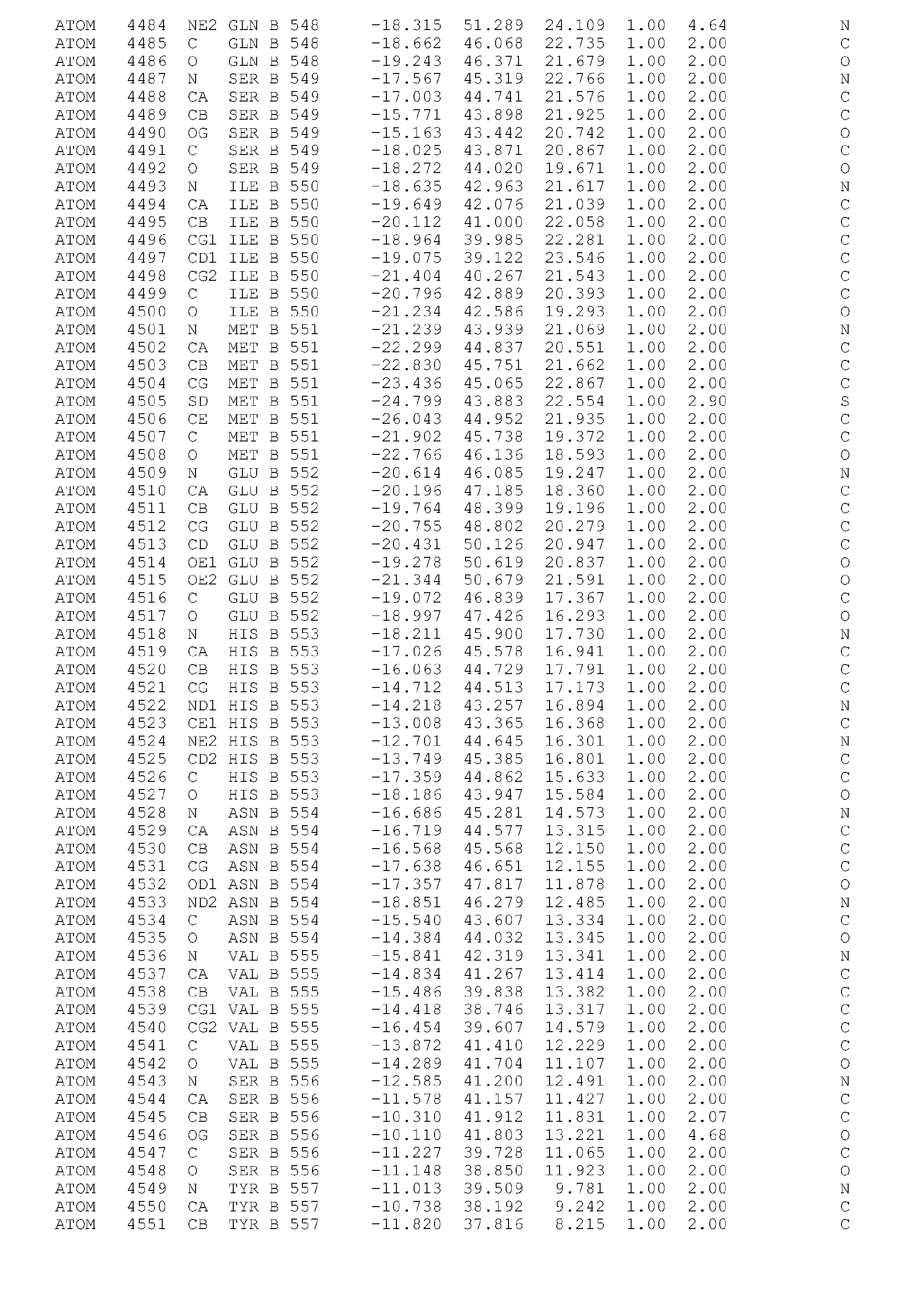

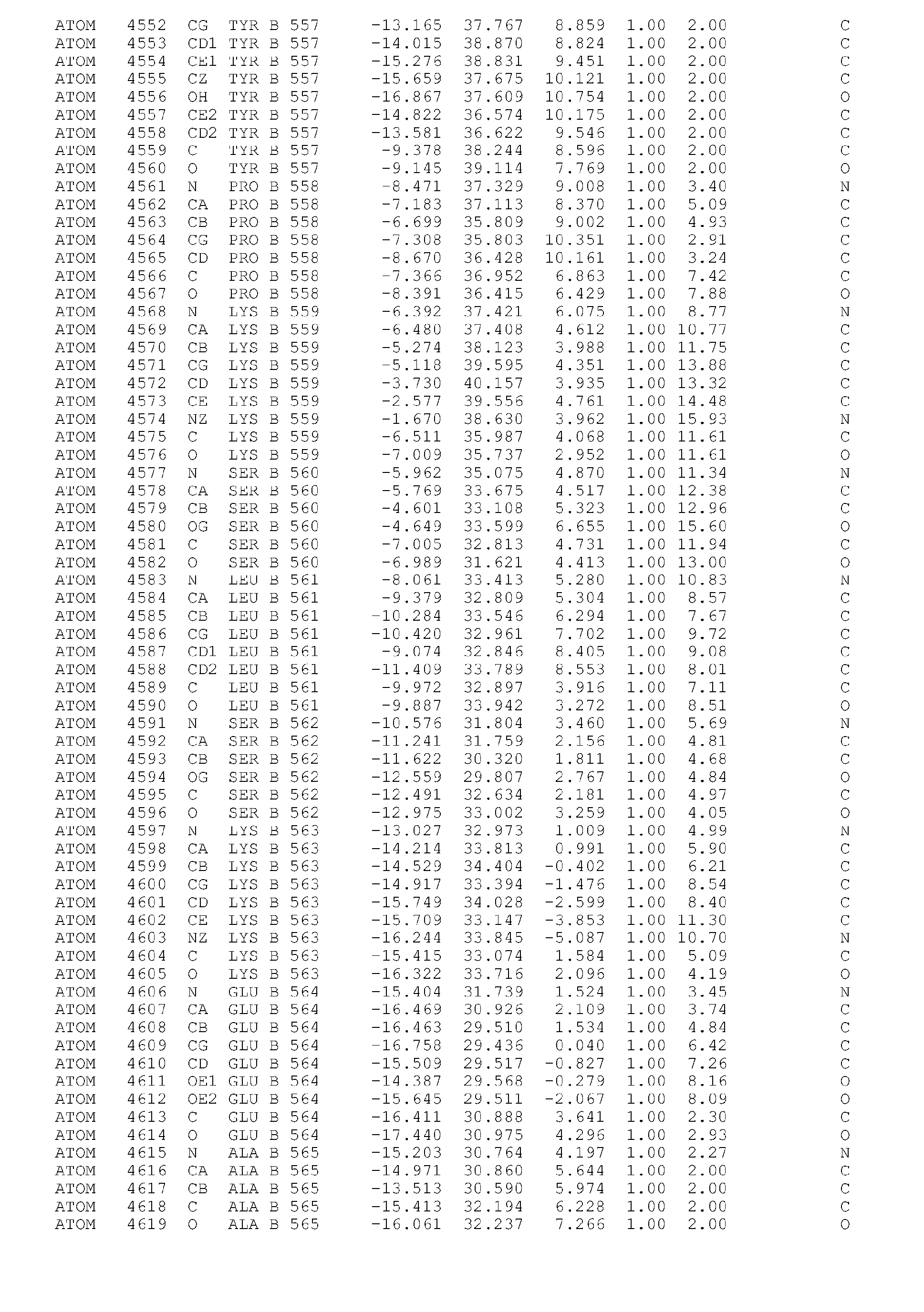

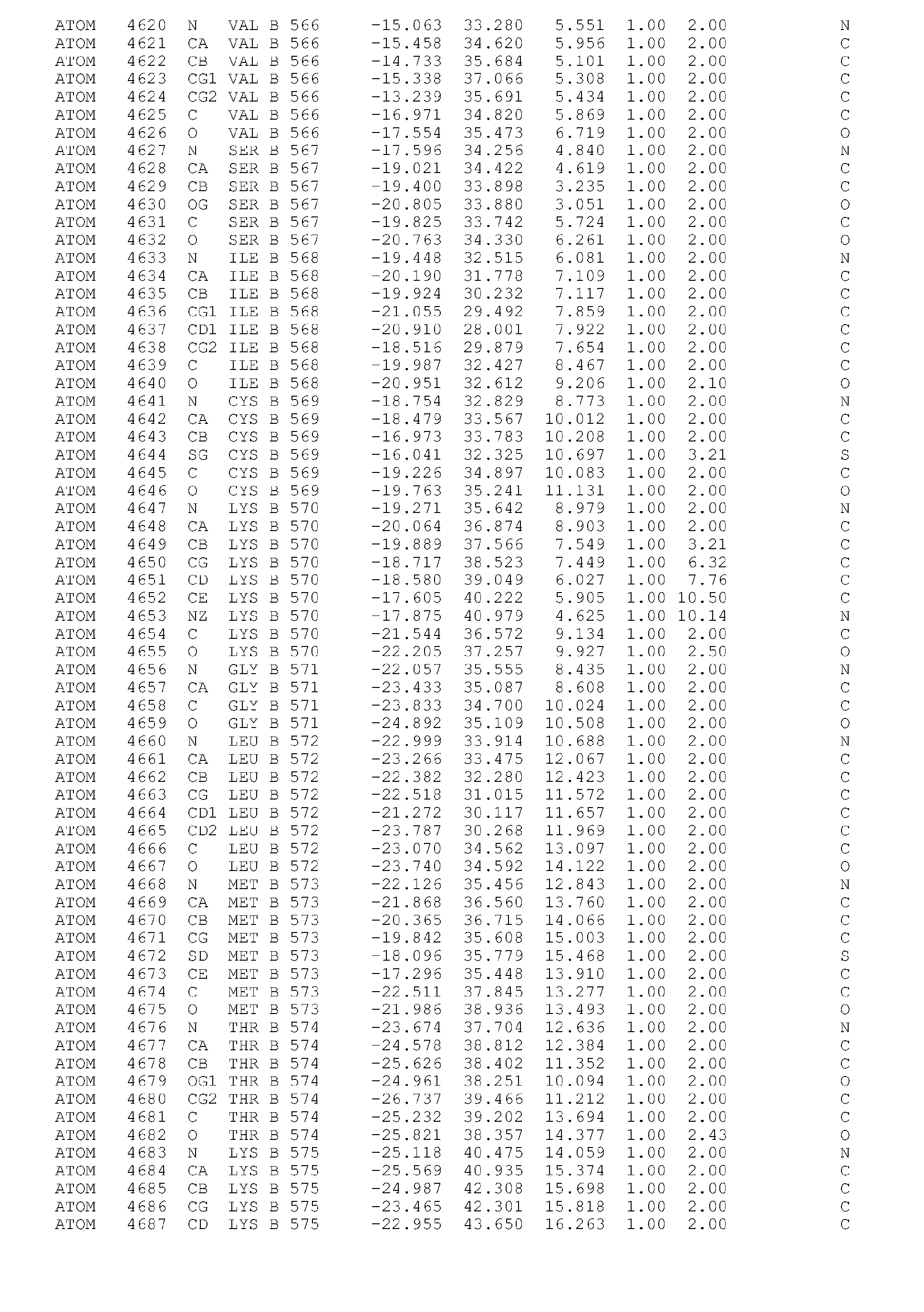

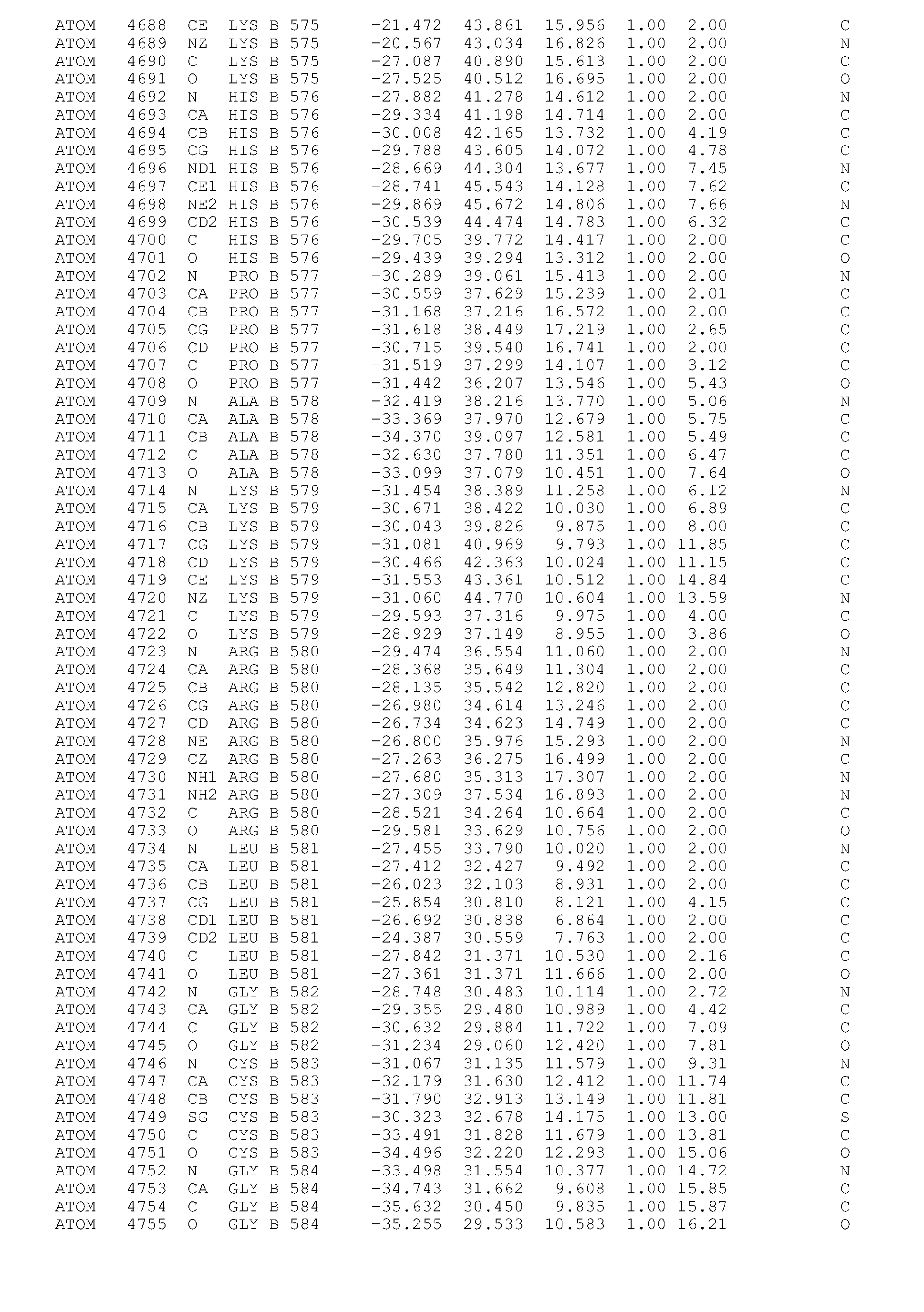

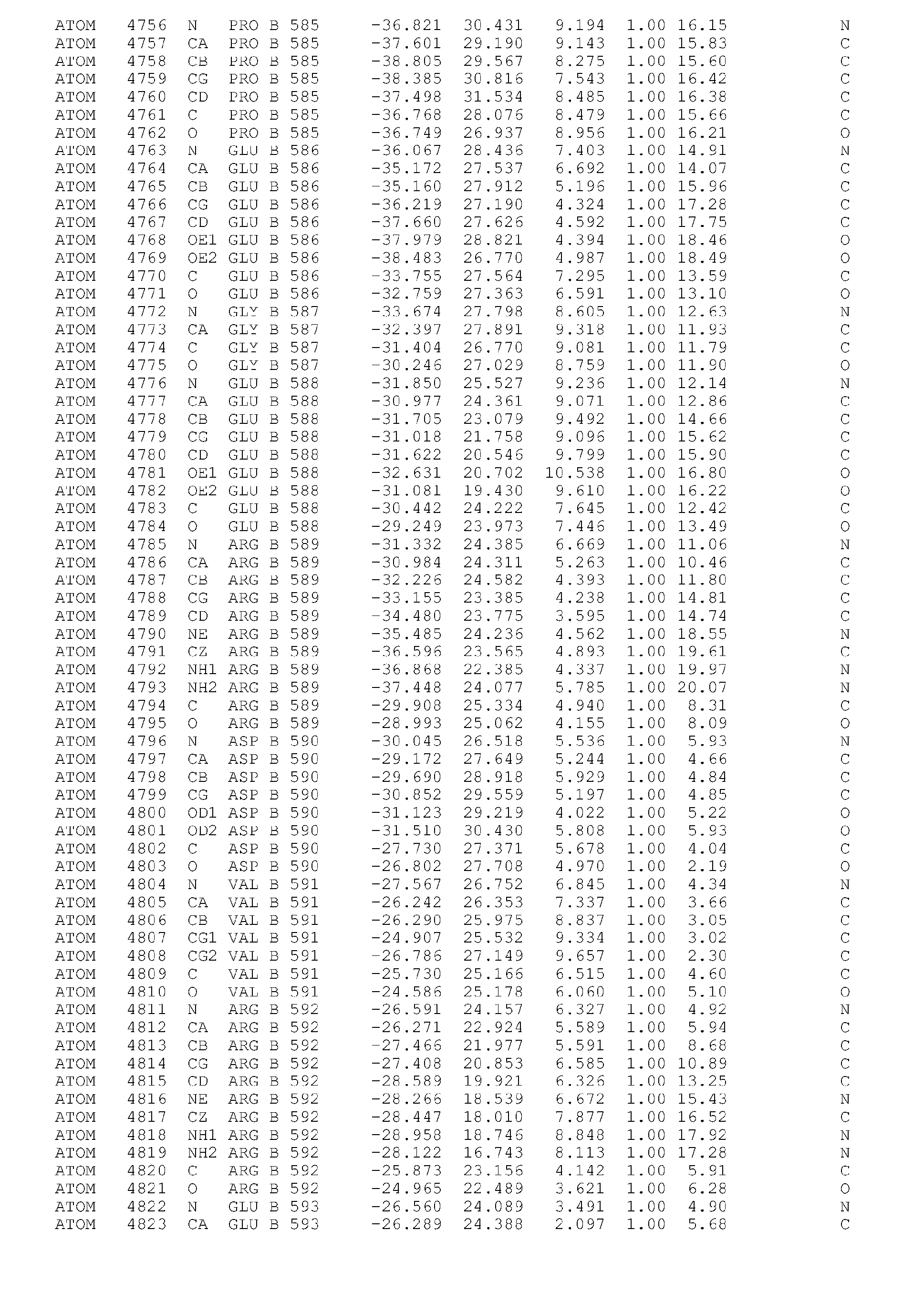

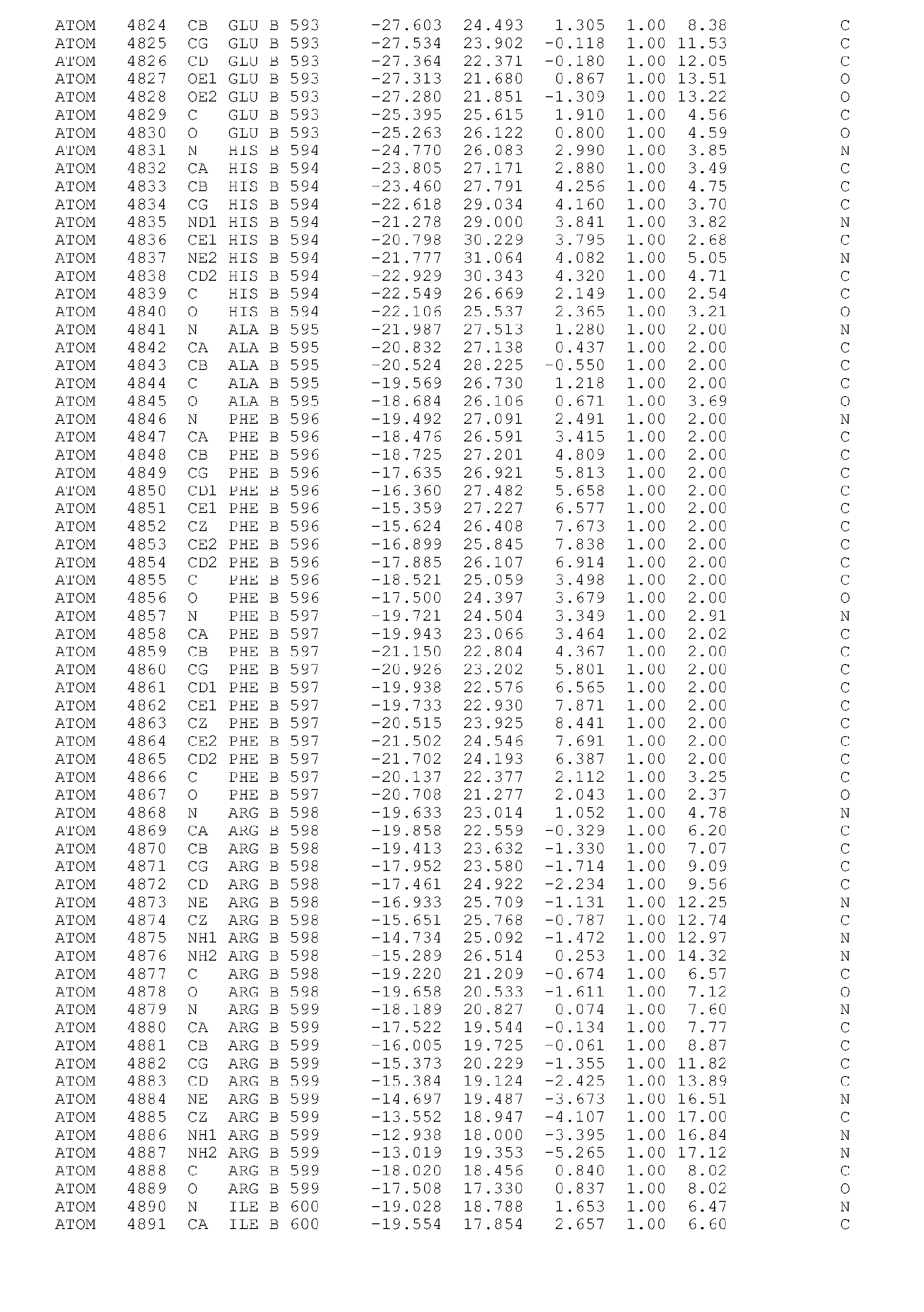

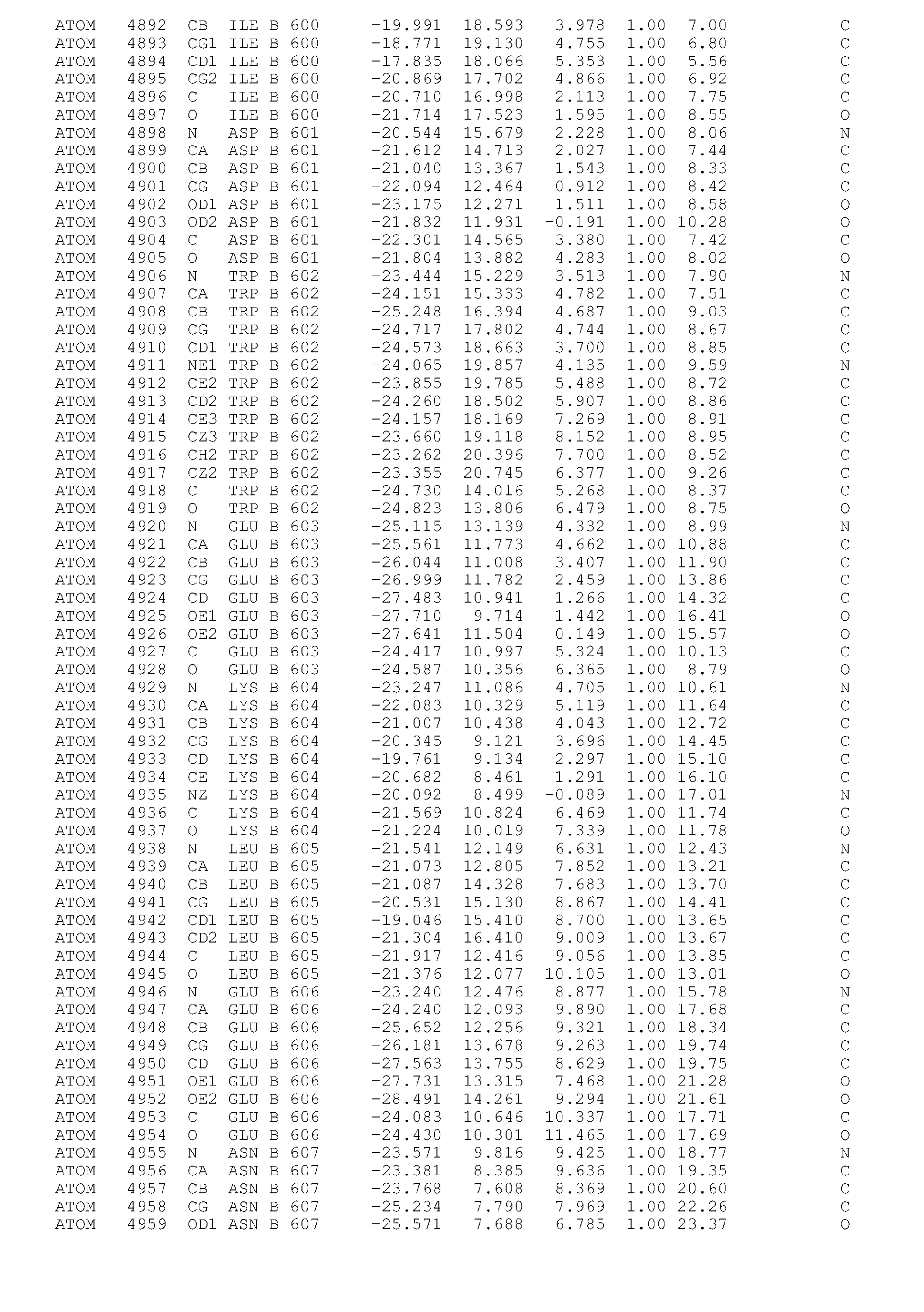

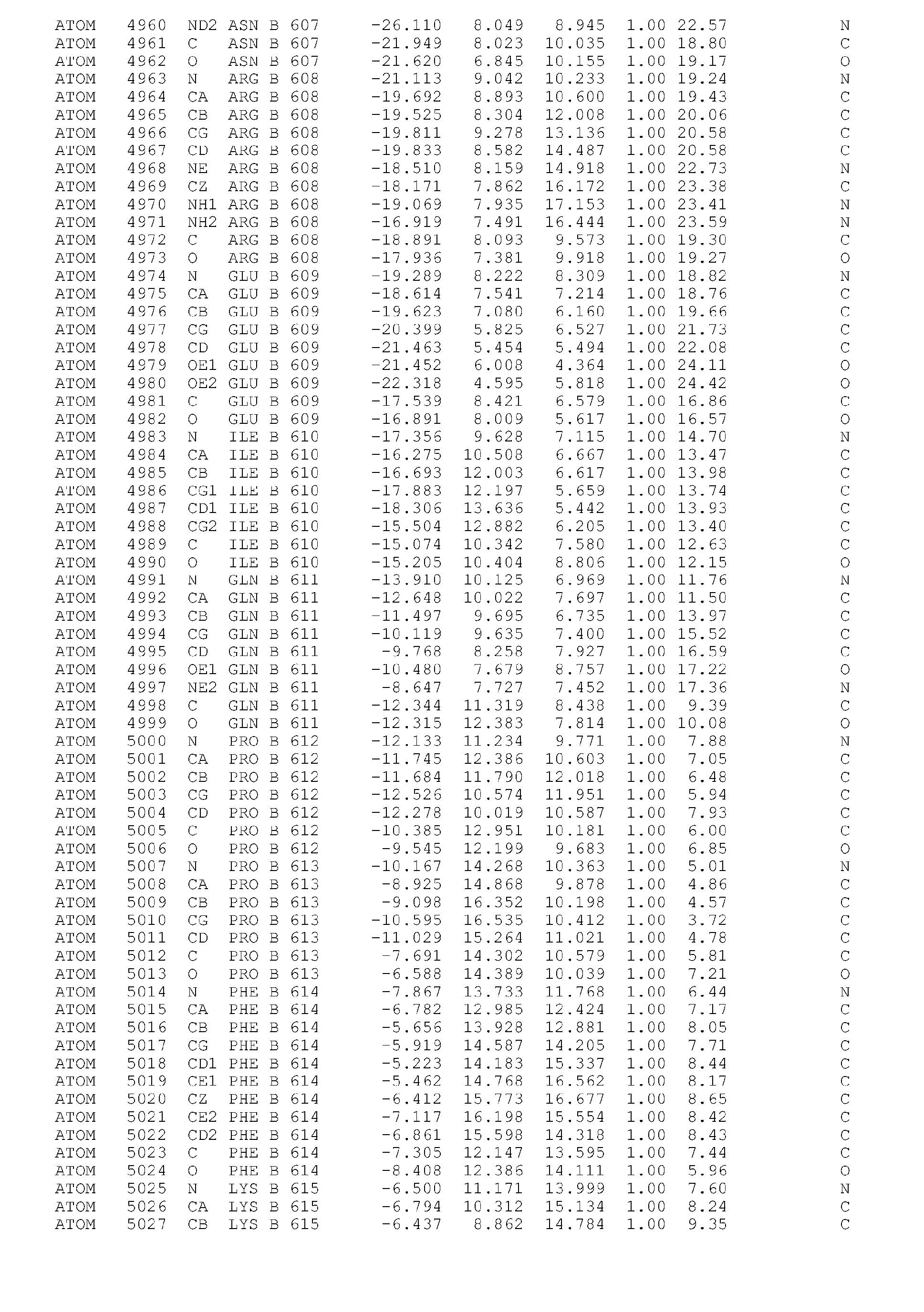

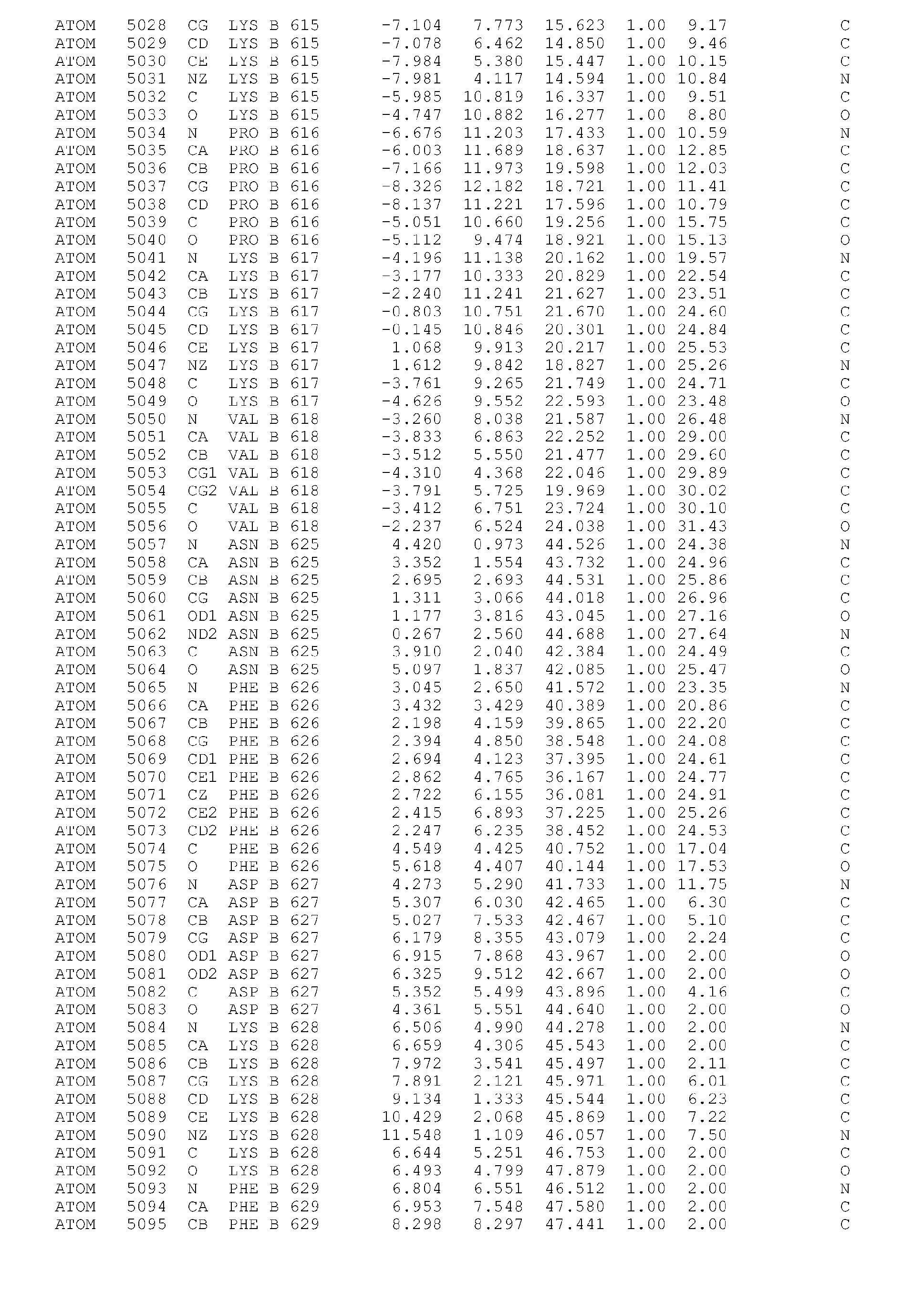

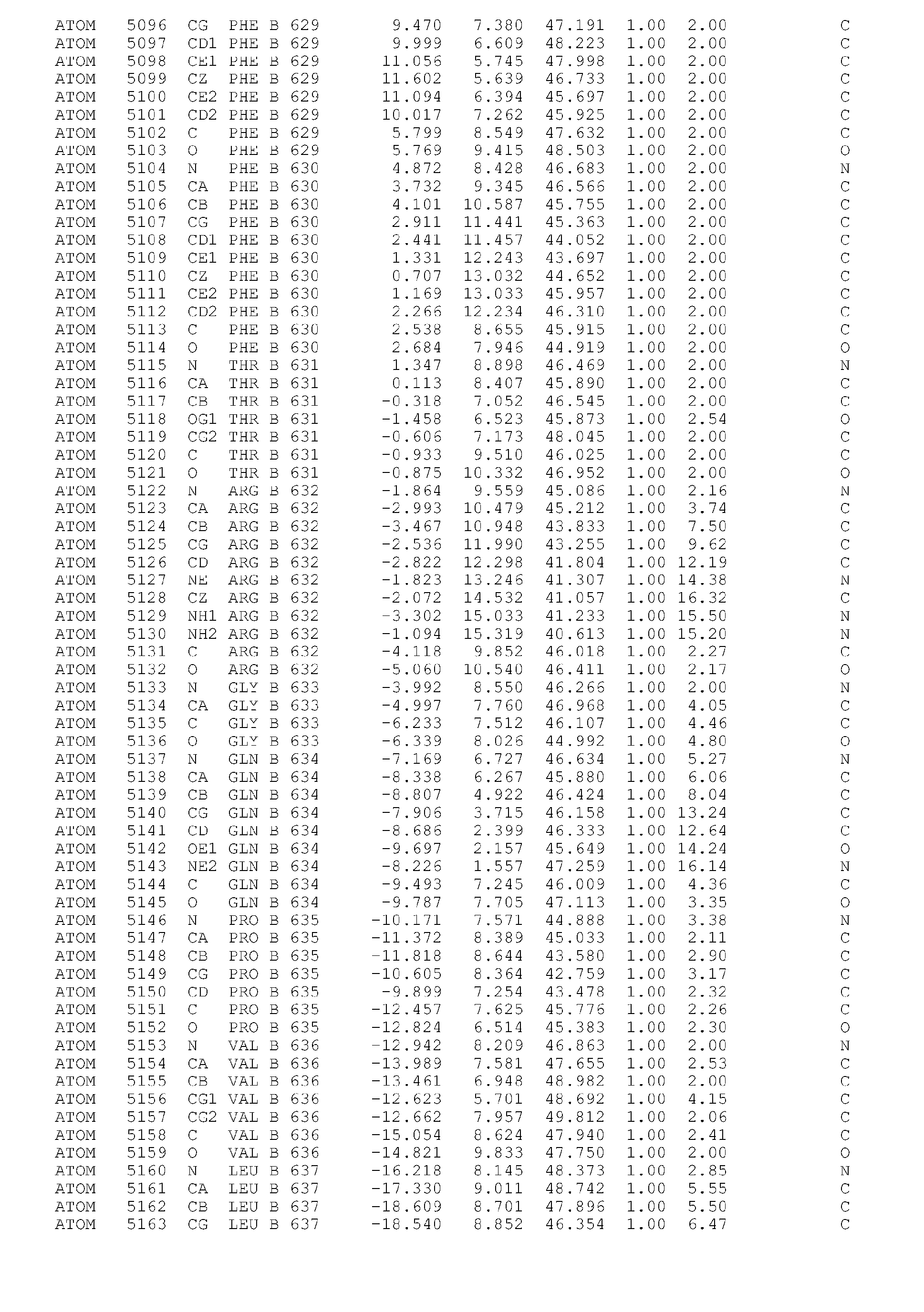

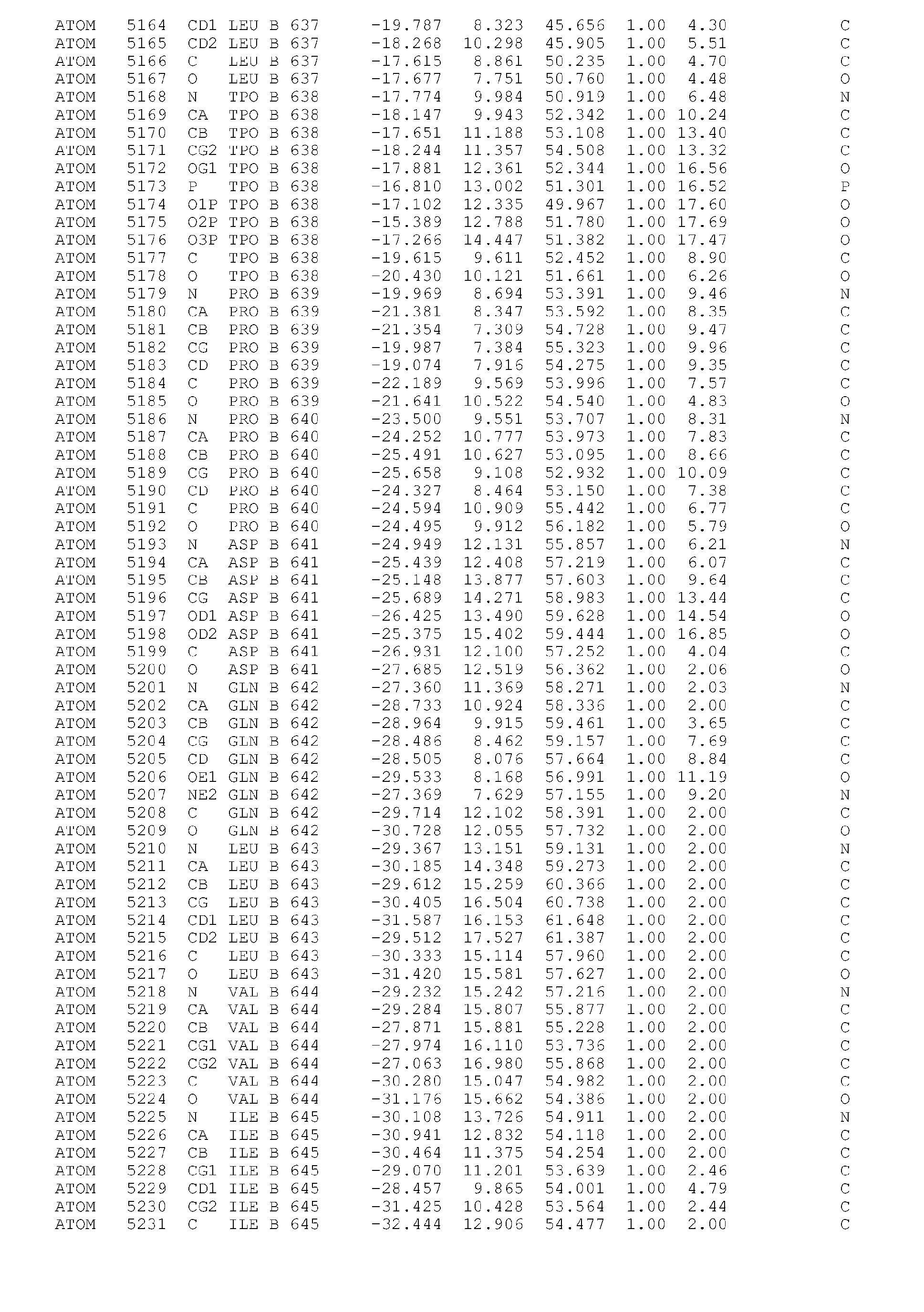

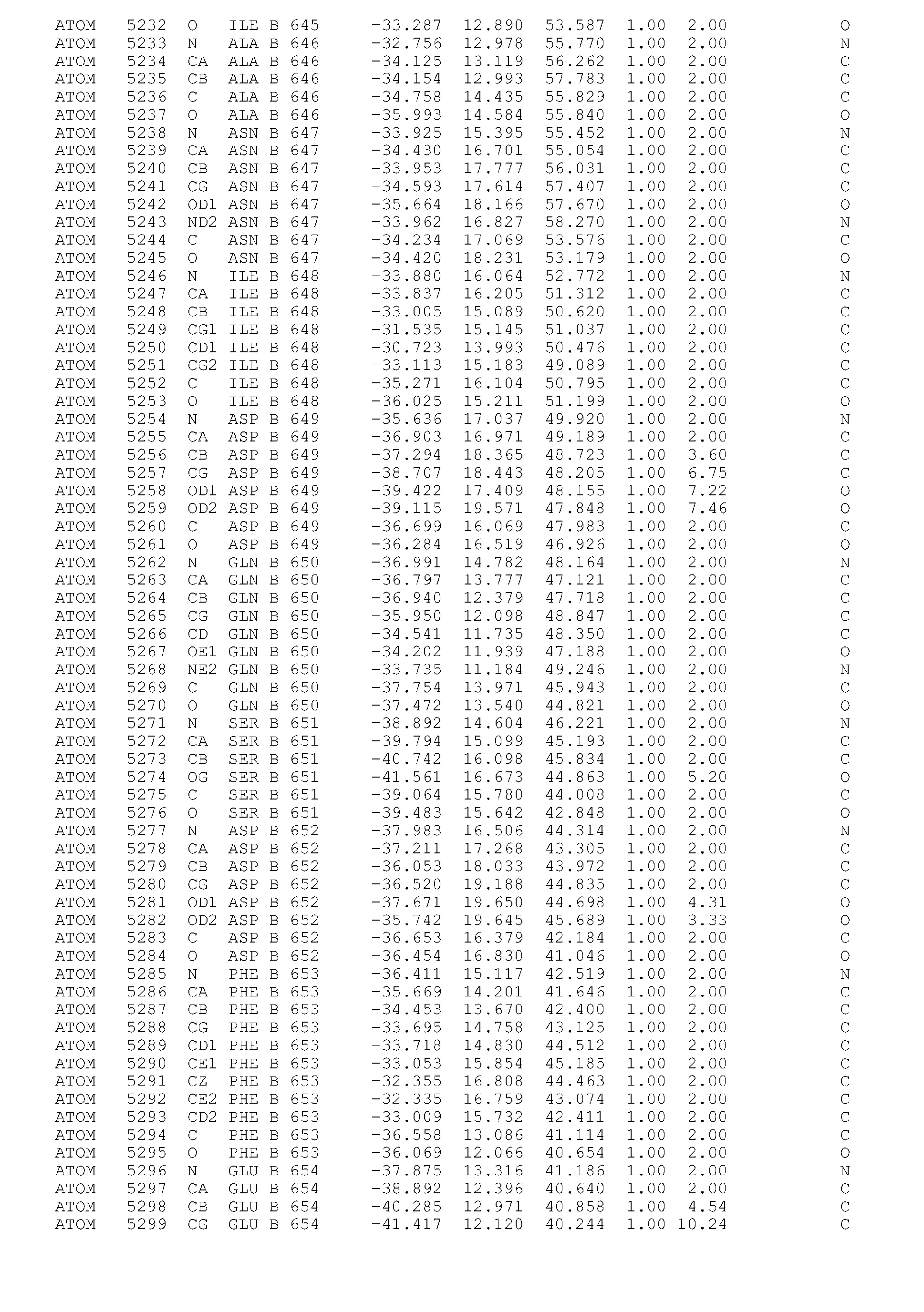

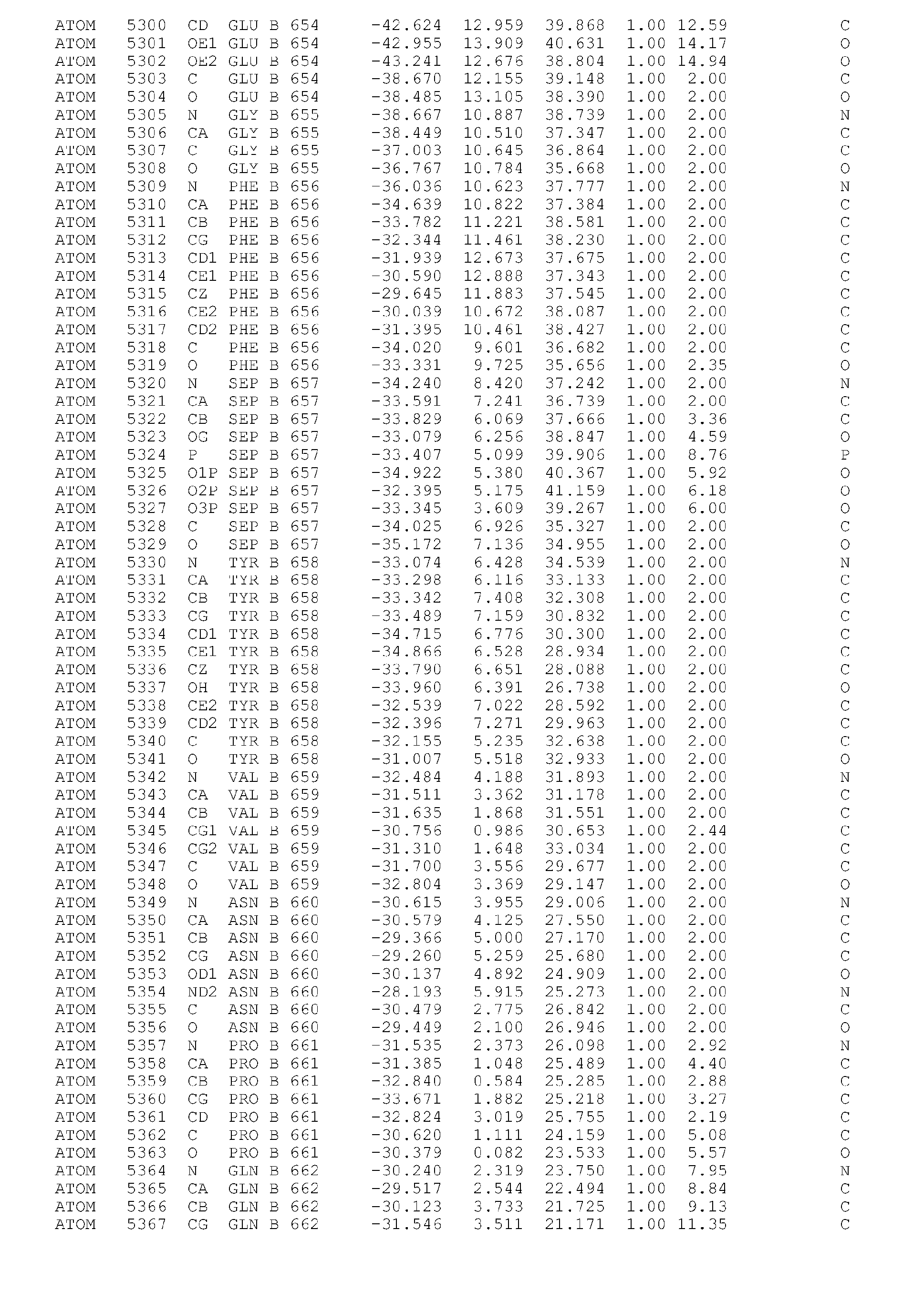

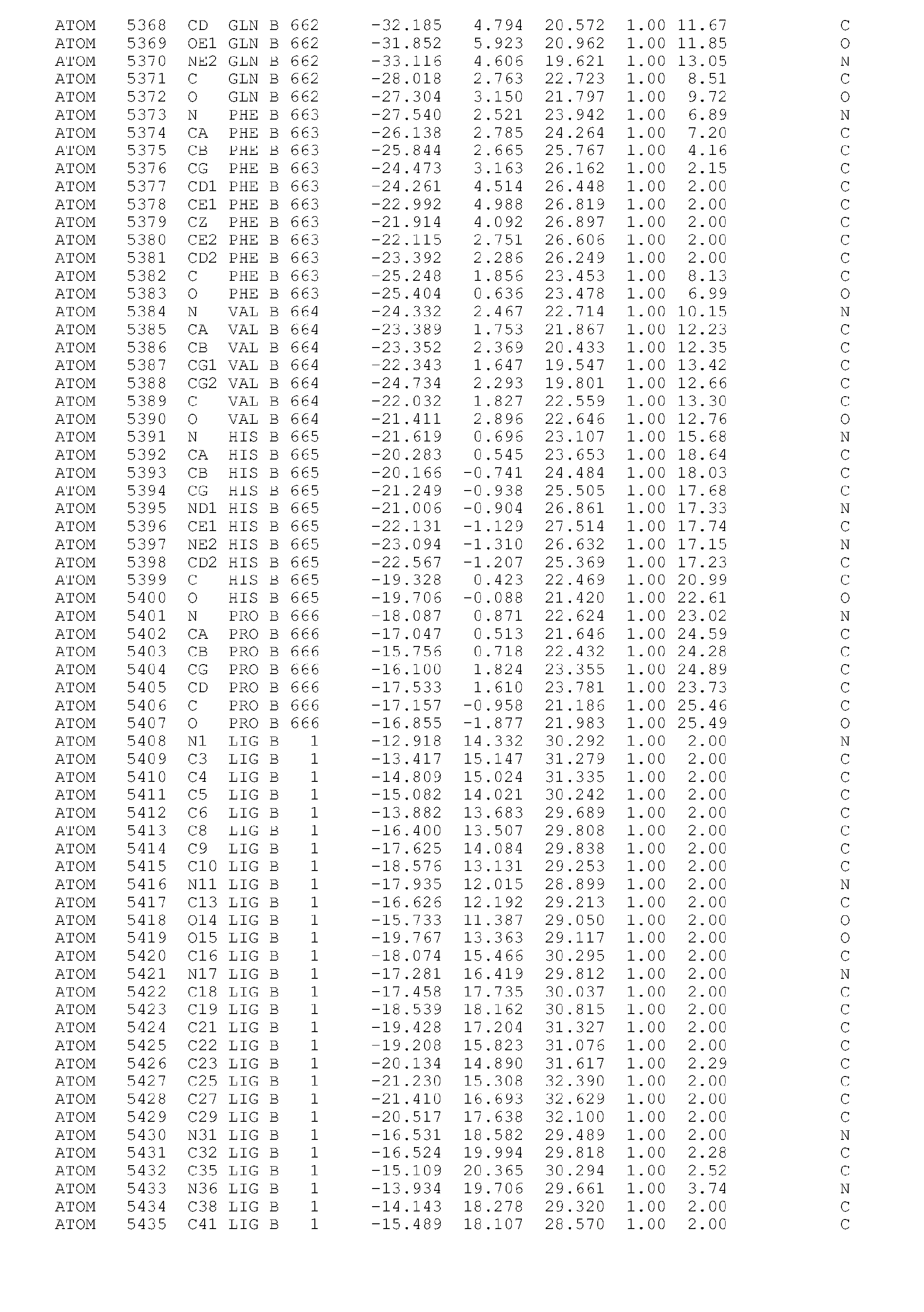

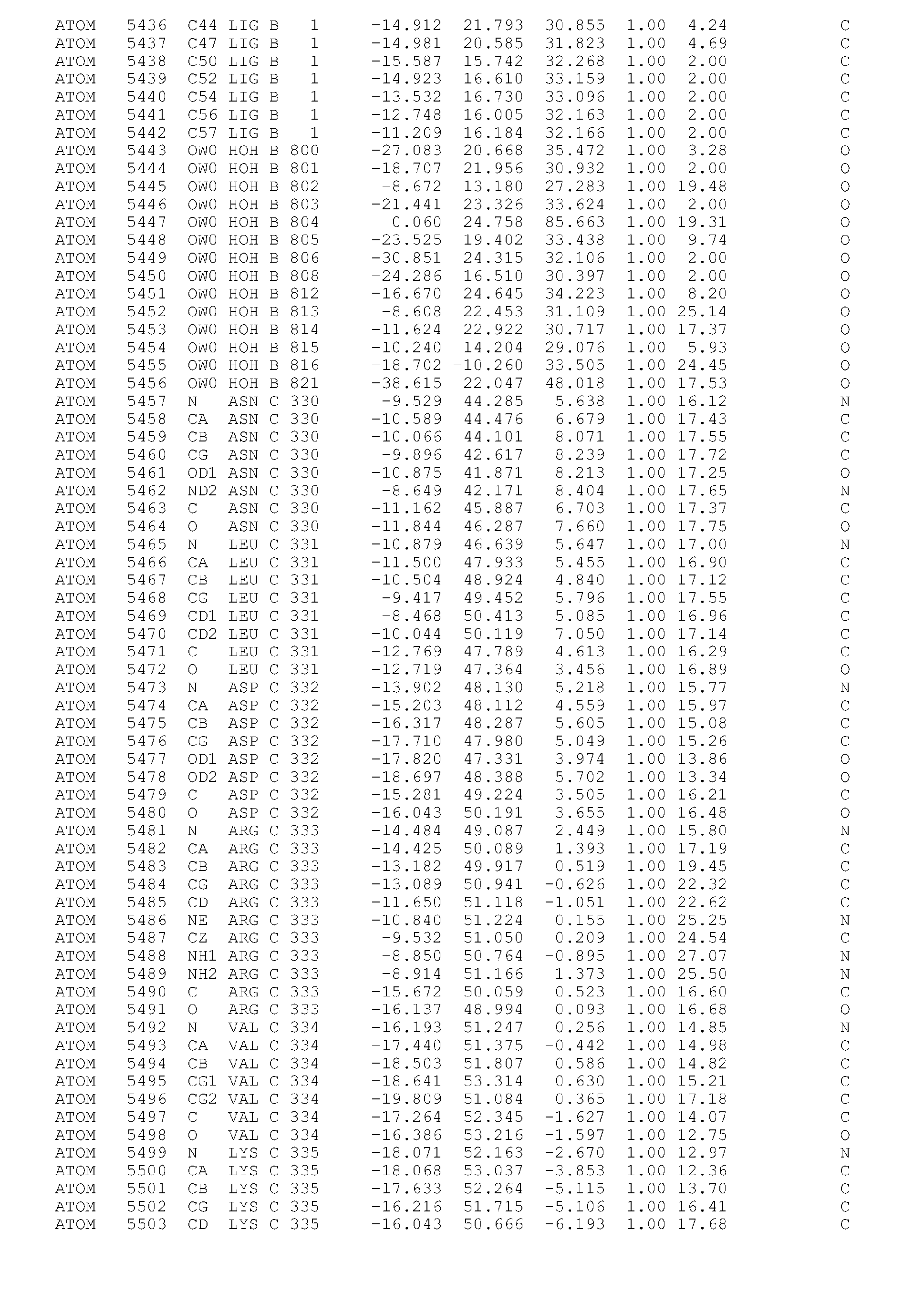

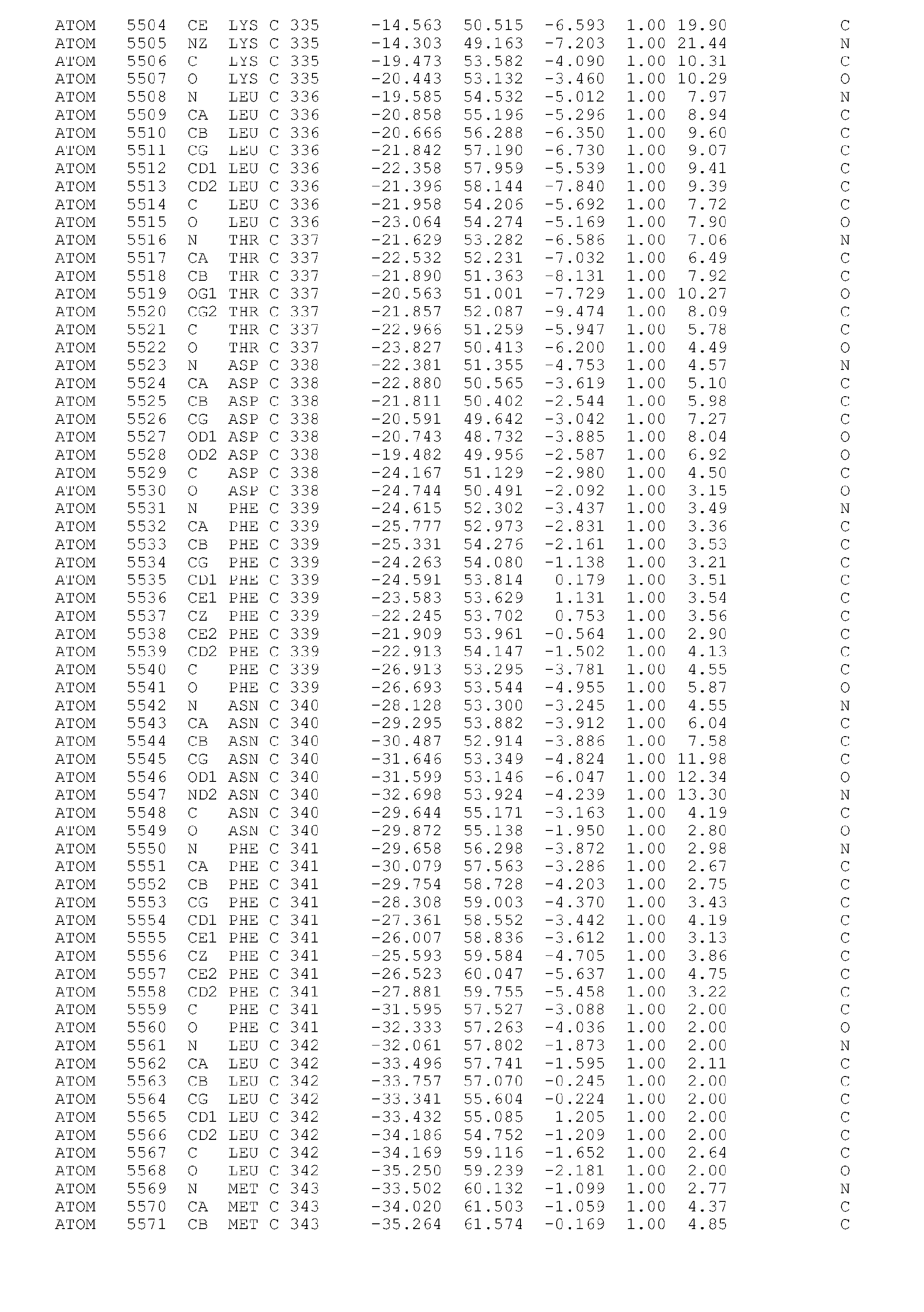

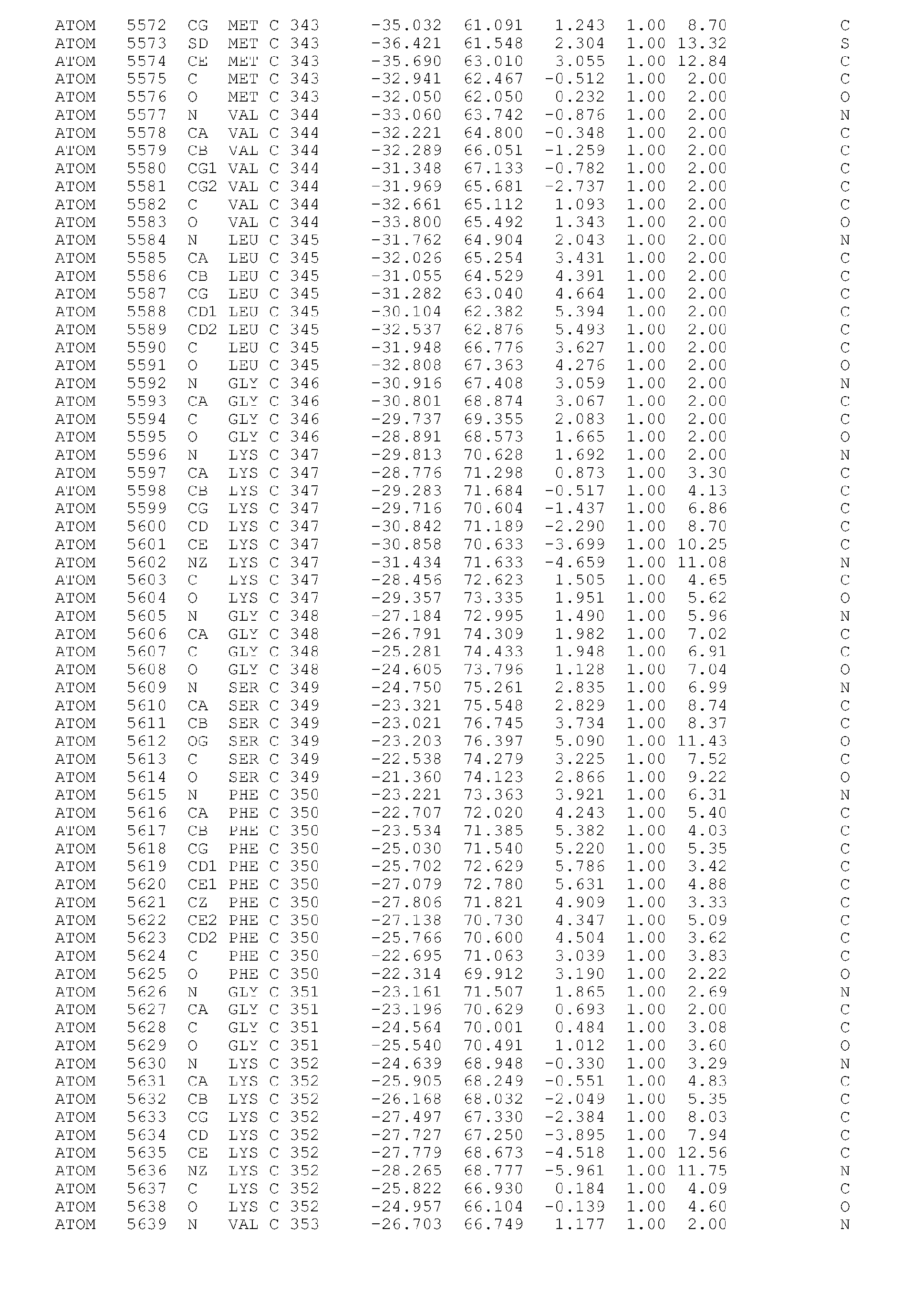

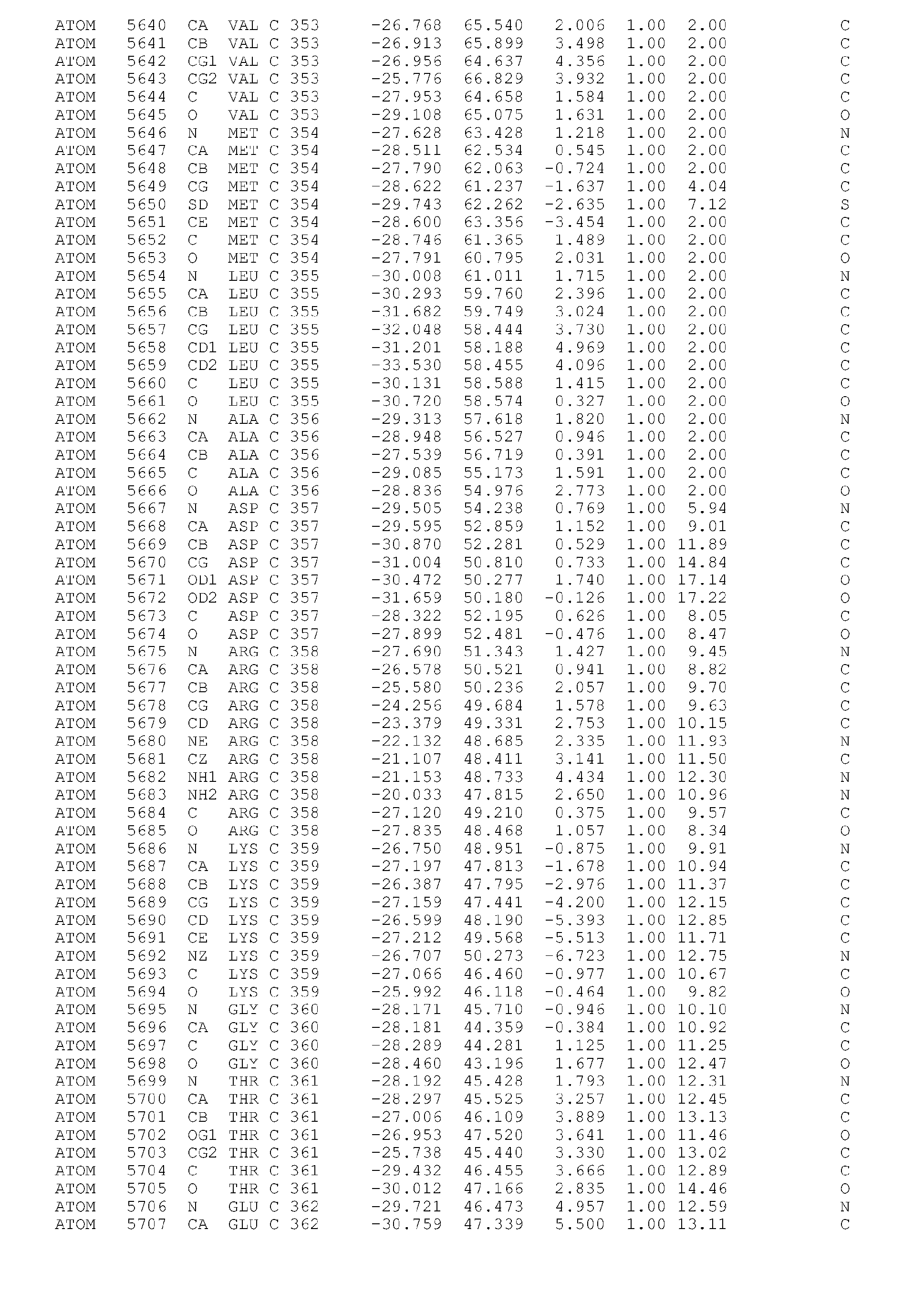

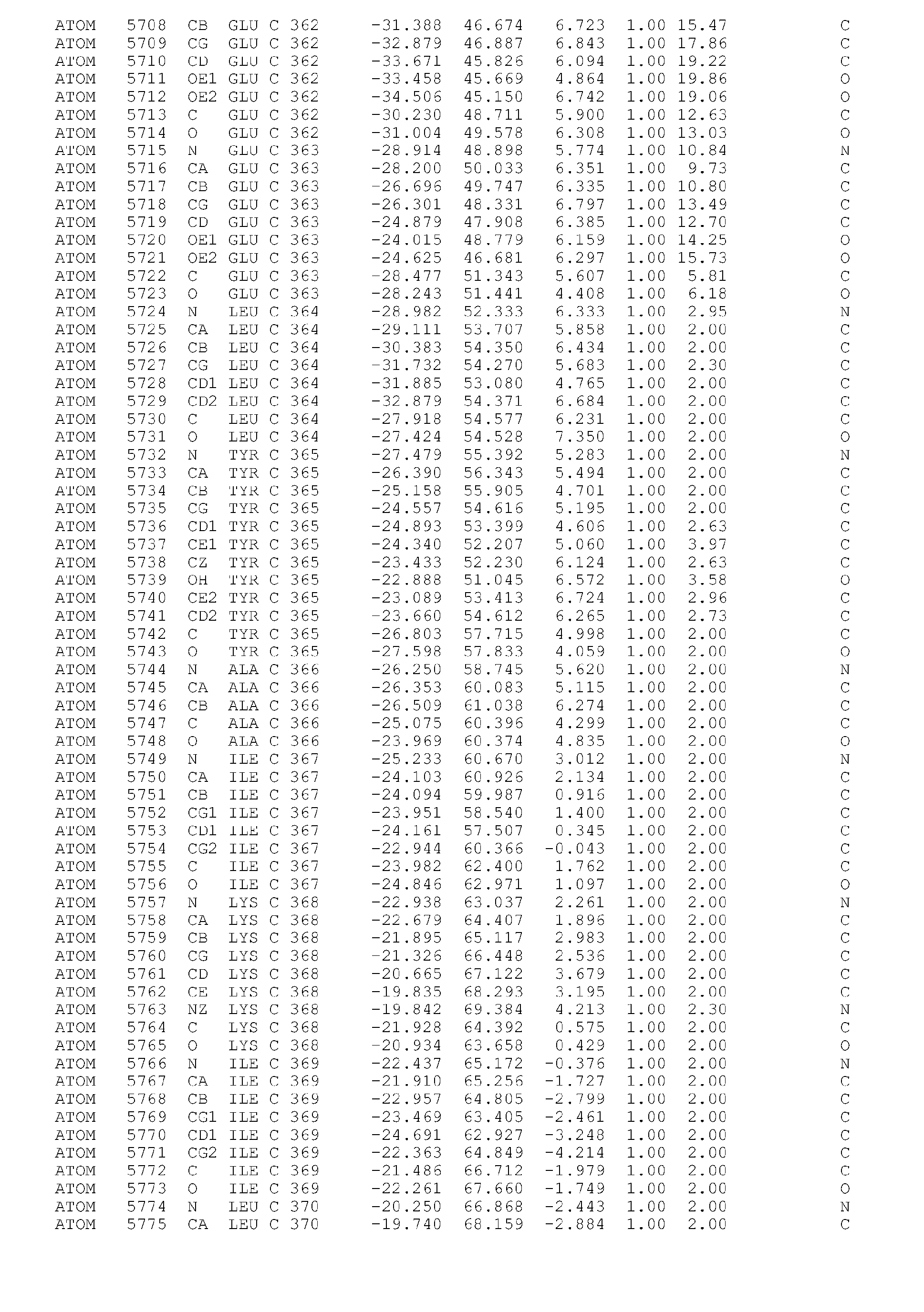

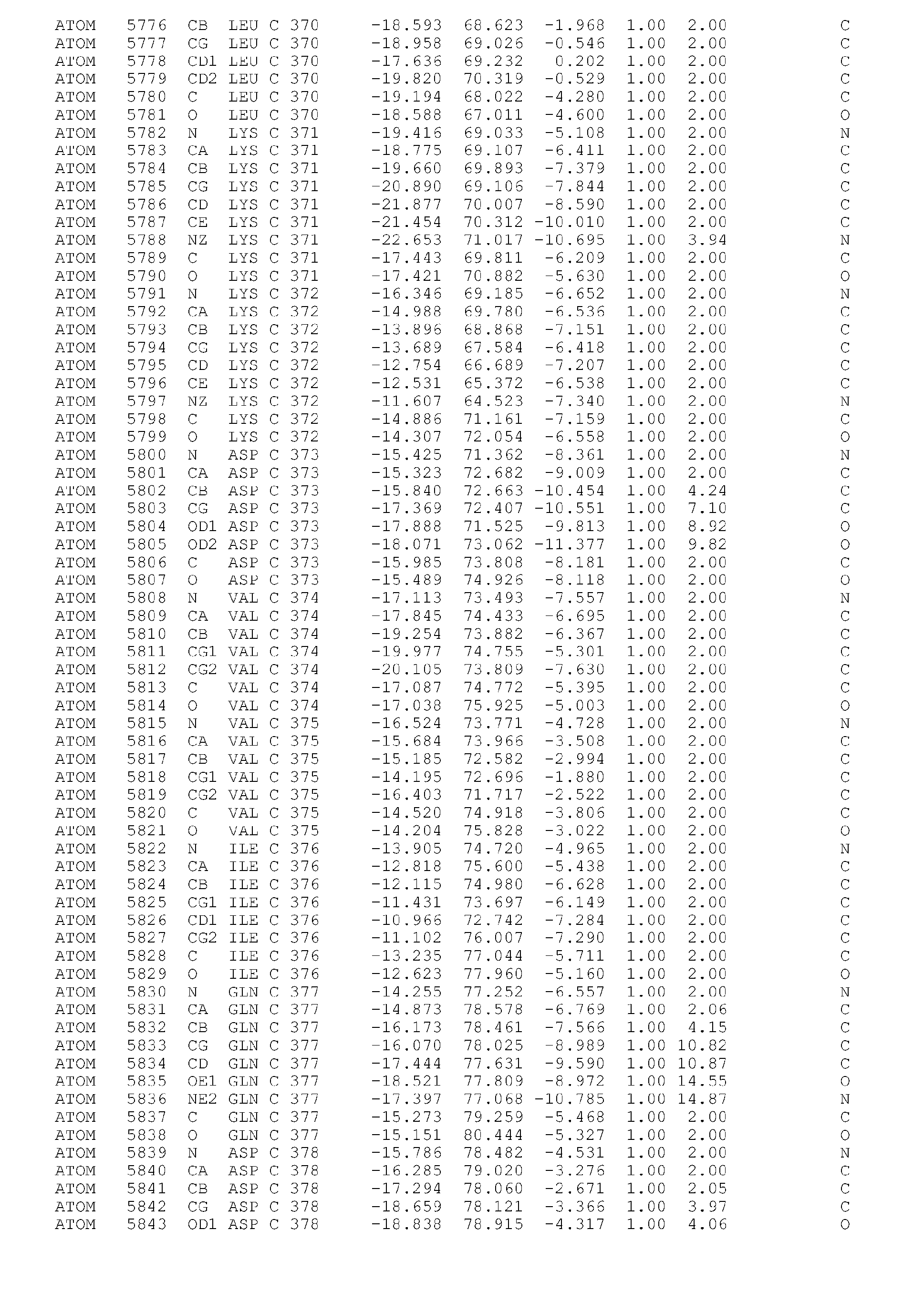

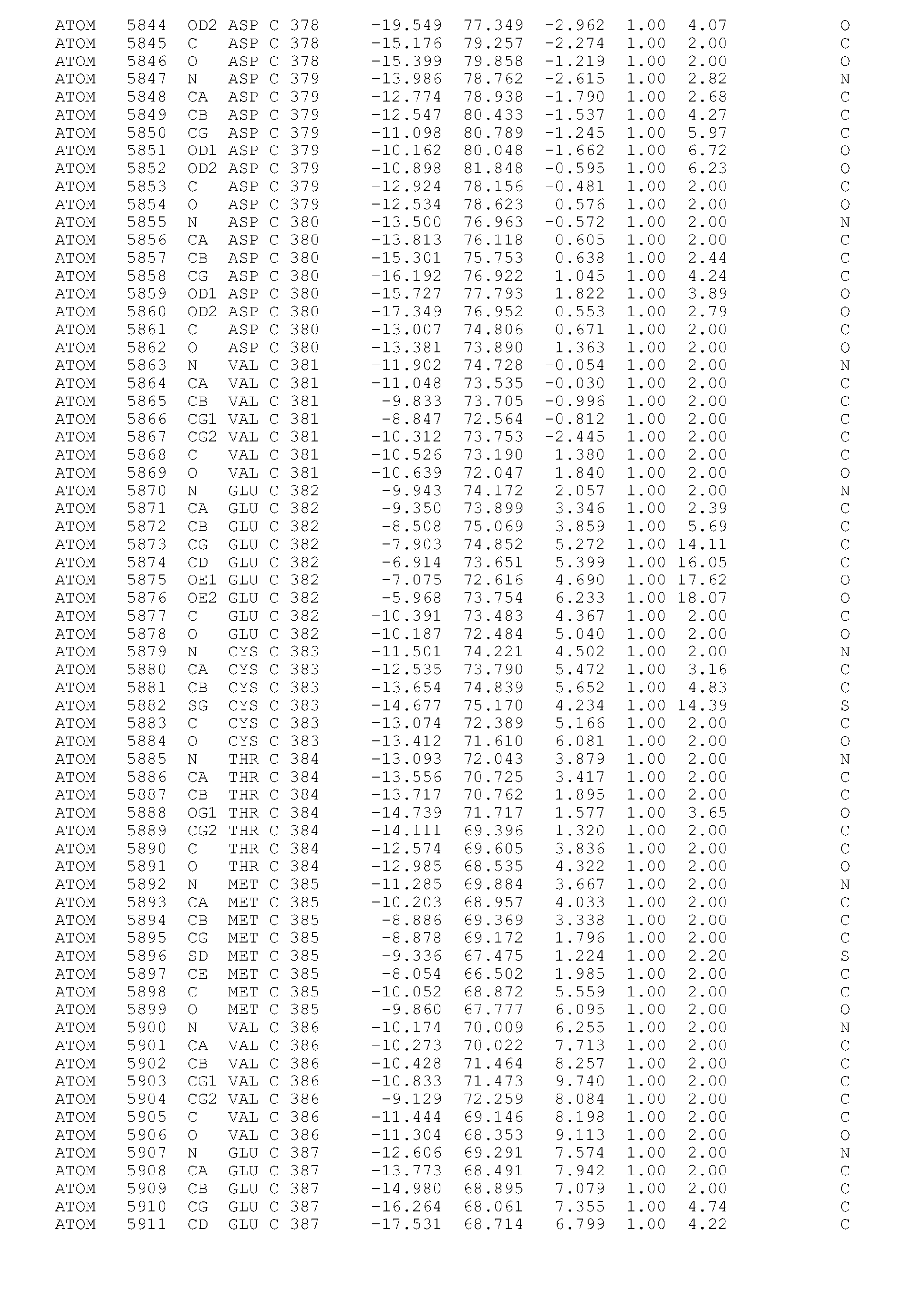

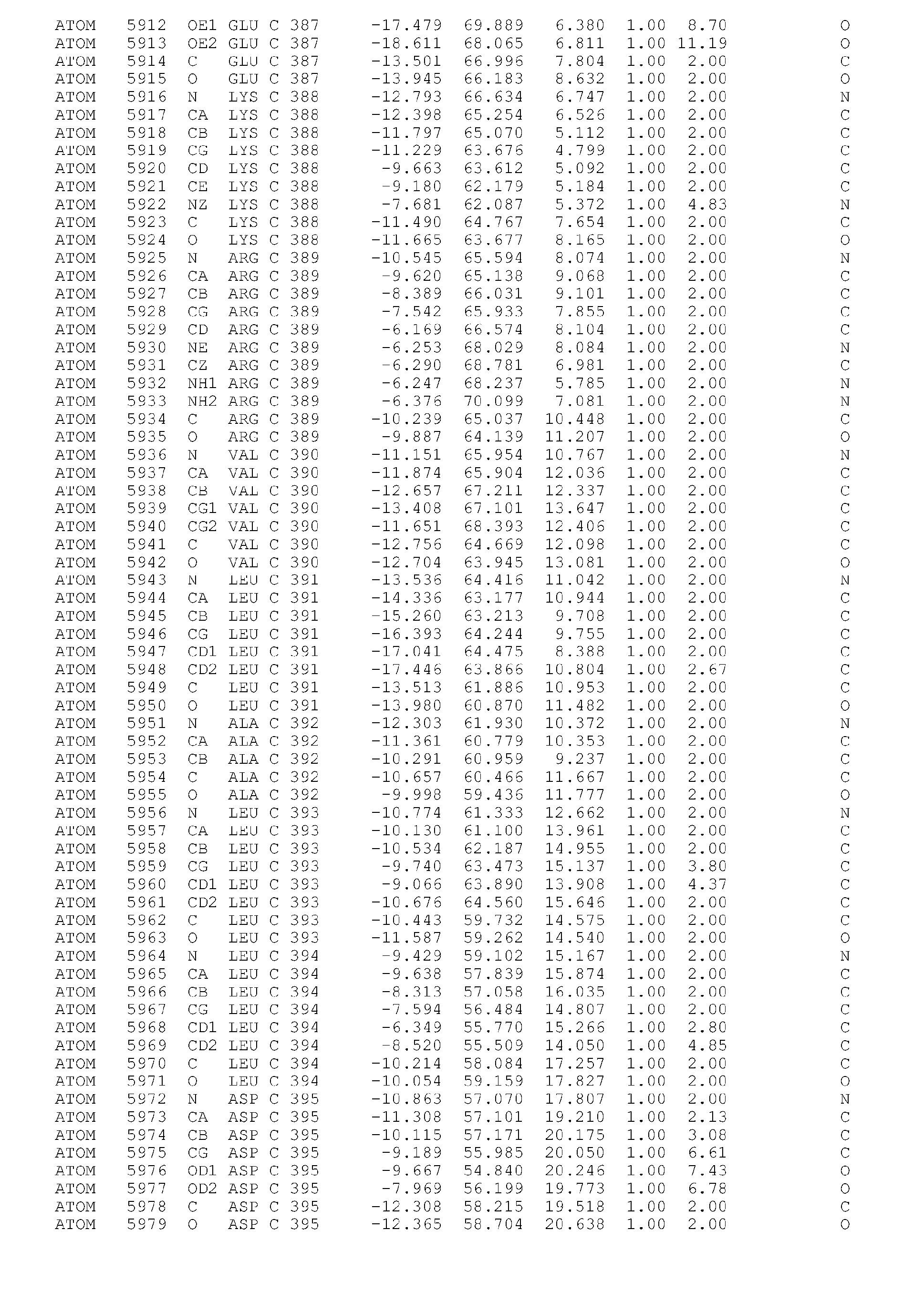

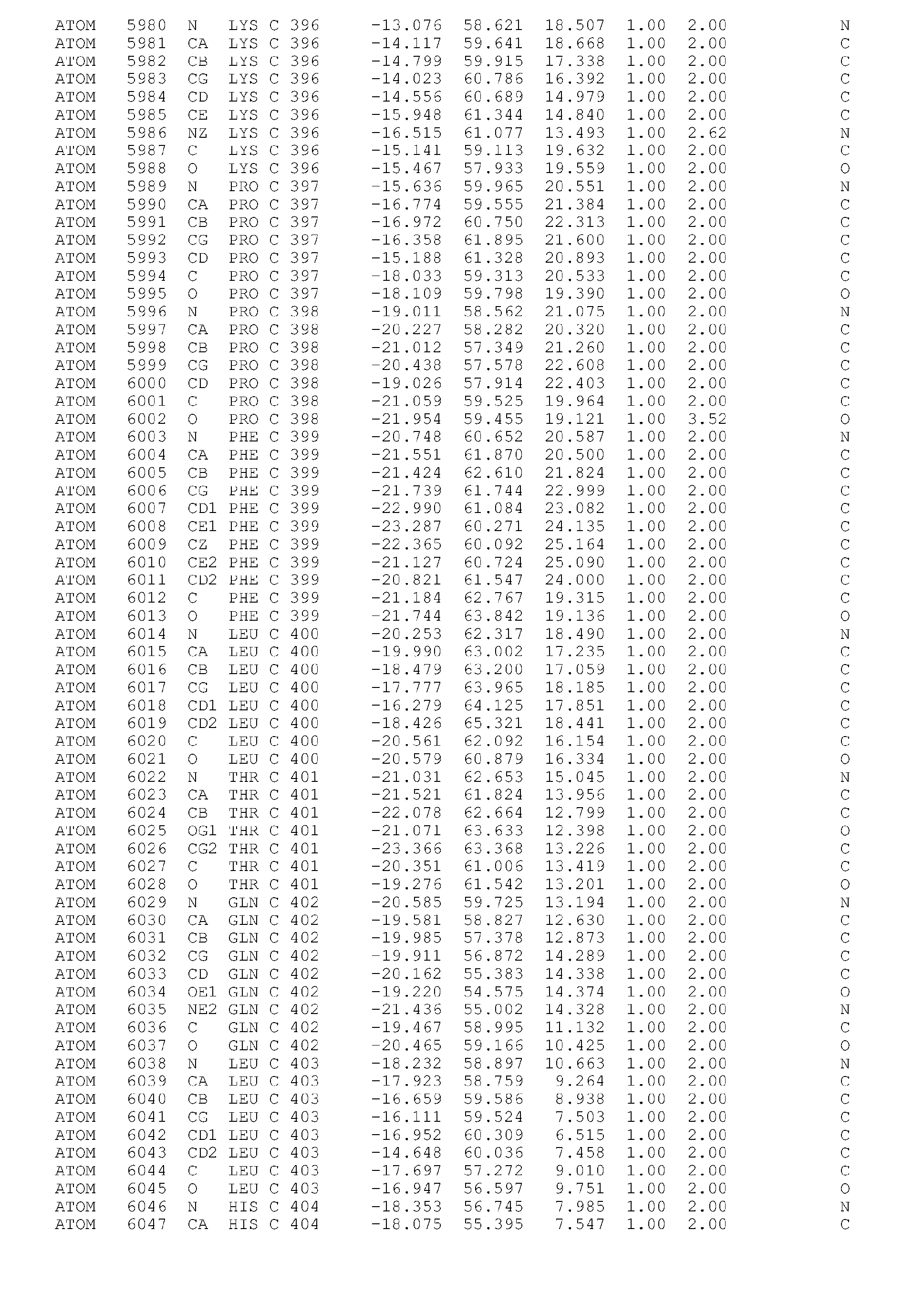

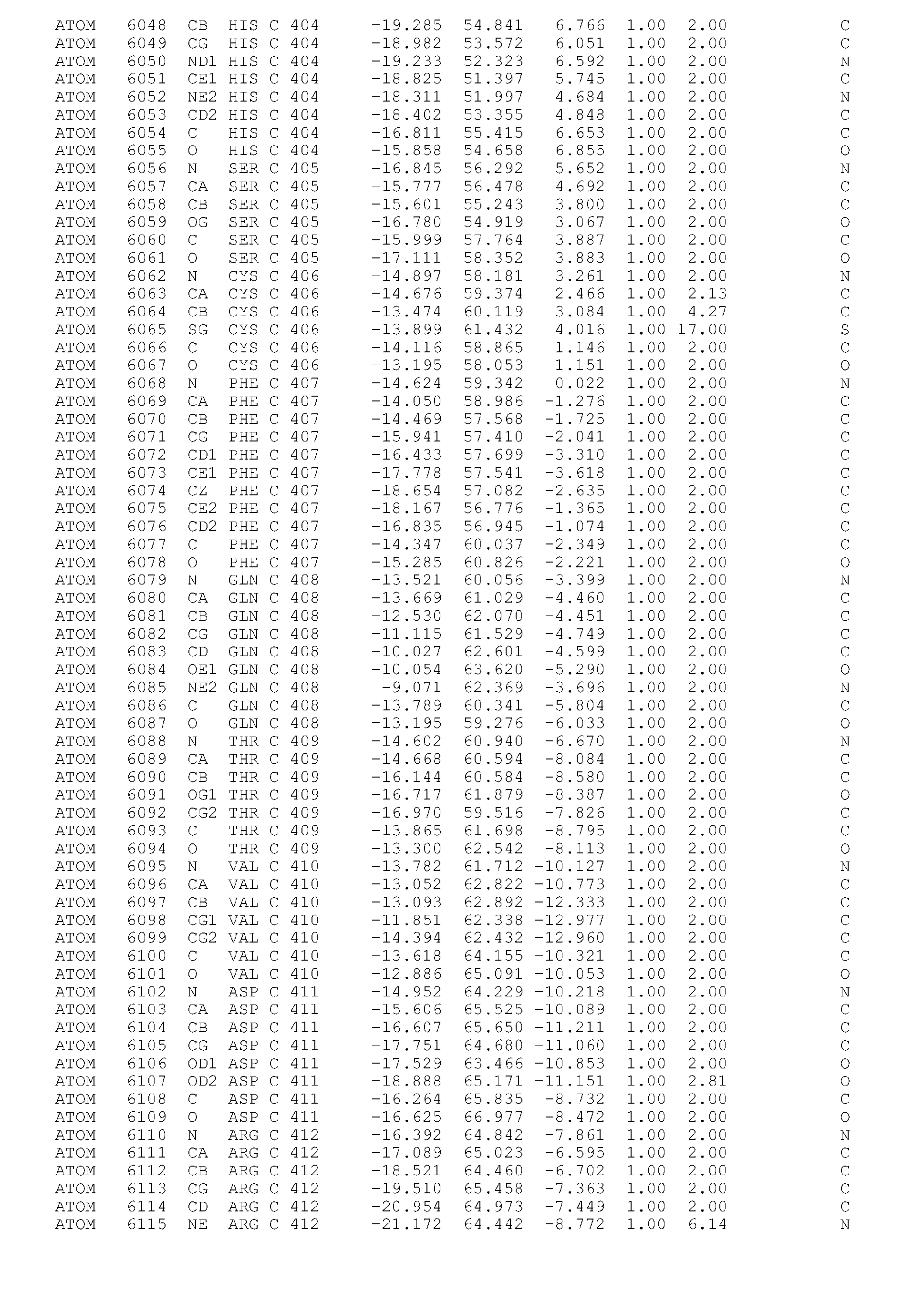

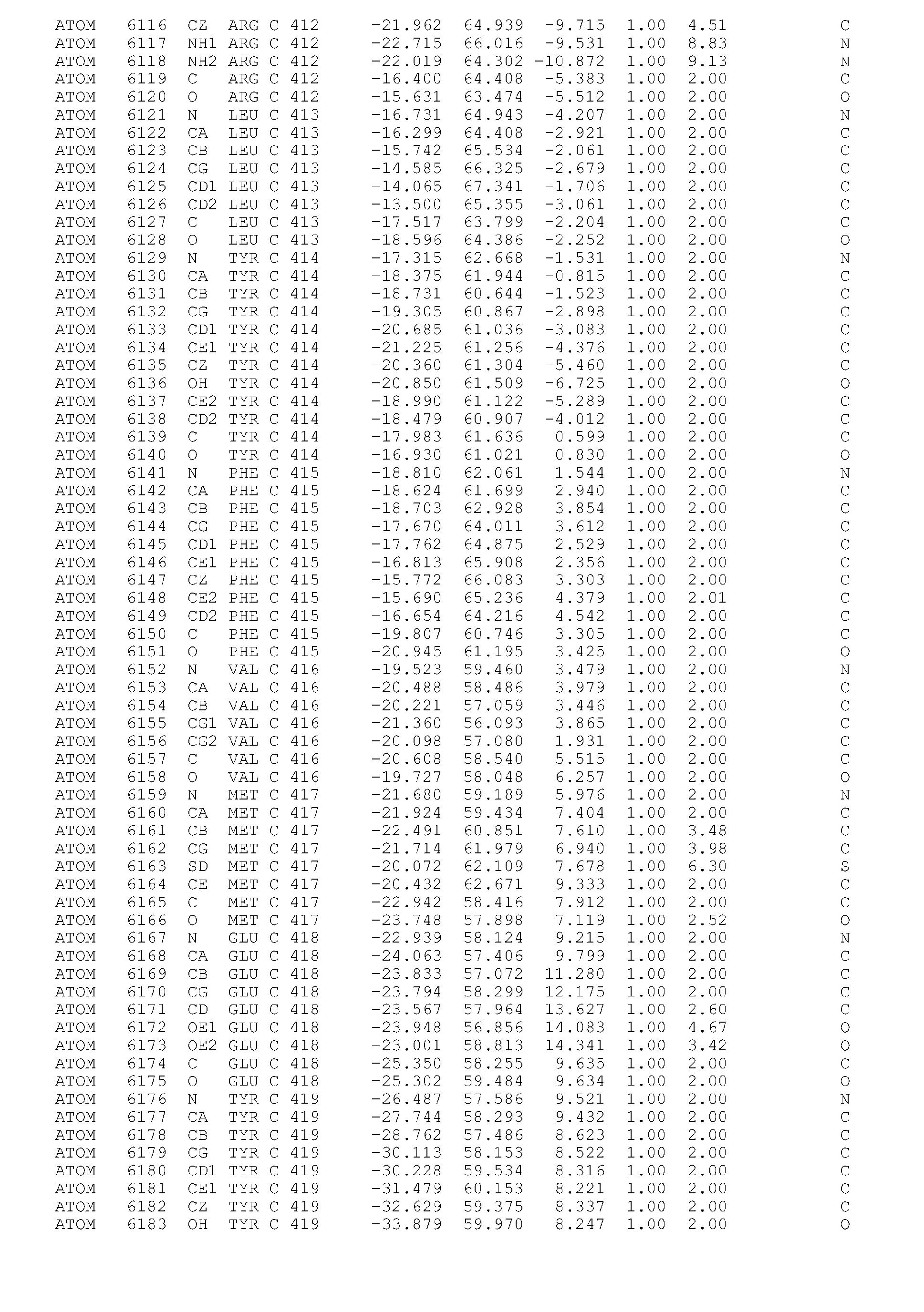

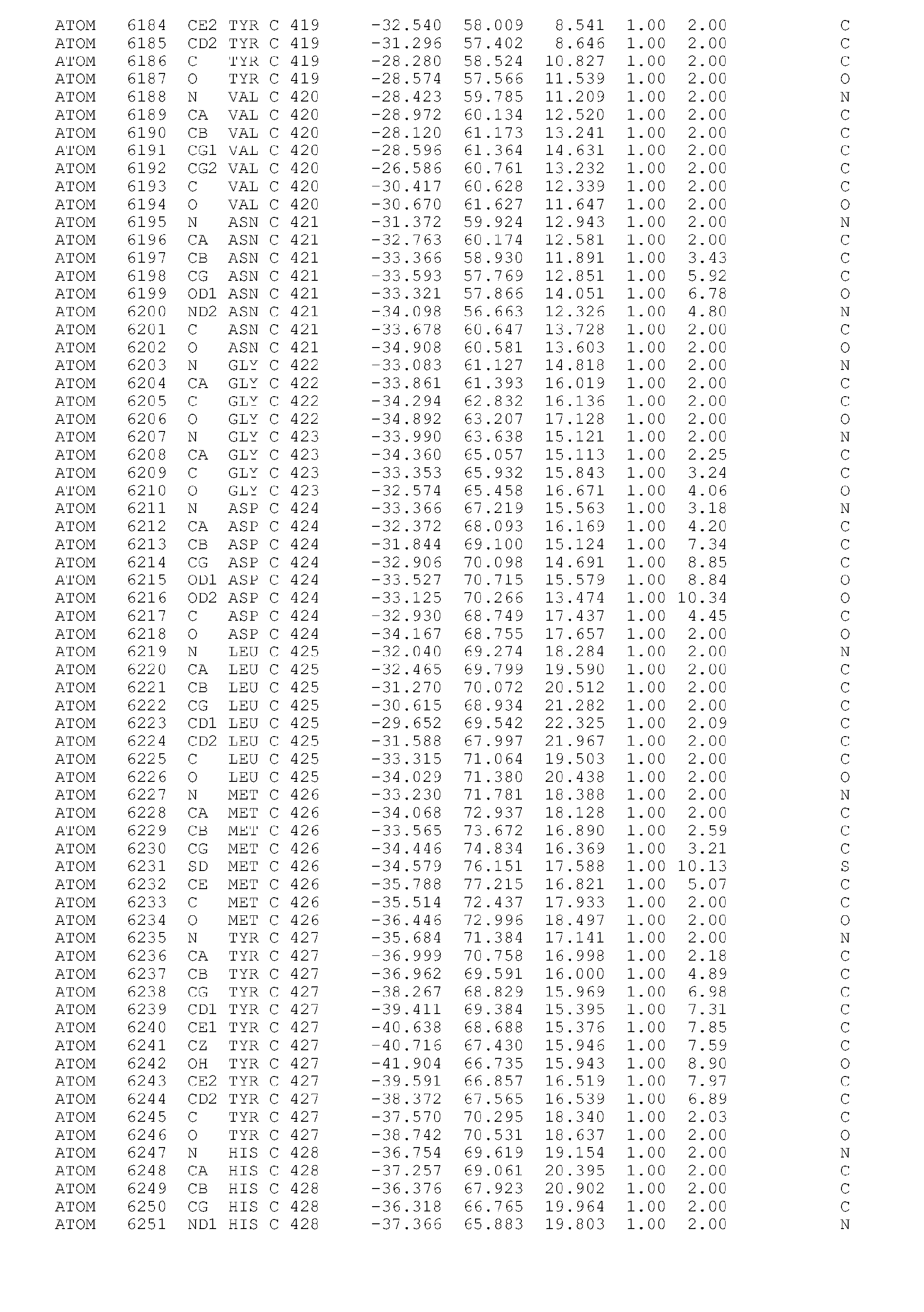

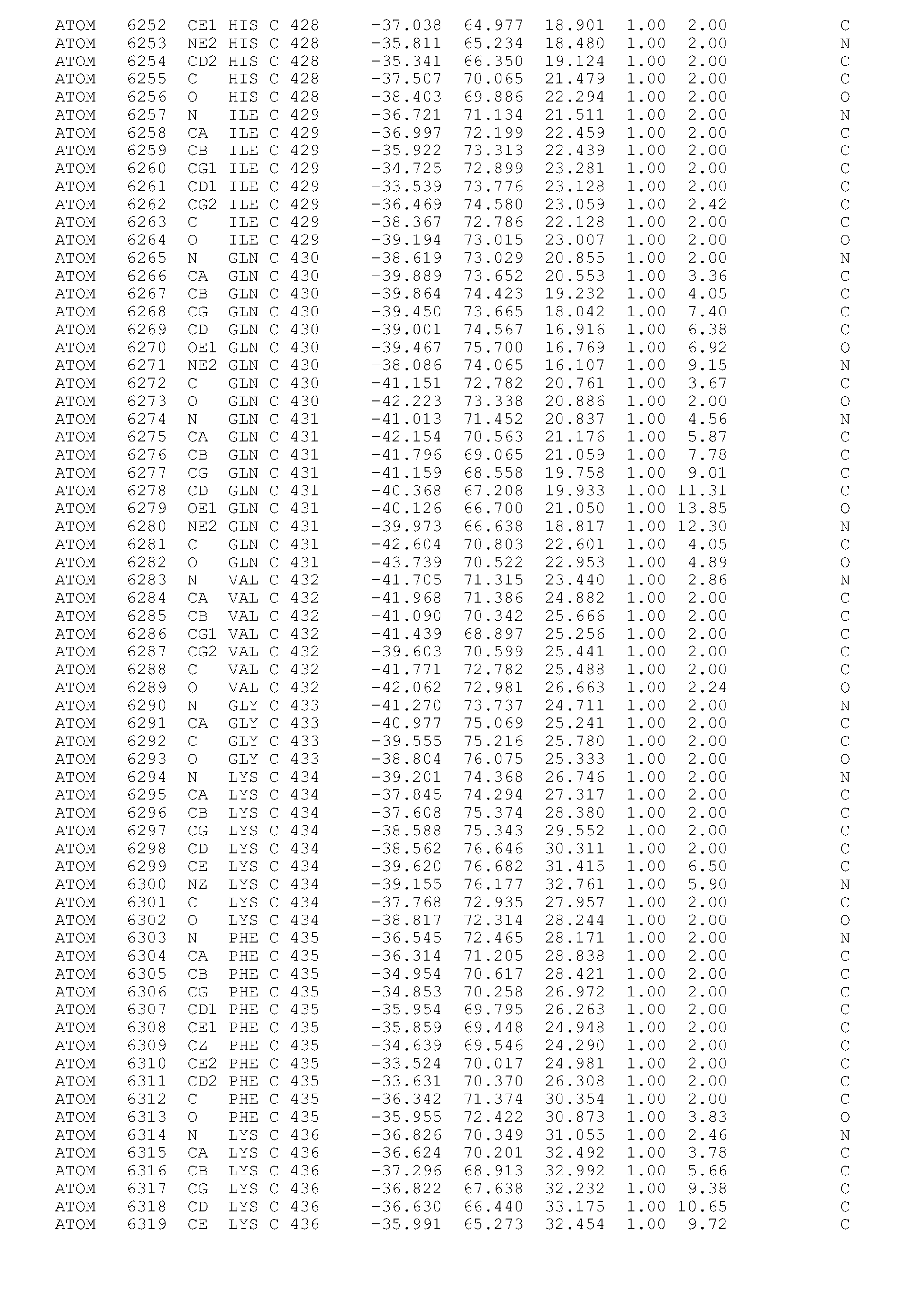

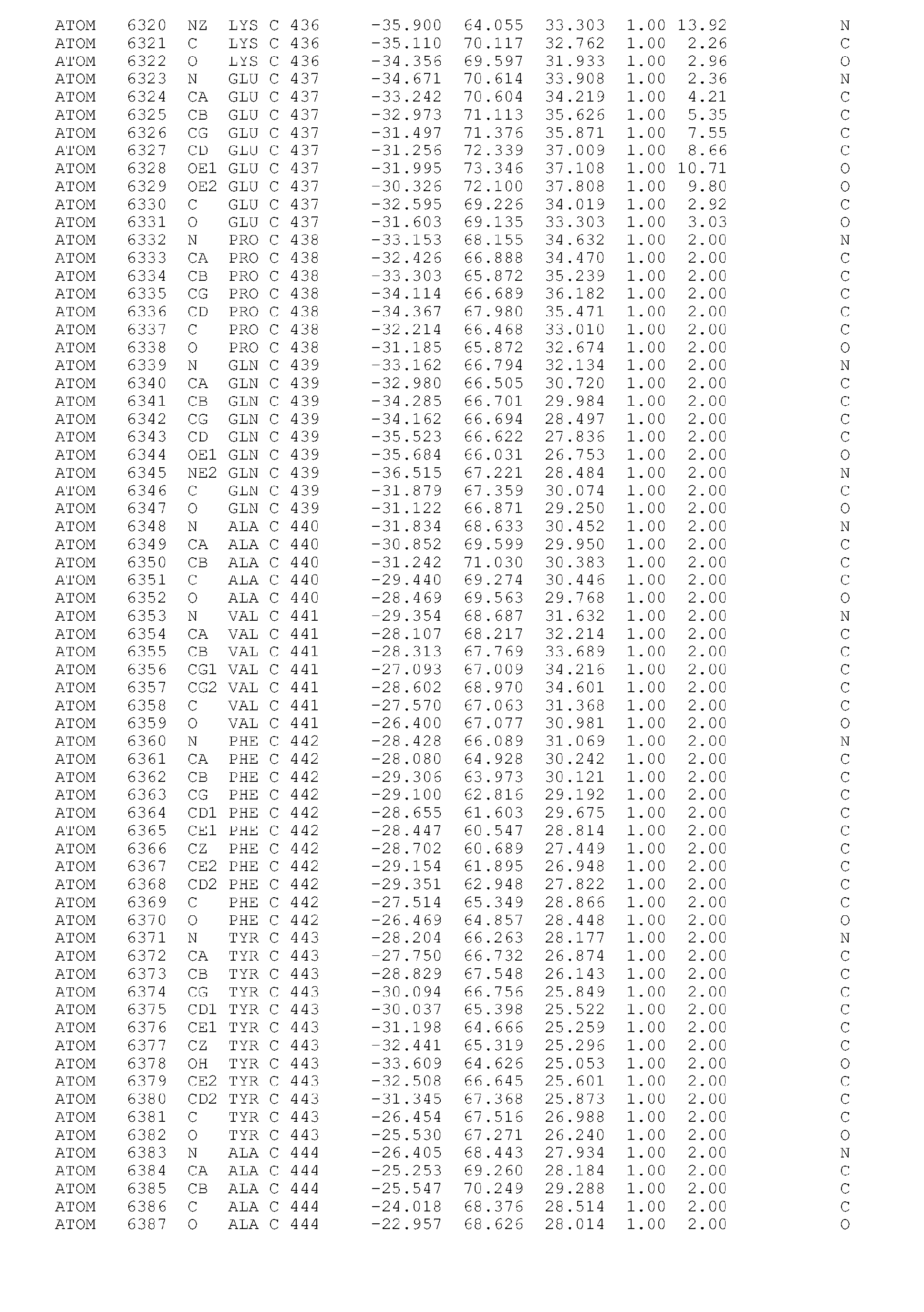

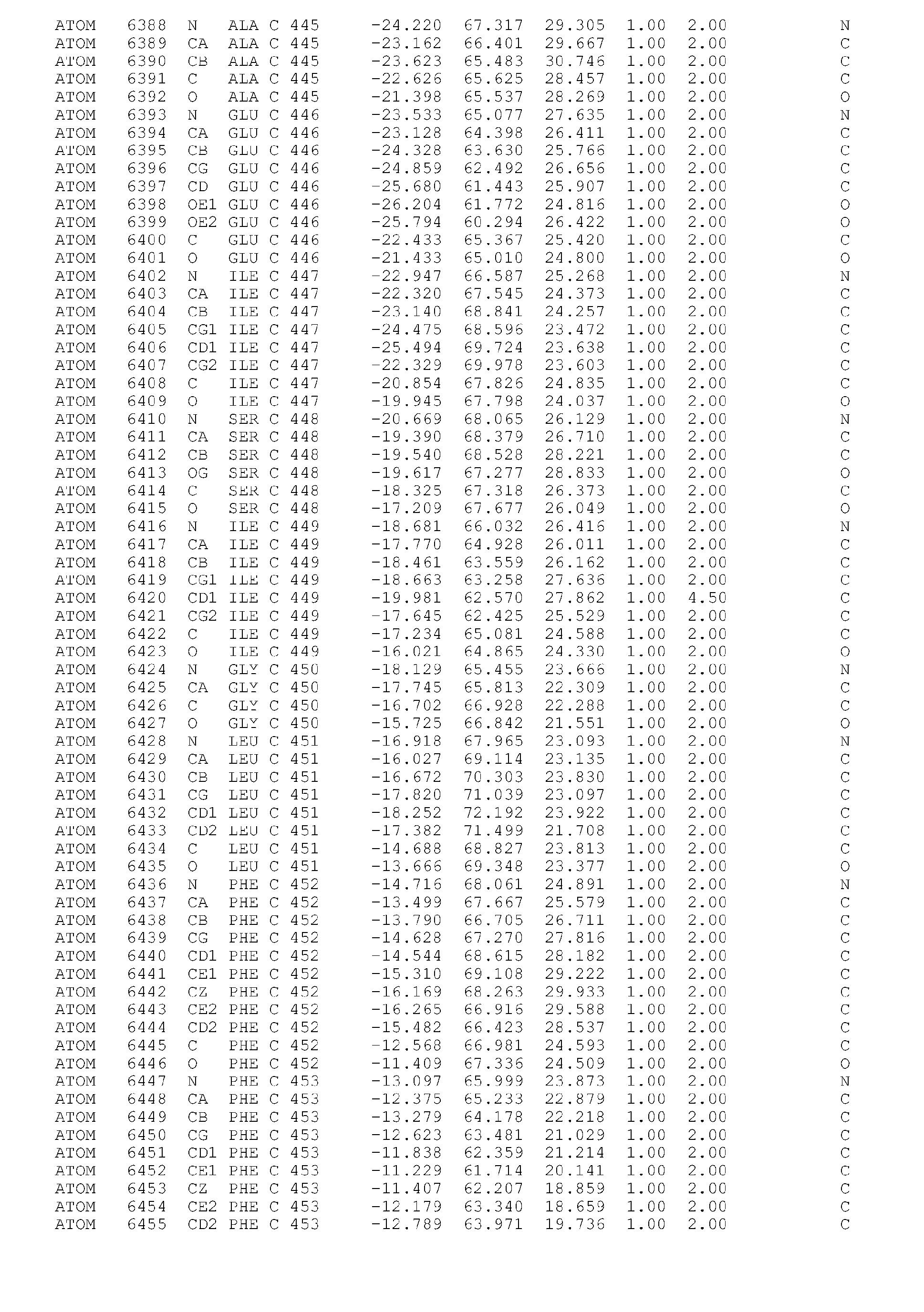

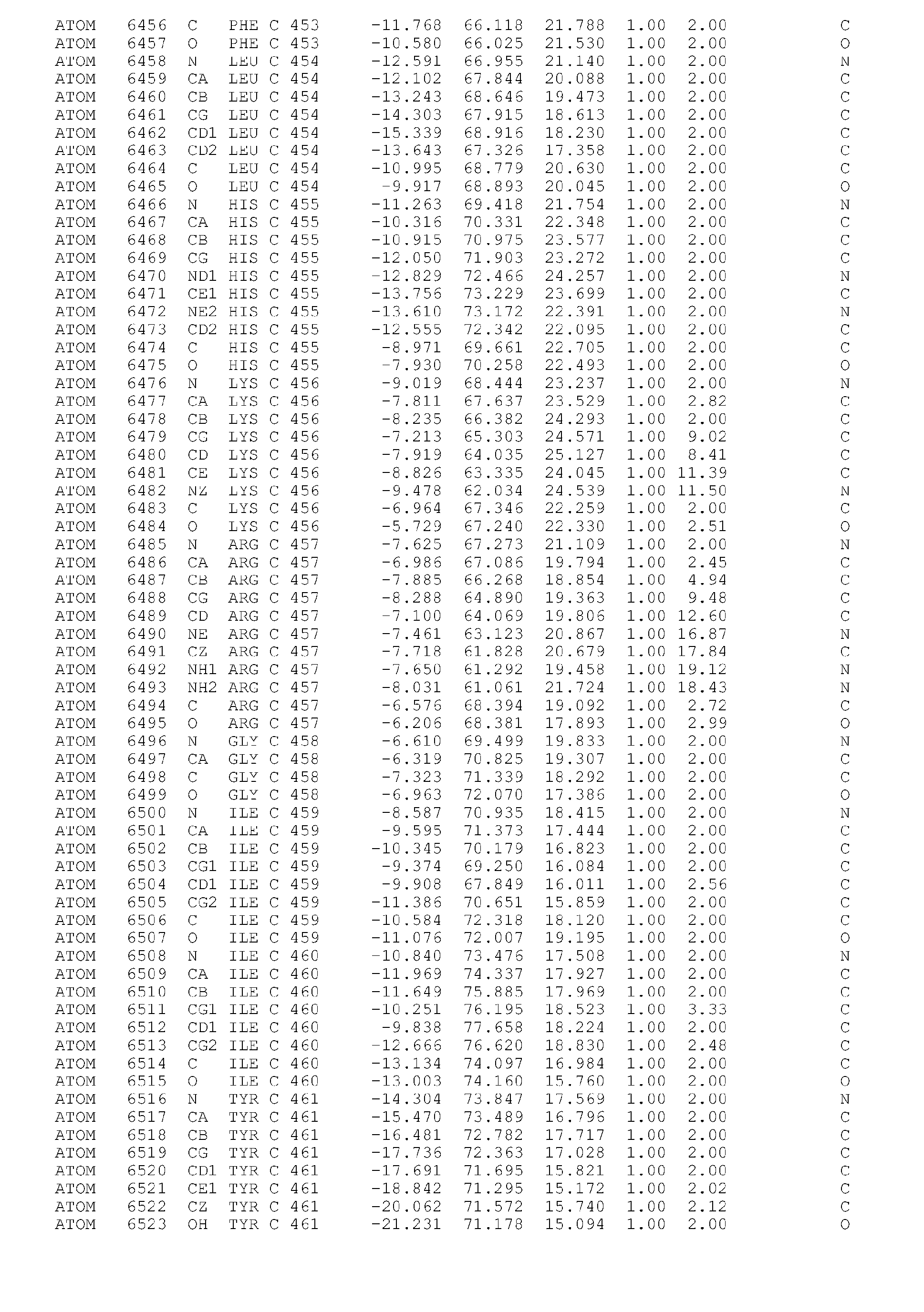

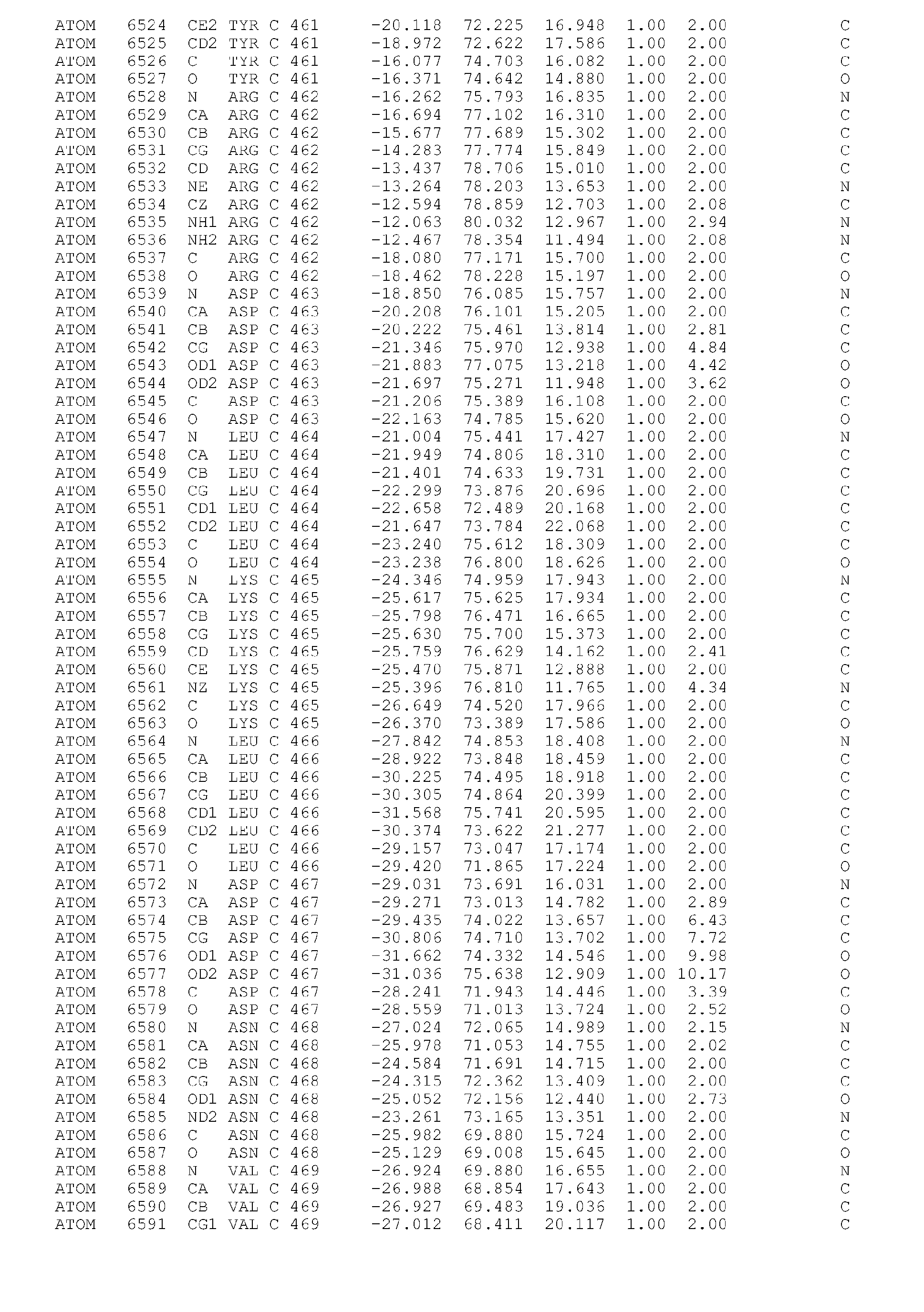

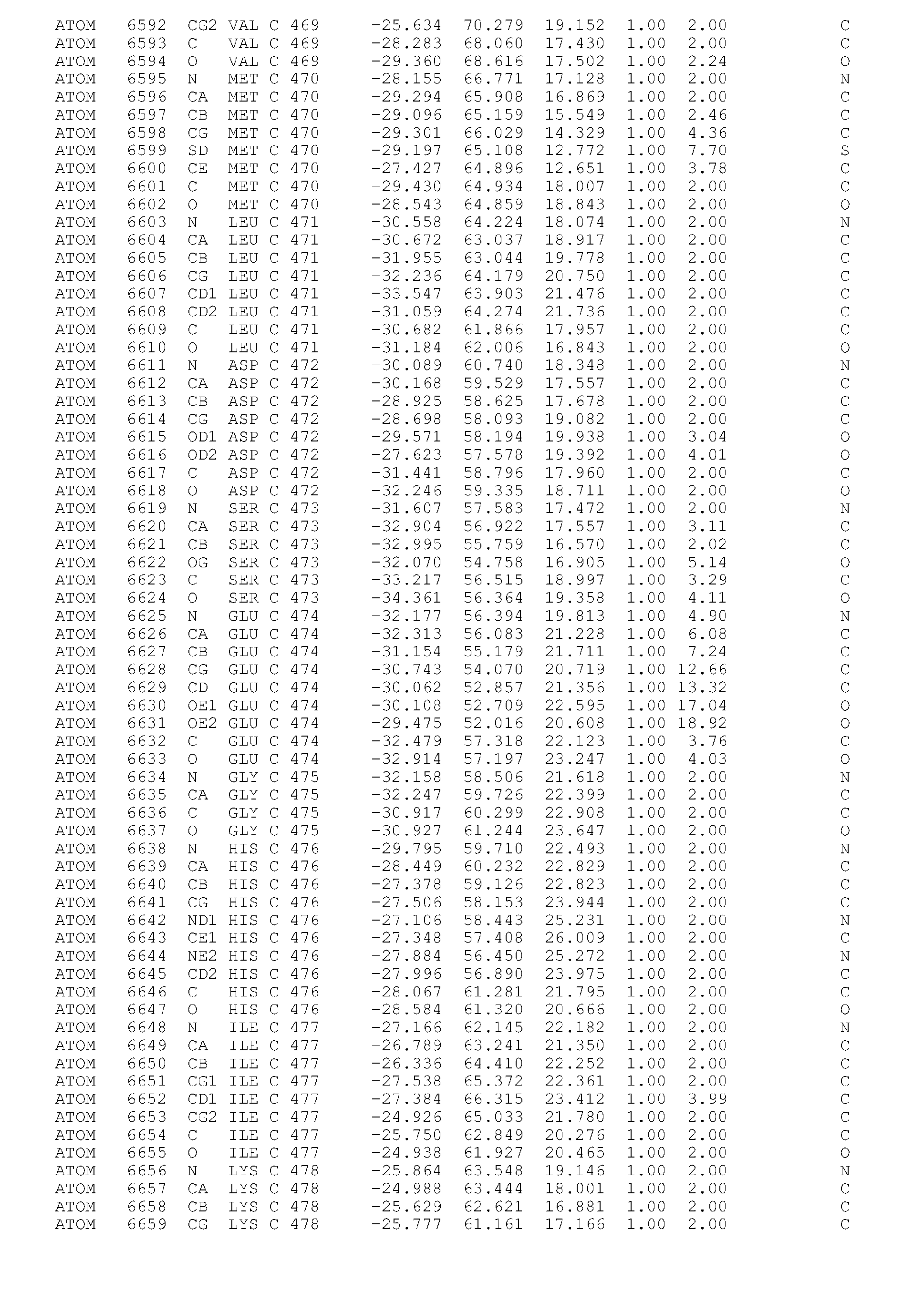

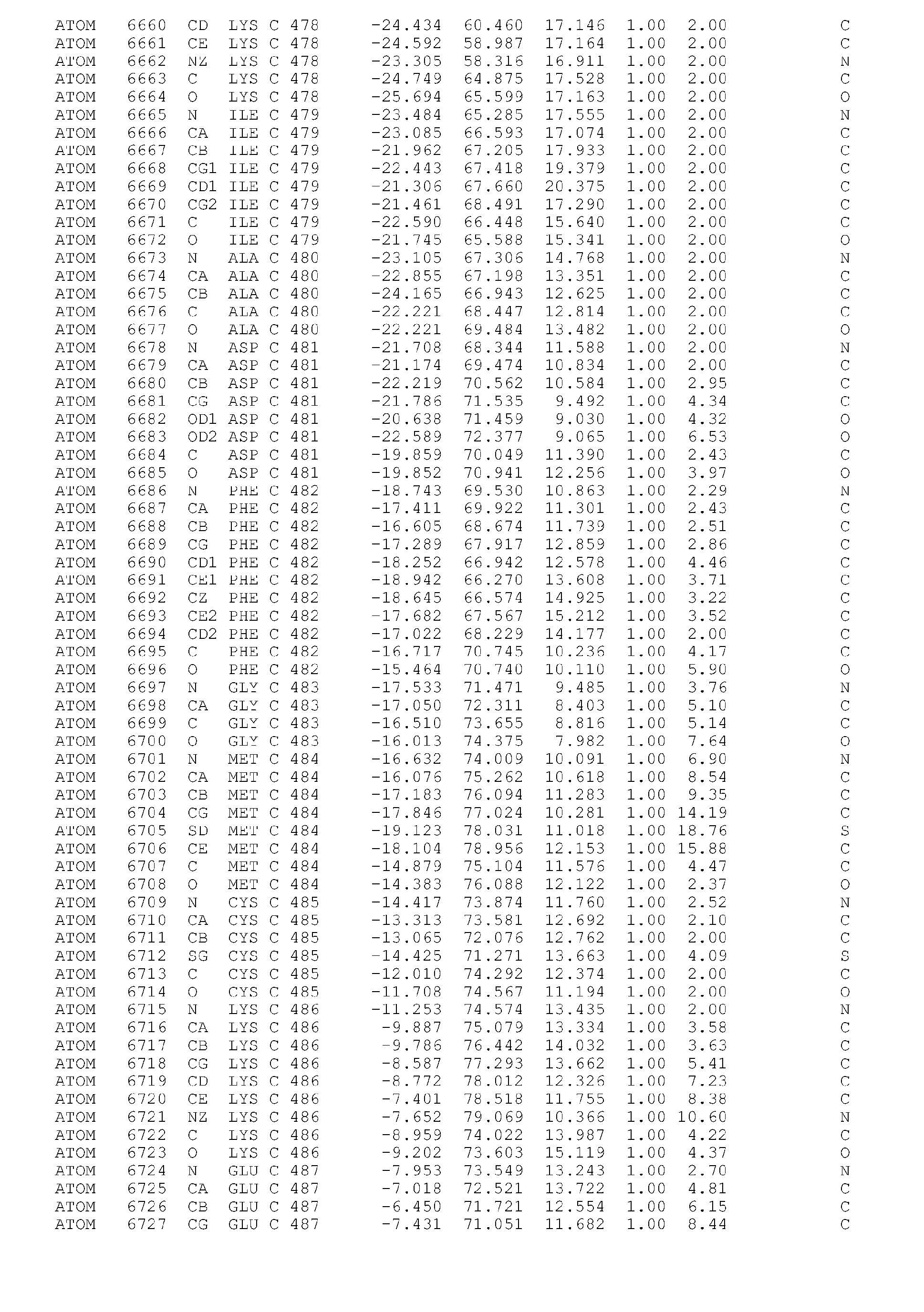

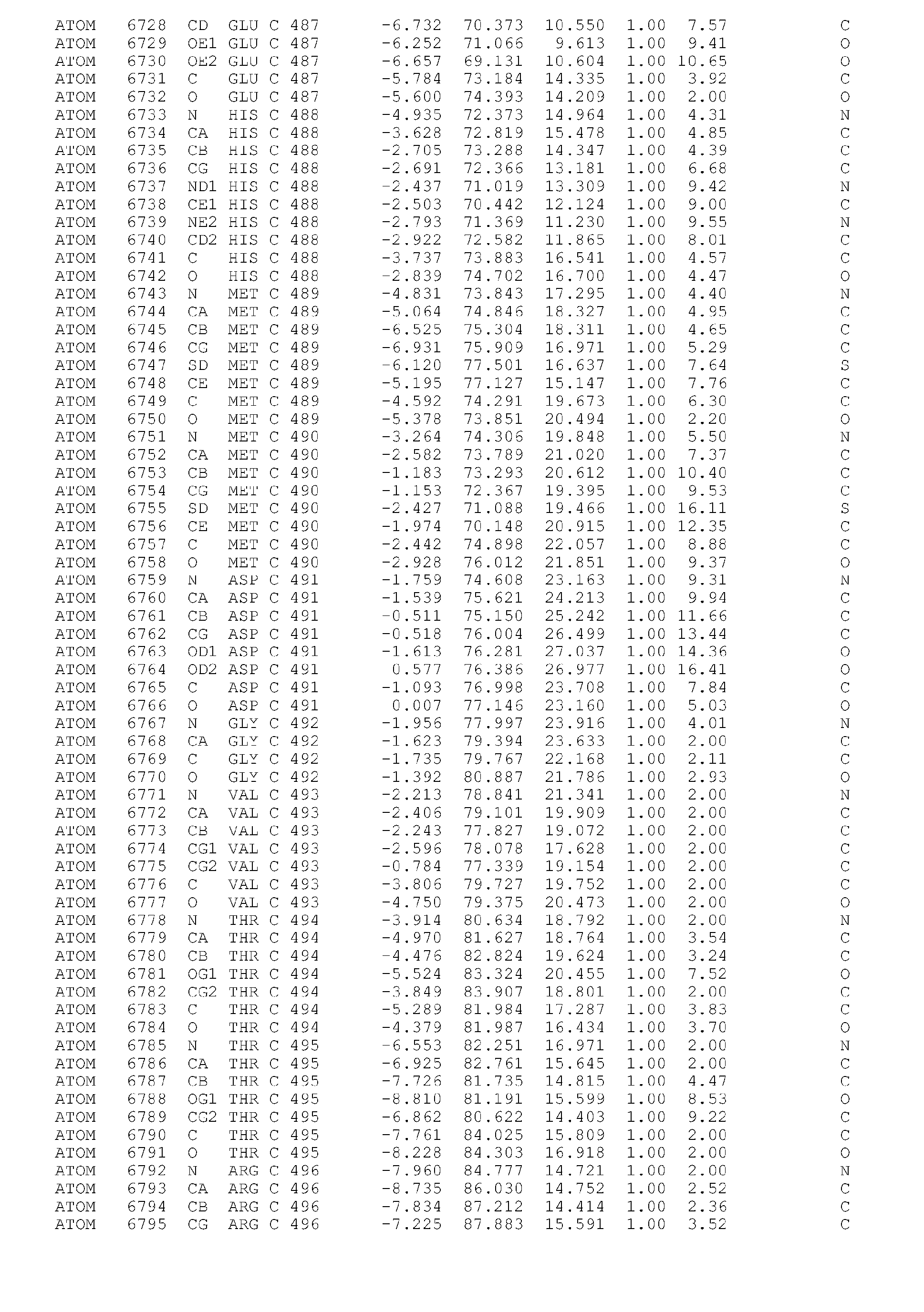

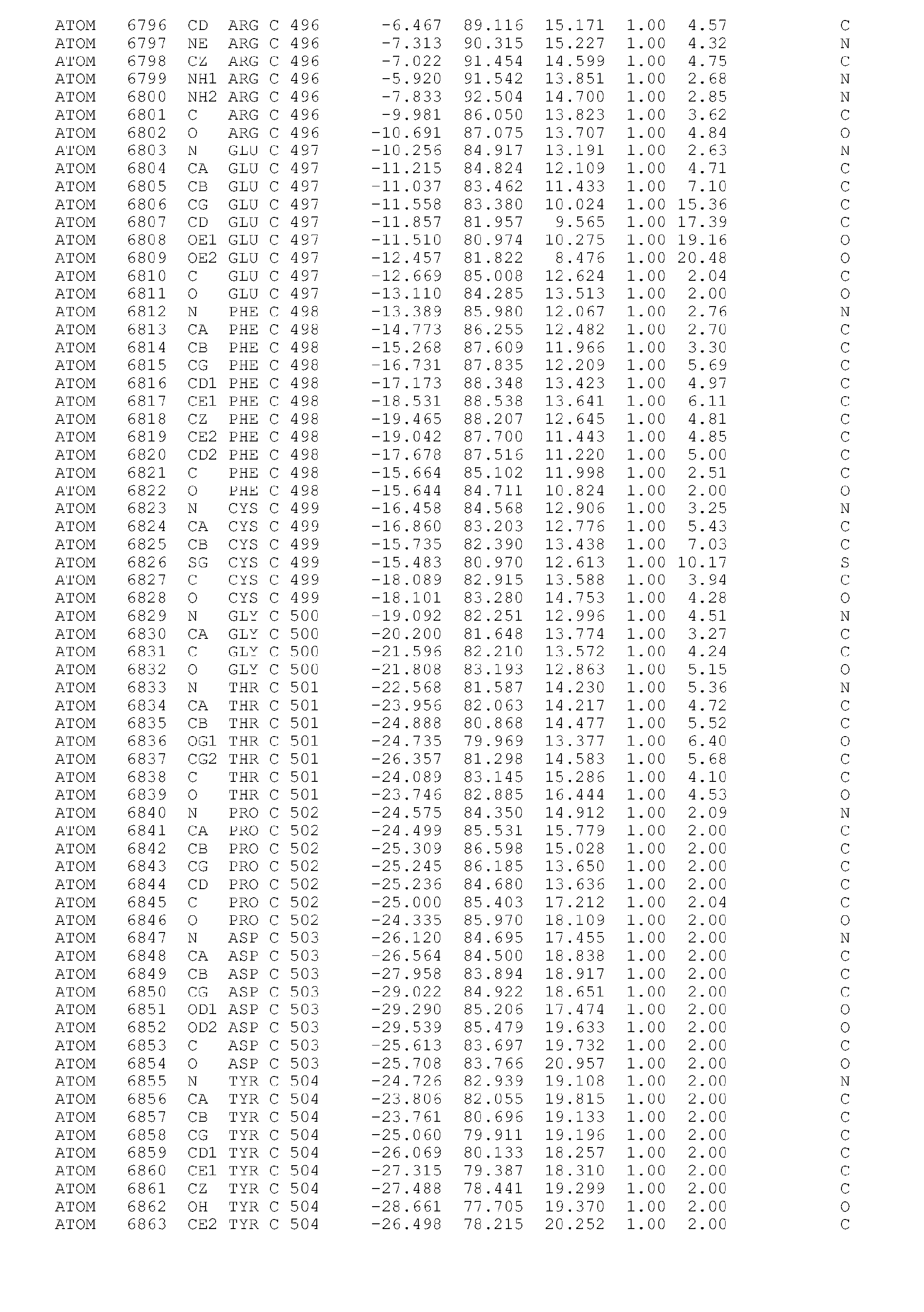

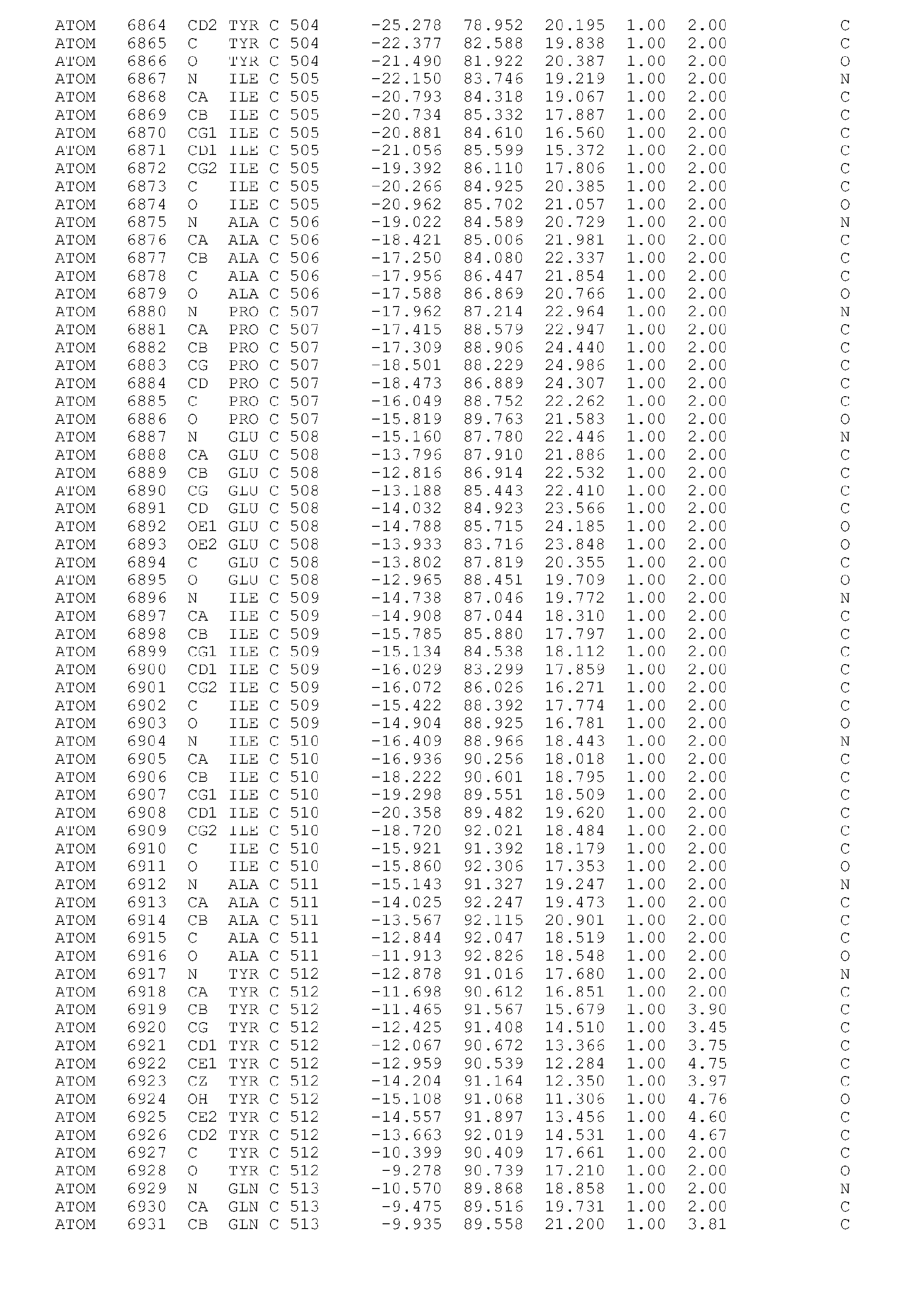

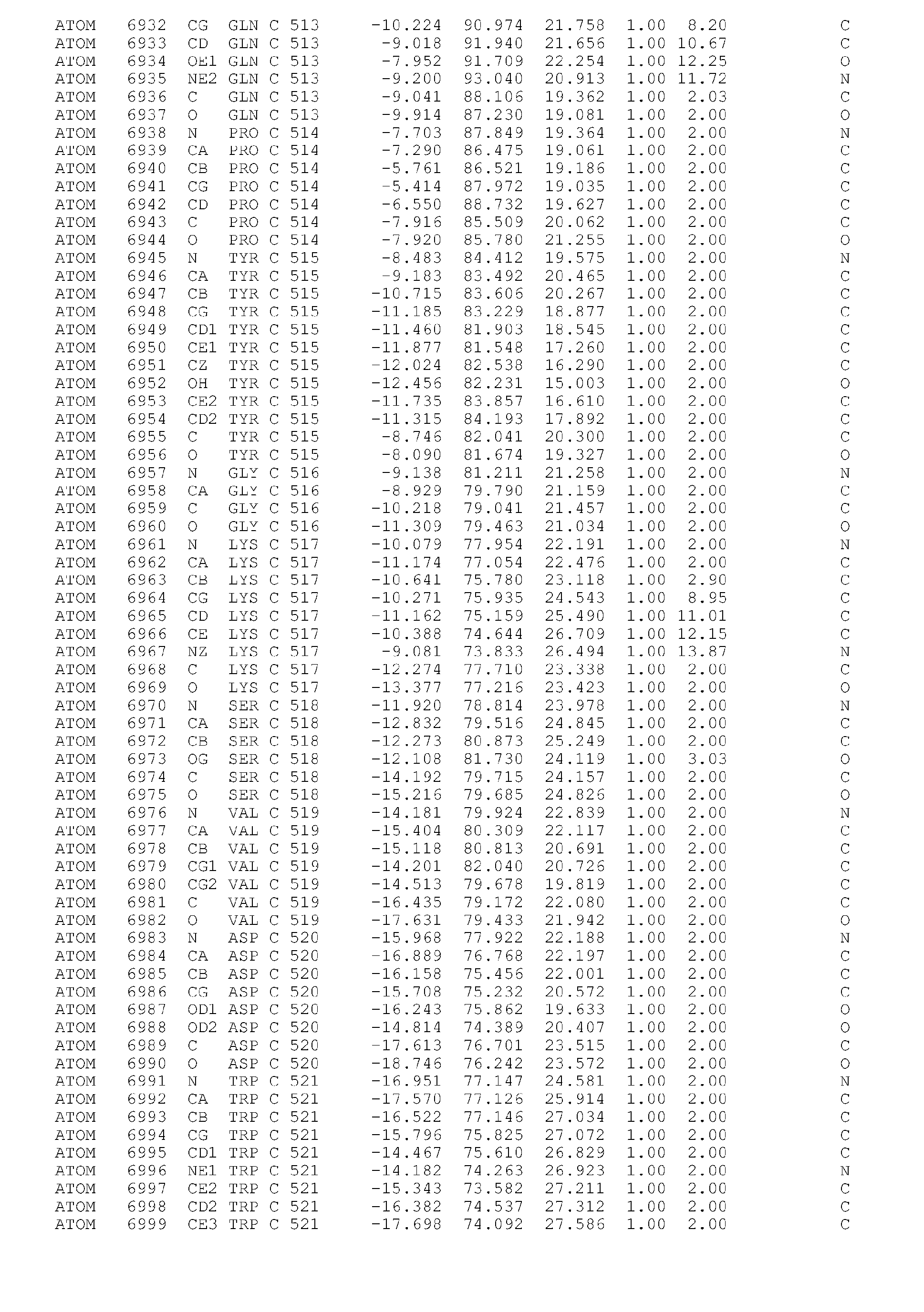

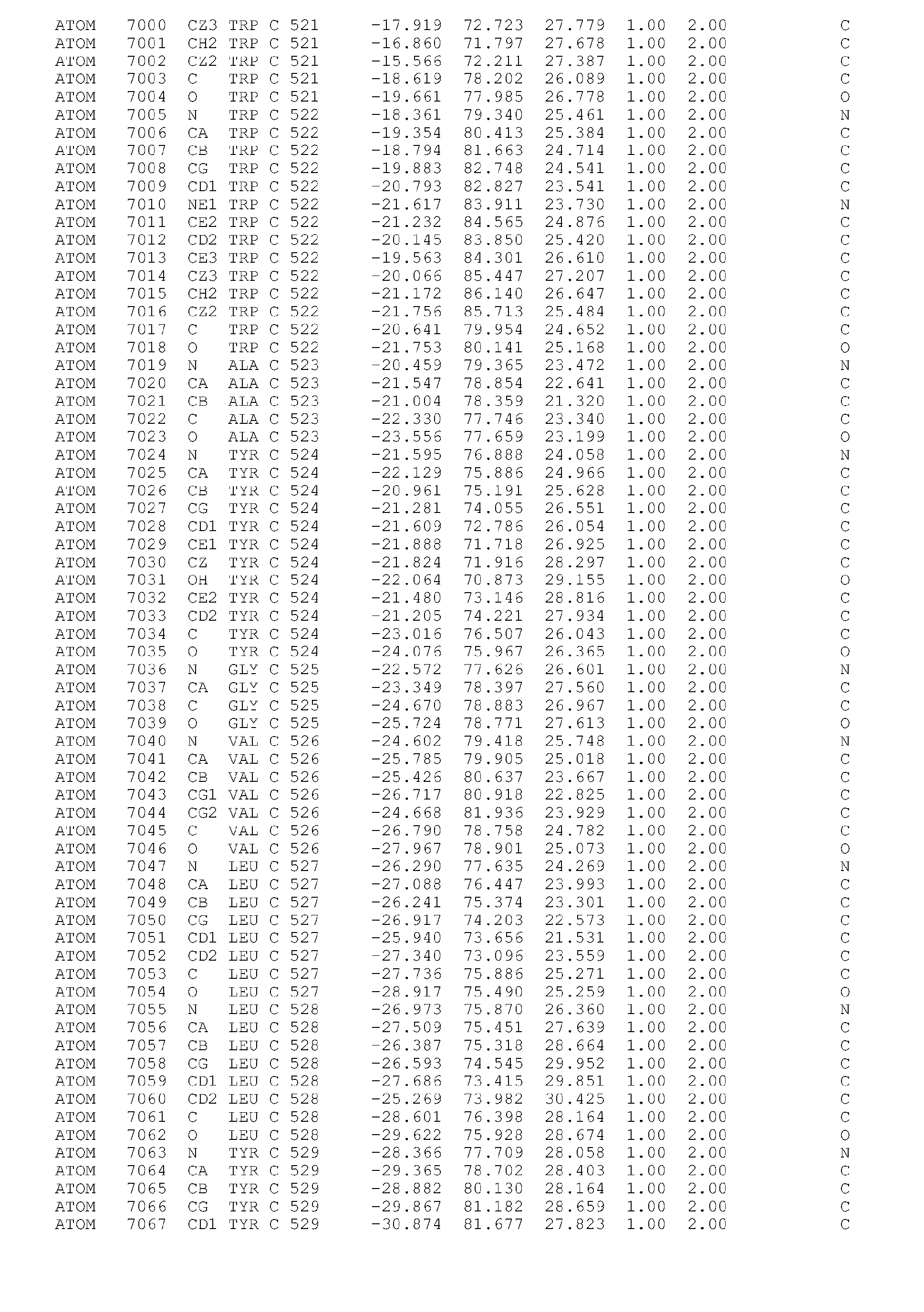

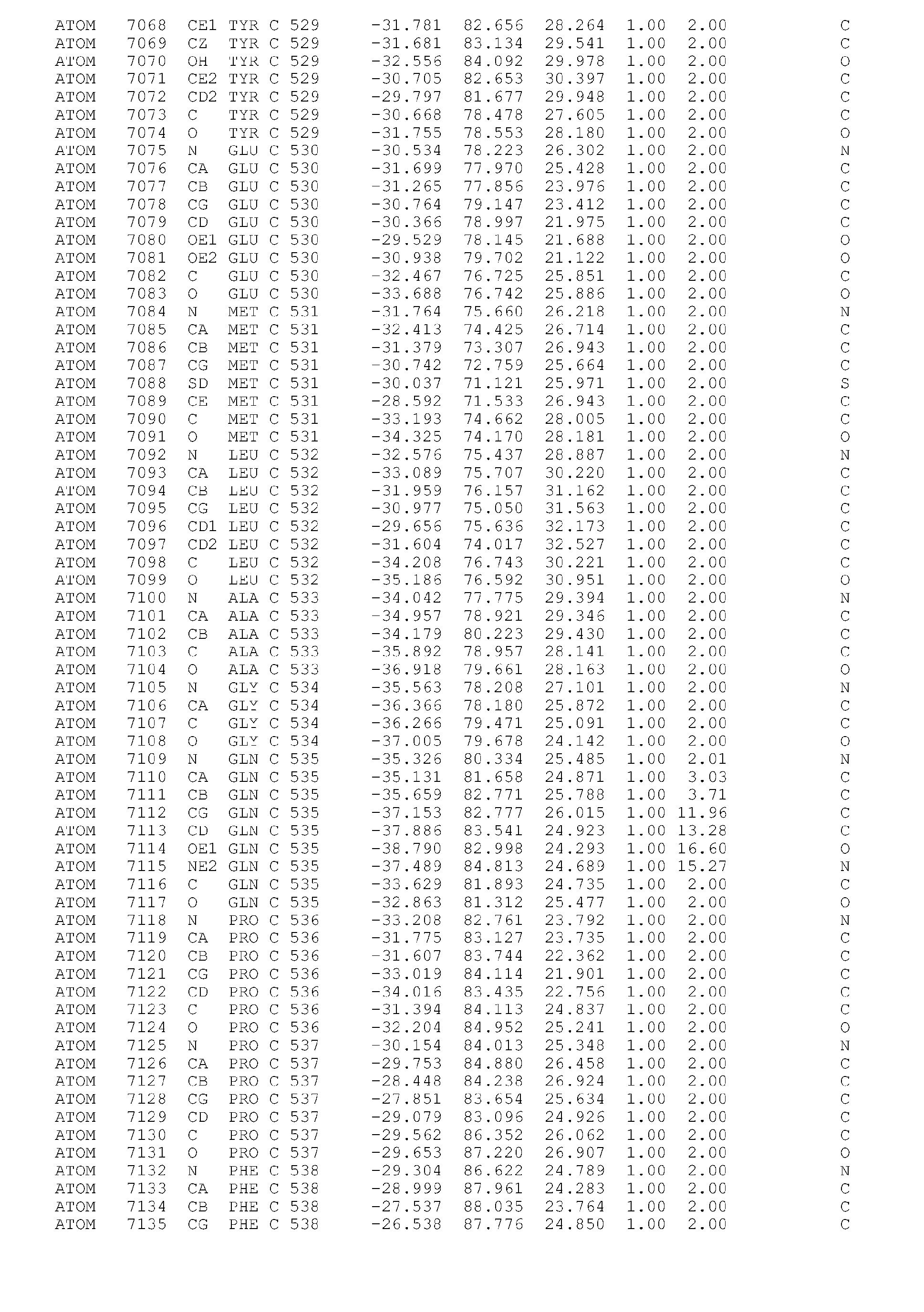

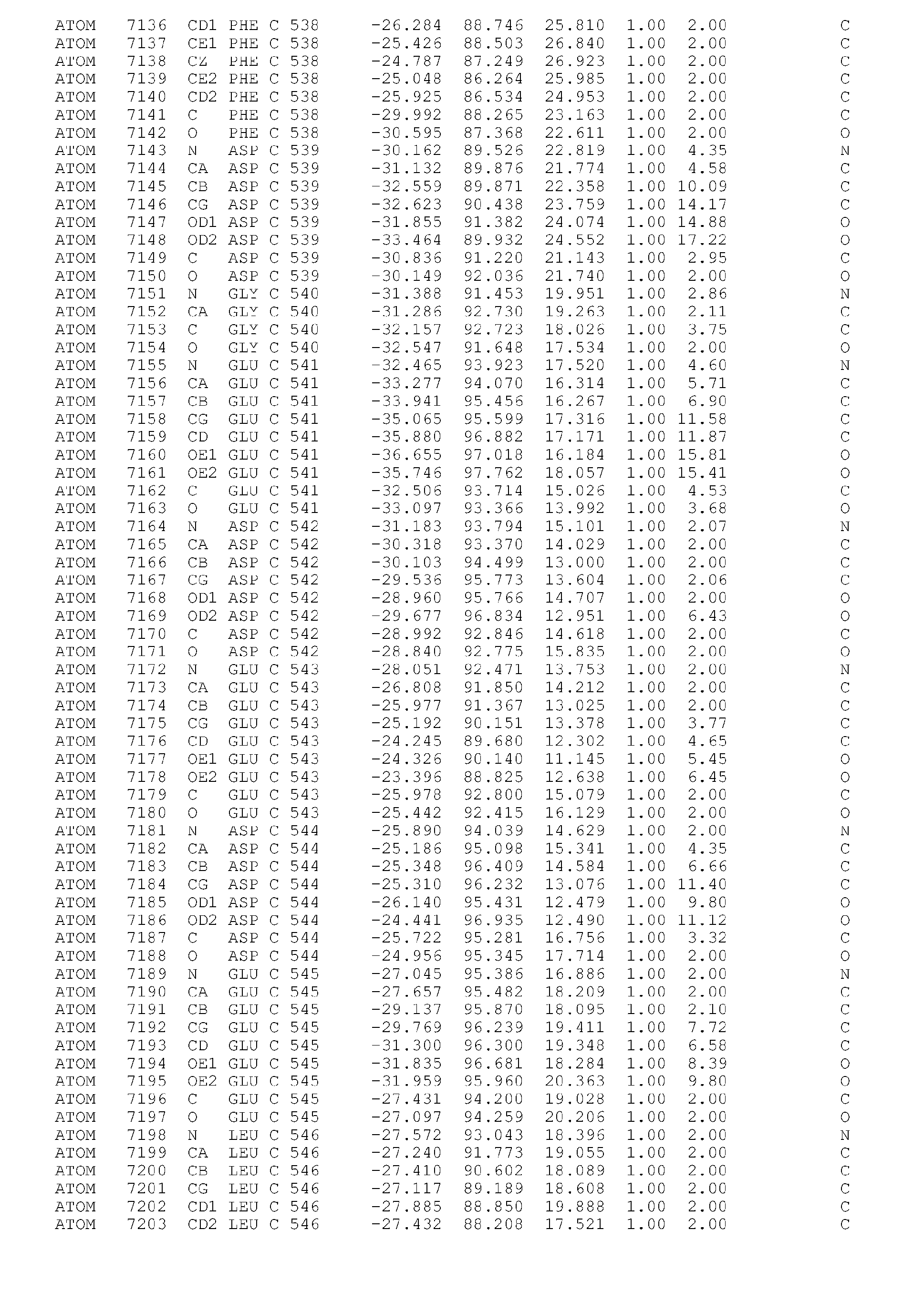

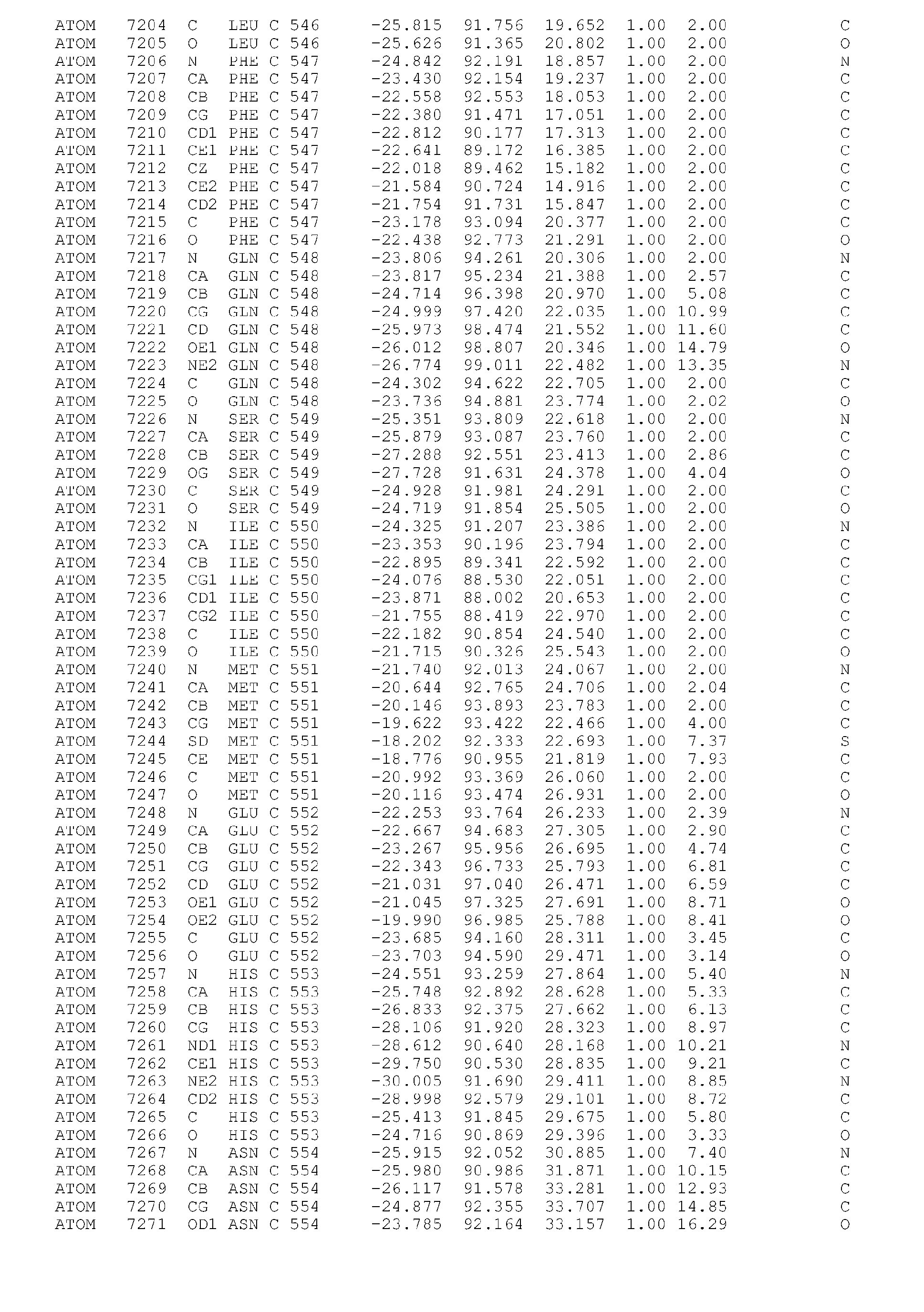

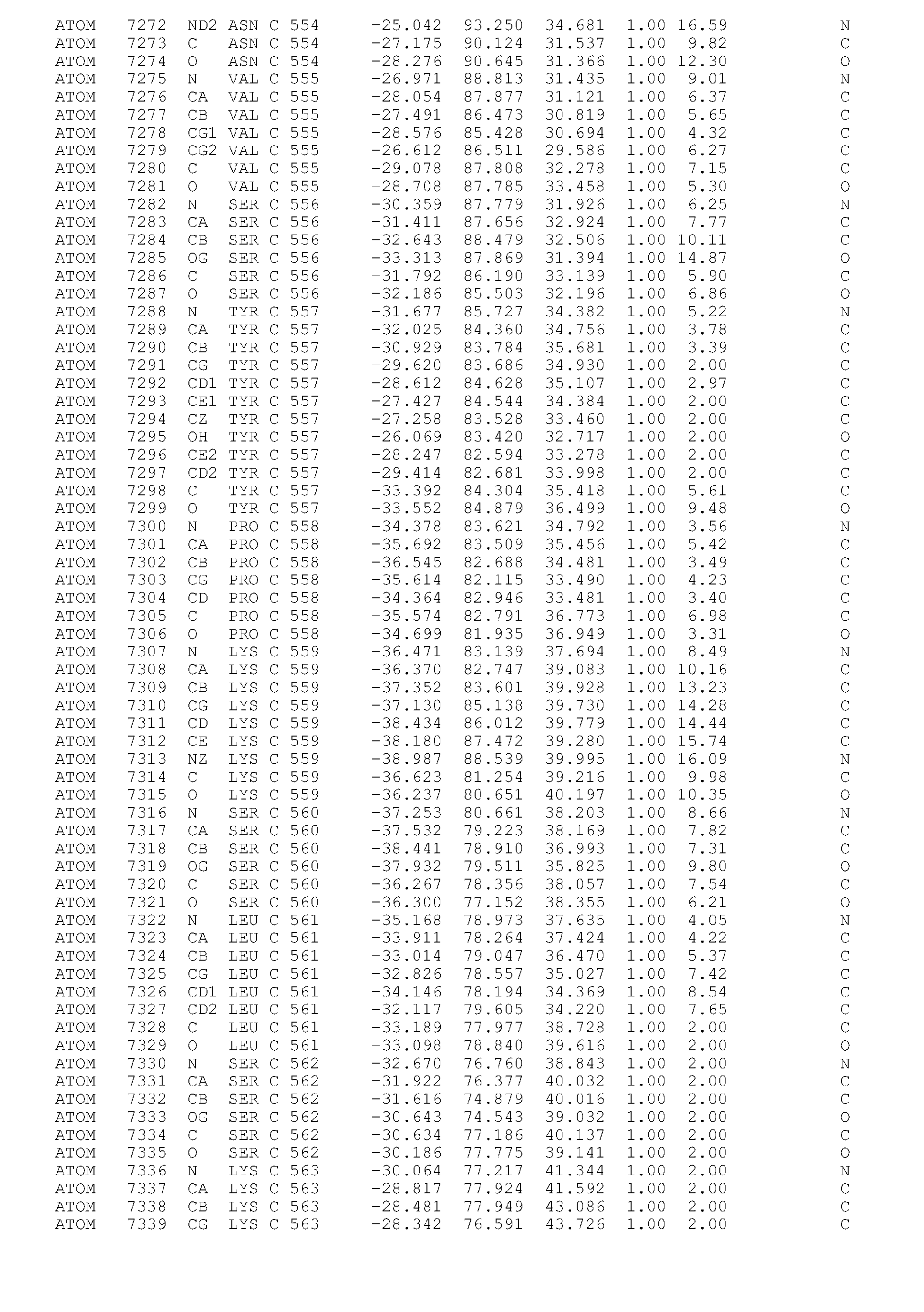

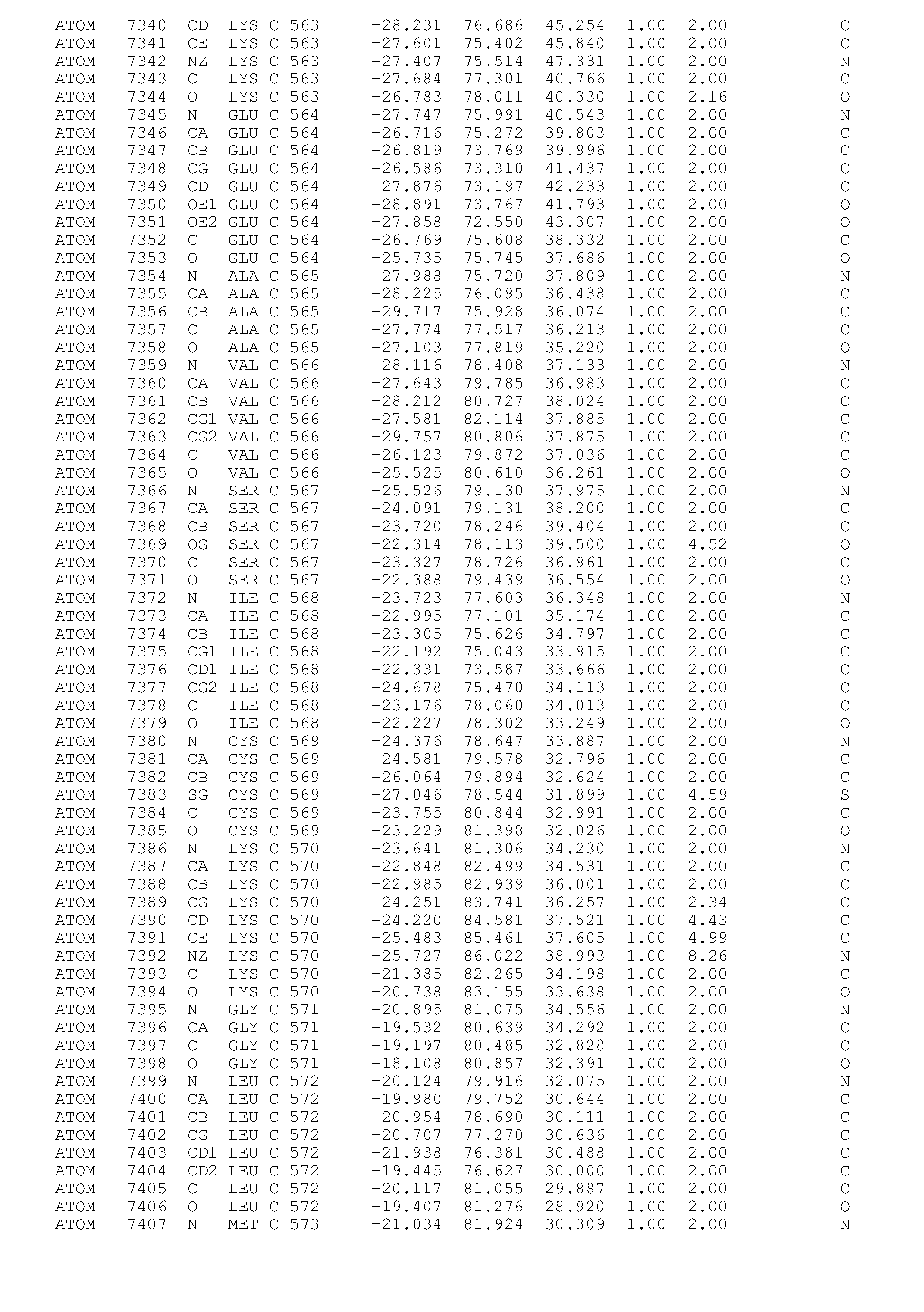

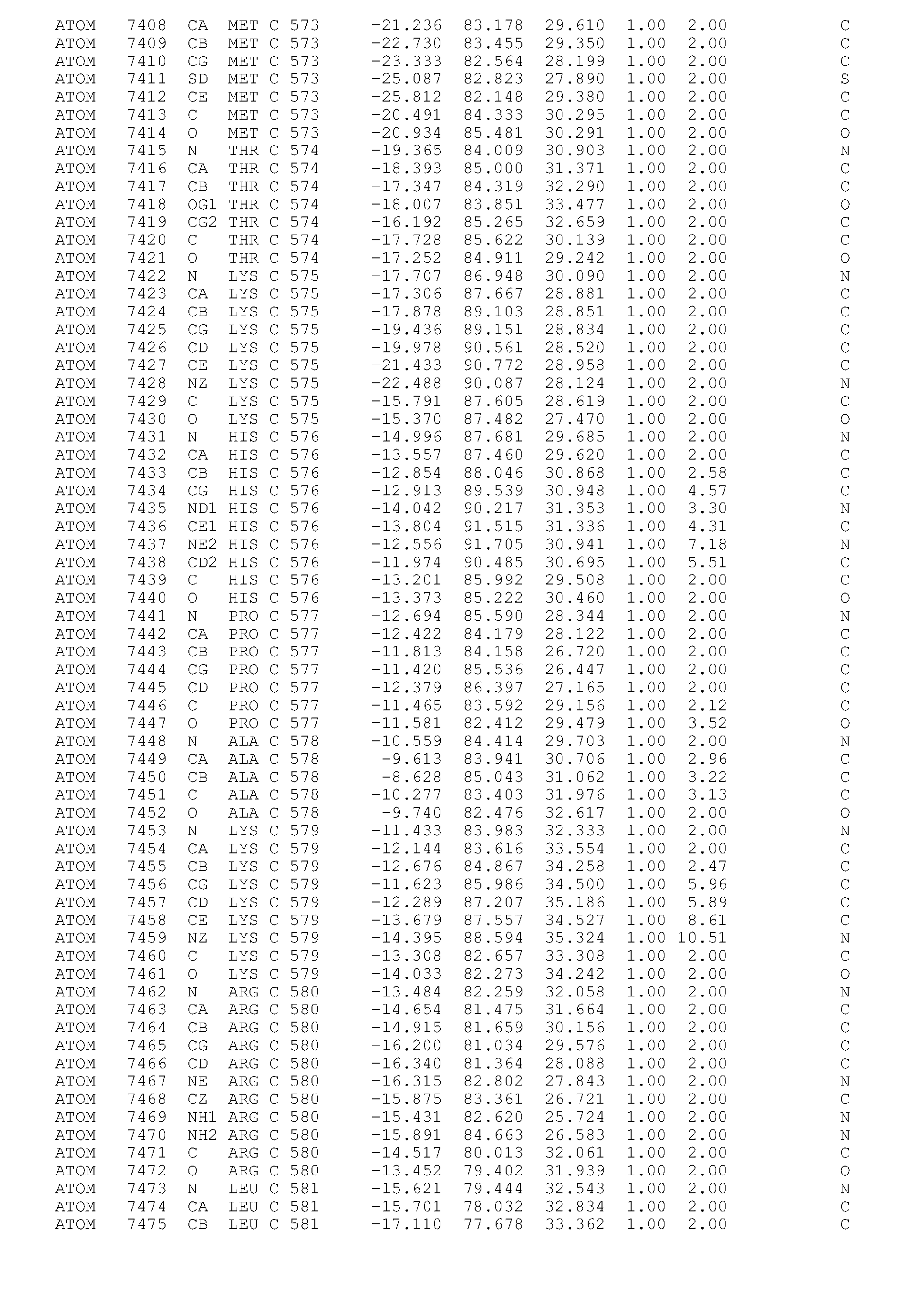

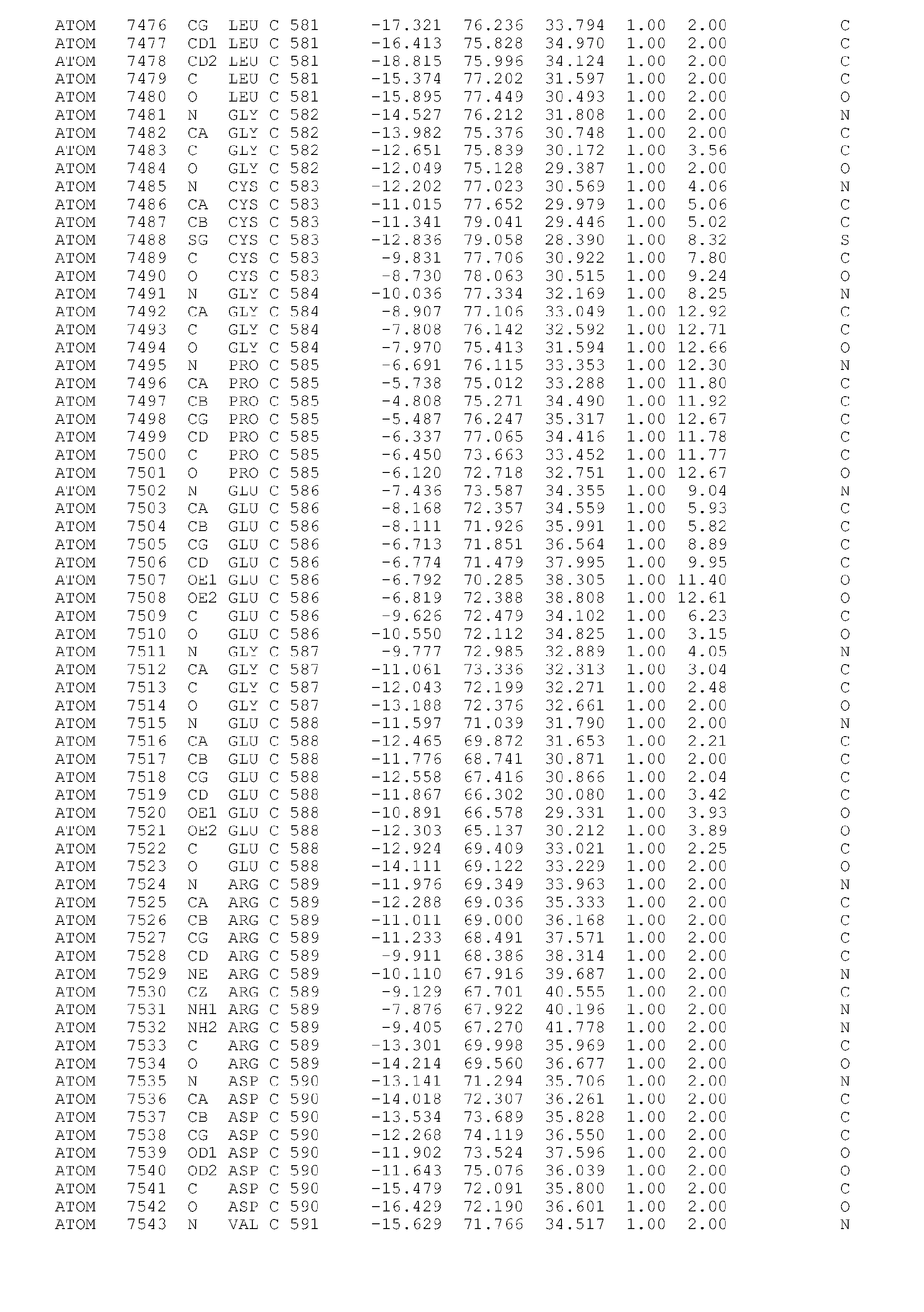

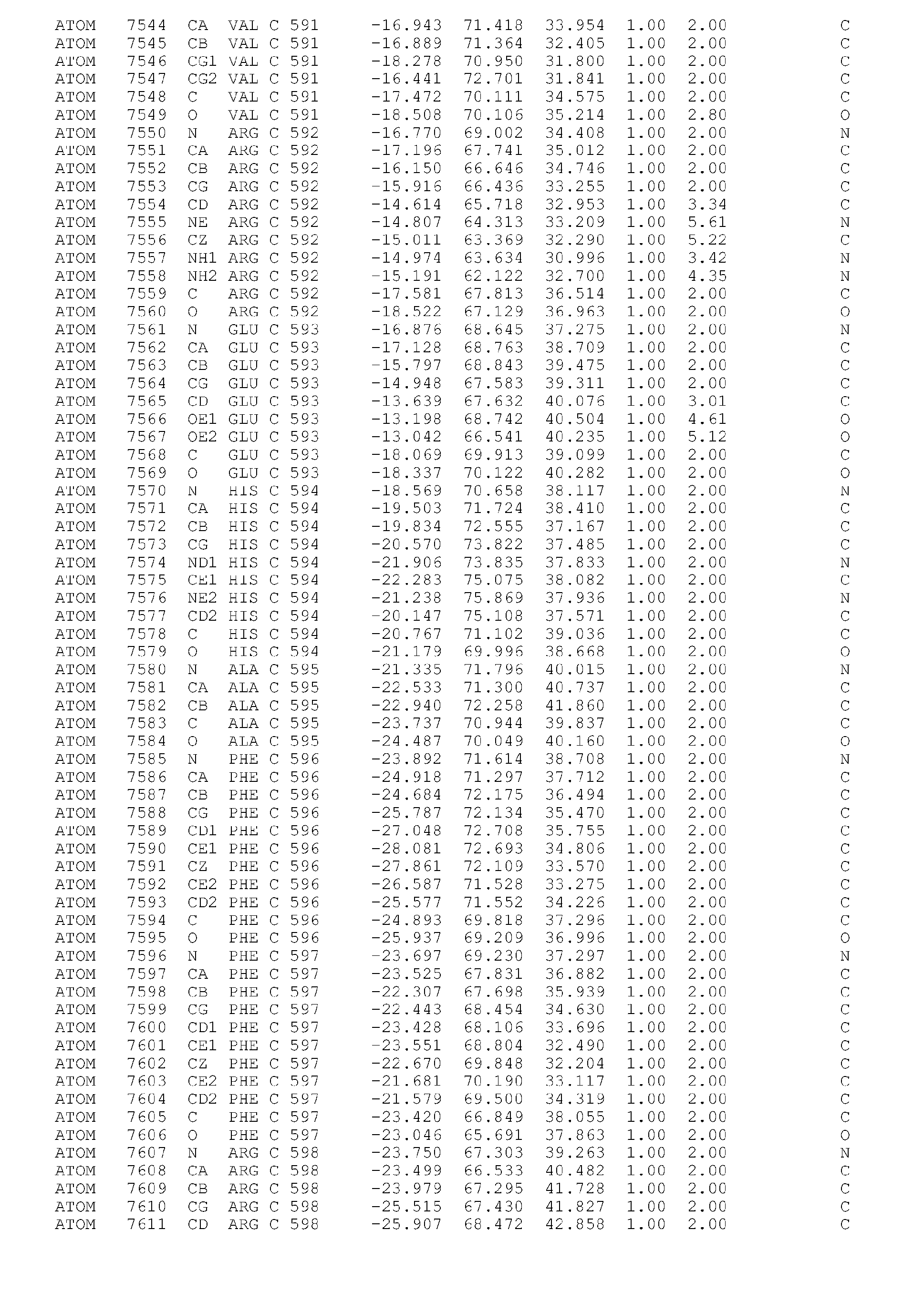

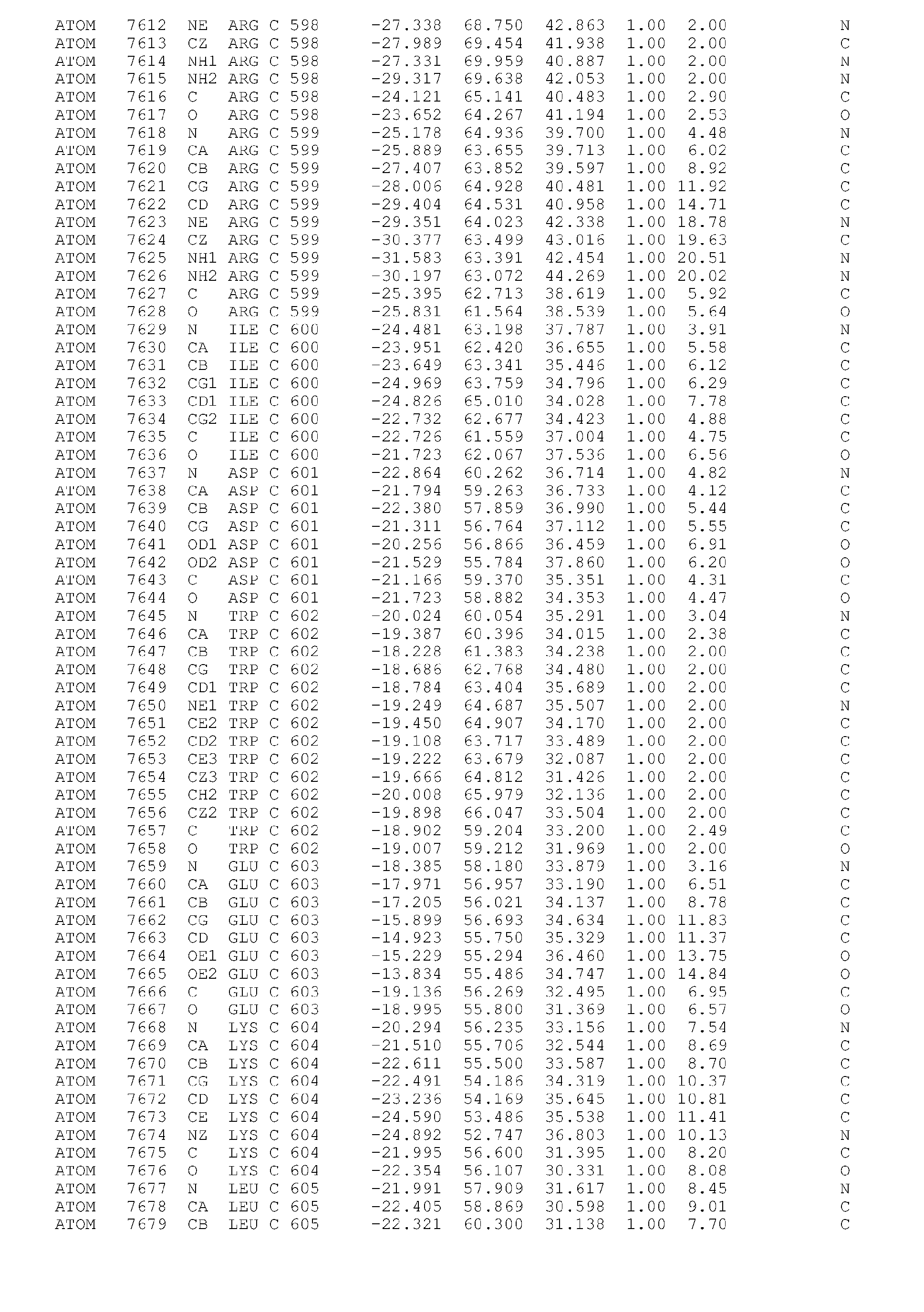

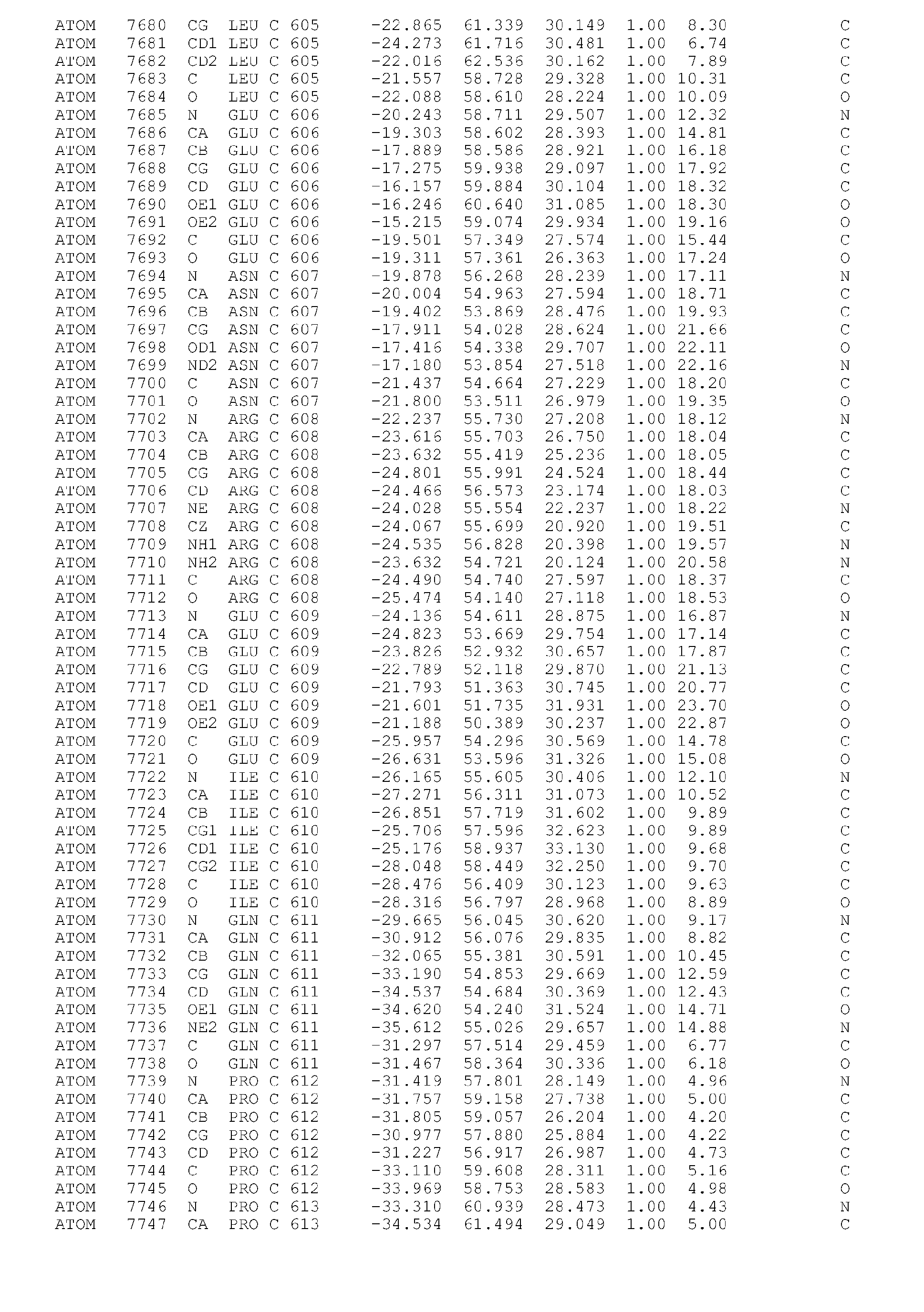

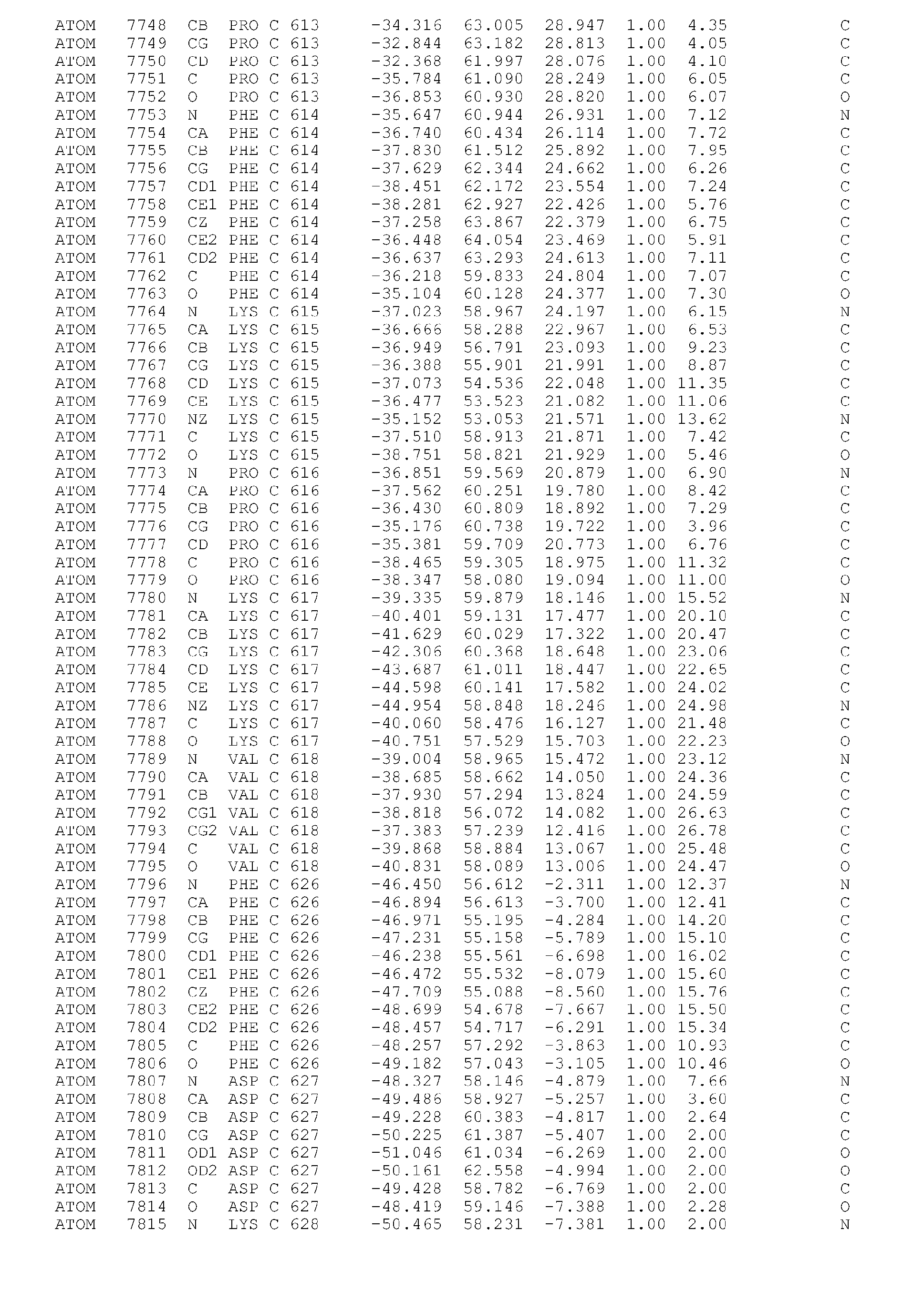

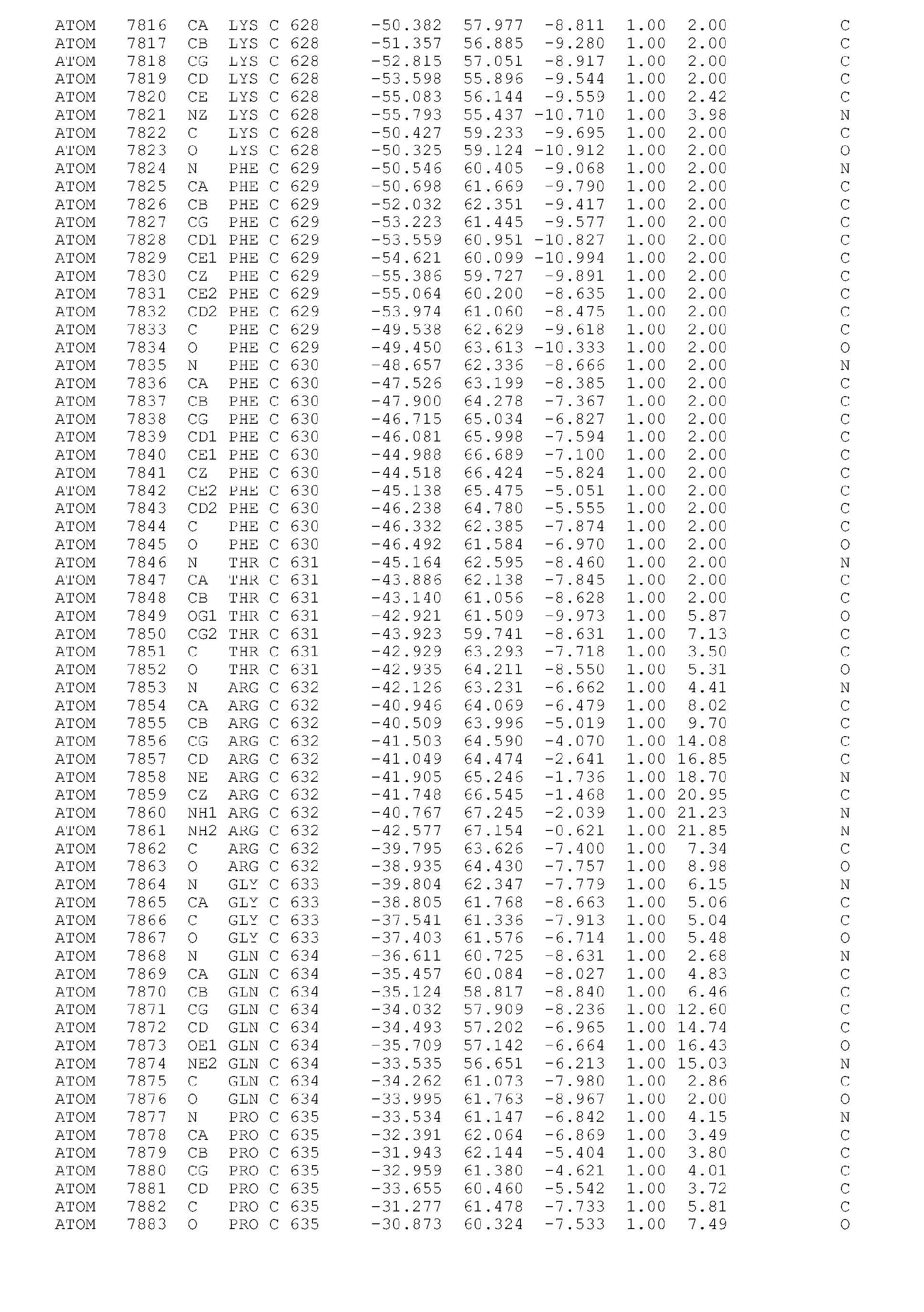

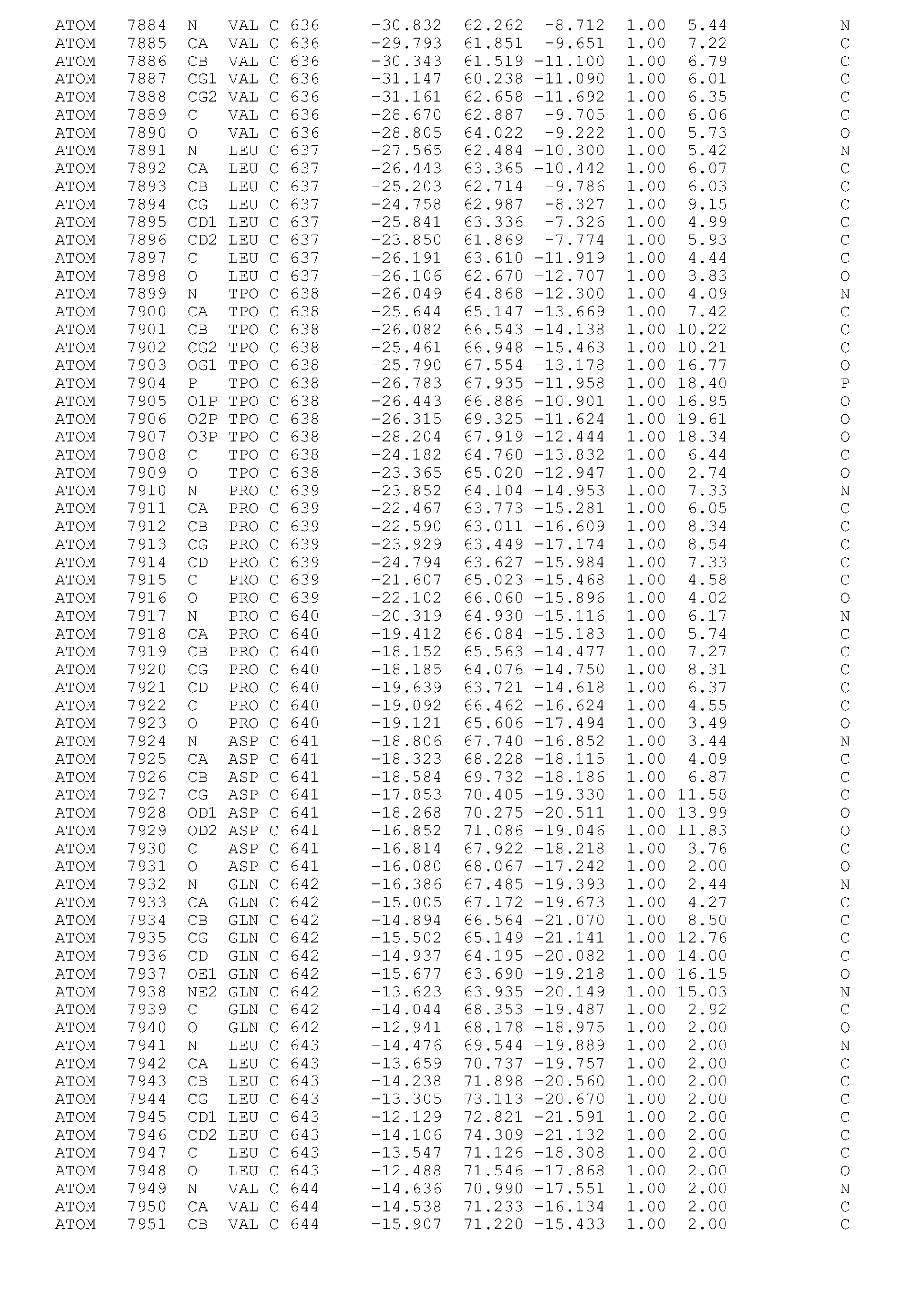

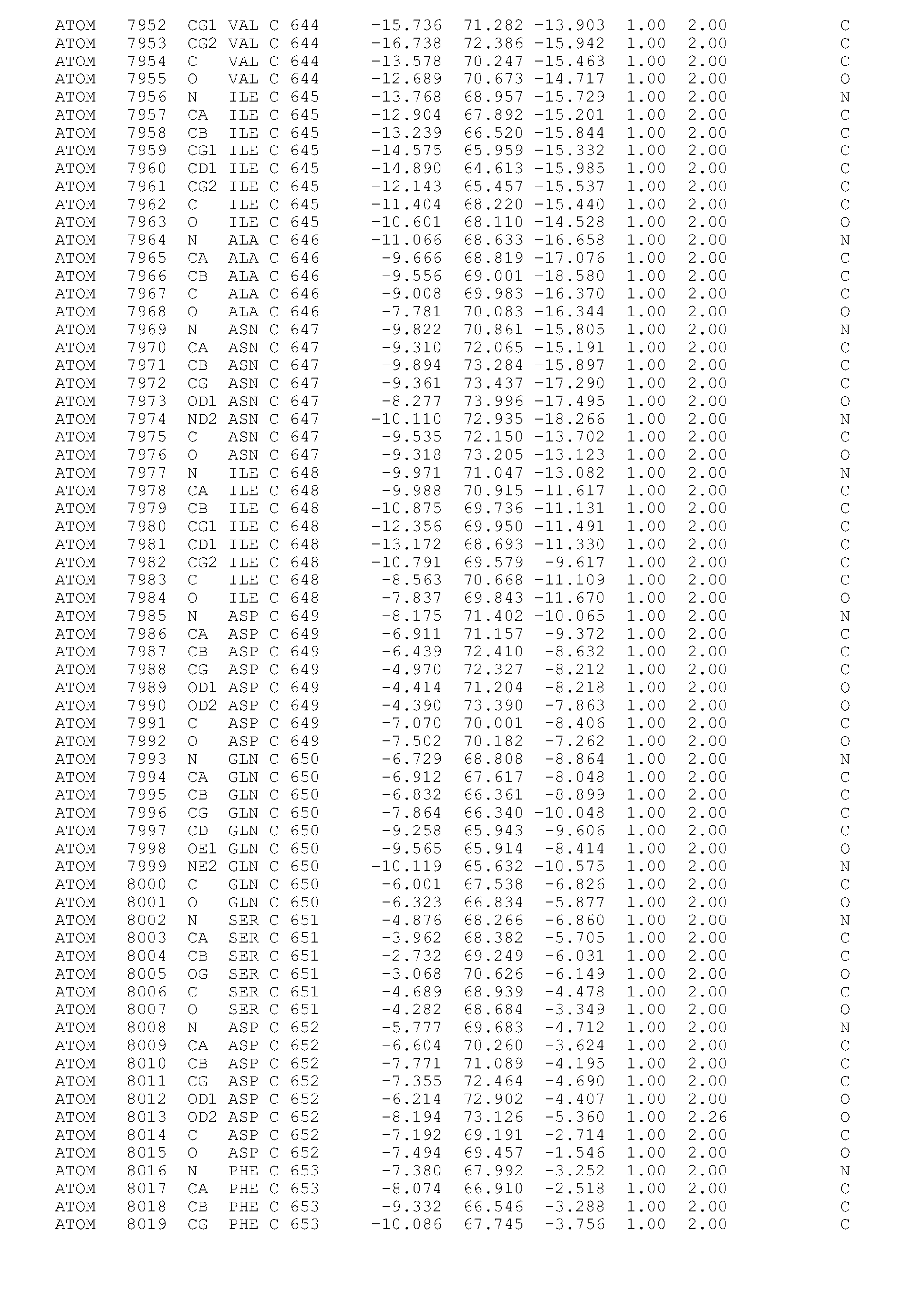

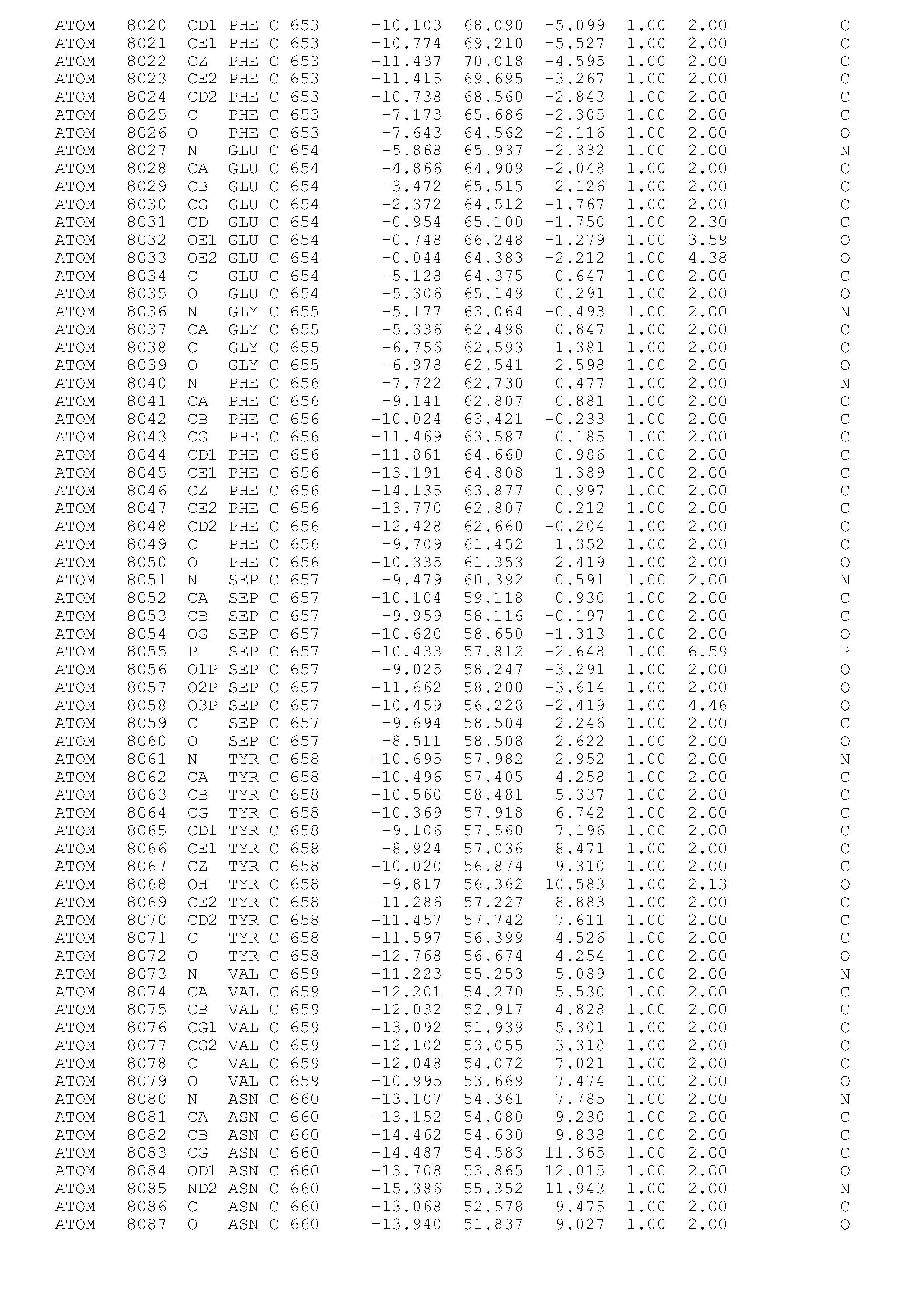

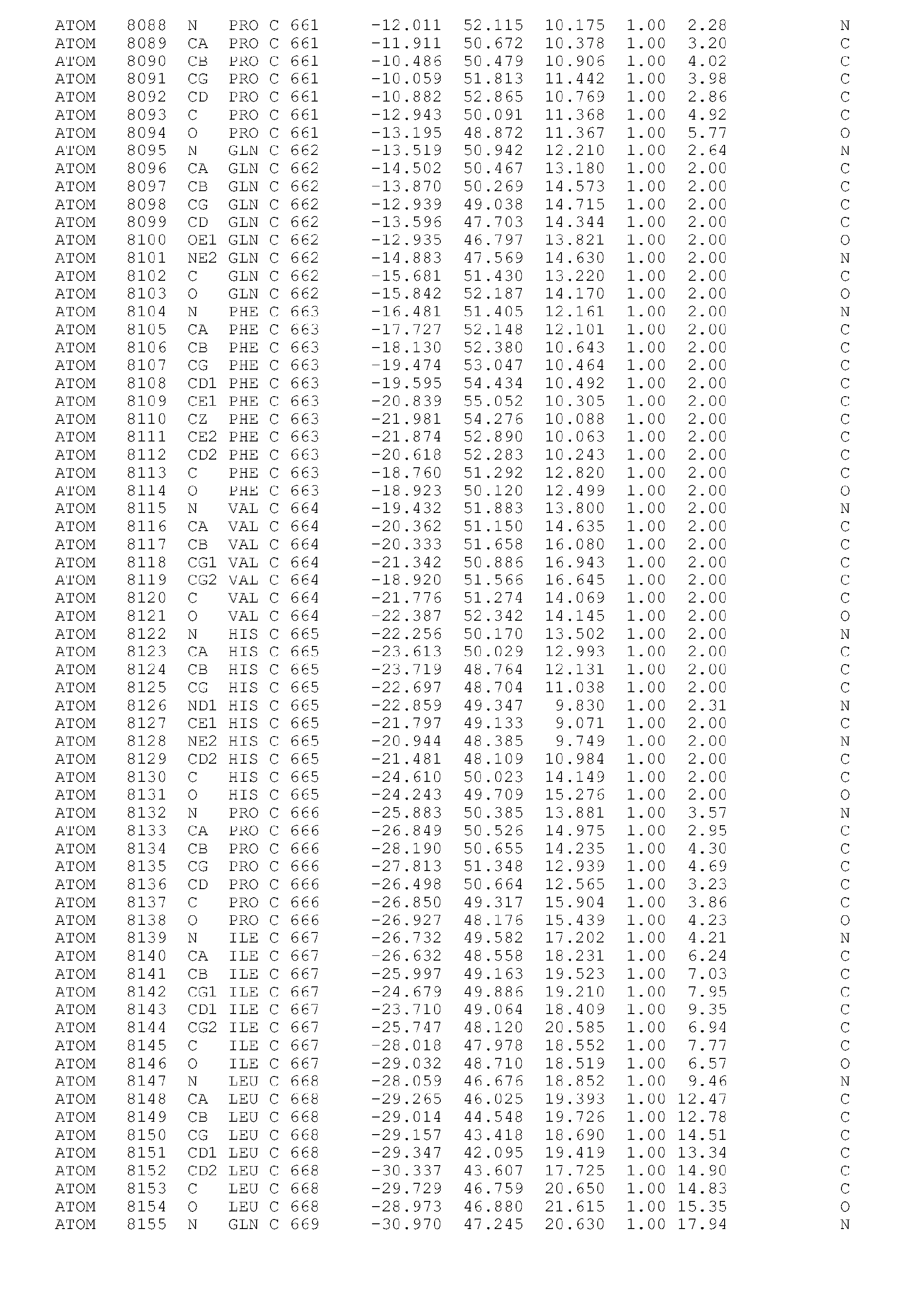

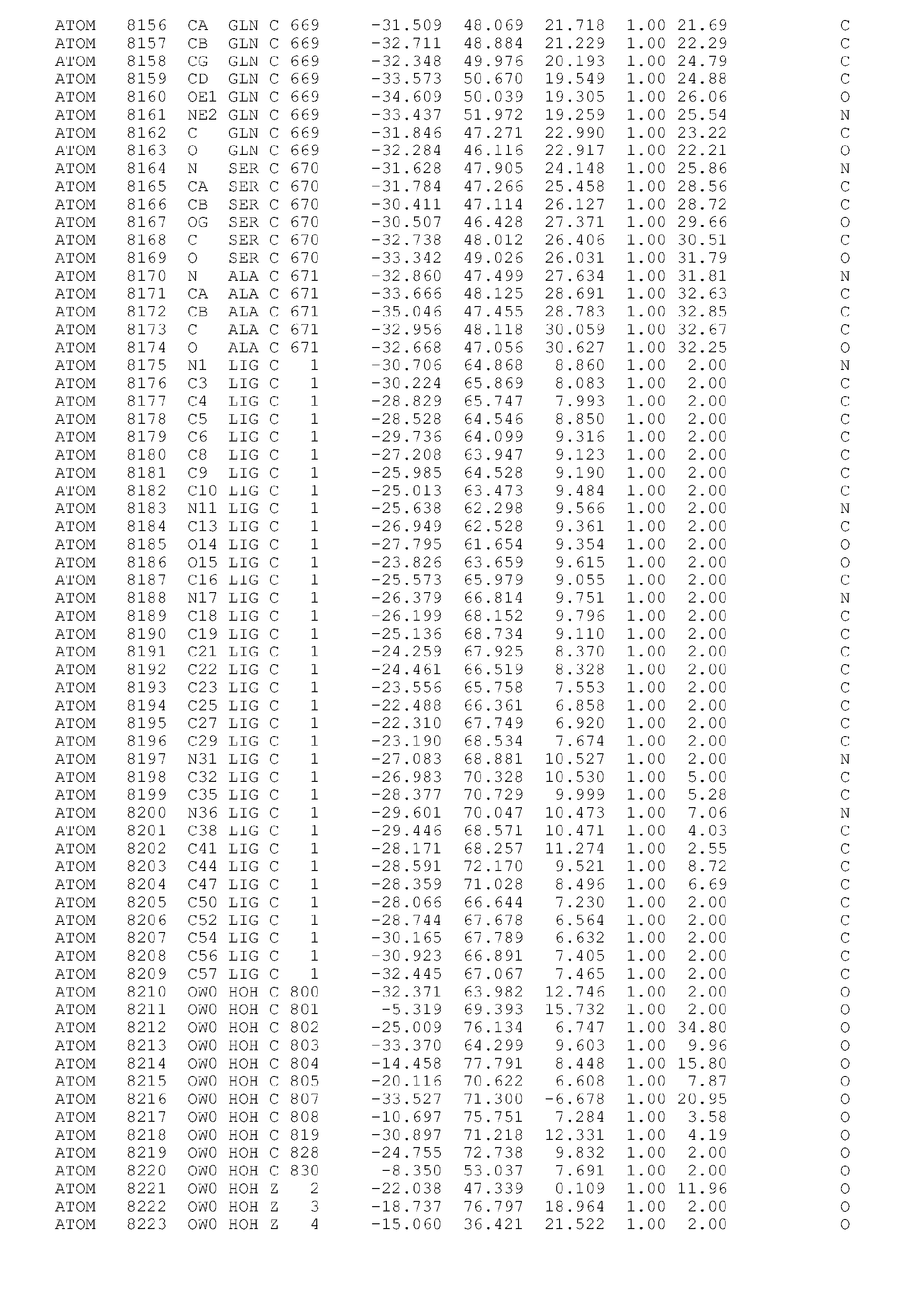

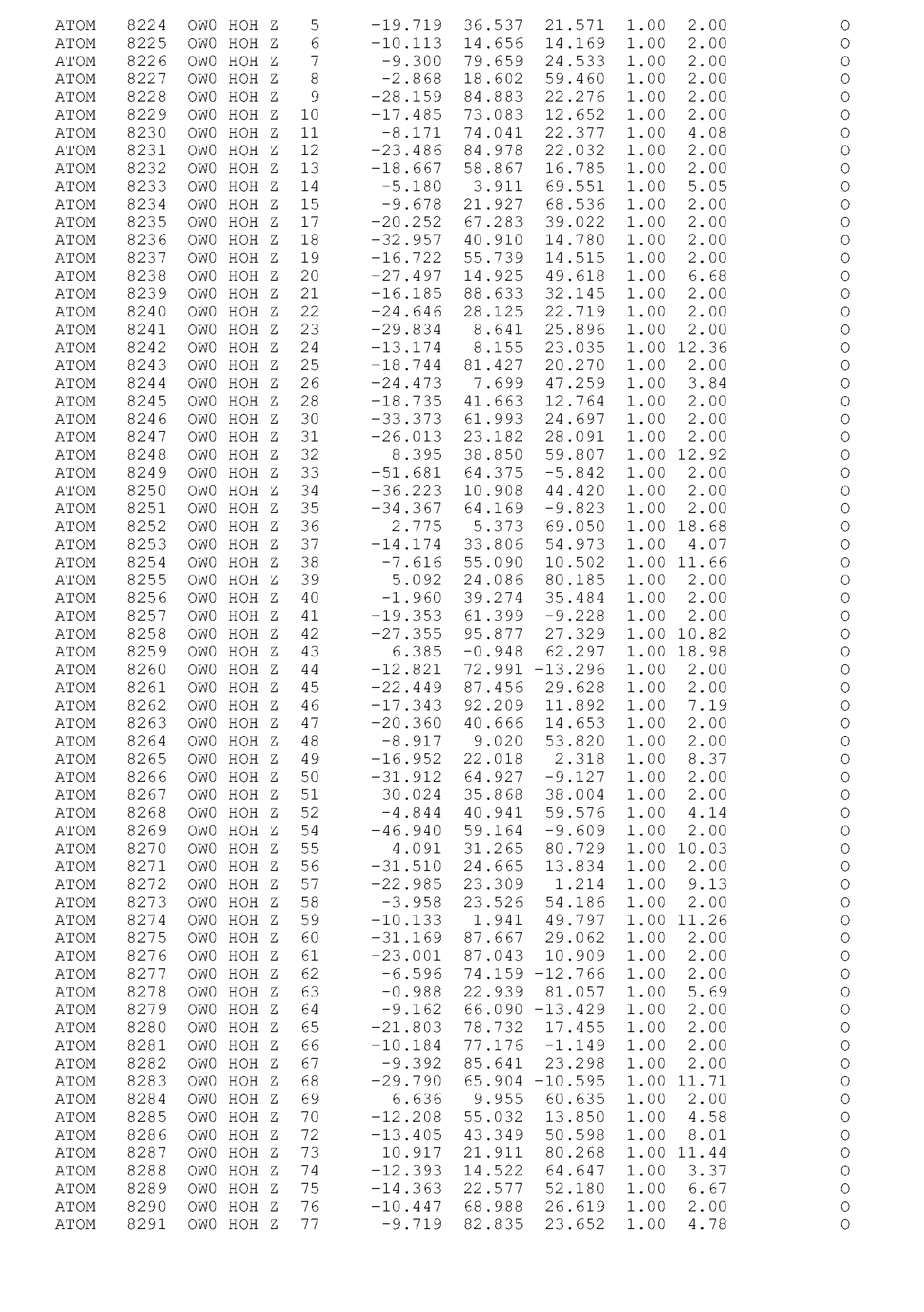

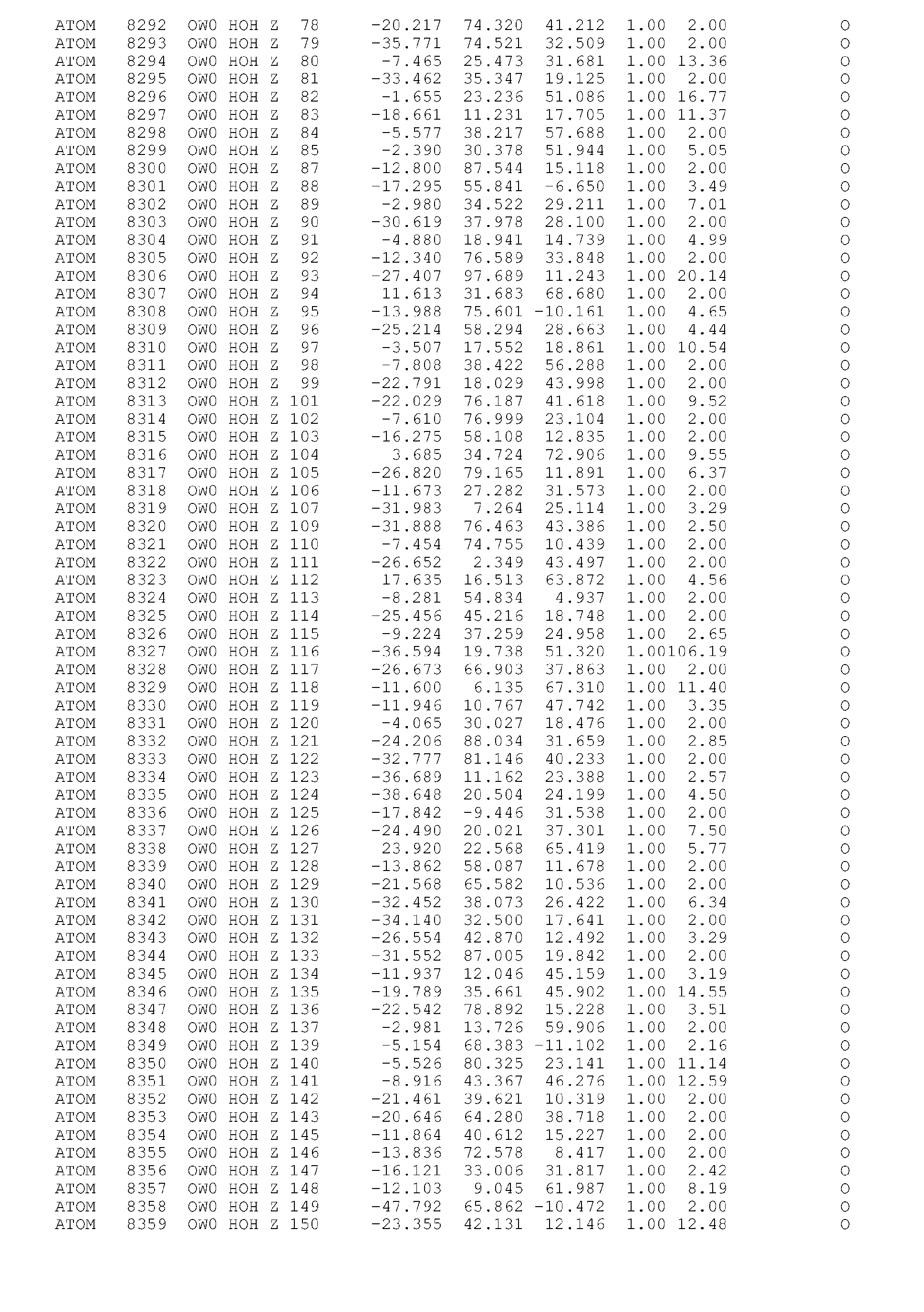

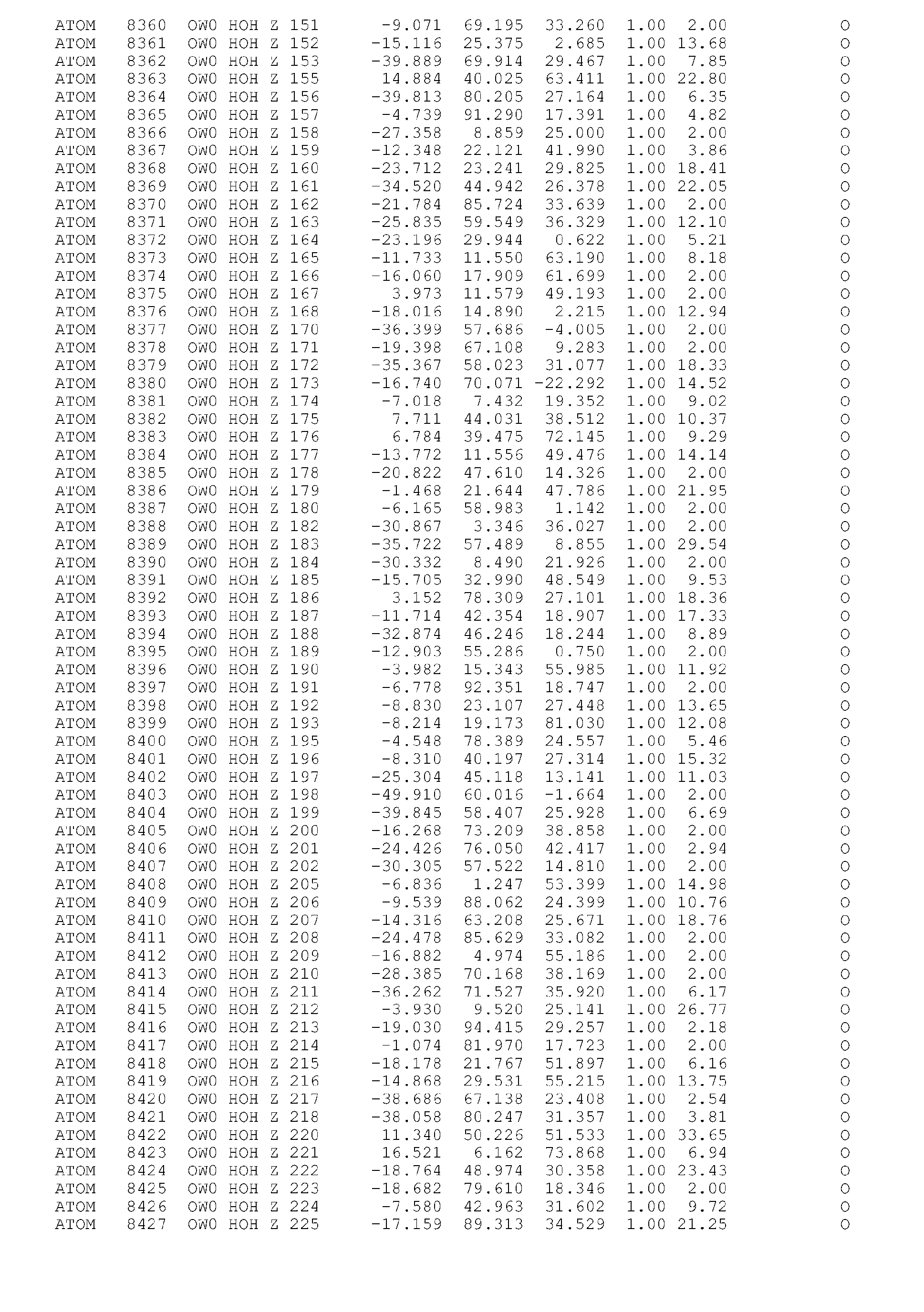

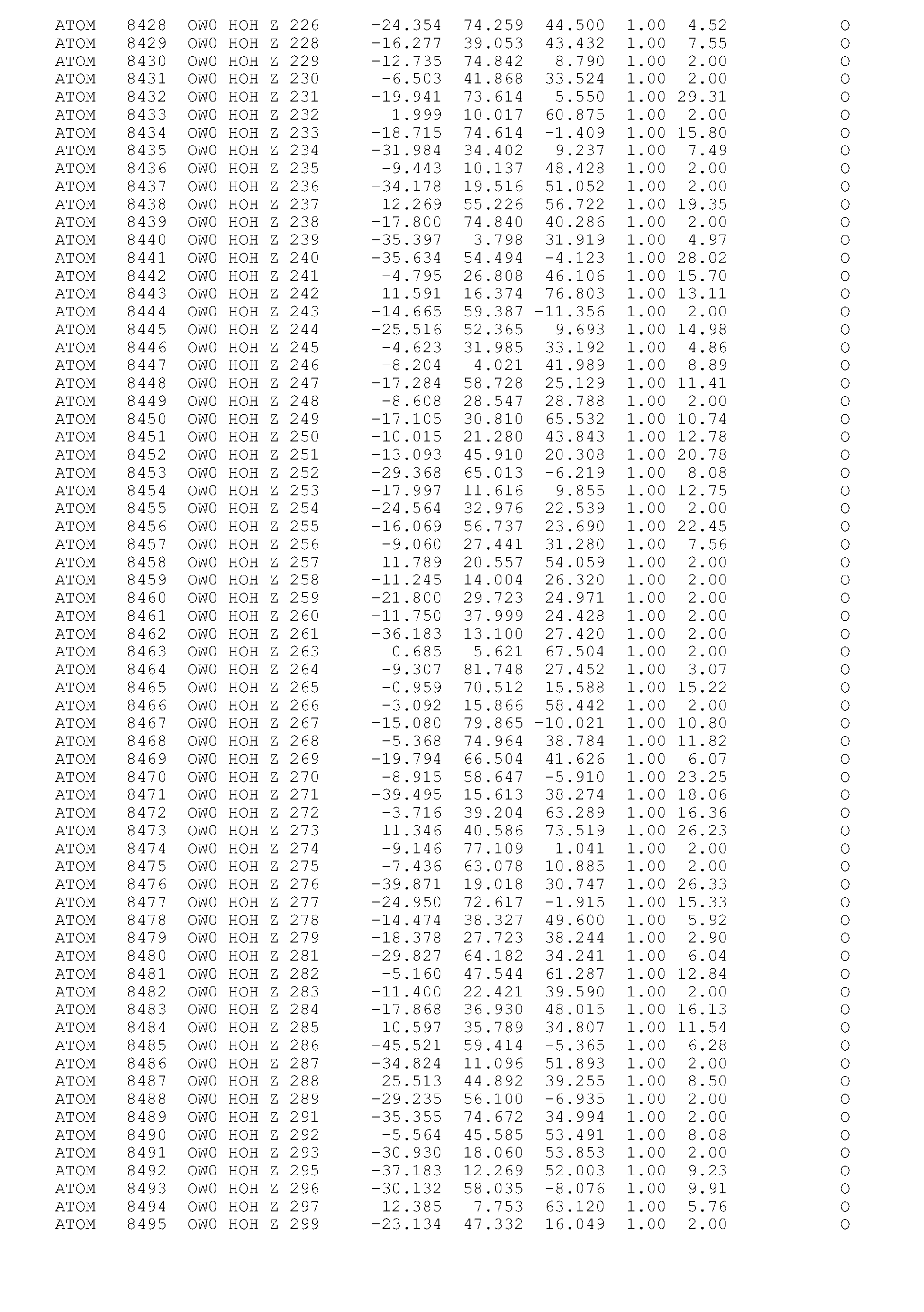

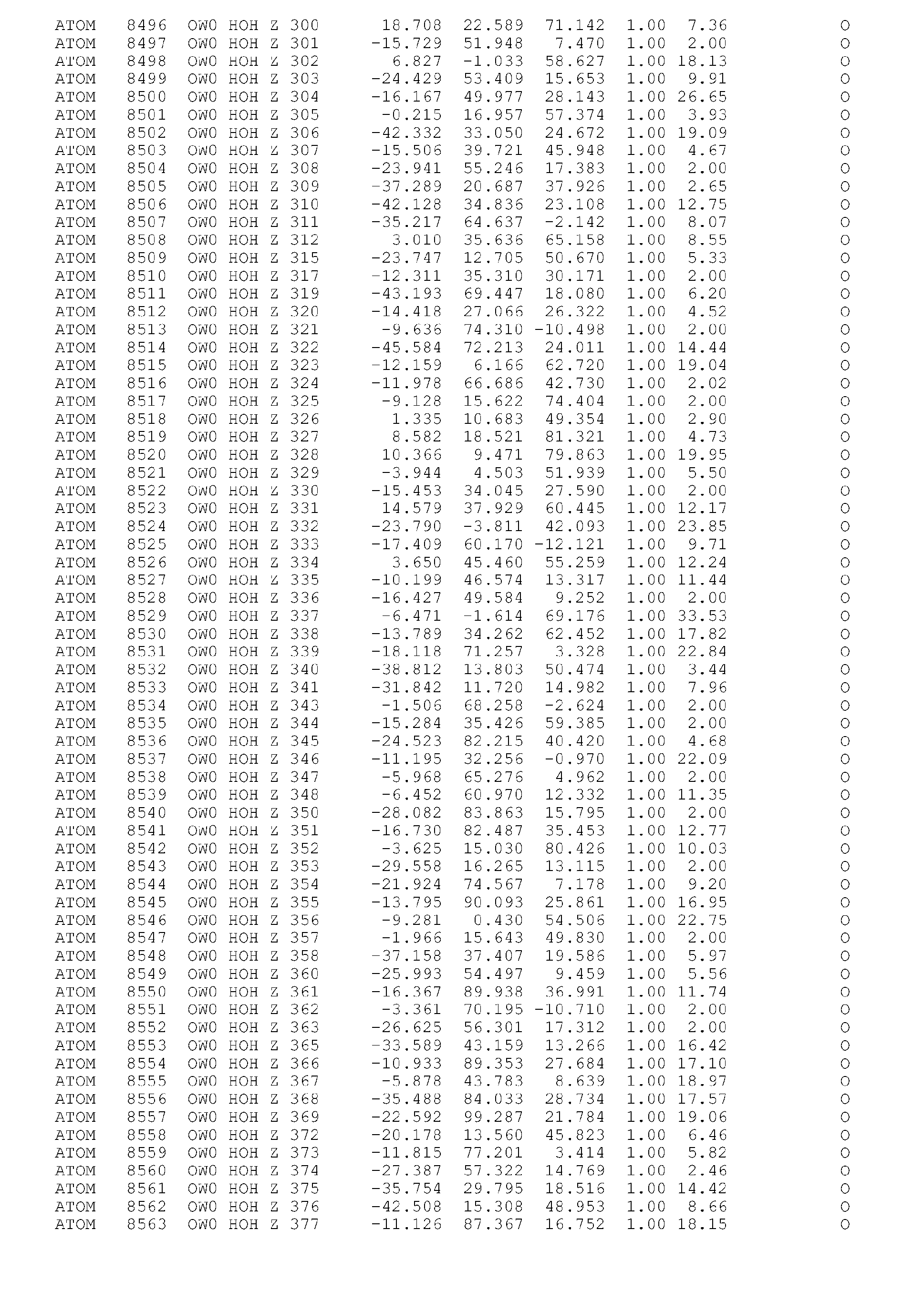

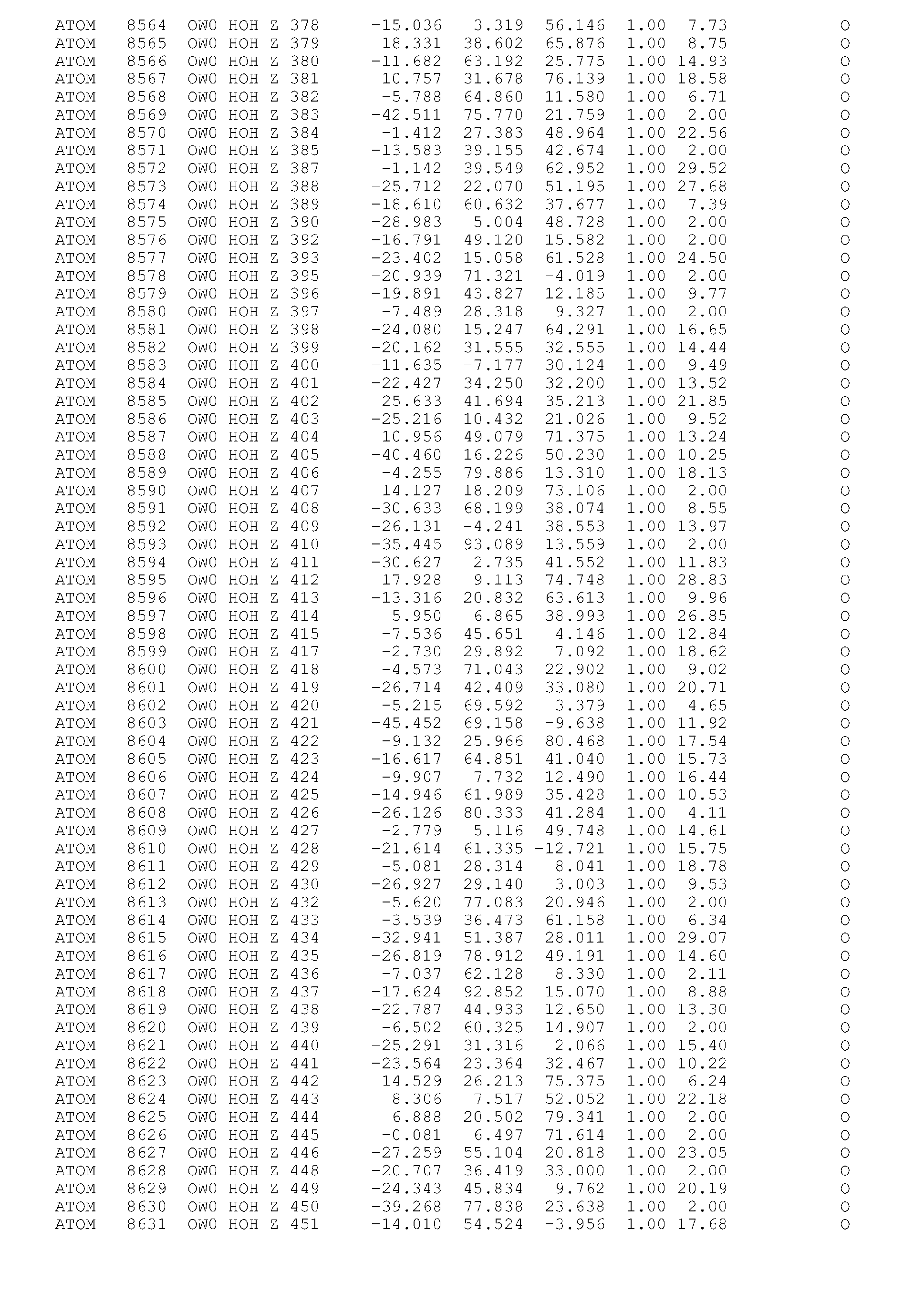

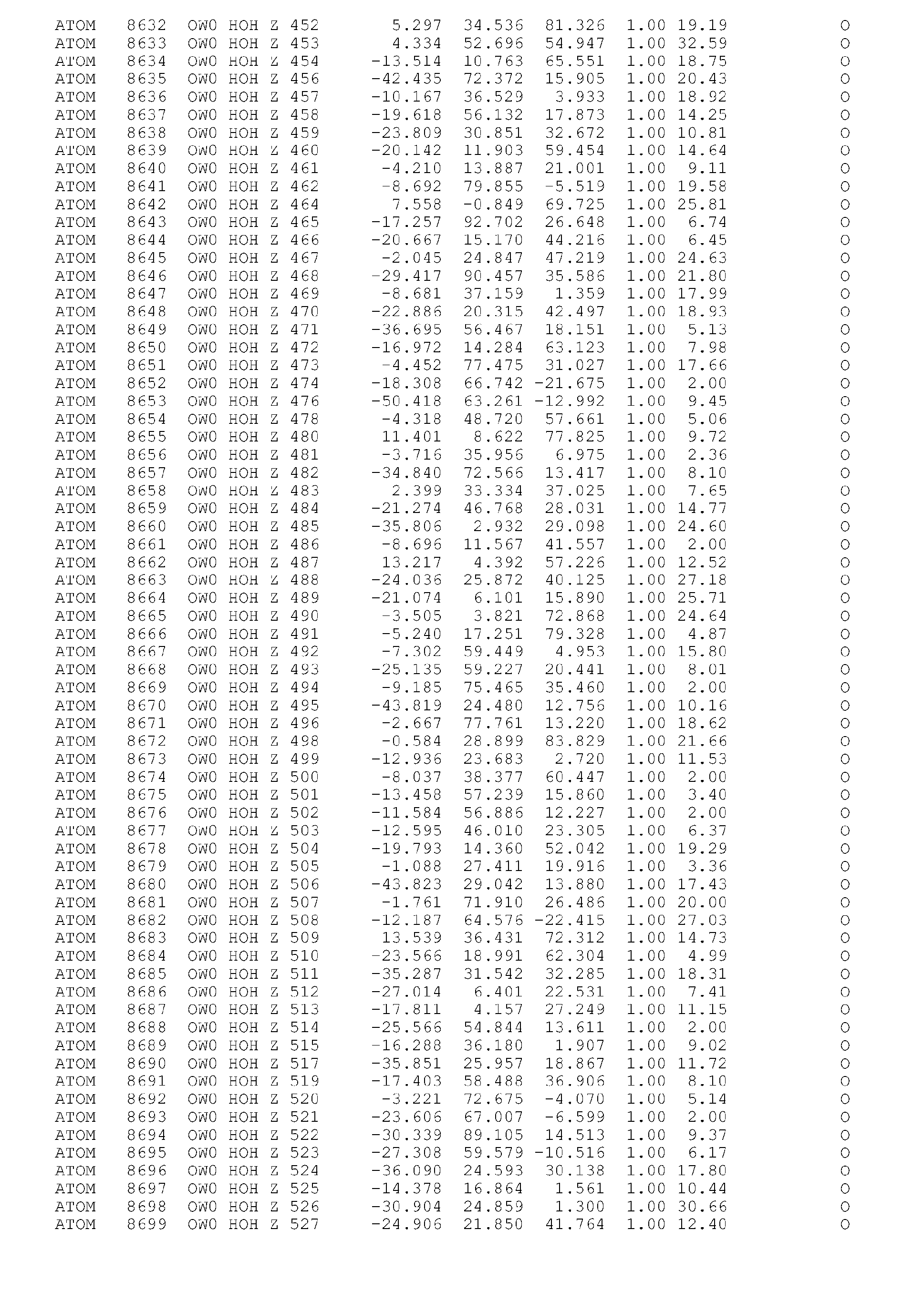

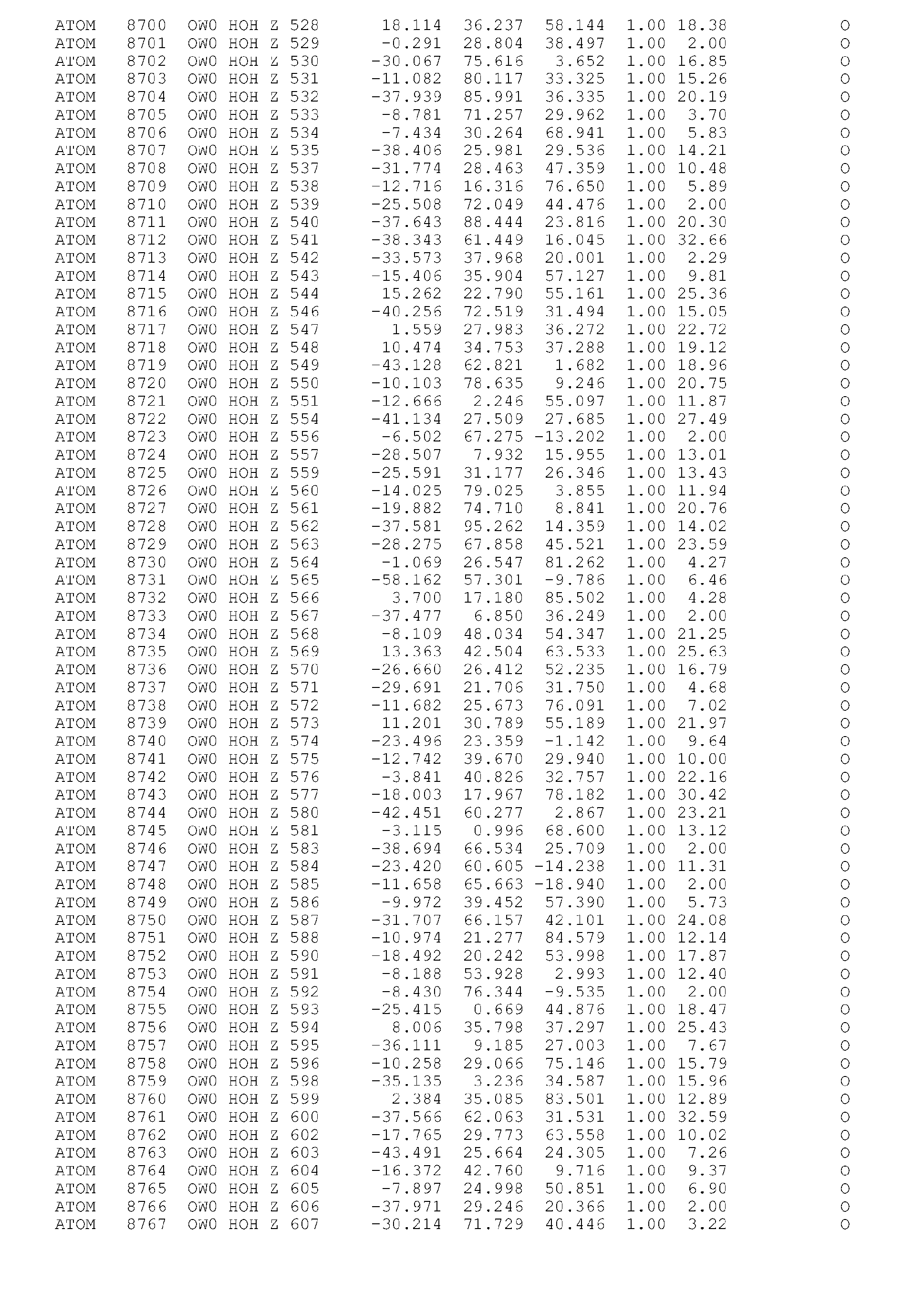

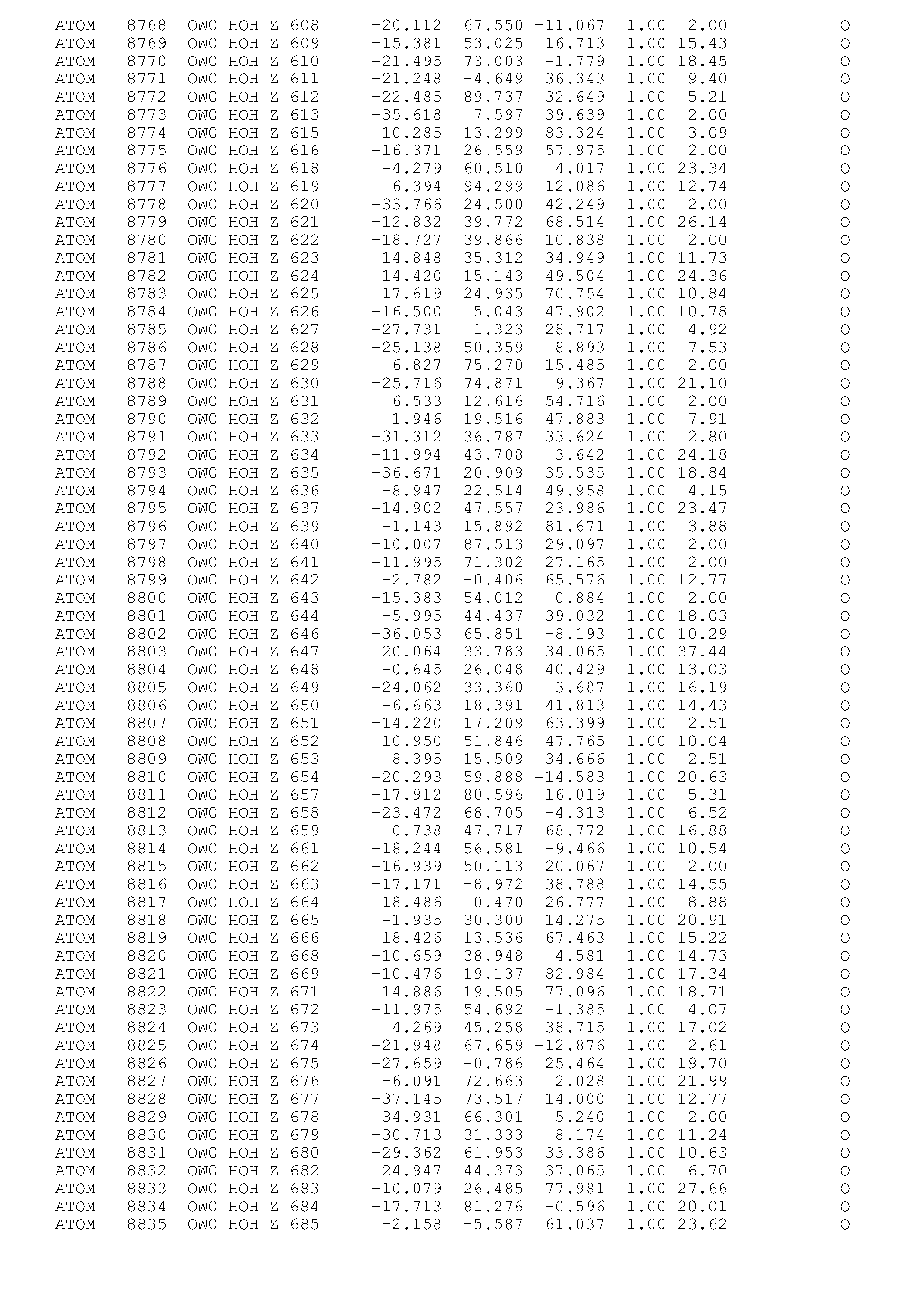

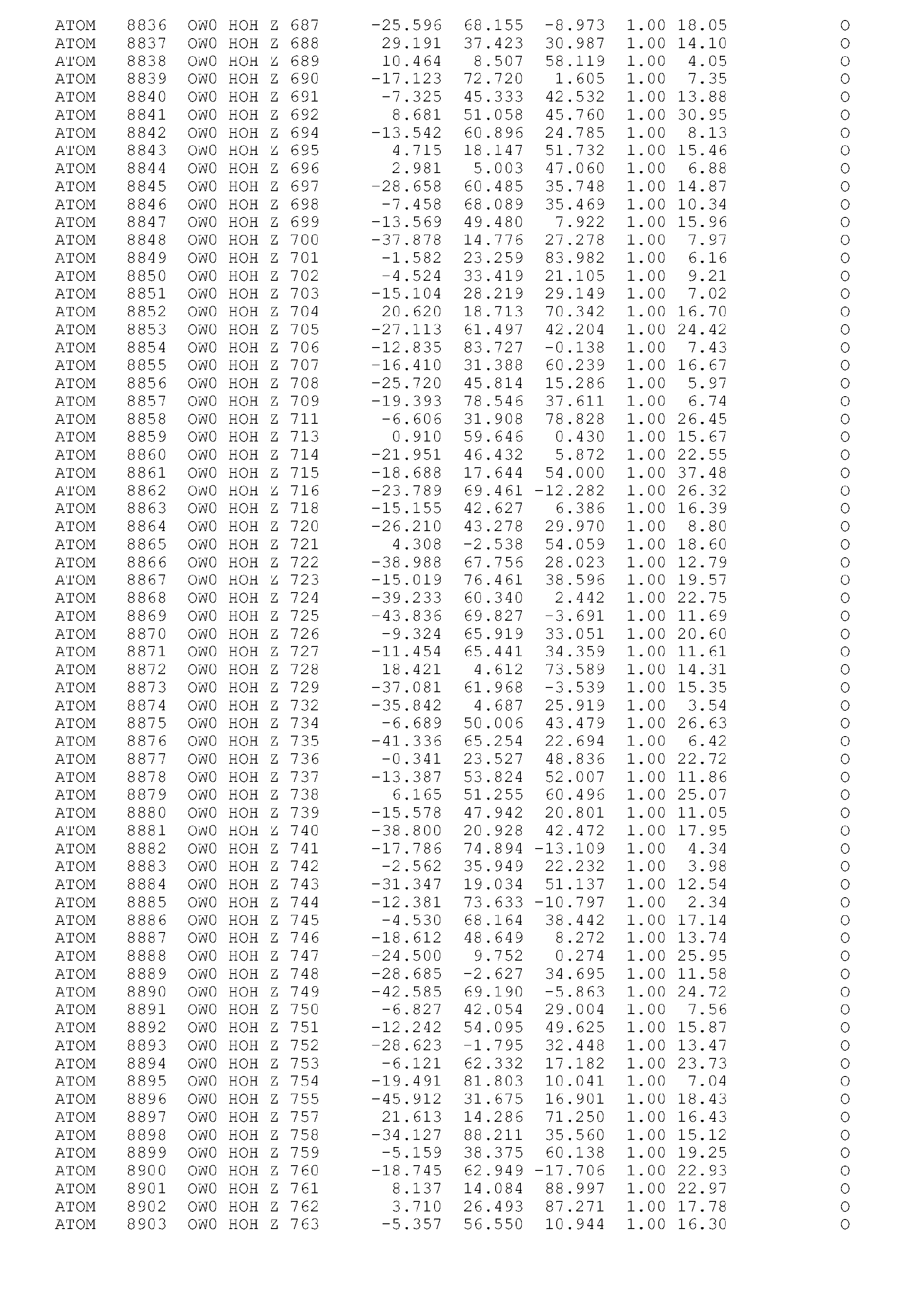

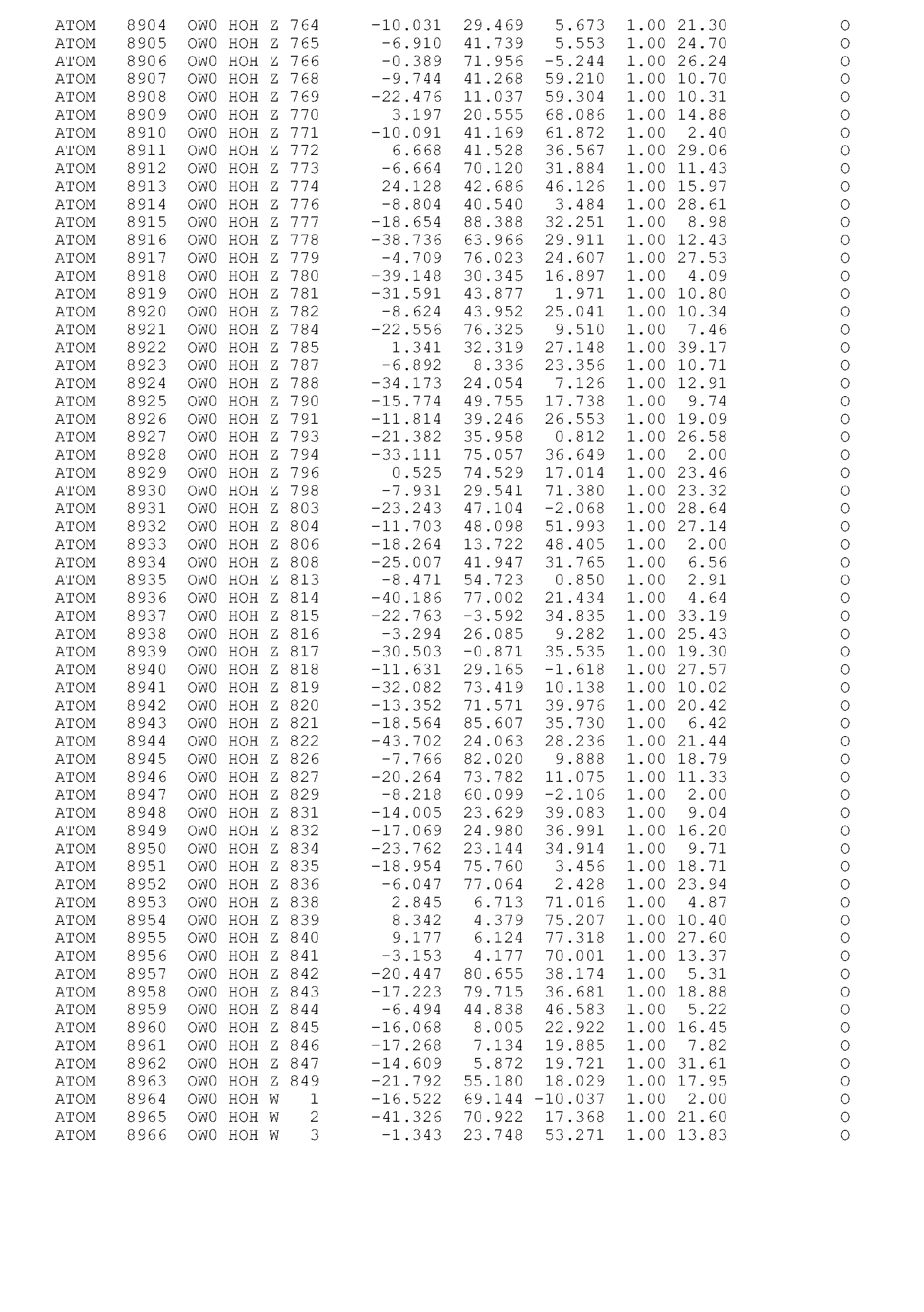

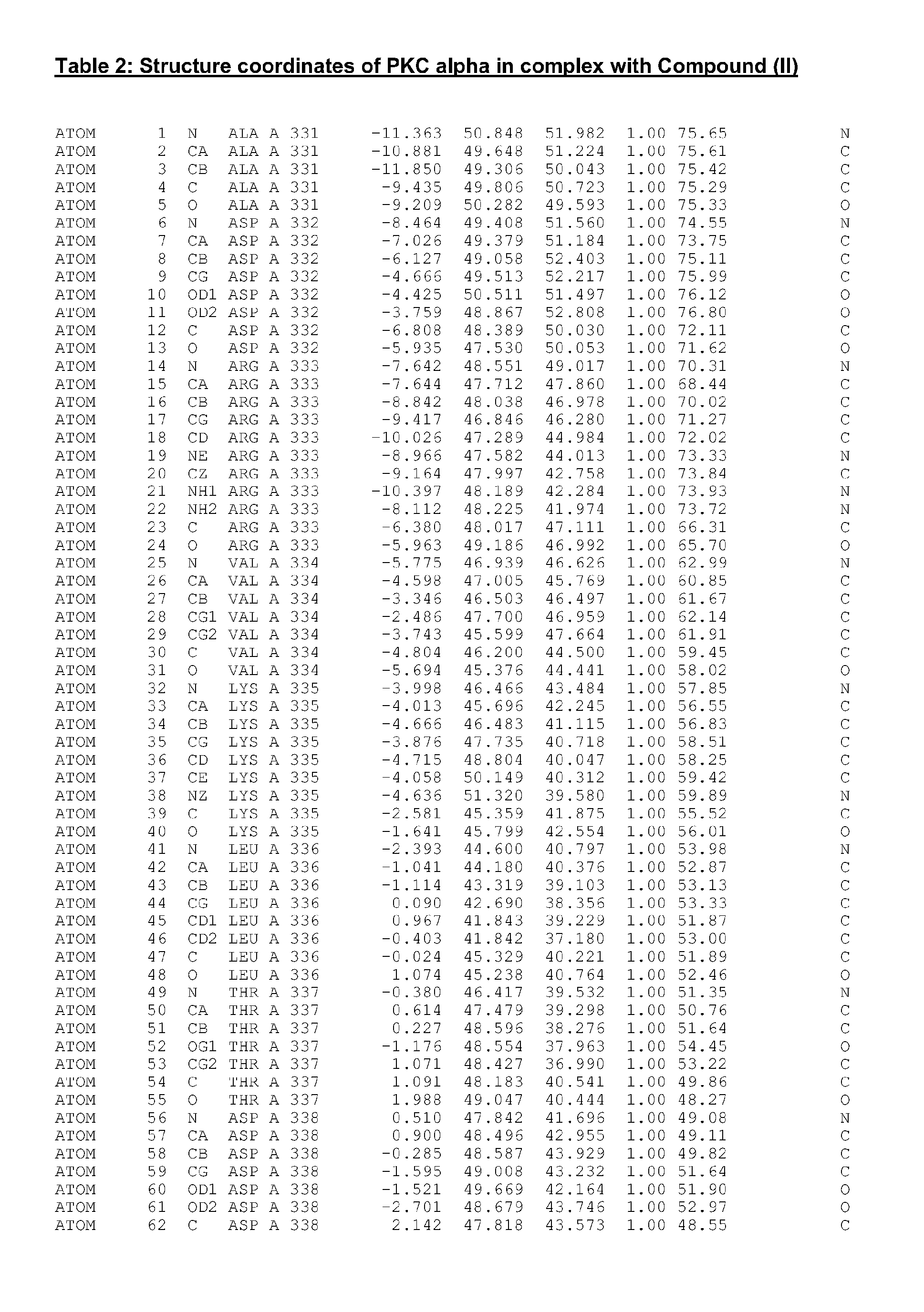

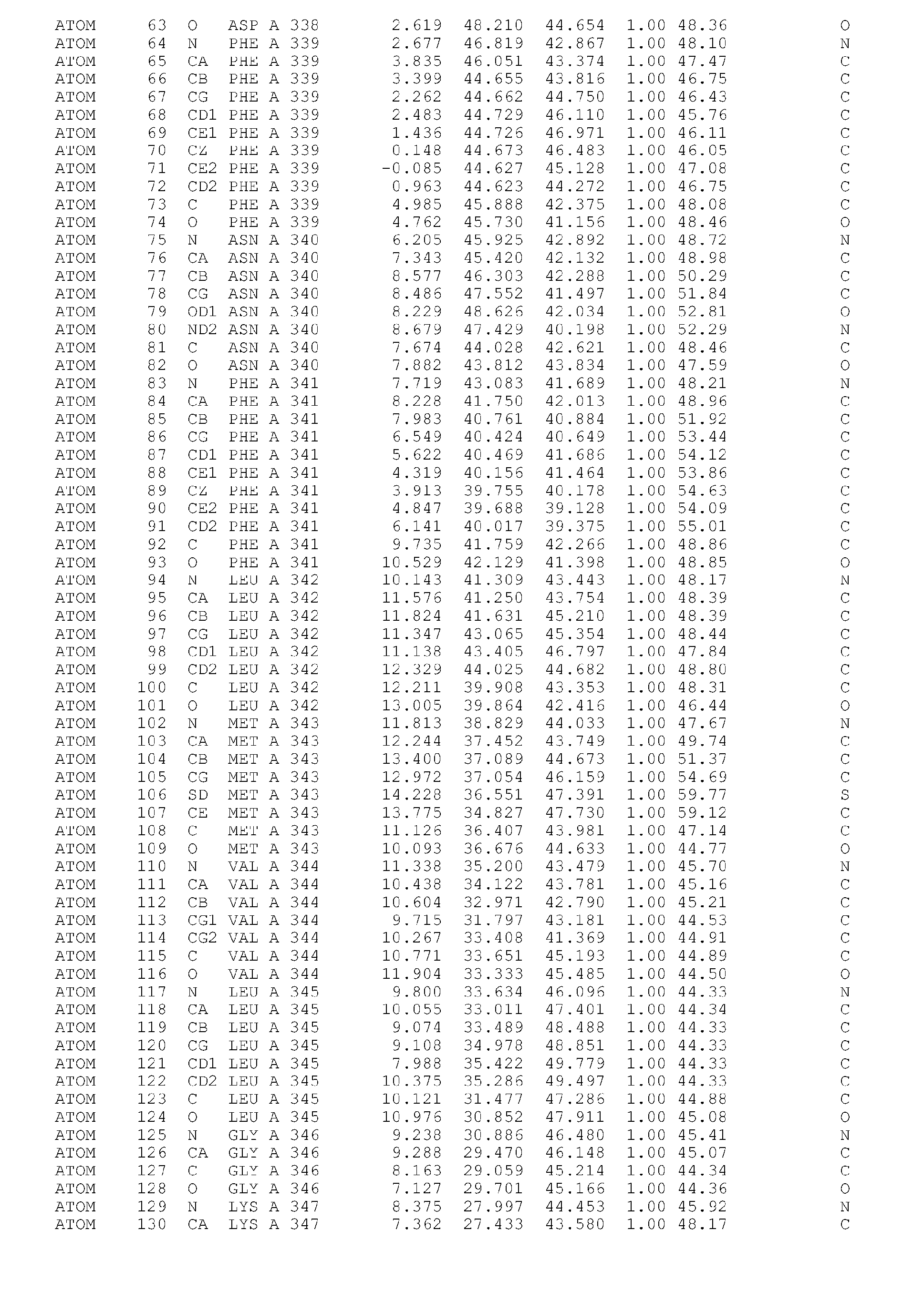

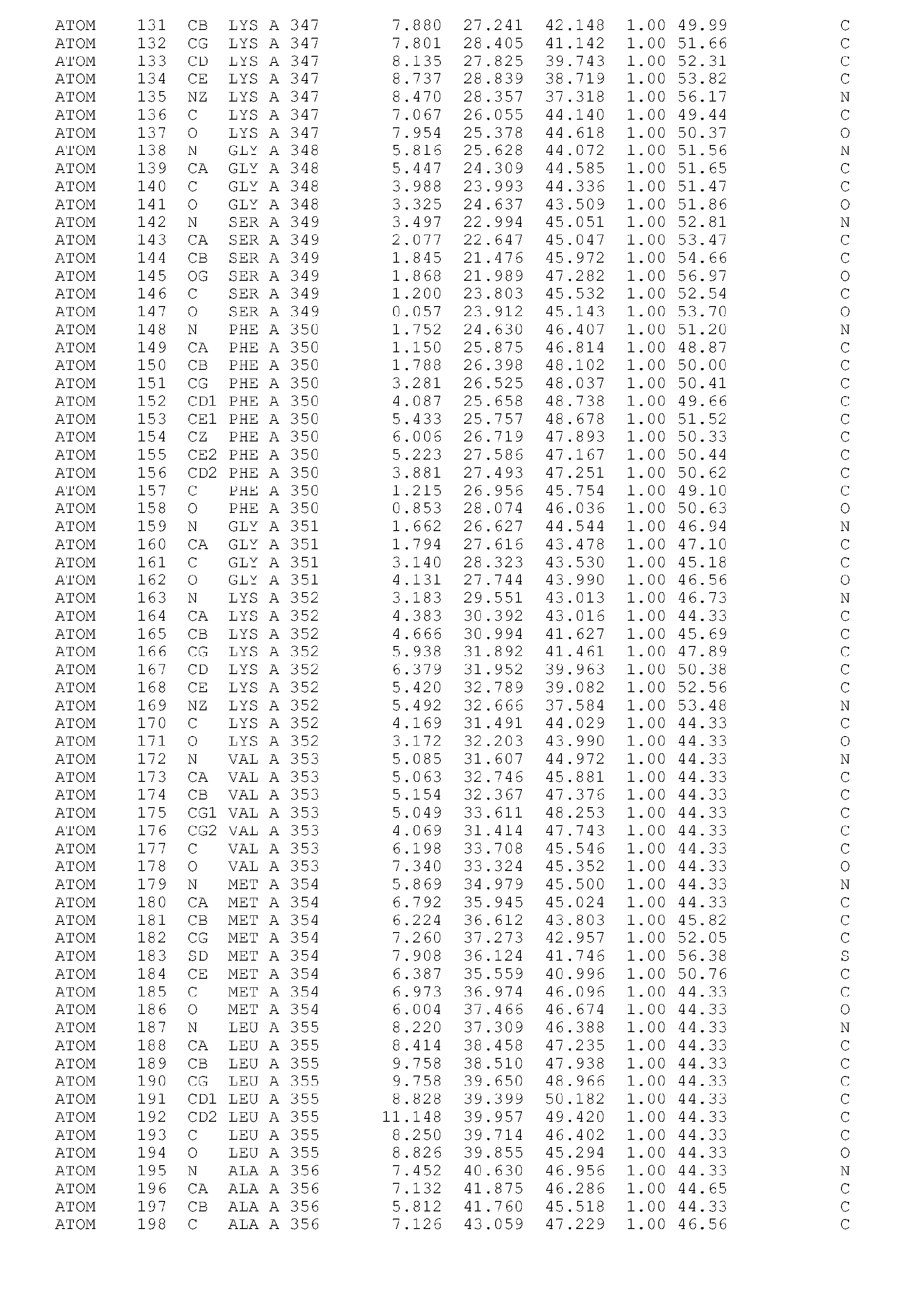

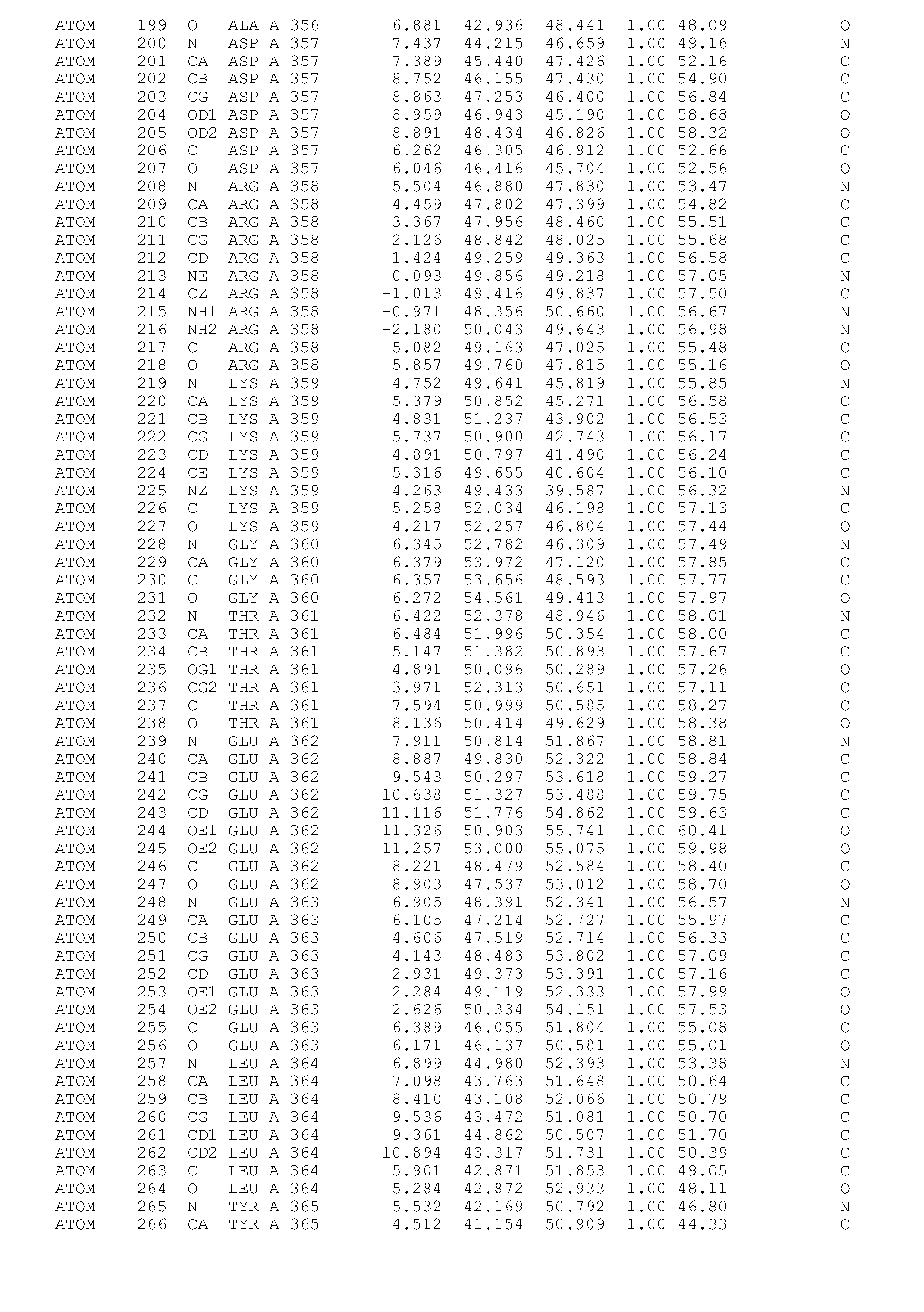

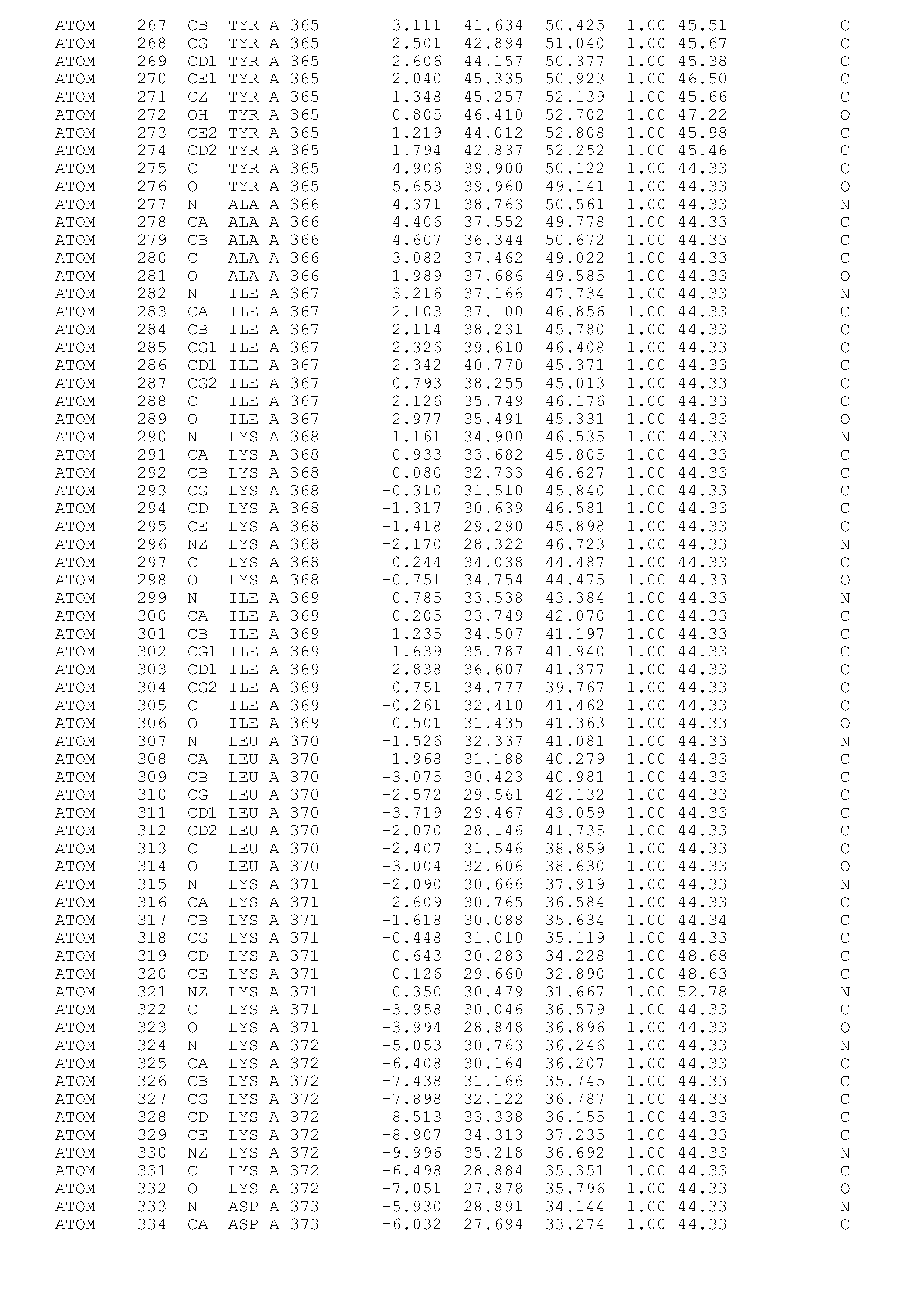

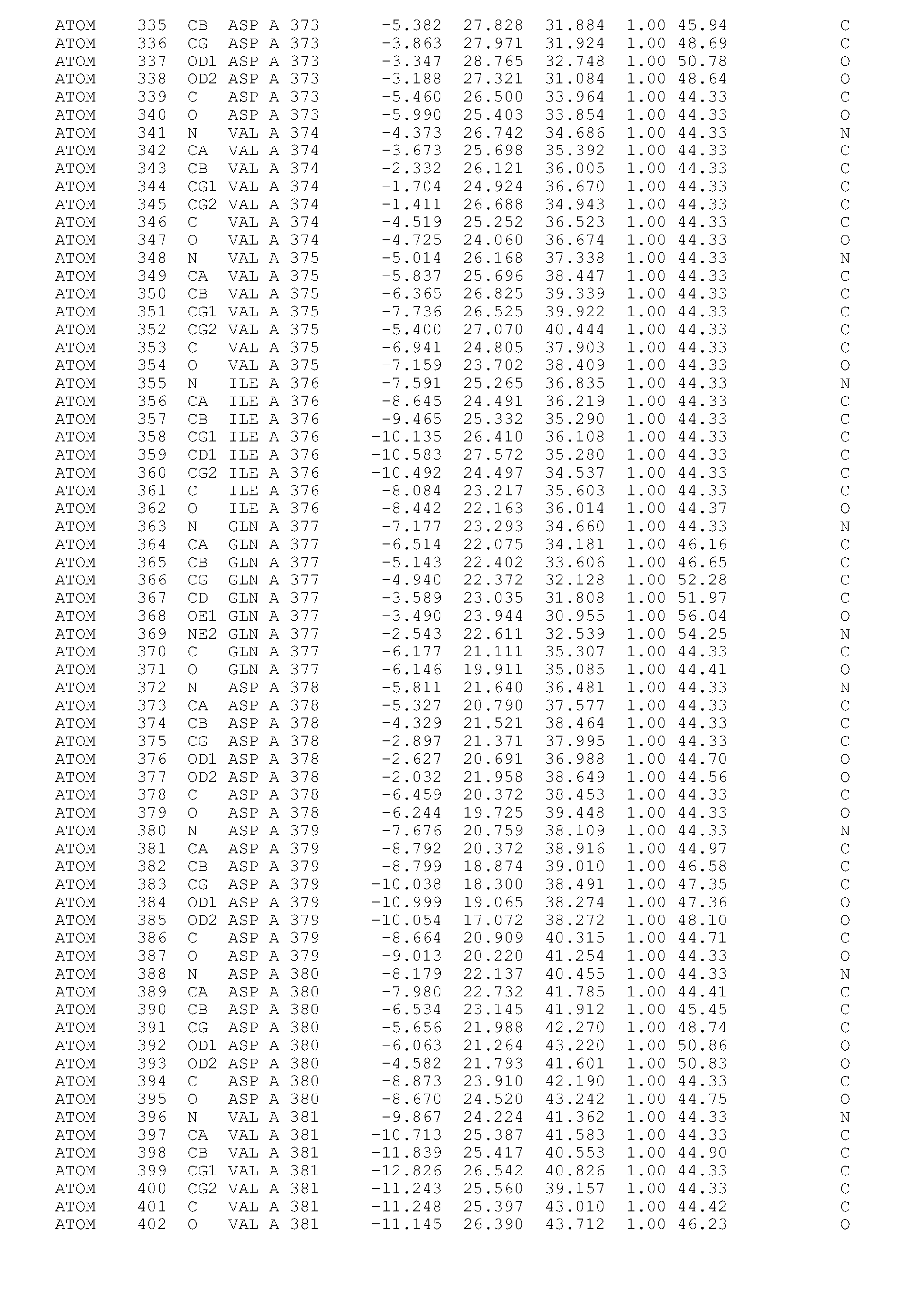

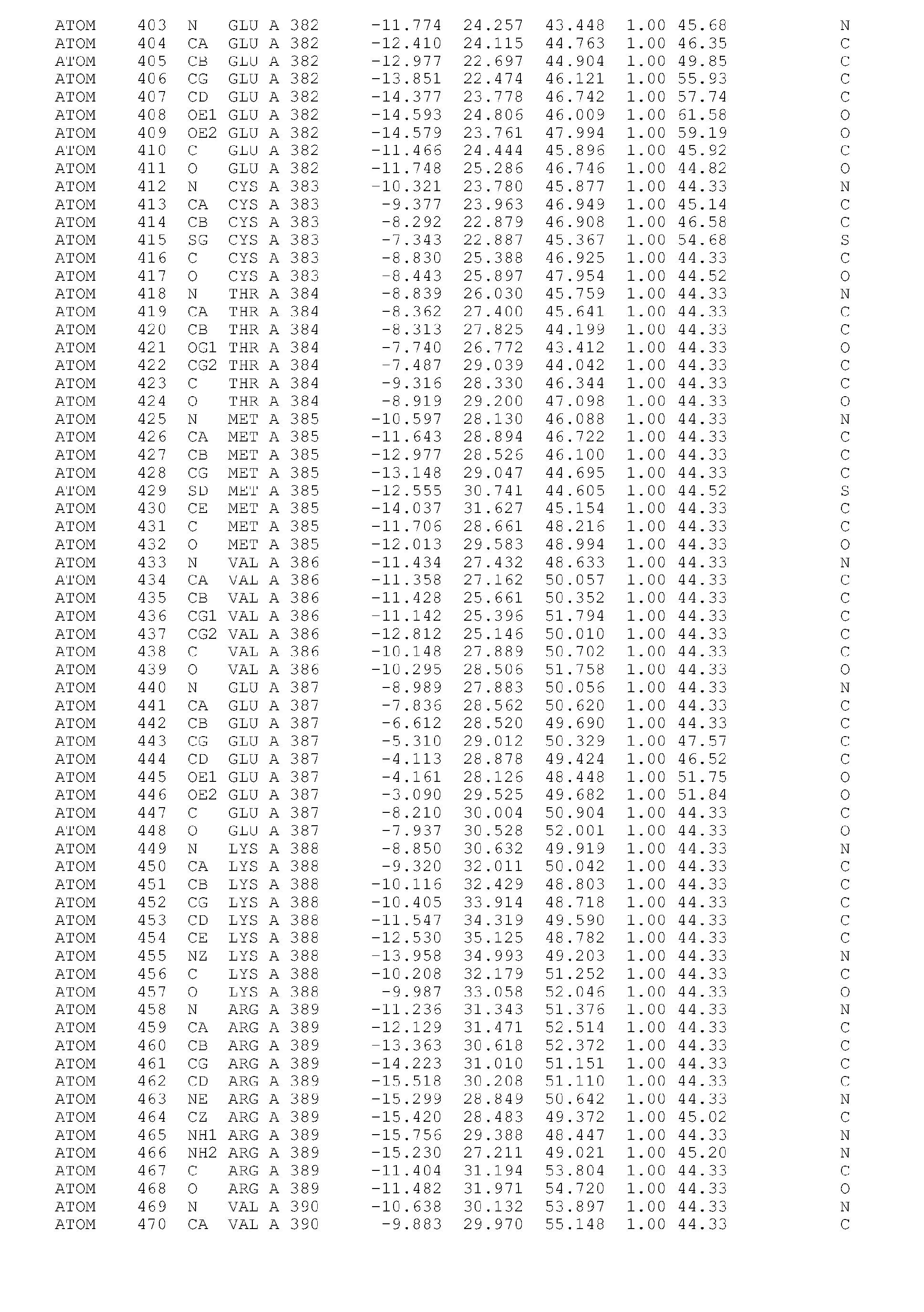

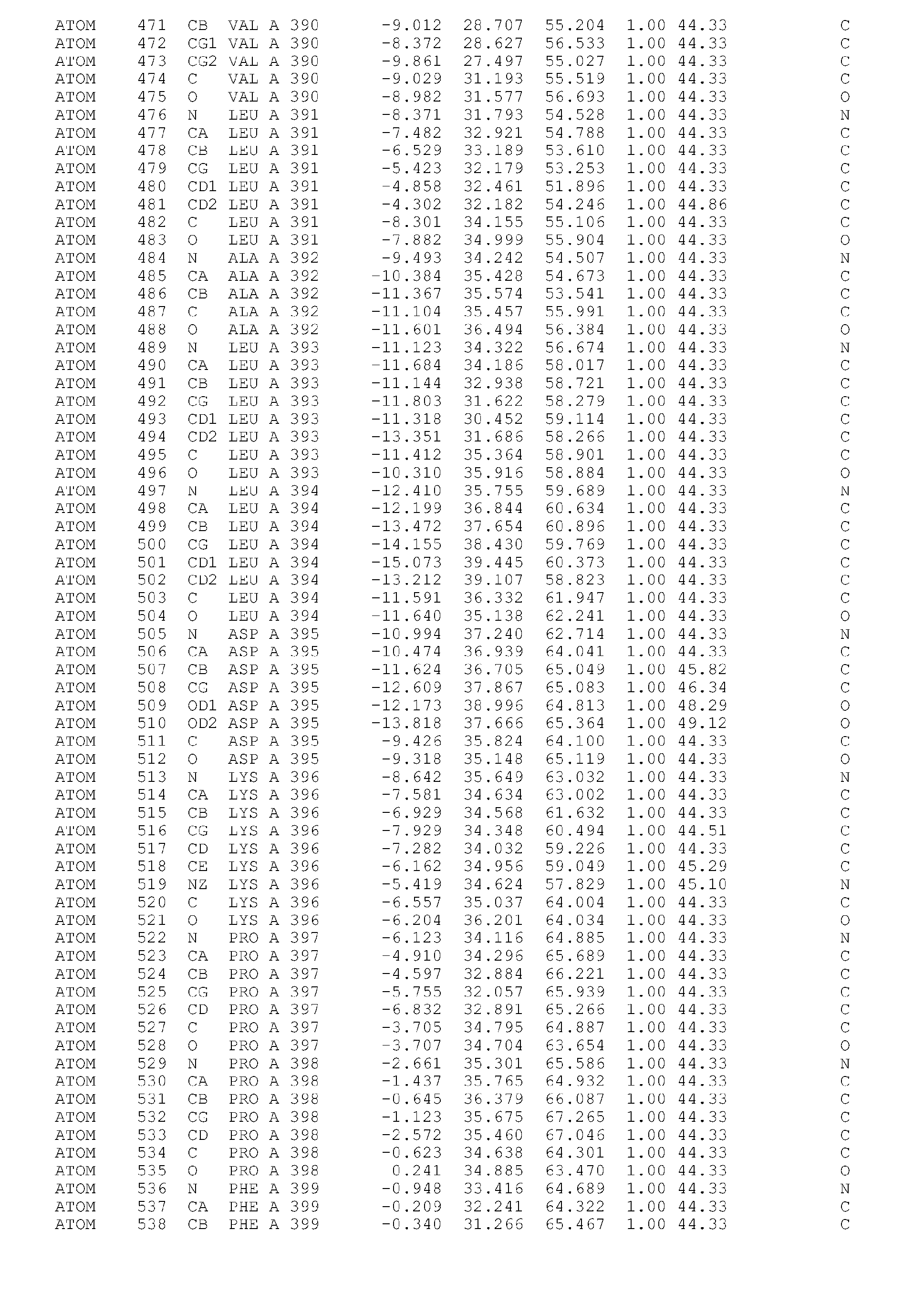

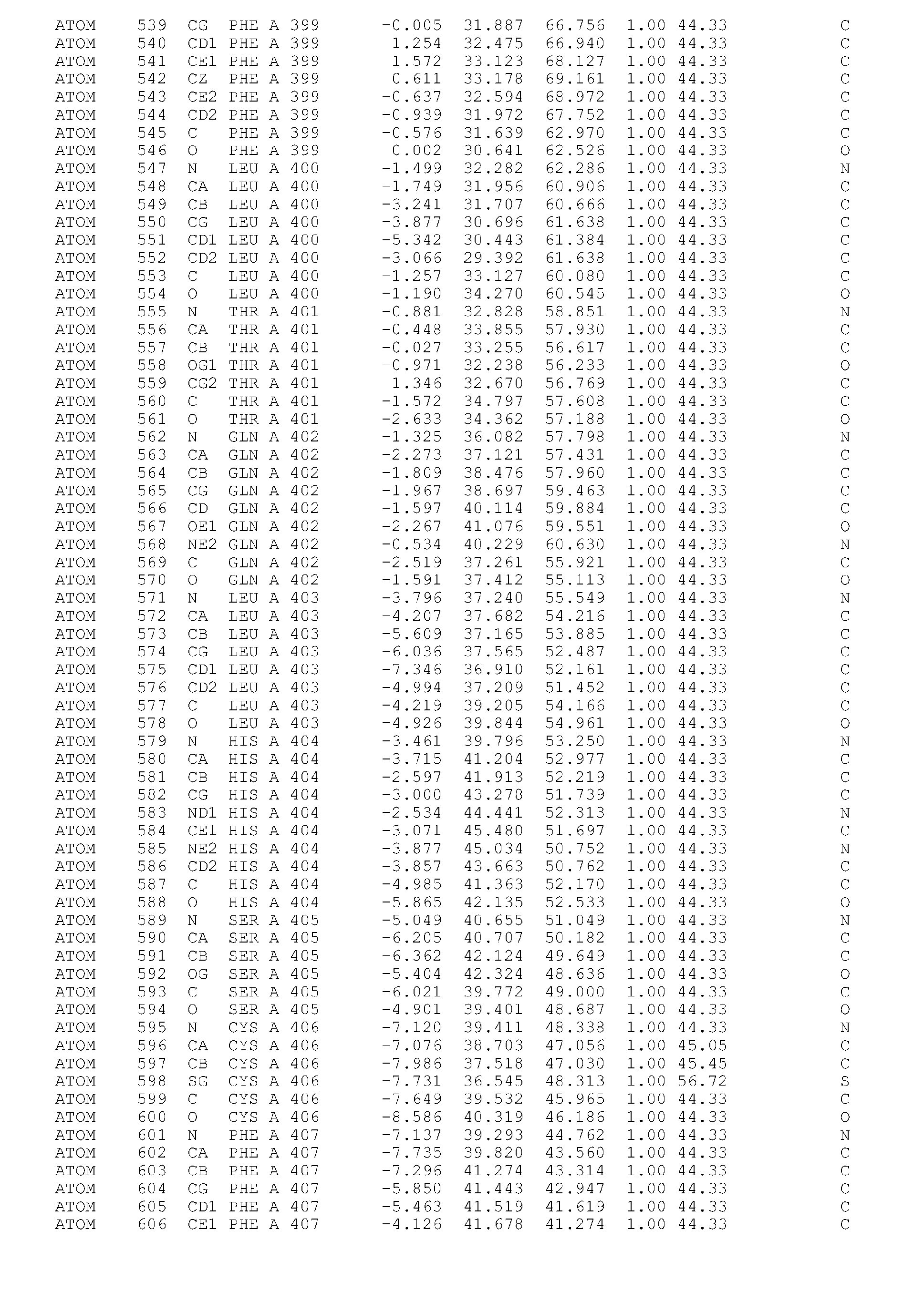

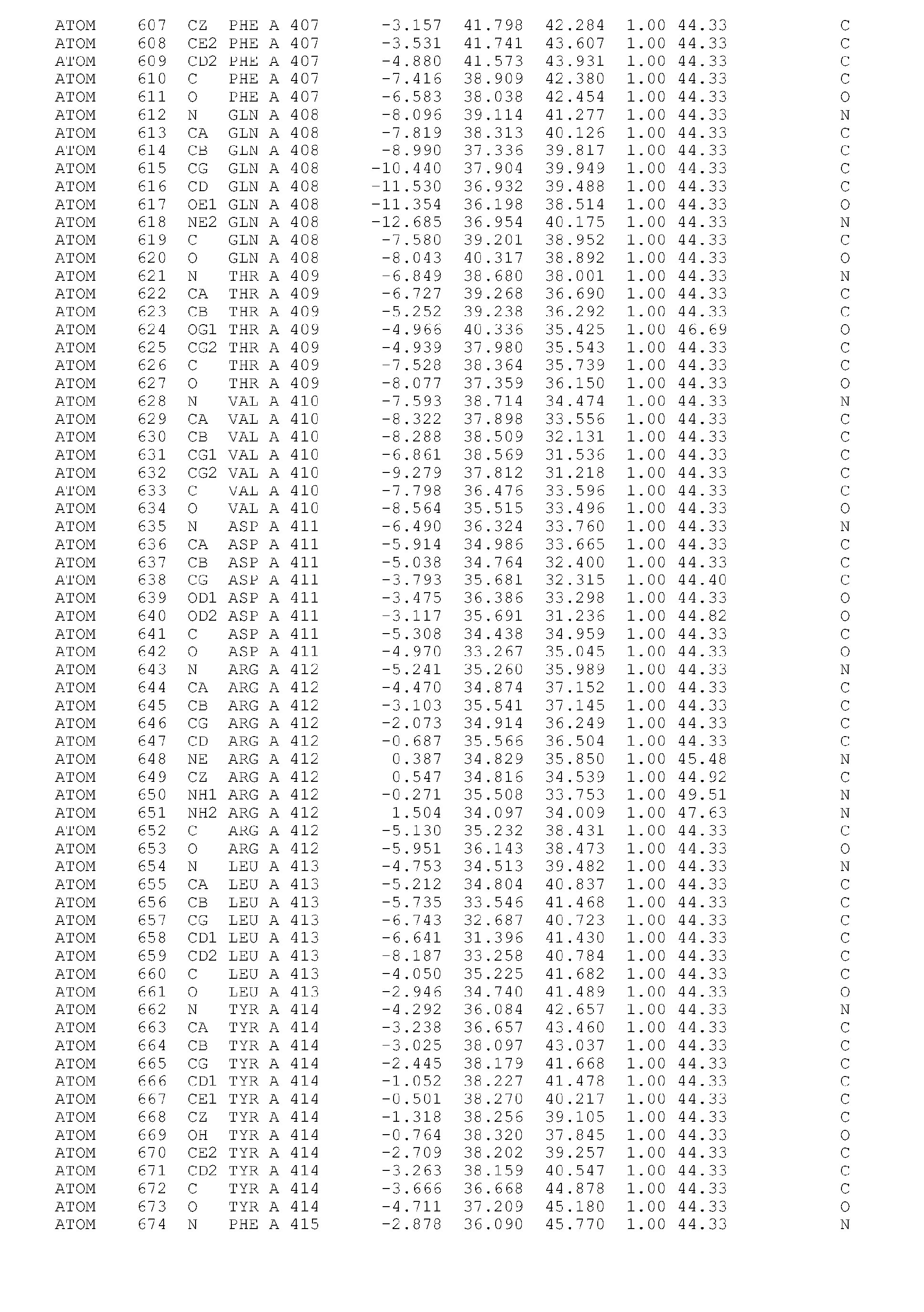

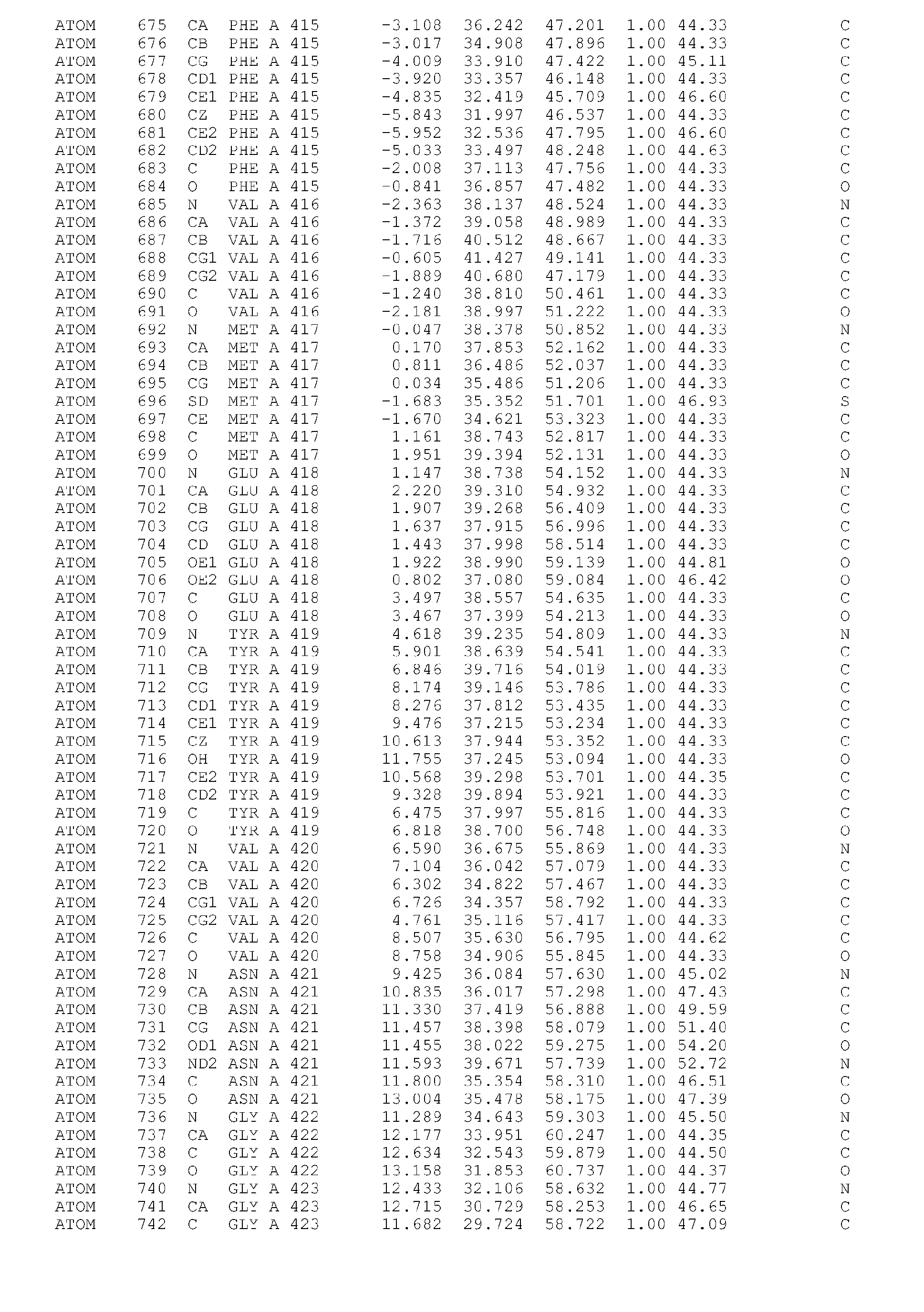

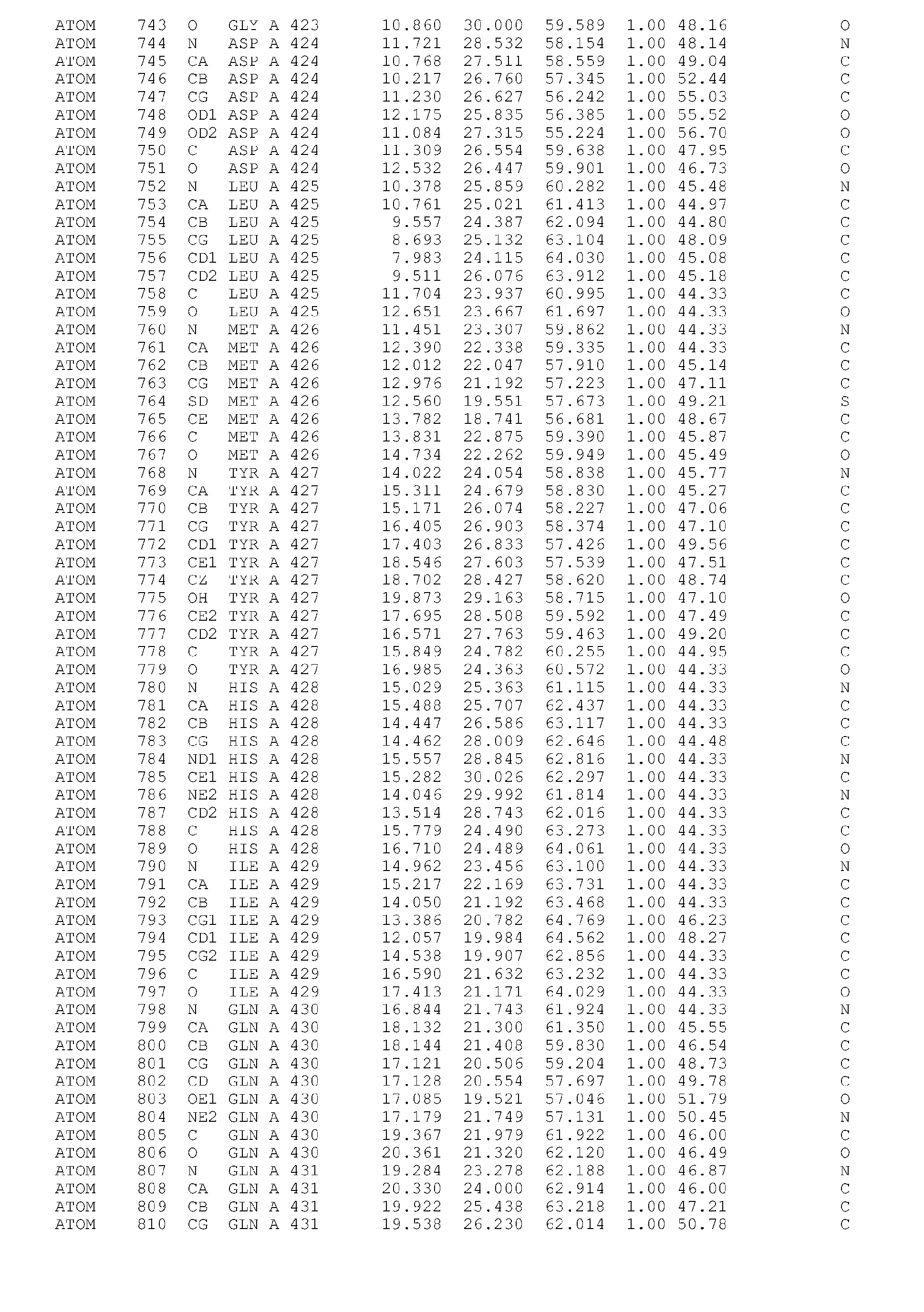

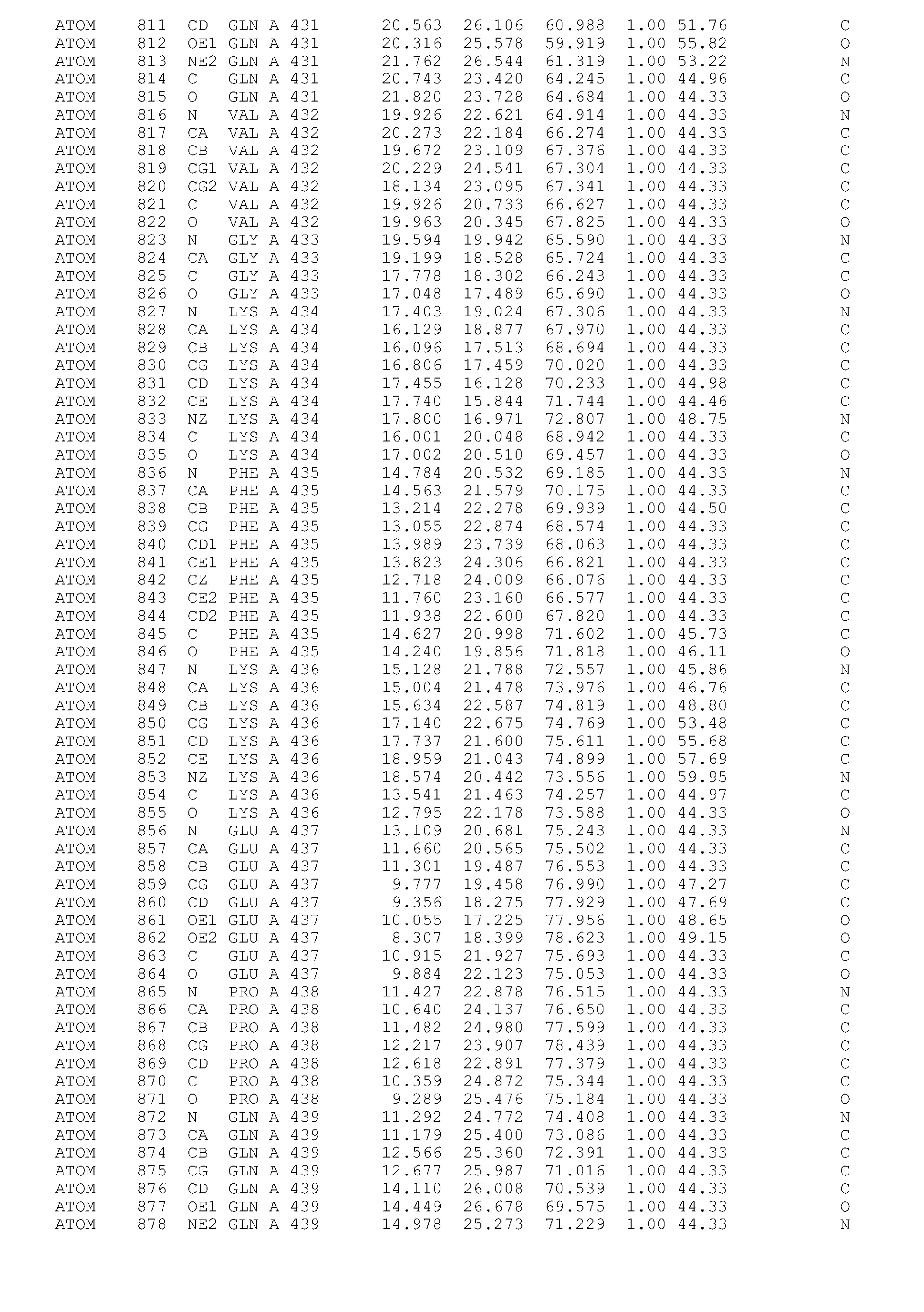

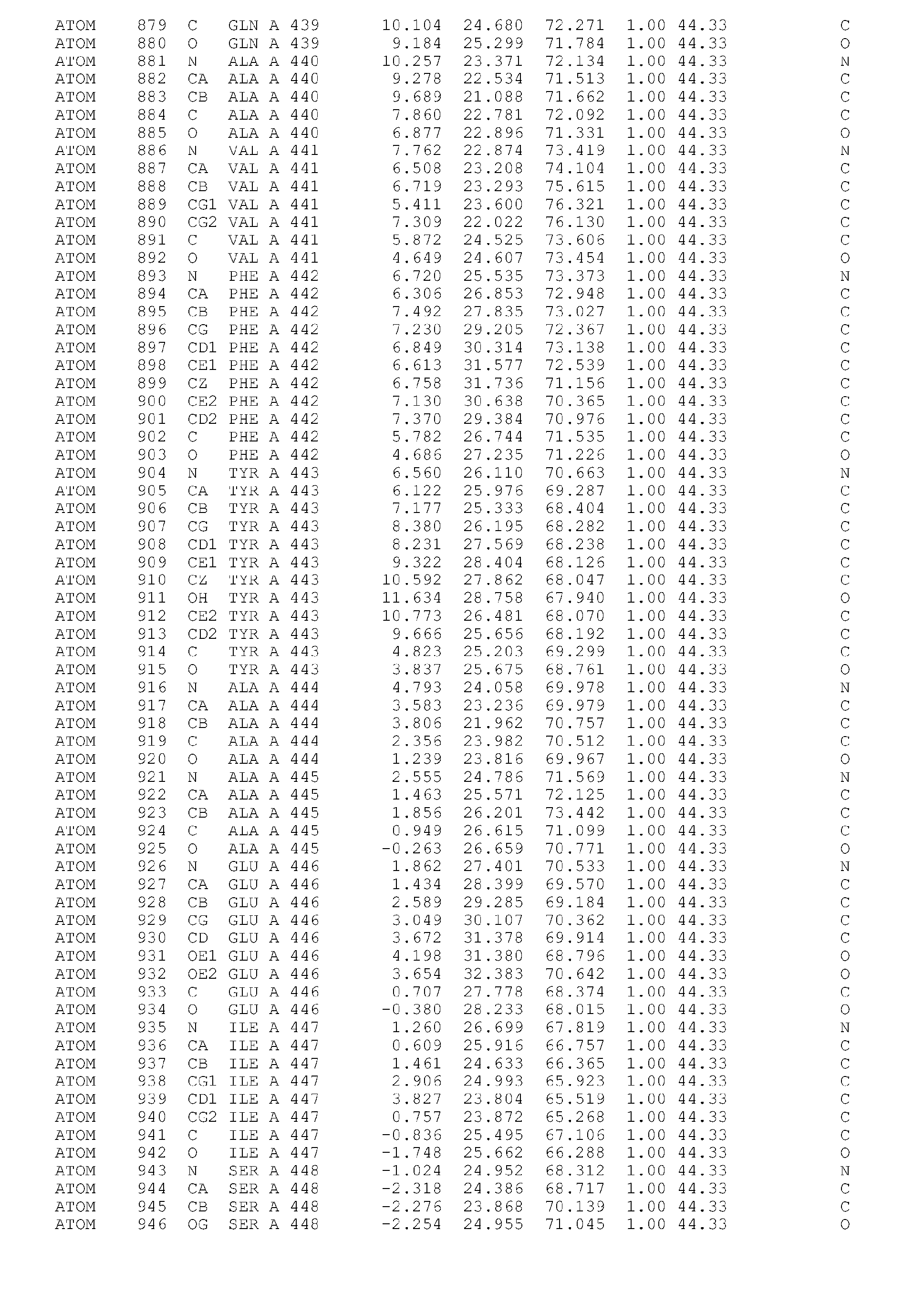

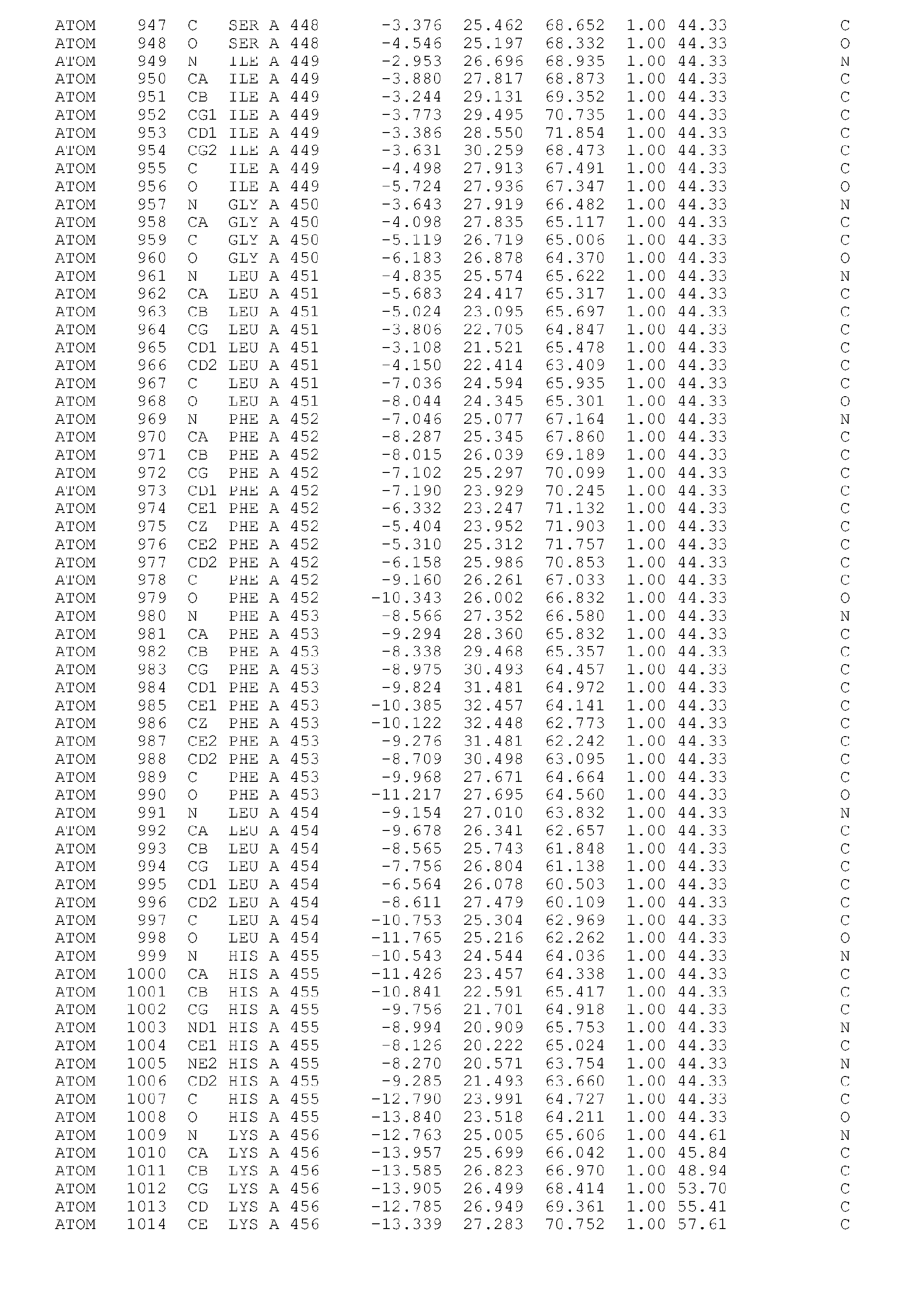

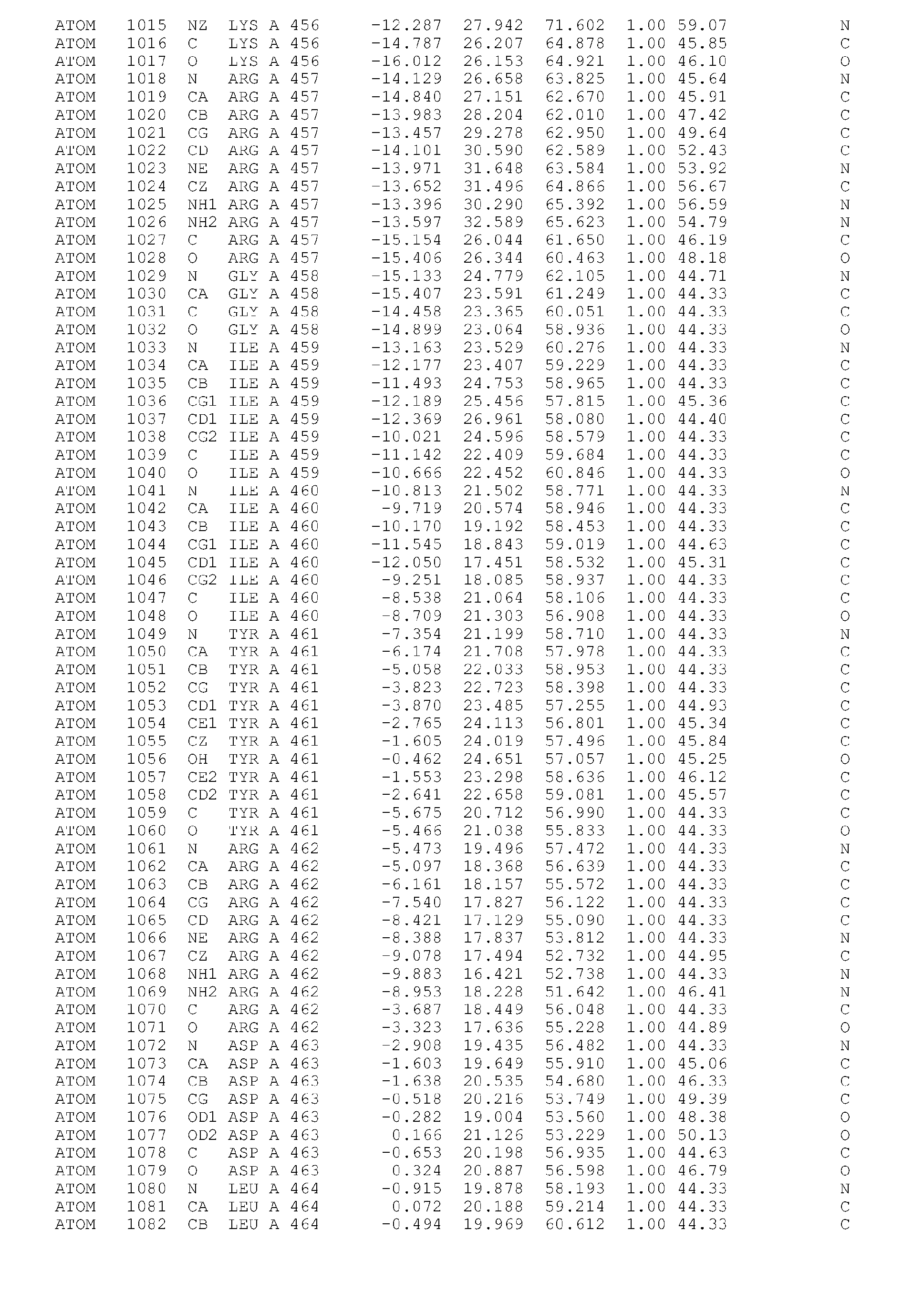

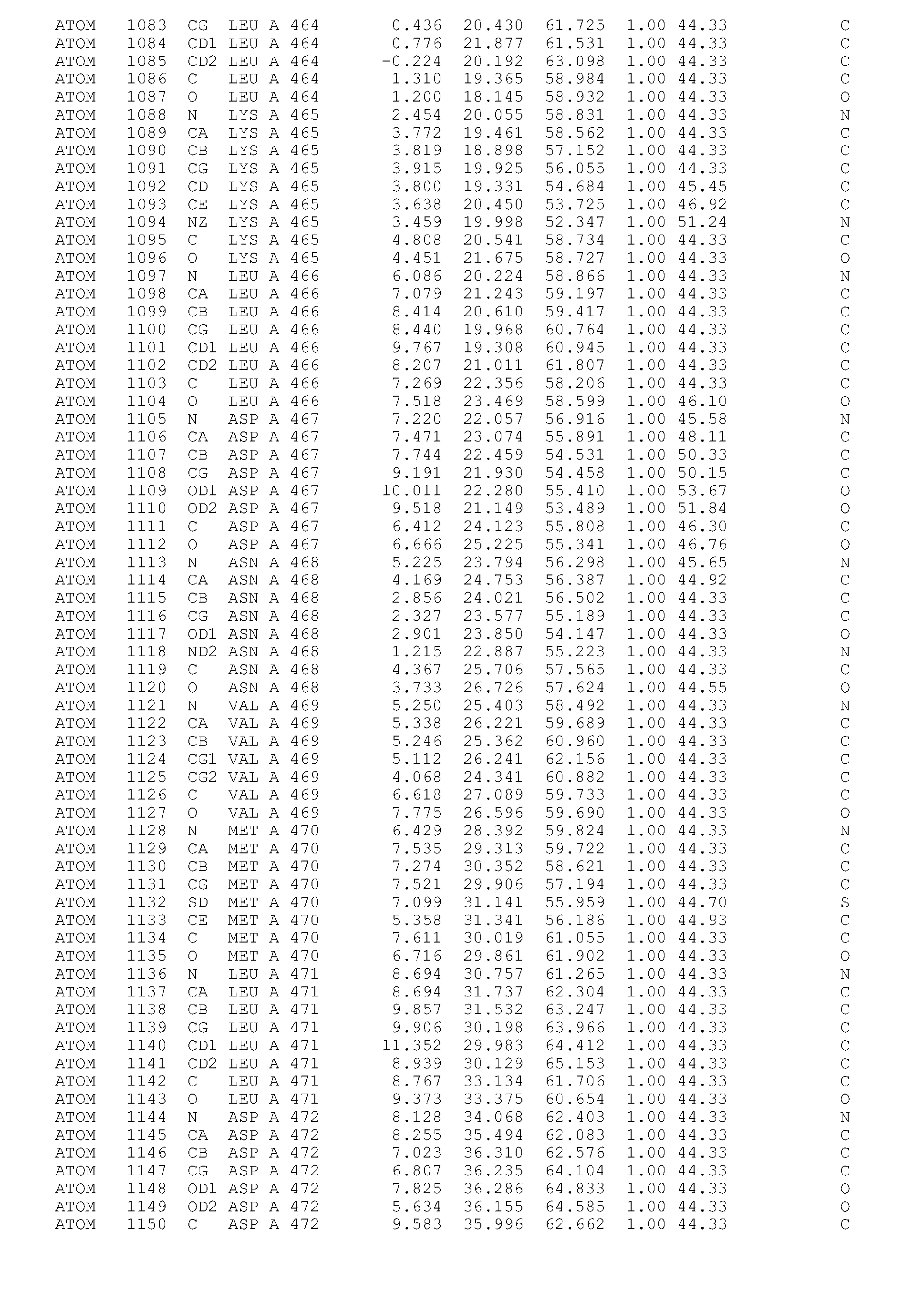

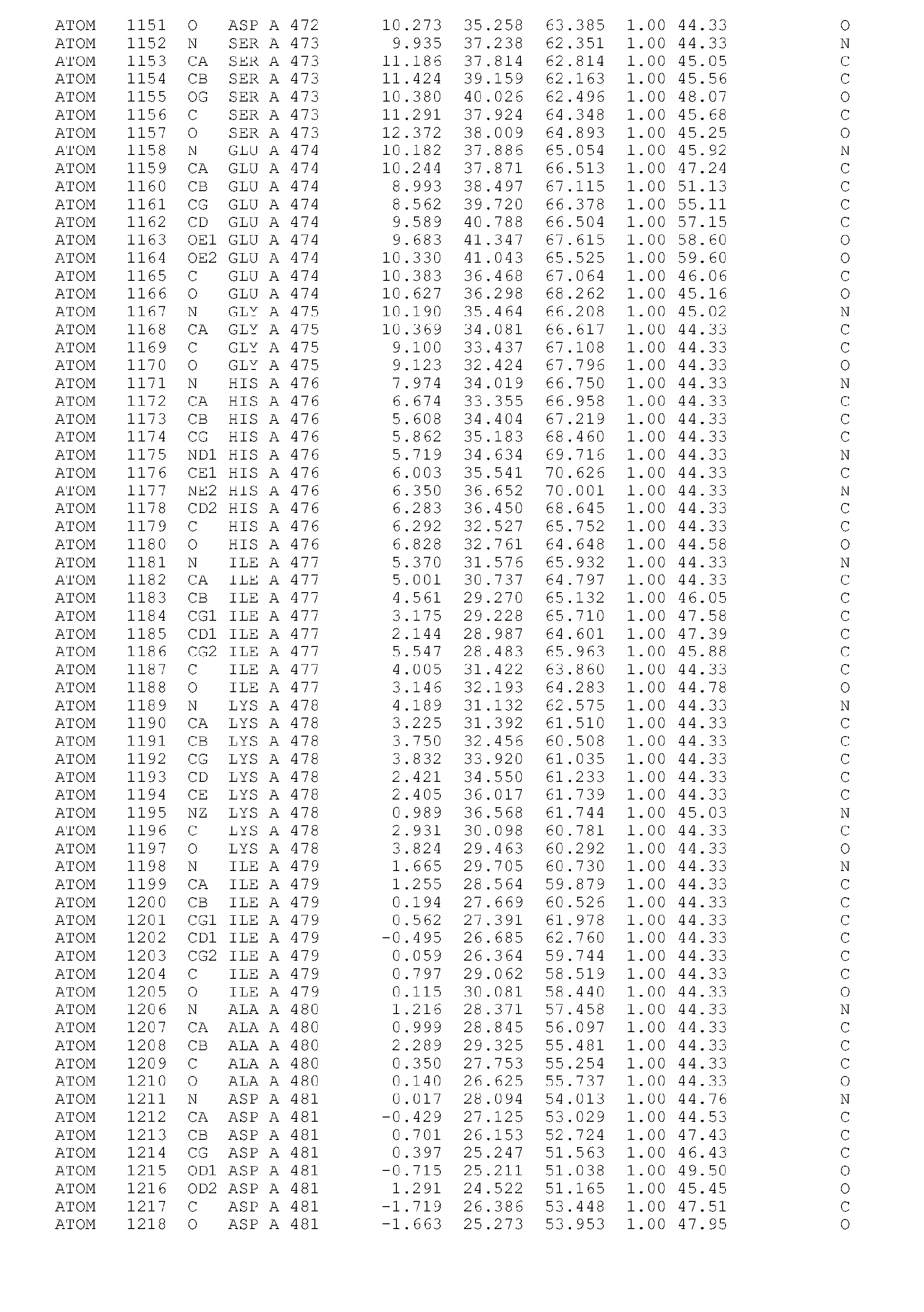

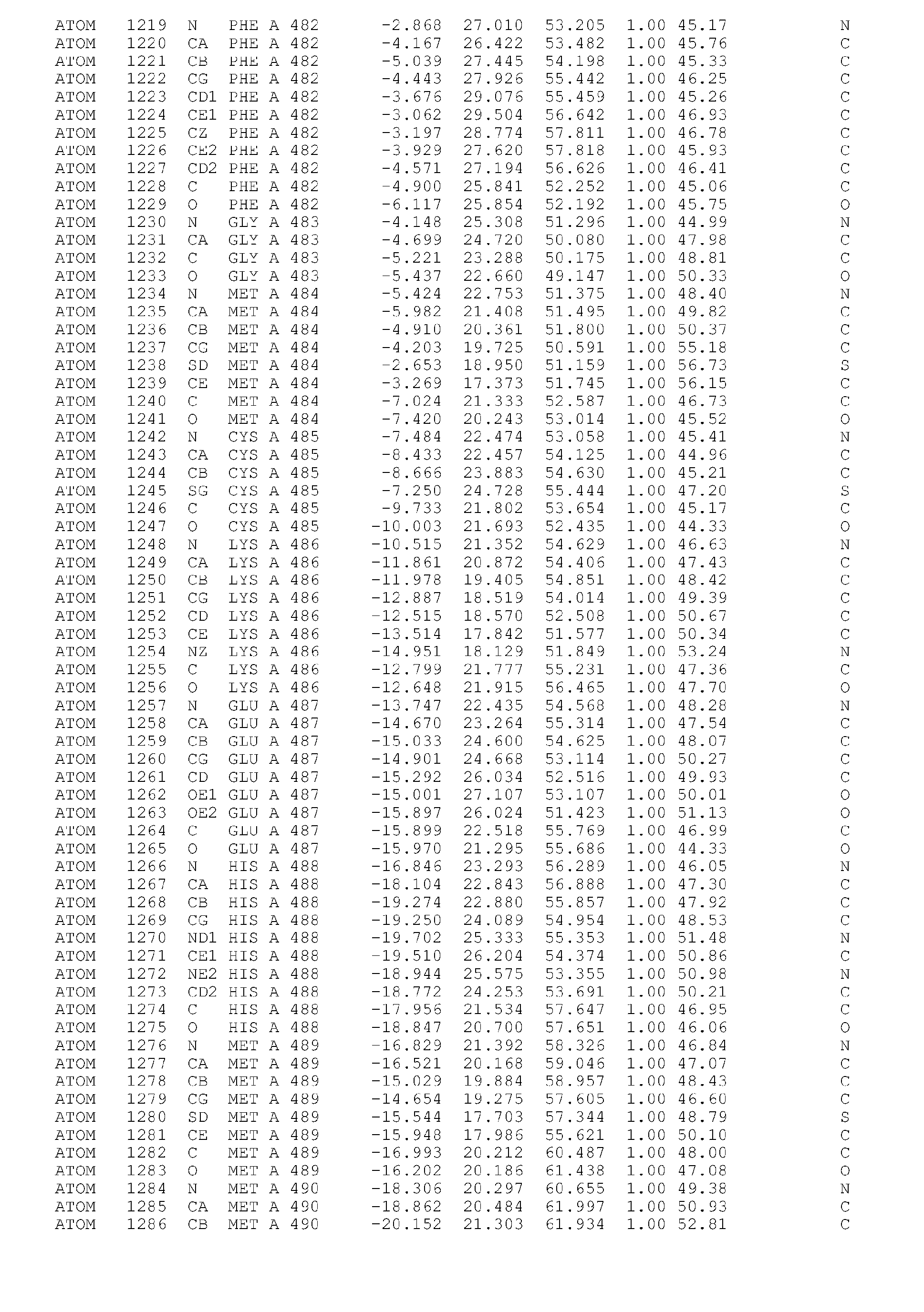

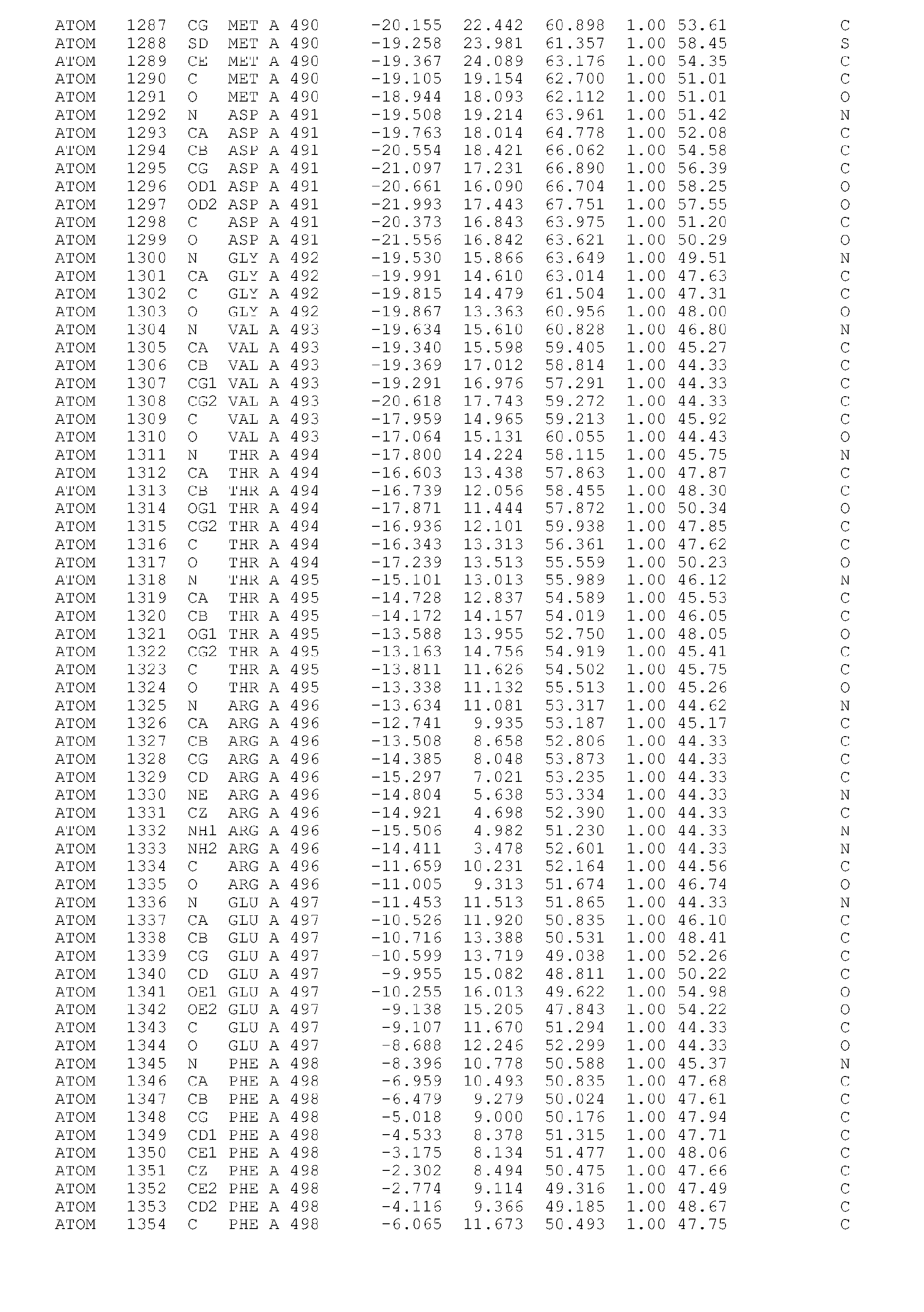

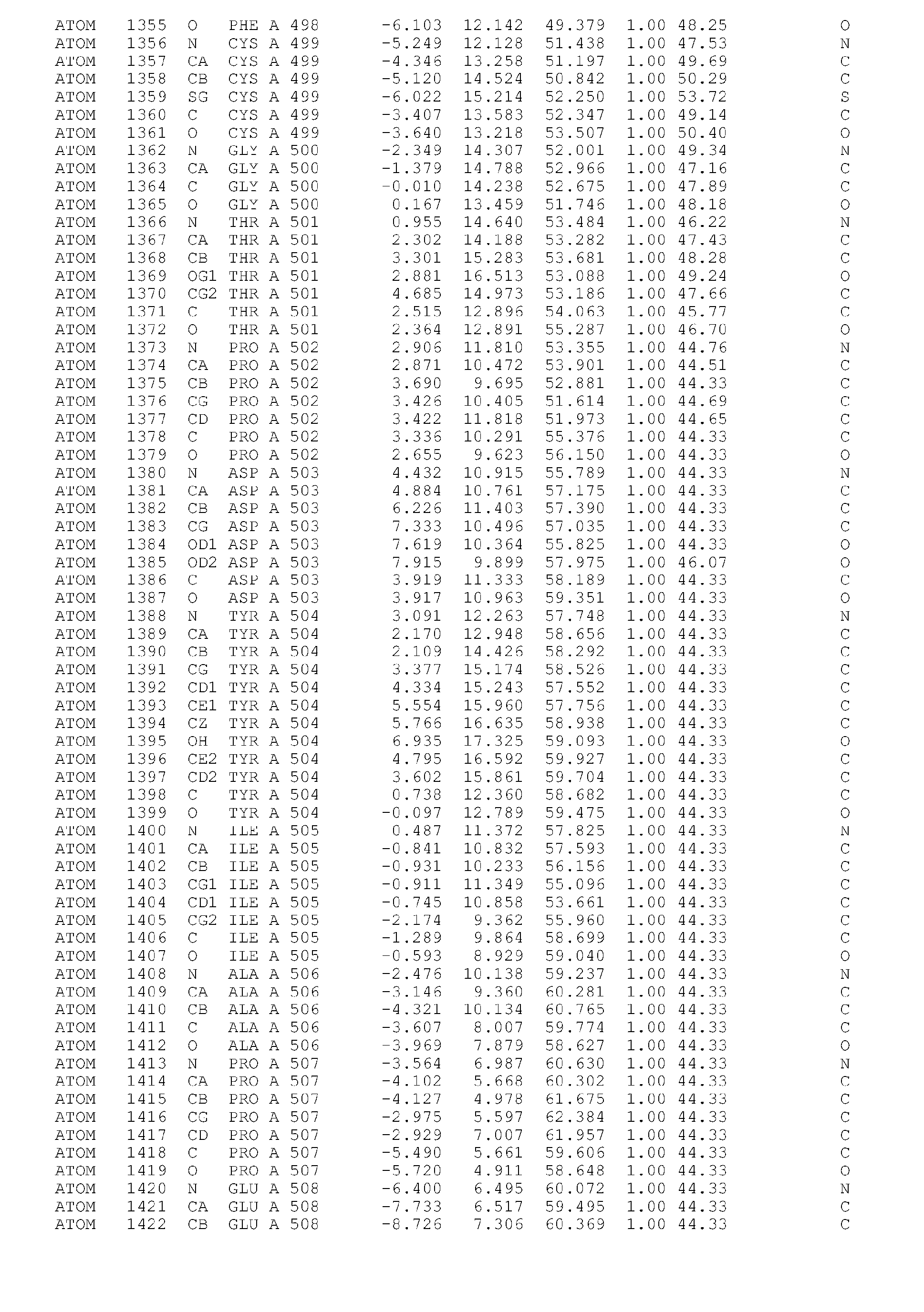

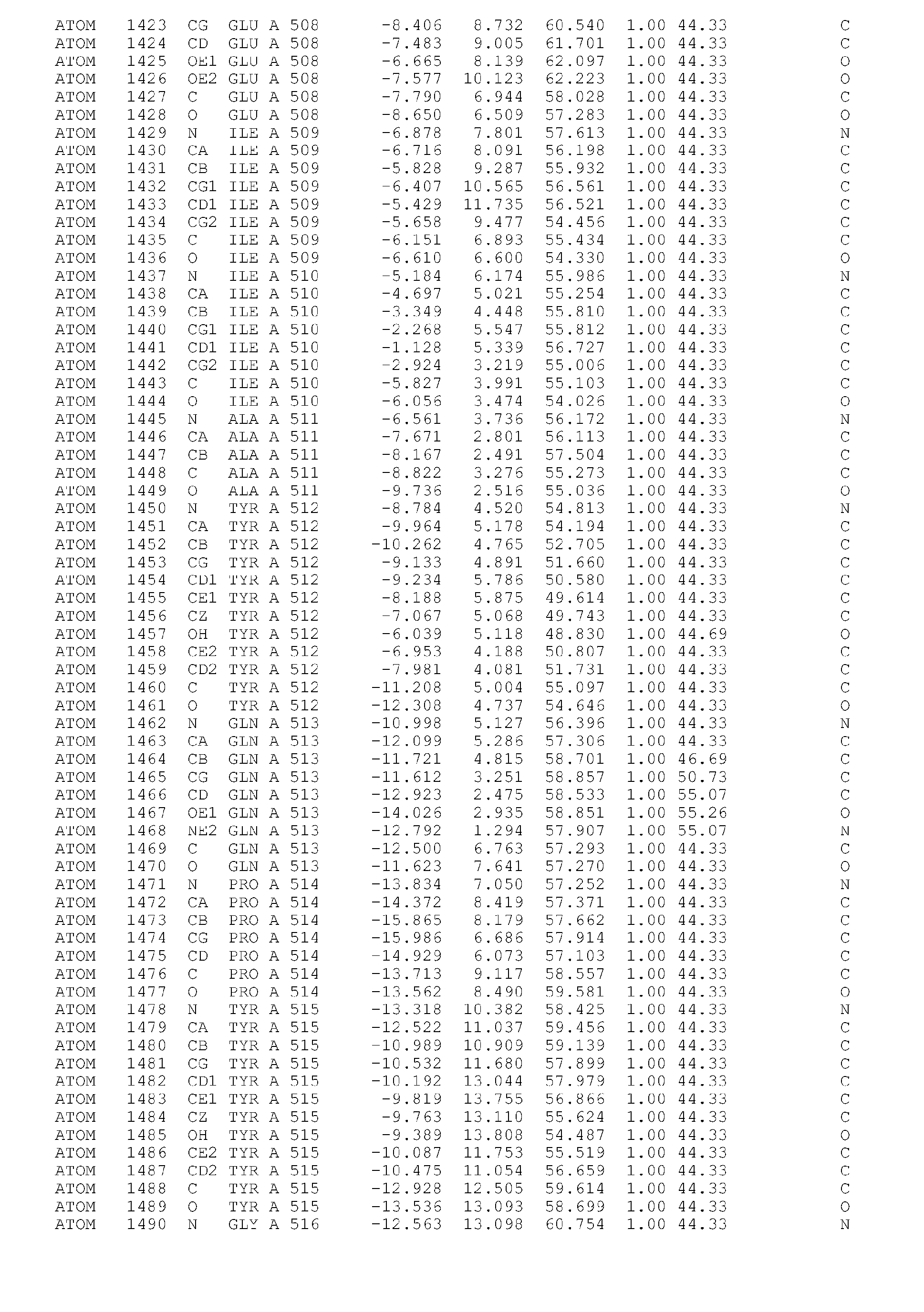

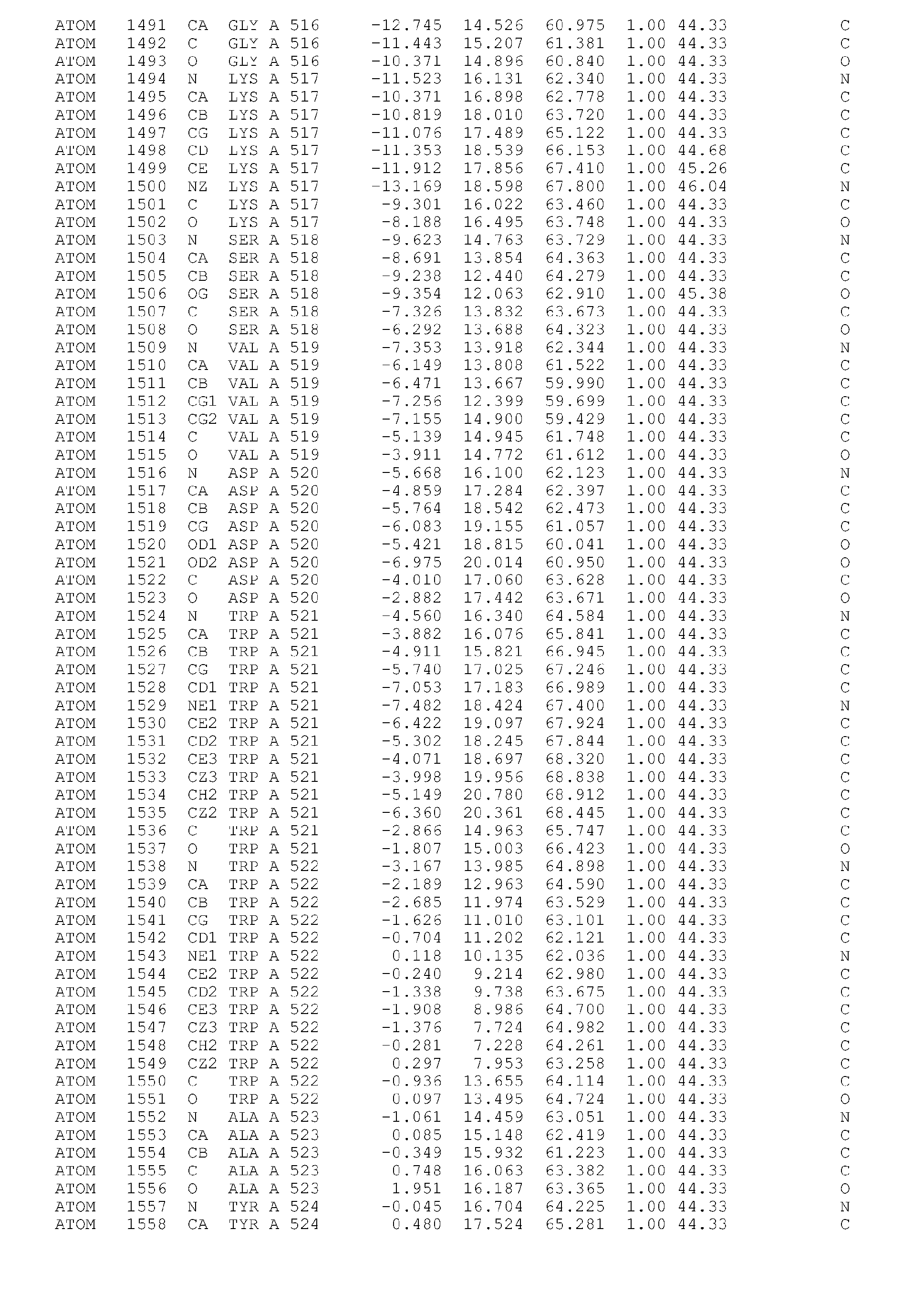

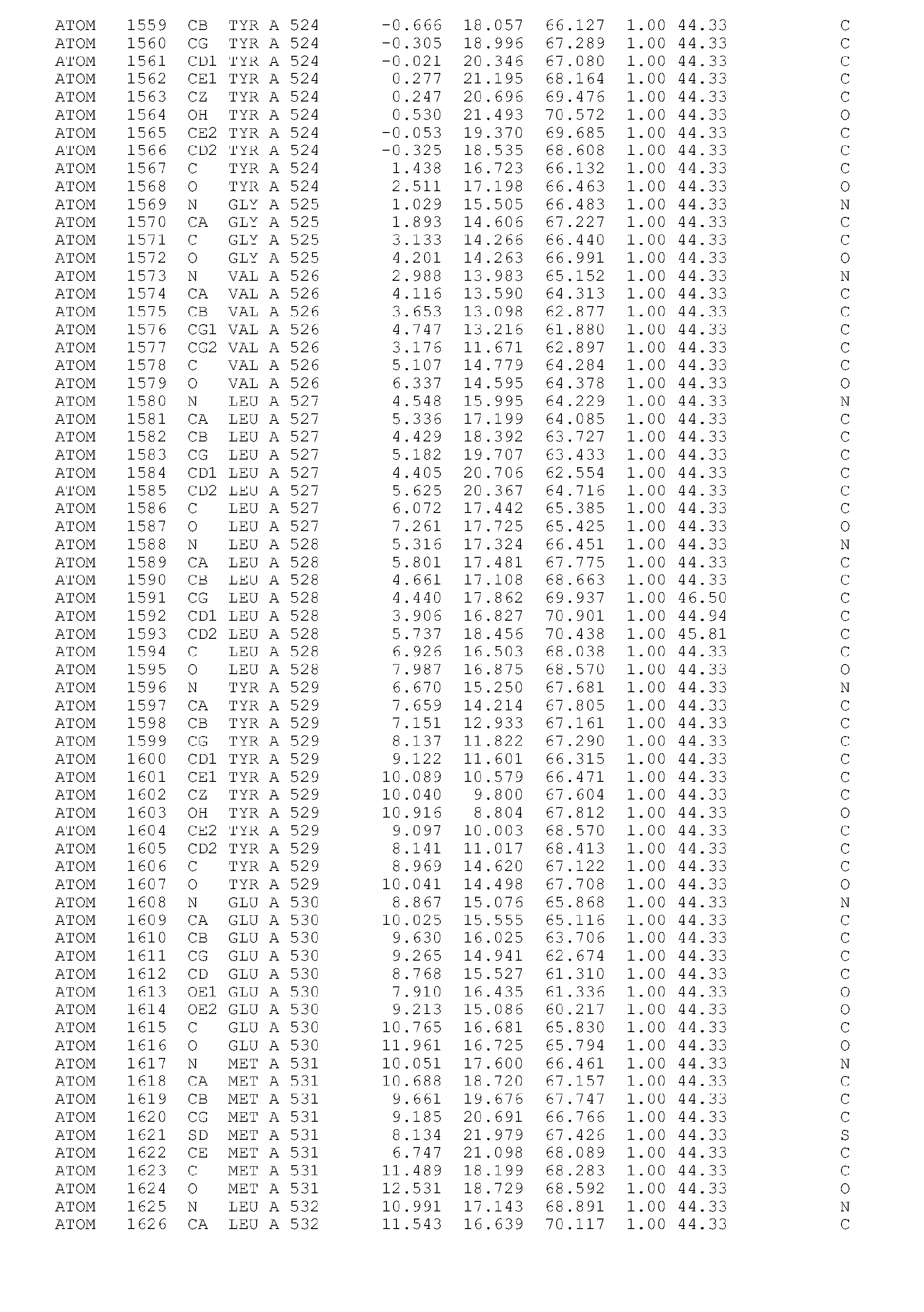

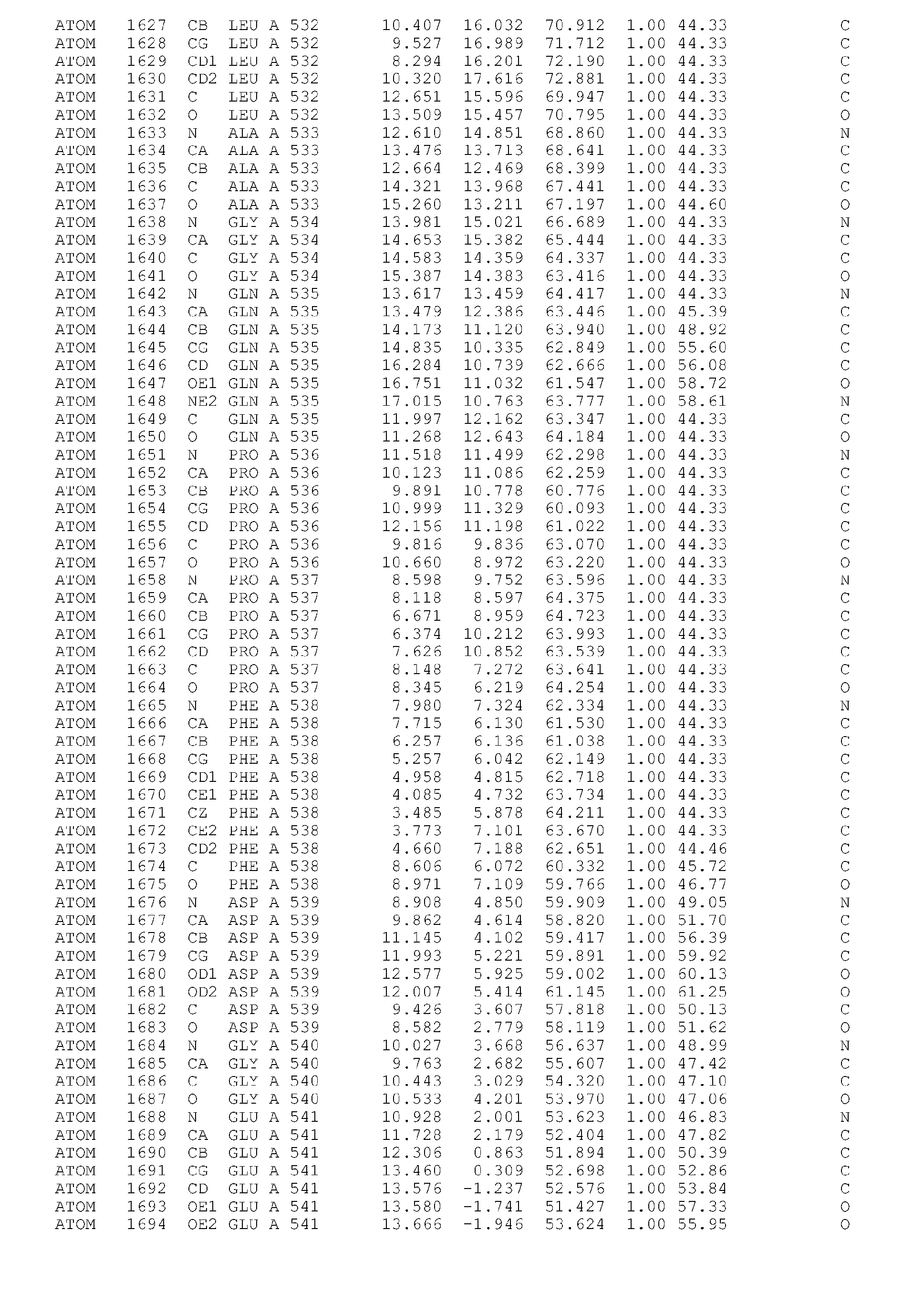

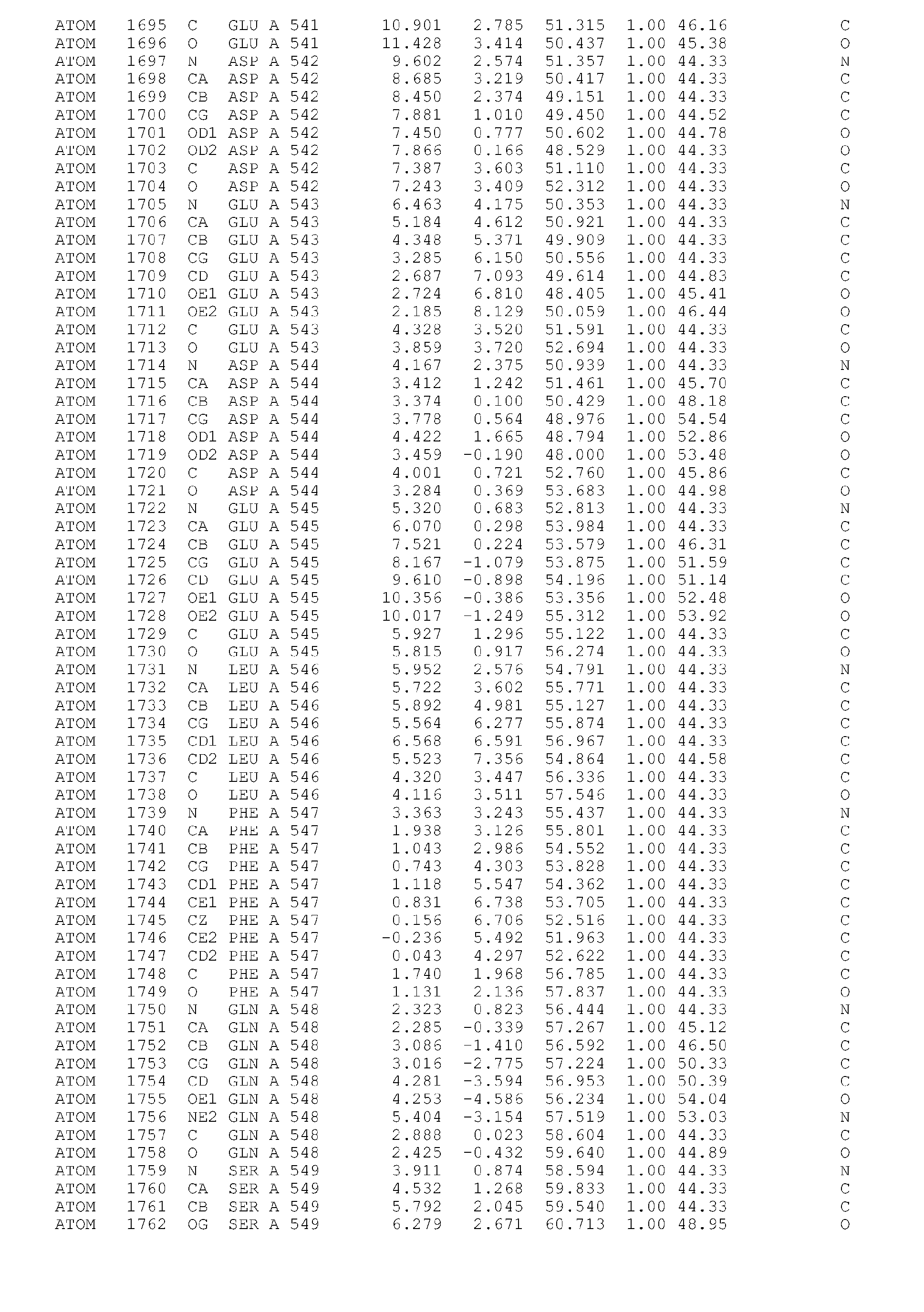

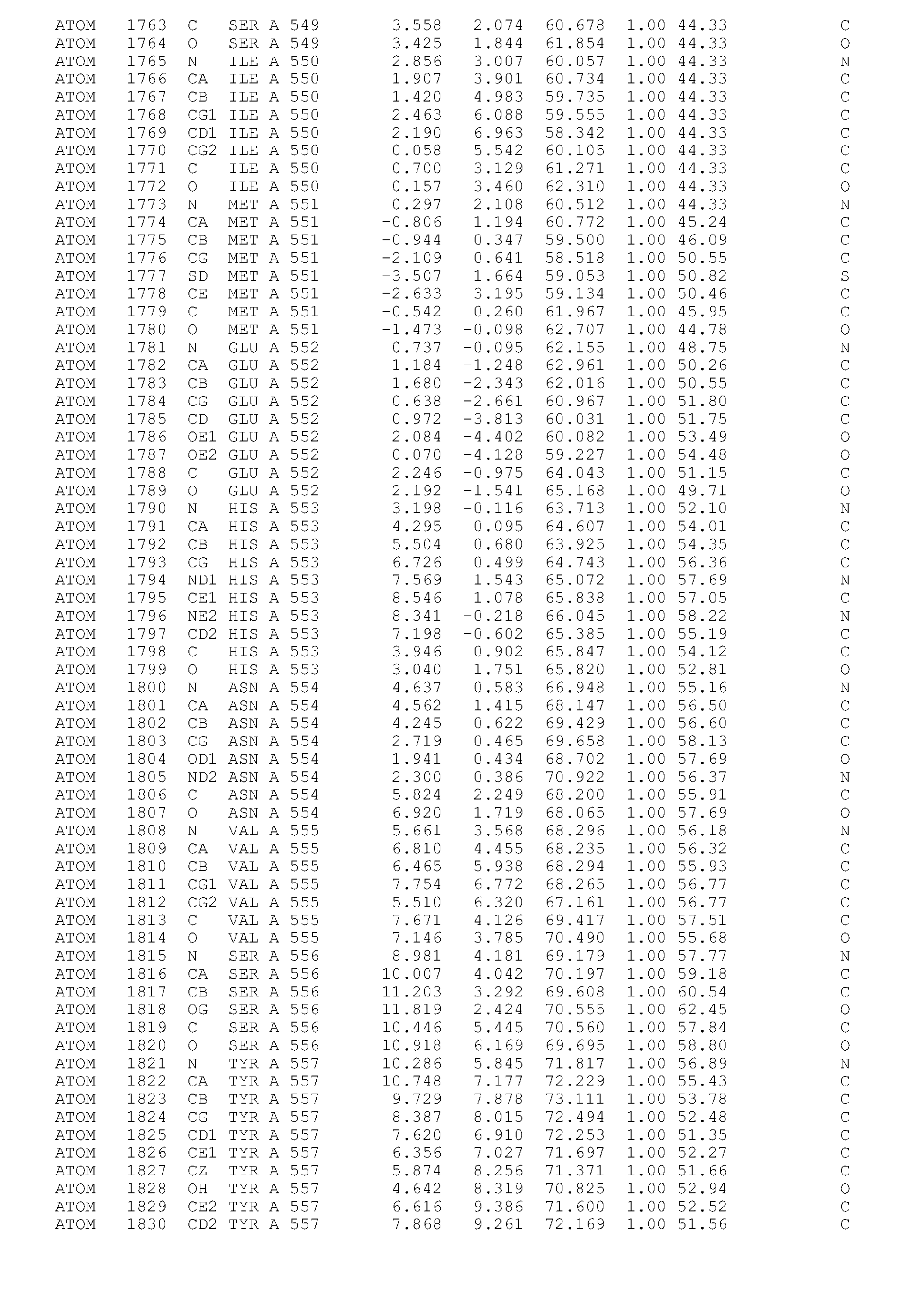

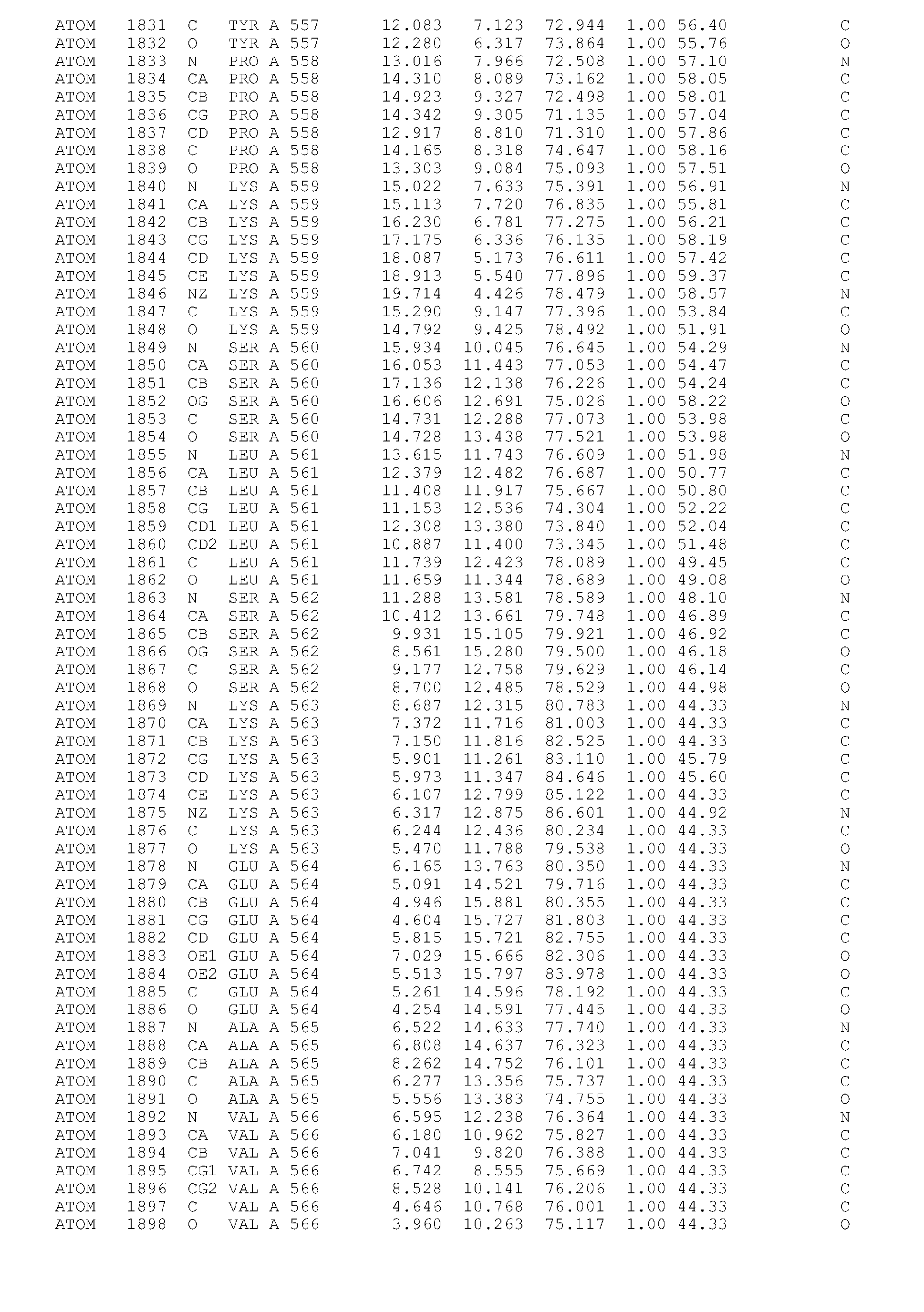

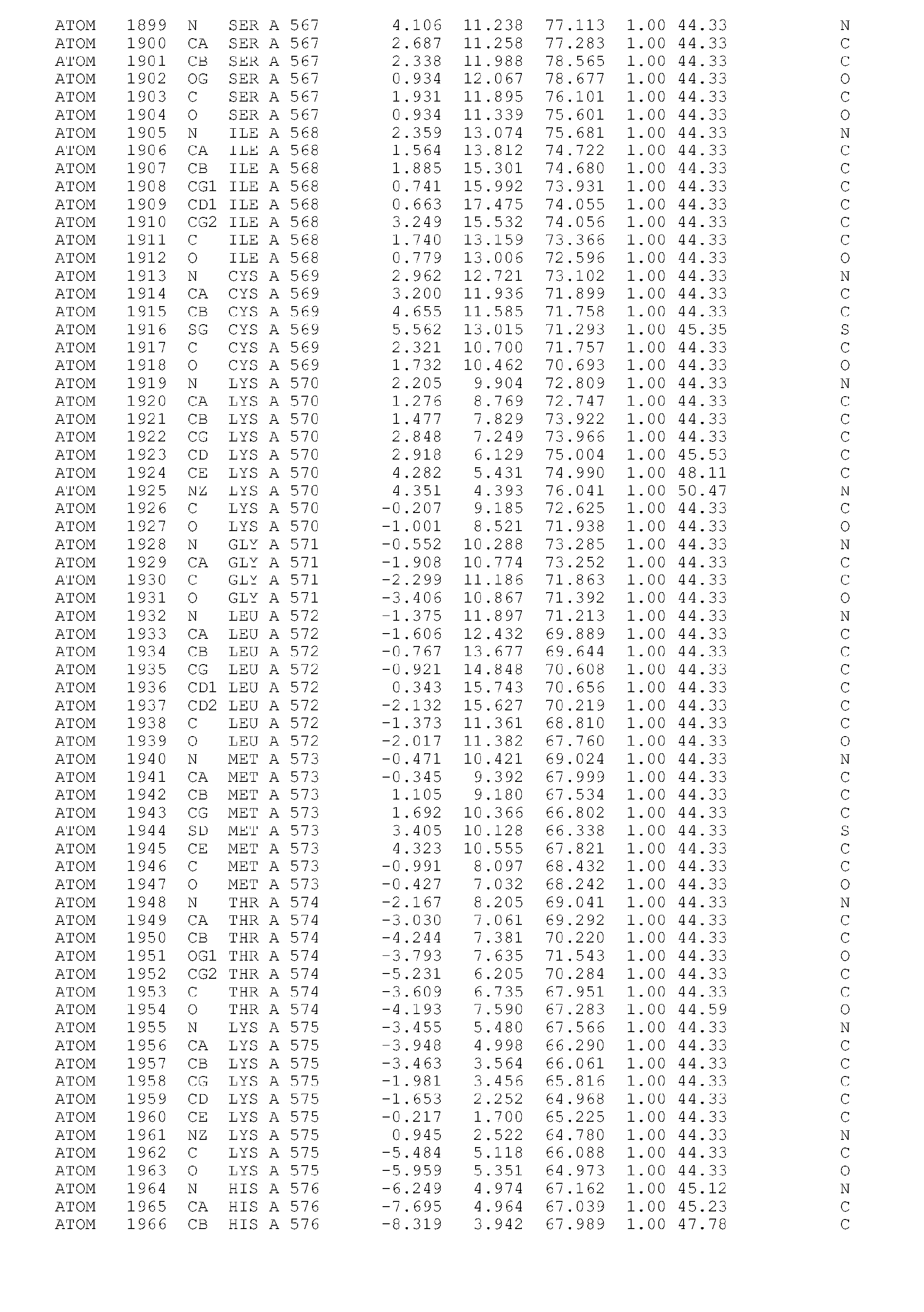

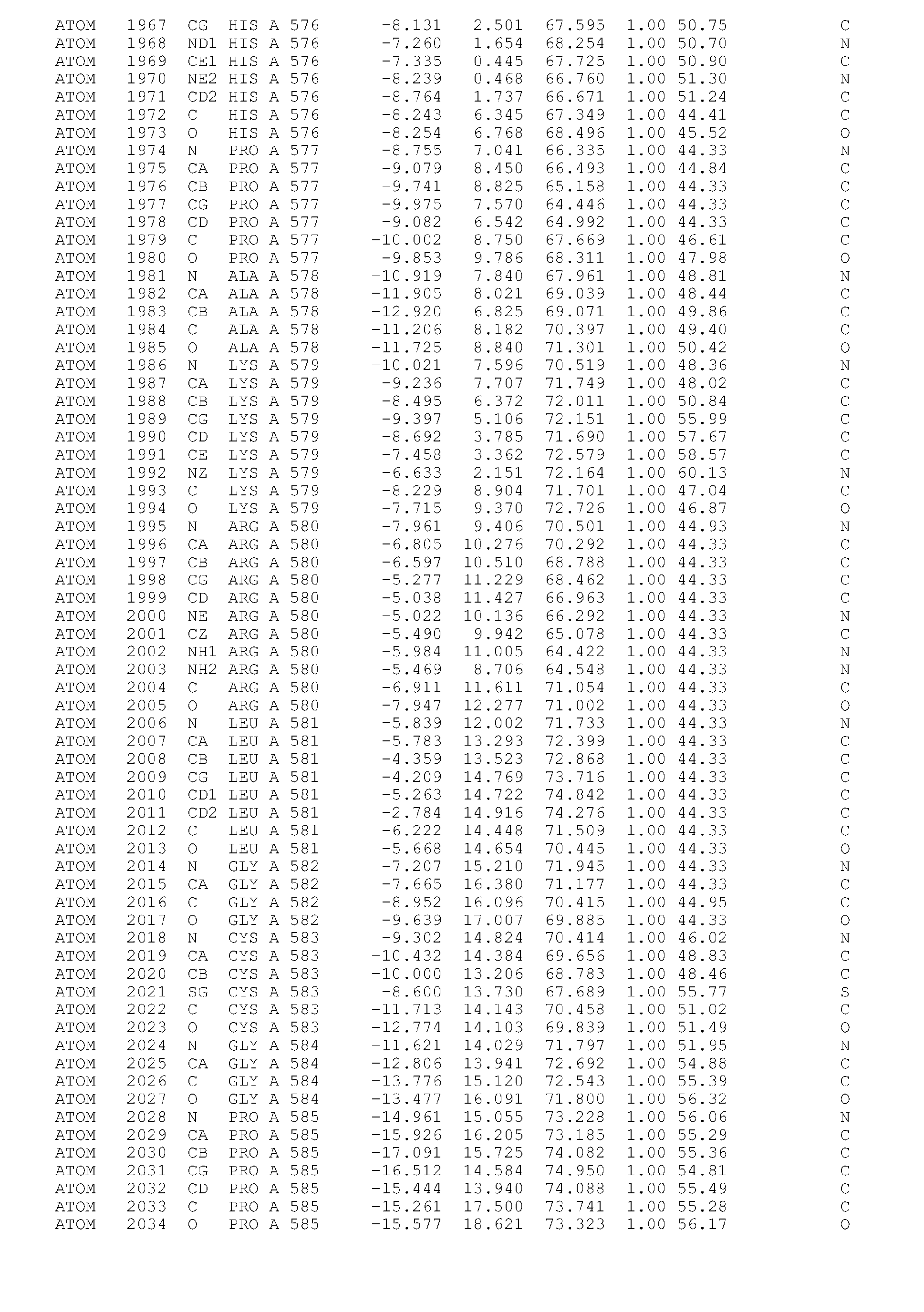

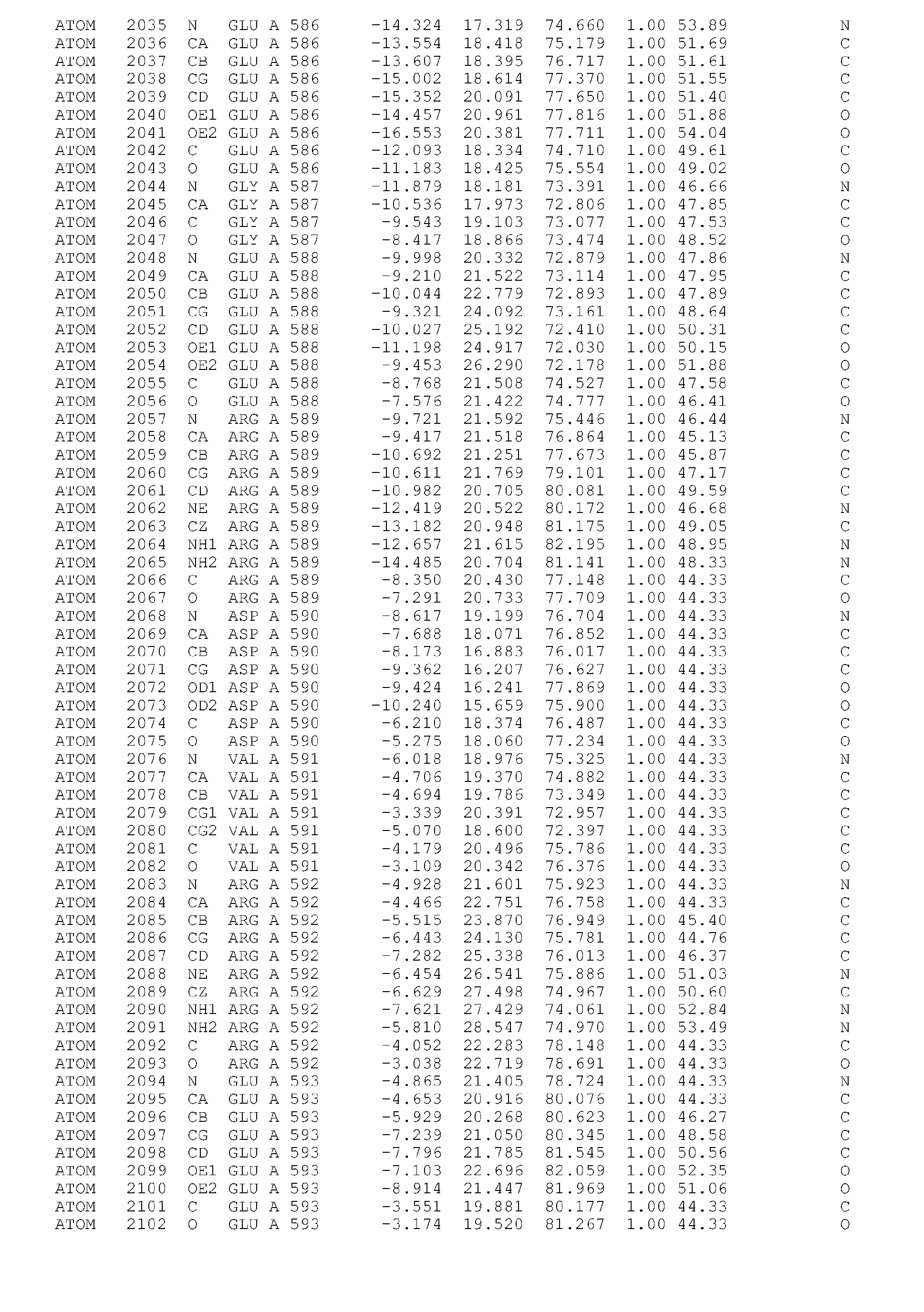

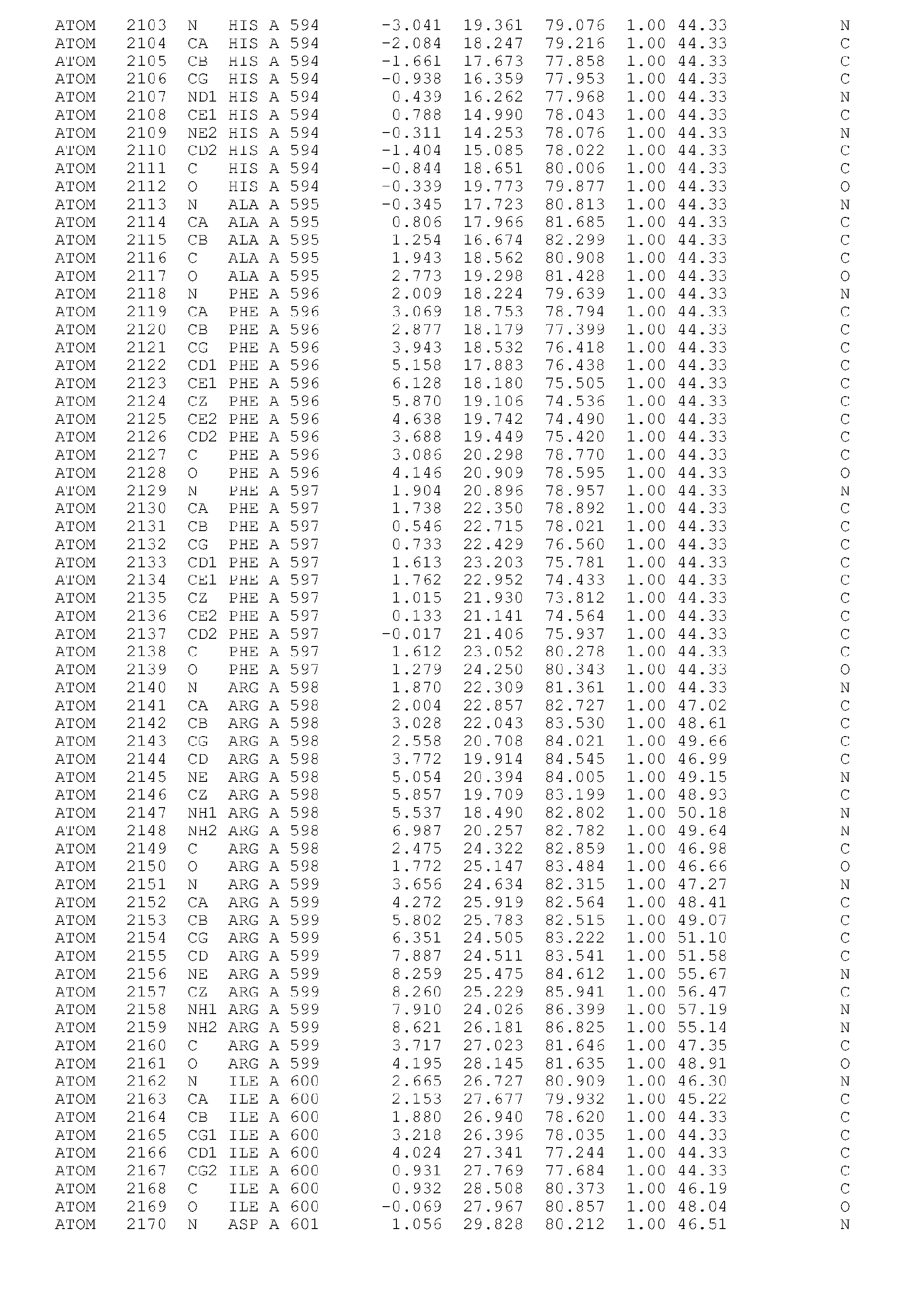

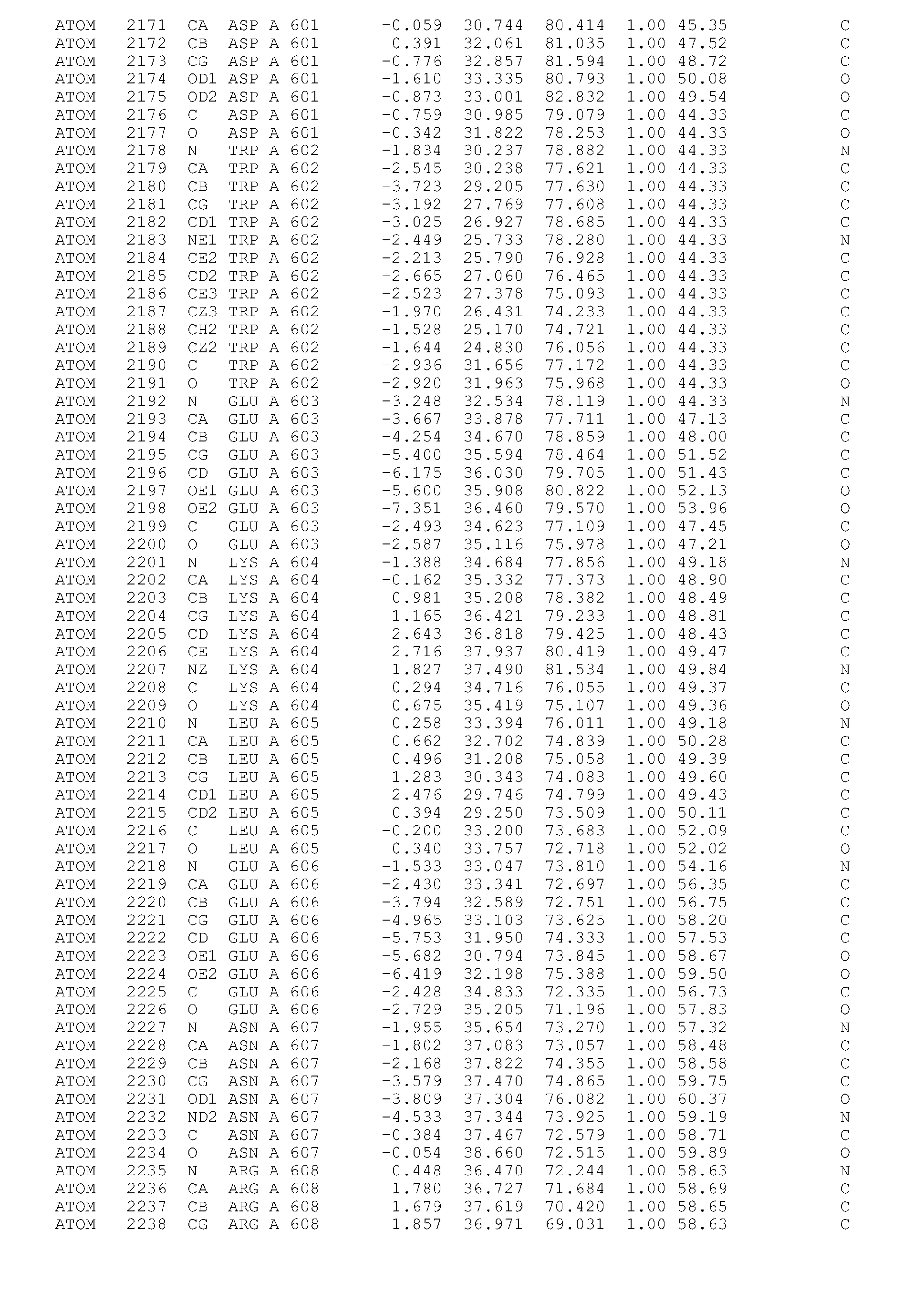

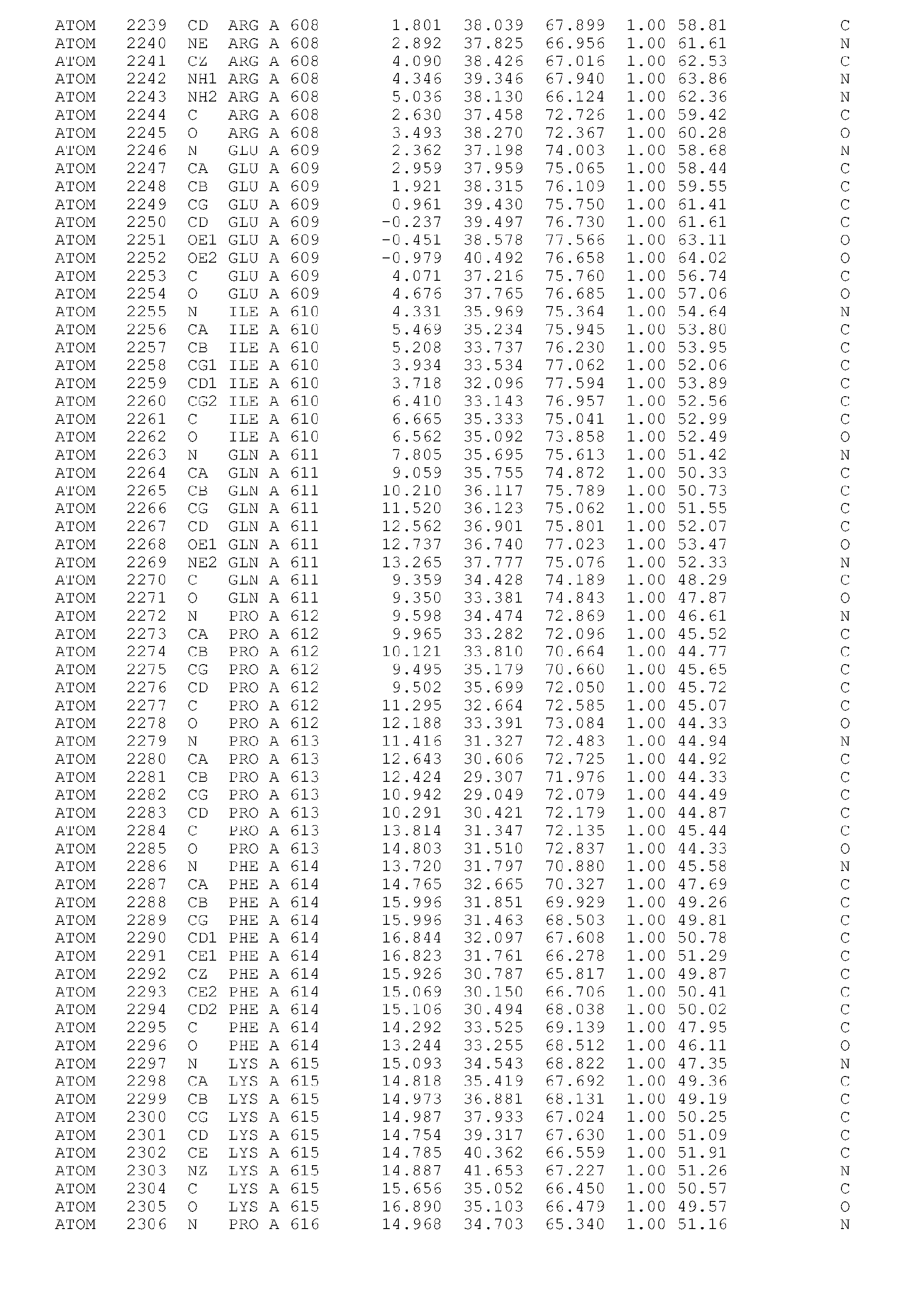

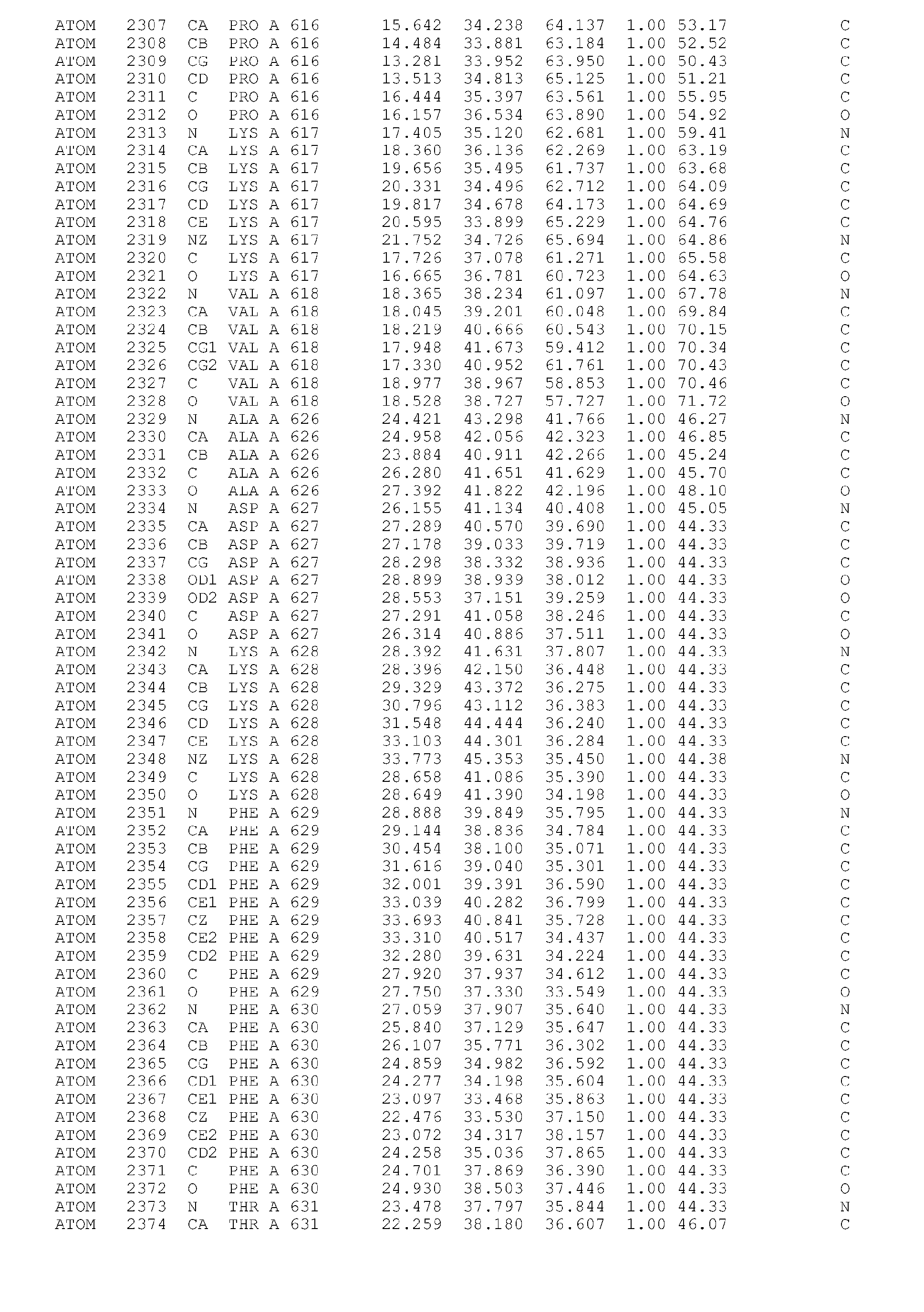

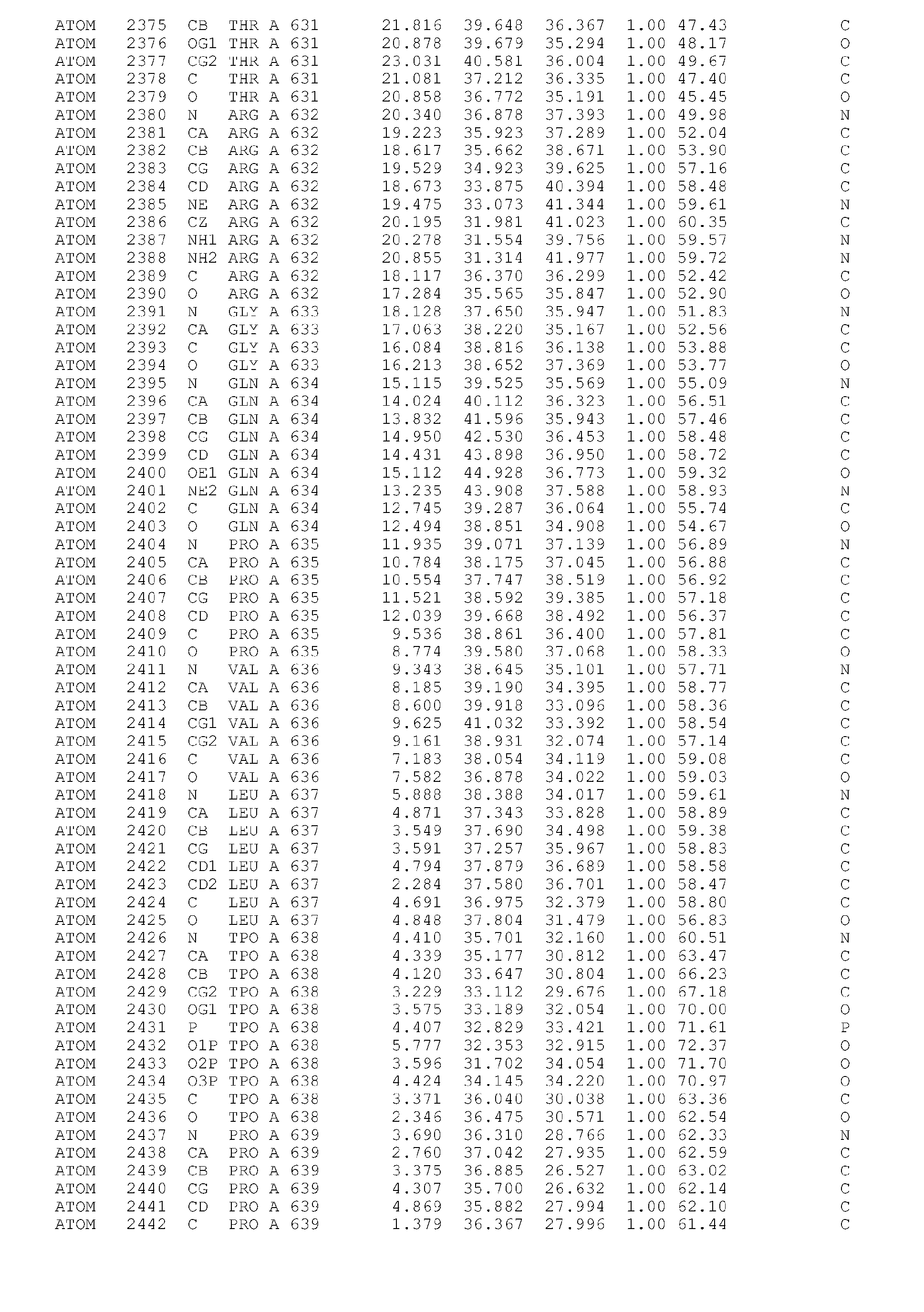

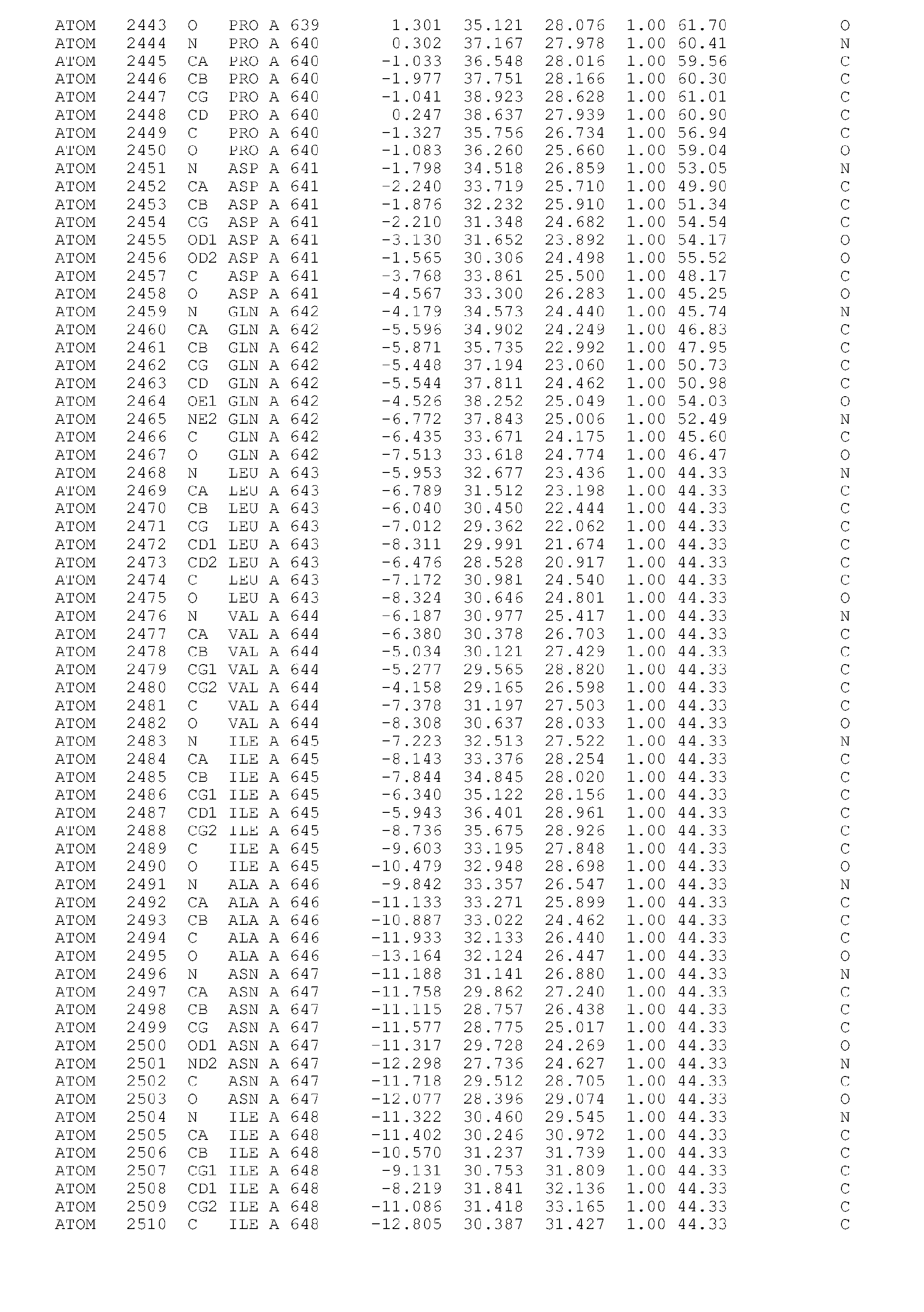

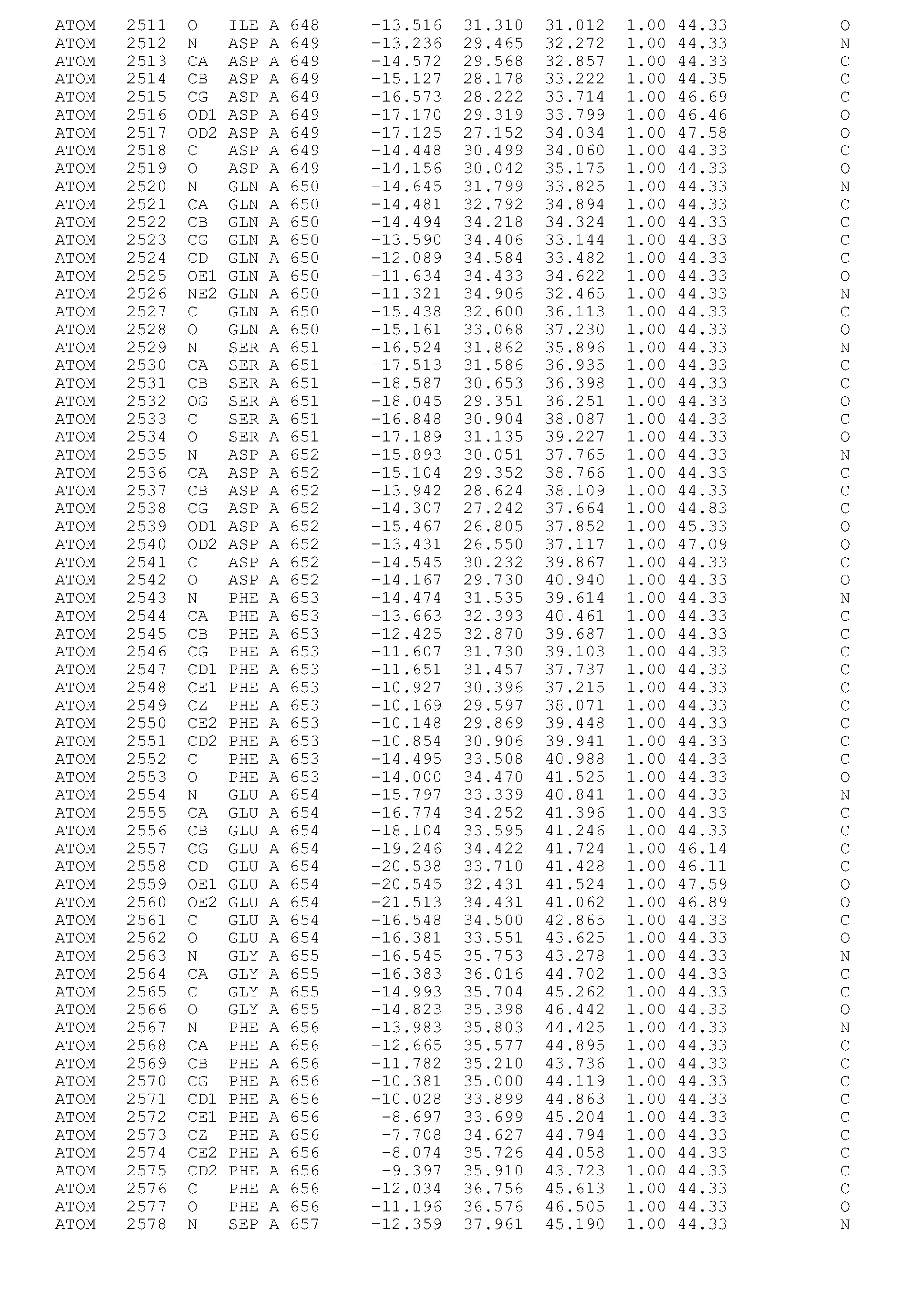

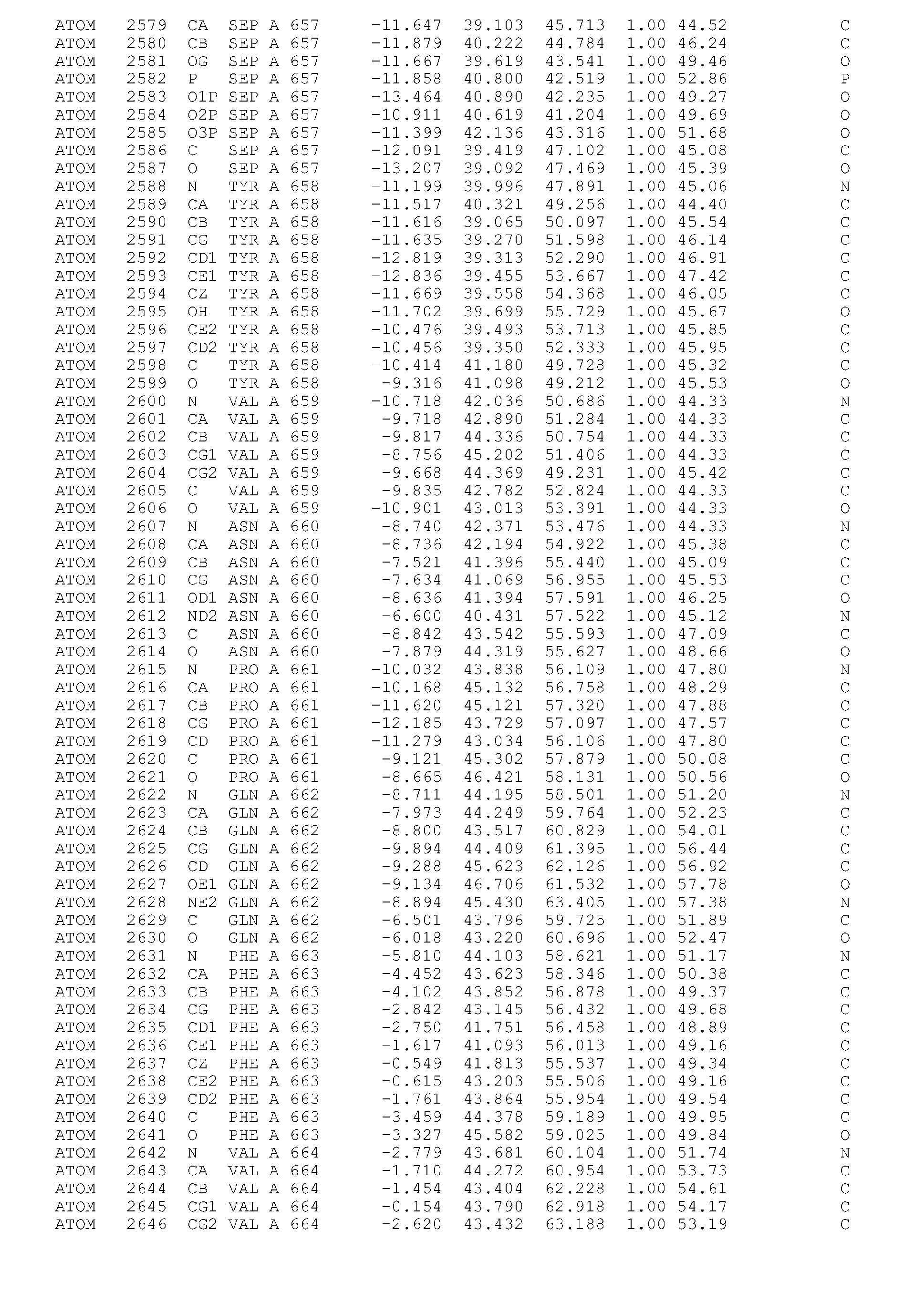

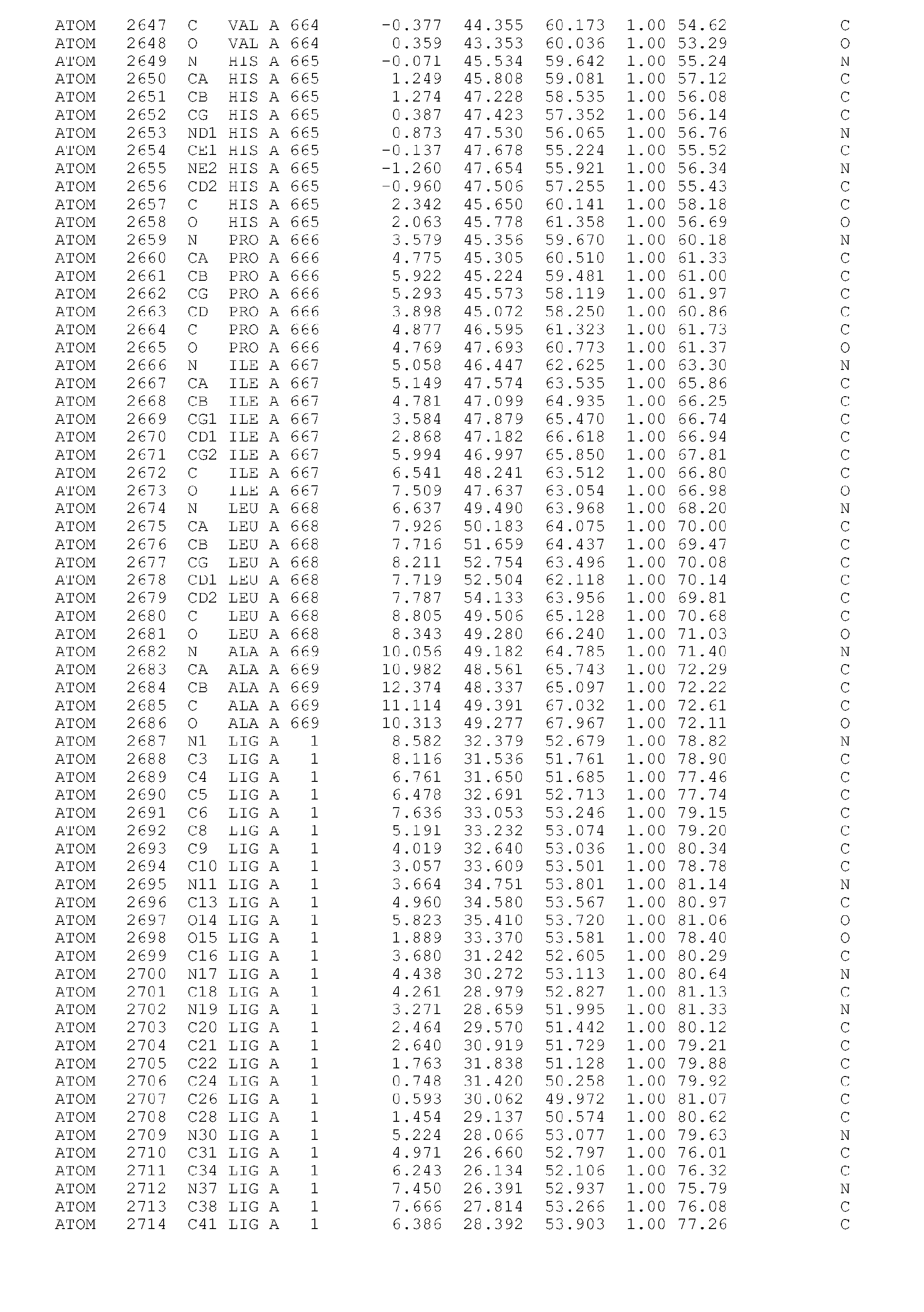

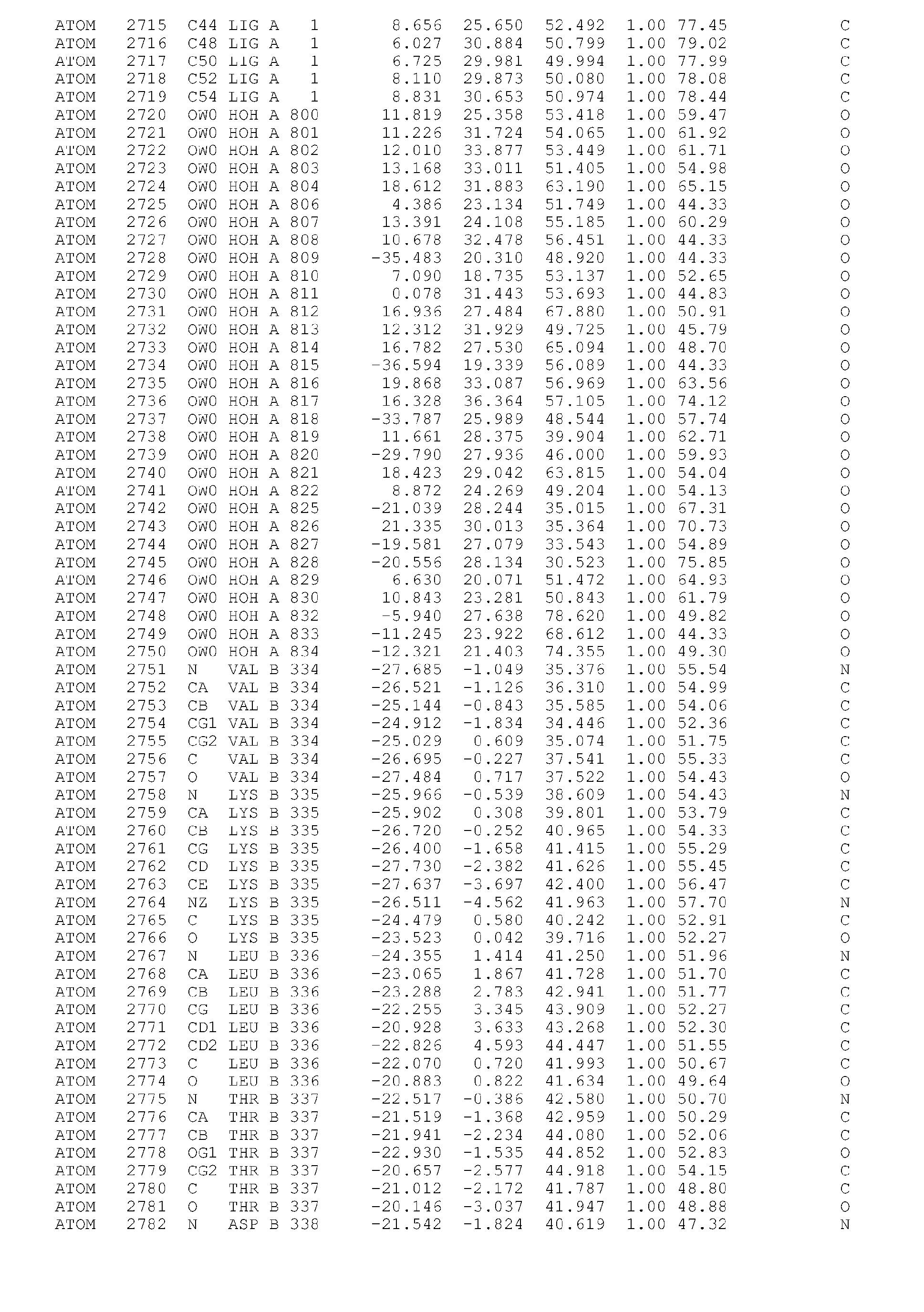

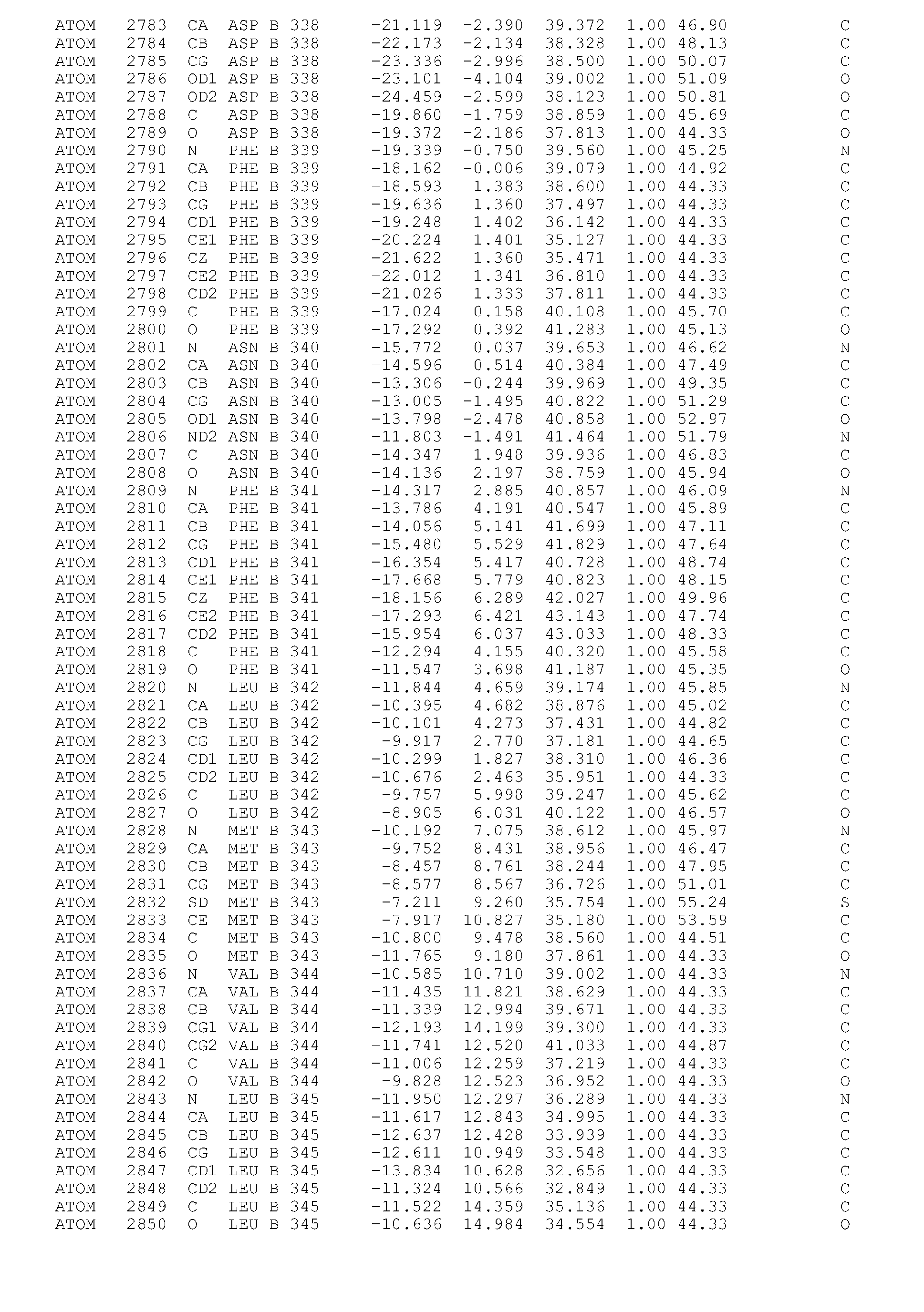

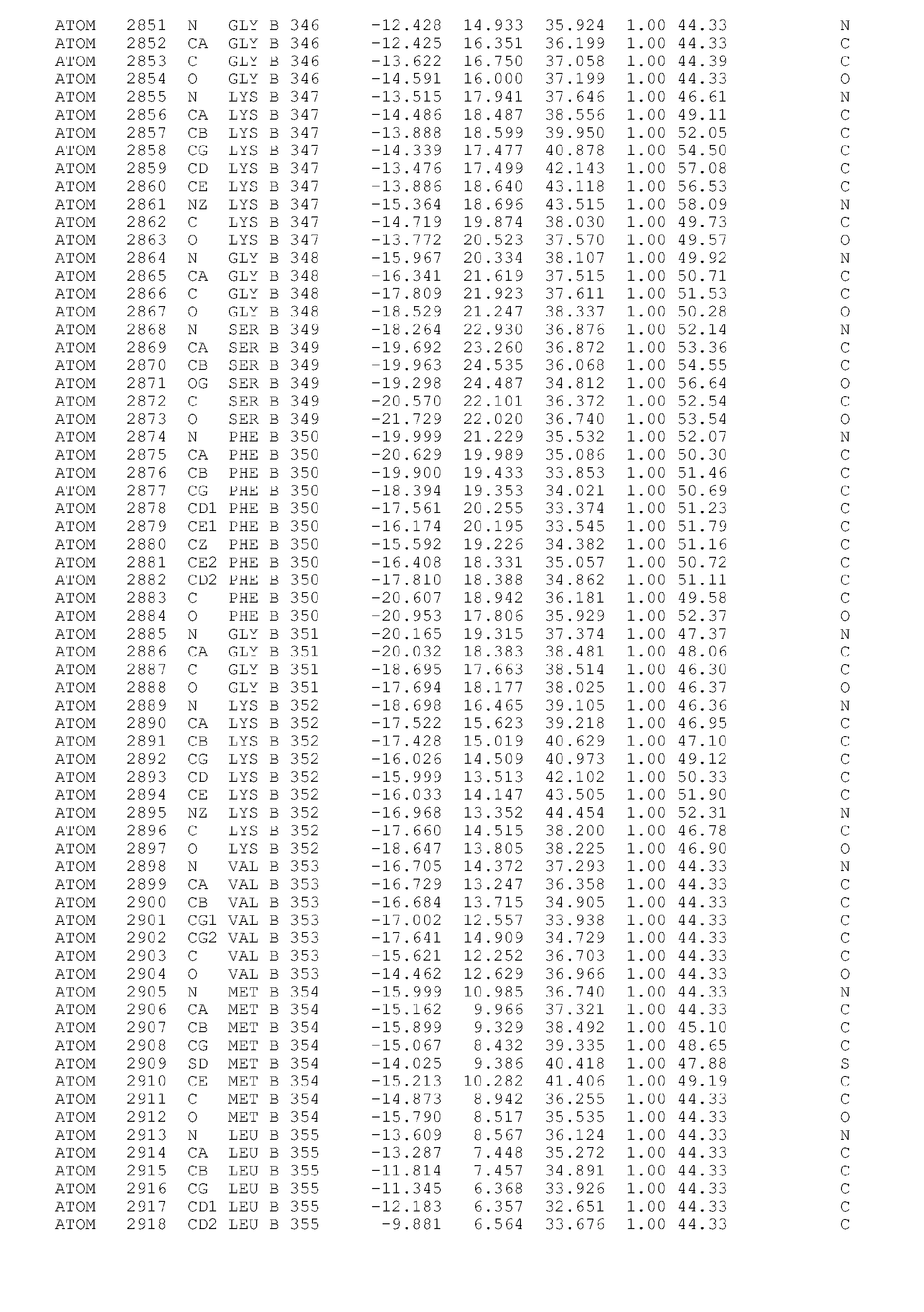

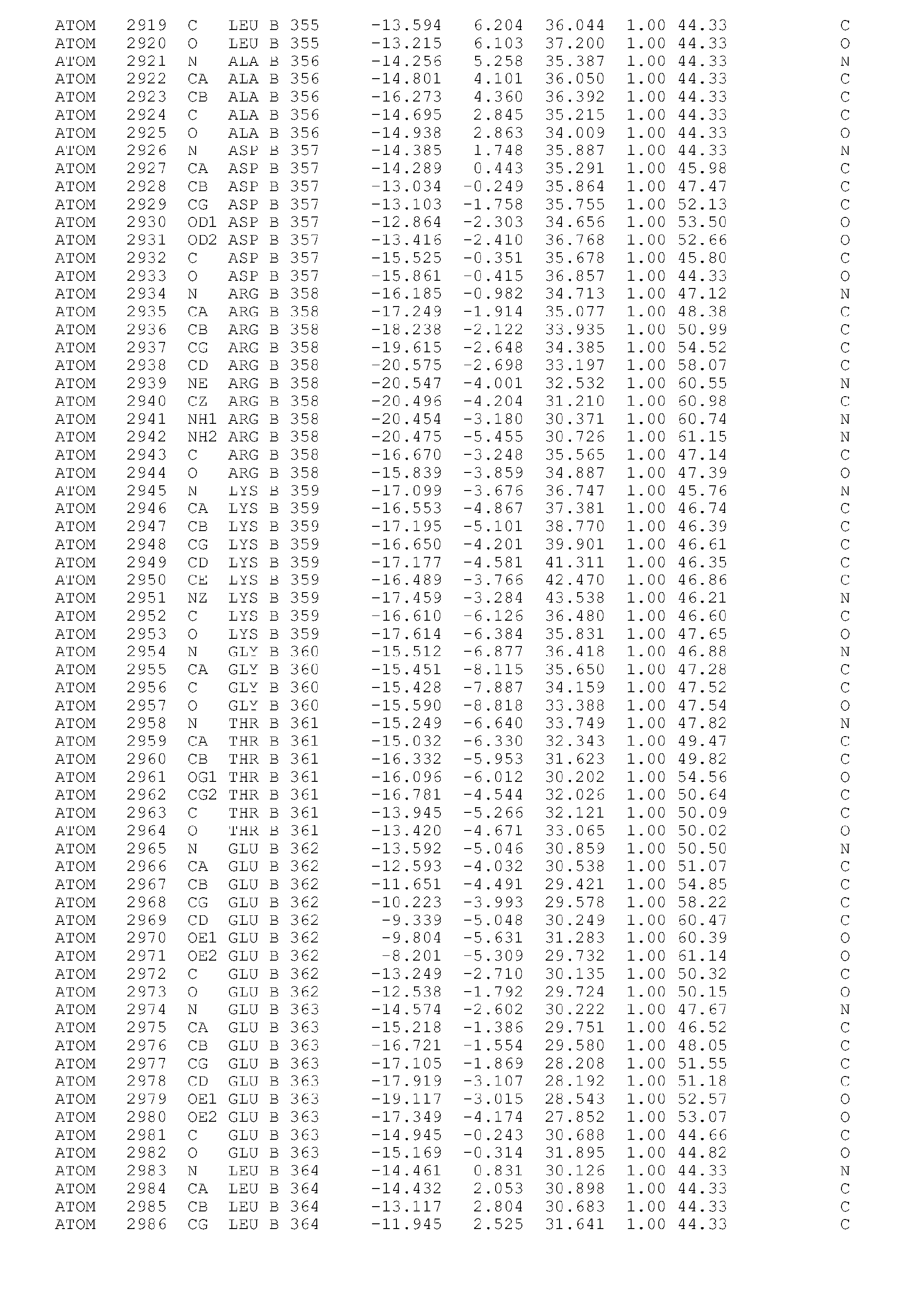

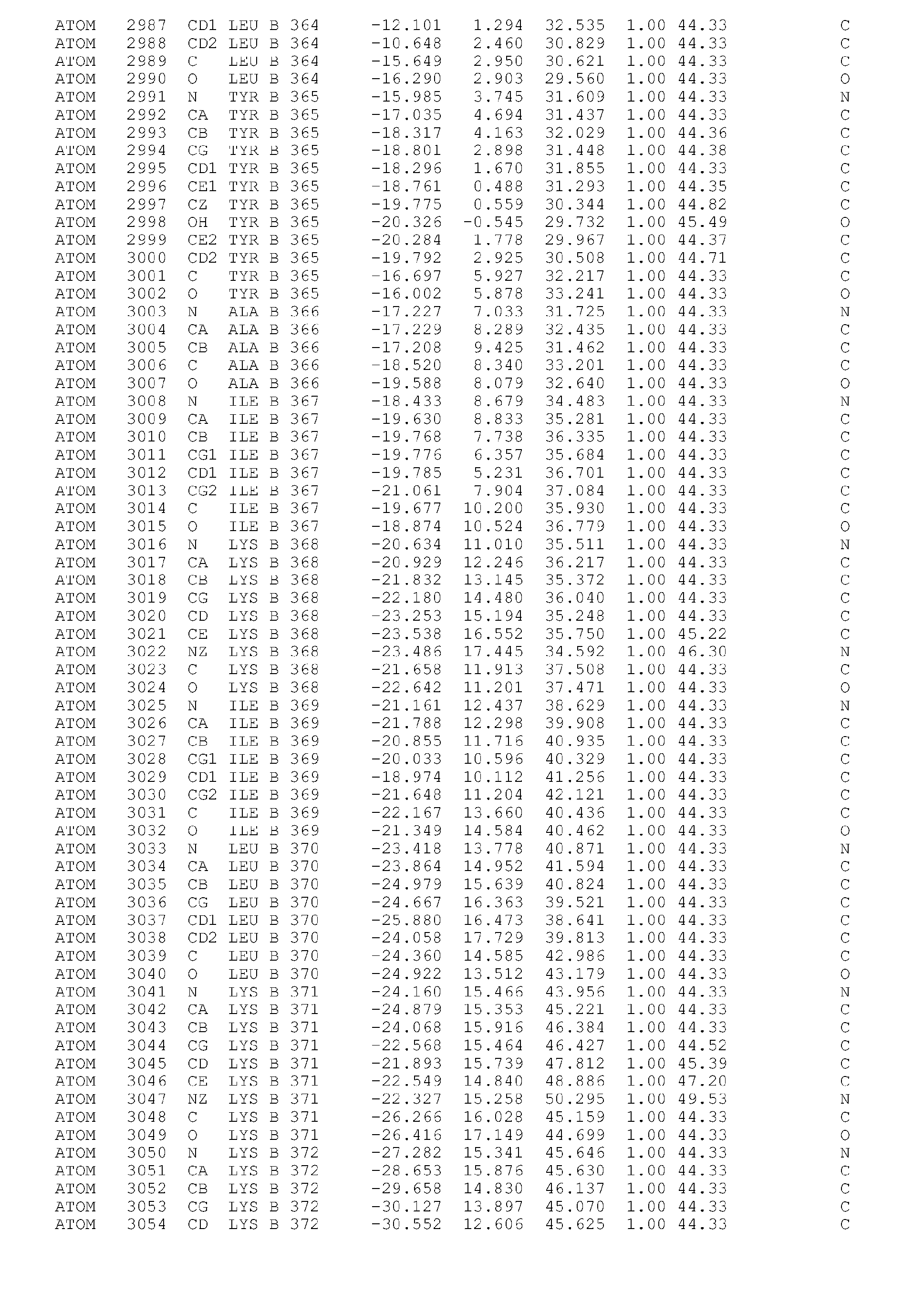

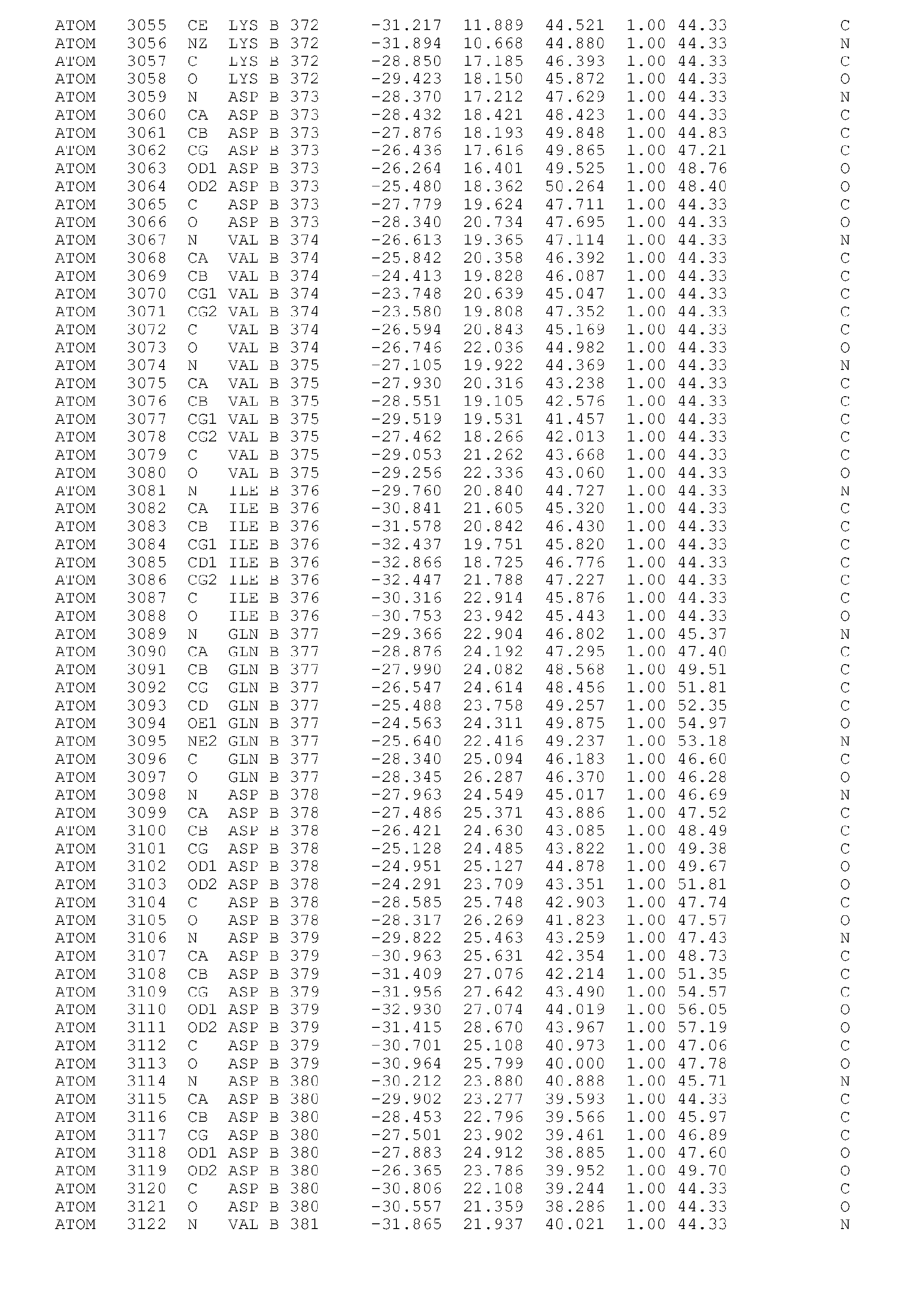

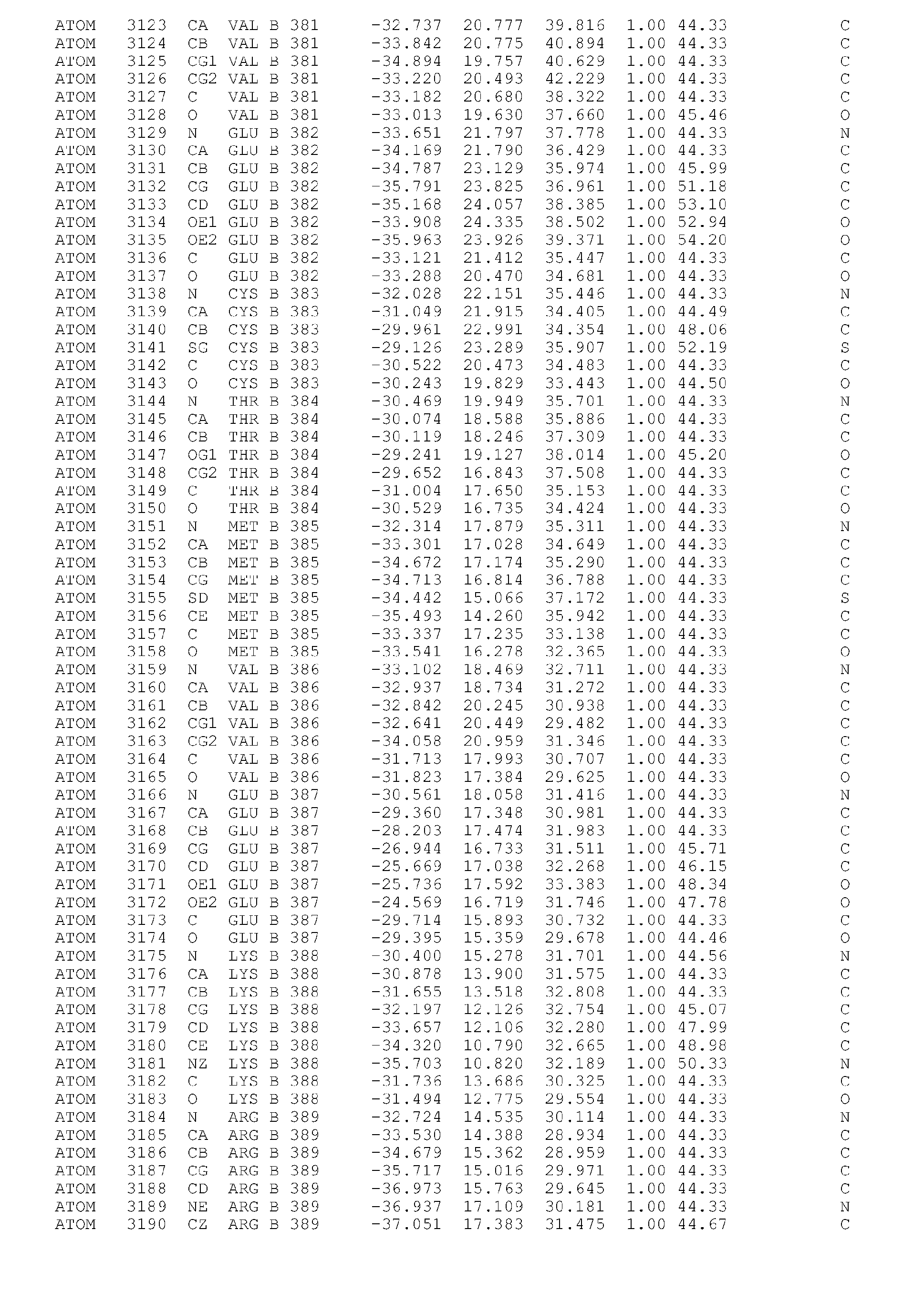

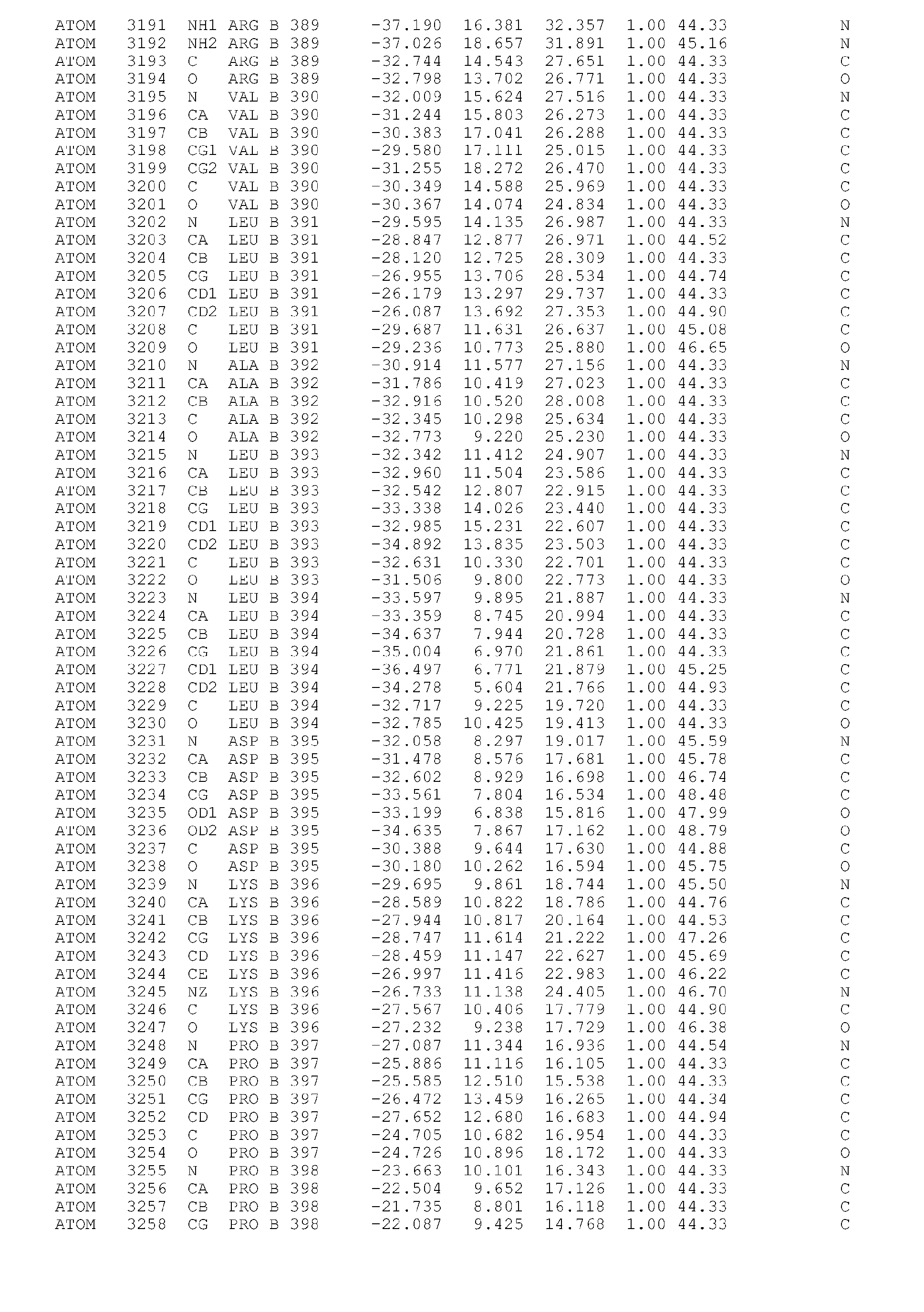

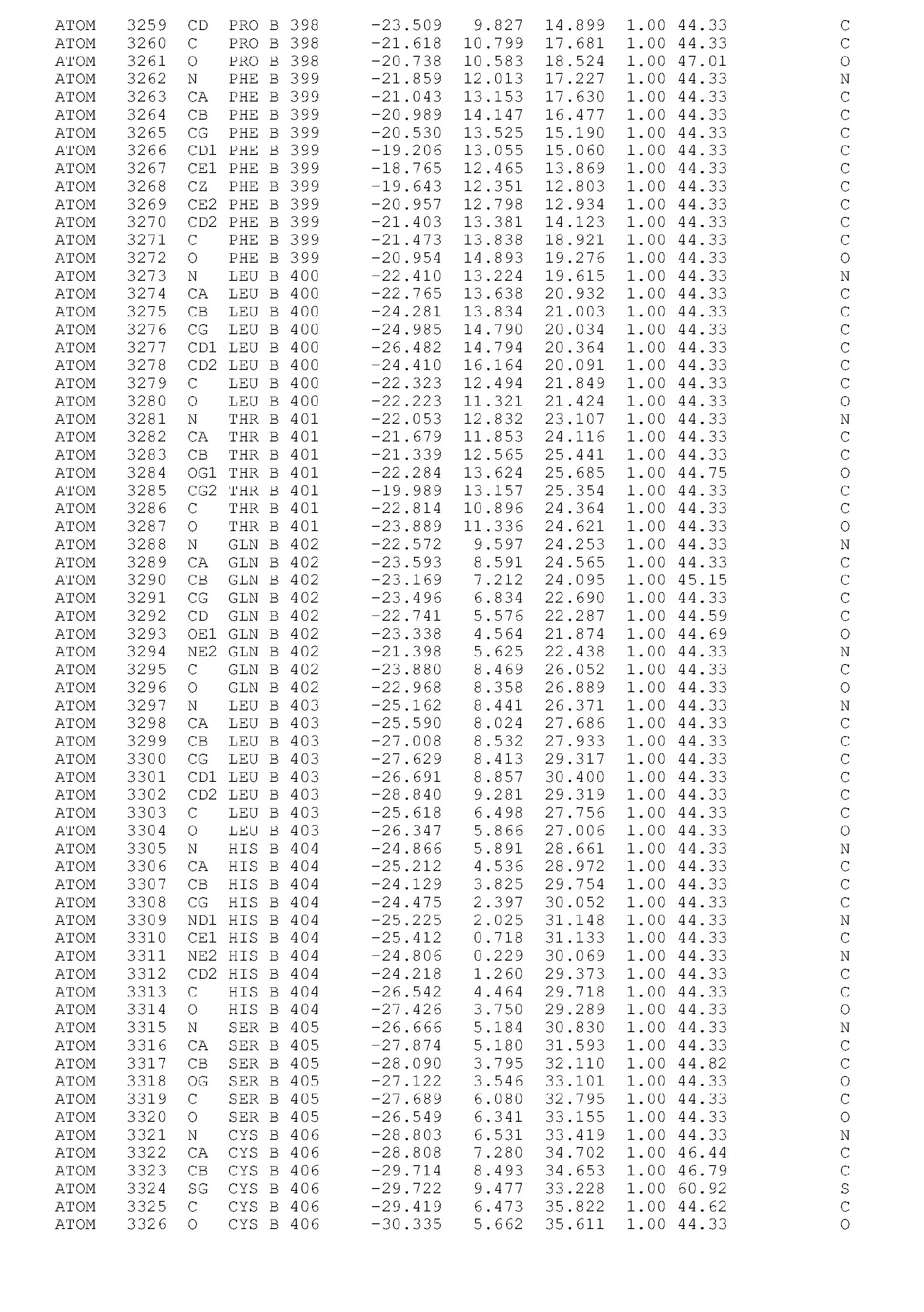

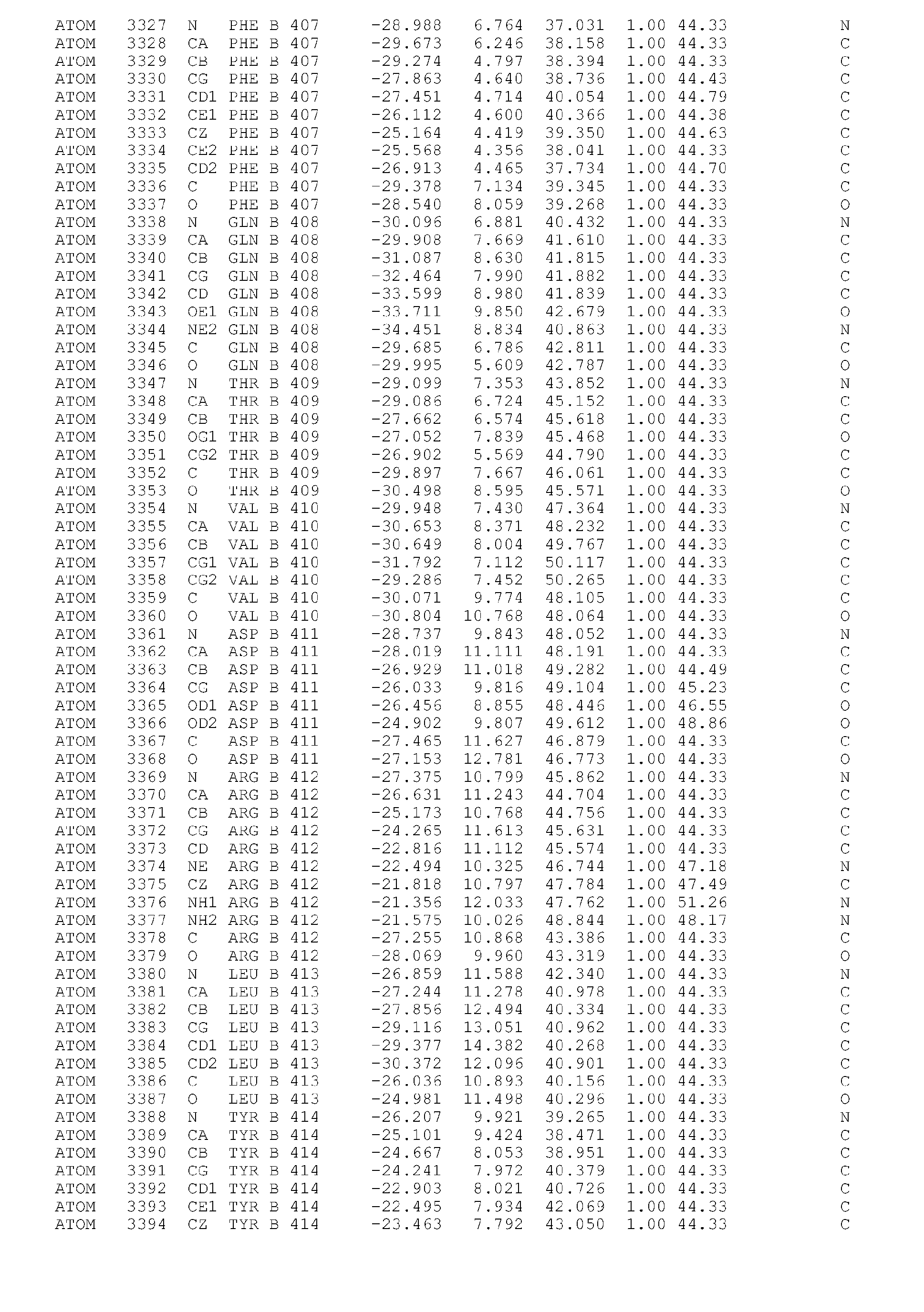

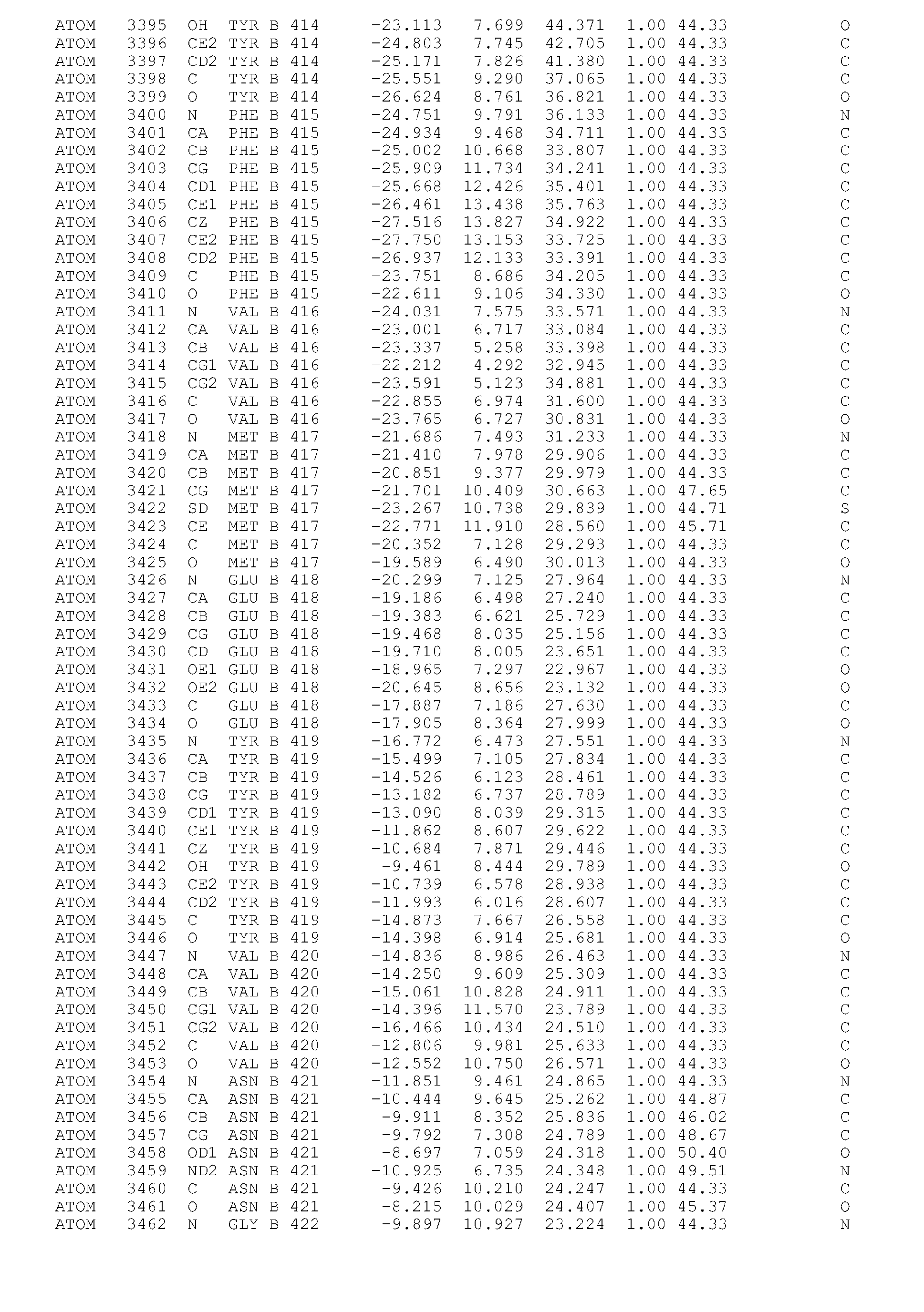

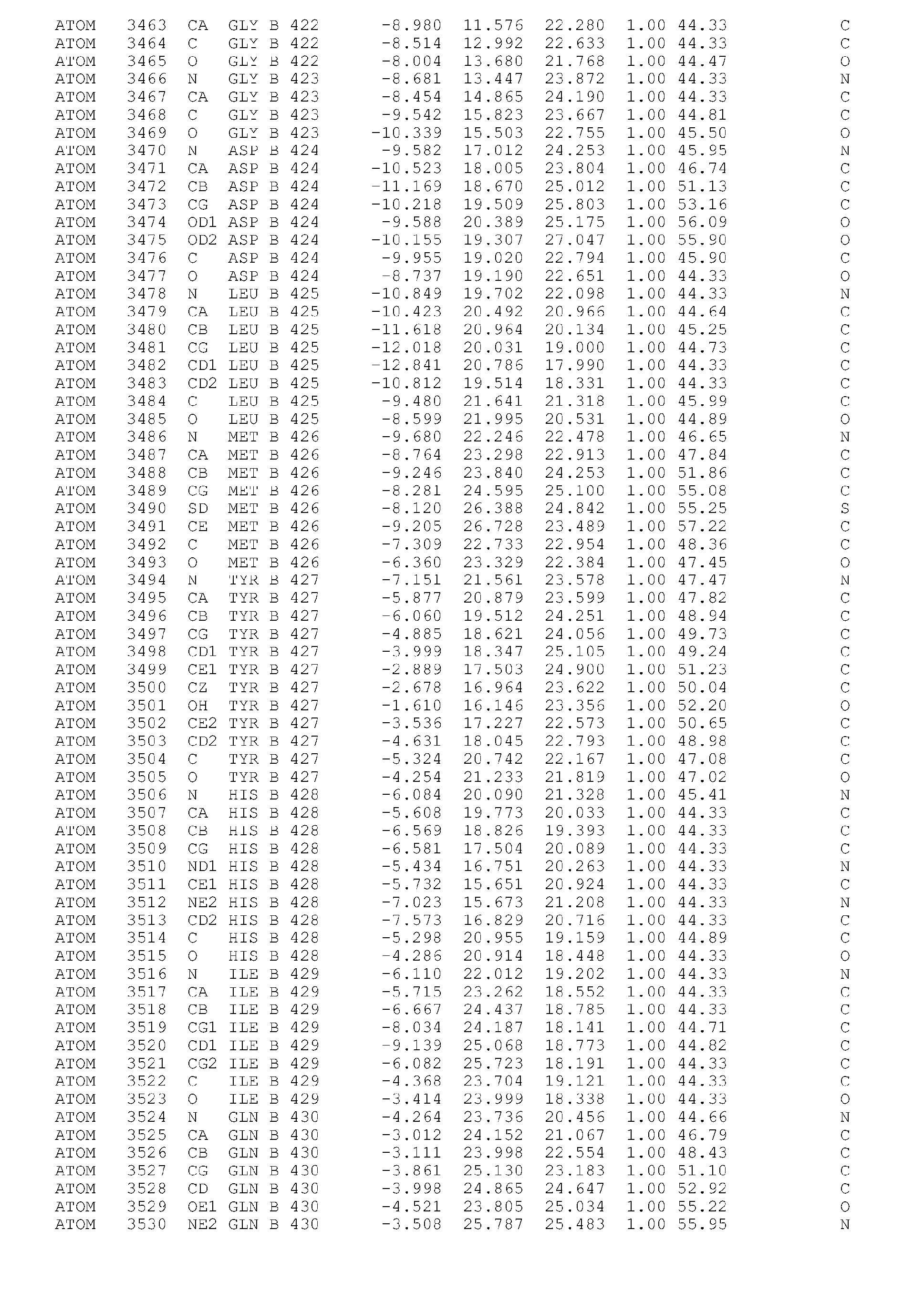

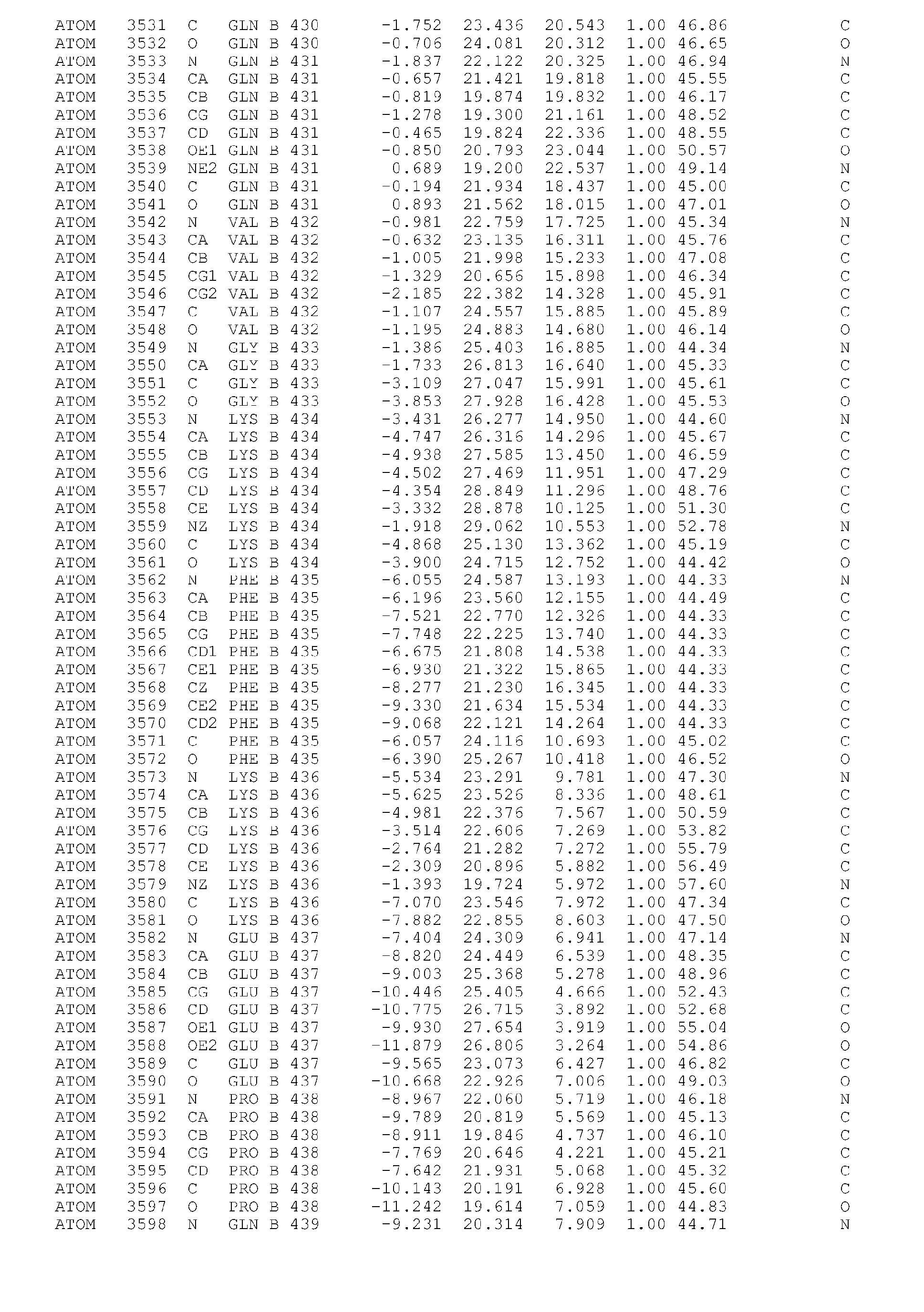

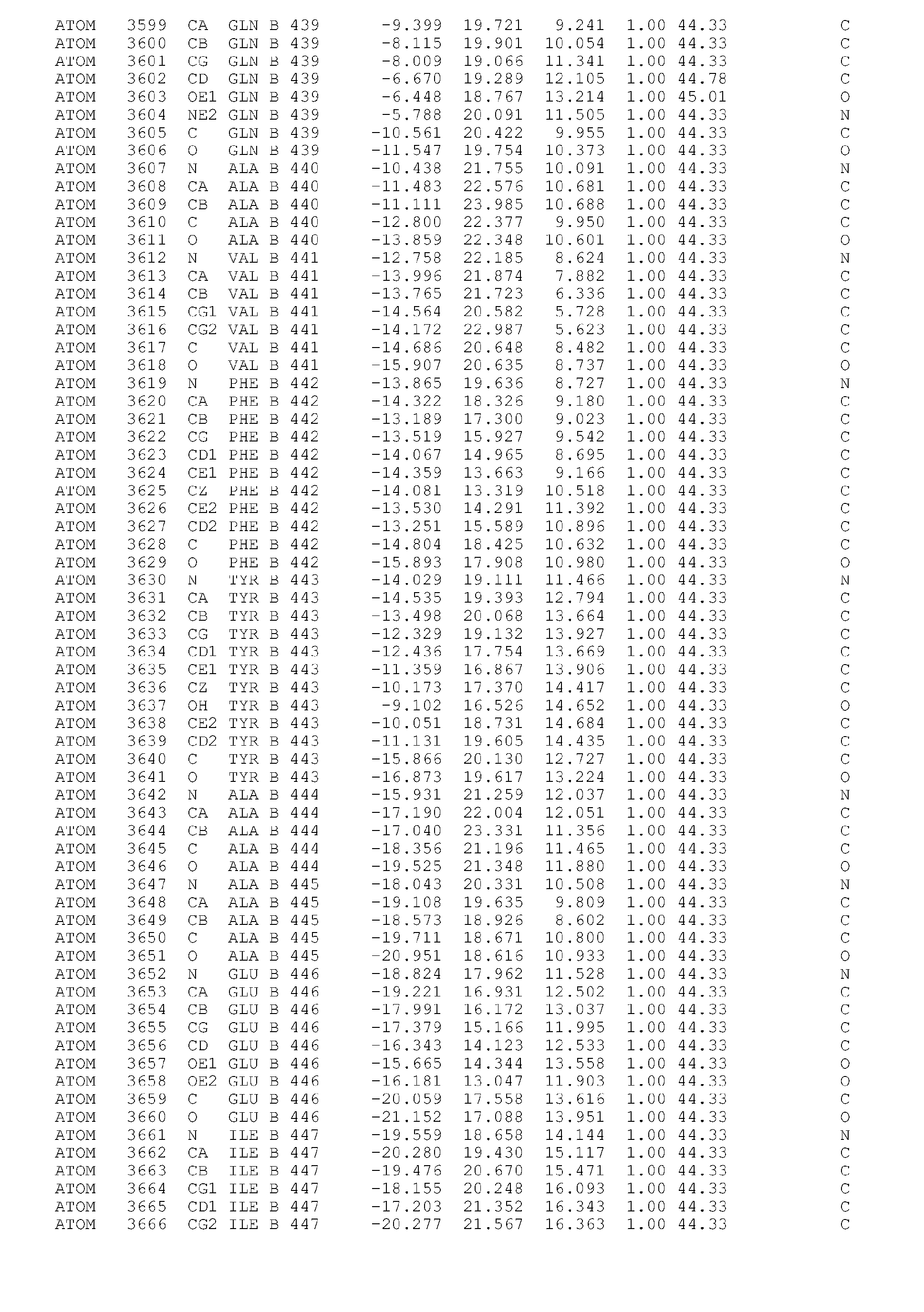

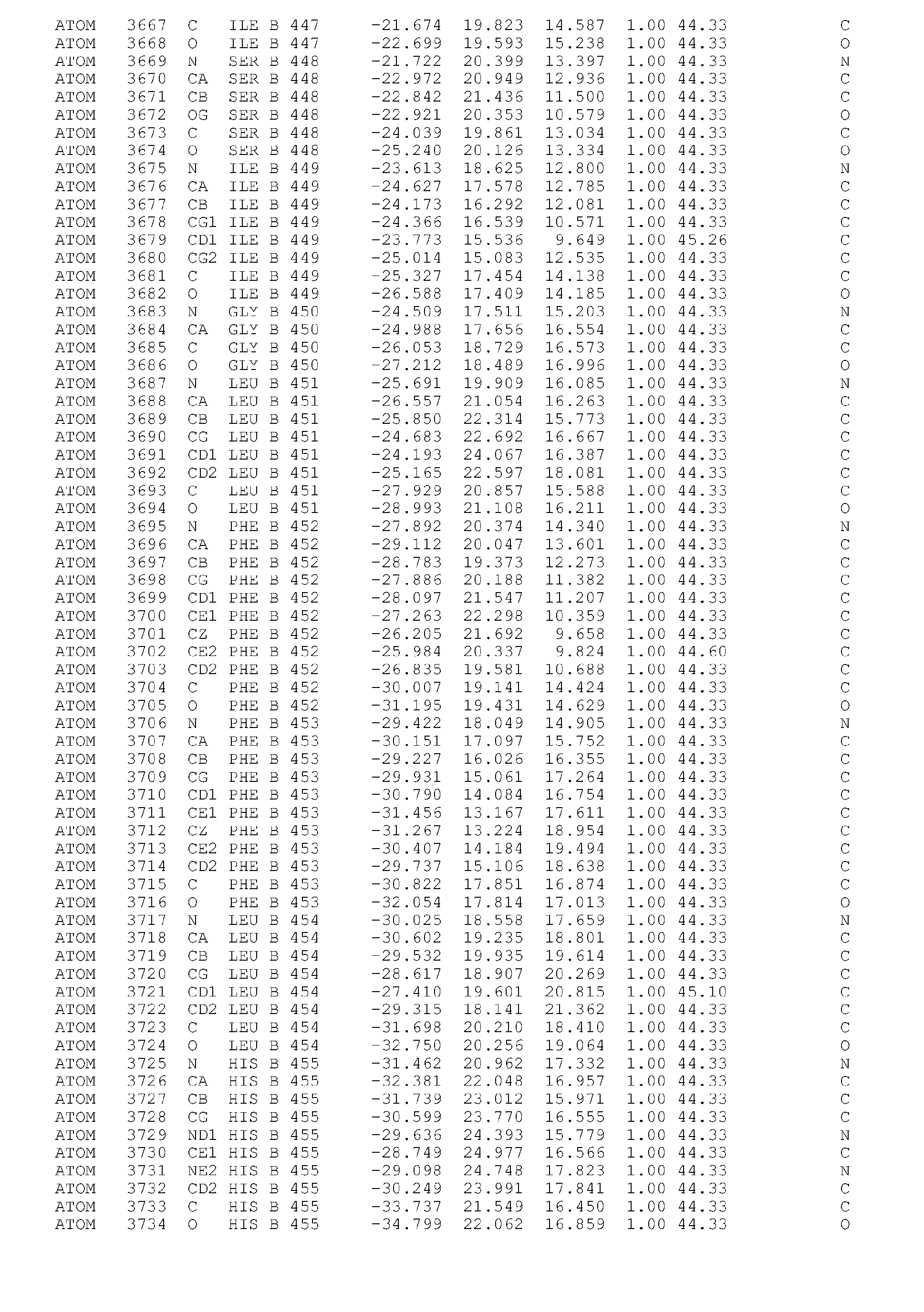

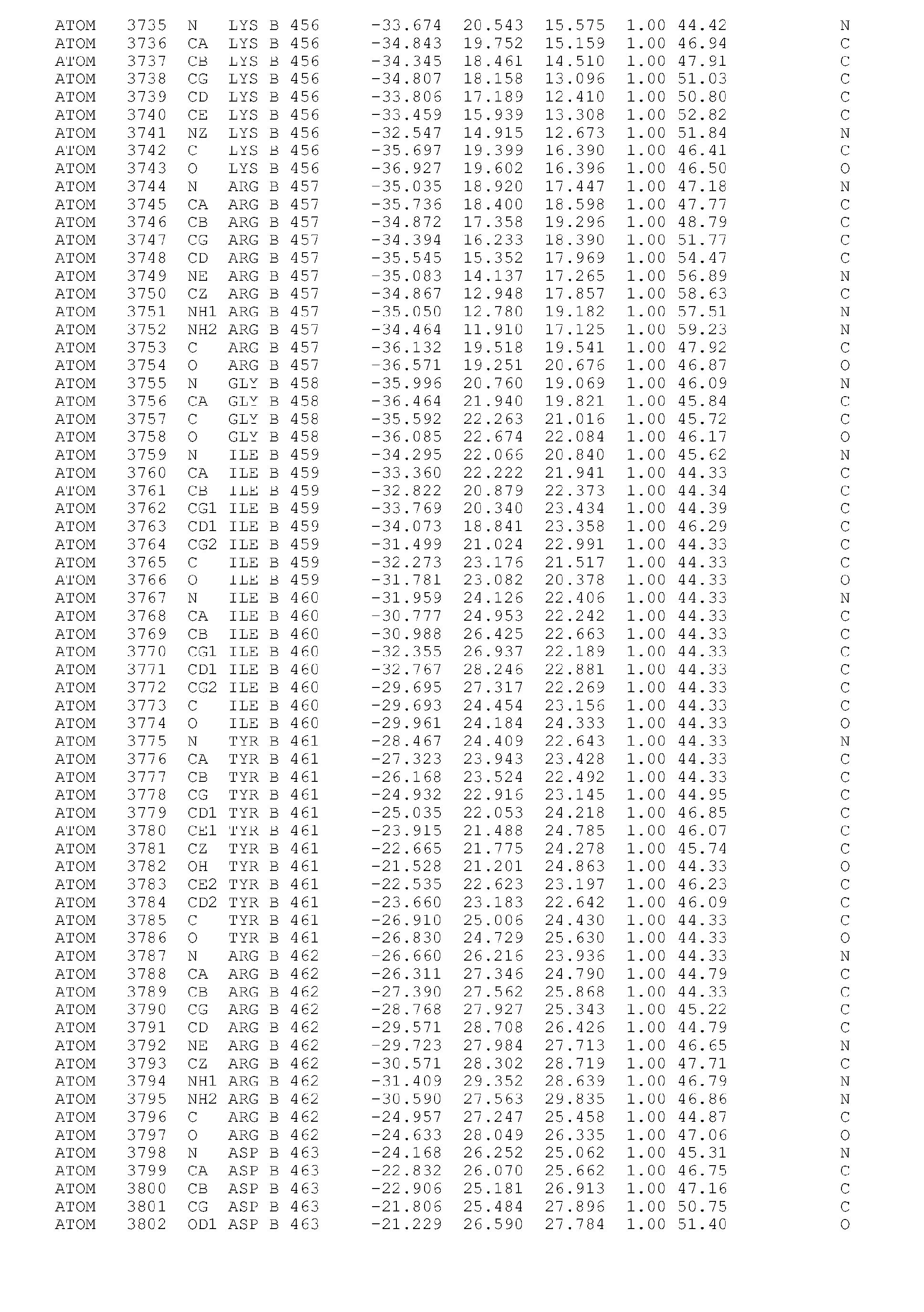

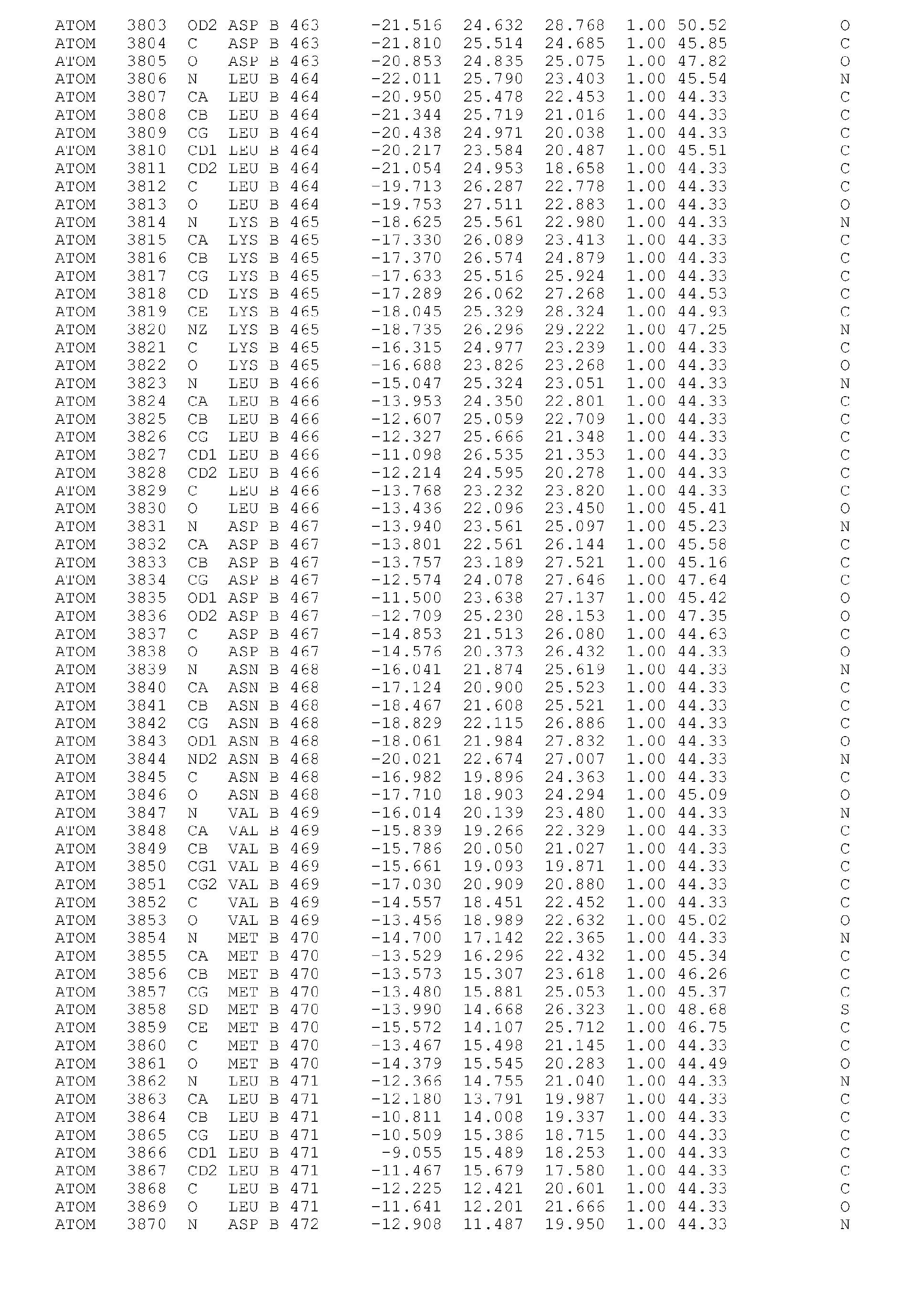

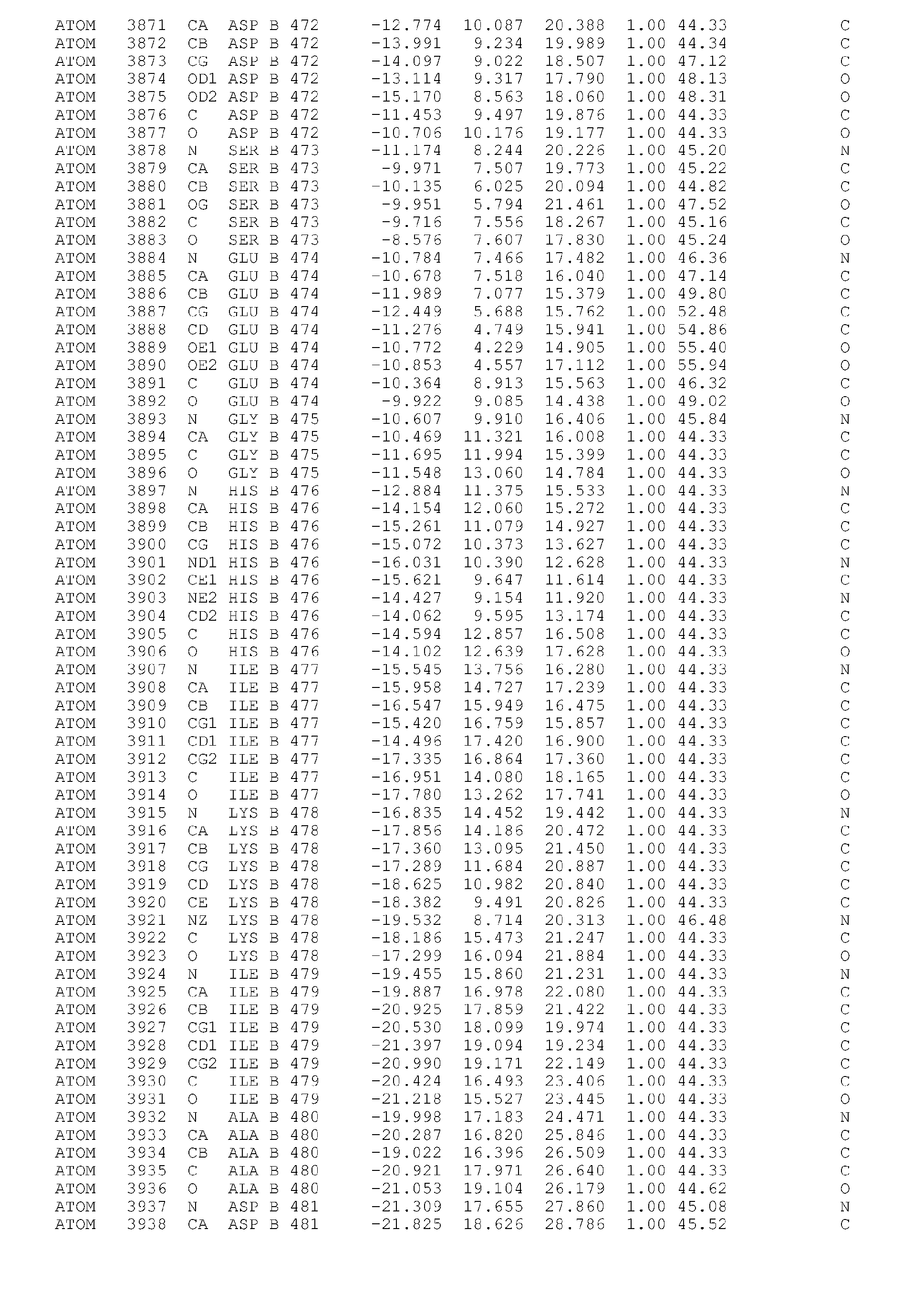

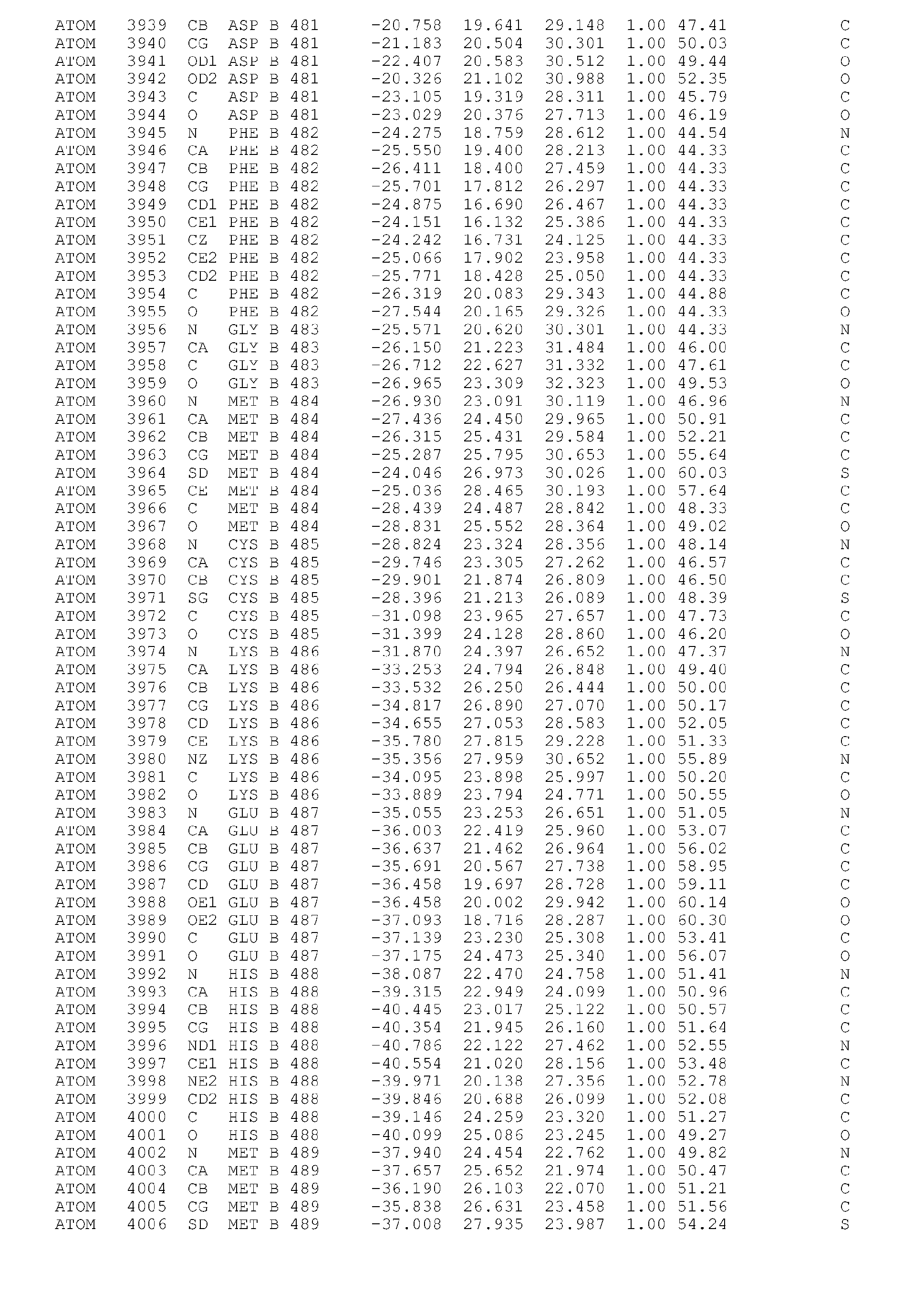

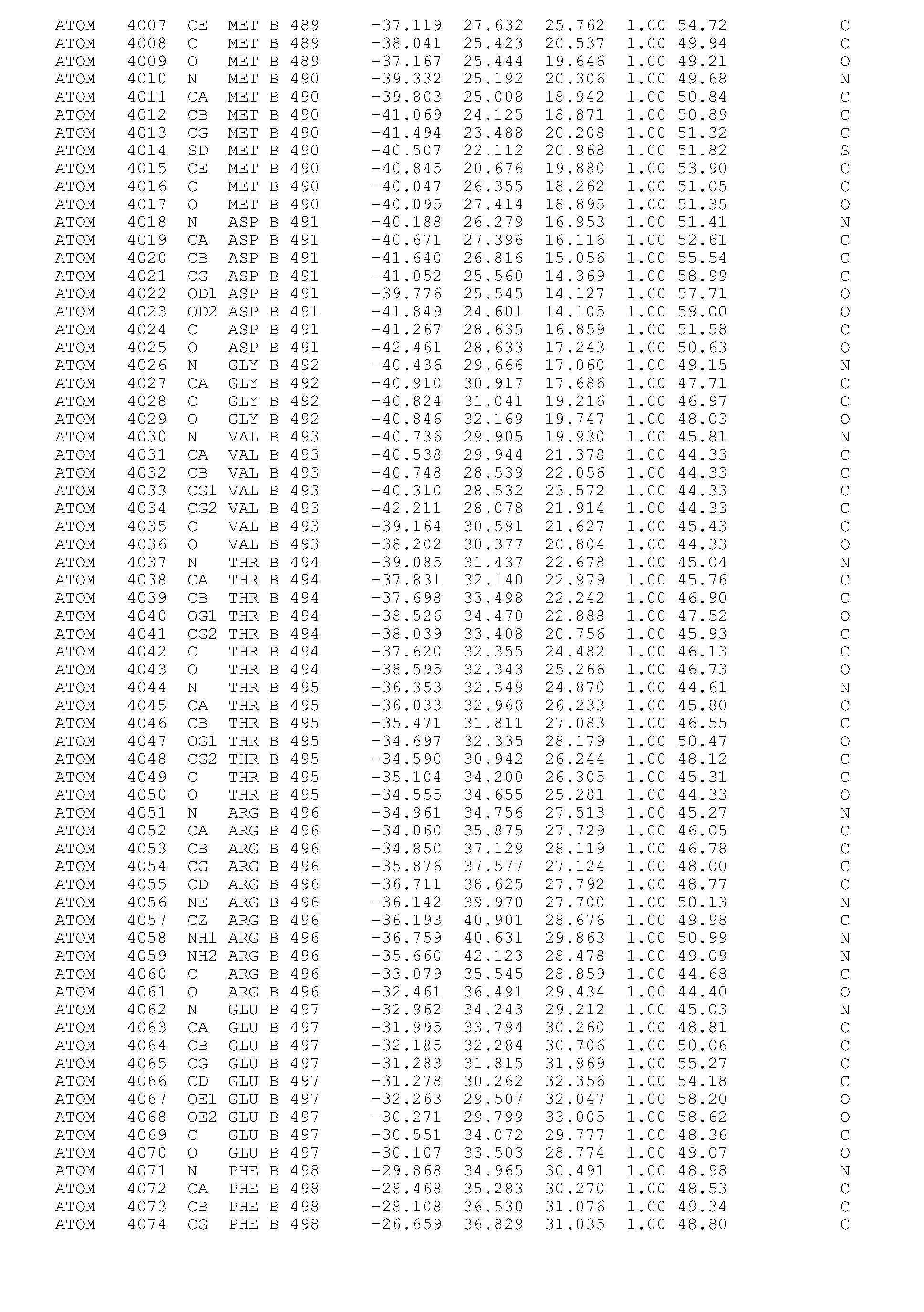

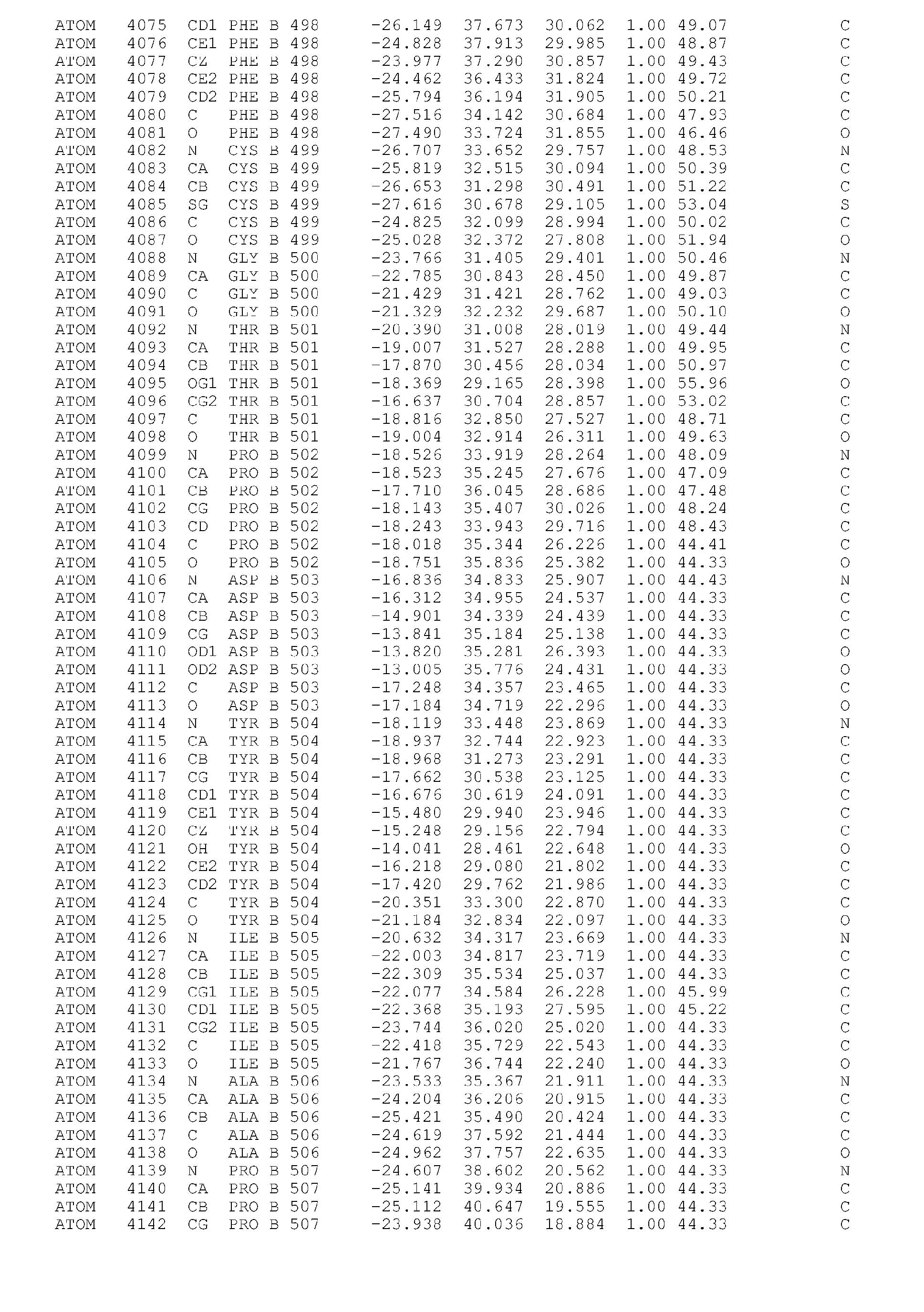

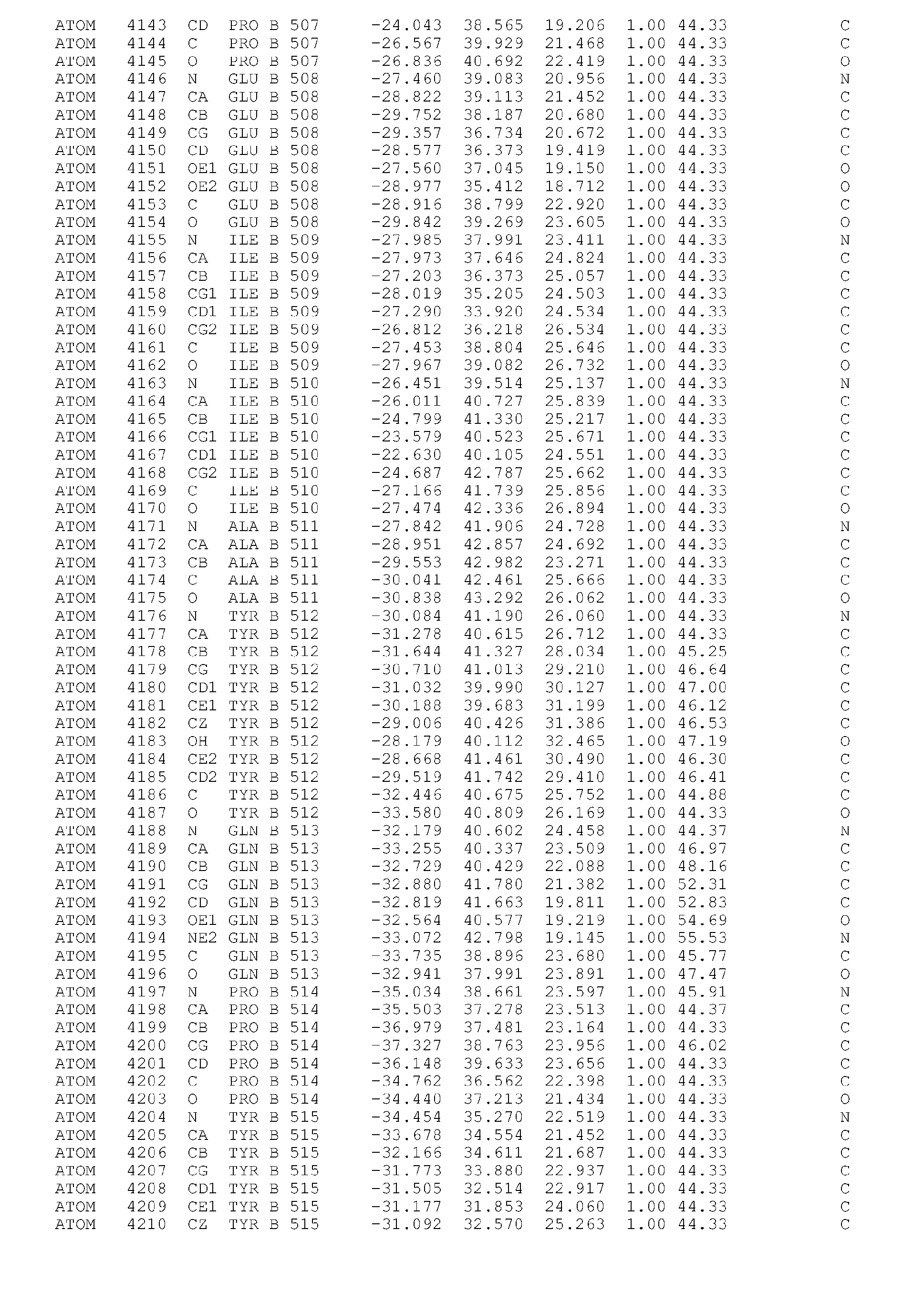

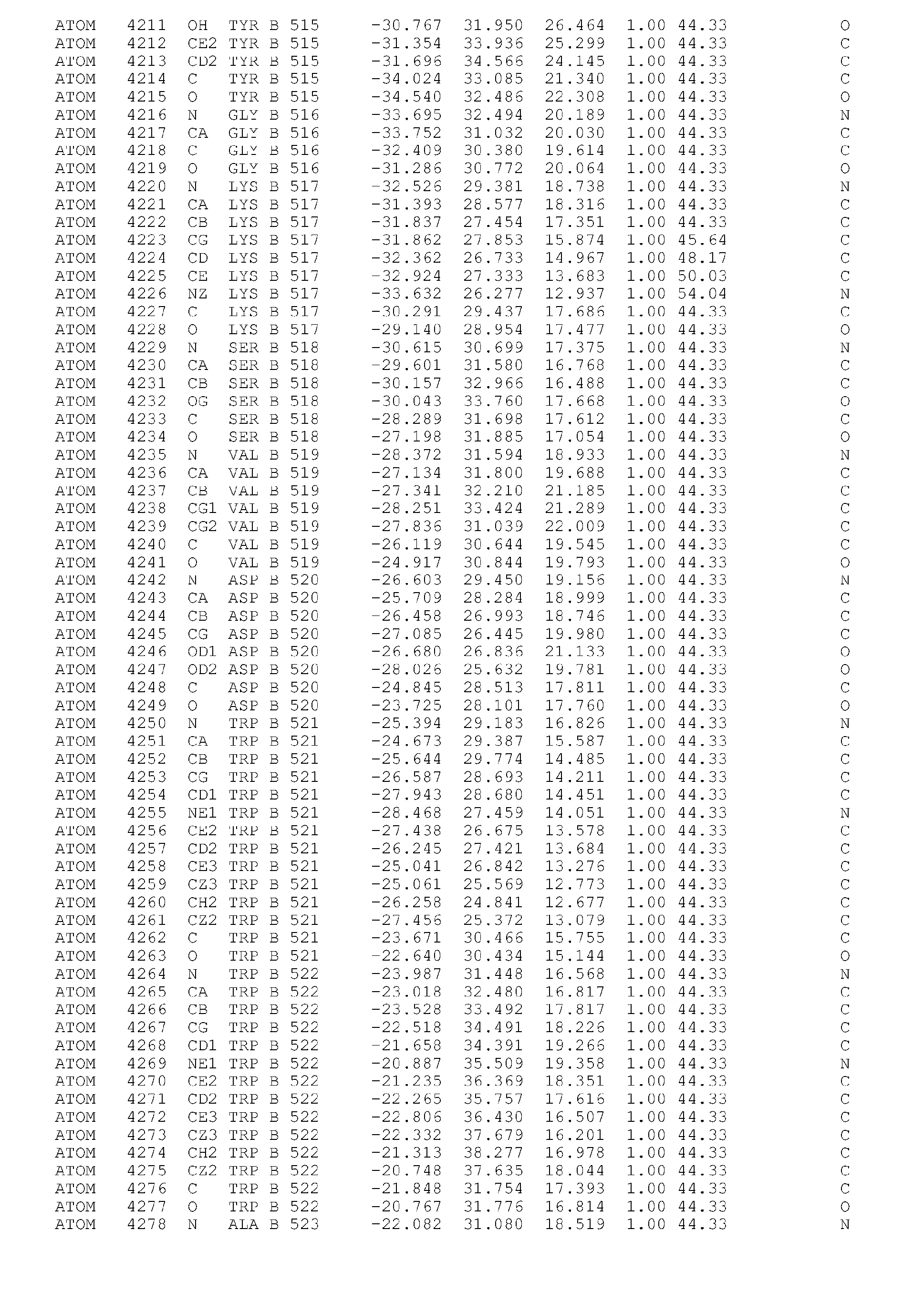

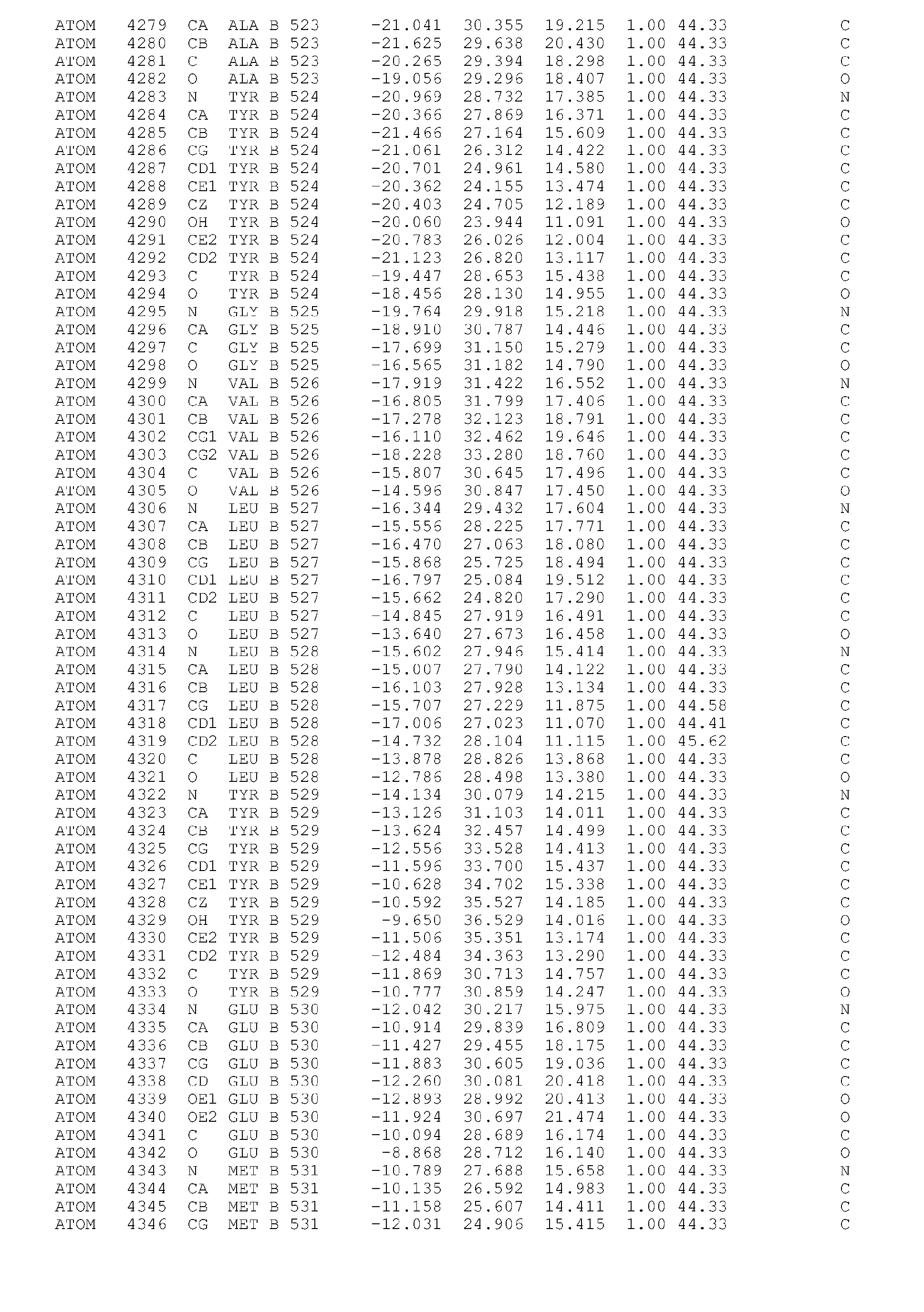

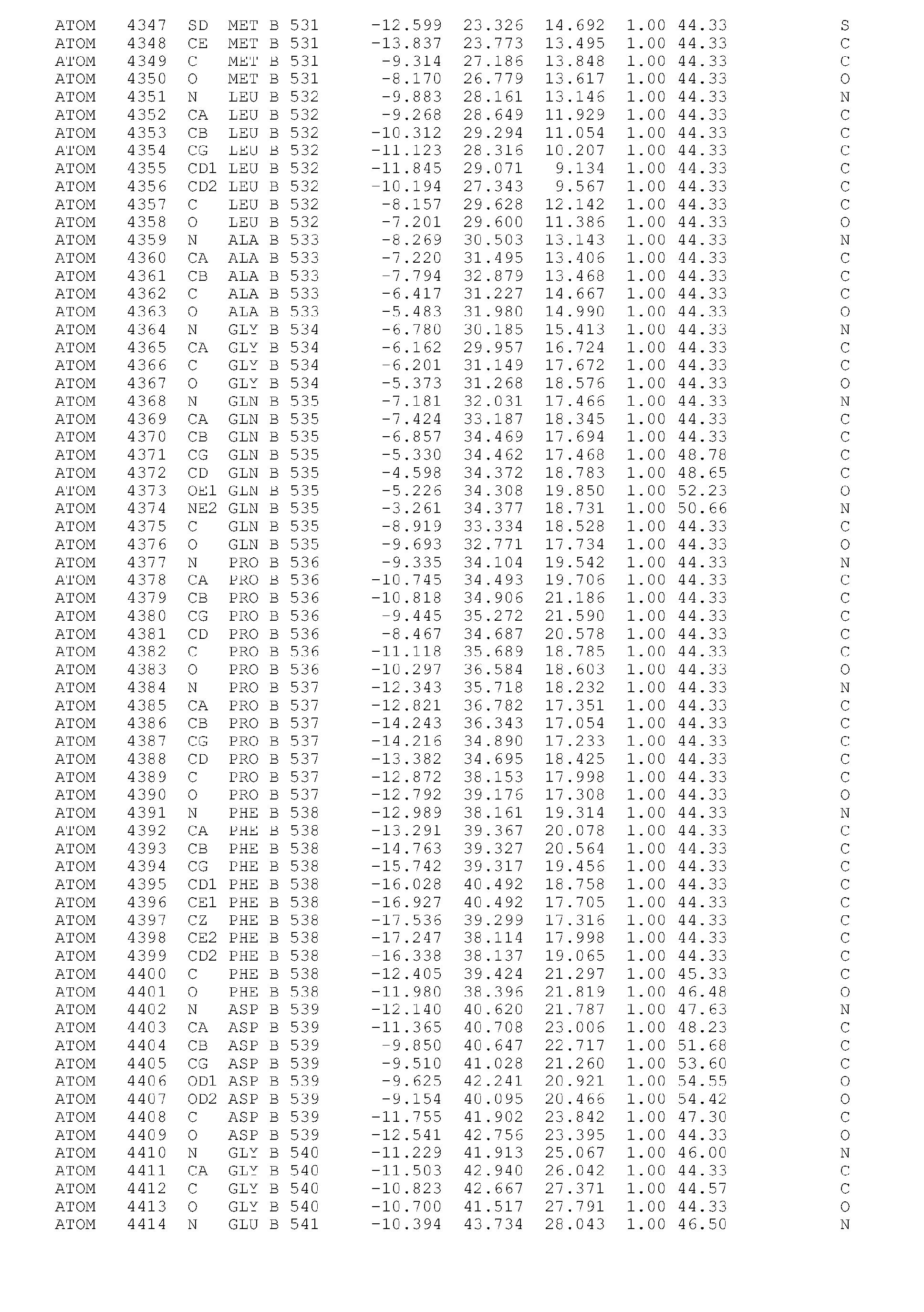

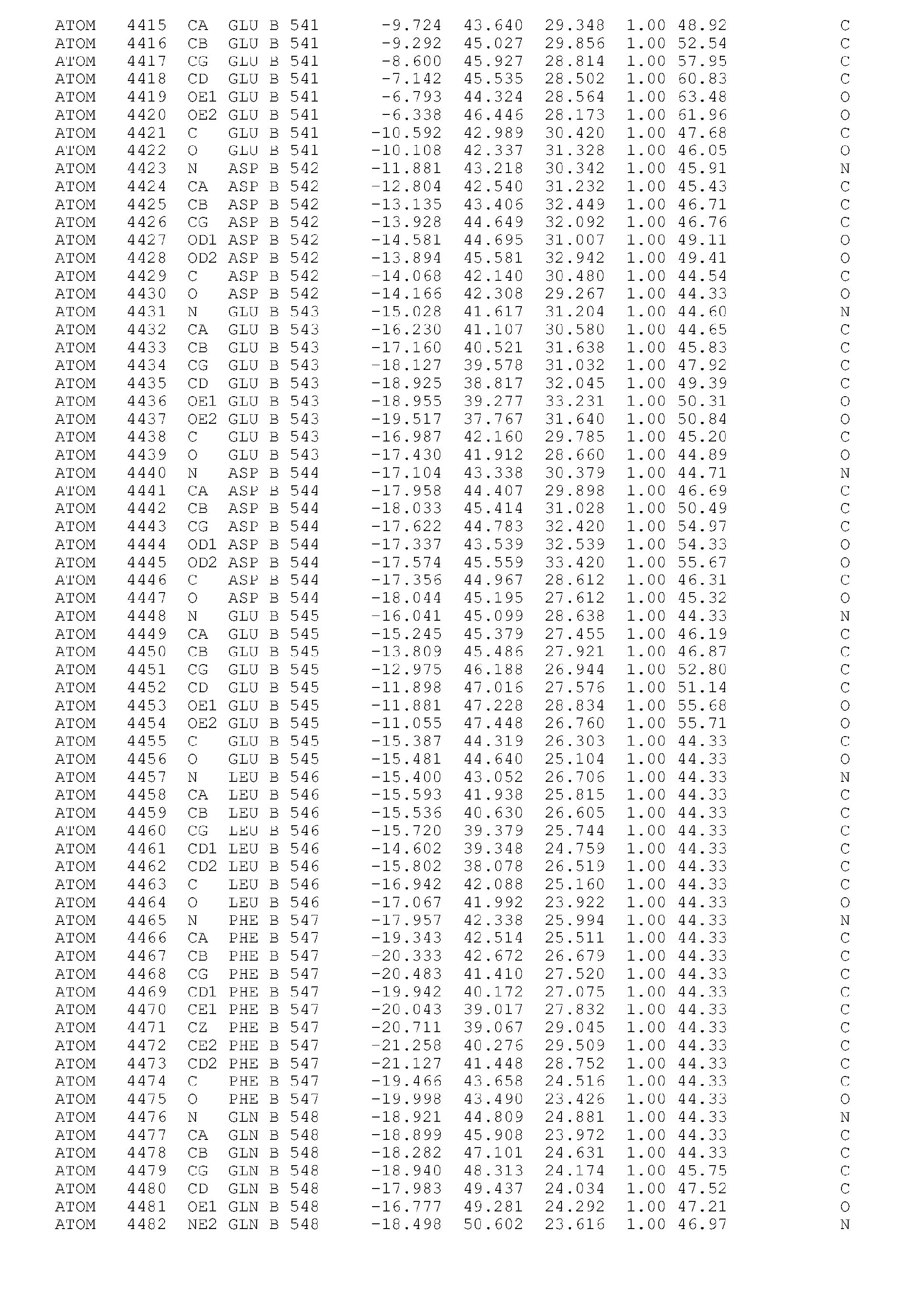

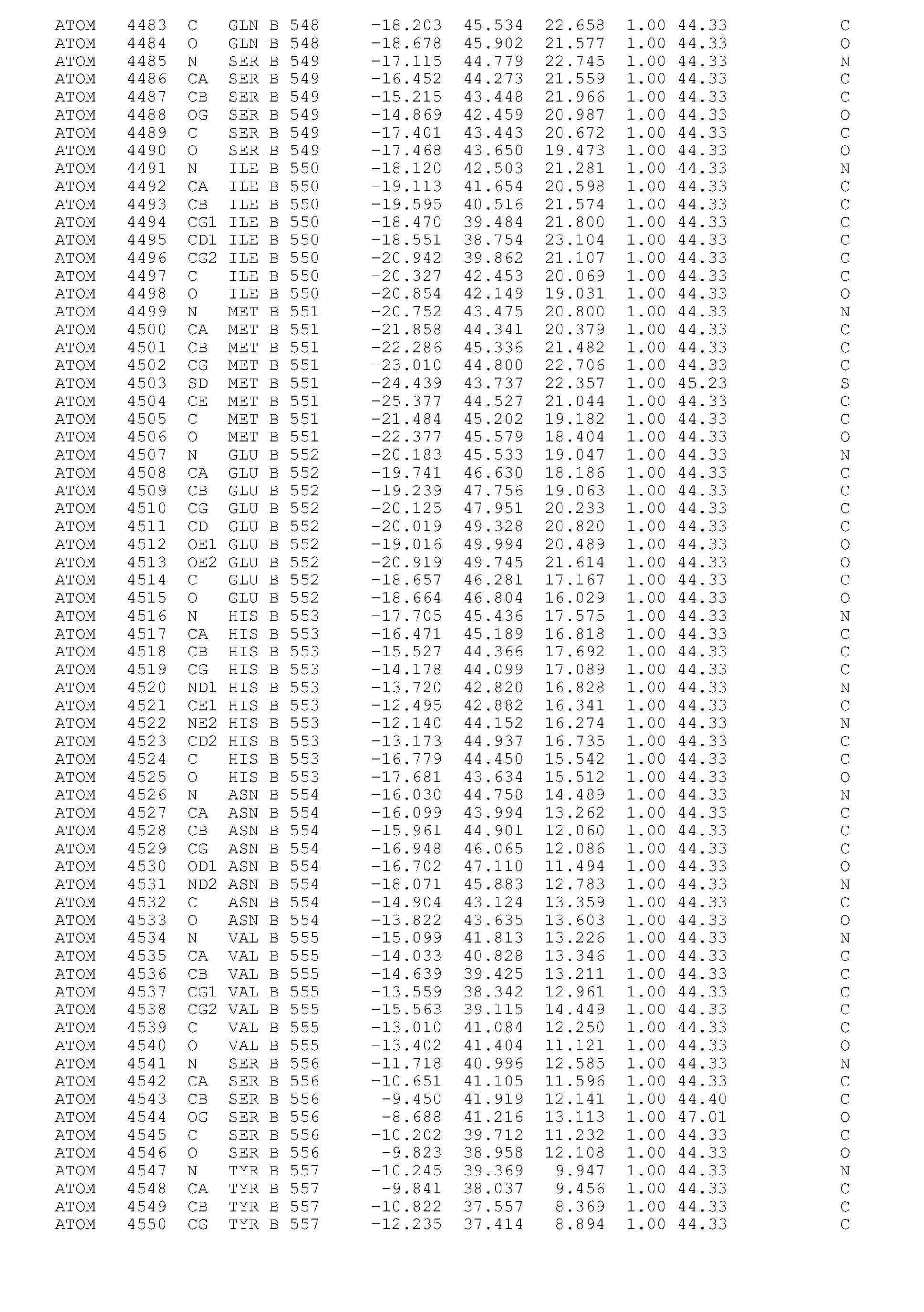

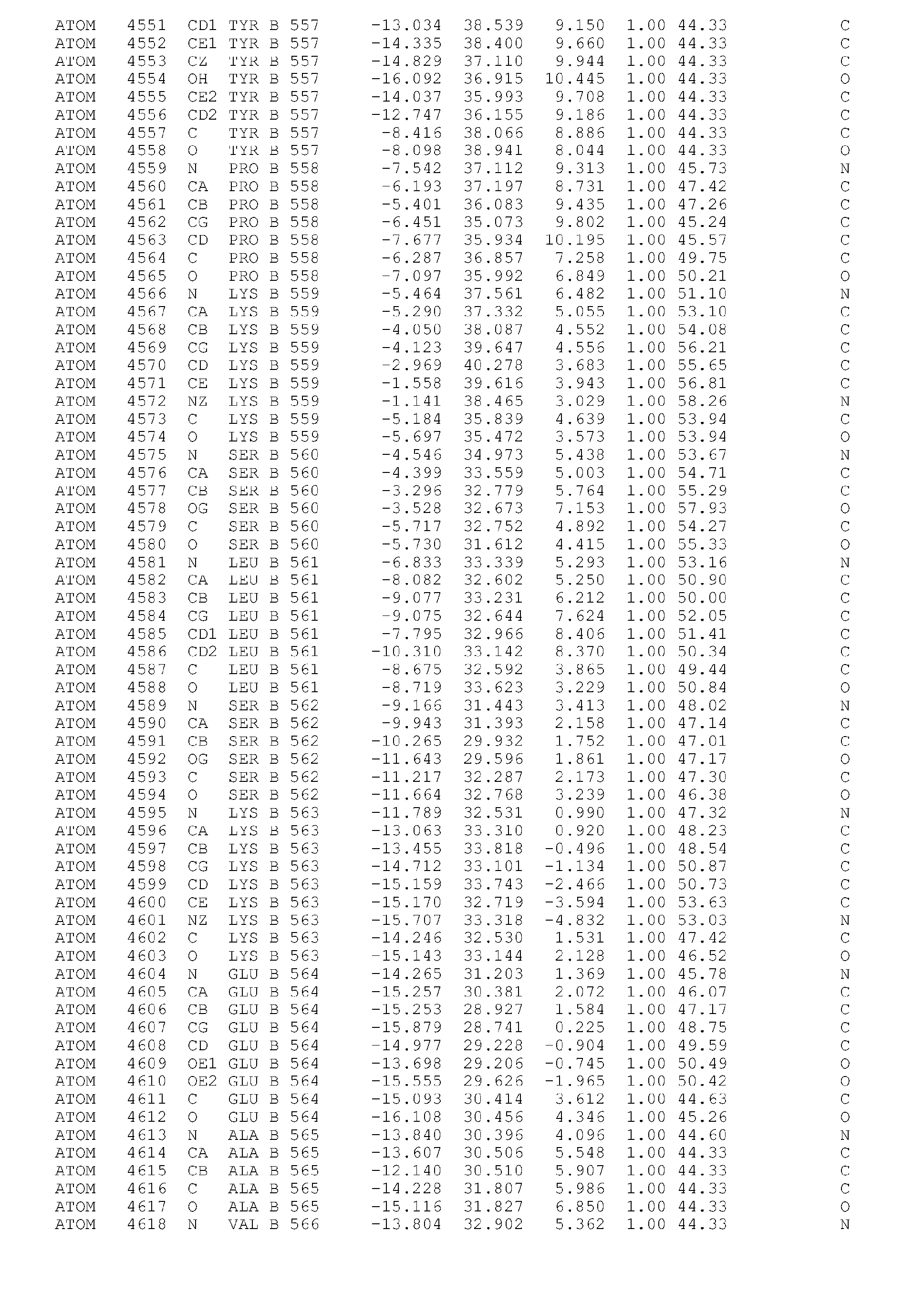

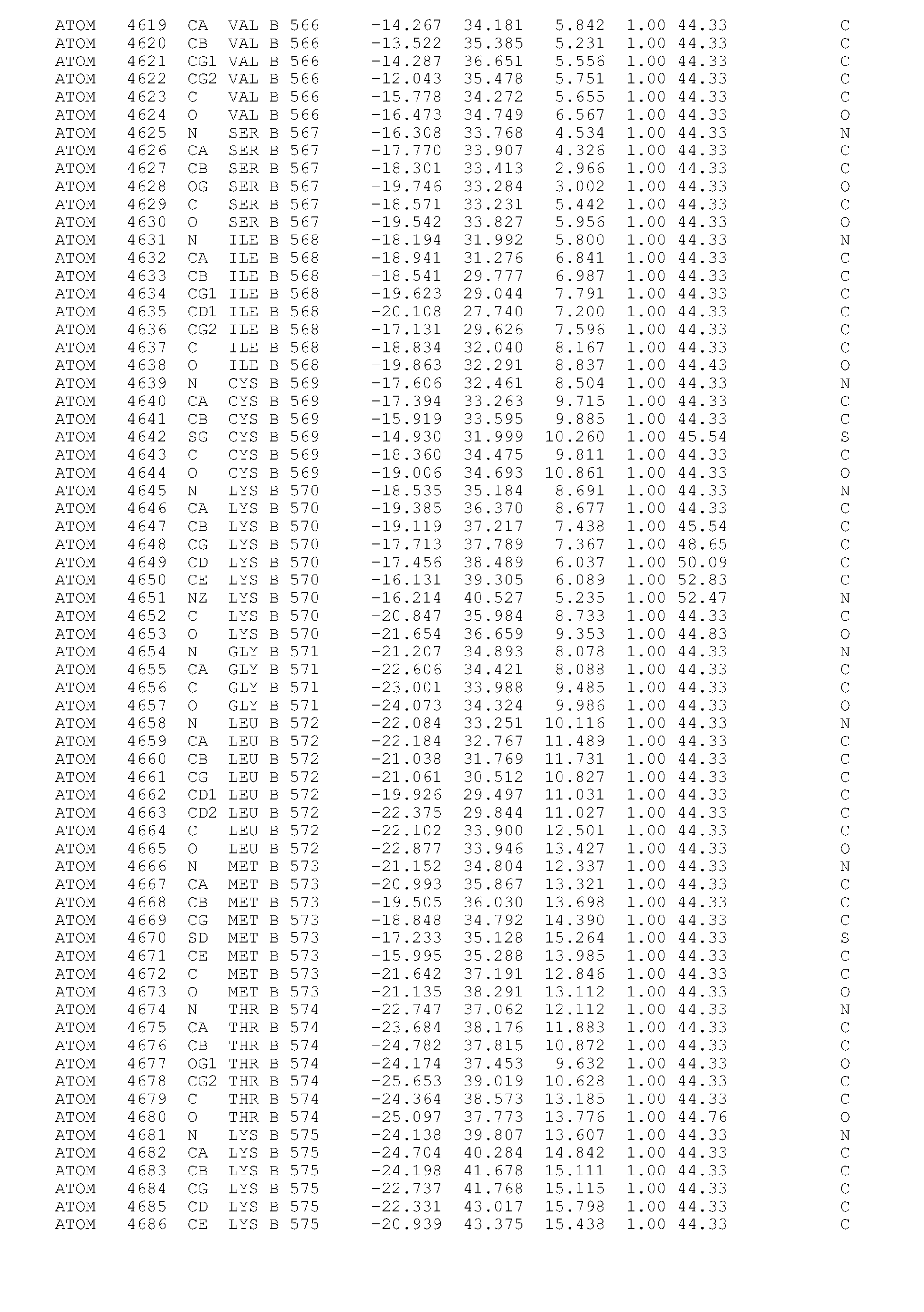

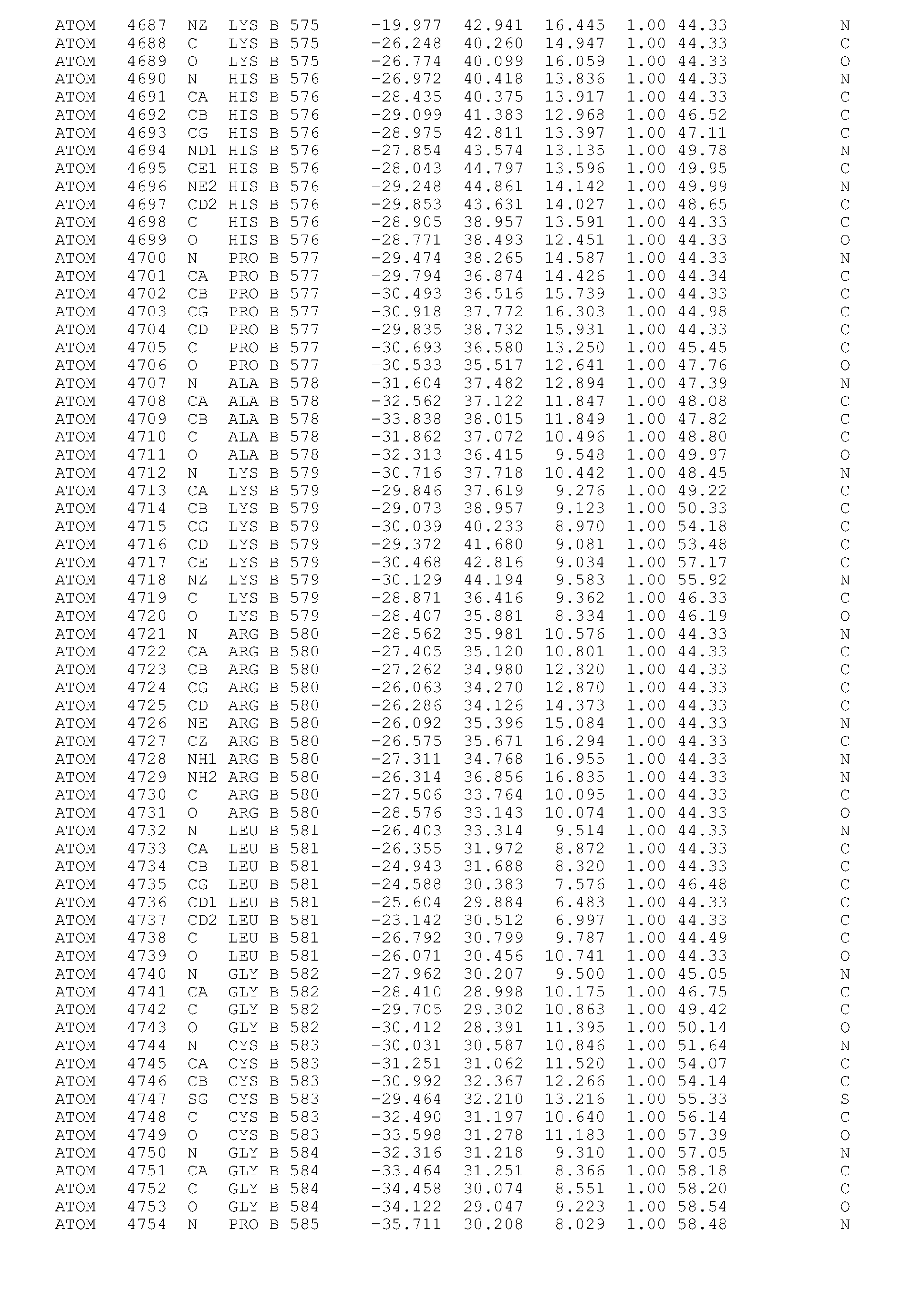

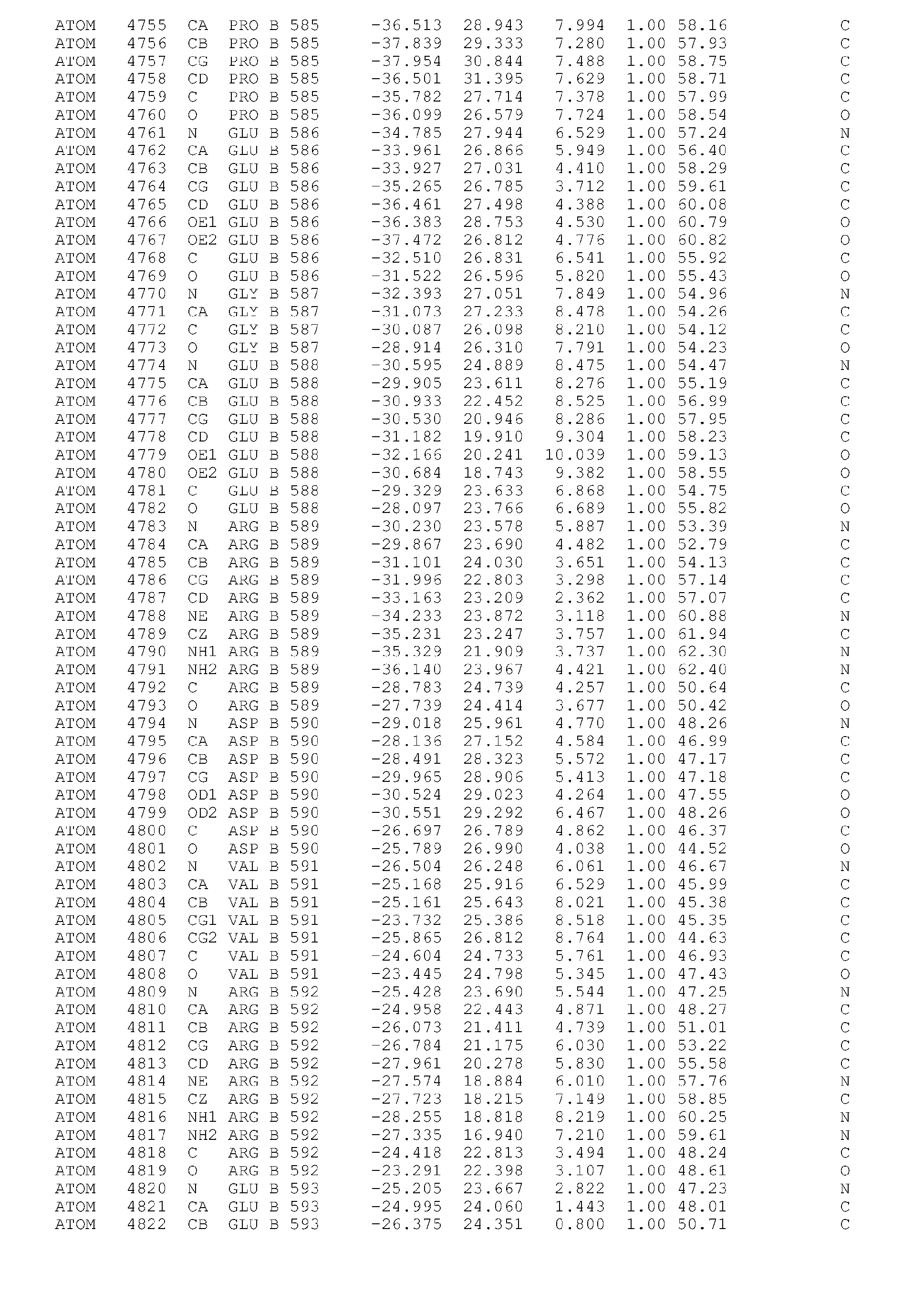

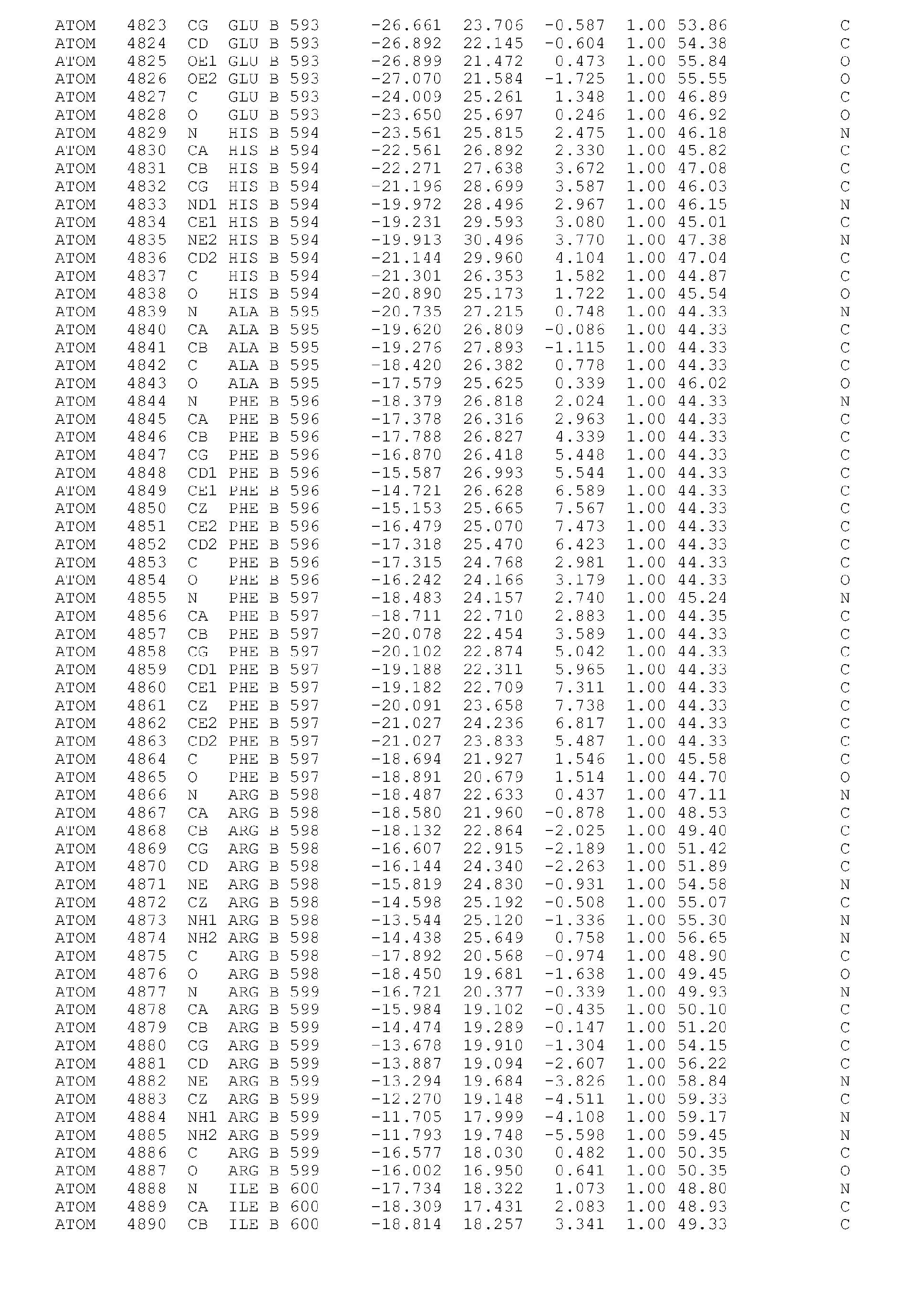

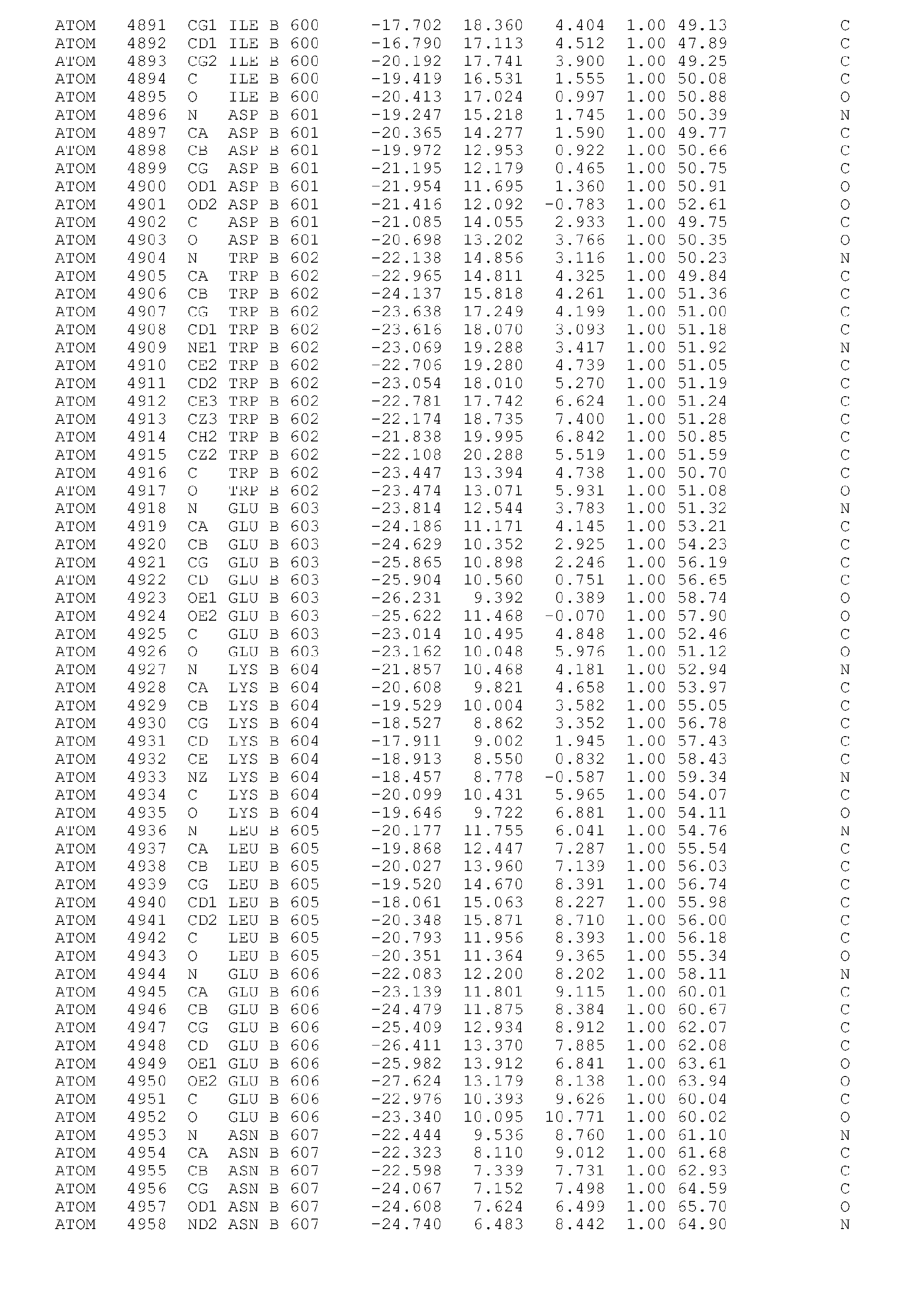

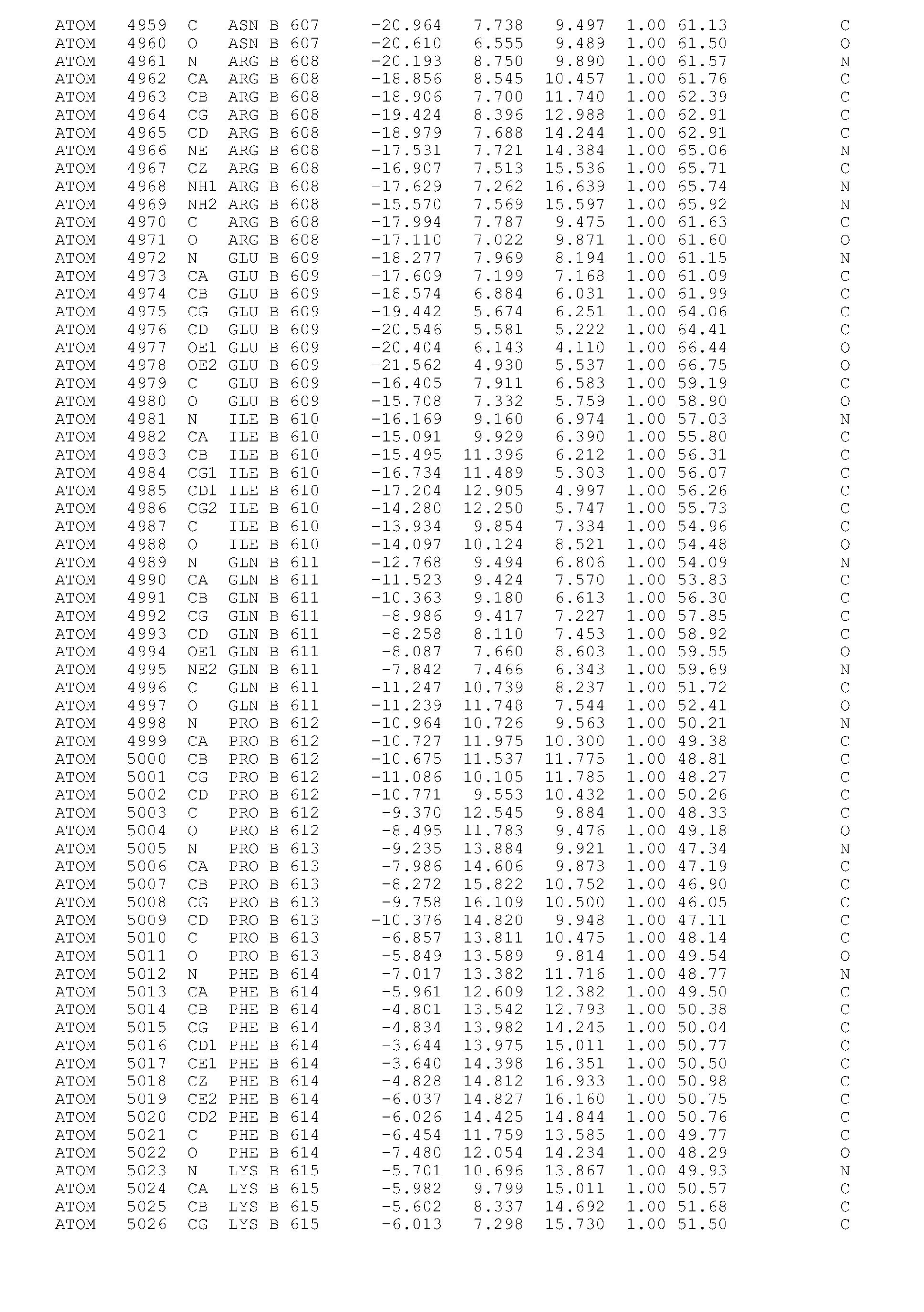

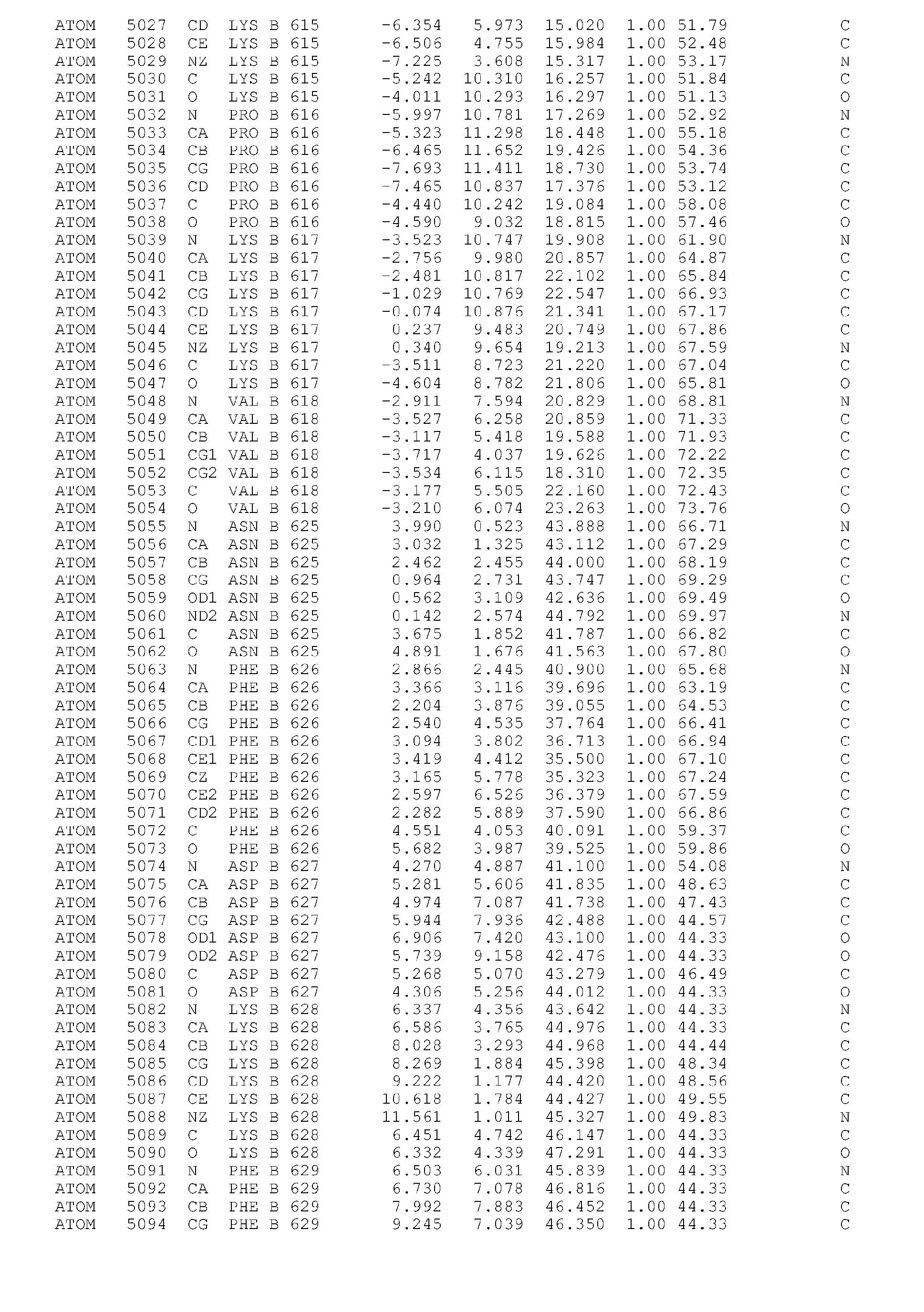

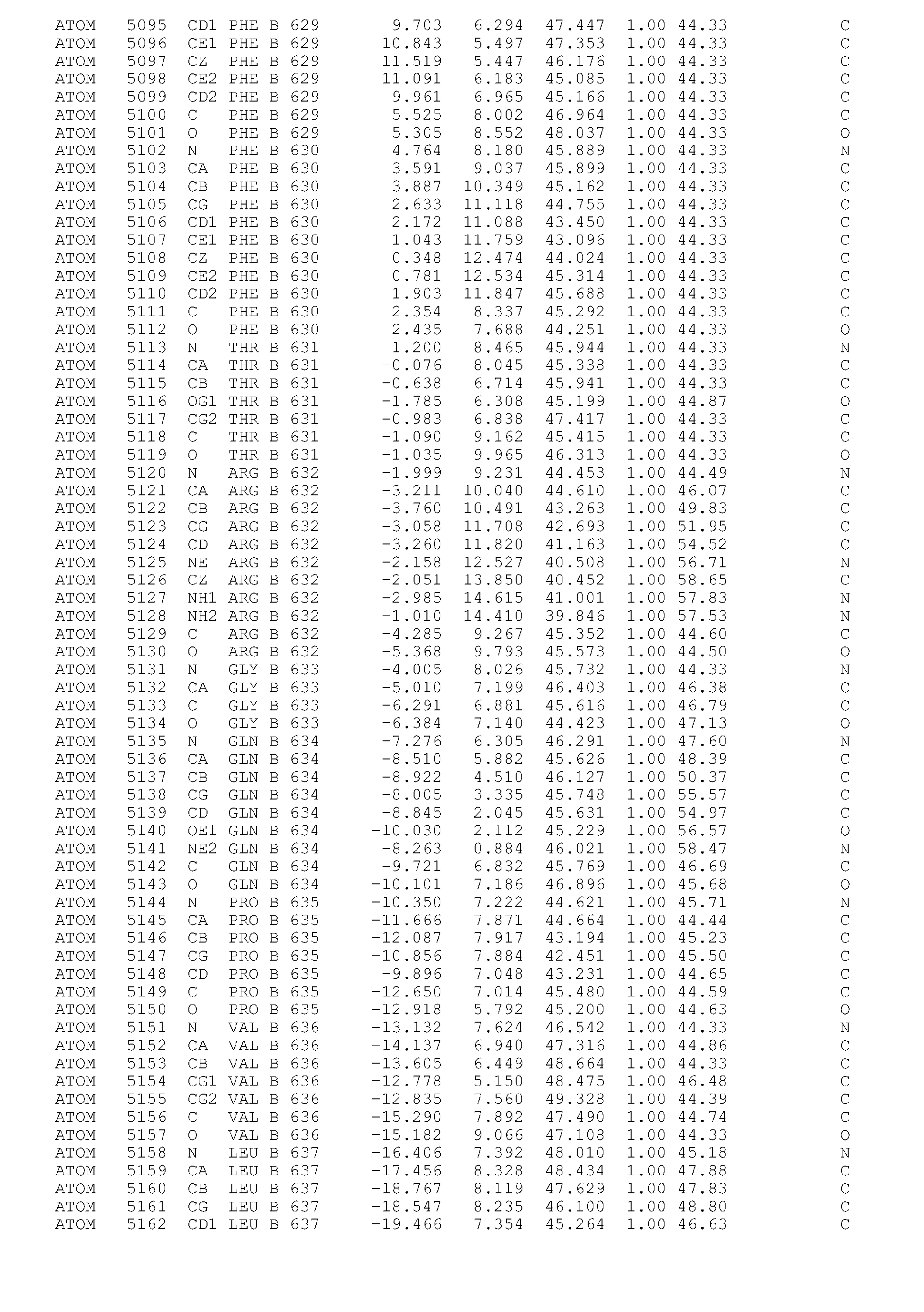

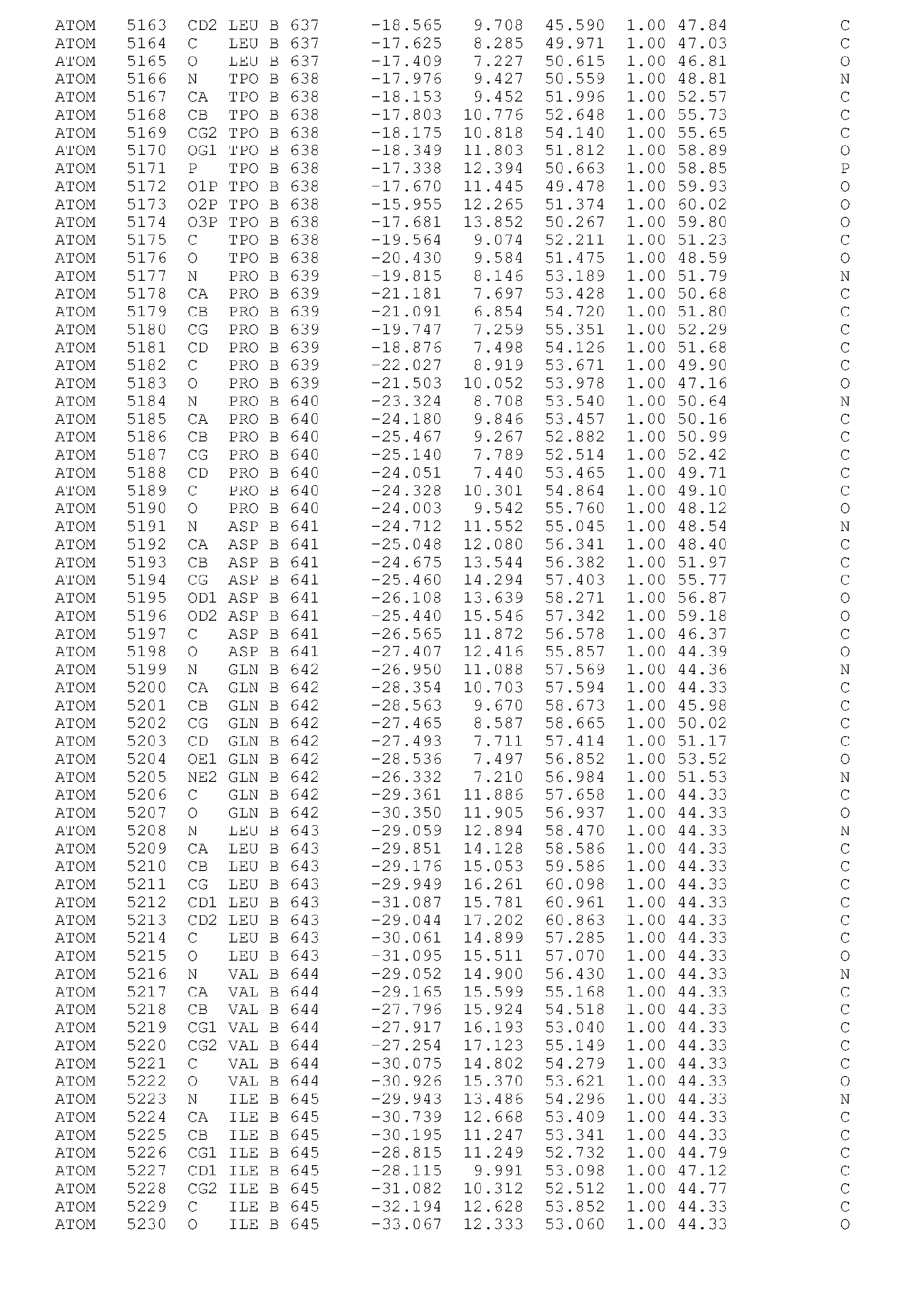

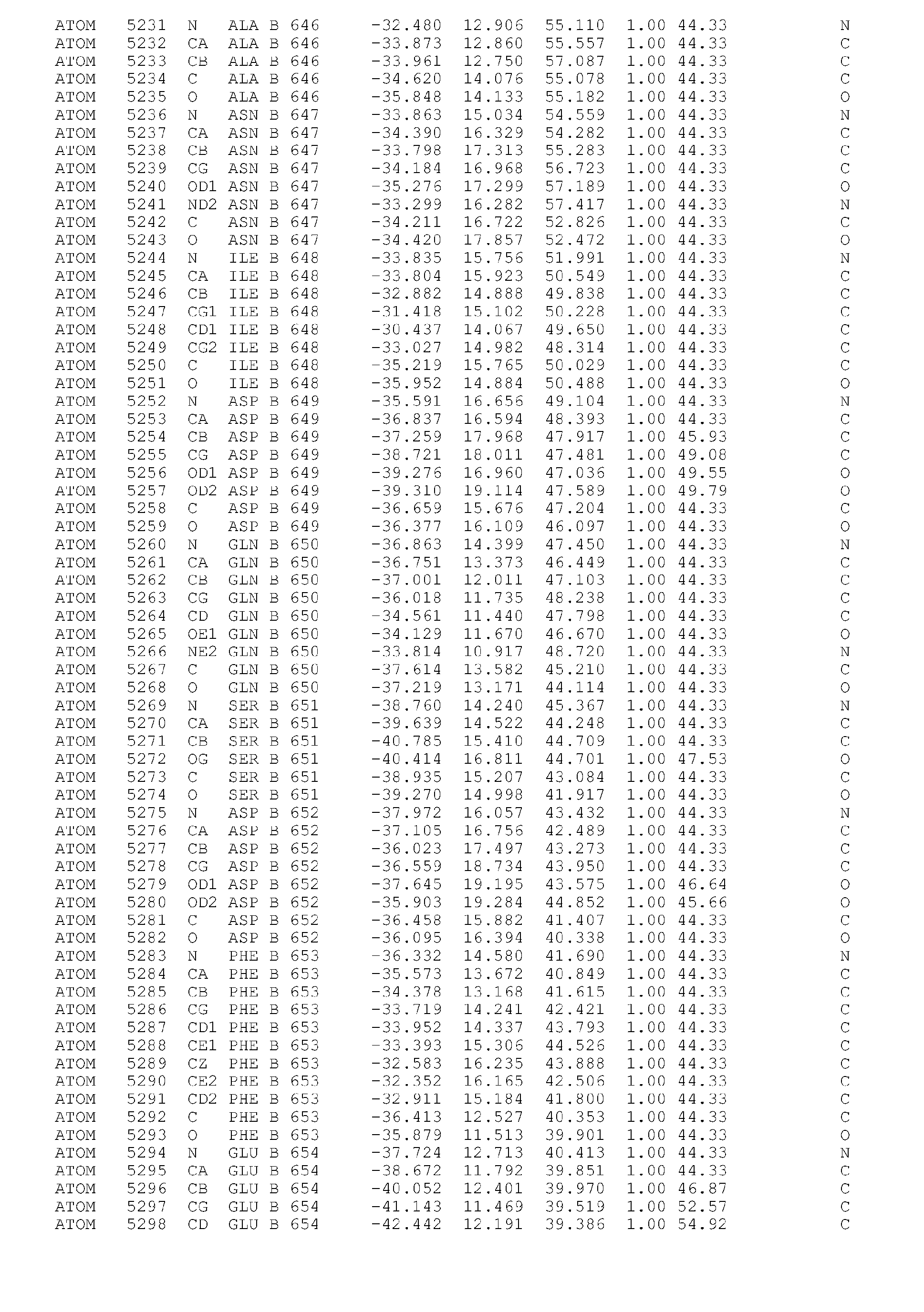

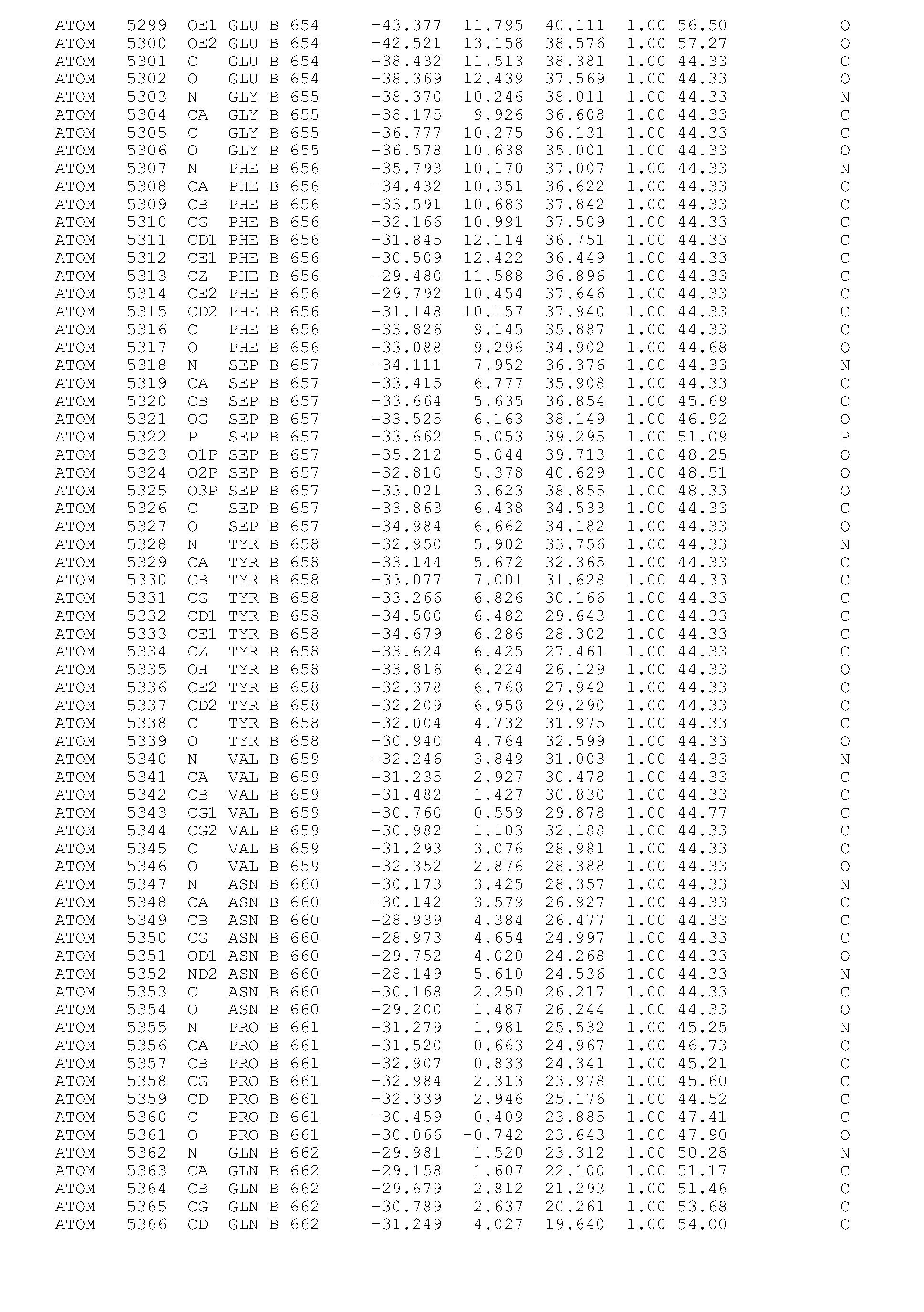

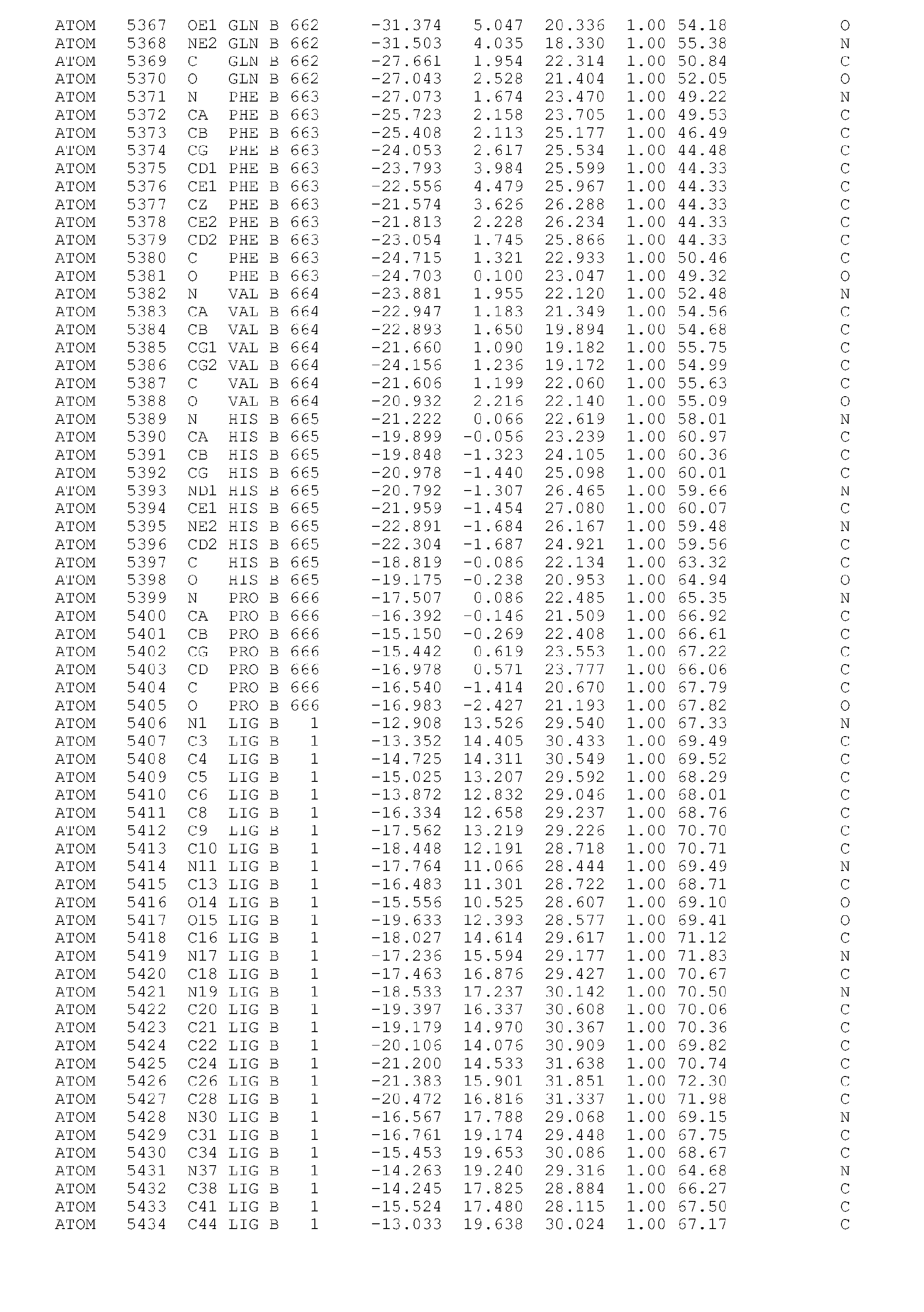

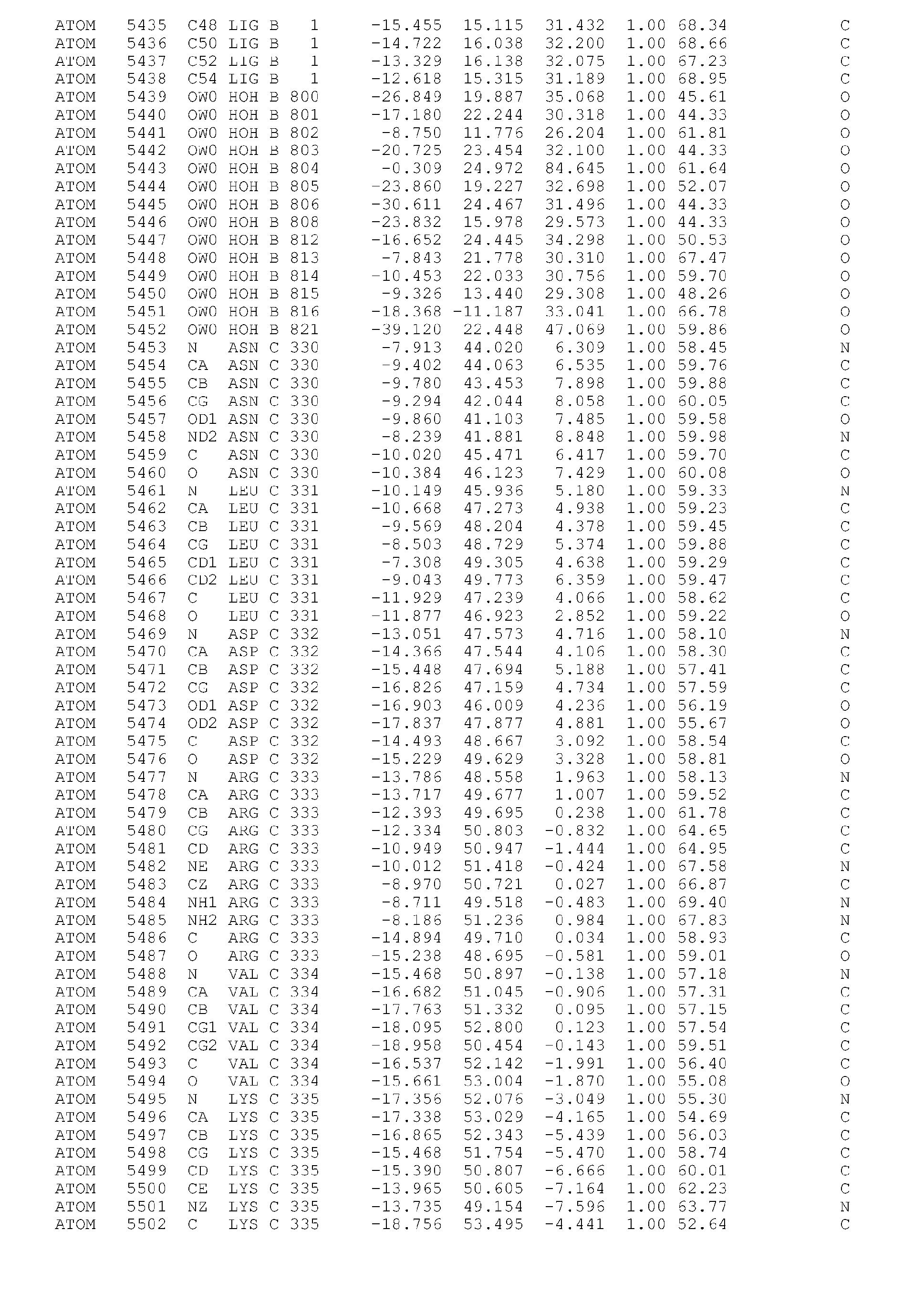

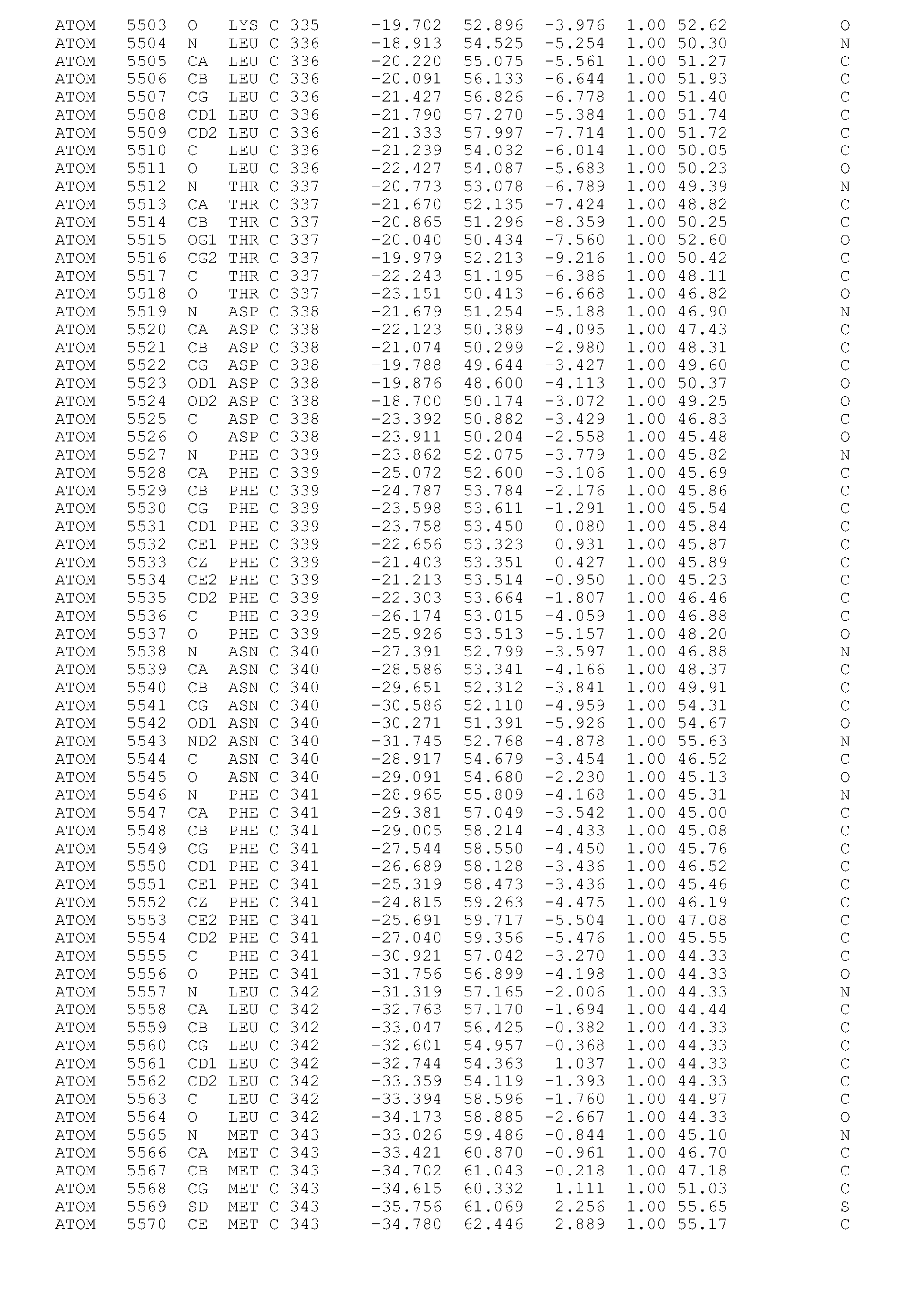

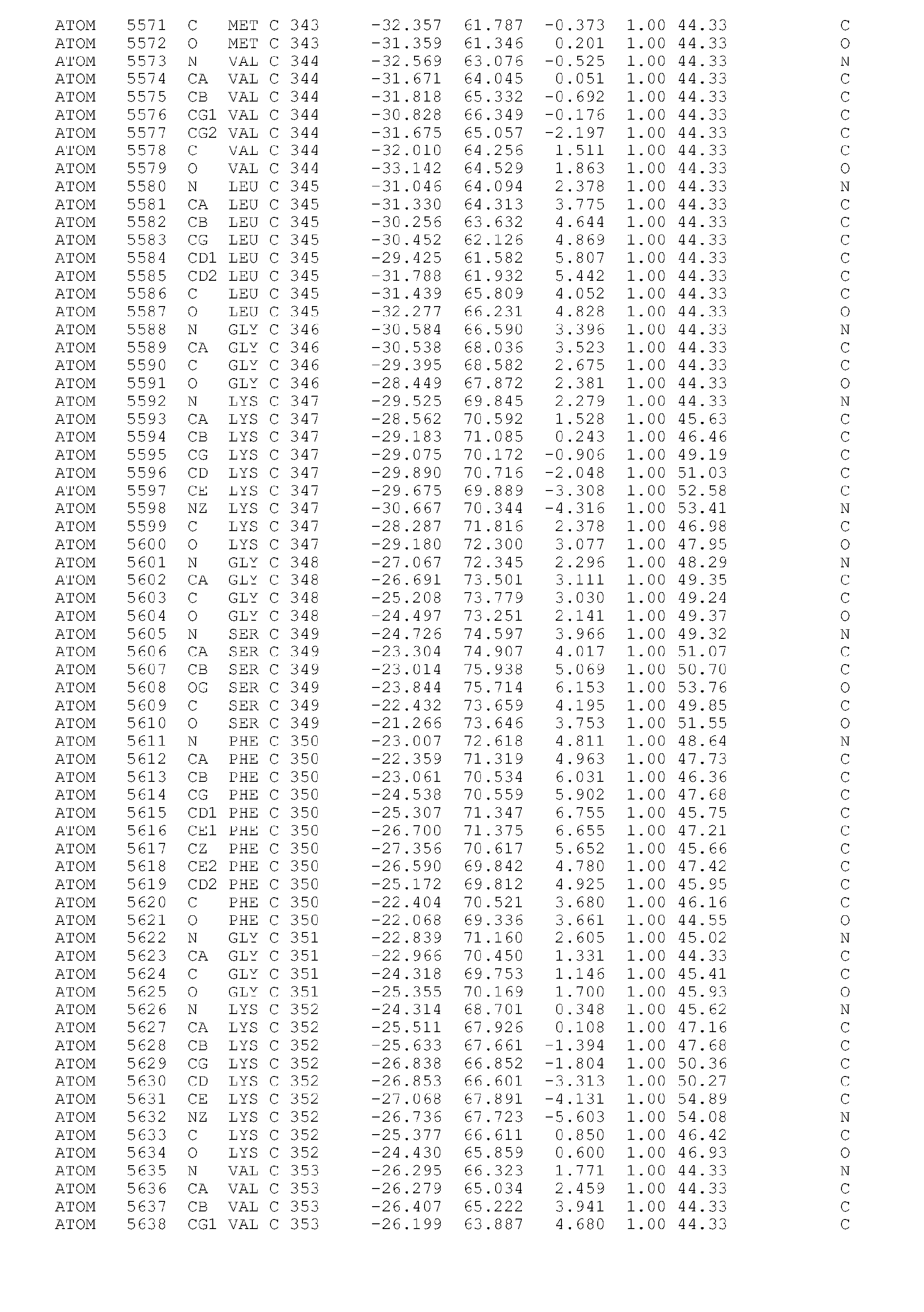

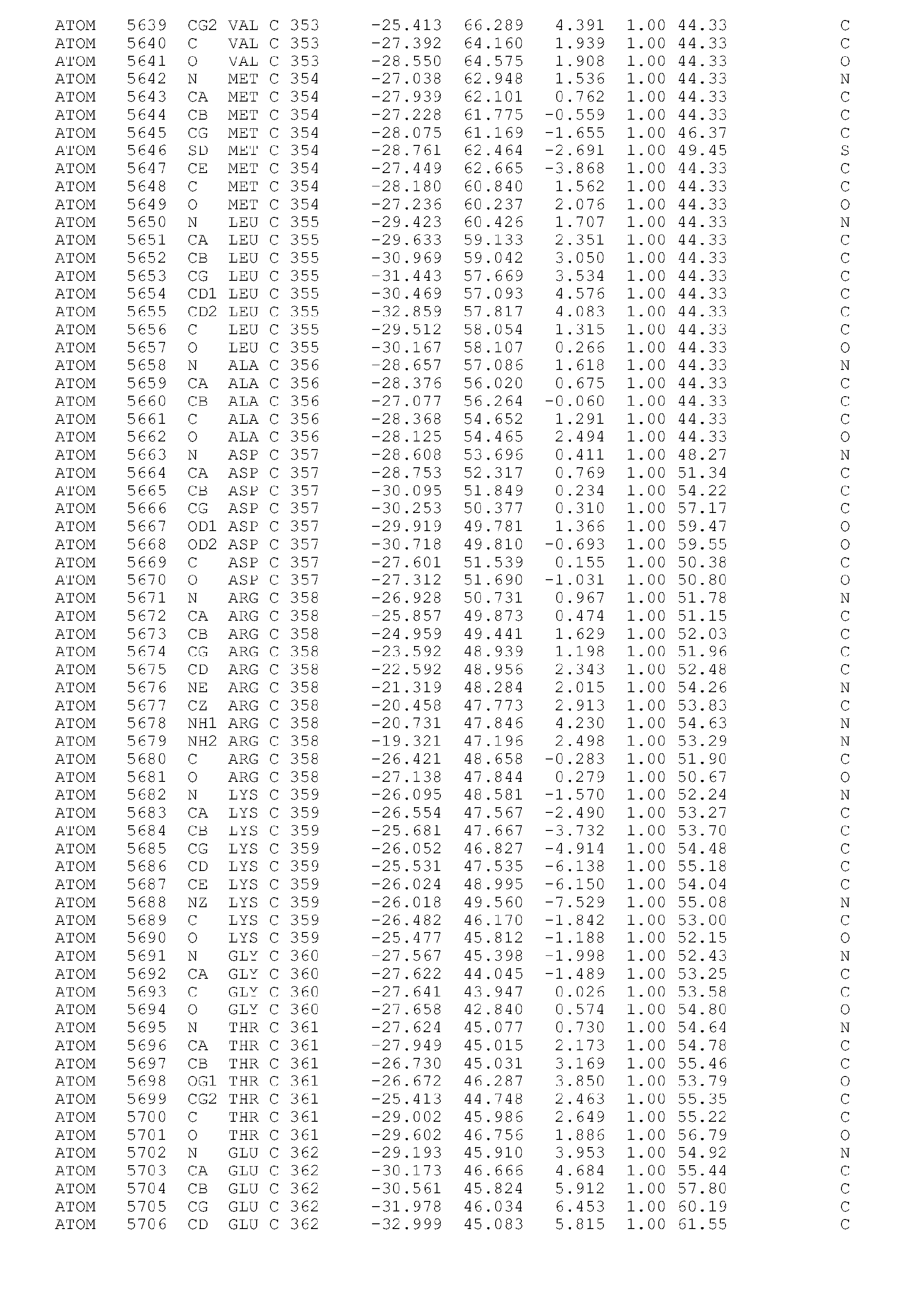

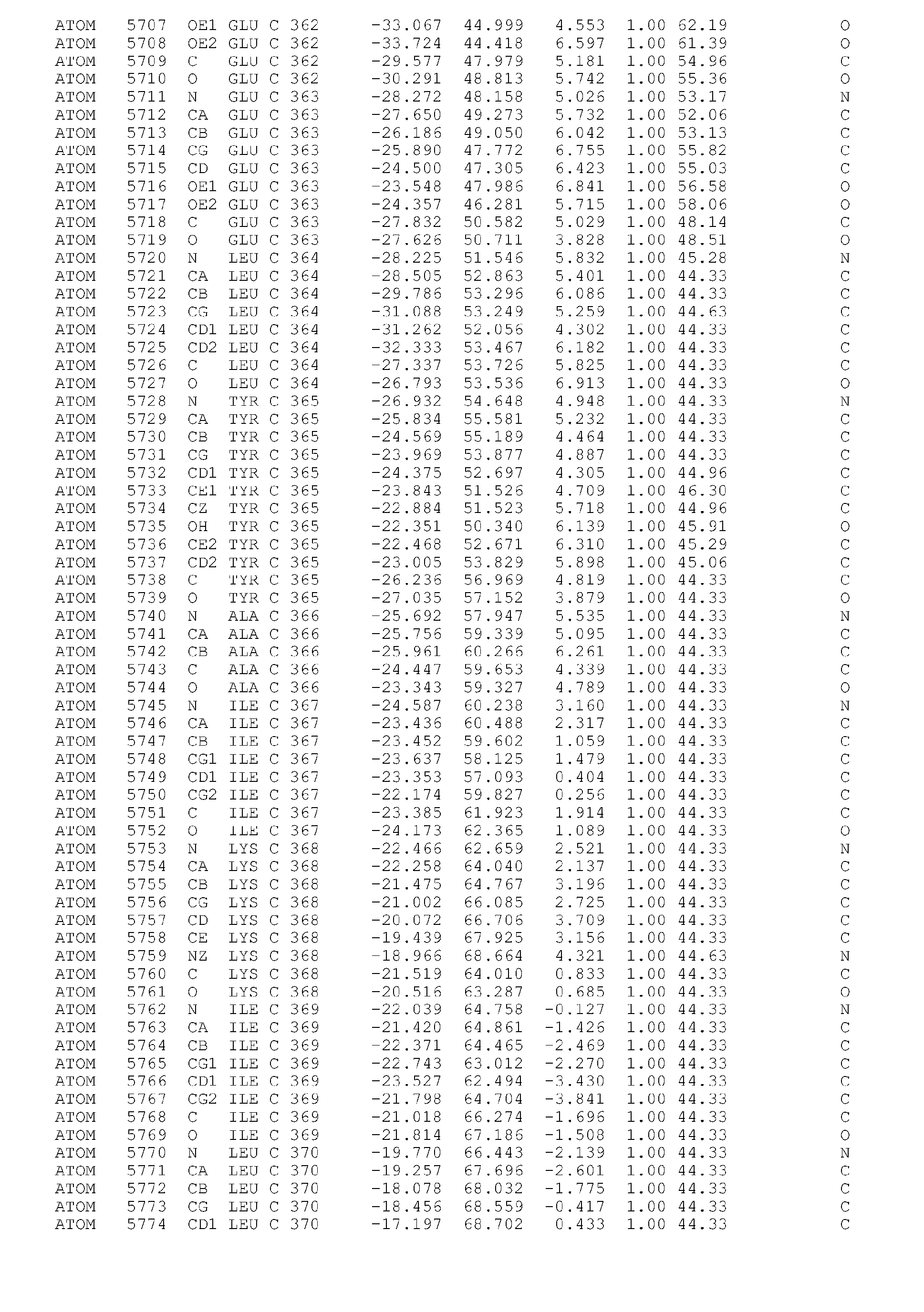

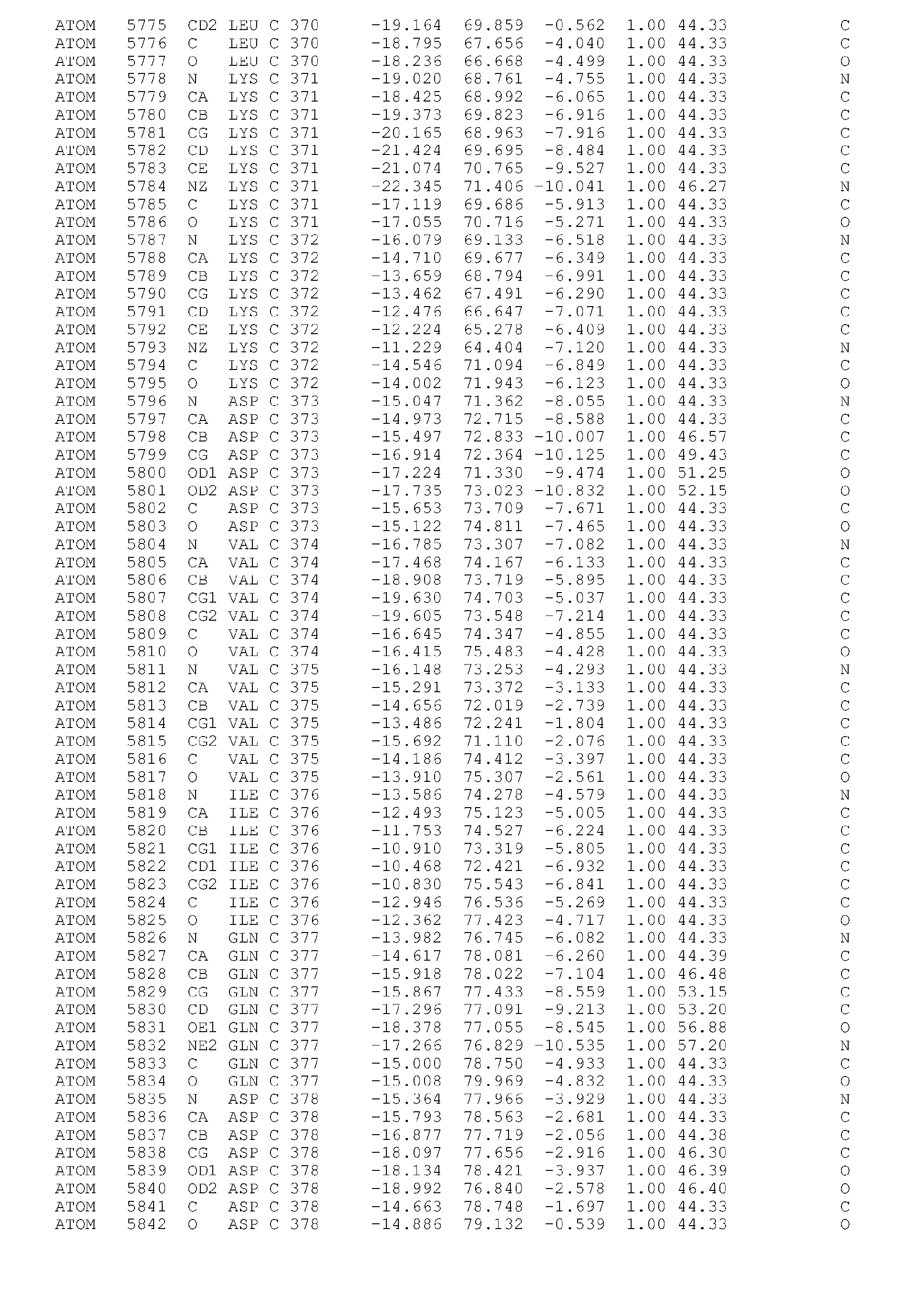

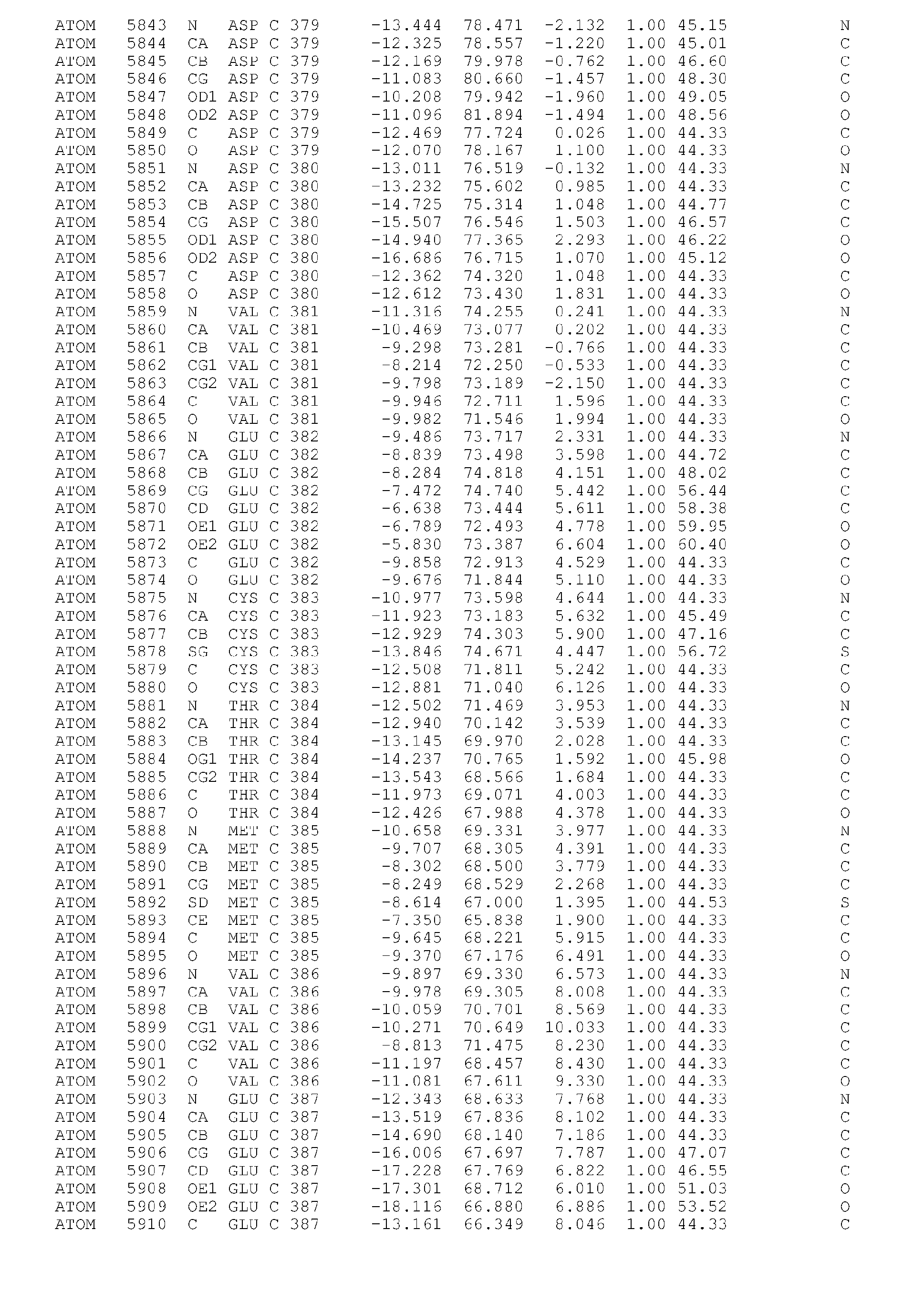

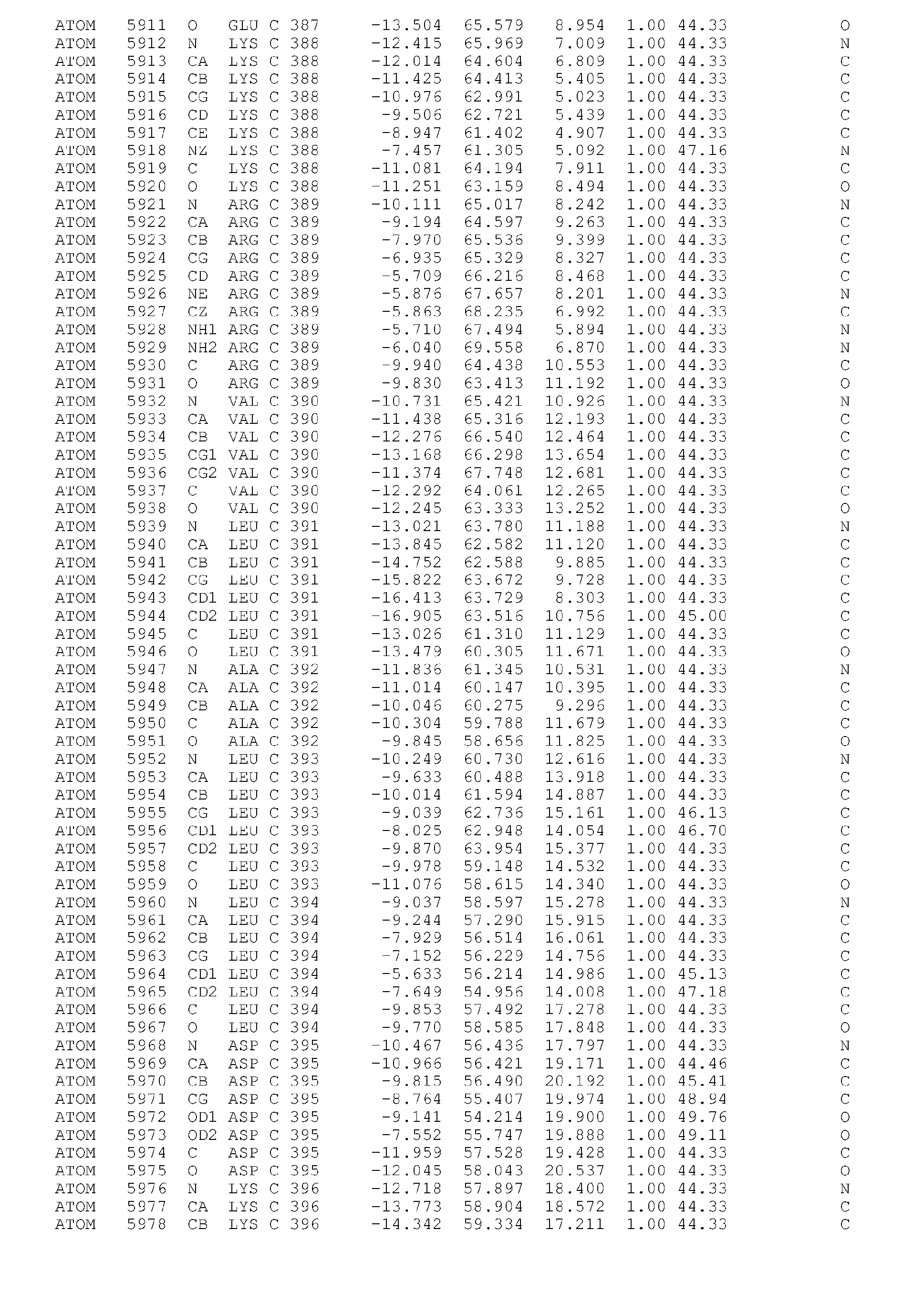

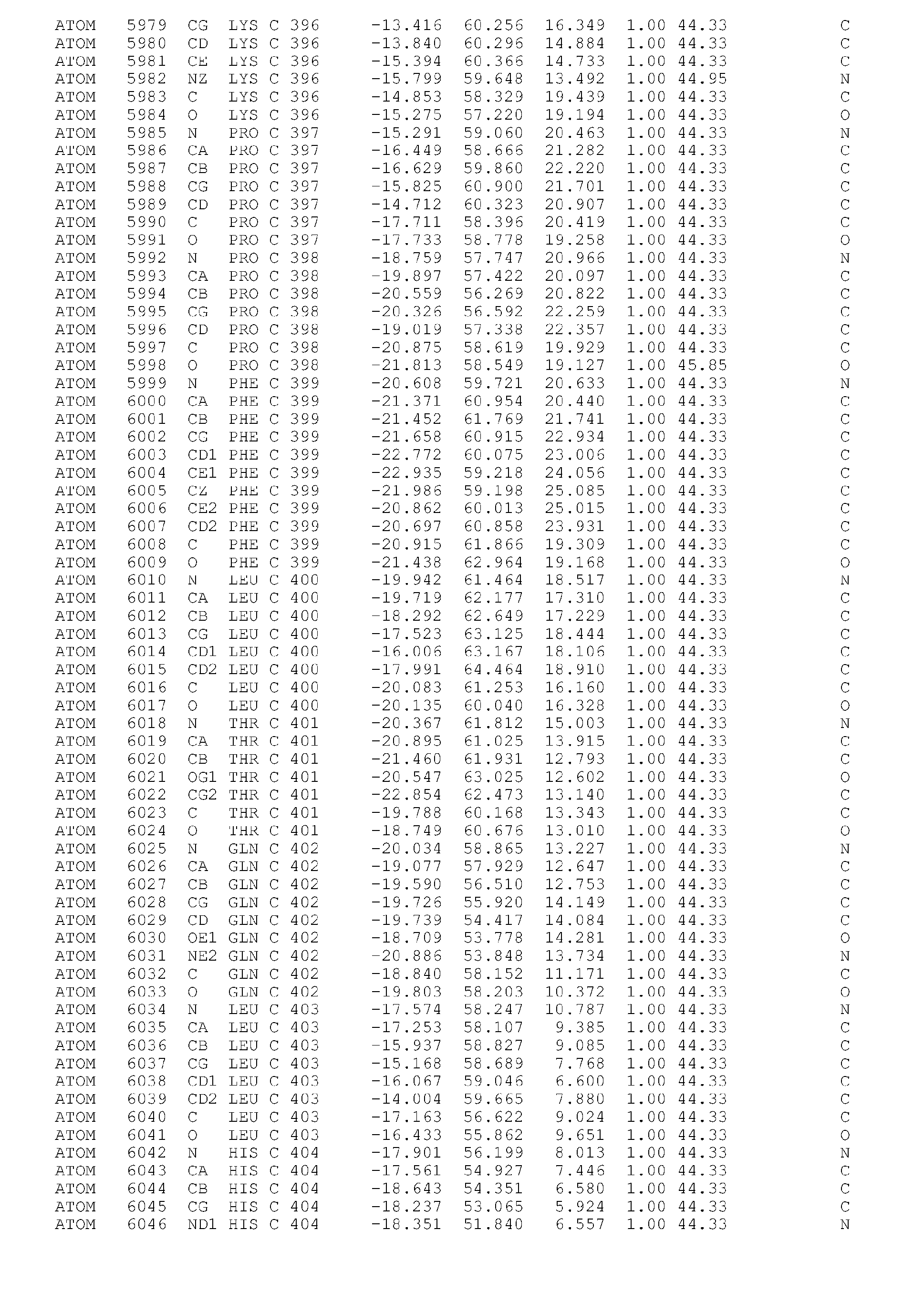

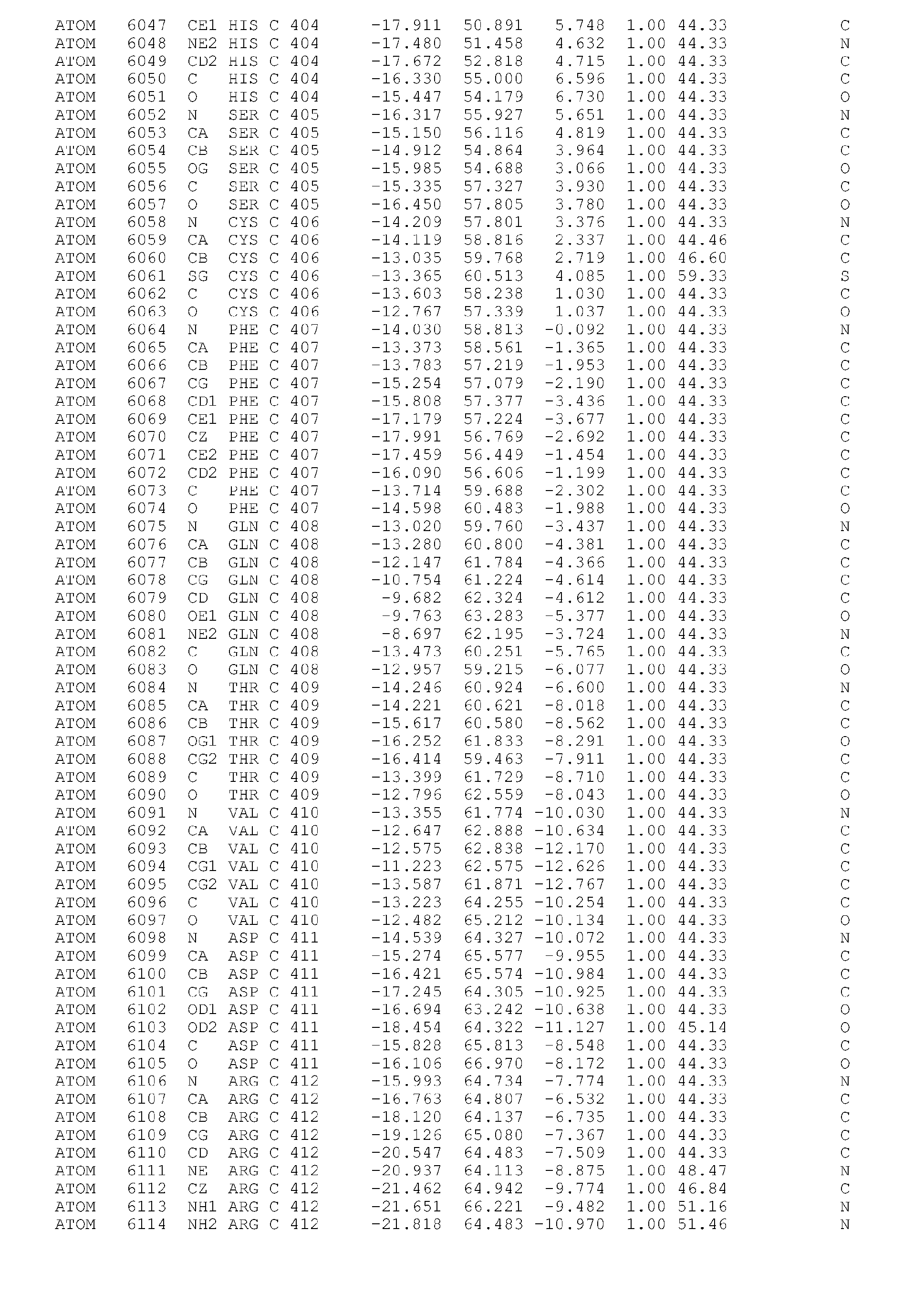

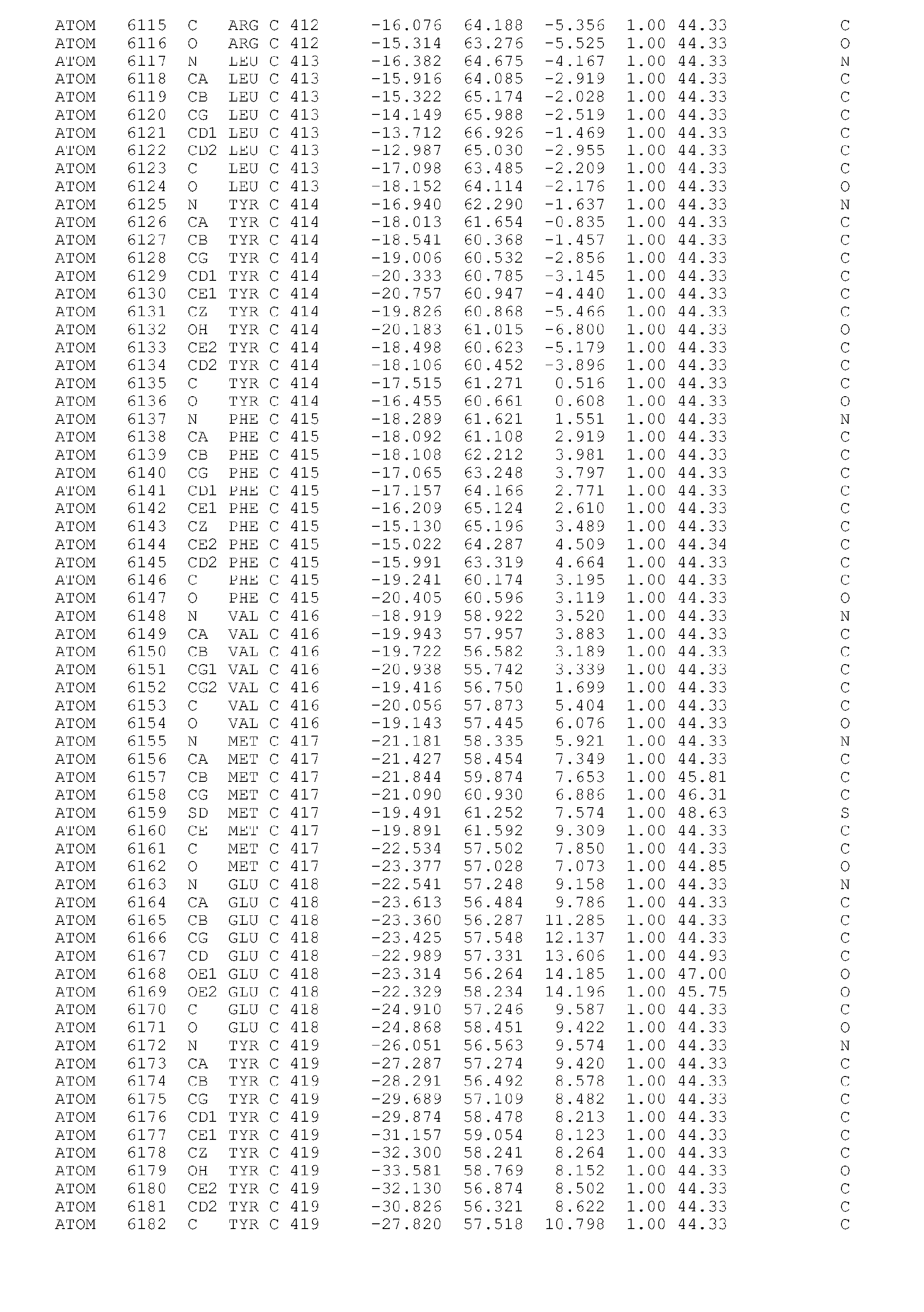

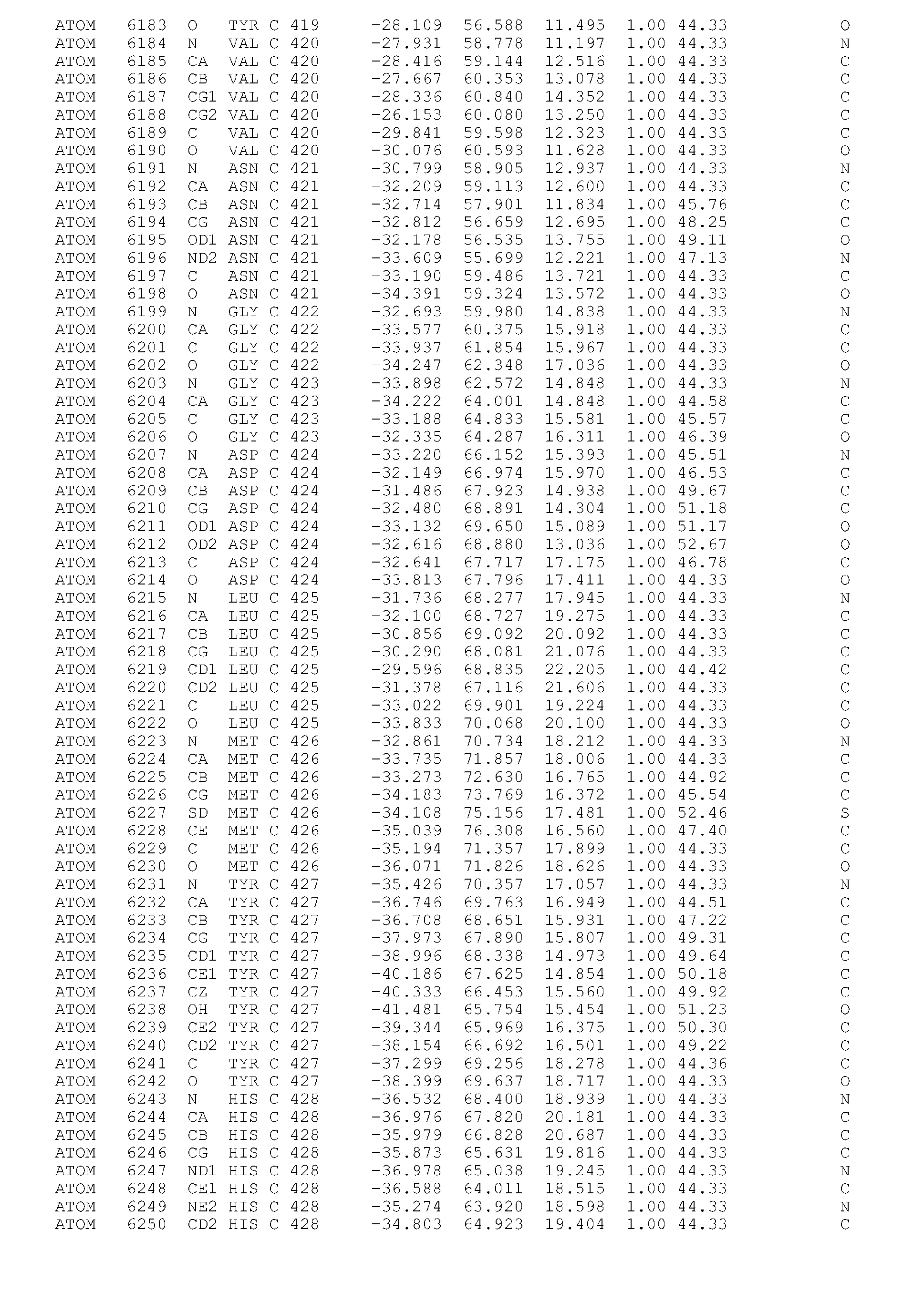

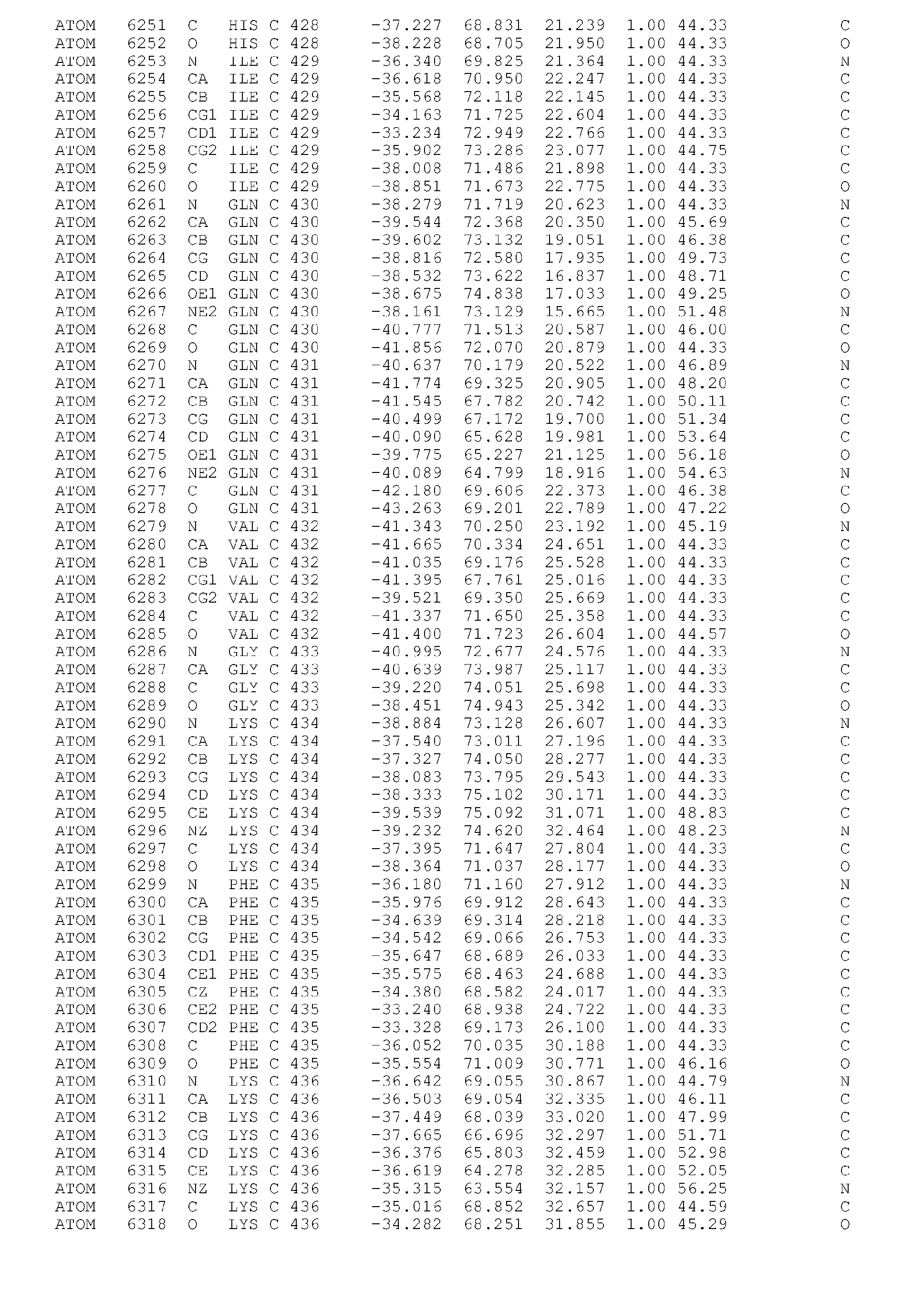

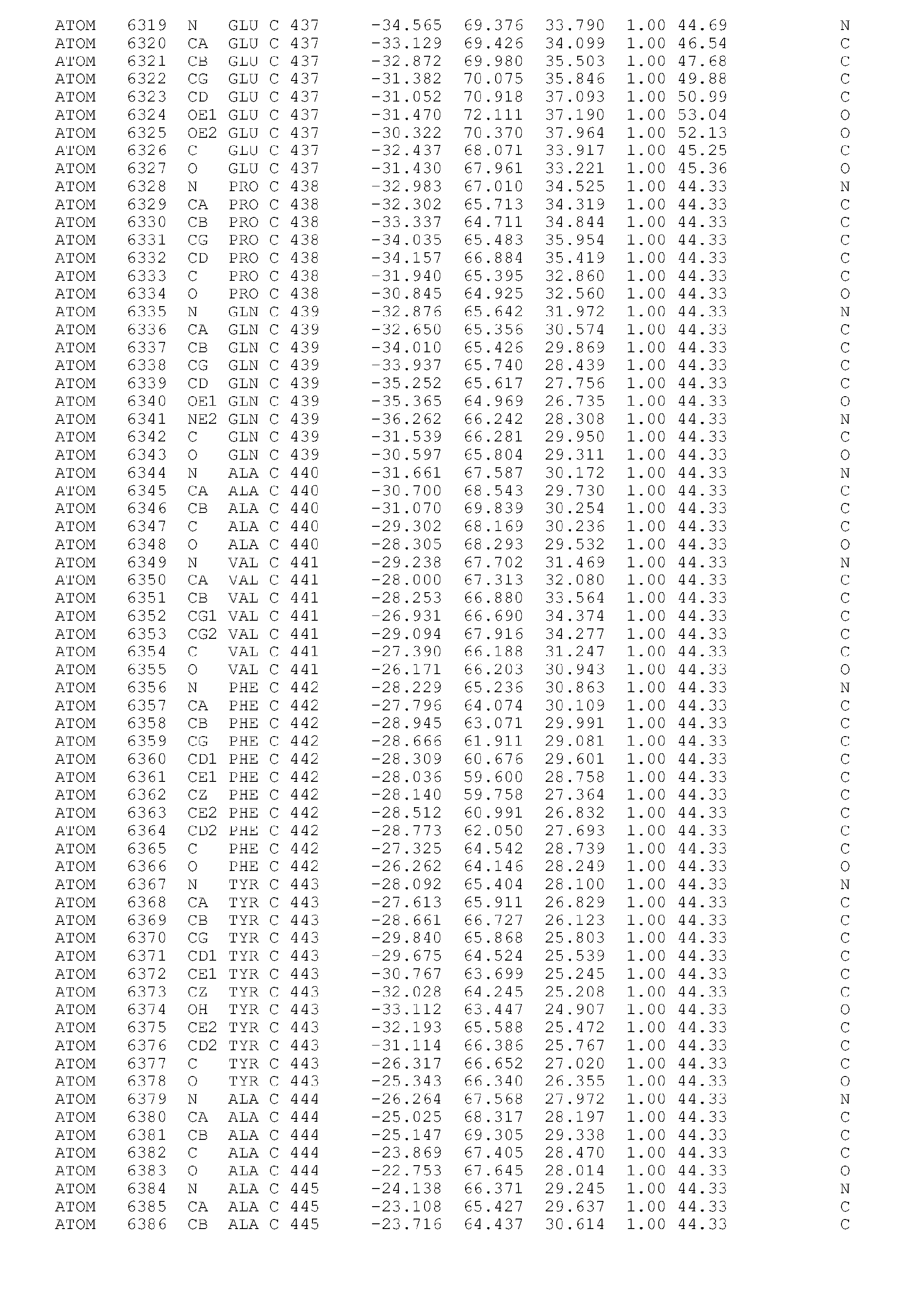

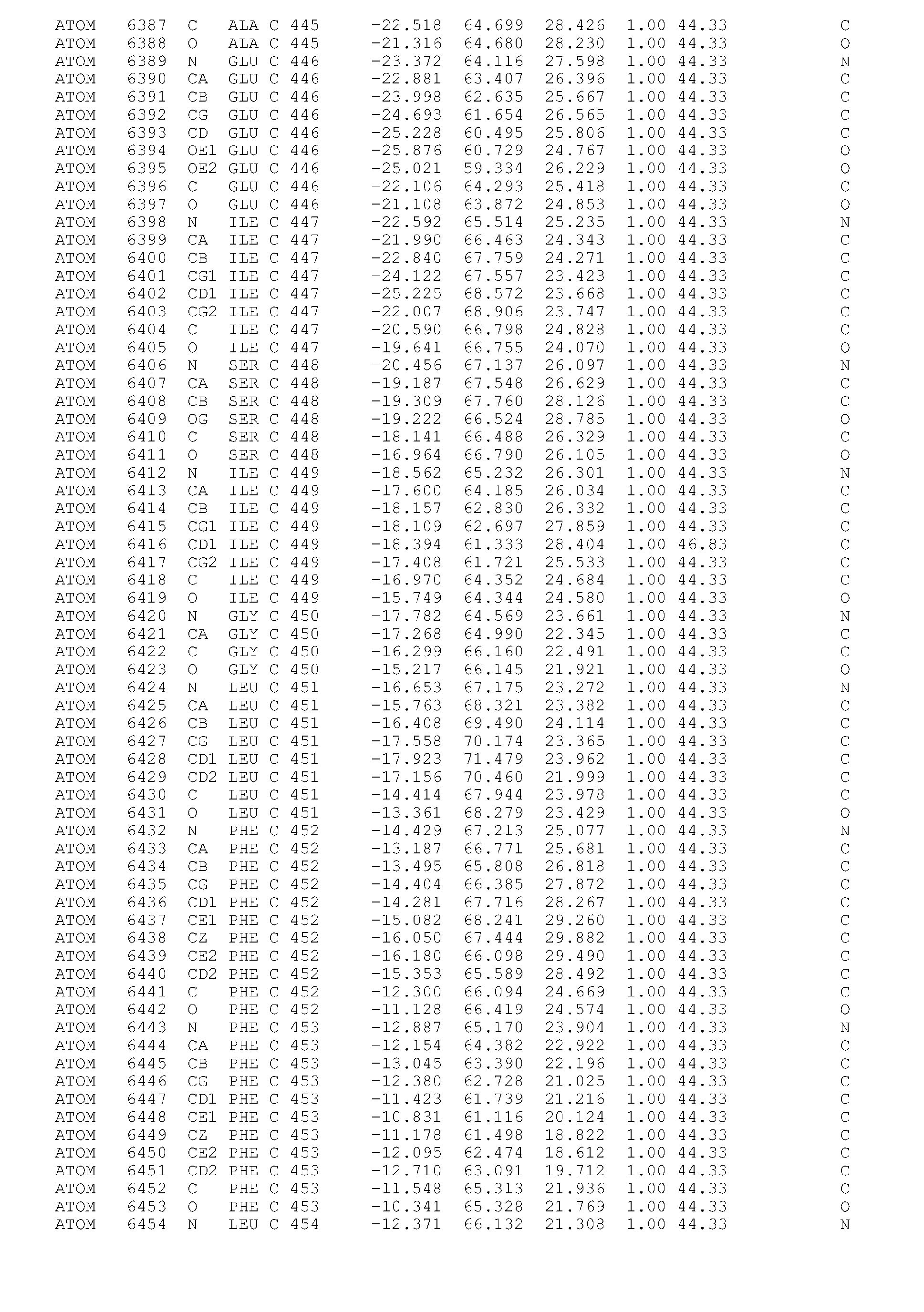

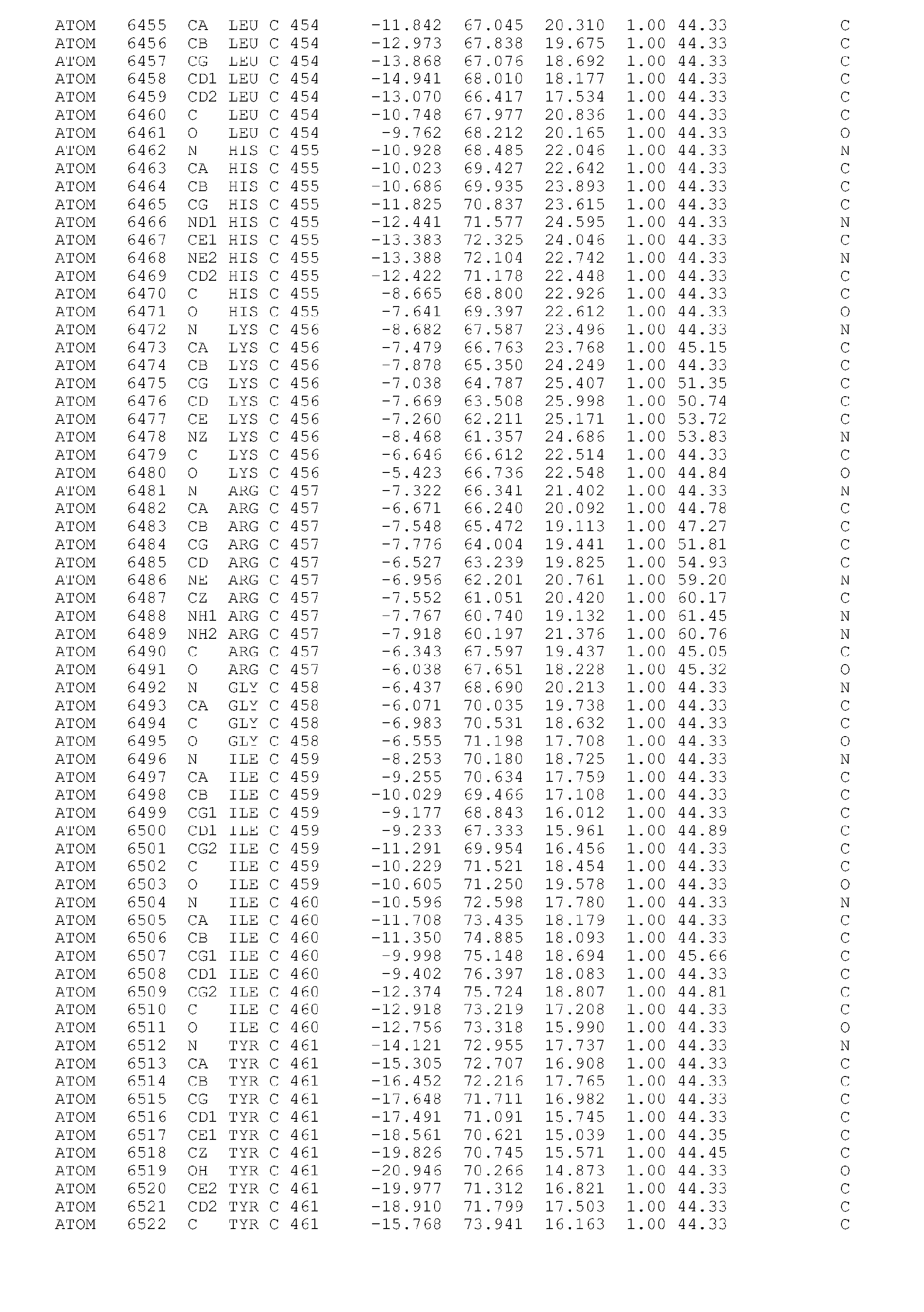

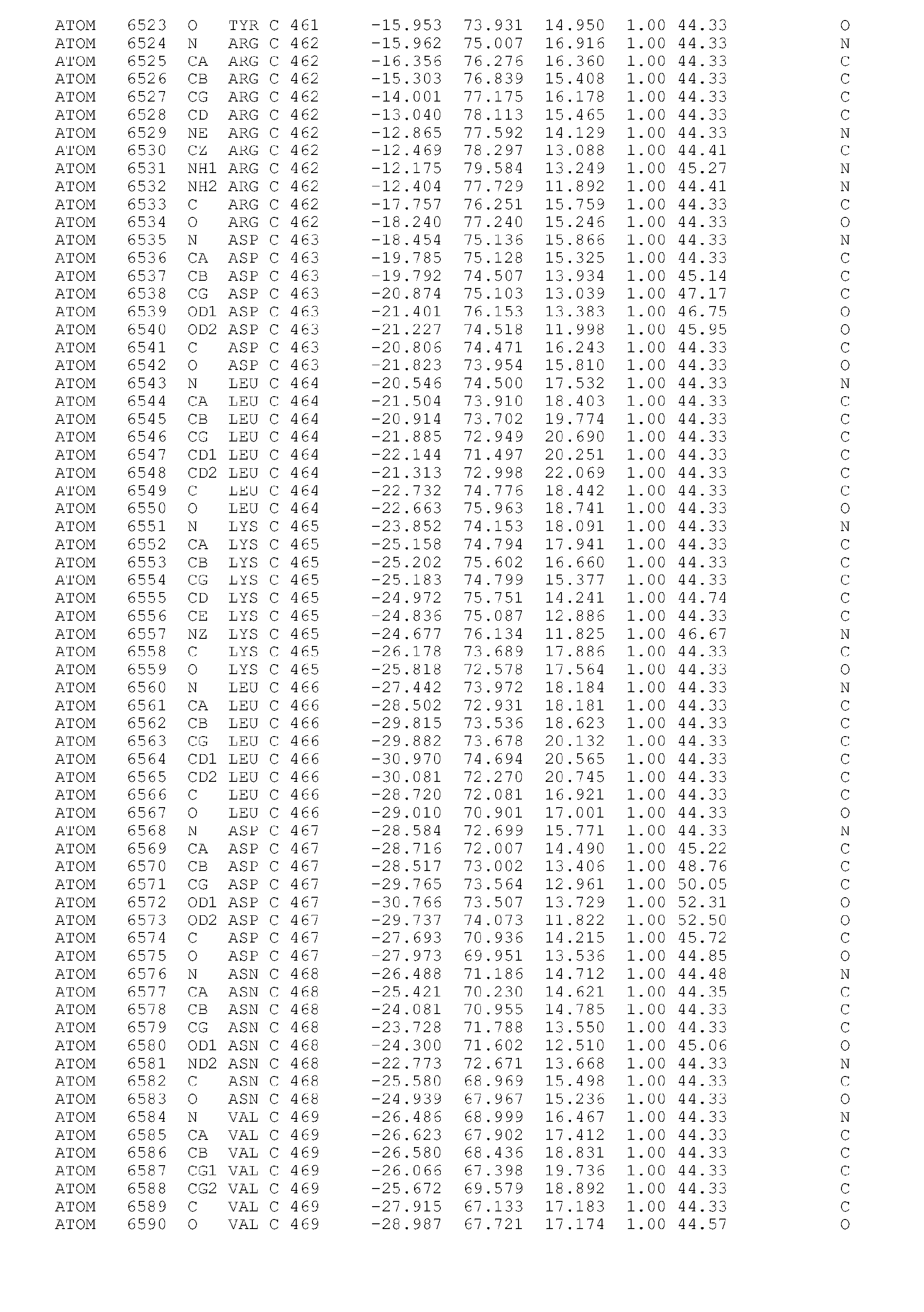

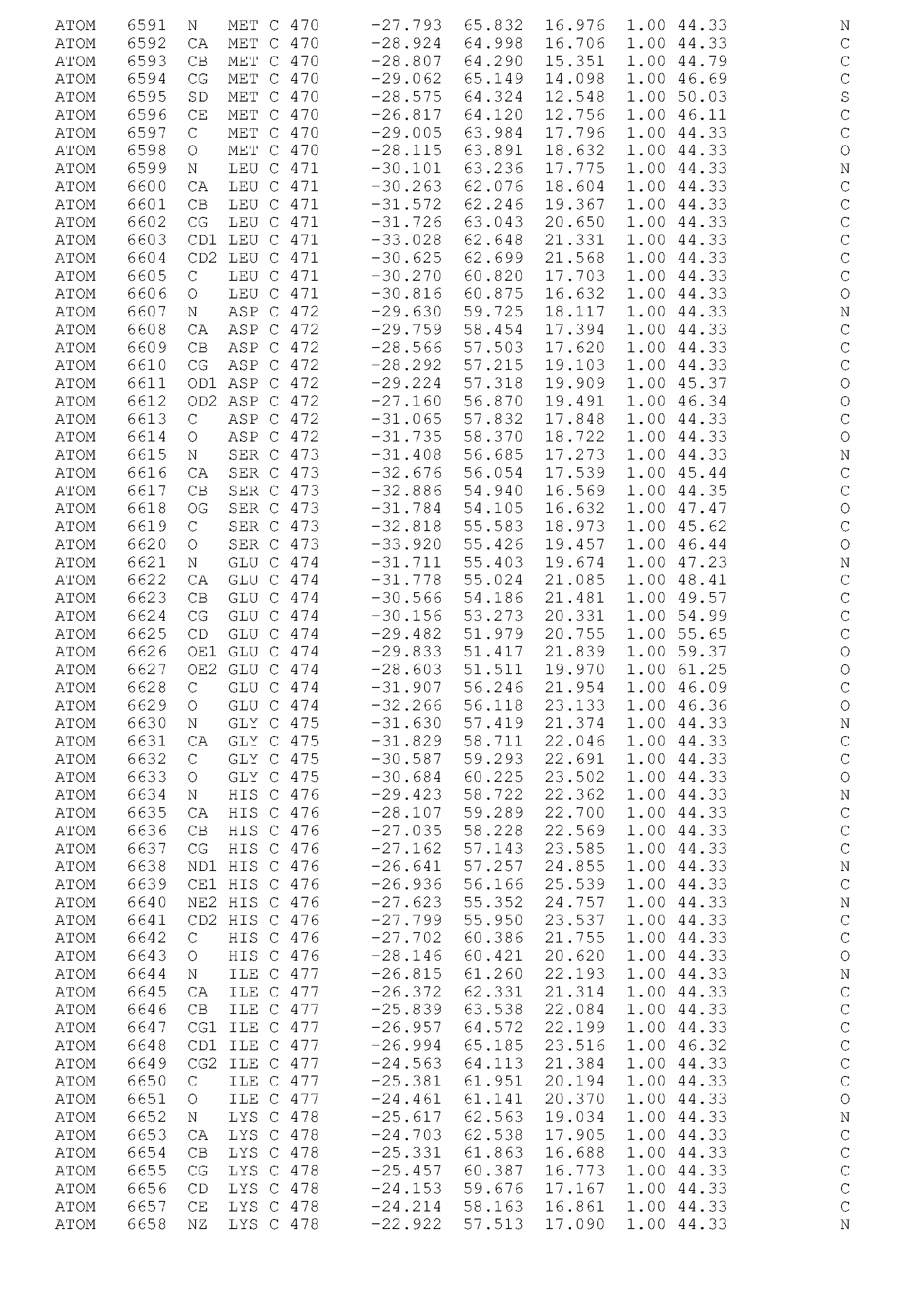

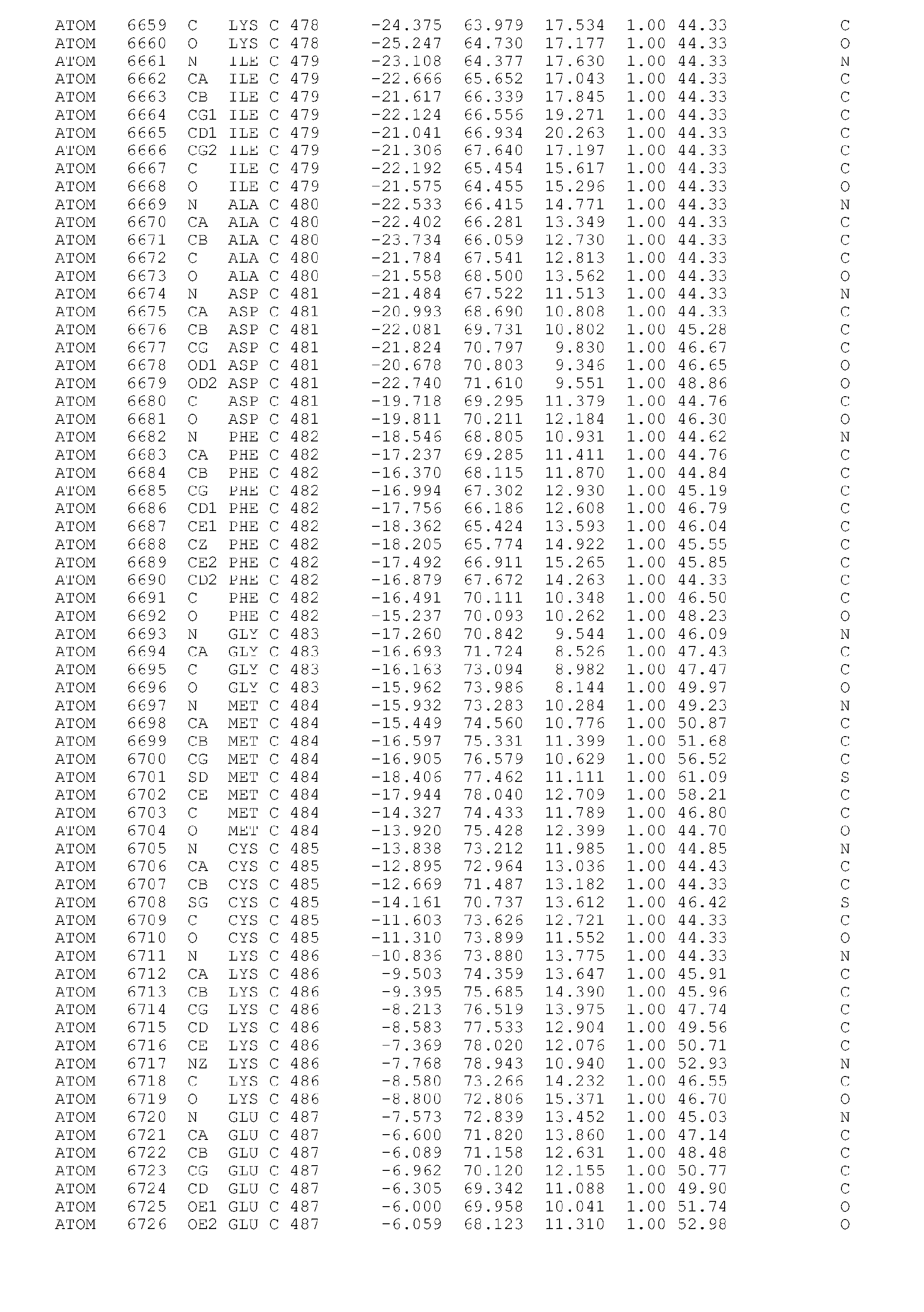

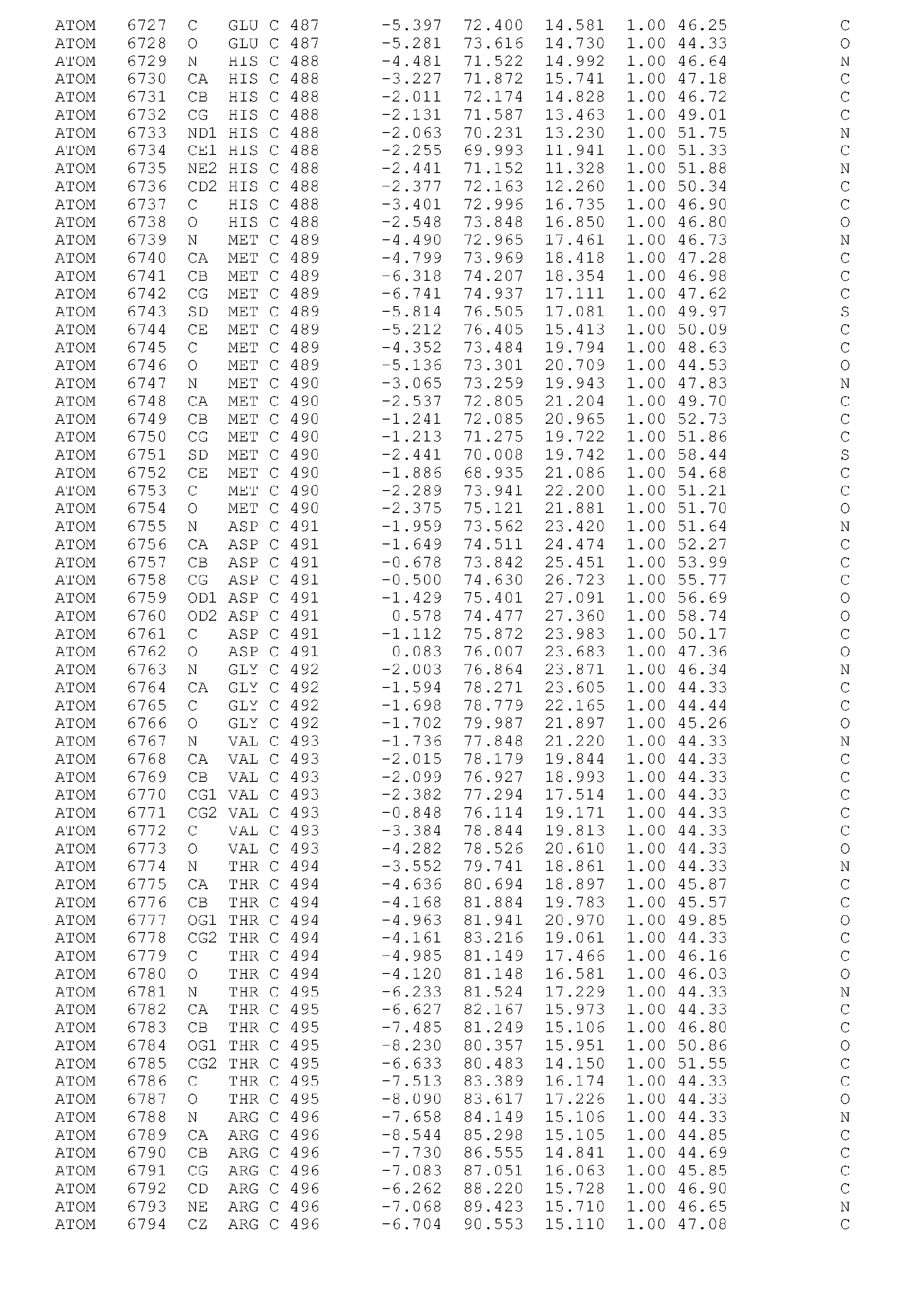

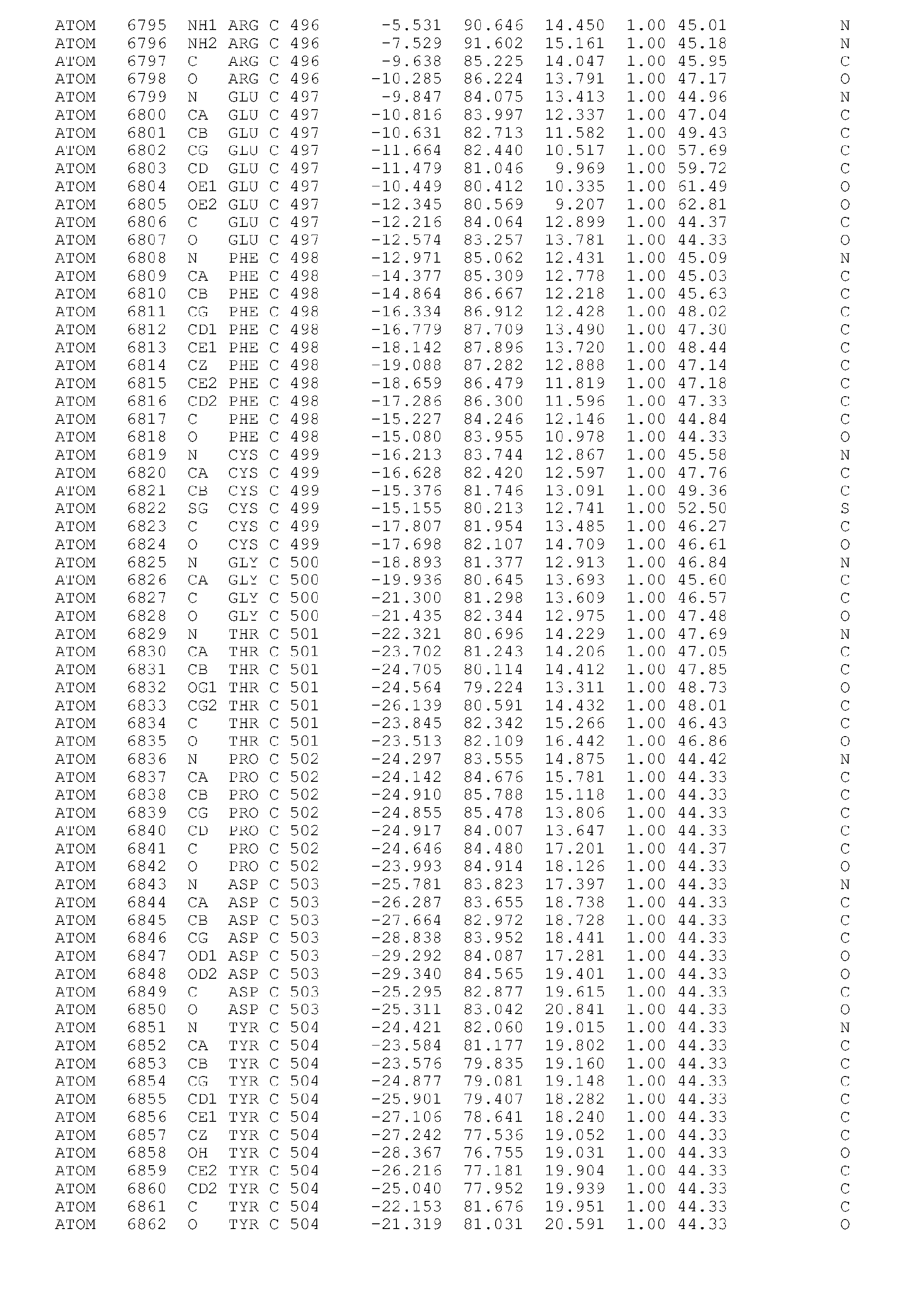

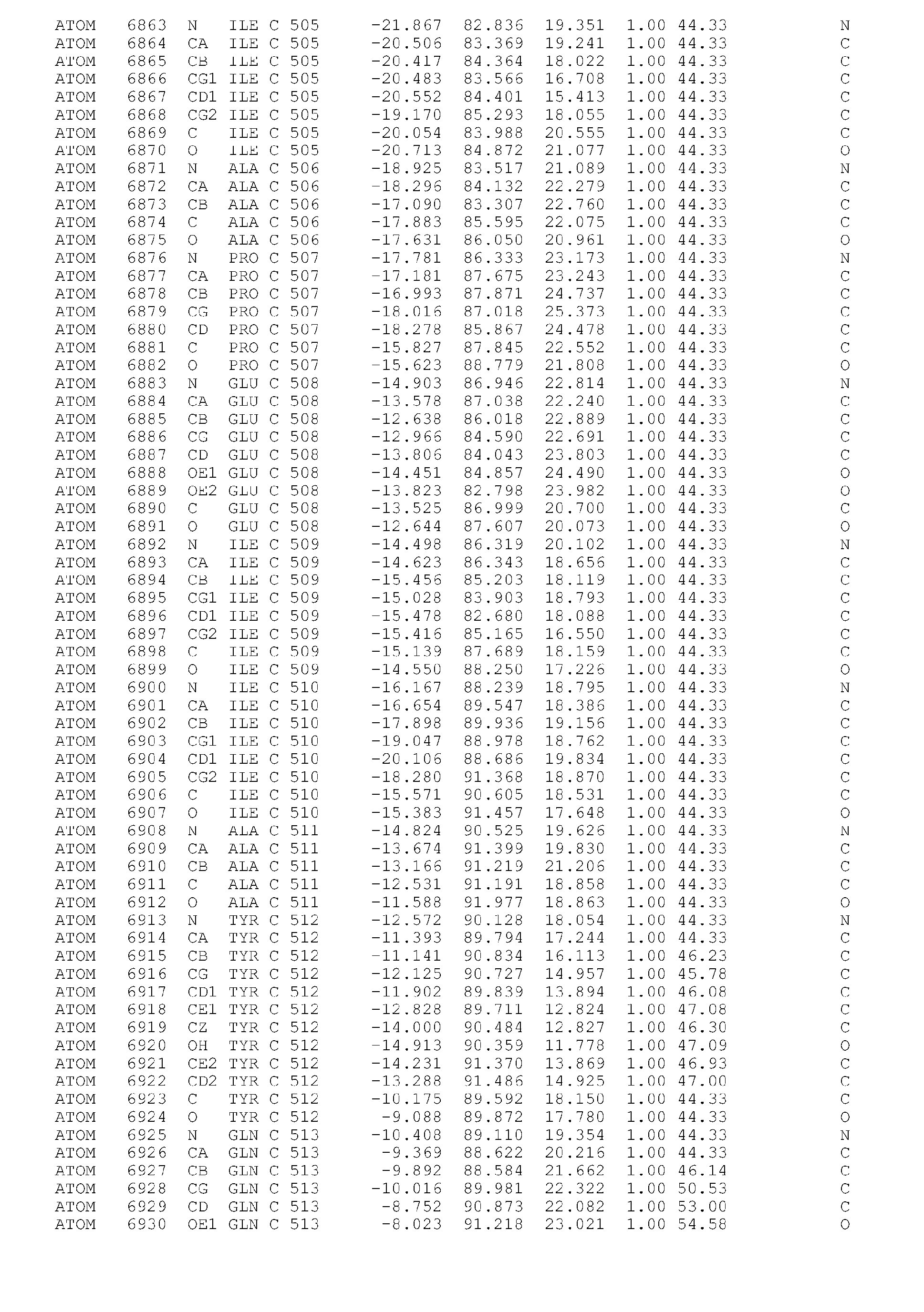

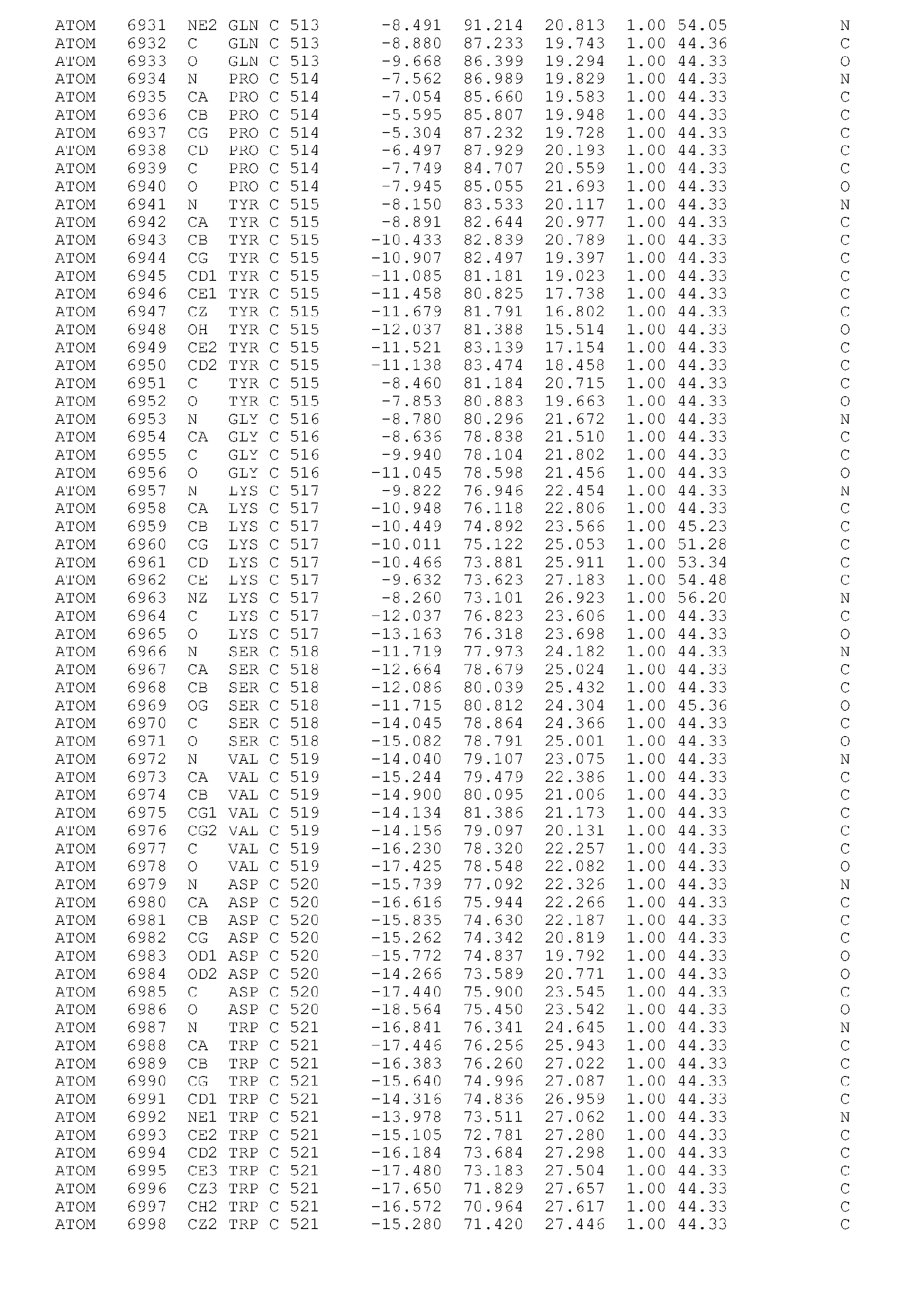

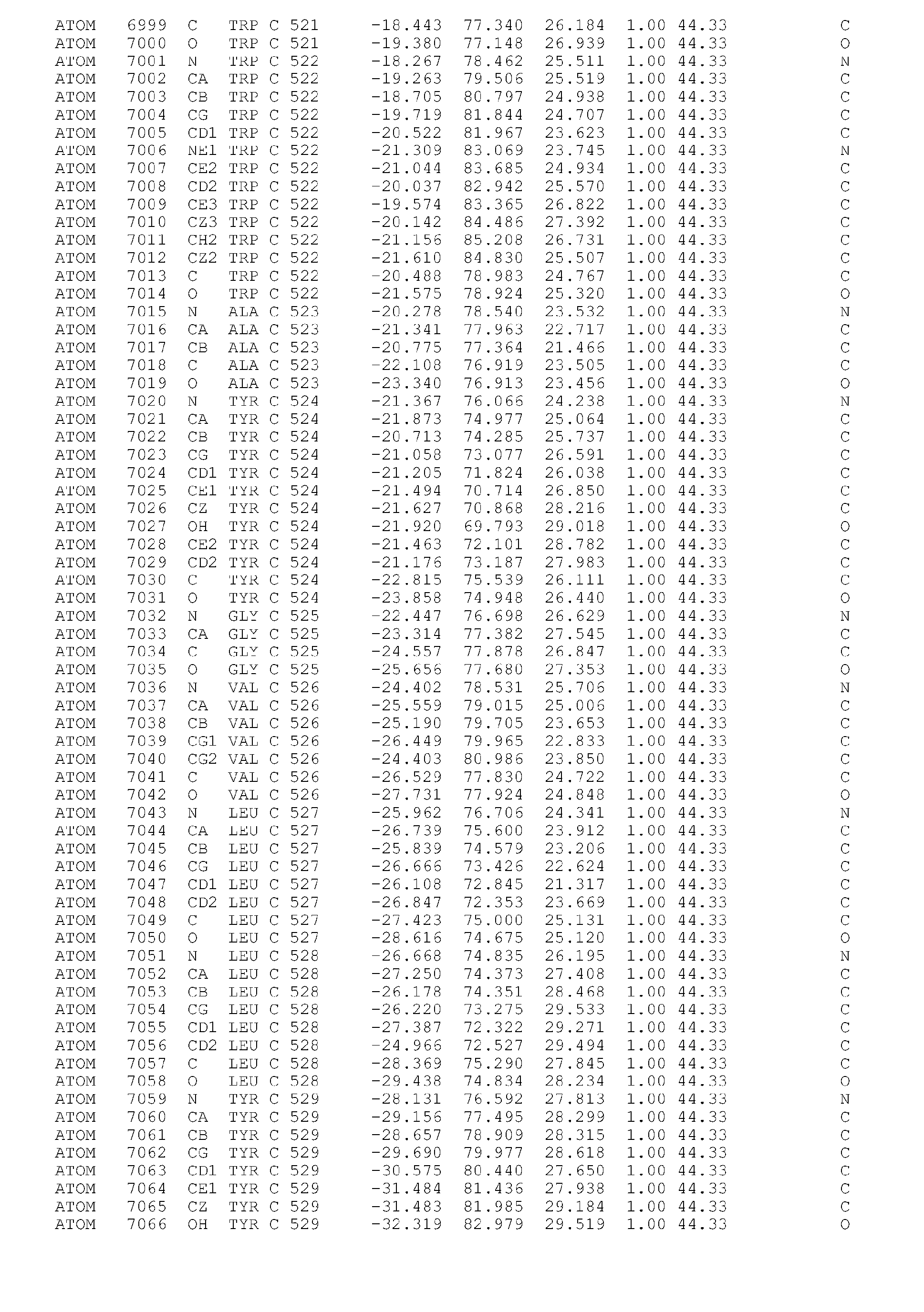

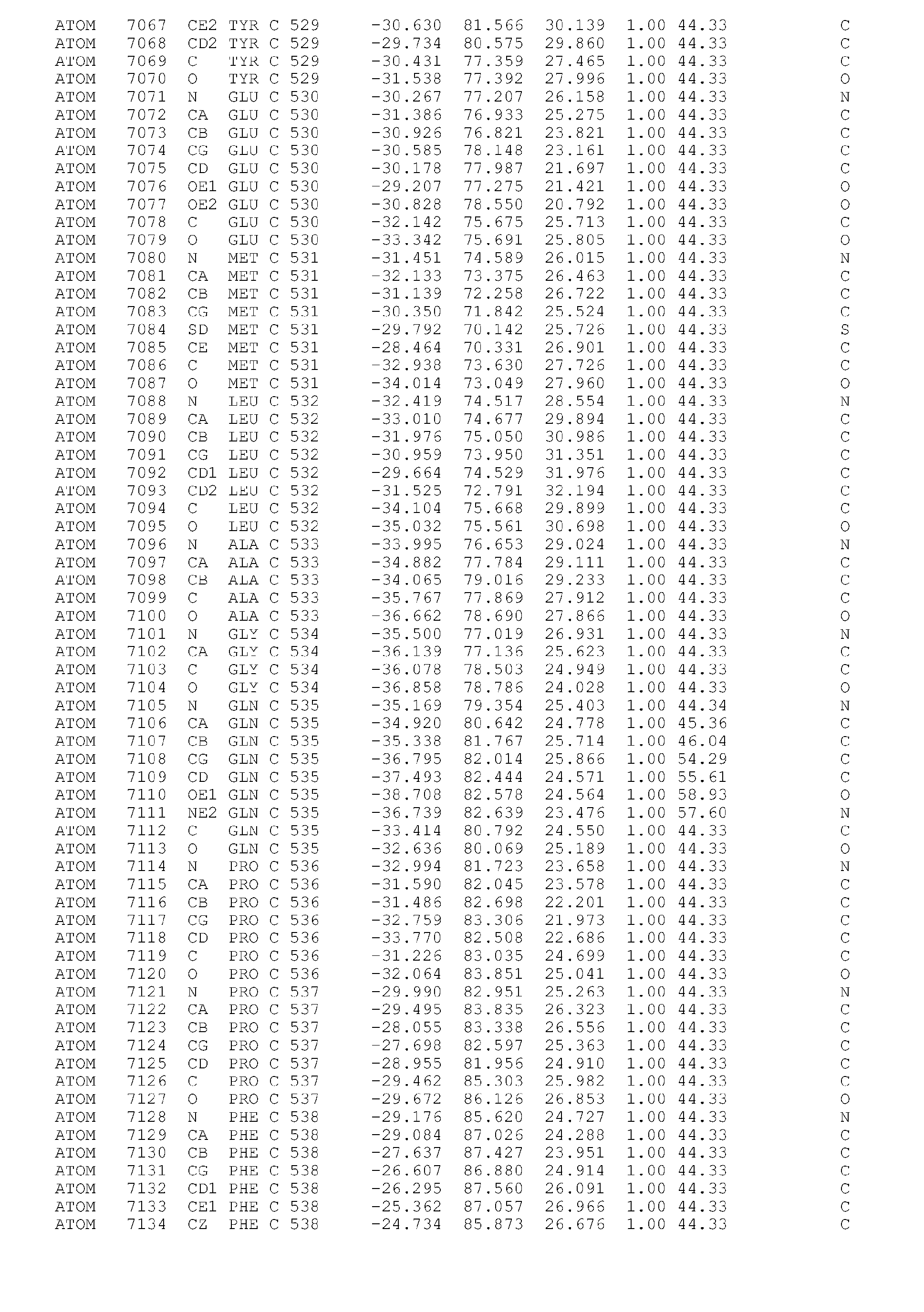

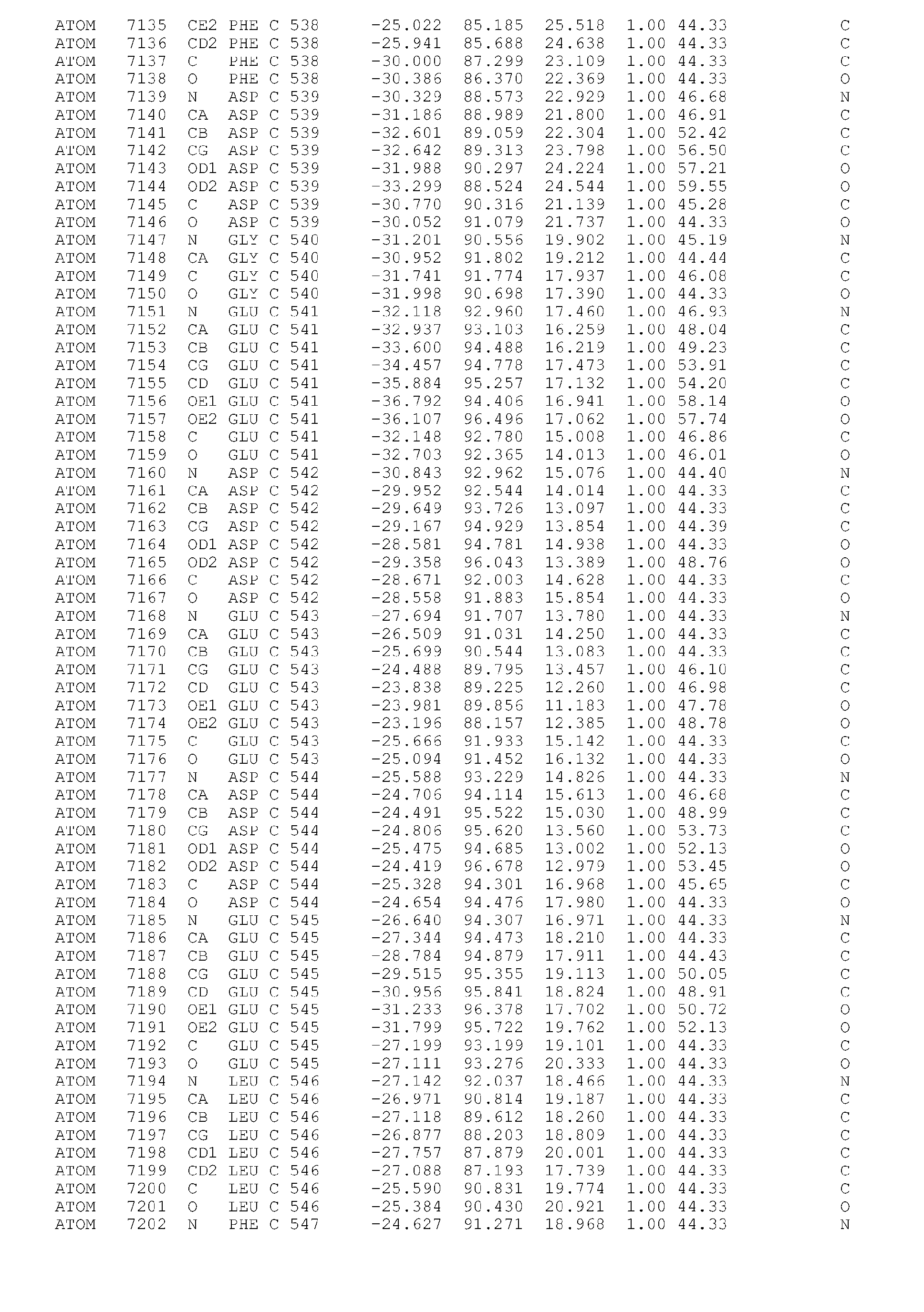

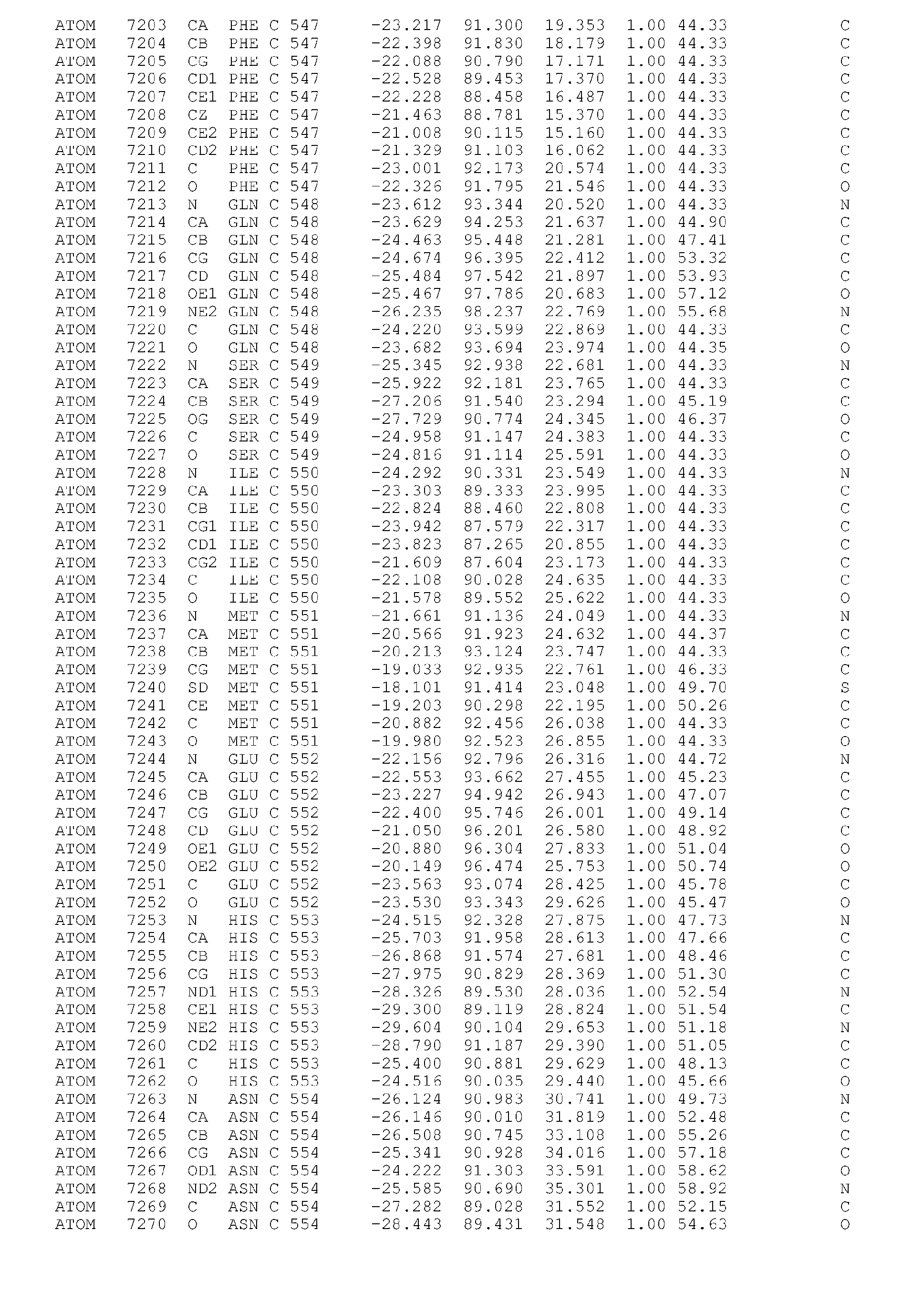

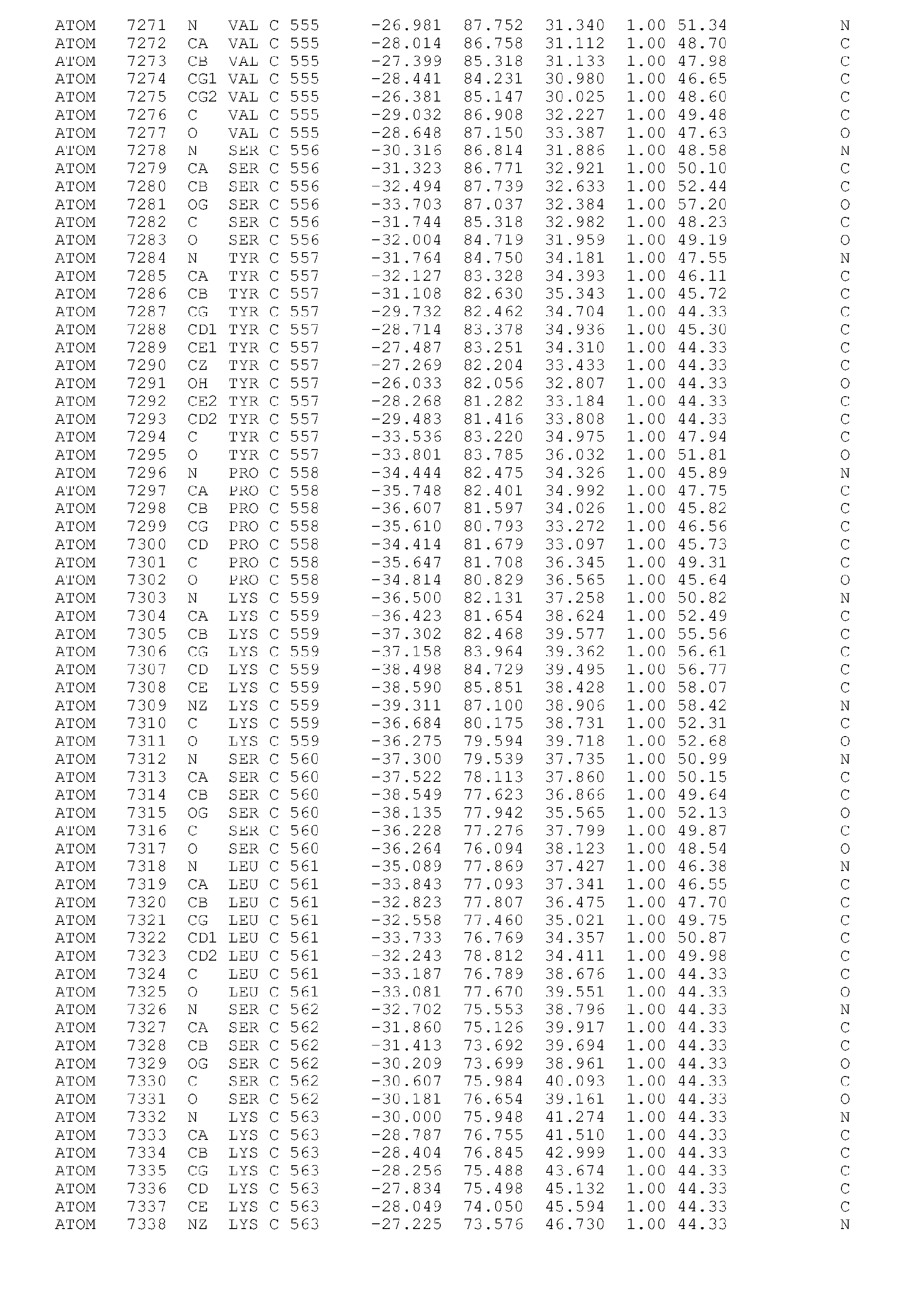

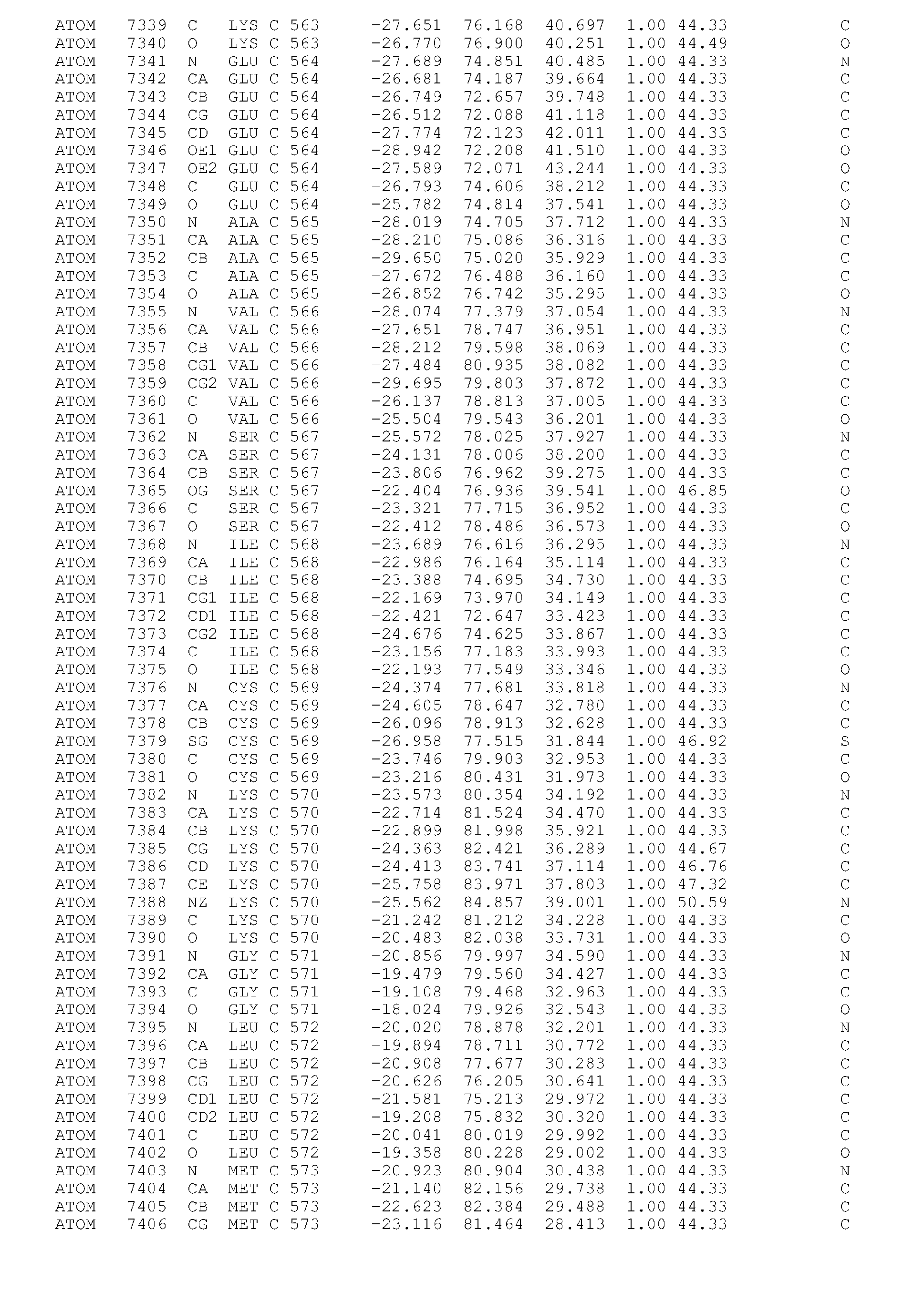

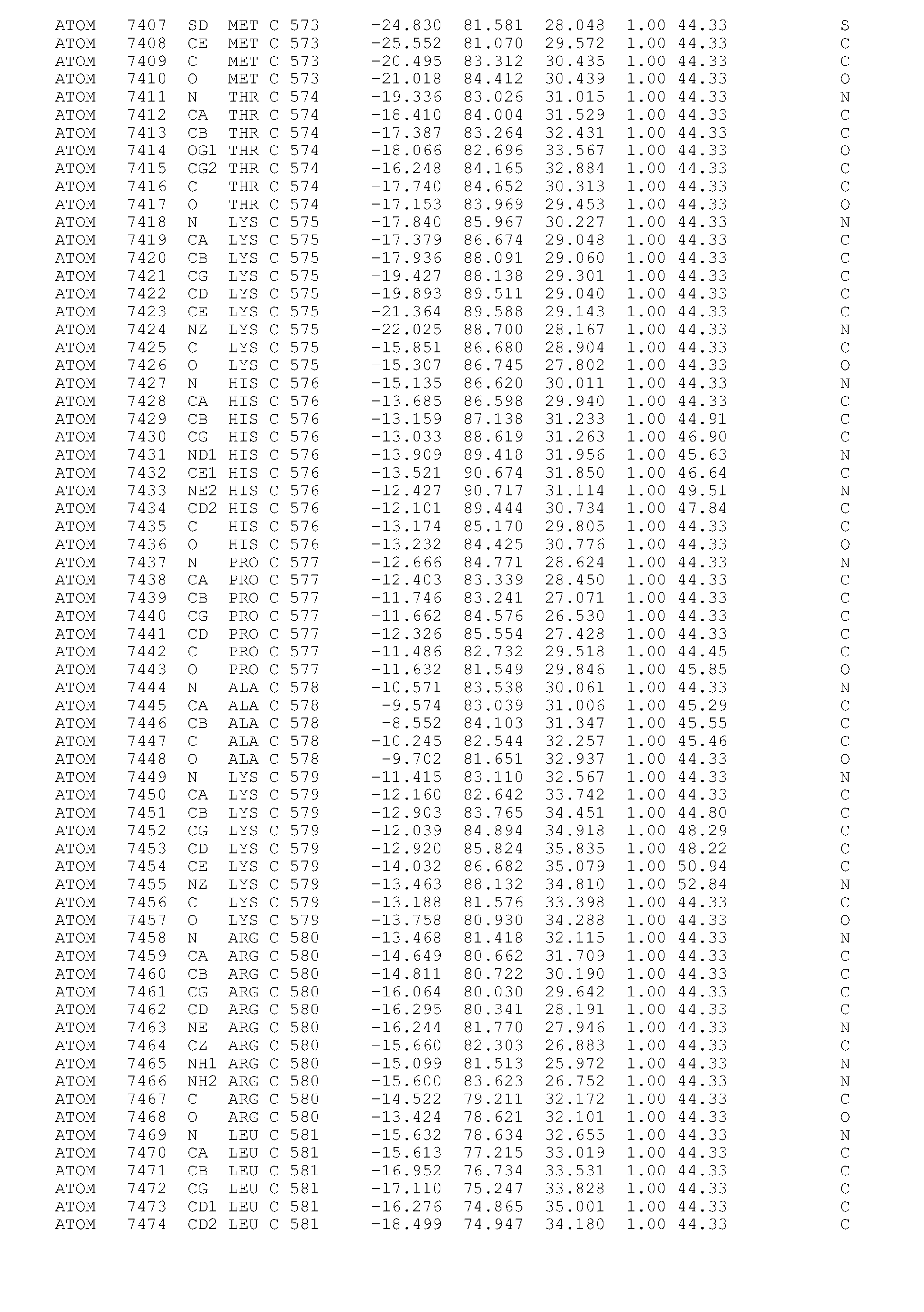

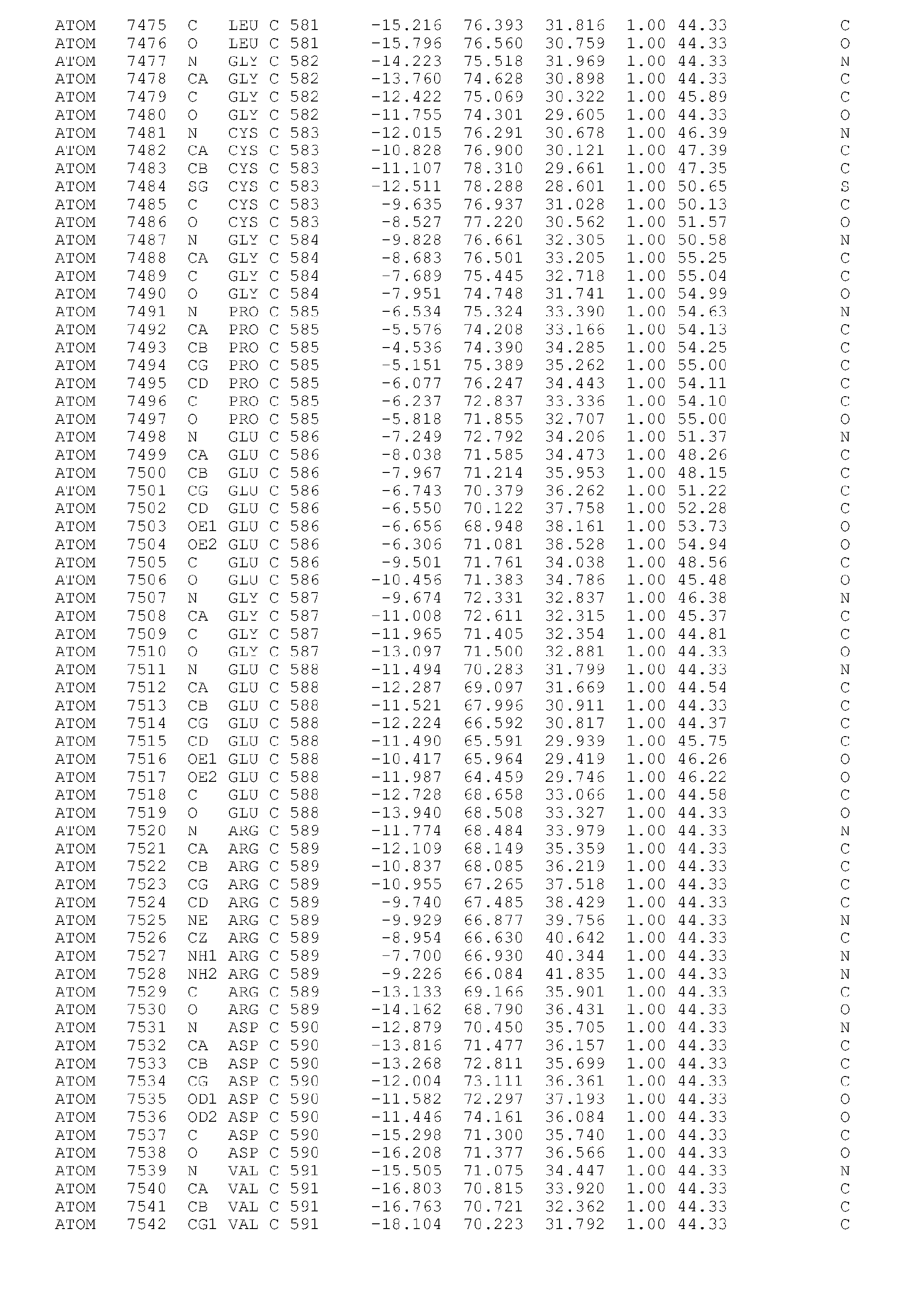

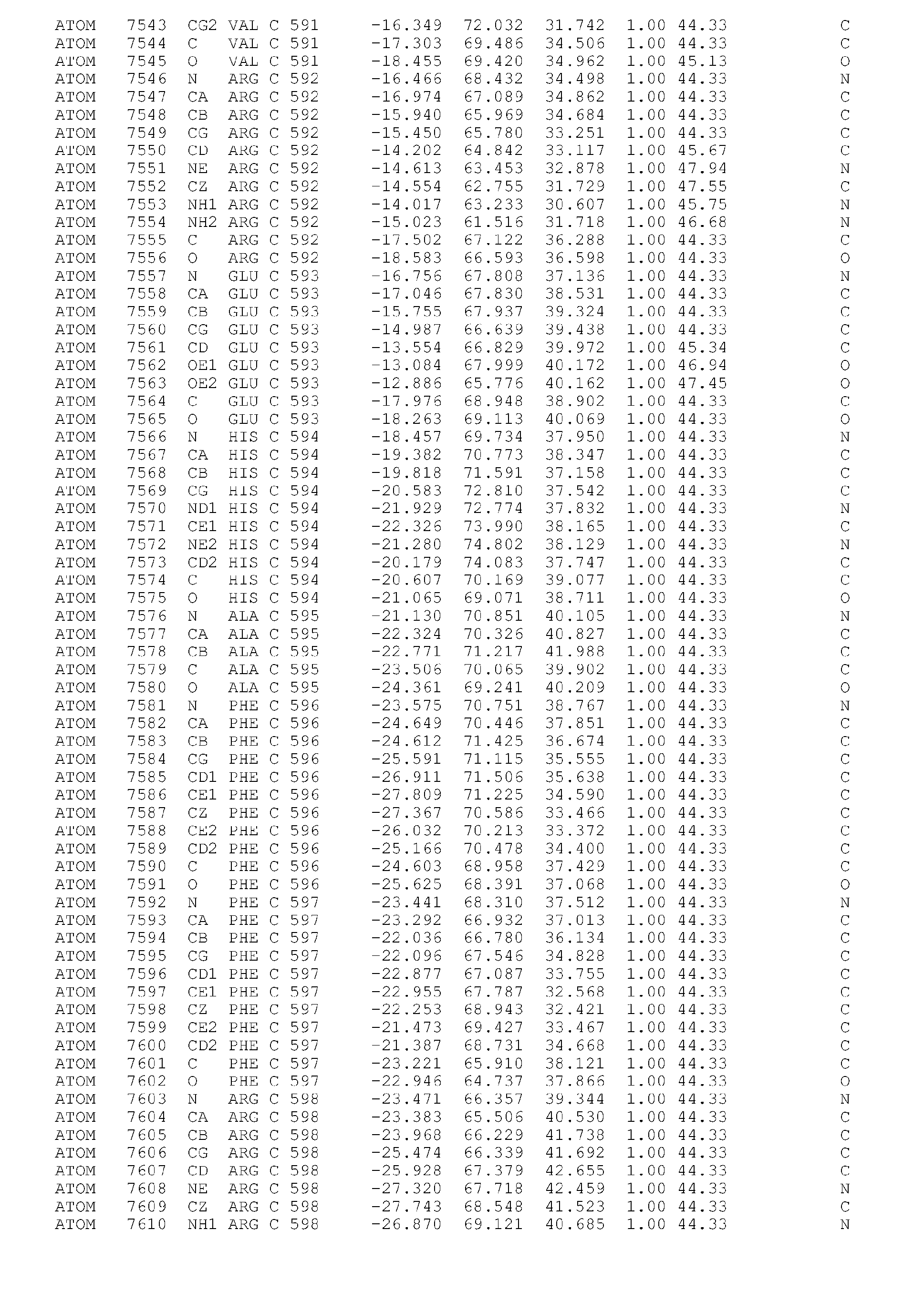

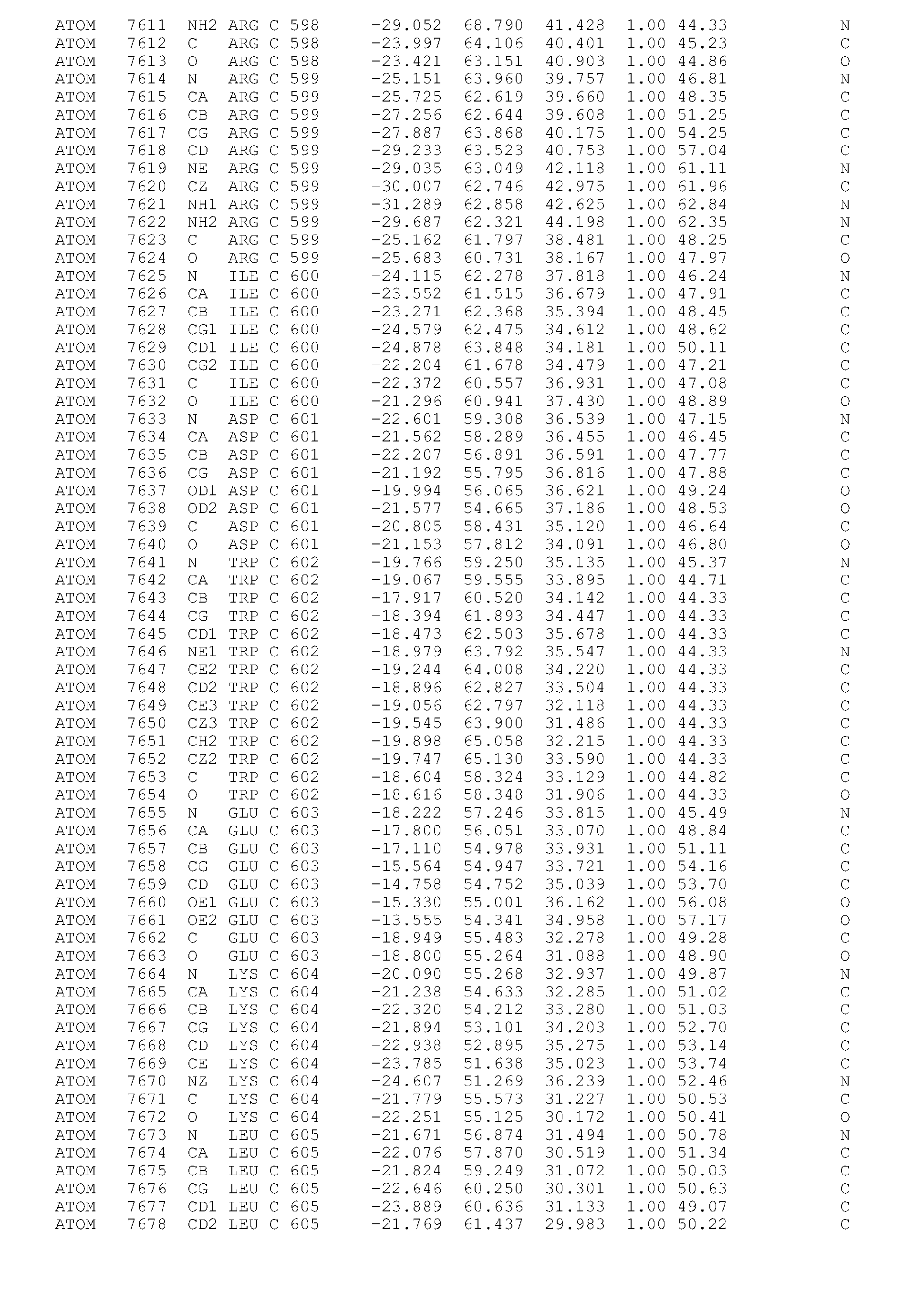

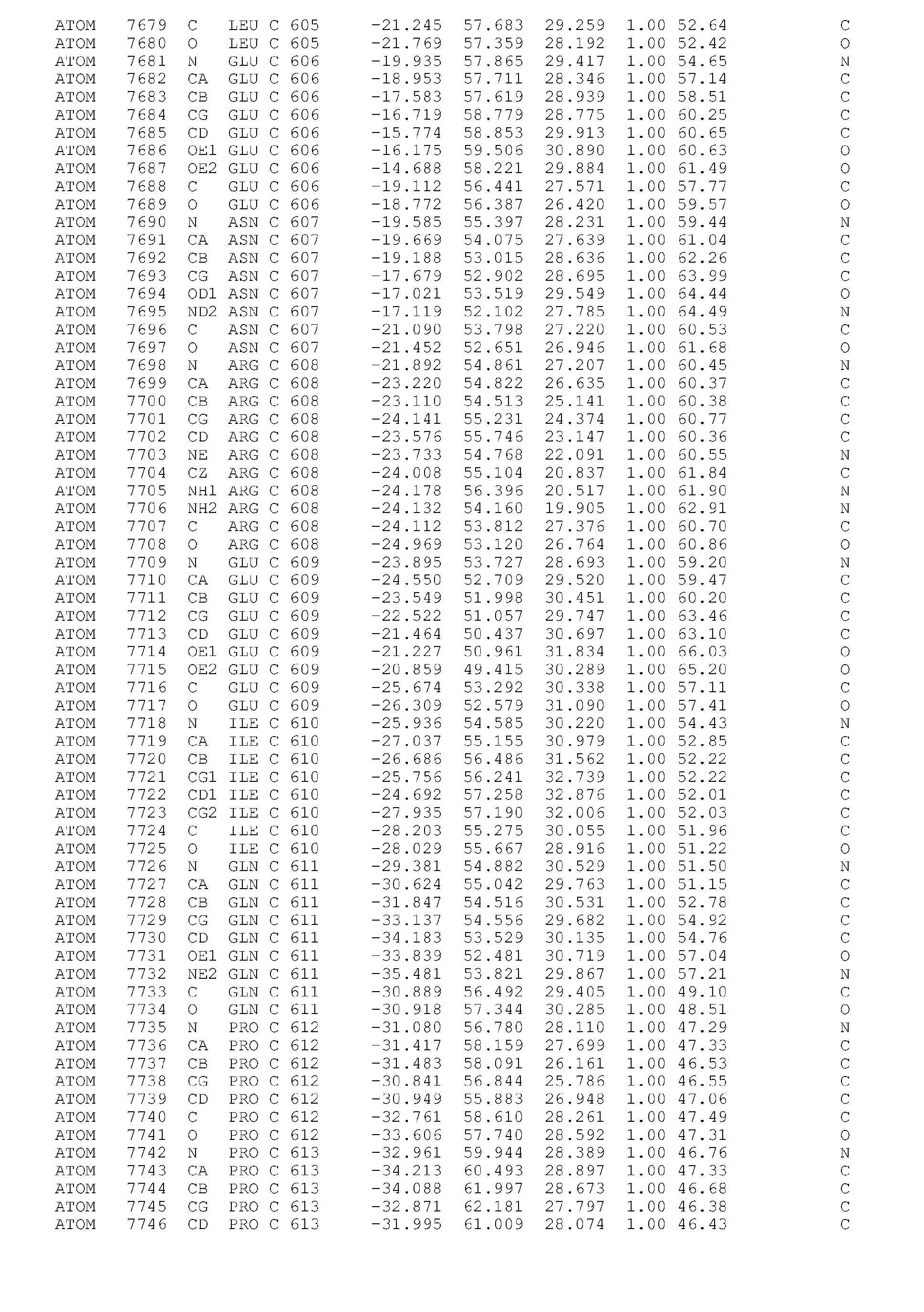

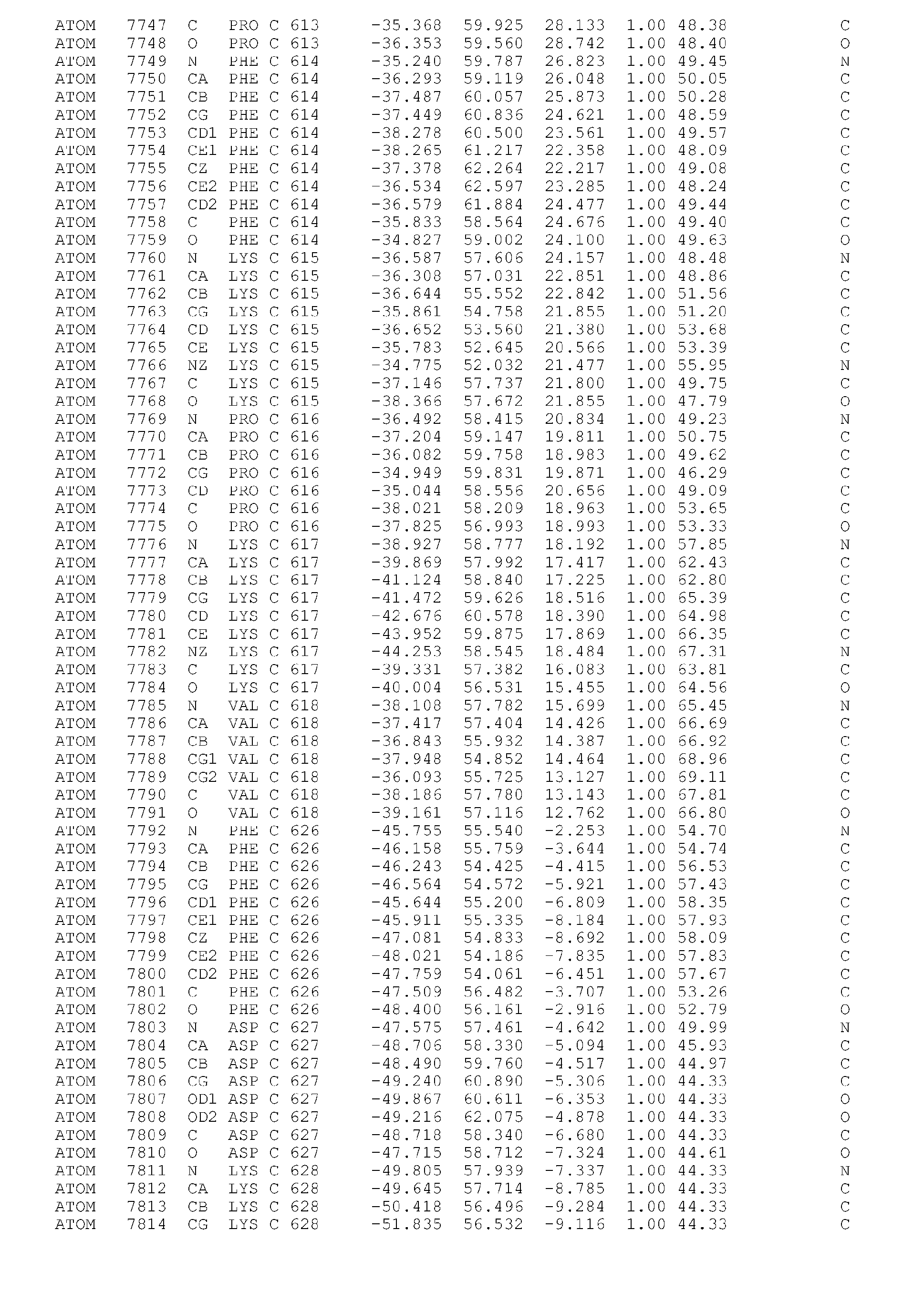

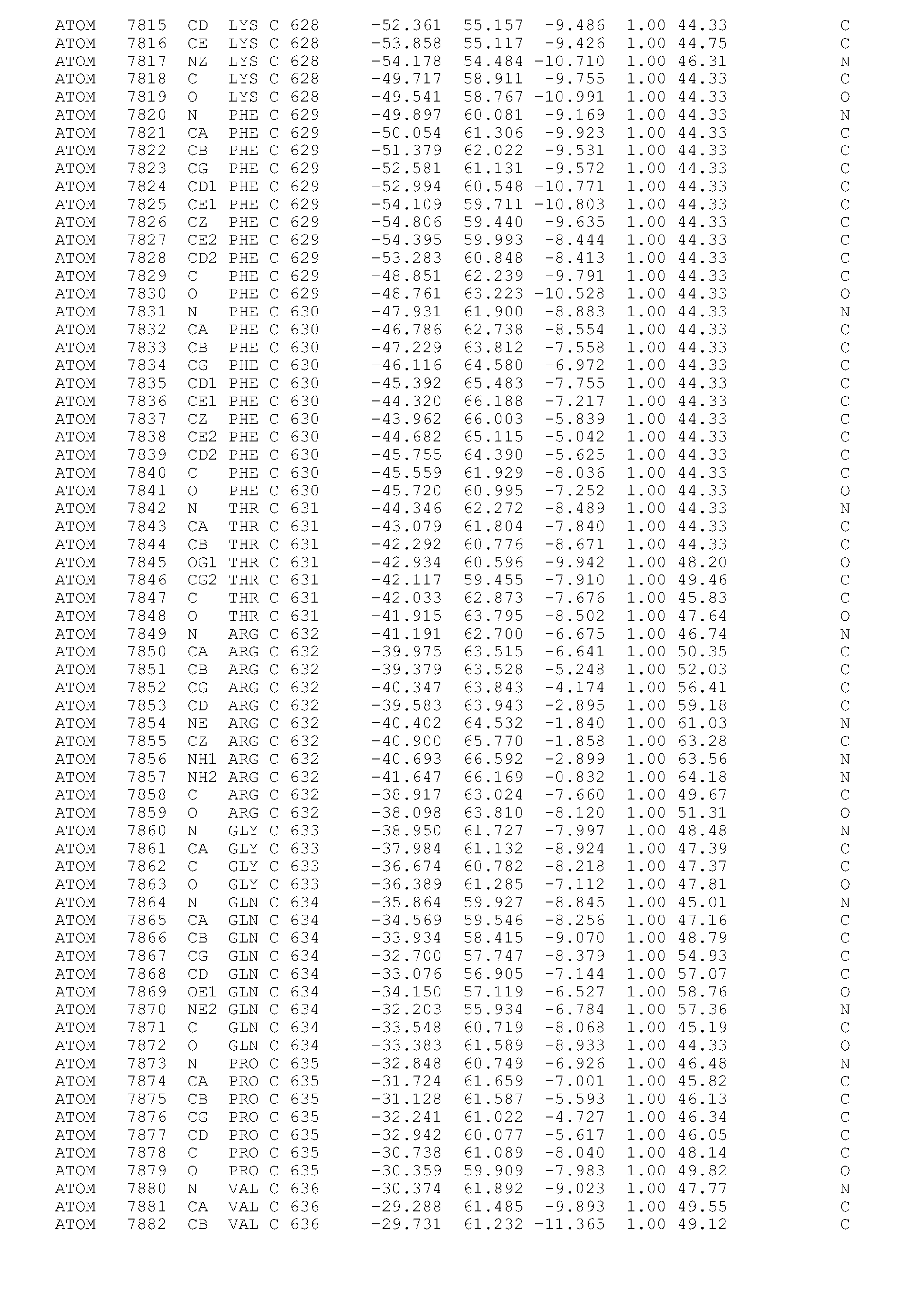

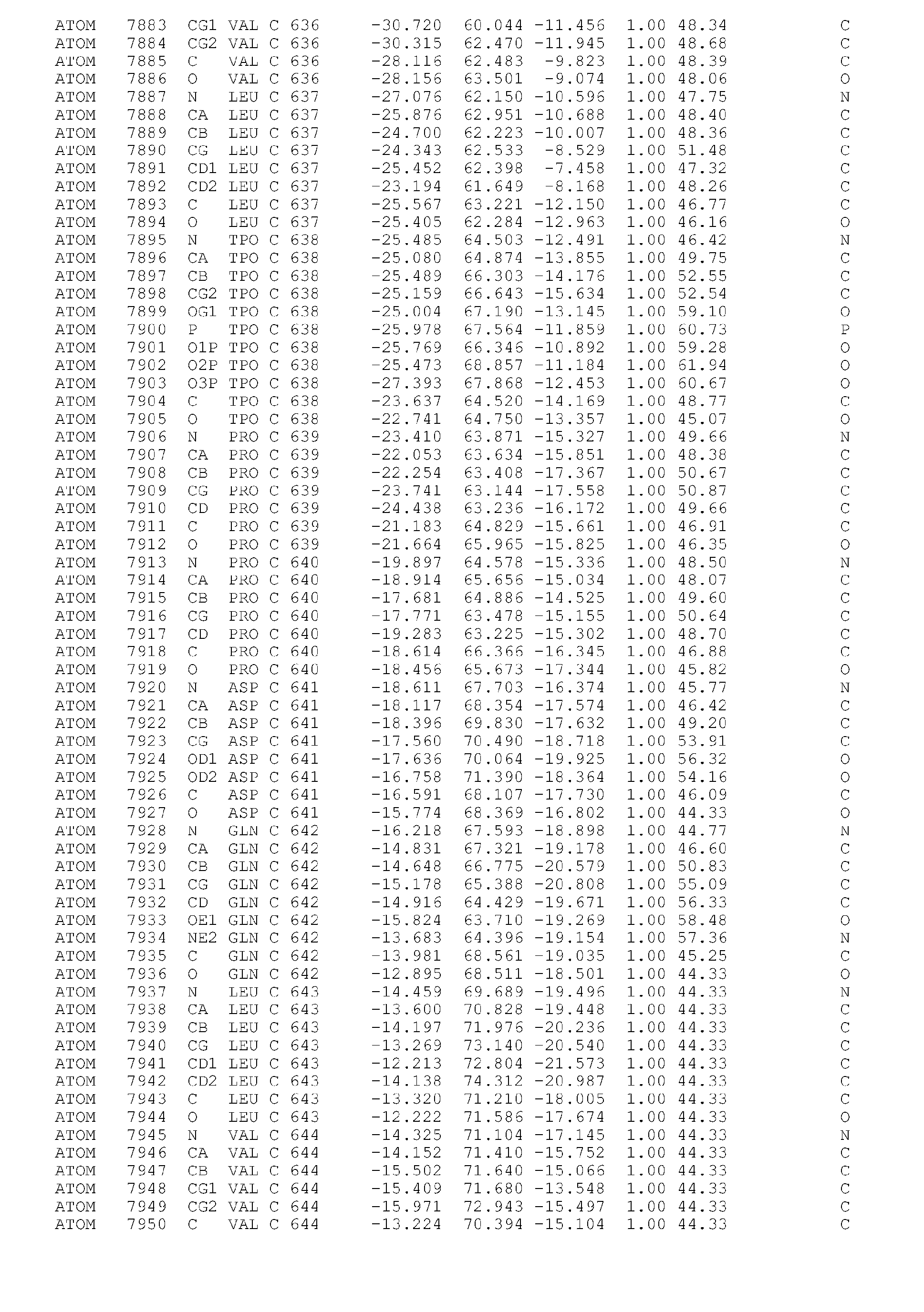

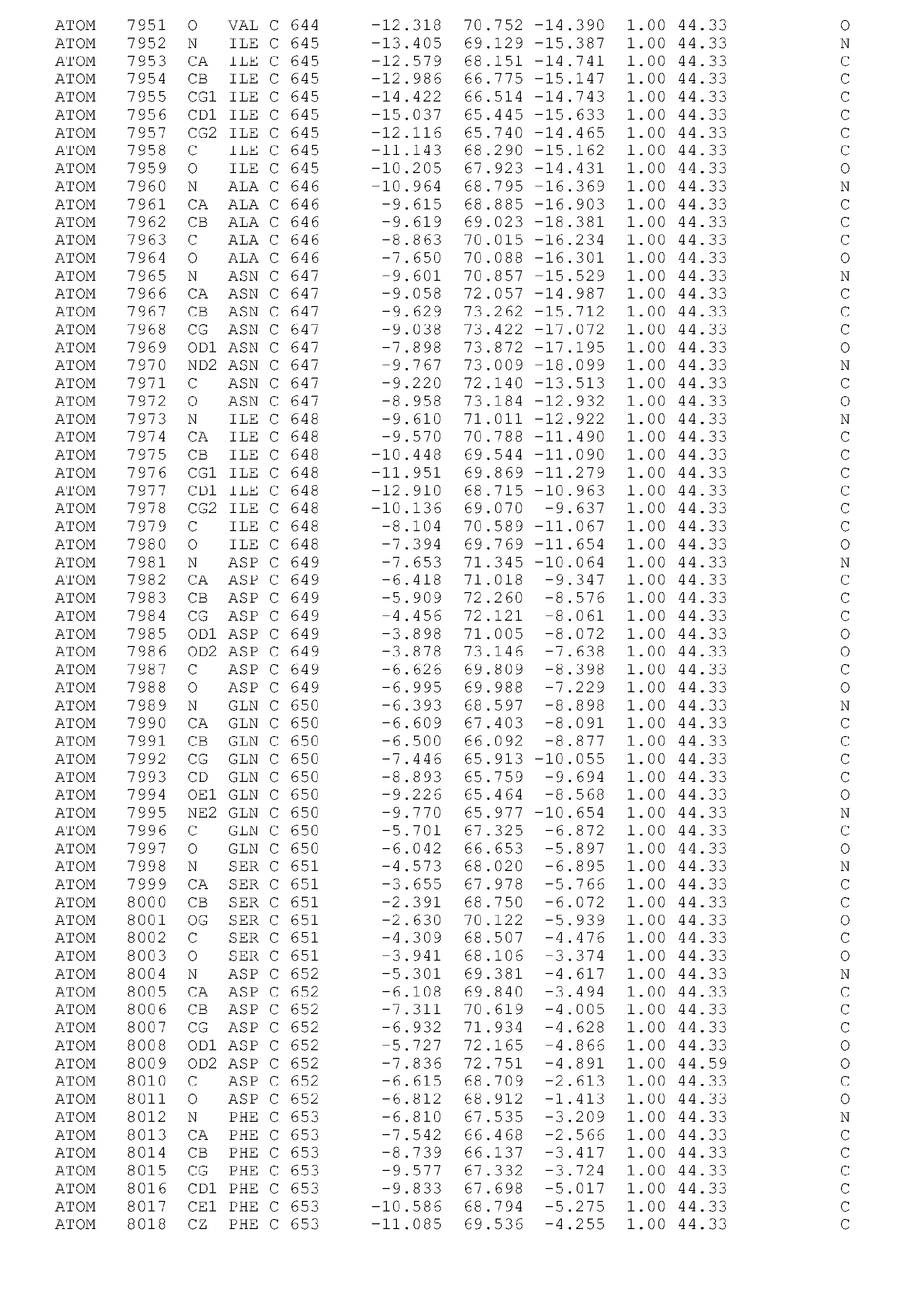

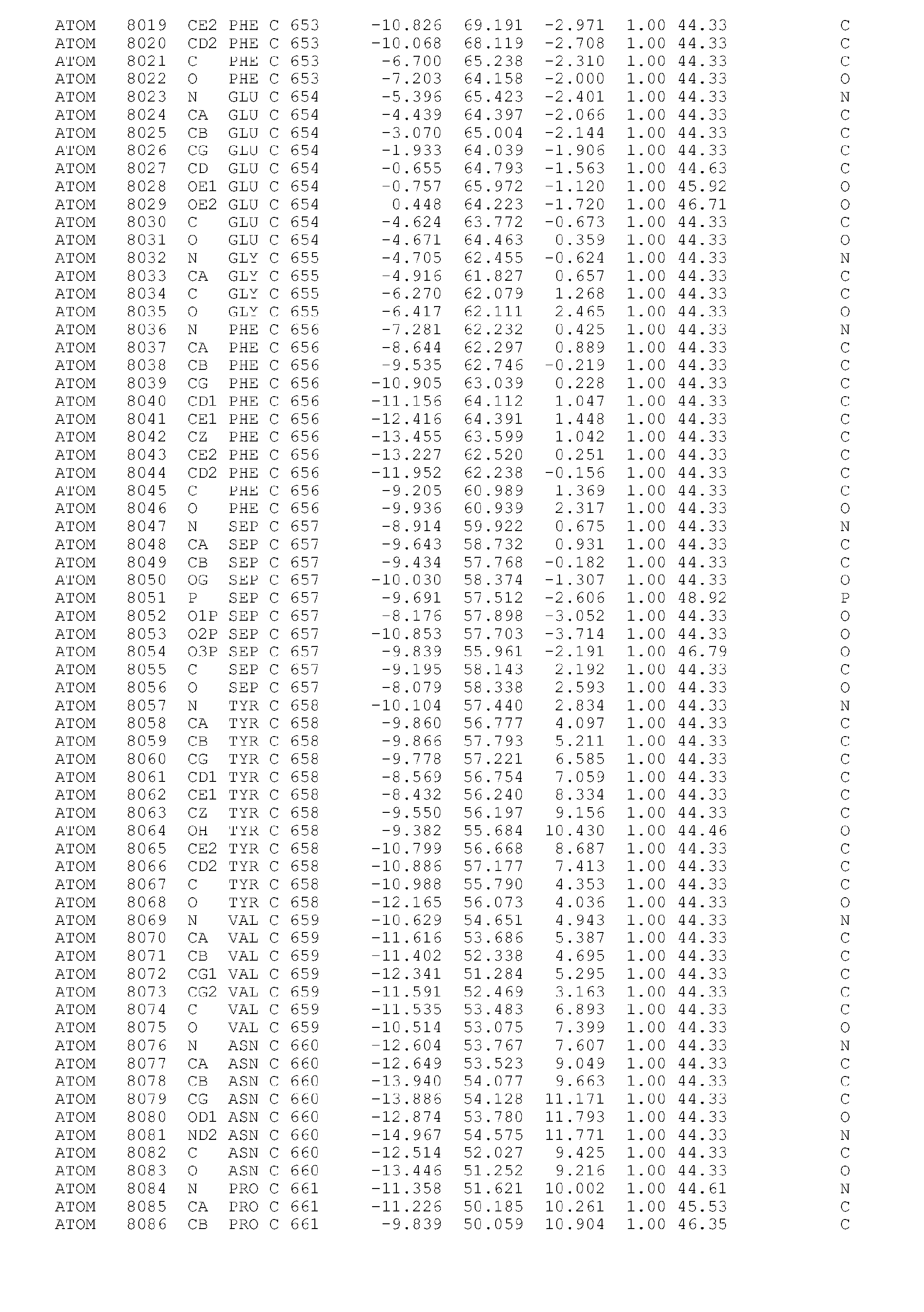

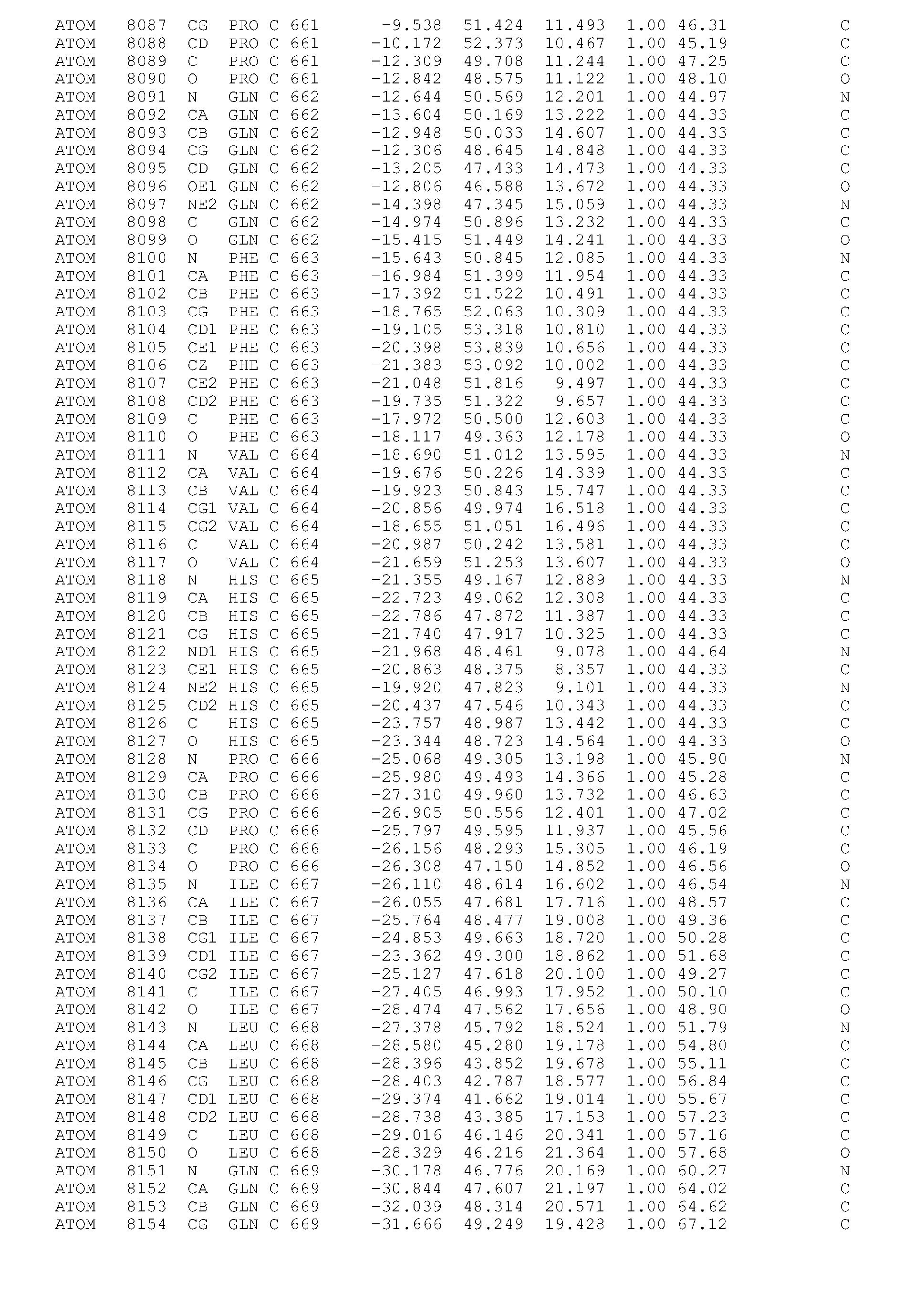

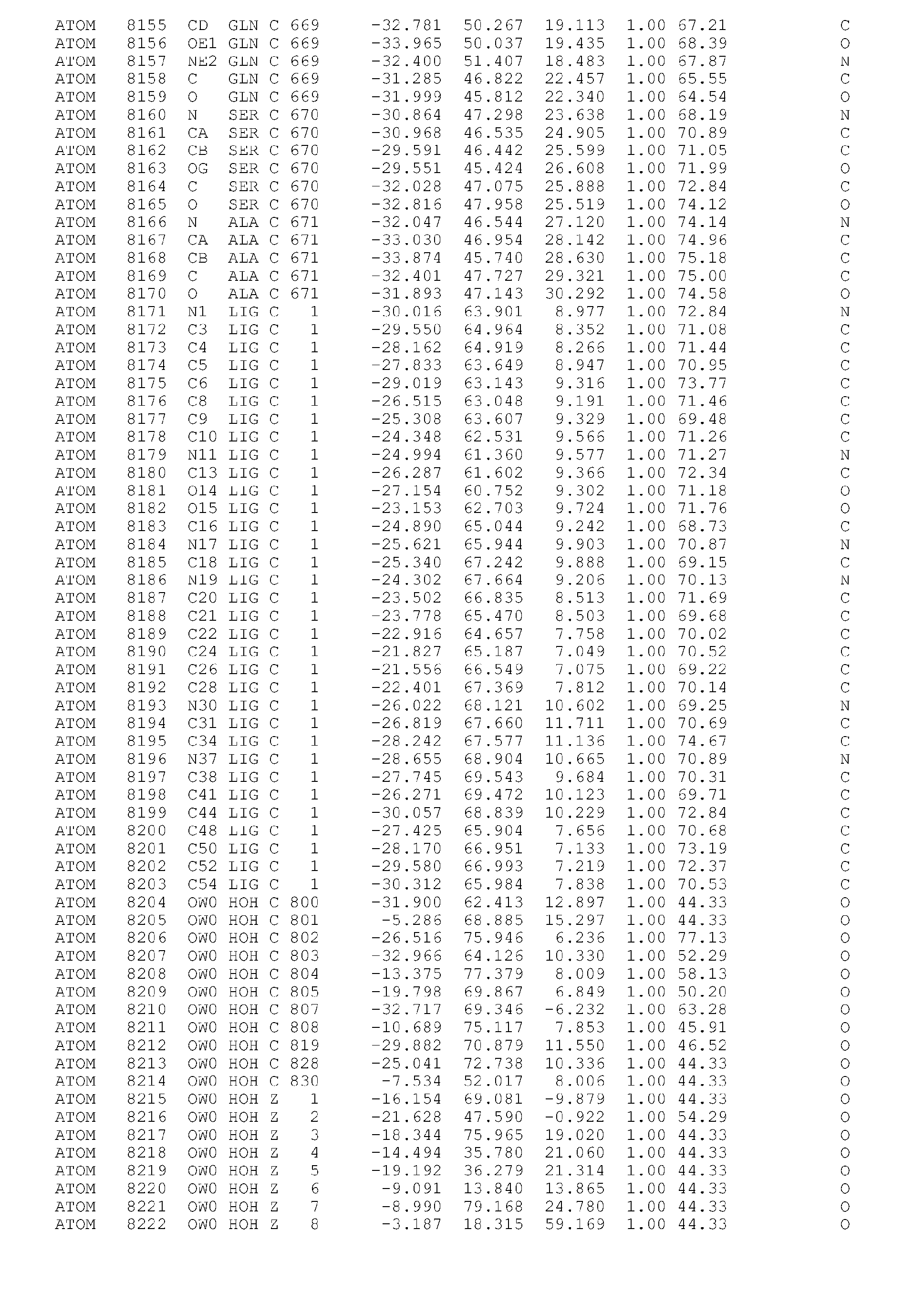

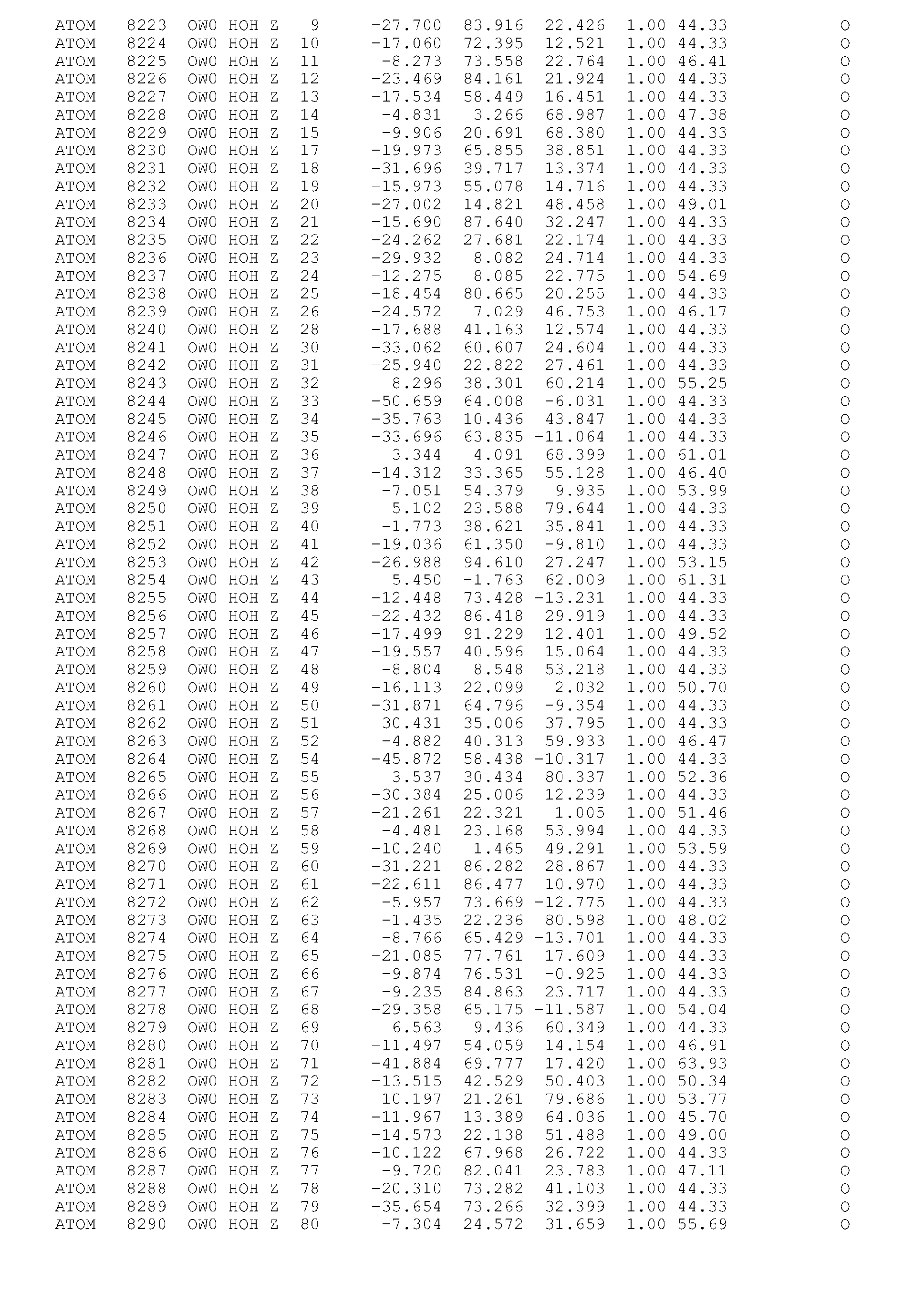

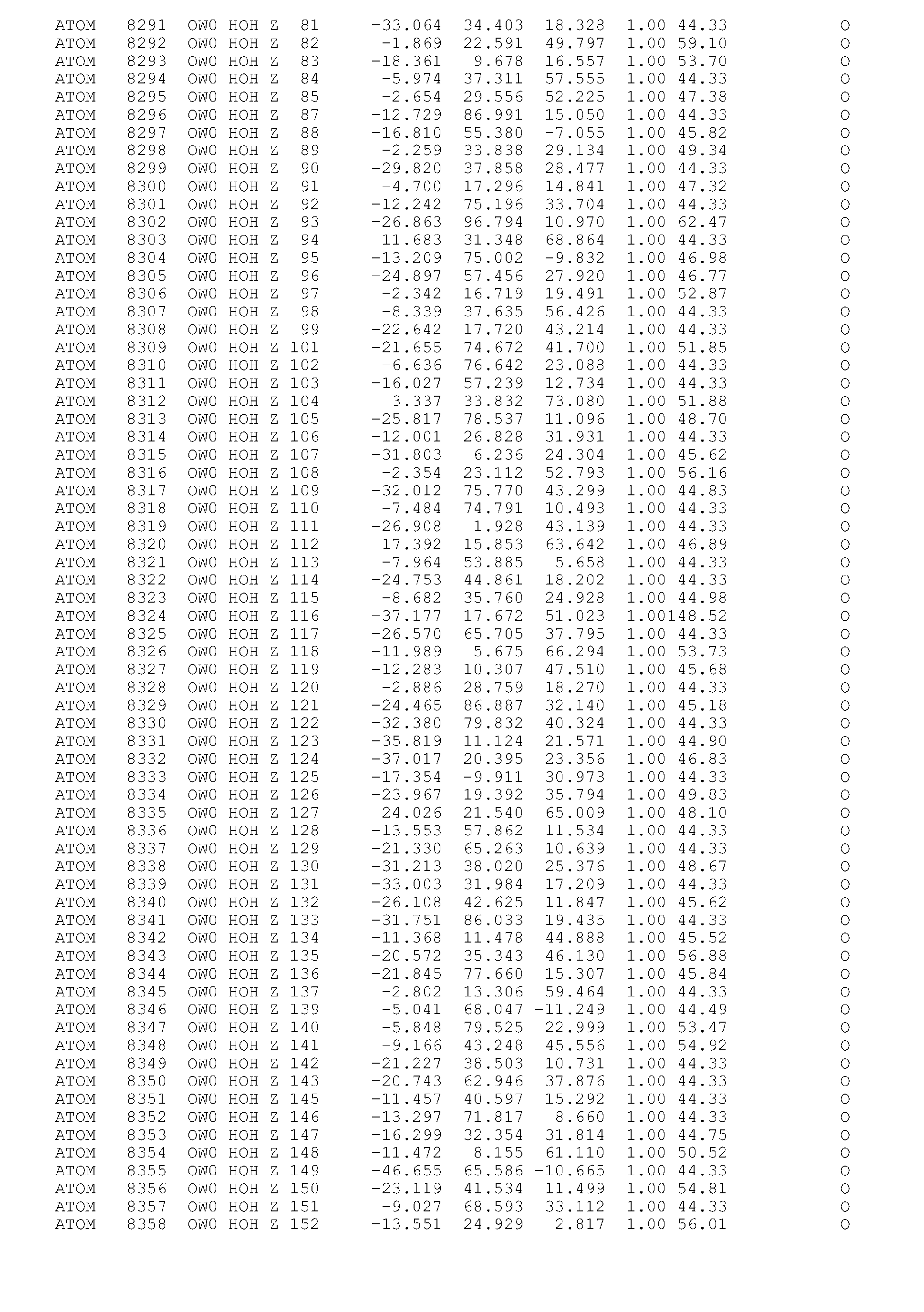

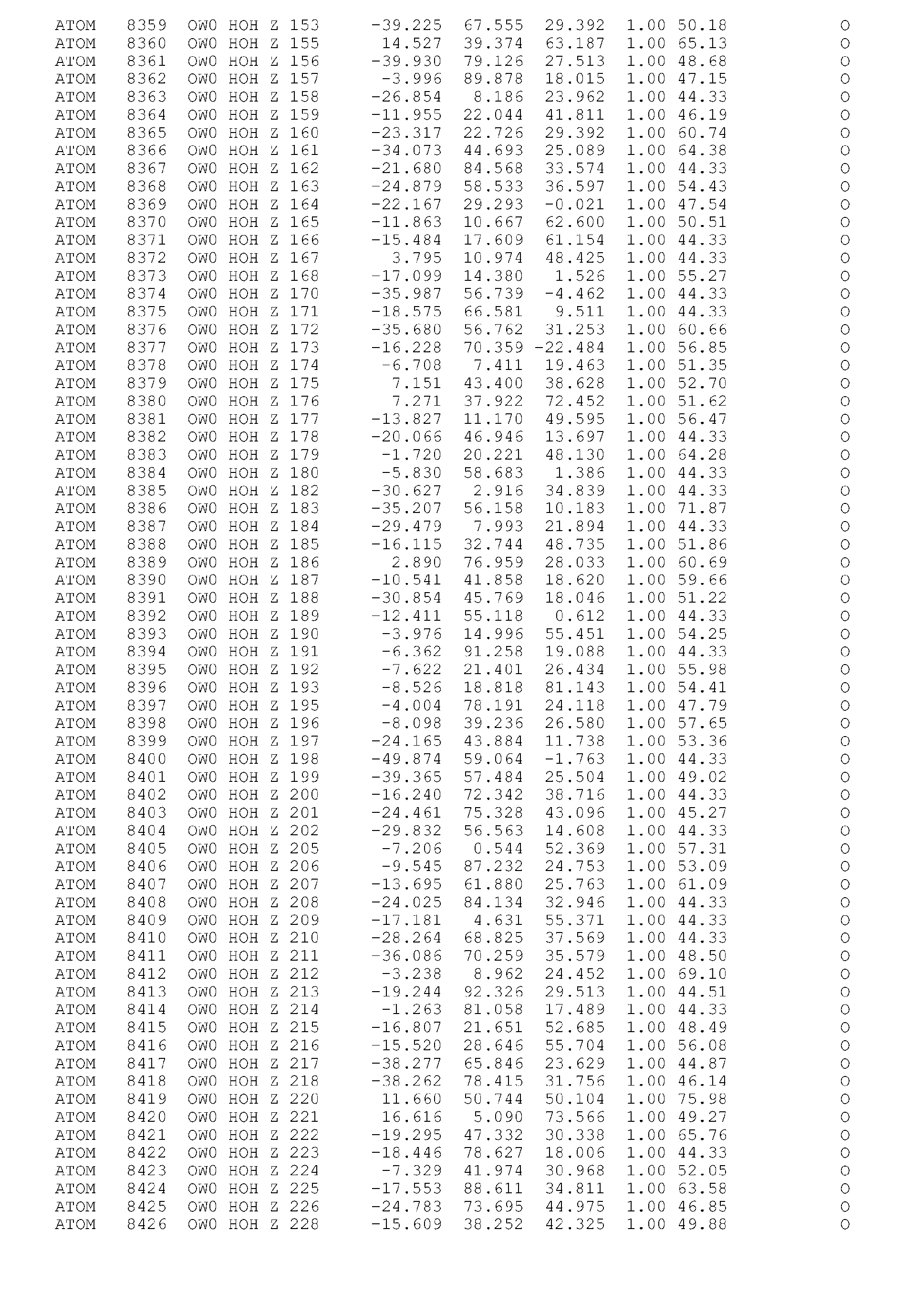

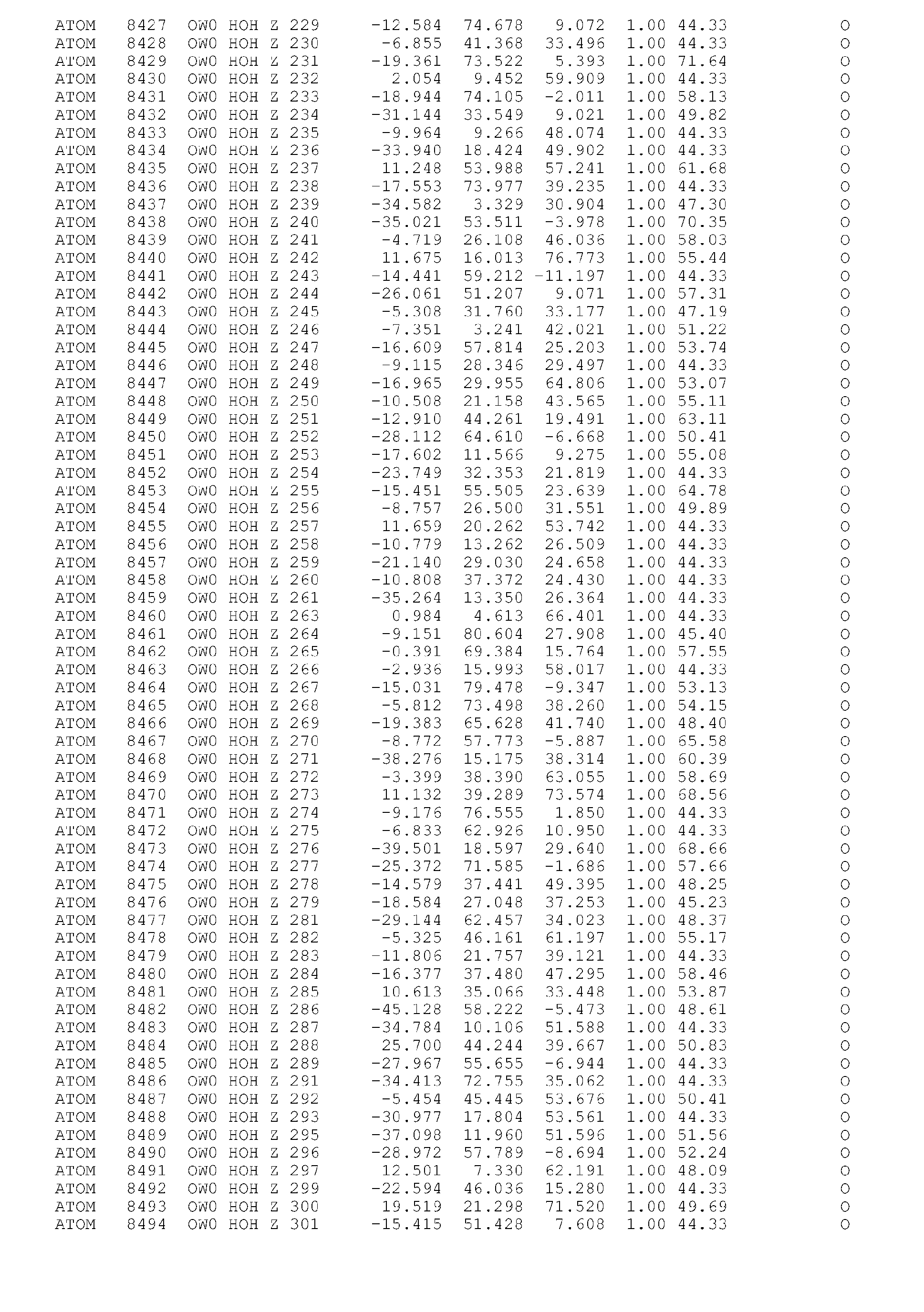

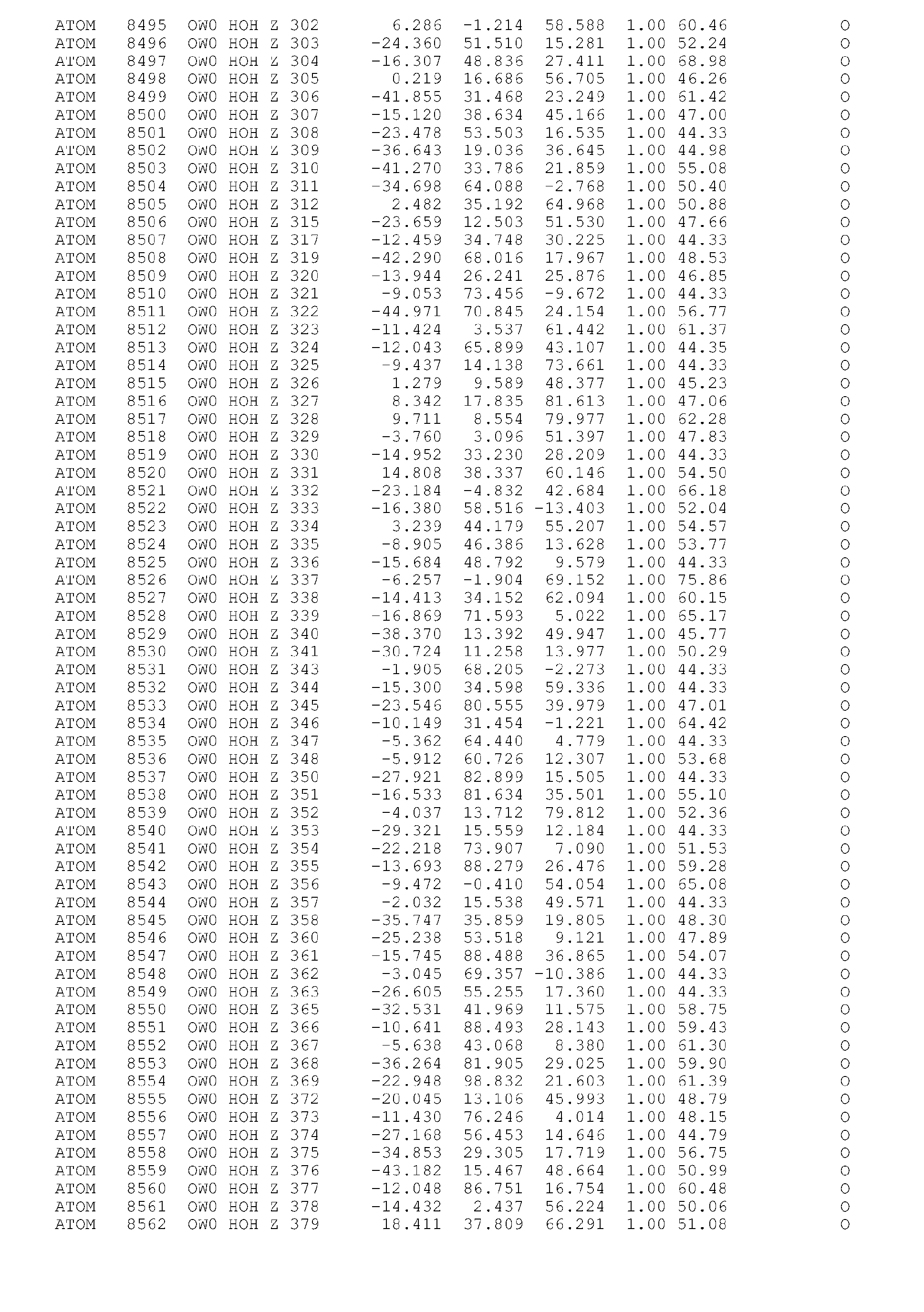

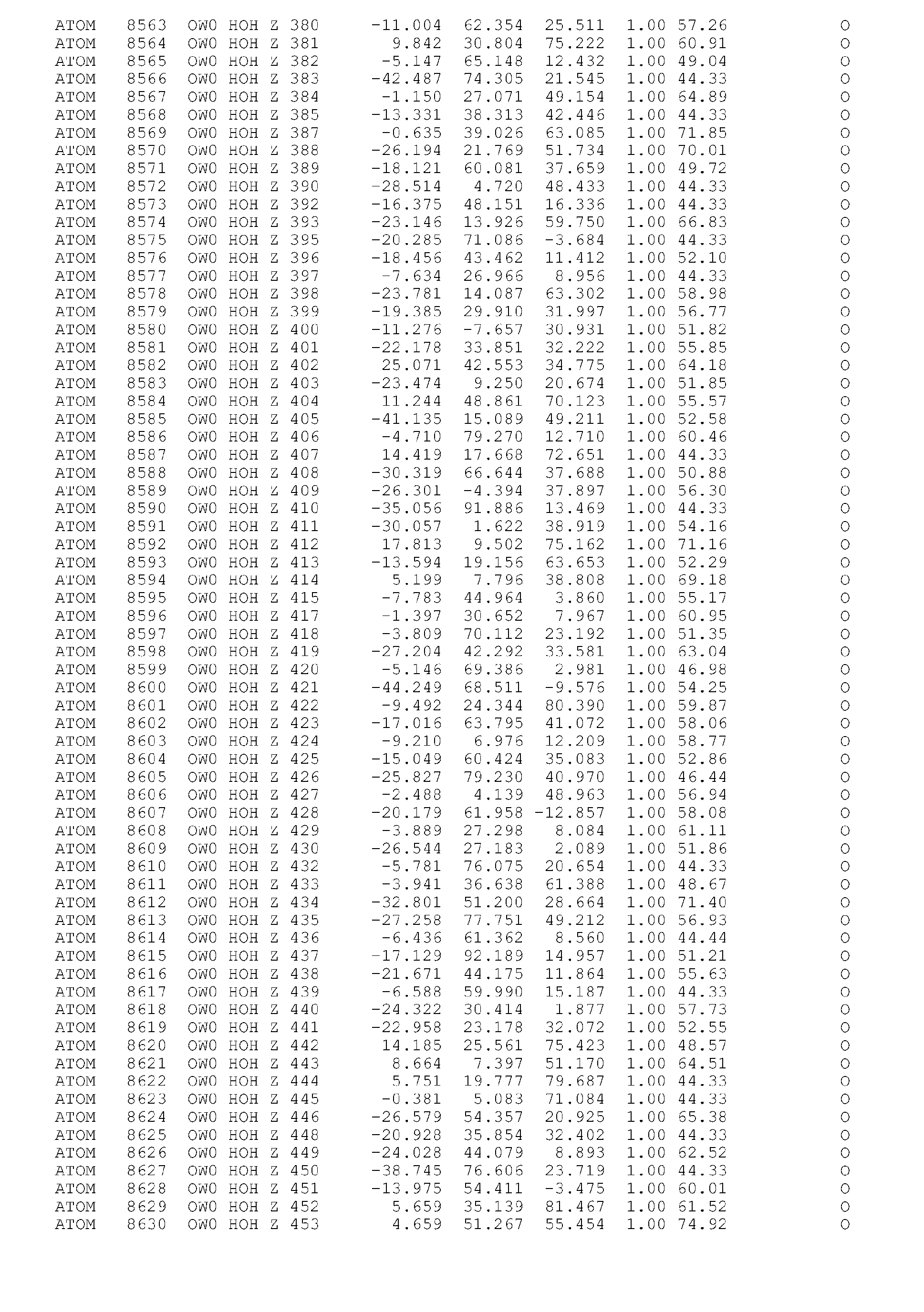

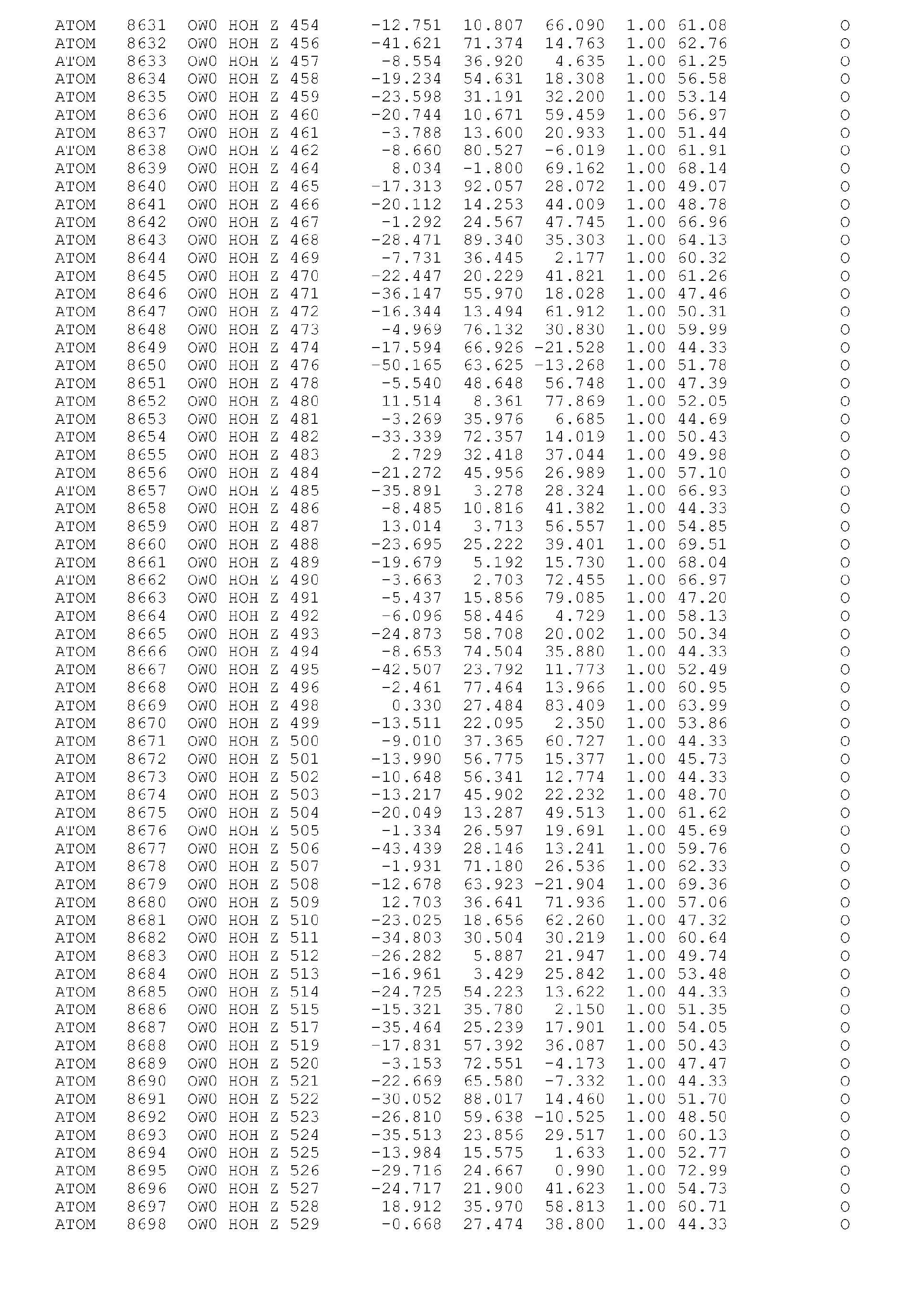

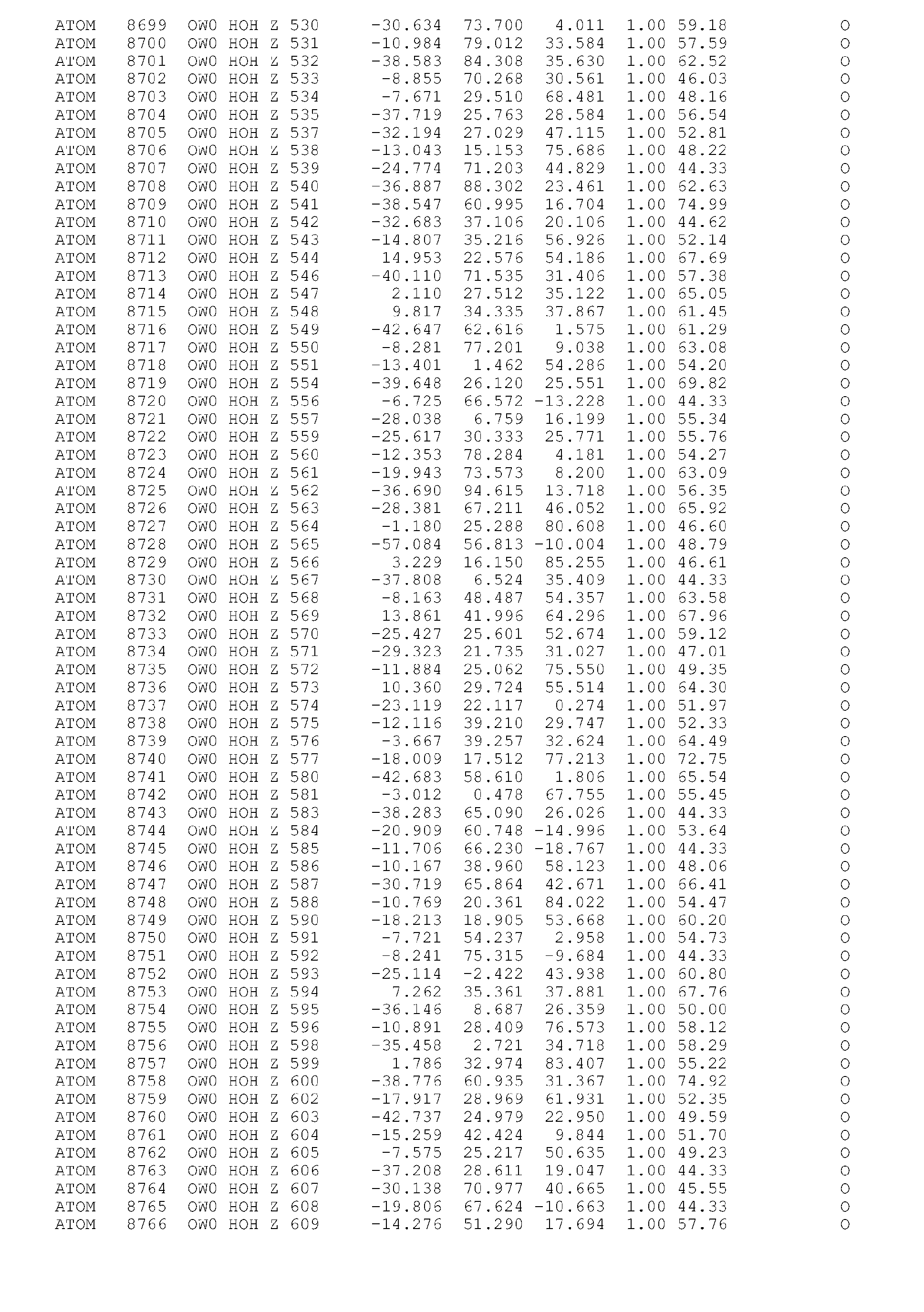

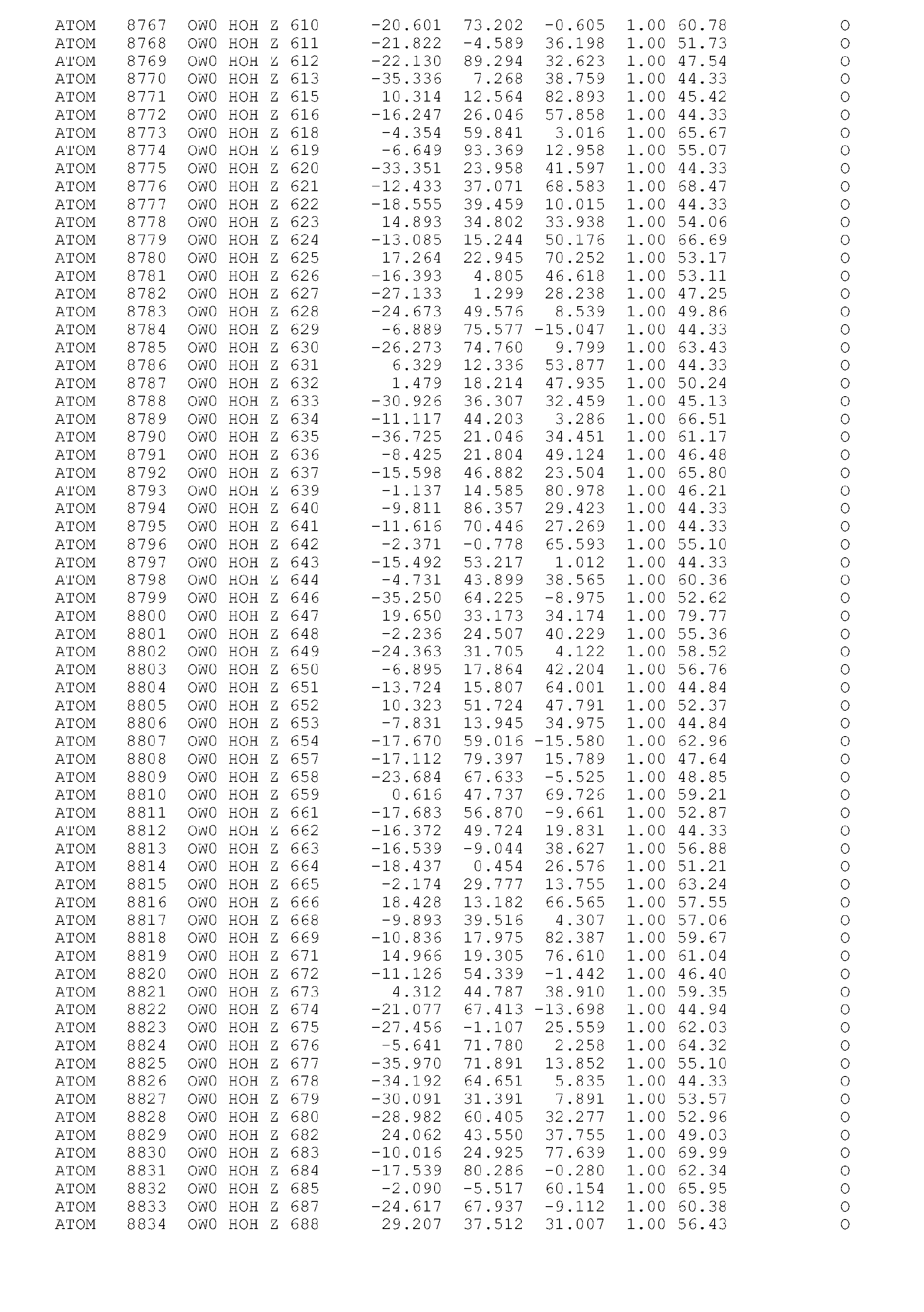

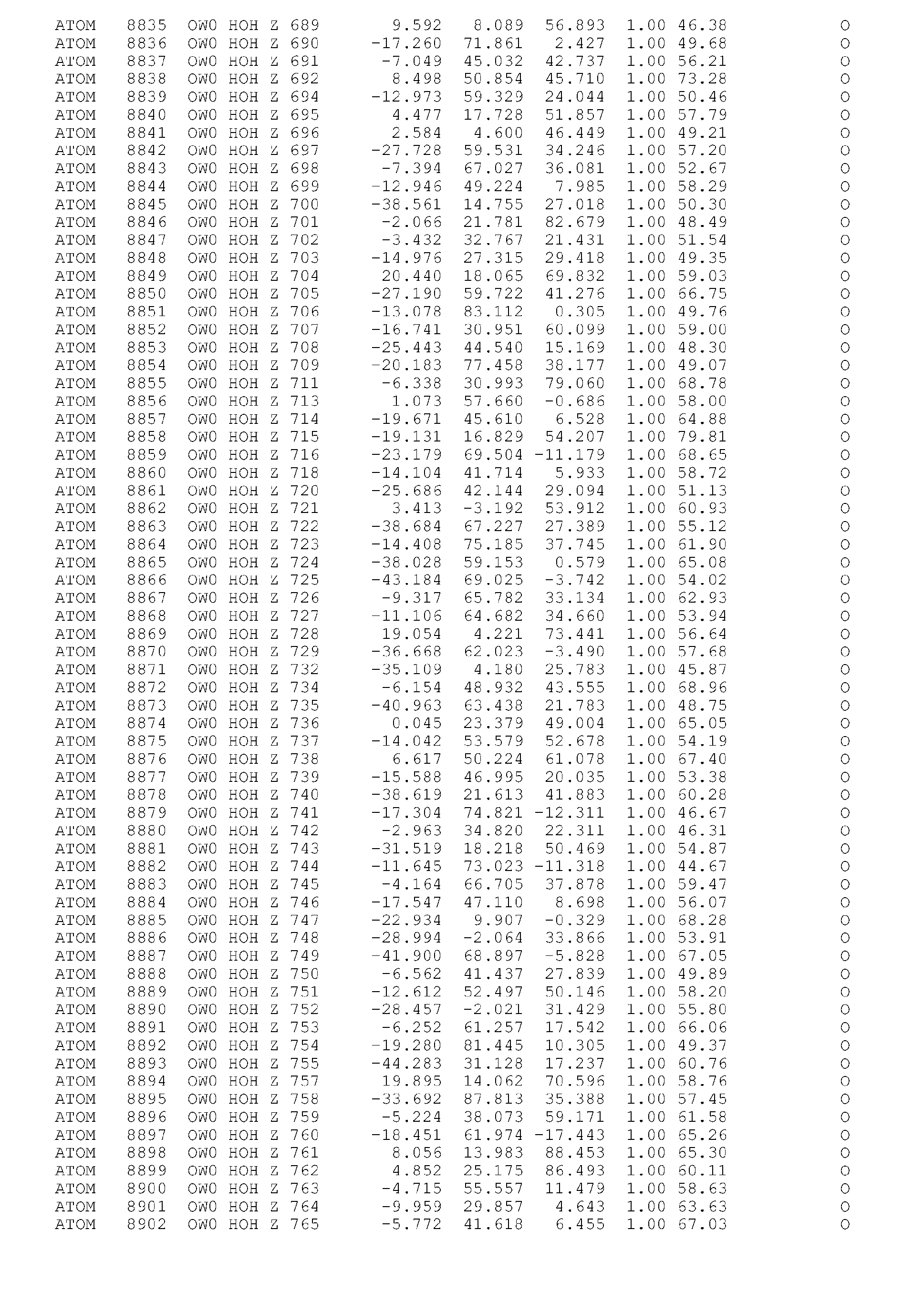

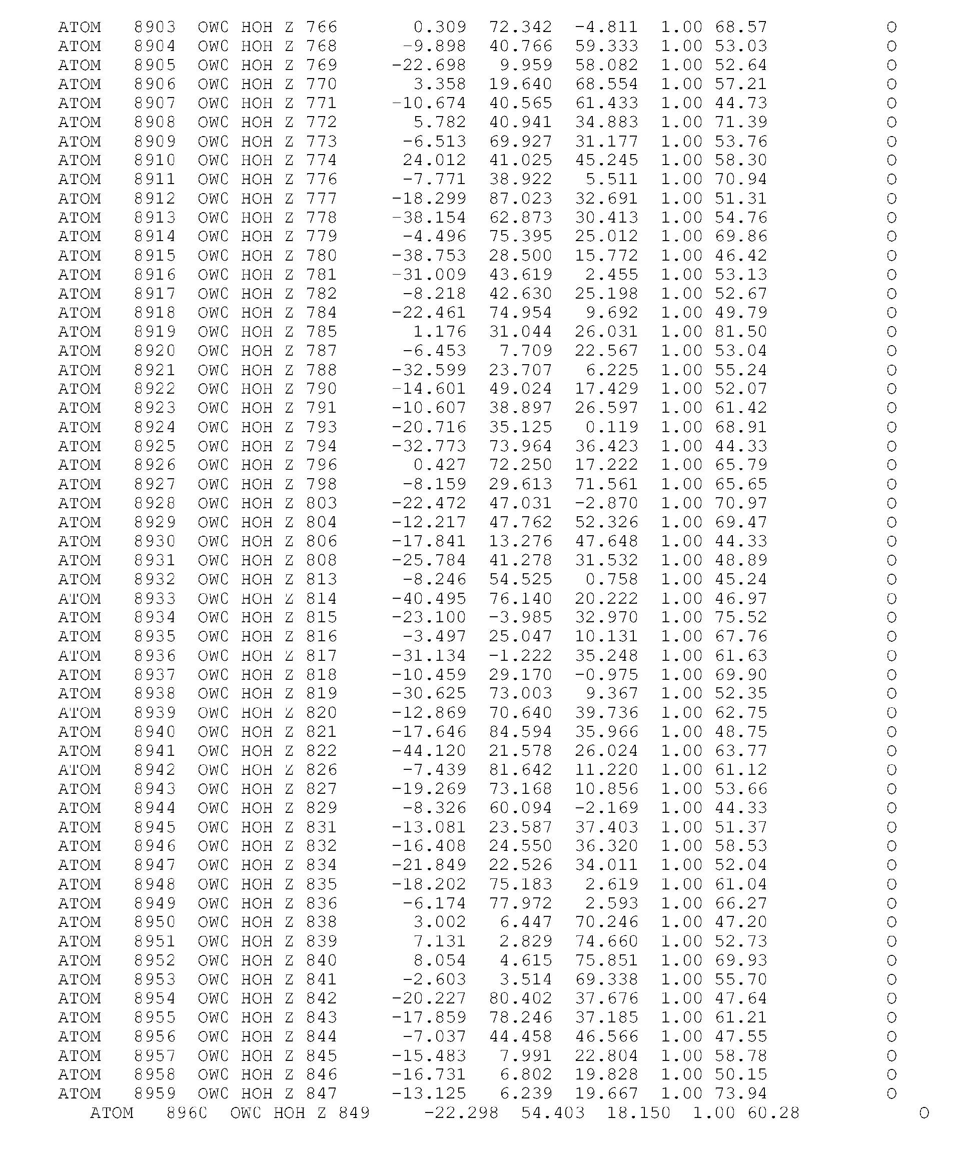

## Claims

1. A crystal comprising PKC alpha kinase catalytic domain and belonging to space group P2₁2₁2₁ with a unit cell dimension of a = 44.2 ± 5 Angstroms, b = 101.3 ± 5 Angstroms, c = 252.0 ± 5 Angstroms.

2. A crystal of the PKC alpha kinase catalytic domain comprising the sequence of SEQ ID NO:1 or a mutant thereof.

3. A crystal of any of claims 1 or 2, wherein the catalytic domain essentially consists of the sequence of SEQ ID. No. 2.

4. A crystal of any of claims 1-3 bound to at least one ligand or low molecular weight compound.

5. A crystal of Claim 4, wherein said ligand is the Compound (I) as defined below:

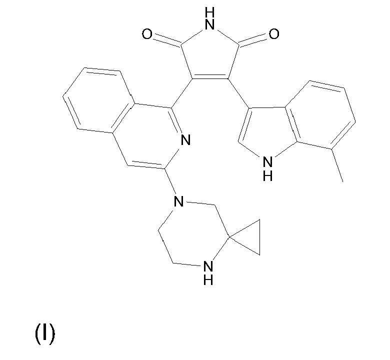

6. A crystal of Claim 5, having a three-dimensional structure **characterized by** the structure coordinates of Table 1.

7. A crystal of Claim 4, wherein said ligand is the Compound (II) as defined below:

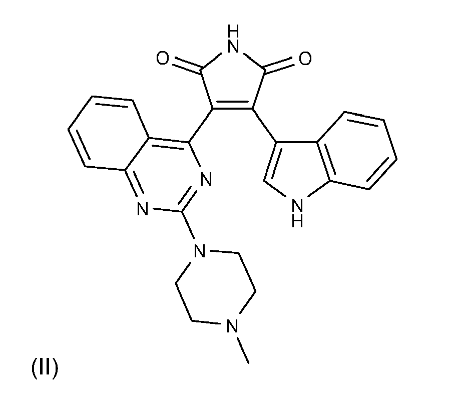

8. A crystal of Claim 7, having a three-dimensional structure **characterized by** the structure coordinates of Table 2.

9. A computer readable medium comprising data storage material encoded with computer readable data wherein said data comprises the structure coordinates of Table 1 or Table 2.

10. A method for making a crystal comprising PKC alpha catalytic domain, said method comprising the steps of:
(i) expressing a construct comprising a nucleotide sequence encoding PKC alpha catalytic domain for production of a PKC alpha protein;
(ii) purifying a sufficient amount of the PKC alpha protein produced at step (i) under appropriate conditions for crystallization; and,
(iii) crystallizing the PKC alpha protein purified at step (ii).

11. The method of Claim 10, wherein in step (i), expression occurs in the presence of an inhibitor, for example in the presence of Compound (I) or Compound (II) as defined below:

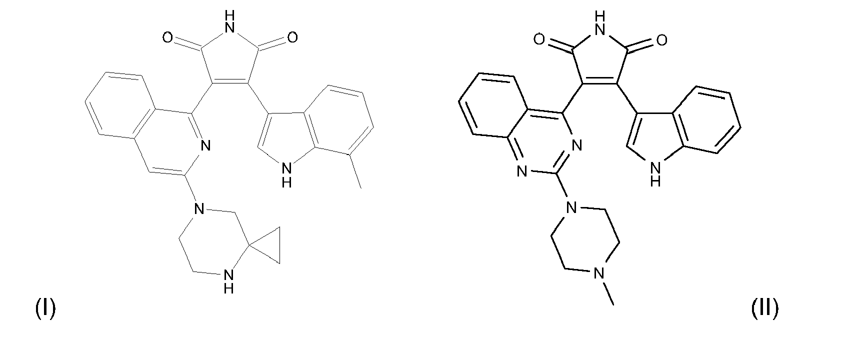

12. The method of Claim 10 or 11, comprising:
(i) expressing a construct comprising the nucleotide sequence encoding PKC alpha catalytic domain as shown in SEQ ID NO:2;
(ii) purifying a sufficient amount of the PKC alpha protein expressed at step (i) over a Ni-NTA column and a size exclusion column at a temperature preferably below 10°C; and,
(iii) crystallizing the PKC alpha protein purified at step (b), using hanging drop vapour diffusion with a drop size of 2 µl protein solution plus 1µl reservoir solution.

13. The method of Claim 12, wherein no ATP is added to the protein after purification over the Ni-NTA column.

14. A crystal comprising the PKC alpha catalytic domain obtainable according to any of the methods of Claims 10 to 13.

15. Use of a crystal according to any one of Claims 1 to 9 or 14, for the generation of crystal structure data.

16. A method for determining the three-dimensional structure of a complex comprising the catalytic domain of Conventional PKC, a fragment or homologue thereof bound to at least one ligand comprising:
(i) obtaining x-ray diffraction data for crystals of the complex,
(ii) utilizing the atomic coordinates of Table 1 or Table 2 to define the three-dimensional structure of the complex.

17. A method of identifying a ligand that binds to PKC alpha comprising the steps of:
(i) using the three dimensional structure of the catalytic domain of PKC alpha to select and/or design a potential ligand that binds to PKC alpha,
(ii) identifying among the potential ligand selected in step (i), those ligands that bind to PKC alpha in an in vitro or cell-based assay.

18. The method of Claim 16, wherein the ligand is selected and/or designed among the potential ligands that virtually bind to the residues lining the ATP binding sites and/or the hydrophobic motif binding site, according to a computer aided modelling programm.

19. The method of Claim 18, wherein the ligand is selected and/or designed among those that virtually bind to at least one or more amino acids of the ATP binding sites selected from the group consisting of : M343 - L355, L364 - 1369, F399 - T409, E418 - L425, D467 - D472 and K478 - F482, and/or at least with one or more amino acids of the regions hydrophobic motif binding sites selected from the group consisting of: Y365 - L393 and H404 - M417.

20. The method of Claim 19, wherein the ligand is selected and/or designed among those that virtually bind to at least one or more amino acids of the ATP binding sites selected from the group consisting of: L345, G346, F350, G351, V353, A366, K368, T401, M417, Y419, V420, M470, A480 and D481, and/or at least with one or more amino acids of the hydrophobic motif binding site selected from the group consisting of: 1367, K372, V375, 1376, V381, T384, M385, K388, C406, Q408, L413 and F415.

21. A method for selecting a ligand that binds to PKC alpha, comprising:
a. co-crystallizing or incubating a candidate compound or mix of compounds with PKC alpha under appropriate conditions;
b. determining by X-ray or NMR methods the amino acids of PKC alpha which interact with the candidate compound; and,
c. selecting the compound which interacts at least with one or more amino acids of the binding pocket selected among the group consisting of M343 - L355, L364 - 1369, F399 - T409, E418 - L425, D467 - D472, K478 - F482, Y365 - L393 and H404 - M417.

22. The method of Claim 21, wherein the ligand is selected and/or designed among those that virtually bind to at least one or more amino acids of the ATP binding sites selected from the group consisting of: L345, G346, F350, G351, V353, A366, K368, T401, M417, Y419, V420, M470, A480 and D481, and/or at least with one or more amino acids of the hydrophobic motif binding sites selected from the group consisting of: 1367, K372, V375, 1376, V381, T384, M385, K388, C406, Q408, L413 and F415.

23. A method to design ligand to PKC alpha, wherein said method comprises:
a. providing a first ligand that binds to one or more amino acids of a first binding region of PKC alpha, for example the ATP binding site;
b. providing a second ligand that binds to one or more amino amino acids of a second binding region of PKC alpha, for example the activation loop; and,
c. linking said first ligand to said second ligand to design a ligand that binds to the first and second binding regions of PKC alpha.


SEQUENCE LISTING

<110> Novartis AG

<120> Crystalline forms of PKC alpha kinase

<130> I0121

<160> 5

<170> PatentIn version 3.2

<210> 1
<211> 353
<212> PRT
<213> Homo sapiens

<400> 1

Pro Ser Glu Asp Arg Lys Gln Pro Ser Asn Asn Leu Asp Arg Val Lys
1               5                   10                  15

Leu Thr Asp Phe Asn Phe Leu Met Val Leu Gly Lys Gly Ser Phe Gly
            20                  25                  30

Lys Val Met Leu Ala Asp Arg Lys Gly Thr Glu Glu Leu Tyr Ala Ile
        35                  40                  45

Lys Ile Leu Lys Lys Asp Val Val Ile Gln Asp Asp Asp Val Glu Cys
    50                  55                  60

Thr Met Val Glu Lys Arg Val Leu Ala Leu Leu Asp Lys Pro Pro Phe
65                  70                  75                  80

Leu Thr Gln Leu His Ser Cys Phe Gln Thr Val Asp Arg Leu Tyr Phe
                85                  90                  95

Val Met Glu Tyr Val Asn Gly Gly Asp Leu Met Tyr His Ile Gln Gln
            100                 105                 110

Val Gly Lys Phe Lys Glu Pro Gln Ala Val Phe Tyr Ala Ala Glu Ile
        115                 120                 125

Ser Ile Gly Leu Phe Phe Leu His Lys Arg Gly Ile Ile Tyr Arg Asp
    130                 135                 140

Leu Lys Leu Asp Asn Val Met Leu Asp Ser Glu Gly His Ile Lys Ile
145                 150                 155                 160

Ala Asp Phe Gly Met Cys Lys Glu His Met Met Asp Gly Val Thr Thr
                165                 170                 175

Arg Thr Phe Cys Gly Thr Pro Asp Tyr Ile Ala Pro Glu Ile Ile Ala
            180                 185                 190

Tyr Gln Pro Tyr Gly Lys Ser Val Asp Trp Trp Ala Tyr Gly Val Leu
        195                 200                 205

Leu Tyr Glu Met Leu Ala Gly Gln Pro Pro Phe Asp Gly Glu Asp Glu
    210                 215                 220

Asp Glu Leu Phe Gln Ser Ile Met Glu His Asn Val Ser Tyr Pro Lys
225                 230                 235                 240

Ser Leu Ser Lys Glu Ala Val Ser Ile Cys Lys Gly Leu Met Thr Lys
                245                 250                 255

His Pro Ala Lys Arg Leu Gly Cys Gly Pro Glu Gly Glu Arg Asp Val
            260                 265                 270

Arg Glu His Ala Phe Phe Arg Arg Ile Asp Trp Glu Lys Leu Glu Asn
        275                 280                 285

Arg Glu Ile Gln Pro Pro Phe Lys Pro Lys Val Cys Gly Lys Gly Ala
    290                 295                 300

Glu Asn Phe Asp Lys Phe Phe Thr Arg Gly Gln Pro Val Leu Thr Pro
305                 310                 315                 320

Pro Asp Gln Leu Val Ile Ala Asn Ile Asp Gln Ser Asp Phe Glu Gly
                325                 330                 335

Phe Ser Tyr Val Asn Pro Gln Phe Val His Pro Ile Leu Gln Ser Ala
            340                 345                 350

Val

<210> 2
<211> 353
<212> PRT
<213> Homo sapiens

<400> 2

Pro Ser Glu Asp Arg Lys Gln Pro Ser Asn Asn Leu Asp Arg Val Lys
1               5                   10                  15

Leu Thr Asp Phe Asn Phe Leu Met Val Leu Gly Lys Gly Ser Phe Gly
            20                  25                  30

Lys Val Met Leu Ala Asp Arg Lys Gly Thr Glu Glu Leu Tyr Ala Ile
        35                  40                  45

Lys Ile Leu Lys Lys Asp Val Val Ile Gln Asp Asp Asp Val Glu Cys
    50                  55                  60

Thr Met Val Glu Lys Arg Val Leu Ala Leu Leu Asp Lys Pro Pro Phe
65                  70                  75                  80

Leu Thr Gln Leu His Ser Cys Phe Gln Thr Val Asp Arg Leu Tyr Phe
                85                  90                  95

Val Met Glu Tyr Val Asn Gly Gly Asp Leu Met Tyr His Ile Gln Gln
            100                 105                 110

Val Gly Lys Phe Lys Glu Pro Gln Ala Val Phe Tyr Ala Ala Glu Ile
        115                 120                 125

Ser Ile Gly Leu Phe Phe Leu His Lys Arg Gly Ile Ile Tyr Arg Asp
    130                 135                 140

Leu Lys Leu Asp Asn Val Met Leu Asp Ser Glu Gly His Ile Lys Ile
145                 150                 155                 160

Ala Asp Phe Gly Met Cys Lys Glu His Met Met Asp Gly Val Thr Thr
                165                 170                 175

Arg Glu Phe Cys Gly Thr Pro Asp Tyr Ile Ala Pro Glu Ile Ile Ala
            180                 185                 190

Tyr Gln Pro Tyr Gly Lys Ser Val Asp Trp Trp Ala Tyr Gly Val Leu
        195                 200                 205

Leu Tyr Glu Met Leu Ala Gly Gln Pro Pro Phe Asp Gly Glu Asp Glu
    210                 215                 220

Asp Glu Leu Phe Gln Ser Ile Met Glu His Asn Val Ser Tyr Pro Lys
225                 230                 235                 240

Ser Leu Ser Lys Glu Ala Val Ser Ile Cys Lys Gly Leu Met Thr Lys
                245                 250                 255

His Pro Ala Lys Arg Leu Gly Cys Gly Pro Glu Gly Glu Arg Asp Val
            260                 265                 270

Arg Glu His Ala Phe Phe Arg Arg Ile Asp Trp Glu Lys Leu Glu Asn
        275                 280                 285

Arg Glu Ile Gln Pro Pro Phe Lys Pro Lys Val Cys Gly Lys Gly Ala
    290                 295                 300

Glu Asn Phe Asp Lys Phe Phe Thr Arg Gly Gln Pro Val Leu Thr Pro
305                 310                 315                 320

Pro Asp Gln Leu Val Ile Ala Asn Ile Asp Gln Ser Asp Phe Glu Gly
                325                 330                 335

Phe Ser Tyr Val Asn Pro Gln Phe Val His Pro Ile Leu Gln Ser Ala
            340                 345                 350

Val

<210> 3
<211> 54
<212> DNA
<213> Homo sapiens

<400> 3
gattcgtcat caatggtgat gatggtgatg tactgcactc tgtaagatgg ggtg 54


<210> 4
<211> 53
<212> DNA
<213> Homo sapiens

<400> 4
ggatccctgc aggcctcgag ttcccaccat gccctctgaa gacaggaaac aac 53


<210> 5
<211> 49
<212> DNA
<213> Homo sapiens

<400> 5
atggatggag tcacgaccag ggagttctgt gggactccag attatatcg 49